# EUROPEAN PATENT SPECIFICATION

(11) **EP 2 097 419 B1**
(45) Date of publication and mention of the grant of the patent: **25.05.2011**
(21) Application number: 08832770.5
(22) Date of filing: 22.12.2008
(51) Int. Cl.: C07D 491/04

(54) **BENZOFUROPYRIMIDINONES AS PROTEIN KINASE INHIBITORS**
BENZOFUROPYRIMIDINONE ALS PROTEINKINASE-HEMMER
BENZOFUROPYRIMIDINONES EN TANT QU'INHIBITEURS DE PROTÉINE KINASE

(30) Priority: 21.12.2007 US 8907 P; 25.03.2008 US 70971 P
(43) Date of publication of application: 09.09.2009
(73) Proprietor: Exelixis, Inc., South San Francisco, CA 94083-0511 (US)
(72) Inventor: BROWN, S., David, San Carlos, CA 94070 (US); DU, Hongwang, Millbrae, CA 94030 (US); FRANZINI, Maurizio, Redwood City, CA 94061 (US); GALAN, Adam, Antoni, Alameda, CA 94501 (US); HUANG, Ping, Mountain View, CA 94040 (US); KEARNEY, Patrick, San Francisco, CA 94110 (US); KIM, Moon, Hwan, Palo Alto, CA 94303 (US); KOLTUN, Elena, S., Foster City, CA 94404 (US); RICHARDS, Steven, James, Brisbane, CA 94005 (US); TSUHAKO, Amy, L., Milpitas, CA 95035 (US); ZAHARIA, Cristiana, A., Foster City, CA 94404 (US)
(74) Representative: Marchant, Michael John
(86) International application number: PCT/US2008/087939
(87) International publication number: WO 2009/086264

(56) References cited:
- EP-A- 1 776 982
- WO-A-95/19970
- WO-A-2004/111058
- WO-A-2006/023381
- WO-A-2006/050965
- DATABASE CHEMCATS [Online] CHEMICAL ABSTRACTS SERVICE, COLUMBUS, OHIO, US; 12 July 2006 (2006-07-12), XP002518019 retrieved from STN & "Aurora Screening Library" 13 June 2008 (2008-06-13), AURORA FINE CHEMICALS LLC , SAN DIEGO, CA, 92126, USA
- H. D. H. SHOWALTER ET AL.: "Tyrosine Kinase Inhibitors. 16. 6,5,6-Tricyclic Benzothieno[3,2-d]pyrimidines and Pyrimido[5,4-b]- and -[4,5-b]indoles as Potent Inhibitors of the Epidermal Growth Factor Receptor Tyrosine Kinase" J. MED. CHEM., vol. 42, no. 26, 9 December 1999 (1999-12-09), pages 5464-5474, XP002518018

## Description

### FIELD OF THE INVENTION

This disclosure relates to certain benzofuropyrimidinone compounds. In particular, this disclosure relates to certain benzofuropyrimidinone compounds useful as inhibitors of protein kinases.

### BACKGROUND OF THE INVENTION

The PIM protein kinase family which consists of the closely related PIM-1, 2, and 3, have been implicated in diverse biological processes such as cell survival, proliferation, and differentiation. PIM-1 is involved in a number of signaling pathways that are highly relevant to tumorigenesis [reviewed in Bachmann & Moroy, Internat. J. Biochem. Cell Biol., 37, 726-730 (2005)]. Many of these are involved in cell cycle progression and apoptosis. It has been shown that PIM-1 acts as an anti-apoptotic factor via inactivation of the pro-apoptotic factor Bad. This finding suggested a direct role of PIM-1 in preventing cell death since the inactivation of Bad can enhance Bcl-2 activity and thereby promotes cell survival [Aho et al., FEBS Letters, 571, 43-49 (2004)]. PIM-1Has also been recognized as a positive regulator of cell cycle progression. PIM-1 binds and phosphorylates CDC25A, which leads to an increase in its phosphatase activity and promotion of G1/S transition [reviewed in Losman et al., JBC, 278, 4800-4805 (1999)]. In addition, the cyclin kinase inhibitor p21^{Waf} which inhibits G1/S progression was found to be inactivated by PIM-1 [Wang et al., Biochim. Biophys. Act. 1593, 45-55 (2002)]. Furthermore, by means of phosphorylation, Pim-1 inactivates C-TAK1 and activates Cdc25C which results in acceleration of G2/M transition [Bachman et al., JBC, 279, 48319-48 (2004)].

PIM-1 appears to be an essential player in hematopoetic proliferation. Kinase active PIM-1 is required for the gp130-mediated STAT3 proliferation signal [Hirano et al., Oncogene 19, 2548-2556, (2000)]

PIM-1 is overexpressed or even mutated in a number of tumors and different types of tumor cell lines and leads to genomic instability. Examples for a possible involvement of PIM-1 in human tumors are prostate cancer, oral cancer, and Burkitt lymphoma (Gaidano & Dalla Faver, 1993). All these findings point to an important role of PIM-1 in the initiation and progression of human cancer, and it appears that small molecule inhibition of PIM-1 activity is a promising therapeutic strategy. Finally, PIM-2 and PIM-3 have overlapping functions with PIM-1 and inhibition of more than one isoform may provide additional therapeutic benefits.

CDC7, a serine/threonine kinase, plays an essential role in initiation of DNA replication in eukaryotic cells (Jiang et al., EMBO J 18:5703 (1999)). After assembly of the pre-replication complex to the replication origin, the CDC7 kinase phosphorylates MCM (minichromosome maintenance) proteins and allows for recruitment of CDC45 and DNA polymerase thereby initiating DNA replication (Kim et al., Mutation Research 532:29(2003)). CDC7 requires association with one of its cofactors, ASK (also known as DBF4) or ASKL1 (also known as Drf1), for kinase activation (Ogino et al., J Biol Chem 276:31376 (2001); Sato et al., Genes to Cells 8:451 (2003); Montagnoli et al., EMBO J 21:3171 (2002); Yoshizawa-Sugata et al., J Biol Chem 280, 13062 (2005)). Mice deficient for CDC7 die between day 3.5 and 6.5 indicating that CDC7 plays a role for early embryonic development (Kim et al., EMBO J 21:2168 (2002)). Conditional knock-down of CDC7 in mouse ES cell lines (CDC7-/-tg) revealed immediate inhibition of cell proliferation, rapid cessation of DNA synthesis and arrest in S phase progression (Kim et al. (2002)). CDC7 has been implicated in DNA damage checkpoint signaling in response to Etoposide treatment or DNA single strand breaks (Costanzo et al., J Mol Cell 11:203 (2003)). A role for CDC7 in DNA damage response is supported by the observation that CDC7 depleted mouse ES cells accumulate RAD51 foci in the nucleus (Kim et al. (2002)). Deletion of CDC7 in yeast results in hypersensitivity to hydroxyurea treatment (Weinreich et al., EMBO J 18:5334 (1999)).

The serine/threonine kinase CDC7 plays an important role in the initiation of DNA replication and recently has been implicated in S phase checkpoint signaling(reviewed in Kim, Yamada and Masai, "Functions of mammalian CDC7 kinase in initiation/monitoring of DNA replication and development" Mutat Res 532(1-2):29-40 (2003)). The CDC7 kinase forms a complex with Dbf4, its regulatory subunit also known as ASK to generate an active Ser/Thr kinase. CDC7/Dbf4 kinase activity is required for initiation of DNA replication and subsequent transition into S-phase of the cell cycle. A second activator protein of CDC7 called Drf1 or ASKL1 has been identified in human cells, and appears to be involved in both S and M phase progression (Montagnoli et al., "Drf1, a novel regulatory subunit for human CDC7 kinase" EMBO J 21(12):3171-81 (2002); Yoshizawa-Sugata, "A second human Dbf4/ASK-related protein, Drf1/ASKL1, is required for efficient progression of S and M phases" Biol Chem 280(13):13062-70 (2005)). CDC7 knock-out mice are embryonic lethal between E3.5 and E6.5 (Kim et al., "Inactivation of CDC7 kinase in mouse ES cells results in S-phase arrest and p53-dependent cell death" EMBO J 21(9):2168-79 (2002)). However, the analysis of conditional CDC7 as well as conditional Dbf4 knock-out ES cell lines revealed the essential roles of both proteins in mammalian cell proliferation and DNA synthesis (Kim et al., "Hypomorphic mutation in an essential cell-cycle kinase causes growth retardation and impaired spermatogenesis" EMBO J 22(19):5260-72 (2003); Yamashita et al, "Functional analyses of mouse ASK, an activation subunit for CDC7 kinase, using conditional ASK knockout ES cells" Genes Cells 10(6):551-63 (2005)).

Recently, CDC7 has emerged as an attractive target for cancer therapy. Depletion of CDC7 using siRNA oligonucleotides results in induction of apoptosis in cancer cell lines while normal dermal fibroblast cells are spared) Montagnoli et al., Cancer Res 64, 7110 (2004)). Further, CDC7 mediated phosphorylation sites on MCM2, MCM4 and MCM6 in tumor cells have been identified, but the functional relevance of those sites remains to be determined (Montagnoli et al., J of Biol Chem 281:10281 (2006); Tsuji et al., Mol Biol Cell 17:4459-4472 (2006); Masai et al., J Biol Chem 281:39249-39261 (2006); Sheu et al., Mol Cell 24:101-113 (2006)). There is evidence that the CDC7/Dbf4 complex is a target of the S checkpoint response to genotoxic stress. In HU-treated S. cerevisiae, Rad53 phosphorylates Dbf4 resulting in a removal of the kinase complex from chromatin and in inhibition of CDC7/Dbf4 kinase activity. Deletion of CDC7 results in HU hypersensitivity (Weinreich M and Stillman B, 1999). Further, Xenopus egg extracts treated with Etoposide, a Topoisomerase II inhibitor used in the clinic as anti-cancer agent, resulted in activation of a DNA damage checkpoint that required ATR, blocking CDC7/Dbf4 kinase activity (Costanzo 2003). This is contrary to recent data indicating that the CDC7/Dbf4 kinase is active during replication stress and contributes to hyper-phosphorylation of MCM2 in response to HU and Etoposide treatment (Tenca P et al., 2007). Further depletion of CDC7 using siRNA in the presence of those drugs increased cell death.
WO 2006/050965 relates to novel pyrimidine compounds and their preparation, for the modulation of the histamine H4 receptor and the treatment or prevention of conditions mediated by the histamine H4 receptor.
WO 2004/111058 discusses heteroaryl-fused pyrimidinyl compounds and compositions thereof and use of such compounds, either alone or in combination with at least one additional therapeutic agent, in the prophylaxis or treatment of proliferative diseases.
WO 2006/023381 relates to treating inflammatory and immune diseases with certain pyrimidinone compounds that bind to CXCR3 receptors.
EP 1 776 982 relates to fused heterocyclic compounds and methods of using them to treat or prevent diseases mediated by the histamine H4 receptor alone or by the histamine H1 and H4 receptors in combination.

Accordingly, there is a need for potent and specific inhibitors of PIM, CDC7 or CK2, or any combination thereof. There is also a need for methods of treating PIM, CDC7, or CK2 mediated diseases, such as cancer, are also needed

### SUMMARY OF THE INVENTION

This disclosure relates to compounds and pharmaceutical compositions of the compounds for inhibiting protein kinases such as PIM (PIM-1, PIM-2 and/or PIM-3), CDC7 or CK2.

One aspect of this dislcosure relates to compounds exemplified by Formula I as described in Claim 1*.*

Another aspect of this dislcosure relates to a pharmaceutical composition, comprising a compound according to Formula I as recited in Claim 1 and a pharmaceutically acceptable carrier, excipient, or diluent.

Another aspect of this dislcosure relates to an in vitro method of inhibiting PIM, CDC7 or CK2 in a cell, comprising contacting the cell, in which inhibition of PIM, CDC7 or CK2 is desired, with a compound according to Formula I as recited in Claim 1.

Another aspect of this dislcosure relates to an in vitro method of inhibiting PIM, CDC7 or CK2 in a cell, comprising contacting a cell in which inhibition of PIM is desired with a pharmaceutical composition comprising a compound according to Formula I as recited in Claim 1 and a pharmaceutically acceptable carrier, excipient, or diluent.

Another aspect of this dislcosure relates to a compound according to Formula I as recited in Claim 1, or a pharmaceutical composition comprising a compound according to Formula I as recited in Claim 1 and a pharmaceutically acceptable carrier, excipient, or diluent, for use in the treatment of cancer selected from pancreatic cancer, prostate cancer, hepatocellular carcinoma, lymphomas, leukemias, colorectal cancer (including colorectal carcinoma).

There are many different aspects of the compounds, pharmaceutical compositions thereof, and methods of use thereof, as described hereinbelow. The transitional term "comprising" as used herein, which is synonymous with "including," "containing," or "characterized by," is inclusive or open-ended and does not exclude additional, unrecited elements or method steps.

The foregoing only summarizes certain aspects of this dislcosure and is not intended to be limiting in nature. These aspects and other aspects and embodiments are described more fully below.

### DETAILED DESCRIPTION OF THE INVENTION

There are many different aspects of the disclosure described hereinbelow, and each aspect is non-limiting in regard to the scope of the disclosure. The terms "aspects" and "embodiments" are meant to be non-limiting regardless of where the terms "aspect" or "embodiment" appears in this specification. The transitional term "comprising" as used herein, which is synonymous with "including," "containing," or "characterized by," is inclusive or open-ended and does not exclude additional, unrecited elements or methods.

This disclosure relates to a compound according to formula I: 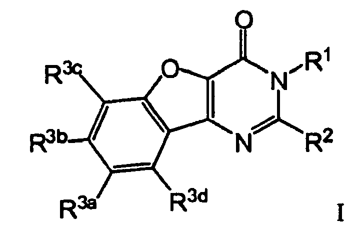 or a pharmaceutically acceptable salt thereof; wherein:
R¹ is hydrogen or alkyl;
R² is selected from aminocarbonylalkylaminoalkyl, aminoalkylaminoalkyl, dialkylaminoalkylaminoalkyl, carboxyalkylaminoalkyl, cycloakylaminoalkyl, dialkylaminoarylalkylaminoalkyl, heteroarylalkylaminoalkyl, arylalkyl optionally substituted at any aryl or alkyl position with 1-3 groups selected from halo and -NH₂, heterocycloalkylarylalkylaminoalkyl optionally substituted at the heterocycloalkyl portion with alkyl, aminoalkyl optionally substituted with 1, 2 or 3 -OH, alkylamino optionally substituted with 1, 2 or 3 -OH, alkylaminoalkyl optionally substituted with 1, 2 or 3 -OH, alkoxyalkylaminoalkyl, heterocycloalkylaminoalkyl optionally substituted with alkyl at the heterocycloalkyl portion, hydroxyalkyl, cycloalkylaminoalkyl, arylamino(alkyl)alkyl optionally substituted at any ring position with 1, 2 or 3 halo, heterocycloalkylalkylaminoalkyl optionally substituted at any ring position with 1-2 alkyl, arylalkylaminoalkyl optionally substituted at the aryl position with dialkylamino, halo, alkoxy, heteroaryl, or alkylheterocycloalkyl, dialkylaminoalkyl optionally substituted with 1, 2 or 3 -OH, arylaminoalkyl optionally substituted at the aryl portion with 1, 2 or 3 groups selected from halo, heterocycloalkyl, alkylheterocycloalkyl and dialkylamino, heteroarylaminoalkyl, arylamino, aryloxyalkyl, unsubstituted heteroarylalkyl, heteroarylalkyl substituted at any alkyl position with alkyl, aryl, arylalkyl or amino, -(C₄-C₇)cycloalkyl optionally substituted with -NH₂, -NHC(O)-O-(CH₃)₃, or aminoalkyl, -N(H)C(O)-O-alkyl, alkylpiperazinylcarbonyl, alkylheterocycloarylalkoxyalkyl, heteroaryl optionally substituted at any ring position with 1, 2 or 3 substituents selected from amino, alkyl, alkylamino, halo, -O-heterocycloalkyl, alkoxy, aminoalkyl, dialkylaminoalkylamino, heterocycloalkylalkylamino and heterocycloalkyl, heterocycloalkylalkylamino, heterocycloalkylalkyl optionally substituted with 1, 2 or 3 R⁴ groups at any ring position, aryl substituted with 1, 2 or 3 R⁵ groups at any ring position, -NHC(O)R⁷, aminoalkylamino, -CR¹¹R¹², heterocycloalkyl optionally substituted with 1, 2 or 3 R¹⁰ groups, heteroarylalkyl substituted at any ring position with 1, 2 or 3 alkyl, halo, aryl or arylalkyl groups, heteroarylamino, heterocycloalkylalkoxyalkyl, dialkylaminoalkylamino, heterocycloalkylamino, carboxyalkyl, arylalkylamino optionally substituted with heterocycloalkyl or heterocycloalkylalkyl, and heterocycloalkyloxyalkyl;
or R¹ and R², together with the carbon atoms to which they are attached, join to form a five membered heterocycloalkyl ring;
R^{3a} is selected from halo, alkyl, -NO₂ alkoxy, alkynyl optionally substituted with R¹⁴, alkoxycarbonylalkyl, arylalkoxy, -C(O)N(H)alkyl, -N(H)-C(O)-alkyl, - C(O)-alkyl, -CN, phenyl, -OCF₃, -N(H)R¹³, -OH, -CF₃, -S-CH₃ and hydroxymethylalkynyl;
R^{3b}, R^{3c} and R^{3d} are each independently selected from H, -OH, -N⁺(O)OH, alkoxyl, and halo;
or R^{3a} is hydrogen and R^{3b}, R^{3c} and R^{3d} are each independently selected from -CF₃, -OH, alkoxy, and halo;
or R^{3a} and R^{3d}, together with the carbons to which they are attached, join to form a 5 membered heteroaryl optionally substituted with methyl or -NH₂, or a 5-6 membered heterocycloalkyl;
R⁴ is selected from -OH, amino, aminoalkyl, halo, alkyl optionally substituted with -OH, alkoxy, alkylaminoalkyl, heteroarylalkyl, -C(O)OH, -C(O)-O-alkyl, -C(O)-alkyl, oxo, aryl optionally substituted with alkyl, arylalkyl or halo, heteroaryl, -OH, dialkylamino, dialkylaminoalkyl, alkylamino, spiro-heterocycloalkyl, -NHC(O)R⁸, -C(O)NHR⁹, arylalkylaminocarbonyl optionally substituted with halo at any ring position of the aryl, heterocycloalkylalkylamino, dialkylaminoalkylcarbonyl, dialkylaminocarbonylalkyl, heterocycloalkylalkyl optionally substituted with -CF₃, heterocycloalkyl optionally substituted with alkyl, arylalkyl optionally substituted with
   -CF₃, alkoxyalkyl and heterocycloalkylcarbonyl optionally substituted with -OH or halo;
R⁵ is selected from alkyl, -OH, amino, aminoalkyl, -C(O)N(H)-heteroarylalkyl, halo, -NO₂ -C(O)-N(H)-heterocycloalkylalkyl, alkylaminoalkyl, heteroaryl, cycloalkylaminoalkyl, alkylamino, dialkylamino, -C(O)Oalkyl, -C(O)OH, heterocycloalkyl, -N(H)-alkylheterocycloalkylC(O)-O-alkyl, -O-alkyl-C(O)-N(H)-alkylcycloalkyl, -C(O)-N(H)-alkyl, -C(O)N(H)alkylaryl, -C(O)N(H)-cycloalkyl, alkylthio, alkylsulfonyl, -O-alkylheterocycloalkyl, heteroarylalkylamino, -CF₃, heterocycloalkylalkylamino optionally substituted with alkyl at any ring position, alkylsulfonyl, -NHC(O)R⁶, alkoxycarbonylheterocycloalkylaminoalkyl, heterocycloalkylaminoalkyl optionally substituted at the heterocycloalkyl portion with alkoxycarbonyl, dialkylaminoalkyl, dialkylaminoalkylamino, and alkoxy;
R⁶ is selected from dialkylaminoalkyl, heteroarylamino, heterocycloalkyl, heterocycloalkylalkyl optionally substituted with -OH, cycloalkyl, heteroarylalkyl, alkoxyalkyl, heterocycloalkyl, heteroaryl optionally substituted with 1, 2 or 3 groups selected from halo, -NH₂, aminoalkylaminocarbonyl, heteroaryl, hydroxyalkyl, alkoxy, alkyl, -C(O)-O-alkyl and -C(O)-O-H, alkyl, alkoxy, and aryl optionally substituted 1, 2 or 3 halo, -N(H)C(O)CH₃, alkyl or alkoxy;
R⁷ is selected from heterocycloalkylalkyl, arylalkyl optionally substituted at any ring position with 1, 2 or 3 halo groups, dialkylaminoalkyl, heterocycloalkyl, heteroaryl optionally substituted at any ring position with 1, 2 or 3 groups selected from halo and -COOH, and alkoxyalkyl;
R⁸ is selected from arylalkyl, heterocycloalkyl and alkyl;
R⁹ is selected from H, alkyl, arylalkyl optionally substituted with halo at any ring position of the aryl, heterocycloalkyl, arylalkylaminocarbonyl optionally substituted with halo and dialkylaminoalkyl;
R¹⁰ is selected from alkyl, oxo, heteroaryl, dialkylaminoalkylcarbonyl, dialkylaminocarbonylalkyl, aminoalkyl, -OH, halo, heteroarylcarbonyl, dialkylaminoalkyl, heterocycloalkyl optionally substituted with alkyl, -C(O)-O-alkyl, arylalkylcarbonyl, arylcarbonyl, alkylcarbonyl, alkoxyalkylcarbonyl, heterocycloalkylcarbonyl, heteroarylalkyl, -O-heterocycloalkyl and arylalkyl optionally substituted with alkoxy or arylalkoxy;
R¹¹ is selected from aryl optionally substituted with halo, heteroarylalkyl, cycloalkyl, spiro-cycloalkyl and arylalkyl optionally substituted with alkoxy or phenylmethylmethoxy;
R¹² is selected from -NH₂ and heterocycloalkyl optionally substituted with alkyl;
R¹³ is selected from arylalkyl wherein the aryl portion of arylalkyl is optionally substituted with 1, 2 or 3 alkoxy, halo, methyl, methoxy, -CF₃, cycloalkyl (such as cyclohexyl), and heteroarylalkyl (such as pyridinealkyl); and
R¹⁴ is selected from hydroxylalkyl, H and TMS.

Optionally, R^{3a} is selected from halo, alkyl,-NO₂, alkoxy, alkynyl optionally substituted with R¹⁴, alkoxycarbonylalkyl, arylalkoxy,
-C(O)N(H) alkyl, -N(H)-C(O)-alkyl, -C(O)-alkyl, -CN, phenyl, -OCF₃, -N(H)R¹³,-OH,
-CF₃, -S-CH₃ and hydroxymethylalkynyl; and
R^{3b}, R^{3c} and R^{3d} are each H.

Optionally, R^{3a} is halo, alkoxy or -OCF₃; and R^{3b}, R^{3c} and R^{3d} are each H.
This disclosure also relates to a compound of Formula I, 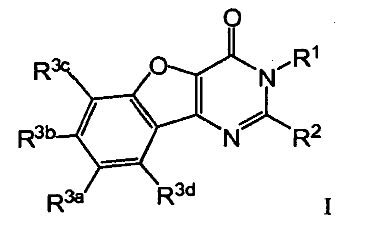 or a pharmaceutically acceptable salt thereof; wherein:
R¹ is hydrogen or alkyl;
R² is selected from aminocarbonylalkylaminoalkyl, aminoalkylaminoalkyl, dialkylaminoalkylaminoalkyl, carboxyalkylaminoalkyl, cycloakylaminoalkyl, dialkylaminoarylalkylaminoalkyl, heteroarylalkylaminoalkyl, arylalkyl, heterocycloalkylarylalkylaminoalkyl optionally substituted at the heterocycloalkyl portion with alkyl, alkoxyalkylaminoalkyl, heterocycloalkylaminoalkyl optionally substituted with alkyl at the heterocycloalkyl portion, hydroxyalkyl, cycloalkylaminoalkyl, arylamino(alkyl)alkyl optionally substituted at any ring position with 1, 2 or 3 halo, heterocycloalkylalkylaminoalkyl optionally substituted at any ring position with 1-2 alkyl, arylalkylaminoalkyl optionally substituted at the aryl position with dialkylamino, halo, alkoxy, heteroaryl, or alkylheterocycloalkyl, arylaminoalkyl optionally substituted at the aryl portion with 1, 2 or 3 groups selected from halo, alkylheterocycloalkyl and dialkylamino, heteroarylaminoalkyl, arylamino, aryloxyalkyl, heteroarylalkyl optionally substituted with arylalkyl, alkyl or aryl,-N(H)C(O)-O-alkyl, alkylpiperazinylcarbonyl, alkylheterocycloarylalkoxyalkyl, heteroaryl optionally substituted at any ring position with 1, 2 or 3 substituents selected from -O-heterocycloalkyl, dialkylaminoalkylamino and heterocycloalkylalkylamino, heterocycloalkylalkylamino, heterocycloalkylalkyl optionally substituted with 1, 2 or 3 R⁴ groups at any ring position, aryl substituted with 1, 2 or 3 R⁵ groups at any ring position, -NHC(O)R⁷, aminoalkylamino,-CR¹¹R¹², heterocycloalkyl optionally substituted with 1, 2 or 3 R¹⁰ groups, heteroarylalkyl optionally substituted at any ring position with 1, 2 or 3 alkyl or aryl groups, heteroarylamino, heterocycloalkylalkoxyalkyl, dialkylaminoalkylamino, heterocycloalkylamino, carboxyalkyl, and heterocycloalkyloxyalkyl;
or R¹ and R², together with the carbon atoms to which they are attached, join to form a five membered heterocycloalkyl ring;
R^{3a} is selected from halo, alkyl, -NO₂ alkoxy, alkynyl optionally substituted with R¹⁴, alkoxycarbonylalkyl, arylalkoxy, -C(O)N(H)alkyl, -N(H)-C(O)-alkyl, - C(O)-alkyl, -CN, phenyl, -OCF₃, -N(H)R¹³, -OH, -CF₃, -S-CH₃ and hydroxymethylalkynyl;
R^{3b}, R^{3c} and R^{3d} are each independently selected from H, -OH, -N⁺(O)OH, alkoxyl, and halo;
or R^{3a} is hydrogen and R^{3b}, R^{3c} and R^{3d} are each independently selected from -CF₃, -OH, alkoxy, and halo;
or R^{3a} and R^{3d}, together with the carbons to which they are attached, join to form a 5 membered heteroaryl optionally substituted with methyl or -NH₂, or a 5-6 membered heterocycloalkyl;
R⁴ is selected from -OH, amino, aminoalkyl, halo, alkyl optionally substituted with -OH, alkoxy, alkylaminoalkyl, heteroarylalkyl, -C(O)OH, -C(O)-O-alkyl, -C(O)-alkyl, oxo, aryl optionally substituted with alkyl or halo, heteroaryl, -OH, dialkylamino, dialkylaminoalkyl, alkylamino, spiro-heterocycloalkyl, -NHC(O)R⁸, -C(O)NHR⁹, arylalkylaminocarbonyl optionally substituted with halo at any ring position of the aryl, heterocycloalkylalkylamino, dialkylaminoalkylcarbonyl, dialkylaminocarbonylalkyl, heterocycloalkylalkyl optionally substituted with -CF₃, heterocycloalkyl optionally substituted with alkyl, alkoxyalkyl, arylalkyl optionally substituted with -CF₃, and heterocycloalkylcarbonyl optionally substituted with -OH or halo;
R⁵ is selected from -C(O)N(H)-heteroarylalkyl, -C(O)-N(H)-heterocycloalkylalkyl, alkylaminoalkyl, cycloalkylaminoalkyl, -N(H)-alkylheterocycloalkylC(O)-O-alkyl, -O-alkyl-C(O)-N(H)-alkylcycloalkyl, -C(O)N(H)alkylaryl, -C(O)N(H)-cycloalkyl, -O-alkylheterocycloalkyl, heteroarylalkylamino, heterocycloalkylalkylamino optionally substituted with alkyl at any ring position, alkoxycarbonylheterocycloalkylaminoalkyl, heterocycloalkylaminoalkyl optionally substituted at the heterocycloalkyl portion with alkoxycarbonyl, dialkylaminoalkyl and dialkylaminoalkylamino;
R⁶ is selected from dialkylaminoalkyl, heteroarylamino, heterocycloalkyl, heterocycloalkylalkyl optionally substituted with -OH, cycloalkyl, heterocycloalkylalkyl, heteroarylalkyl, alkoxyalkyl, heterocycloalkyl, heteroaryl optionally substituted with 1, 2 or 3 groups selected from halo, -NH₂, aminoalkylaminocarbonyl, heteroaryl, hydroxyalkyl, alkoxy, alkyl, -C(O)-O-alkyl and -C(O)-O-H, alkyl, alkoxy, and aryl optionally substituted 1, 2 or 3 halo, - N(H)C(O)CH₃, alkyl or alkoxy;
R⁷ is selected from heterocycloalkylalkyl, arylalkyl optionally substituted at any ring position with 1, 2 or 3 halo groups, dialkylaminoalkyl, heterocycloalkyl, heteroaryl optionally substituted at any ring position with 1, 2 or 3 groups selected from halo and
   -COOH, and alkoxyalkyl;
R⁸ is selected from arylalkyl, heterocycloalkyl and alkyl;
R⁹ is selected from H, alkyl, arylalkyl optionally substituted with halo at any ring position of the aryl, heterocycloalkyl, arylalkylaminocarbonyl optionally substituted with halo and dialkylaminoalkyl;
R¹⁰ is selected from alkyl, oxo, heteroaryl, dialkylaminoalkylcarbonyl, dialkylaminocarbonylalkyl, aminoalkyl, -OH, halo, heteroarylcarbonyl, dialkylaminoalkyl, heterocycloalkyl (piperidinyl) optionally substituted with alkyl, - C(O)-O-alkyl, arylalkylcarbonyl, arylcarbonyl, alkylcarbonyl, alkoxyalkylcarbonyl, heterocycloalkylcarbonyl, heteroarylalkyl, -O-heterocycloalkyl and arylalkyl;
R¹¹ is selected from aryl optionally substituted with halo, heteroarylalkyl, cycloalkyl, spiro-cycloalkyl and arylalkyl optionally substituted with alkoxy or phenylmethylmethoxy;
R¹² is selected from -NH₂ and heterocycloalkyl optionally substituted with alkyl;
R¹³ is selected from arylalkyl wherein the aryl portion of arylalkyl is optionally substituted with 1, 2 or 3 alkoxy, halo, methyl, methoxy; -CF₃, cycloalkyl (such as cyclohexyl), and heteroarylalkyl (such as pyridinealkyl); and
R¹⁴ is selected from hydroxylalkyl, H and TMS.

All of the compounds disclosed herein include either their free base form or their pharmaceutically acceptable salts whether it is stated in the specification that these compounds can exist as their pharmaceutically acceptable salt or not. So, for instance, for any given embodiment of the compound of Formula I (including embodiments relating to the compounds themselves or use thereof), this embodiment includes either its free base form or any of its pharmaceutically acceptable salts, whether this is stated within this embodiment or not.

Optionally, R₂ is -CH₂-R₁₅, wherein R₁₅ is selected from aminocarbonylalkylamino, dialkylaminoalkylamino, carboxyalkylamino, cycloakylamino, dialkylaminoarylalkylamino, heteroarylalkylamino, heterocycloalkylarylalkylaminol optionally substituted at the heterocycloalkyl portion with alkyl, amino, alkylamino optionally substituted with 1, 2 or 3 -OH, alkoxyalkylamino, heterocycloalkylamino optionally substituted with alkyl at the heterocycloalkyl portion, cycloalkylamino, arylamino(alkyl) optionally substituted at any ring position with 1, 2 or 3 halo, 4-(4-methylpiperazine-1yl) phenyl] methyloxy, heterocycloalkylalkylamino optionally substituted at any ring position with alkyl, arylalkylamino optionally substituted at the aryl position with dialkylamino, halo, alkoxy, heteroaryl, or alkylheterocycloalkyl, dialkylamino optionally substituted with 1, 2 or 3 -OH, and arylamino optionally substituted at the aryl portion with 1, 2 or 3 groups selected from halo, alkylheterocycloalkyl and dialkylamino.

Optionally, R^{3a} is halo, alkyl, -NO₂, alkoxy, alkynyl optionally substituted with R¹⁴, alkoxycarbonylalkyl, arylalkoxy, - C(O)N(H)alkyl,
-N(H)-C(O)-alkyl, -C(O)-alkyl, -CN, phenyl, -OCF₃, -N(H)R¹³, -OH, -CF₃, -S-CH₃ and hydroxymethylalkynyl; and
R^{3b}, R^{3c} and R^{3d} are each H.

Optionally, R^{3a} is halo, alkoxy or -OCF_{3;} and R^{3b}, R^{3c} and R^{3d} are each H.

In the options for formula I as described herein, R² in formula I is a heterocycloalkyl selected from azetidinyl, pyrrolidinyl, piperazinyl and piperidinyl optionally substituted with 1, 2 or 3 R¹⁰.

In the options for formula I as described herein, R² in formula I is a heterocycloalkyl selected from azetidinyl, pyrrolidinyl, piperazinyl and piperidinyl optionally substituted with 1, 2 or 3 R¹⁰.

In the options for formula I as described herein, R² in formula I is a heterocycloalkyl selected from azetidinyl, pyrrolidinyl, piperazinyl and piperidinyl optionally substituted with 1, 2 or 3 R¹⁰; R^{3a} is halo, alkoxy or -OCF₃; and R^{3b}, R^{3c} and R^{3d} are each H.

In the options for formula I as described herein, R² in formula I is a heteroaryl optionally substituted at any ring position with 1, 2 or 3 substituents selected from amino, alkylamino, halo, -O-heterocycloalkyl, alkoxy, aminoalkyl, dialkylaminoalkylamino, heterocycloalkylalkylamino and heterocycloalkyl.

In the options for formula I as described herein, R² in formula I is a heteroaryl optionally substituted at any ring position with 1, 2 or 3 substituents selected from amino, alkylamino, halo, -O-heterocycloalkyl, alkoxy, aminoalkyl, dialkylaminoalkylamino, heterocycloalkylalkylamino and heterocycloalkyl; R^{3a} is halo, alkoxy or -OCF_{3;} and R^{3b}, R^{3c} and R^{3d} are each H.

In the options for formula I as described herein, R² in formula I is a heterocycloalkylalkyl optionally substituted with 1, 2 or 3 R⁴ groups at any ring position.

In the options for formula I as described herein, R¹ in formula I is H.

In the options for formula I as described herein, R² in formula I is a heterocycloalkylalkyl optionally substituted with 1, 2 or 3 R⁴ groups at any ring position; R^{3a} is halo, alkoxy or -OCF_{3;} and R^{3b}, R^{3c} and R^{3d} are each H.

In the options for formula I as described herein, R² in formula I is an aryl substituted with 1, 2 or 3 R⁵ groups at any ring position.

In the options for formula I as described herein, R² in formula I is an aryl substituted with 1, 2 or 3 R⁵ groups at any ring position; R^{3a} is halo, alkoxy or -OCF₃; and R^{3b}, R^{3c} and R^{3d} are each H.

In the options for formula I as described herein, R² in formula I is a heterocycloalkyl optionally substituted with 1, 2 or 3 R¹⁰ group.

In the options for formula I as described herein, R² in formula I is a heterocycloalkyl optionally substituted with 1, 2 or 3 R¹⁰ groups; R^{3a} is halo, alkoxy or -OCF₃; and R^{3b}, R^{3c} and R^{3d} are each H.

In the options for formula I as described herein, R² in formula I is a heteroarylalkyl optionally substituted at any ring position with 1, 2 or 3 alkyl or aryl groups.

In the options for formula I as described herein, R² in formula I is a heteroarylalkyl optionally substituted at any ring position with 1,2 or 3 alkyl or aryl groups; R^{3a} is halo, alkoxy or -OCF₃; and R^{3b}, R^{3c} and R^{3d} are each H.

In the options for formula I as described herein, R¹ in formula I is heteroaryl, such as pyridinyl or imidazolyl, which can be optionally substituted as described above in formula I.

In the options for formula I as described herein, R² in formula I is heterocycloalkyl, such as morpholinyl, piperidinyl, pyrrolidinyl, piperazinyl or tetrahydrofuan, which can be optionally substituted as described above in formula I.

In the options for formula I as described herein, R² in formula I is heterocycloalkylaminoalkyl, wherein the heterocycloalkyl potion is piperidinyl, piperazinyl, pyrrolidinyl or imidazolyl, which can be optionally substituted as described above in formula I.

In the options for formula I as described herein, R² in formula I is heterocycloalkylalkyl, wherein the heterocycloalkyl potion is morpholinyl, piperidinyl, pyrrolidinyl, piperazinyl or tetrahydrofuanyl, which can be optionally substituted as described above in formula I.

In the options for formula I as described herein, R² in formula I is alkylheterocycloalkyl, wherein the heterocycloalkyl potion is piperizinyl, which can be optionally substituted as described above in formula I.

In the options for formula I as described herein, R² in formula I is heteroaryl, such as thienyl, furan, pyrazol, thiazol, isoxazol, tetrahydroisoquinolinyl and imidazol, which can be optionally substituted as described above in formula I.

In the options for formula I as described herein, R² in formula I is phenyl which is substituted as described above in formula I.

In the options for formula I as described herein, R⁶ is heteroaryl, such as imidazol, oxazole, pyridine, pyrimidine, isoxazole or furanyl, which can be optionally substituted as described above in formula I.

In the options for formula I as described herein, R^{3a} is Br, Cl or -OCH₃, and R^{3b}, R^{3c} and R^{3d} are each H.

In the options for formula I as described herein, R¹ is H.

In the options for formula I as described herein, R² is heterocycloalkyl, such as piperidinyl, piperazinyl, pyrrolidinyl and morpholinyl, which can be optionally substituted as described above in formula I.

In the options for formula I as described herein, R² is selected from -(C₁-C₃)alkyl-phenyl optionally substituted with 1-3 halo, -NH-phenyl,
-NH-piperidinyl, -NH-pyridinyl, -NH(C₁-C₃)alkylphenyl optionally substituted at any phenyl position with piperazinyl or methylpiperazinyl, -NH(C₁-C₃)alkyl-N(CH₃)₂, -NH(C₁-C₃)alkyl-OH, phenyl substituted with 1, 2 or 3 Xa, phenyl substituted with 0-2 Xb and 1 Xc group, methylpiperazinylphenylalkoxyalkyl (methylpiperazinylphenylmethoxymethyl), methylpiperazinylcarbonyl, 2-chlorophenyl-4-methylpiperazinylmethyl, (4-methylpiperazin-1-yl)(phenyl)methyl, 1-(4-methylpiperazin-1-yl)-2-phenylethyl, 2-chlorophenyl(4-methylpiperazin-1-yl)methyl, 4-oxo-3-phenyl-1,3,8-triazaspiro[4.5]dec-1-yl)methyl, -(C₁-C₃)alkylC(O)OH, hydroxyalkyl, -(C₁-C₃)alkyl-N(Rza)-aryl optionally substituted with chloro, fluoro, piperazinyl, methylpiperazinyl and dialkylamino, -(5-10)membered heteroaryl optionally substituted with 1 or 2 groups selected from halo, -(C₁-C₆)alkyl, -(C₁-C₆)alkoxy, piperidinylalkylamino, piperdinylalkylamino, alkylamino, aminoalkyl, dialkylaminoalkylamino, piperidinyloxy, piperidinyl, and amino, -(C₁-C₃)alkyl-O-phenyl, -(C₁-C₃)alkyl-O-(C₁-C₃)alkyl-(5-6 membered)heterocycloalkyl, -(C₁-C₃)alkyl-N(H)-heteroaryl, -(C₁-C₃)alkyl-(5-10)membered heteroaryl optionally substituted with -(C₁-C₃)alkyl, halo or phenyl, oxopyrrolidinyl optionally substituted with OH or piperidinyl, -(C₁-C₄)alkyl-(3-9 membered)heterocycloalkyl optionally substituted at the (3-9 membered)heterocycloalkyl with Xd, -(C₁-C₆)alkyl-NRzb-(C₁-C₄)alkyl wherein the - (C₁-C₄)alkyl portion is optionally substituted with Xe, -(C₁-C₃)alkyl-NH-(C₃-C₆)cycloalkyl, -(CH₂)-NH-(C₃-C₆)cyclohexyl, -(C₁-C₃)alkyl-NH₂, wherein the -(C₁-C₃)alkyl- portion of -(C₁-C₃)alkyl-NH₂ is optionally substituted with Xf, -(3-9 membered)heterocycloalkyl optionally substituted with Xg, -(C₃-C₆)-cycloalkyl (cyclohexyl) optionally substituted with amino, -NHC(O)-O-(CH₃)₃, aminoalkyl and dialkylaminoalkylamino;
Xa is selected from halo, phenyl substituted with a group selected from - COOH,
-COOCH₃, NO₂, -(C₁-C₃)alkoxy, methylthio, -(C₁-C₃)alkyl, -NH₂, -OH, -N[(C₁-C₃)alkyl]₂, -CF₃ and methylsulfonyl;
Xb, when present, is independently selected from alkyl, -NH₂ and halo;
Xc is selected from -(5-6 membered)heteroaryl (imidazole), -(5-6 membered)heterocycloalkyl (piperazinyl), alkylcarbonylamino, alkylaminocarbonyl, cycloalkylaminoalkyl (cyclohexylaminoalkyl), -NH(C₁-C₃)alkyl-(3-6 membered)heterocycloalkyl, dimethylamino-(C₁-C₃)alkylcarbonylamino, cycloalkylaminocarbonyl, dialkylaminoalkylcarbonylamino, cycloalkylmethylaminocarbonylmethyloxy, phenylalkylaminocarbonyl, heterocycloalkylaminoalkyl optionally substituted with alkoxycarbonyl, dialkylaminoalkyl, morpholinylalkoxy, alkylaminoalkyl, dialkylaminoalkylamino, alkoxycarbonylheterocycloalkylalkylamino, heterocycloalkylalkylamino optionally substituted with methyl, heterocycloalkylalkylcarbonylamino optionally substituted with -OH, , heterocycloalkylcarbonylamino optionally substituted with 1 or 2 groups selected from halo and methyl, heteroarylcarbonylamino optionally substituted with 1 or 2 groups selected from amino, alkyl, halo, -C(O)OH, pyrazolyl, -OCH₃ and - C(O)OCH₃,
-N(H)C(O)phenyl optionally substituted with 1 or 2 groups selected from halo, methyl, methoxy and -NHC(O)CH₃, -N(H)C(O)alkyl, -N(H)C(O)(C₁-C₃)alkylpyridinyl,
-N(H)C(O)(C₁-C₃)alkylpiperidinyl, -N(H)C(O)-1H-pyrrolo[2,3-b]pyridinyl, -N(H)C(O)cyclohexyl, N(H)C(O)cyclopentyl, -N(H)C(O)(C₁-C₃)alkylmorpholinyl, -N(H)C(O)(C₁-C₃)alkylpyridinyl, -N(H)C(O)(C₁-C₃)alkylimidazolyl, aminoalkyl, and -N(H)C(O)N(H)pyrimidinyl;
Xd is selected from alkyl, 1-3 halo, -COOH, phenyl optionally substituted with 1 or 2 groups selected from halo, methyl and methylphenyl, phenylmethyl, spiro-piperidine, trifluoromethylphenylmethyl, -(C₁-C₃)alkoxy, pyridinyl, dimethylaminoalkyl, dimethylamino, hydroxylalkyl, dimethylaminoalkylaminocarbonyl, alkylamino, aminoalkyl, dimethylaminocarbonylalkyl, diethylaminoalkylcarbonyl, -(C₁-C₃)alkyl-(5-6 membered)heterocycloalkyl, (5-6 membered)heterocycloalkyl optionally substituted with -(C₁-C₃)alkyl, -NH₂, -OH, heterocycloalkylalkylamino, alkoxyalkyl, -C(O)CH₃, -C(O)NH(C₁-C₃)alkylphenyl optionally substituted with 1-3 halo at any phenyl position, one -OH and one -C(O)NH(C₁-C₃)alkylphenyl optionally substituted with 1-3 halo at any phenyl position, -C(O)-heterocycloalkyl optionally substituted with
-OH or halo, alkoxycarbonyl, aminocarbonyl, one -OH and one methyl, one -OH and one -C(O)OH, one -OH and one -NHC(O)piperidinyl, one -OH and one alkyl;
Xe is selected from dialkylamino, amino, 1-3 -OH, alkoxy, 4-methylpiperazinylphenyl, dimethylaminophenyl, phenyl optionally substituted with 1-3 groups selected from halo and methoxy, heteroaryl, -(C₁-C₃)alkylC(O)NH₂, - C(O)NH₂,
-C(O)OH, -(C₁-C₃)alkylC(O)OH, heterocycloalkyl optionally substituted with 1-2 alkyl;
Xf is selected from cycloalkyl, spirocycloalkyl, phenyl, phenylalkyl optionally substituted with phenylmethyloxy or alkoxy and thienylalkyl;
Xg is selected from alkyl, alkylcarbonyl, heterocycloalkylcarbonyl, dialkylaminoalkylcarbonyl, 1-methylpiperidinyl, dialkylaminoalkyl, heteroarylcarbonyl, alkoxyalkylcarbonyl, phenylcarbonyl, phenylalkylcarbonyl, oxo, phenylalkyl, -(5-6 membered)heteroarylalkyl, piperidinyloxy, -OH, oxo, 1-2 halo and 1-2 methyl;
Rza is H or methyl; and
Rzb is H or alkyl optionally substituted with 1-3 -OH.
In an embodiment of the present invention, R₂ is selected -NH-phenyl, -NH-piperidinyl, -NH-pyridinyl, -NH(C₁-C₃)alkylphenyl optionally substituted at any phenyl position with piperazinyl or methylpiperazinyl, -NH(C₁-C₃)alkyl-N(CH₃)₂, -NH(C₁-C₃)alkyl-OH, - (C₁-C₃)alkyl-O-phenyl, -(C₁-C₃)alkyl-O-(C₁-C₃)alkyl-(5-6 membered)heterocycloalkyl, -(C₁-C₃)alkyl-N(H)-heteroaryl, -(C₁-C₃)alkyl-(5-10)membered heteroaryl optionally substituted with -(C₁-C₃)alkyl, halo or phenyl, oxopyrrolidinyl optionally substituted with OH or piperidinyl, -(C₁-C₄)alkyl-(3-9 membered)heterocycloalkyl optionally substituted at the (3-9 membered)heterocycloalkyl with Xd, -(C₁-C₆)alkyl-NRzb-(C₁-C₄)alkyl wherein the - (C₁-C₄)alkyl portion is substituted with Xe, -(C₁-C₃)alkyl-NH-(C₃-C₆)cycloalkyl, - (CH₂)-NH-(C₃-C₆)cyclohexyl, -(C₁-C₃)alkyl-NH₂, wherein the -(C₁-C₃)alkyl- portion of -(C₁-C₃)alkyl-NH₂ is substituted with Xf, and -(3-9 membered)heterocycloalkyl optionally substituted with Xg;
Xd is selected from (C₁-C₁₂) alkyl, 1-3 halo, -COOH, phenyl optionally substituted with 1 or 2 groups selected from halo, methyl and methylphenyl, phenylmethyl, spiro-piperidine, trifluoromethylphenylmethyl, -(C₁-C₃)alkoxy, pyridinyl, dimethylamino (C₁-C₁₂) alkyl, dimethylamino, hydroxy (C₁-C₁₂) alkyl, dimethylamino (C₁-C₁₂) alkylaminocarbonyl, (C₁-C₁₂) alkylamino, amino (C₁-C₁₂) alkyl dimethylaminocarbonyl (C₁-C₁₂) alkyl, diethylamino (C₁-C₁₂) alkylcarbonyl, -(C₁-C₃)alkyl-(5-6 membered)heterocycloalkyl, (5-6 membered)heterocycloalkyl optionally substituted with -(C₁-C₃)alkyl, -NH₂, -OH, (4-12 membered) heterocycloalkyl (C₁-C₁₂) alkylamino, (C₁-C₁₂) alkoxy (C₁-C₁₂) alkyl, -C(O)CH₃, -C(O)NH(C₁-C₃)alkylphenyl optionally substituted with 1-3 halo at any phenyl position, one -OH and one -C(O)NH(C₁-C₃)alkylphenyl optionally substituted with 1-3 halo at any phenyl position, -C(O)-(4-12 membered) heterocycloalkyl optionally substituted with
-OH or halo, (C₁-C₁₂) alkoxycarbonyl, aminocarbonyl, one -OH and one methyl, one -OH and one -C(O)OH, one -OH and one -NHC(O)piperidinyl, one -OH and one (C₁-C₁₂) alkyl;
Xe is selected from dialkylamino, amino, 1-3 -OH, (C₁-C₁₂) alkoxy, 4-methylpiperazinylphenyl, dimethylaminophenyl, phenyl optionally substituted with 1-3 groups selected from halo and methoxy, (5-12 membered) heteroaryl, -(C₁-C₃)alkylC(O)NH₂,-C(O)NH₂,
-C(O)OH, -(C₁-C₃)alkylC(O)OH, (4-12 membered) heterocycloakyl optionally substituted with 1-2 (C₁-C₁₂) alkyl;
Xf is selected from (C₂-C₁₄) cycloalkyl, spiro (C₂-C₁₄) cycloalkyl, phenyl, phenyl (C₁-C₁₂) alkyl optionally substituted with phenylmethyloxy or (C₁-C₁₂) alkoxy and thienyl (C₁-C₁₂) alkyl;
Xg is selected from (C₁-C₁₂) alkyl, (C₁-C₁₂) alkylcarbonyl, (4-12 membered) heterocycloalkylcarbonyl, dialkylamino (C₁-C₁₂) alkylcarbonyl, 1-methylpiperidinyl, dialkylamino (C₁-C₁₂) alkyl, (5-12 membered) heteroarylcarbonyl, (C₁-C₁₂) alkoxy (C₁-C₁₂) alkylcarbonyl, phenylcarbonyl, phenyl (C₁-C₁₂) alkylcarbonyl, oxo, phenyl (C₁-C₁₂) alkyl, -(5-6 membered)heteroaryl (C₁-C₁₂) alkyl, piperidinyloxy, -OH, oxo, 1-2 halo and 1-2 methyl;
Rzb is H or alkyl optionally substituted with 1-3 -OH;
R^{3a} is selected from halo, (C₁-C₁₂)alkyl, -NO₂, (C₁-C₁₂)alkoxy, (C₁-C₁₂)alkynyl optionally substituted with R¹⁴, (C₁-C₁₂)alkoxycarbonyl(C₁-C₁₂)alkyl, (C₆-C₁₄)aryl(C₁-C₁₂)alkoxy, -C(O)N(H)(C₁-C₁₂)alkyl, -N(H)-C(O)-(C₁-C₁₂)alkyl, - C(O)-(C₁-C₁₂)alkyl, -CN, phenyl, -OCF₃, -N(H)R¹³, -OH, -CF₃, -S-CH₃ and hydroxymethyl(C₁-C₁₂)alkynyl;
R^{3b}, R^{3c} and R^{3d} are each independently selected from H, -OH, -N⁺(O)OH, (C₁-C₁₂)alkoxyl, and halo;
or R^{3a} is hydrogen and R^{3b}, R^{3c} and R^{3d} are each independently selected from - CF₃, -OH, (C₁-C₁₂)alkoxy, and halo;
or R^{3a} and R^{3d}, together with the carbons to which they are attached, join to form a 5 membered heteroaryl optionally substituted with methyl or -NH₂, or a 5-6 membered heterocycloalkyl;
R¹³ is selected from (C₆-C₁₄)aryl(C₁-C₁₂)alkyl wherein the aryl portion of arylalkyl is optionally substituted with 1, 2 or 3 (C₁-C₁₂)alkoxy, halo, methyl, methoxy, -CF₃, (C₃-C₁₄)cycloalkyl, and (5-12 membered)heteroaryl(C₁-C₁₂)alkyl; and
R¹⁴ is selected from hydroxyl(C₁-C₁₂)alkyl, H and TMS.

Optionally R₂ can be any one of the following (22).

(1), R₂ is -(C₁-C₃)alkyl-phenyl optionally substituted with 1-3 halo.

(2), R₂ is selected from -NH-phenyl, -NH-piperidinyl, -NH-pyridinyl, -NH(C₁-C₃)alkylphenyl optionally substituted at any phenyl position with
piperazinyl or methylpiperazinyl, -NH(C₁-C₃)alkyl-N(CH₃)₂ and -NH(C₁-C₃)alkyl-OH.

(3), R₂ is phenyl substituted with 1-3 Xa groups, wherein Xa is selected from halo, -COOH, -COOCH₃, NO₂, -(C₁-C₃)alkoxy, methylthio, -(C₁-C₃)alkyl, -NH₂, -OH, -N[(C₁-C₃)akyl]₂, -CF₃ and methylsulfonyl.

(4), R₂ is phenyl substituted with 0-2 Xb and 1 Xc group, wherein Xb, when present, is selected from alkyl, -NH₂ and halo, and Xc is selected from -(5-6 membered)heteroaryl (such as, for example, imidazole), -(5-6 membered)heterocycloalkyl (such as, for example, piperazinyl), alkylcarbonylamino, alkylaminocarbonyl, cycloalkylaminoalkyl (such as, for example, cyclohexylaminoalkyl), -NH(C₁-C₃)alkyl-(3-6 membered)heterocycloalkyl, dimethylamino-(C₁-C₃)alkylcarbonylamino, cycloalkylaminocarbonyl, dialkylaminoalkylcarbonylamino, cycloalkylmethylaminocarbonylmethyloxy, phenylalkylaminocarbonyl, heterocycloalkylaminoalkyl optionally substituted with alkoxycarbonyl (such as, for example, piperidinylaminomethyl or methylpiperdinylaminomethyl), heterocycloalkylalkylaminoalkyl (such as, for example, piperidinylethylaminomethyl), dialkylaminoalkyl, morpholinylalkoxy, alkylaminoalkyl, dialkylaminoalkylamino (such as, for example, dimethylamino(C₁-C₅)alkylamino such as 3-(dimethylamino)-2,2-dimethylpropyl]amino), alkoxycarbonylheterocycloalkylalkylamino, heterocycloalkylalkylamino optionally substituted with methyl (such as, for example, piperidinylmethylamino, piperidinylethylamino, tetrahydrofuranmethylamino, methylpiperidinylmethylamino or imidazolemethylamino), heterocycloalkylcarbonylamino (pyrrolidinylcarbonylamino), heterocycloalkylalkylcarbonylamino optionally substituted with -OH (such as, for example, 2-piperidin-4-ylacetamide or 3-morpholinylpropanamide),
heterocycloalkylcarbonylamino optionally substituted with 1-2 groups selected from halo and methyl (such as, for example, -N(H)C(O)piperidinyl optionally substituted with 1 or 2 groups selected from halo and methyl, -N(H)C(O)pyrrolidinyl, or -N(H)C(O)tetrahydrofuranyl,), heteroarylcarbonylamino optionally substituted with 1 or 2 groups selected from amino, alkyl, halo, -C(O)OH, pyrazolyl, -OCH₃ and -C(O)OCH₃ (such as, for example, -N(H)C(O)pyridinyl optionally substituted with 1 or 2 groups selected from -NH₂, methyl, halo, -C(O)OH, pyrazolyl, -OCH₃ and
-C(O)OCH₃, -N(H)C(O)imidazolyl optionally substituted with methyl, -N(H)C(O)quinoxalinyl, -N(H)C(O)pyrimidinyl optionally substituted with alkyl, -N(H)C(O)furanyl, -N(H)C(O)pyrazinyl, -N(H)C(O)isoxazolyl optionally substituted with methyl, -N(H)C(O)oxazolyl, or -N(H)C(O)indazolyl optionally substituted with amino), -N(H)C(O)phenyl optionally substituted with 1 or 2 groups selected from halo, methyl, methoxy and -NHC(O)CH₃, -N(H)C(O)alkyl, -N(H)C(O)(C₁-C₃)alkylpyridinyl,
-N(H)C(O)(C₁-C₃)alkylpiperidinyl, -N(H)C(O)-1H-pyrrolo[2,3-b]pyridinyl, -N(H)C(O)cyclohexyl, N(H)C(O)cyclopentyl, -N(H)C(O)(C₁-C₃)alkylmorpholinyl, -N(H)C(O)(C₁-C₃)alkylpyridinyl, -N(H)C(O)(C₁-C₃)alkylimidazolyl, aminoalkyl (such as, for example, aminomethyl,), and -N(H)C(O)N(H)pyrimidinyl.

(5), R₂ is methylpiperazinylphenylalkoxyalkyl (methylpiperazinylphenylmethoxymethyl), methylpiperazinylcarbonyl, 2-chlorophenyl-4-methylpiperazinylmethyl, (4-methylpiperazin-1-yl)(phenyl)methyl, 1-(4-methylpiperazin-1-yl)-2-phenylethyl, or 2-chlorophenyl(4-methylpiperazin-1-yl)methyl, 4-oxo-3-phenyl-1,3,8-triazaspiro[4.5]dec-1-yl)methyl.
(6), R₂ is -(C₁-C₃)alkylC(O)OH.
(7), R₂ is hydroxyalkyl.
(8), R₂ is -(C₁-C₃)alkyl-N(Rz)-aryl (wherein aryl can be, for example, phenyl or 2,3-dihydro-1H-indenyl) optionally substituted with chloro, fluoro, piperazinyl, methylpiperazinyl or dialkylamino(dimethylamino), and Rz is H or methyl.

(9), R₂ is -(5-10)membered heteroaryl (such as, for example, thienyl, pyridinyl, indazolyl, imidazolyl, thiazolyl, isoxazolyl, pyrazolyl, pyrimidinyl, benzimidazoly, pyrazolo[1,5-a]pyrimidinyl, tetrahydroisoquinolinyl, or 5,6,7,8-tetrahydroimidazo[1,2-a]pyrazinyl), optionally substituted with 1 or 2 groups selected from halo, -(C₁-C₆)alkyl, -(C₁-C₆)alkoxy, piperidinylalkylamino, piperdinylalkylamino, alkylamino, aminoalkyl, dialkylaminoalkylamino, piperidinyloxy, piperidinyl, and amino. Non-limiting examples include 5-chloro-2-thienyl, thienyl, 3-methyl-1H-indazolyl, 3-methyl-1H-indazolyl, 3-[(2-methylpropyl)oxy]pyridin-4-yl, 3-amino-1H-indazolyl, 3-amino-5-chloro-1H-indazolyl, 1H-benzimidazolyl, 1H-imidazolyl, 1,3-thiazolyl, 2-amino-5-chloropyrimidin-4-yl, 3-[(piperidin-4-ylmethyl)amino]-1H-indazolyl, and (2S)-2,3-dihydro-1H-indol-2-yl.

(10), R₂ is -(C₁-C₃)akyl-O-phenyl.

(11), R₂ is -(C₁-C₃)alkyl-O-(C₁-C₃)alkyl-(5-6 membered)heterocycloalkyl, wherein the heterocycloalkyl can be, for example, pyrrolidinyl.

(12), R₂ is -(C₁-C₃)alkyl-N(H)-heteroaryl wherein the heteroaryl can be, for example, pyridinyl.

(13), R₂ is -(C₁-C₃)alkyl-(5-10)membered heteroaryl (wherein the heteroaryl can be, for example, imidazolyl or pyrazolyl) optionally substituted with -(C₁-C₃)alkyl, halo and phenyl.

(14), R₂ is oxopyrrolidinyl optionally substituted with OH and/or piperidinyl.

(15), R₂ is -(C₁-C₄)alkyl-(3-9 membered)heterocycloalkyl optionally substituted at any position of the (3-9 membered)heterocycloalkyl with Xd, wherein Xd is selected from alkyl(such as, for example, methyl), 1-3 halo, -COOH, phenyl optionally substituted with 1 or 2 groups selected from halo, methyl and methylphenyl, phenylmethyl, spiro-piperidine, trifluoromethylphenylmethyl, -(C₁-C₃)alkoxy, pyridinyl, dimethylaminoalkyl (such as, for example, dimethylamino-(C₁-C₃)alkyl), dimethylamino, hydroxylalkyl, dimethylaminoalkylaminocarbonyl (such as, for example, dimethylamino(C₁-C₃)alkylaminocarbonyl), alkylamino, aminoalkyl (such as, for example, aminomethyl or aminoethyl), dimethylaminocarbonylalkyl (such as, for example, dimethylaminocarbonylmethyl), diethylaminoalkylcarbonyl (such as, for example, diethylaminomethylcarbonyl), -(C₁-C₃)alkyl-(5-6 membered)heterocycloalkyl, (5-6 membered)heterocycloalkyl (such as, for example, piperidinyl or morpholinyl) optionally substituted with -(C₁-C₃)alkyl, -NH₂, -OH, heterocycloalkylalkylamino, alkoxyalkyl (such as for example, methoxyethyl), -C(O)CH₃, -C(O)NH(C₁-C₃)alkylphenyl optionally substituted with 1-3 halo at any phenyl position, one -OH and one -C(O)NH(C₁-C₃)alkylphenyl optionally substituted with 1-3 halo at any phenyl position,-C(O)-heterocycloalkyl (such as, for example, morpholinyl or piperidinyl) optionally substituted with -OH or halo, alkoxycarbonyl, aminocarbonyl, one hydroxyl and one methyl, one -OH and one -C(O)OH, one -OH and one - NHC(O)piperidinyl, and one -OH and one alkyl. In this embodiment, the (3-9 membered)heterocycloalkyl can be, for example, piperazinyl, piperidinyl, pyrrolidinyl, azetidinyl, morpholinyl, 1,4-diazepanyl, 2,5-diazabicyclo[2.2.1]heptyl, azabicyclo[2.2.1]heptane such as anti-7-hydroxy-2-azabicyclo[2.2.1]heptane, 7-hydroxy-2-azabicyclo[2.2.1]heptanyl and (7S)-7-hydroxy-2-azabicyclo[2.2.1]heptanyl, 8-azabicylo[3.2.1]oct-8-yl such as 3-hydroxy-8-azabicylo[3.2.1]oct-8-yl, (1S,4S)-2,5-diazabicyclo[2.2.1]heptyl such as (1S,4S)-5-methyl-2,5-diazabicyclo[2.2.1]heptyl and (1S,4S)-5-ethyl-2,5-diazabicyclo[2.2.1]heptyl, 2,5-dihydro-1H-pyrrolyl, (1R,4R)-2,5-diazabicyclo[2.2.1]heptan-2-yl such as (1R,4R)-5-methyl-2,5-diazabicyclo[2.2.1]heptan-2-yl, (1R,5S)-8-azabicyclo[3.2.1]octyl such as (1R,5S)-3-amino-8-azabicyclo[3.2.1]octyl, (8aS)-hexahydropyrrolo[1,2-a]pyrazin-2(1H)-yl, dimethylpiperazinyl such as (2R,6S)-2,6-dimethylpiperazinyl or (3aR,6aS)-hexahydropyrrolo[3,4-c]pyrrolyl. Optionally, the -(C₁-C₄)alkyl-(3-9 membered)heterocycloalkyl described above is -CH₂-(5-7 membered)heterocycloalkyl, which can be optionally substituted with any of the optional substituents described above for -(C₁-C₄)alkyl-(3-9 membered)heterocycloalkyl. Optionally, R₂ is -(CH₂)-(5-6 membered)heterocycloalkyl optionally substituted with any of the optional substituents described above for -(C₁-C₄)alkyl-(3-9 membered)heterocycloalkyl. Optionally, Xd is bonded to the -(3-9 membered)heterocycloalkyl portion of the -(C₁-C₄)alkyl-(3-9 membered)heterocycloalkyl in the (S) stereochemical configuration. Optionally, Xd is bonded to the -(3-9 membered)heterocycloalkyl portion of the -(C₁-C₄)alkyl-(3-9 membered)heterocycloalkyl in the (R) stereochemical configuration.

(16), R₂ is -(C₁-C₆)alkyl-NRz-(C₁-C₄)alkyl wherein the -(C₁-C₄)alkyl portion is optionally substituted with Xe, and Xe is selected from dialkylamino (such as, for example, dimethylamino), amino, 1-3 -OH, alkoxy, 4-methylpiperazinylphenyl, dimethylaminophenyl, phenyl optionally substituted with 1-3 groups selected from halo and methoxy, heteroaryl (such as, for example, furanyl, pyridinyl or imidizolyl), -(C₁-C₃)alkylC(O)NH₂, -C(O)NH₂, -C(O)OH, -(C₁-C₃)alkylC(O)OH, heterocycloalkyl (such as, for example, morpholinyl, pyrrolidinyl piperidinyl or piperazinyl) optionally substituted with 1-2 alkyl (non-limiting examples include 1,1-dimethyl-2-pyrrolidinyl, 1,1-dimethyl-2-piperidinyl, and 4-methylpiperazinyl), wherein Rz is H or alkyl optionally substituted with 1-3 -OH. Optionally, the -(C₁-C₆)alkyl-NRz-(C₁-C₄)alkyl above is -(C₁-C₃)alkyl-NRz-(C₁-C₄)alkyl which can be optionally substituted with any of the optional substituents described above for -(C₁-C₆)alkyl-NRz-(C₁-C₄)alkyl.

(17), R₂ is -(C₁-C₃)alkyl-NH-(C₃-C₆)cycloalkyl such as, for example, -(CH₂)-NH-(C₃-C₆)cyclohexyl.

(18), R₂ is -(C₁-C₃)alkyl-NH₂ , wherein the -(C₁-C₃)alkyl- potion is optionally substituted with Xf, and Xf is selected from cycloalkyl (such as, for example, cyclohexyl), spirocycloalkyl, phenyl, phenylalkyl optionally substituted with phenylmethyloxy or alkoxy and thienylalkyl. Non-limiting examples include any one or more of the following groups: -(CH₂)₃-NH₂, - CH₂-NH₂, -(CH₂)₂-NH₂, -CH₂-CH(CH₃)-NH₂, or -C(CH₃)₂-NH₂.

(19), R₂ is (3-9 membered)heterocycloalkyl (such as, for example, piperazinyl, piperidinyl, pyrrolidinyl, isoxazolyl, azetidinyl, morpholinyl, tetrahydrofuranyl, thiazolidinyl or octahydro-1H-indolyl) optionally substituted with Xg, and Xg is selected from alkyl (such as, for example (C₁-C₃)alkyl), alkylcarbonyl (such as, for example, -C(O)CH₃), heterocycloalkylcarbonyl, dialkylaminoalkylcarbonyl (such as, for example, 4-(dimethylamino)butanoyl, 4-(dimethylamino)propanoyl or 2-(dimethylamino)ethanoyl) 1-methylpiperidinyl, dialkylaminoalkyl (such as, for example, -(dimethylamino)ethyl or 3-(dimethylamino)propyl), heteroarylcarbonyl (such as, for example, 1-(1H-benzimidazol-5-ylcarbonyl)piperidinyl or pyridinylcarbonyl), alkoxyalkylcarbonyl (such as, for example, 3-(methyloxy)propanoyl), phenylcarbonyl, phenylalkylcarbonyl, oxo, phenylalkyl, (5-6 membered)heteroarylalkyl (such as, for example, pyridinylmethyl), piperidinyloxy,-OH, oxo, 1-2 halo and 1-2 methyl. Optionally where R₂ is (3-9 membered)heterocycloalkyl, R₂ is bonded to the parent moiety in the (S) stereochemical configuration. Optionally where R₂ is (3-9 membered)heterocycloalkyl, R₂ is bonded to the parent moiety in the (R) stereochemical configuration. Optionally, R₂ is (5 membered)heterocycloalkyl optionally substituted with methyl. Optionally, R₂ is (5 membered)heterocycloalkyl optionally substituted with halo. Optionally, R₂ is (5 membered)heterocycloalkyl optionally substituted with-OH. Optionally, R₂ is (5 membered)heterocycloalkyl optionally substituted with phenylmethyl. Optionally, R₂ is (5 membered)heterocycloalkyl optionally substituted with -C(O)CH₃. Optionally, R₂ is (5 membered)heterocycloalkyl optionally substituted with dialkylaminoalkylcarbonyl. Optionally, R₂ is (5 membered)heterocycloalkyl optionally substituted with dialkylaminoalkyl heteroarylcarbonyl. Optionally, R₂ is (6 membered)heterocycloalkyl optionally substituted with methyl. Optionally, R₂ is (6 membered)heterocycloalkyl optionally substituted with halo. Optionally, R₂ is (6 membered)heterocycloalkyl optionally substituted with-OH. Optionally, R₂ is (6 membered)heterocycloalkyl optionally substituted with phenylmethyl. Optionally, R₂ is (6 membered)heterocycloalkyl optionally substituted with -C(O)CH₃. Optionally, R₂ is (6 membered)heterocycloalkyl optionally substituted with diakylaminoalkylcarbonyl. Optionally, R₂ is (6 membered)heterocycloalkyl optionally substituted with dialkylaminoalkyl heteroarylcarbonyl.

(20), R₂ is -(C₃-C₆)-cycloalkyl (cyclohexyl) optionally substituted with amino, -NHC(O)-O-(CH₃)₃ or aminoalkyl (such as, for example, aminomethyl).

(21), R₂ is dialkylaminoalkylamino, such as, for example 3-(dimethylamino)propylamino. (22), R₂ is 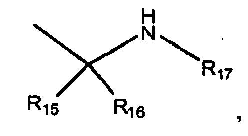 wherein R₁₅ is selected from H or -(C₁-C₆)alkyl, R₁₆ is selected from H, phenyl and -(C₁-C₆)alkyl, and R₁₇ is selected from H, -(C₁-C₃)alkylC(O)NH₂, -(C₁-C₃)alkylC(O)OH and heterocycloalkylalkyl (such as, for example, 1,1-dimethyl-2-pyrrolidin-1-ylethyl or 1,1-dimethyl-2-piperidin-1-ylethyl).

All compounds of formula I disclosed above include any of the disclosed alternatives for each of R₁, R₂, R₃ₐ, R_{3b}, R_{3c}, or R_{3d}, in combination with any other of the disclosed alternatives of R₁, R₂, R₃ₐ, R_{3b}, R_{3c}, or R_{3d}, as well as any pharmaceutically acceptable salt and stereoisomer of any such combination.

Within this disclosure, when a chemical moiety is said to have one or more optional substituents on any ring portion, this is meant to mean the same as when a chemical moiety can have one or more optional substituents on any ring position, which is meant to be the same as when as when a chemical moiety can have one or more optional substituents on a ring, wherein each of the one or more optional substituents replaces any hydrogen atom on any position of the ring, and if there is more than one substituent, then the remaining substituent(s) can replace any other of the remaining hydrogens on this ring.

In other options, any of the alkyl groups referred to above, including alkyl portions attached to other groups, can be a -(C₁-C₆)alkyl group.

In other options, any of the alkyl groups referred to above, including alkyl portions attached to other groups, can be a -(C₁-C₃)alkyl group.

In other options, any of the alkoxy groups referred to above, including alkoxy portions attached to other groups, can be a - (C₁-C₆)alkoxy group.

In other options, any of the alkoxy groups referred to above, including alkoxy portions attached to other groups, can be a - (C₁-C₃)alkoxy group.

In other options, any of the heterocycloalkyl groups referred to above, including heterocycloalkyl portions attached to other groups, can be a (4-6 membered) heterocycloalkyl group.

In other options, any of the cycloalkyl groups referred to above, can be a -(C₃-C₆)cycloalkyl group.

All of the compounds disclosed herein include either their free base form or their pharmaceutically acceptable salts whether it is stated in the specification that these compounds can exist as their pharmaceutically acceptable salt or not. So, for instance, for any given embodiment of the compound of Formula I (including embodiments relating to the compounds themselves or use thereof), this embodiment includes either its free base form or any of its pharmaceutically acceptable salts, whether this is stated within this embodiment or not.

Table 1 illustrates some examples of the compounds of this disclosure that are encompassed within formula I, and their pharmaceutically acceptable salts. Compounds marked with an asterisk (*) are not specifically recited in the appended claims, but are useful for general information.

**TABLE 1**

| **Cpd No.** | **Structure** | **NAME / ACTIVITY** |
|---|---|---|
| **1** | 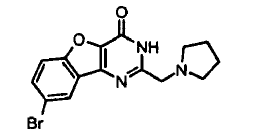 | 8-bromo-2-(pyrrolidin-1-ylmethyl)[1]benzofuro[3,2 -d]pyrimidin-4(3H)-one **Activity = A, B and C** |
| **2** | 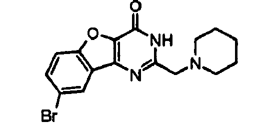 | 8-bromo-2-(piperidin-1-ylmethyl)[1]benzofuro[3,2 -d]pyrimidin-4(3H)-one **Activity** = **A, B and C** |
| **3** | 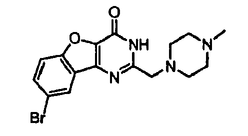 | 8-bromo-2-[(4-methylpiperazin-1-yl)methyl][1]benzofuro[3, 2-d]pyrimidin-4(3H)-one **Activity** = **A, B and C** |
| **4** | 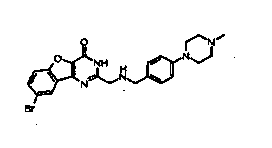 | 8-bromo-2-[({[4-(4-methylpiperazin-1-yl)phenyl]methyl} amino) methyl][1]benzofuro[3,2-d]pyrimidin-4(3H)-one **Activity** = **B and C** |
| **5** | 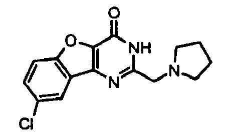 | 8-chloro-2-(pyrrolidin-1-ylmethyl)[1]benzofuro[3,2 -d]pyrimidin-4(3H)-one **Activity** = **A, B and C** |
| **6** | 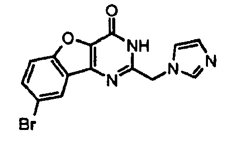 | 8-bromo-2-(1H-imidazol-1-ylmethyl)[1]benzofuro[3,2 -d]pyrimidin-4(3H)-one **Activity** = **B and C** |
| **7** | 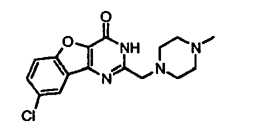 | 8-chloro-2-[(4-methylpiperazin-1-yl)methyl][1]benzofuro[3, 2-d]pyrimidin-4(3H)-one **Activity = B** |
| **8*** | 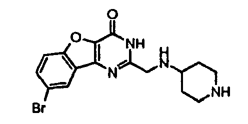 | 8-bromo-2-[(piperidin-4-ylamino)methyl][1]benzof uro[3,2-d]pyrimidin-4(3H)-one **Activity = B** |
| **9** | 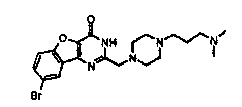 | 8-bromo-2-({4-[3-(dimethylamino)propyl]pi perazin-1-yl}methyl)[1]benzofuro[3, 2-d]pyrimidin-4(3H)-one **Activity** = **A and B** |
| **10** | 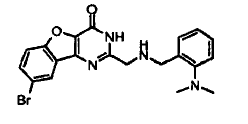 | 8-bromo-2-[({[2-(dimethylamino)phenyl]m ethyl} amino)methyl] [1]be nzofuro[3,2-d]pyrimidin-4(3H)-one **Activity** = **B and C** |
| **11*** | 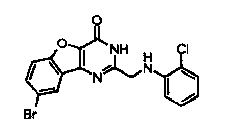 | 8-bromo-2-{[(2-chlorophenyl)amino]meth yl}[1]benzofuro[3,2-d]pyrimidin-4(3H)-one **Activity** = **B and C** |
| **12*** | 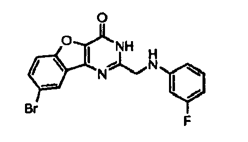 | 8-bromo-2-{[(3-fluorophenyl)amino]meth yl}[1]benzofuro[3,2-d]pyrimidin-4(3H)-one **Activity** = **C** |
| **13** | 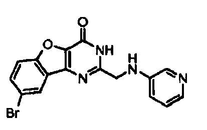 | 8-bromo-2-[(pyridin-3-yl-amino)methyl][1]benzof uro[3,2-d]pyrimidin-4(3H)-one **Activity** = **A, B and C** |
| **14** | 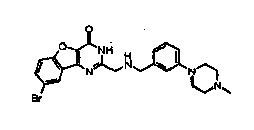 | 8-bromo-2-[({[3-(4-methylpiperazin-1-yl)phenyl]methyl} amino) methyl][1]benzofuro[3,2-d]pyrimidin-4(3H)-one **Activity** = **B and C** |
| **15** | 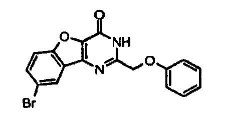 | 8-bromo-2-[(phenyloxy)methyl] [1]be nzofuro[3,2-d]pyrimidin-4(3H)-one **Activity B and C** |
| **16*** | 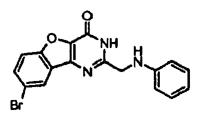 | 8-bromo-2-[(phenylamino)methyl][1] benzofuro[3,2-d]pyrimidin-4(3H)-one **Activity** = **B and C** |
| **17** | 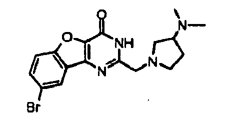 | 8-bromo-2-{[3-(dimethylamino)pyrrolidin -1-1]methyl}[1]benzofuro[3,2 -d]pyrimidin-4(3H)-one **Activity** = **B and C** |
| **18*** | 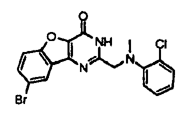 | 8-bromo-2-{[(2-chlorophenyl)(methyl)ami no]methyl}[1]benzofuro[3 ,2-d]pyrimidin-4(3H)-one **Activity = B and C** |
| **19*** | 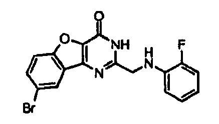 | 8-bromo-2-{[(2-fluorophenyl)amino]meth yl}[1]benzofuro[3,2-d]pyrimidin-4(3H)-one **Activity** = **B and C** |
| **20** | 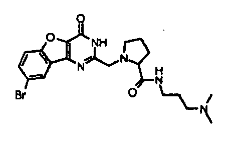 | 1-[(8-bromo-4-oxo-3,4-dihydro[1]benzofuro[3,2-d]pyrimidin-2-yl)methyl]-N-[3-(dimethylamino)propyl]pr olinamide **Activity = B** |
| **21** | 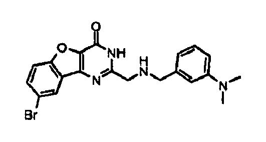 | 8-bromo-2-[({[3-(dimethylamino)phenyl]m ethyl}amino)methyl][1]be nzofuro[3,2-d]pyrimidin-4(3H)-one **Activity = B and C** |
| **22** | 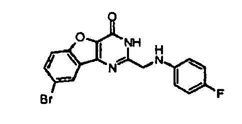 | 8-bromo-2-{[(4-fluorophenyl)amino]meth yl}[1]benzofuro[3,2-d]pyrimidin-4(3H)-one **Activity = B** |
| **23** | 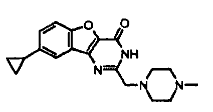 | 8-cyclopropyl-2-[(4-methylpiperazin-1-yl)methyl][1]benzofuro[3, 2-d]pyrimidin-4(3H)-one **Activity = B** |
| **24** | 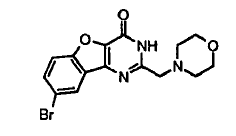 | 8-bromo-2-(morpholin-4-ylmethyl)[1]benzofuro[3,2 -d]pyrimidin-4(3H)-one **Activity** = **B and C** |
| **25** | 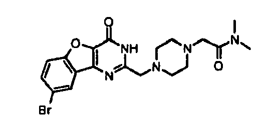 | 2-{4-[(8-bromo-4-oxo-3,4-dihydro[1]benzofuro[3,2-d]pyrimidin-2-yl)methyl]piperazin-1-yl}-N,N-dimethylacetamide **Activity = A, B and C** |
| **26** | 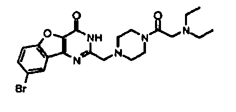 | 8-bromo-2-{[4-(N,N-diethylglycyl)piperazin-1-yl]methyl}[1]benzofuro[3, 2-d]pyrimidin-4(3H)-one **Activity = B** |
| **28** | 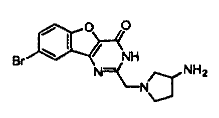 | 2-[(3-aminopyrrolidin-1-yl)methyl]-8-bromo[]benzofuro[3,2-d]pyrimidin-4(3H)-one **Activity** = **A, B and C** |
| **29** | 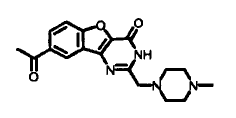 | 8-Acetyl-2-[(4-methylpiperazin-1-yl)methyl][1]benzofuro[3, 2-d]pyrimidin-4(3H)-one **Activity = B** |
| **30*** | 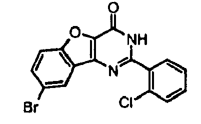 | 8-bromo-2-(2-chlorophenyl)[1]benzofur o[3,2-d]pyrimidin-4(3H)-one **Activity** = **A, B and C** |
| **31*** | 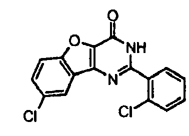 | 8-chloro-2-(2-chlorophenyl)[1]benzofur o[3,2-d]pyrimidin-4(3H)-one **Activity** = **B and C** |
| **32*** | 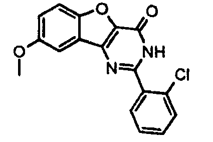 | 2-(2-chlorophenyl)-8-(methyloxy)[1]benzofuro[ 3,2-d]pyrimidin-4(3H)-one **Activity** = **A, B and C** |
| **33*** | 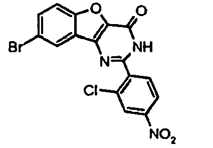 | 8-bromo-2-(2-chloro-4-nitrophenyl)[1]benzofuro[ 3,2-d]pyrimidin-4(3H)-one **Activity = A, B and C** |
| **34*** | 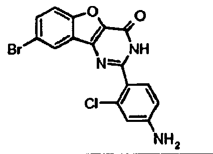 | 2-(4-amino-2-chlorophenyl)-8-bromo[1]benzofuro[3,2-d]pyrimidin-4(3H)-one **Activity** = **A, B and C** |
| **35*** | 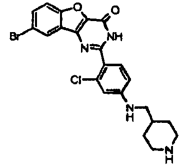 | 8-bromo-2-{2-chloro-4-[(piperidine-4-ylmethyl)amino]phenyl}[1 ]benzofuro[3,2-d]pyrimidin-4(3H)-one **Activity** = **A, B and C** |
| **36*** | 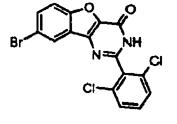 | 8-bromo-2-(2,6-dichlorophenyl)[1]benzofu ro[3,2-d]pyrimidin-4(3H)-one **Activity** = **B and C** |
| **37*** | 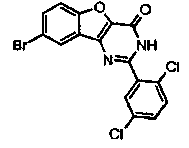 | 8-bromo-2-(2,5-dichlorophenyl)[1]benzofu ro[3,2-d]pyrimidin-4(3H)-one **Activity** = **B and C** |
| **38*** | 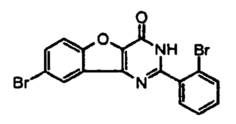 | 8-bromo-2-(2-bromophenyl)[1]benzofur o[3,2-d]pyrimidin-4(3H)-one **Activity** = **B and C** |
| **39*** | 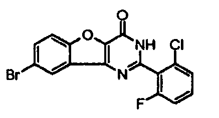 | 8-bromo-2-(2-chloro-6-fluorophenyl)[1]benzofuro [3,2-d]pyrimidin-4(3H)-one **Activity** = **B and C** |
| **40*** | 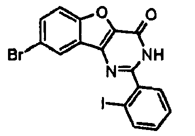 | 8-bromo-2-(2-iodophenyl)[1]benzofuro[ 3,2-d]pyrimidin-4(3H)-one **Activity** = **A, B and C** |
| **41*** | 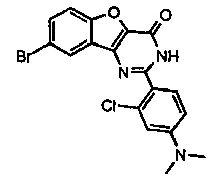 | 8-bromo-2-[2-chloro-4-(dimethylamino)phenyl][1 ]benzofuro[3,2-d]pyrimidin-4(3H)-one **Activity** = **B and C** |
| **42*** | 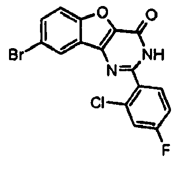 | 8-bromo-2-(2-chloro-4-fluorophenyl)[1]benzofuro [3,2-d]pyrimidin-4(3H)-one **Activity** = **B and C** |
| **43*** | 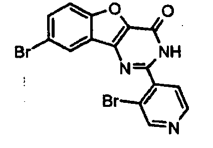 | 8-bromo-2-(3-bromopyridin-4-yl)[1]benzofuro[3,2-d]pyrimidin-4(3H)-one **Activity** = **A, B and C** |
| **44*** | 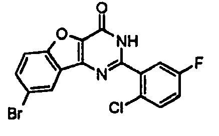 | 8-bromo-2-(2-chloro-5-fluorophenyl)[1]benzofuro [3,2-d]pyrimidin-4(3H)-one **Activity** = **A, B and C** |
| **45** | 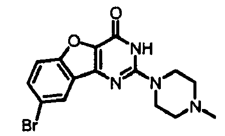 | 8-bromo-2-(4-methylpiperazin-1-yl)[1]benzofuro[3,2-d]pyrimidin-4(3H)-one **Activity** = **A, B and C** |
| **46** | 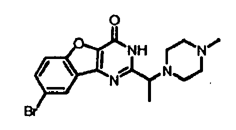 | 8-bromo-2-[1-(4-methylpiperazin-1-yl)ethyl][1]benzofuro[3,2-d]pyrimidin-4(3H)-one **Activity** = **A, B and C** |
| **47*** | 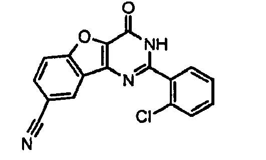 | 2-(2-chlorophenyl)-4-oxo-3,4-dihydro[1]benzofuro[3,2-d]pyrimidine-8-carbonitrile **Activity = B** |
| **48*** | 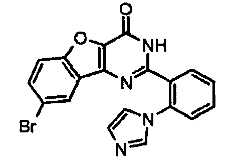 | 8-bromo-2-[2-(1H-imidazol-1-yl)phenyl][1]benzofuro[3, 2-d]pyrimidin-4(3H)-one **Activity** = **A, B and C** |
| **49** | 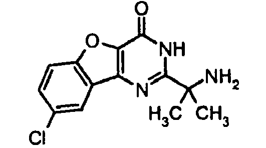 | 2-(1-amino-1-methylethyl)-8-chloro[1]benzofuro[3,2-d]pyrimidin-4(3H)-one **Activity** = **A, B and C** |
| **50*** | 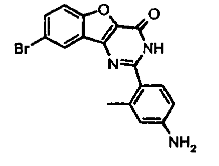 | 2-(4-amino-2-methylphenyl)-8-bromo[1]benzofuro[3,2-d]pyrimidin-4(3H)-one **Activity** = **B and C** |
| **51*** | 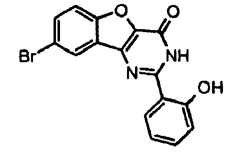 | 8-bromo-2-(2-hydroxyphenyl)[1]benzofu ro[3,2-d]pyrimidin-4(3H)-one **Activity** = **B and C** |
| **52*** | 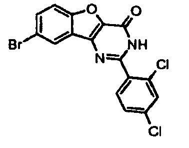 | 8-bromo-2-(2,4-dichlorophenyl)[1]benzofu ro[3,2-d]pyrimidin-4(3H)-one **Activity** = **A, B and C** |
| **53*** | 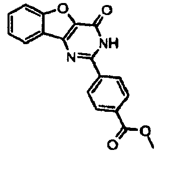 | methyl 4-(4-oxo-3,4-dihydro[1]benzofuro[3,2-d]pyrimidin-2-yl)benzoate **Activity = B** |
| **54*** | 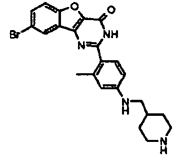 | 8-bromo-2-{2-methyl-4-[(piperidin-4-ylmethyl)amino]phenyl}[1 ]benzofuro[3,2-d]pyrimidin-4(3H)-one **Activity** = **B and C** |
| **55*** | 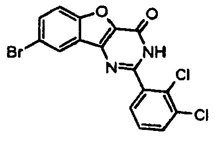 | 8-bromo-2-(2,3-dichlorophenyl)[1]benzofu ro[3,2-d]pyrimidin-4(3H)-one **Activity = A, B and C** |
| **56*** | 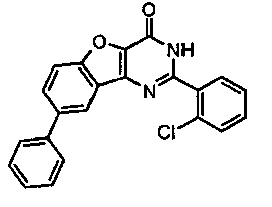 | 2-(2-chlorophenyl)-8-phenyl[1]benzofuro[3,2-d]pyrimidin-4(3H)-one **Activity = B** |
| **57*** | 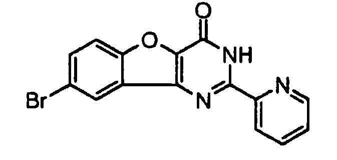 | 8-bromo-2-pyridin-2-yl[1]benzofuro[3,2-d]pyrimidin-4(3H)-one **Activity = B and C** |
| **58*** | 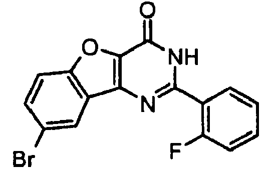 | 8-bromo-2-(2-fluorophenyl)[1]benzofuro [3,2-d]pyrimidin-4(3H)-one **Activity** = **B and C** |
| **59*** | 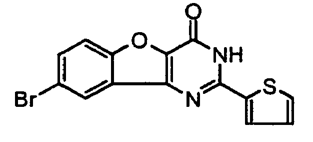 | 8-bromo-2-(2-thienyl)[1]benzofuro[3,2-d]pyrimidin-4(3H)-one **Activity** = **B and C** |
| **60*** | 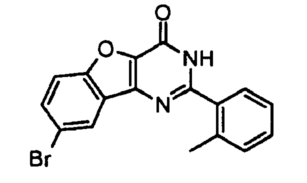 | 8-bromo-2-(2-methylphenyl)[1]benzofur o[3,2-d]pyrimidin-4(3H)-one **Activity** = **A, B and C** |
| **61*** | 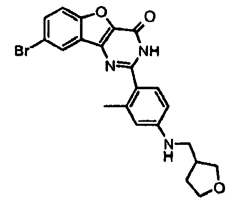 | 8-bromo-2-{2-methyl-4-[(tetrahydrofuran-3-ylmethyl)amino]phenyl}[1 ]benzofuro[3,2-d]pyrimidin-4(3H)-one **Activity = B** |
| **62*** | 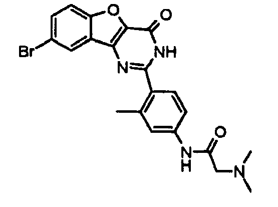 | N-[4-(8-bromo-4-oxo-3,4-dihydro[1]benzofuro[3,2-d]pyrimidin-2-yl)-3-methylphenyl]-N², N²-dimethylglycinamide **Activity** = **B and C** |
| **63*** | 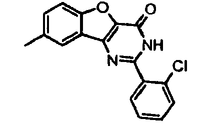 | 2-(2-chlorophenyl)-8-methyl[1]benzofuro[3,2-d]pyrimidin-4(3H)-one **Activity** = **B and C** |
| **64*** | 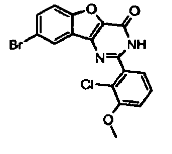 | 8-bromo-2-[2-chloro-3-(methyloxy)phenyl][1]ben zofuo[3,2-d]pyrimidin-4(3H)-one **Activity = A, B and C** |
| **65*** | 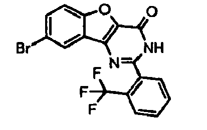 | 8-bromo-2-[2-(trifluoromethyl)phenyl][1 ]benzofuro[3,2-d]pyrimidin-4(3H)-one **Activity** = **B and C** |
| **66*** | 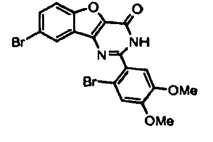 | 8-bromo-2-[2-bromo-4,5-bis(methyloxy)phenyl][1] benzofuro[3,2-d]pyrimidin-4-(3H)-one **Activity** = **B and C** |
| **67*** | 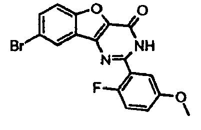 | 8-bromo-2-[2-fluoro-5-(methyloxy)phenyl][1]ben zofuro[3,2-d]pyrimidin-4(3H)-one **Activity = B and C** |
| **68*** | 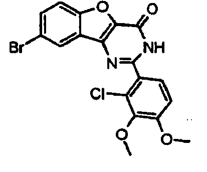 | 8-bromo-2-[2-chloro-3,4-bis(methyloxy)phenyl][1] benzofuro[3,2-d]pyrimidin-4(3H)-one **Activity** = **B and C** |
| **69*** | 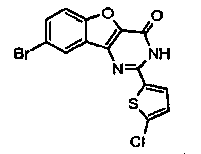 | 8-bromo-2-(5-chloro-2-thienyl)[1]benzofuro[3,2-d]pyrimidin-4(3H)-one **Activity** = **B and C** |
| **70*** | 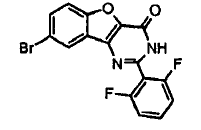 | 8-bromo-2-(2,6-difluorophenyl)[1]benzofu ro[3,2-d]pyrimidin-4(3H)-one **Activity** = **B and C** |
| **71*** | 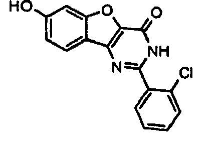 | 2-(2-chlorophenyl)-7-hydroxy[1]benzofuro[3,2-d]pyrimidin-4(3H)-one **Activity = B and C** |
| **72*** | 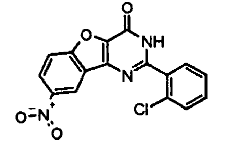 | 2-(2-chlorophenyl)-8-nitro[1]benzofuro[3,2-d]pyrimidin-4(3H)-one **Activity** = **B** |
| **73*** | 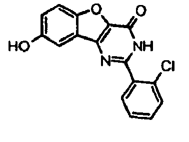 | 2-(2-chlorophenyl)-8-hydroxy[1]benzofuro[3,2-d]pyrimidin-4(3H)-one **Activity** = **B and C** |
| **74** | 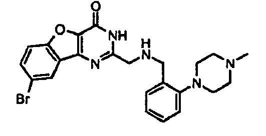 | 8-bromo-2-[({[2-(4-methylpiperazin-1-yl)phenyl]methyl}amino) methyl][1]benzofuro[3,2-d]pyrimidin-4(3H)-one **Activity = B** |
| **75*** | 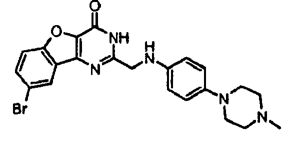 | 8-bromo-2-({[4-(4-methylpiperazin-1-yl)phenyl]amino}methyl)[ 1]benzofuro[3,2-d]pyrimidin-4(3H)-one **Activity = B and C** |
| ***76** | 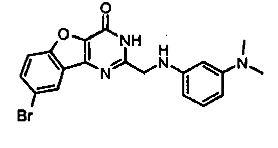 | 8-bromo-2-({[3-(dimethylamino)phenyl]a mino}methyl)[1]benzofur o[3,2-d]pyrimidin-4(3H)-one **Activity = B** |
| ***77** | 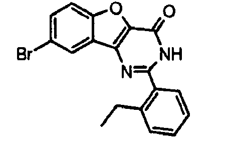 | 8-bromo-2-(2-ethylphenyl)[1]benzofuro[ 3,2-d]pyrimidin-4(3H)-one **Activity = B and C** |
| ***78** | 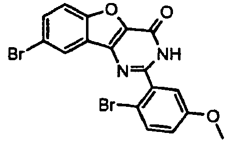 | 8-bromo-2-[2-bromo-5-(methyloxy)phenyl][1]ben zofuro[3,2-d]pyrimidin-4(3H)-one **Activity** = **B and C** |
| ***79** | 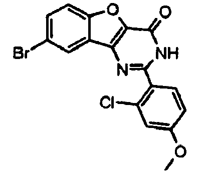 | 8-bromo-2-[2-chloro-4-(methyloxy)phenyl][1]ben zofuro[3,2-d]pyrimidin-4(3H)-one **Activity** = **B and C** |
| ***80** | 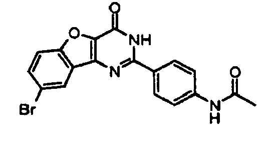 | N-[4-(8-bromo-4-oxo-3,4-dihydro[1]benzofuro[3,2-d]pyrimidin-2-yl)phenyl]acetamide **Activity** = **B and C** |
| **81** | 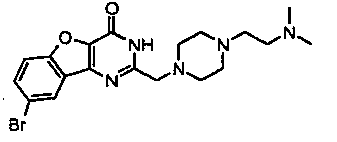 | 8-bromo-2-({4-[2-(dimethylamino)ethyl]pipe razin-1-yl}methyl)[1]benzofuro[3, 2-d]pyrimidin-4(3H)-one **Activity** = **B** |
| **82** | 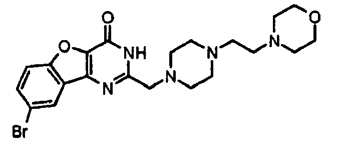 | 8-bromo-2-{[4-(2-morpholin-4-ylethyl)piperazin-1-yl]methyl}[1]benzofuro[3, 2-d]pyrimidin-4(3H)-one **Activity** = **B** |
| ***83** | 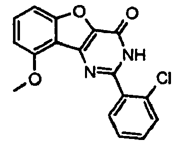 | 2-(2-chlorophenyl)-9-(methyloxy)[1]benzofuro[ 3,2-d]pyrimidin-4(3H)-one **Activity = B and C** |
| **84** | 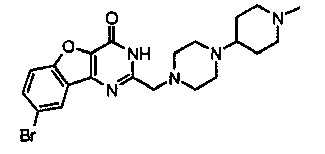 | 8-bromo-2-{[4-(1-methylpiperidin-4-yl)piperazin-1-yl]methyl}[1]benzofuro[3, 2-d]pyrimidin-4(3H)-one **Activity = B** |
| ***85** | 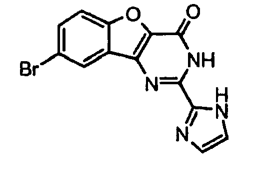 | 8-bromo-2-(1H-imidazol-2-yl)[1]benzofuro[3,2-d]pyrimidin-4(3H)-one **Activity = B and C** |
| ***86** | 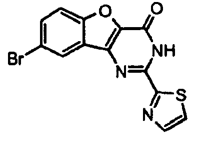 | 8-bromo-2-(1,3-thiazol-2-yl)[1]benzofuro[3,2-d]pyrimidin-4(3H)-one **Activity = A, B and C** |
| ***87** | 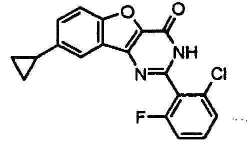 | 2-(2-chloro-6-fluorophenyl)-8-cyclopropyl[1]benzofuro[ 3,2-d]pyrimidin-4(3H)-one **Activity = B and C** |
| ***88** | 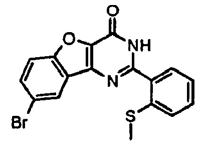 | 8-bromo-2-[2- (methylthio)phenyl][1]ben zofuro[3,2-d]pyrimidin4(3H)-one **Activity = B and C** |
| ***89** | 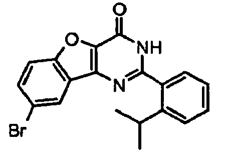 | 8-bromo-2-[2-(1-methylethyl)phenyl][1]ben zofuro[3,2-d]pyrimidin-4(3H)-one **Activity** = **B and C** |
| ***90** | 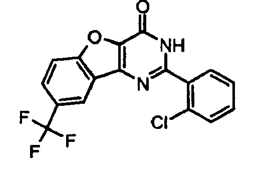 | 2-(2-chlorophenyl)-8-(trifluoromethyl)[1]benzof uro[3,2-d]pyrimidin-4(3H)-one **Activity** = **B** |
| ***91** | 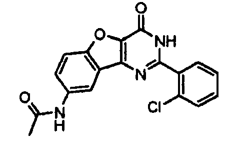 | N-[2-(2-chlorophenyl)-4-oxo-3,4-dihydro[1]benzofuro[3,2-d]pyrimidin-8-yl]acetamide **Activity = B** |
| ***92** | 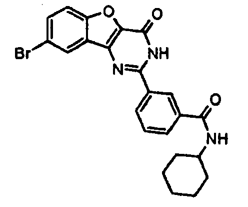 | 3-(8-bromo-4-oxo-3,4-dihydro[1]benzofuro[3,2-d]pyrimidin-2-yl)-N-cyclohexylbenzamide **Activity = B and C** |
| ***93** | 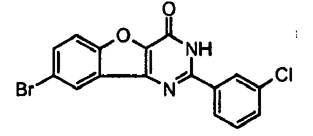 | 8-bromo-2-(3-chlorophenyl)benzofuro[3, 2-d]pyrimidin-4(3H)-one **Activity = B and C** |
| ***94** | 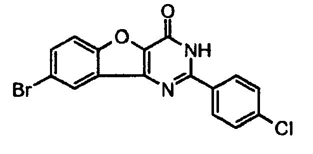 | 8-bromo-2-(4-chlorophenyl)benzofuro[3, 2-d]pyrimidin-4(3H)-one **Activity = B** |
| ***95** | 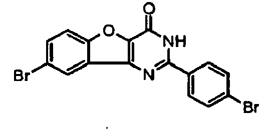 | 8-bromo-2-(4-bromophenyl)benzofuro[3 ,2-d]pyrimidin-4(3H)-one **Activity = B** |
| ***96** | 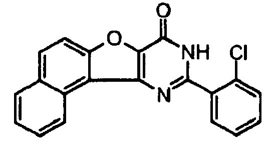 | 10-(2-chlorophenyl)naphtho[1',2' :4,5]furo[3,2-d]pyrimidin-8(9H)-one **Activity = B and C** |
| ***97** | 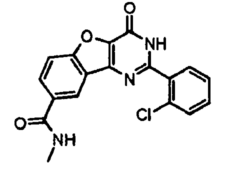 | 2-(2-chlorophenyl)-N-methyl-4-oxo-3,4-dihydrobenzofuro[3,2-d]pyrimidine-8-carboxamide **Activity = B** |
| ***98** | 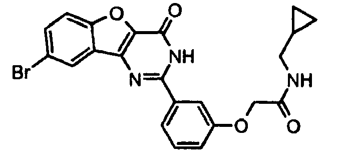 | 2-{[3-(8-bromo-4-oxo-3,4- dihydro[1]benzofuro[3,2d]pyrimidin-2-yl)phenyl]oxy}-N-(cyclopropylmethyl)aceta mide **Activity = B** |
| ***99** | 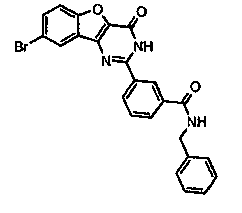 | 3-(8-bromo-4-oxo-3,4-dihydro[1]benzofuro[3,2-d]pyrimidin-2-yl)-N-(phenylmethyl)benzamide **Activity = B and C** |
| **100** | 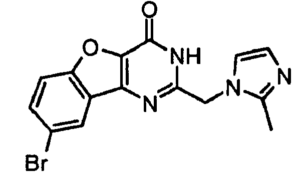 | 8-bromo-2-[(2-methyl-1H-imidazol-1-yl)methyl][1]benzofuro[3, 2-d]pyrimidin-4(3H)-one **Activity = A, B and C** |
| ***101** | 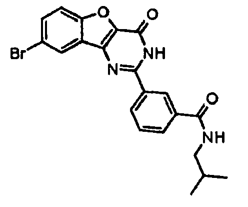 | 3-(8-bromo-4-oxo-3,4-dihydro[1]benzofuro[3,2-d]pyrimidin-2-yl)-N-(2-methylpropyl)benzamide **Activity = B and C** |
| ***102** | 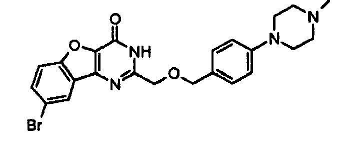 | 8-bromo-2-[({[4-(4-methylpiperazin-1-yl)phenyl]methyl}oxy)met hyl][1]benzofuro[3,2-d]pyrimidin-4(3H)-one **Activity = B and C** |
| **103** | 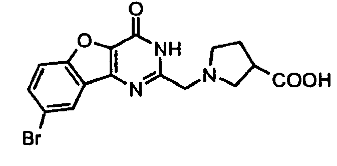 | 1-[(8-bromo-4-oxo-3,4-dihydro[1]benzofuro[3,2-d]pyrimidin-2-yl)methyl]pyrrolidine-3-carboxylic acid **Activity = A, B and C** |
| **104** | 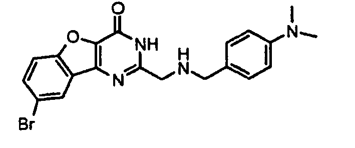 | 8-bromo-2-[({[4-(dimethylamino)phenyl]m ethyl}amino)methyl][1]be nzofuro[3,2-d]pyrimidin-4(3H)-one **Activity = B and C** |
| **105** | 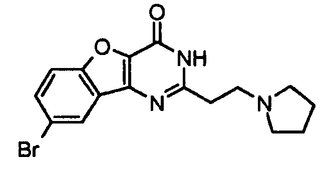 | 8-bromo-2-(2-pyrrolidin-1-ylethyl)[1]benzofuro[3,2-d]pyrimidin-4(3H)-one **Activity = B and C** |
| ***106** | 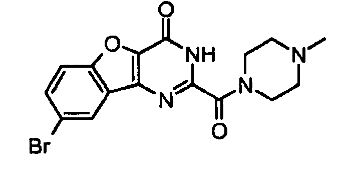 | 8-bromo-2-[(4-methylpiperazin-1-yl)carbonyl][1]benzofuro[ 3,2-d]pyrimidin-4(3H)-one **Activity = B** |
| ***107** | 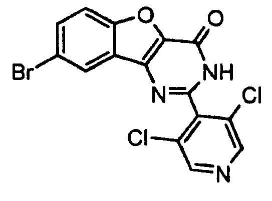 | 8-bromo-2-(3,5-dichloropyridin-4-yl)[1]benzofuro[3,2-d]pyrimidin-4(3H)-one **Activity = A, B and C** |
| **108** | 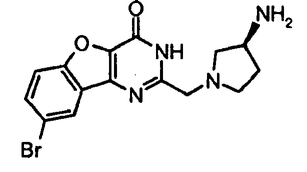 | 2-{[(3S)-3-aminopyrrolidin-1-yl]methyl}-8-bromo[1]benzofuro[3,2-d]pyrimidin-4(3H)-one **Activity = A, B and C** |
| **109** | 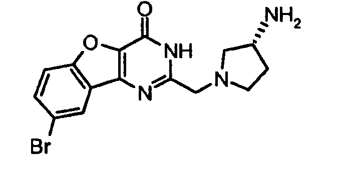 | 2-{[(3R)-3-aminopyrrolidin-1-yl]methyl}-8-bromo[1]benzofuro[3,2-d]pyrimidin-4(3H)-one **Activity = A, B and C** |
| **110** | 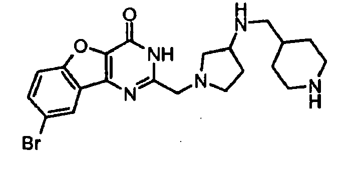 | 8-bromo-2-({3-[(piperidin-4-ylmethyl)amino]pyrrolidin -1-yl} methyl)[1]benzofuro[3, 2-d]pyrimidin-4(3H)-one **Activity = A, B and C** |
| **111** | 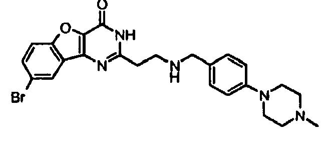 | 8-bromo-2-[2-({[4-(4-methylpiperazin-1-yl)phenyl]methyl}amino)e thyl][1]benzofuro[3,2-d]pyrimidin-4(3H)-one **Activity = A, B and C** |
| ***112** | 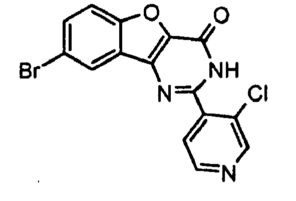 | 8-bromo-2-(3-chloropyridin-4-yl)[1]benzofuro[3,2-d]pyrimidin-4(3H)-one **Activity = A, B and C** |
| **113** | 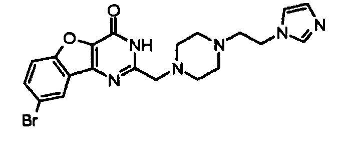 | 8-bromo-2-({4-[2-(1H-imidazol-1-yl)ethyl]piperazin-1-yl}methyl)[1]benzofuro[3, 2-d]pyrimidin-4(3H)-one **Activity = A, B and C** |
| **114** | 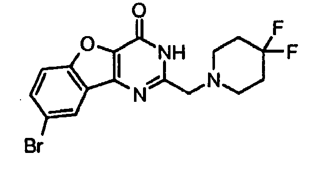 | 8-bromo-2-[(4,4-difluoropiperidin-1-yl)methyl][1]benzofuro[3, 2-d]pyrimidin-4(3H)-one **Activity = A, B and C** |
| **115** | 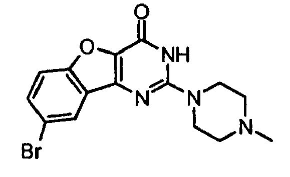 | 8-bromo-2-(4-methylpiperazin-1-yl)[1]benzofuro[3,2-d]pyrimidin-4(3H)-one **Activity = A, B and C** |
| ***116** | 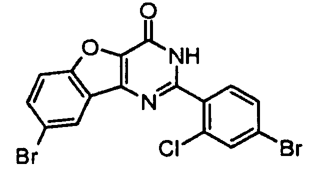 | 8-bromo-2-(4-bromo-2-chlorophenyl)[1]benzofur o[3,2-d]pyrimidin-4(3H)-one **Activity = A, B and C** |
| **117** | 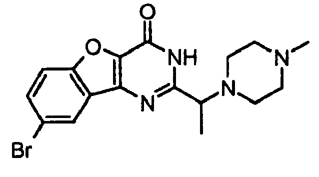 | 8-bromo-2-[1-(4-methylpiperazin-1-yl)ethyl][1]benzofuro[3,2-d]pyrimidin-4(3H)-one **Activity = A, B and C** |
| **118** | 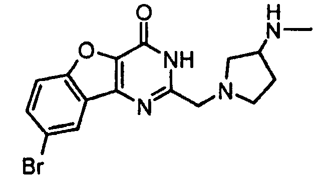 | 8-bromo-2-{[3-(methylamino)pyrrolidin-1-yl]methyl}[1]benzofuro[3, 2-d]pyrimidin-4(3H)-one **Activity = A, B and C** |
| **119** | 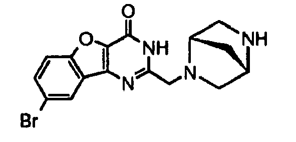 | 8-bromo-2-[(1S,4S)-2,5-diazabicyclo[2.2.1]hept-2-ylmethyl][1]benzofuro[3,2 -d]pyrimidin-4(3H)-one **Activity = A, B and C** |
| ***120** | 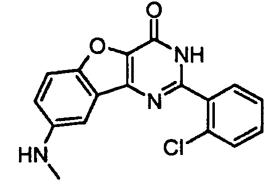 | 2-(2-chlorophenyl)-8-(methylamino)[1]benzofur o[3,2-d]pyrimidin-4(3H)-one **Activity = B** |
| ***121** | 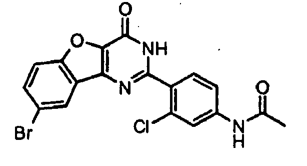 | N-[4-(8-bromo-4-oxo-3,4-dihydro[1]benzofuro[3,2-d]pyrimidin-2-yl)-3- chlorophenyl]acetamide **Activity = A, B and C** |
| **122** | 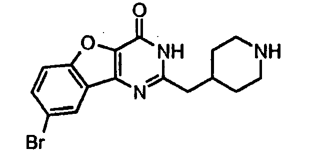 | 8-bromo-2-(piperidin-4-ylmethyl)[1]benzofuro[3,2 -d]pyrimidin-4(3H)-one **Activity = B and C** |
| ***123** | 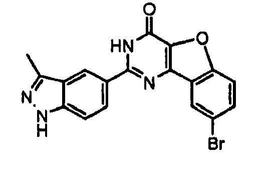 | 8-bromo-2-(3-methyl-1H-indazol-5-yl)[1]benzofuro[3,2-d]pyrimidin-4(3H)-one **Activity = A, B and C** |
| ***124** | 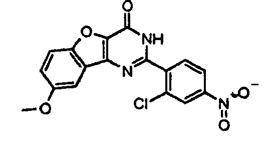 | 2-(2-chloro-4-nitrophenyl)-8-(methyloxy)[1]benzofuro[ 3,2-d]pyrimidin-4(3H)-one **Activity = A, B and C** |
| ***125** | 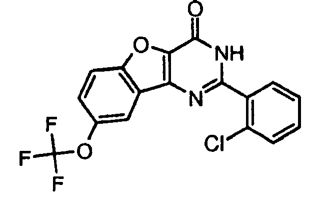 | 2-(2-chlorophenyl)-8-[(trifluoromethyl)oxy][1]b enzofuro[3,2-d]pyrimidin-4(3H)-one **Activity = B** |
| ***126** | 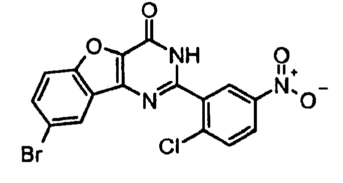 | 8-bromo-2-(2-chloro-5-nitrophenyl)[1]benzofuro[ 3,2-d]pyrimidin-4(3H)-one **Activity = A, B and C** |
| ***127** | 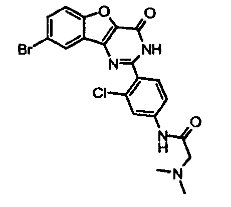 | N-[4-(8-bromo-4-oxo-3,4-dihydro[]benzofuro[3,2-d]pyrimidin-2-yl)-3-chlorophenyl]-N, N²-dimethylglycinamide **Activity = A, B and C** |
| ***128** | 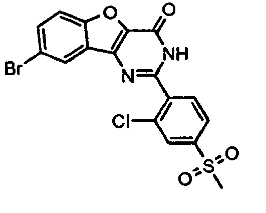 | 8-bromo-2-[2-chloro-4-(methylsulfonyl)phenyl][1 ]benzofuro[3,2-d]pyrimidin-4(3H)-one **Activity = A, B and C** |
| ***129** | 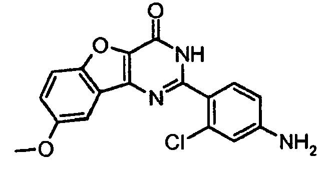 | 2-(4-amino-2-chlorophenyl)-8-(methyloxy)[1]benzofuro[ 3,2-d]pyrimidin-4(3H)-one **Activity = B and C** |
| **130** | 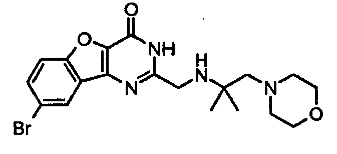 | 8-bromo-2-{[(1,1-dimethyl-2-morpholin-4-ylethyl)amino]methyl}[1] benzofuro[3,2-d]pyrimidin-4(3H)-one **Activity = A, B and C** |
| **131** | 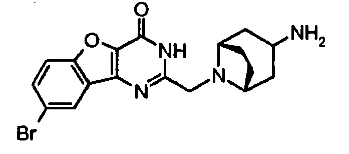 | 2-{[(1R,5S)-3-amino-8-azabicyclo[3.2.1]oct-8-yl]methyl}-8-bromo[1]benzofuro[3,2-d]pyrimidin-4(3H)-one **Activity = B** |
| **132** | 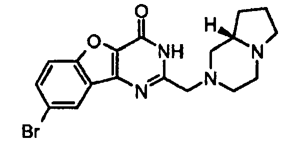 | 8-bromo-2-[(8aS)-hexahydropyrrolo[1,2-a]pyrazin-2(1H)-ylmethyl][1]benzofuro[3,2 -d]pyrimidin-4(3H)-one **Activity = B** |
| **133** | 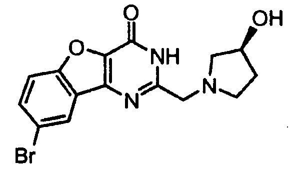 | 8-bromo-2-{[(3S)-3-hydroxypyrrolidin-1-yl]methyl}[1]benzofuro[3, 2-d]pyrimidin-4(3H)-one **Activity = A, B and C** |
| ***134** | 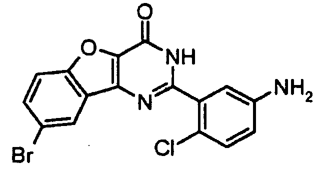 | 2-(5-amino-2-chlorophenyl)-8-bromo[1]benzofuro[3,2-d]pyrimidin-4(3H)-one **Activity = A, B and C** |
| ***135** | 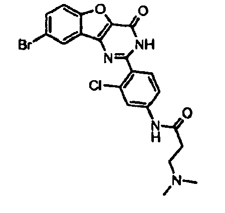 | N-[4-(8-bromo-4-oxo-3,4-dihydro[1]benzofuro[3,2-d]pyrimidin-2-yl)-3-chlorophenyl]-N, N³-dimethyl-beta-alaninamide **Activity = A, B and C** |
| ***136** | 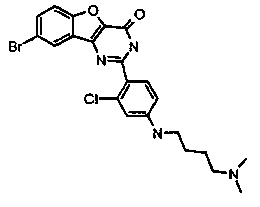 | 8-bromo-2-(2-chloro-4-{[4-(dimethylamino)butyl]ami no}phenyl)[1]benzofuro[3 ,2-d]pyrimidin-4(3H)-one **Activity = B and C** |
| ***137** | 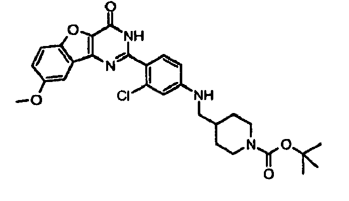 | 1,1-dimethylethyl 4-[({3-chloro-4-[8-(methyloxy)-4-oxo-3,4-dihydro[1]benzofuro[3,2-d]pyrimidin-2-yl]phenylamino)methyl]p iperidine-1-carboxylate **Activity = B and C** |
| ***138** | 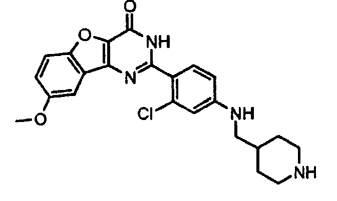 | 2-{2-chloro-4-[(piperidin-4-ylmethyl)amino]phenyl}-8-(methyloxy)[1]benzofuro[ 3,2-d]pyrimidin-4(3H)-one **Activity = A, B and C** |
| ***139** | 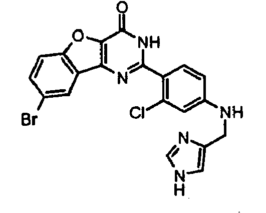 | 8-bromo-2-{2-chloro-4-[(1H-imidazol-4-ylmethyl)amino]phenyl}[1 ]benzofuro[3,2-d]pyrimidin-4(3H)-one **Activity = B and C** |
| ***140** | 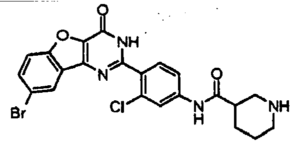 | N-[4-(8-bromo-4-oxo-3,4-dihydro[1]benzofuro[3,2-d]pyrimidin-2-yl)-3-chlorophenyl]piperidine-3-carboxamide **Activity = A, B and C** |
| **141** | 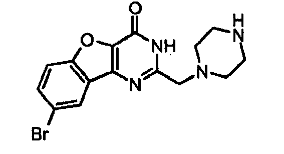 | 8-bromo-2-(piperazin-1-ylmethyl)[1]benzofuro[3,2 -d]pyrimidin-4(3H)-one **Activity = A, B and C** |
| **142** | 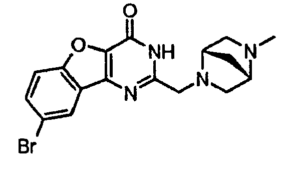 | 8-bromo-2-{[(1S,4S)-5-methyl-2,5-diazabicyclo[2.2.1]hept-2-yl]methyl}[1]benzofuro[3, 2-d]pyrimidin-4(3H)-one **Activity = A, B and C** |
| ***143** | 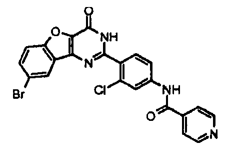 | N-[4-(8-bromo-4-oxo-3,4-dihydro[1]benzofuro[3,2-d]pyrimidin-2-yl)-3-chlorophenyl]pyridine-4-carboxamide **Activity = A, B and C** |
| ***144** | 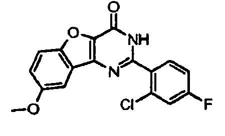 | 2-(2-chloro-4-fluorophenyl)-8-(methyloxy)[1]benzofuro[ 3,2-d]pyrimidin-4(3H)-one **Activity = A, B and C** |
| ***145** | 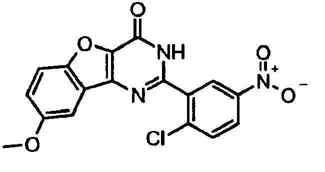 | 2-(2-chloro-5-nitrophenyl)-8-(methyloxy)[1]benzofuro[ 3,2-d]pyrimidin-4(3H)-one **Activity = B and C** |
| ***146** | 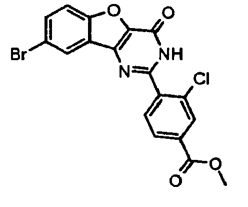 | methyl 4-(8-bromo-4-oxo-3,4-dihydro[1]benzofuro[3,2-d]pyrimidin-2-yl)-3-chlorobenzoate **Activity = B and C** |
| **147** | 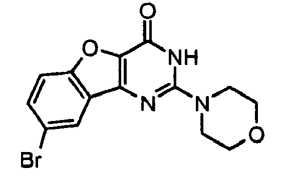 | 8-bromo-2-morpholin-4-yl[1]benzofuro[3,2-d]pyrimidin-4(3H)-one **Activity = A, B and C** |
| ***148** | 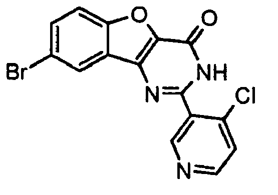 | 8-bromo-2-(4-chloropyridin-3-yl)[1]benzofuro[3,2-d]pyrimidin-4(3H)-one **Activity = A, B and C** |
| **149** | 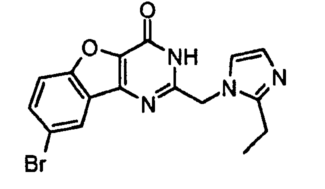 | 8-bromo-2-[(2-ethyl-1H-imidazol-1-yl)methyl][1]benzofuro[3, 2-d]pyrimidin-4(3H)-one **Activity = B and C** |
| **150** | 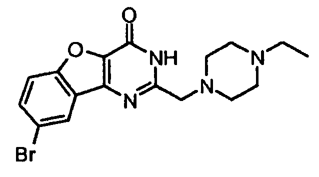 | 8-bromo-2-[(4-ethylpiperazin-1-yl)methyl][1]benzofuro[3, 2-d]pyrimidin-4(3H)-one **Activity = B and C** |
| **151** | 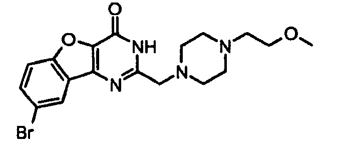 | 8-bromo-2-({4-[2-(methyloxy)ethyl]piperazi n-1-yl}methyl)[1]benzofuro[3, 2-d]pyrimidin-4(3H)-one **Activity = B and C** |
| ***152** | 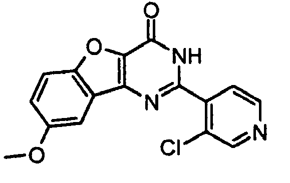 | 2-(3-chloropyridin-4-yl)-8-(methyloxy)[1]benzofuro[ 3,2-d]pyrimidin-4(3H)-one **Activity = B and C** |
| **153** | 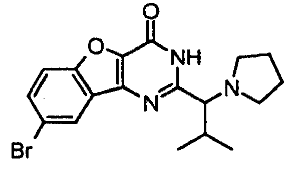 | 8-bromo-2-(2-methyl-1-pyrrolidin-1-ylpropyl)[1]benzofuro[3,2 -d]pyrimidin-4(3H)-one **Activity = A, B and C** |
| ***154** | 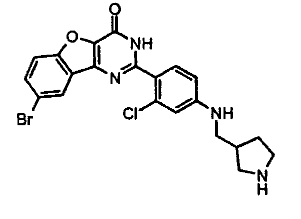 | 8-bromo-2-{2-chloro-4-[(pyrrolidin-3-ylmethyl)amino]phenyl}[1 ]benzofuro[3,2-d]pyrimidin-4(3H)-one **Activity = A, B and C** |
| ***155** | 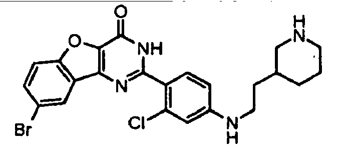 | 8-bromo-2-{2-chloro-4-[(2-piperidin-3-ylethyl)amino]phenyl}[1] benzofuro[3,2-d]pyrimidin-4(3H)-one **Activity = A, B and C** |
| ***156** | 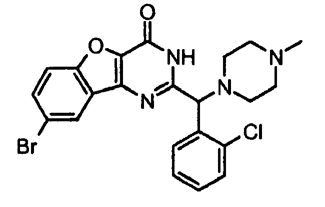 | 8-bromo-2-[(2-chlorophenyl)(4-methylpiperazin-1-yl)methyl][1]benzofuro[3, 2-d]pyrimidin-4(3H)-one **Activity = B** |
| **157** | 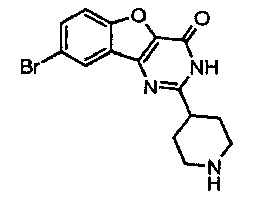 | 8-bromo-2-piperidin-4-yl[1]benzofuro[3,2-d]pyrimidin-4(3H)-one **Activity = A, B and C** |
| **158** | 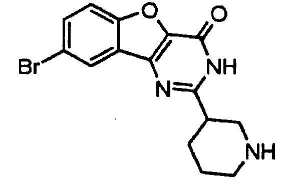 | 8-bromo-2-piperidin-3-yl[1]benzofuro[3,2-d]pyrimidin-4(3H)-one **Activity = A, B and C** |
| ***159** | 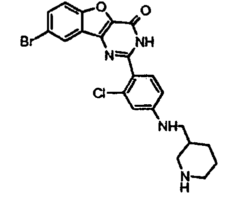 | 8-bromo-2-{2-chloro-4-[(piperidin-3-ylmethyl)amino]phenyl}[1 ]benzofuro[3,2-d]pyrimidin-4(3H)-one **Activity = A, B and C** |
| ***160** | 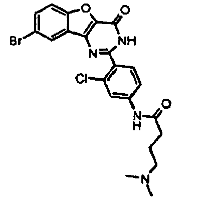 | N-[4-(8-bromo-4-oxo-3,4-dihydro[1]benzofuro[3,2-d]pyrimidin-2-yl)-3-chlorophenyl]-4-(dimethylamino)butanami de **Activity = A, B and C** |
| ***161** | 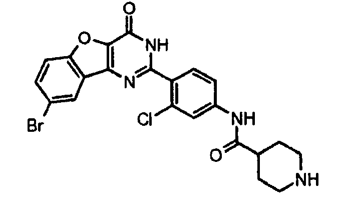 | N-[4-(8-bromo-4-oxo-3,4-dihydro[1]benzofuro[3,2-d]pyrimidin-2-yl)-3-chlorophenyl]piperidine-4-carboxamide **Activity = A, B and C** |
| ***162** | 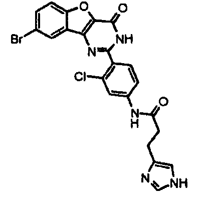 | N-[4-(8-bromo-4-oxo-3,4-dihydro[1]benzofuro[3,2-d]pyrimidin-2-yl)-3-chlorophenyl]-3-(1H-imidazol-4-yl)propanamide **Activity = B and C** |
| ***163** | 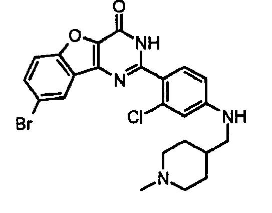 | 8-bromo-2-(2-chloro-4-{[(1-methylpiperidin-4-yl)methyl]amino}phenyl)[ 1]benzofuro[3,2-d]pyrimidin-4(3H)-one **Activity = B and C** |
| **164** | 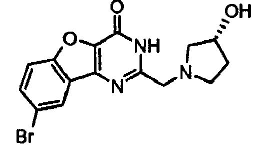 | 8-bromo-2-{[(3R)-3-hydroxypyrrolidin-1-yl]methyl}[1]benzofuro[3, 2-d]pyrimidin-4(3H)-one **Activity = A, B and C** |
| **165** | 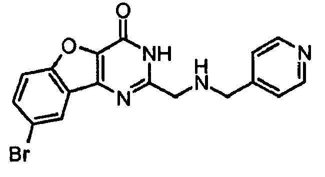 | 8-bromo-2-{[(pyridin-4-ylmethyl)amino]methyl}[ 1]benzofuro[3,2-d]pyrimidin-4(3H)-one **Activity = B and C** |
| **166** | 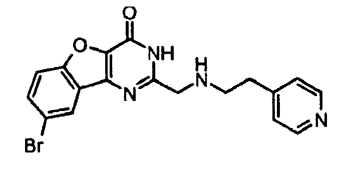 | 8-bromo-2-{[(2-pyridin-4-ylethyl)amino]methyl}[1] benzofuro[3,2-d]pyrimidin-4(3H)-one **Activity = A, B and C** |
| **167** | 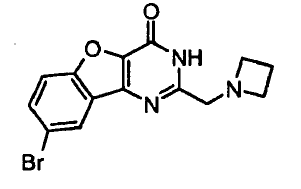 | 2-(azetidin-1-ylmethyl)-8-bromo[1]benzofuro[3,2-d]pyrimidin-4(3H)-one **Activity = A, B and C** |
| **168** | 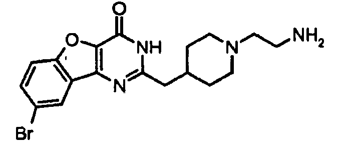 | 2-{[1-(2-aminoethyl)piperidin-4-yl]methyl}-8-bromo[1]benzofuro[3,2-d]pyrimidin-4(3H)-one **Activity = B and C** |
| **169** | 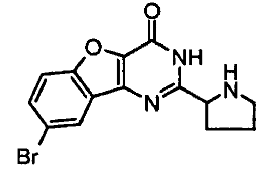 | 8-bromo-2-pyrrolidin-2-yl[1]benzofuro[3,2-d]pyrimidin-4(3H)-one **Activity = A, B and C** |
| ***170** | 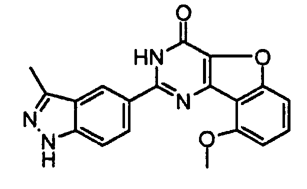 | 2-(3-methyl-1H-indazol-5-yl)-9-(methyloxy)[1]benzofuro[ 3,2-d]pyrimidin-4(3H)-one **Activity = B and C** |
| ***171** | 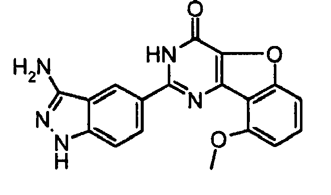 | 2-(3-amino-1H-indazol-5-yl)-9-(methyloxy)[1]benzofuro[ 3,2-d]pyrimidin-4(3H)-one **Activity = B and C** |
| **172** | 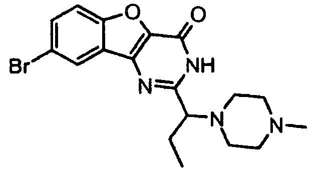 | 8-bromo-2-[1-(4-methylpiperazin-1-yl)propyl][1]benzofuro[3, 2-d]pyrimidin-4(3H)-one **Activity = B and C** |
| ***173** | 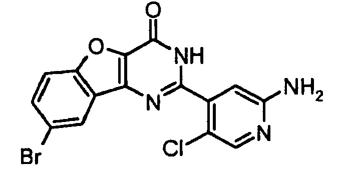 | 2-(2-amino-5-chloropyridin-4-yl)-8-promo[1]benzofuro[3,2-d]pyrimidin-4(3H)-one **Activity = A, B and C** |
| **174** | 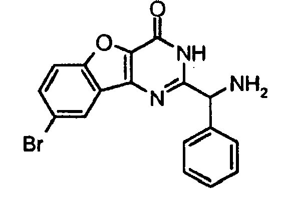 | 2-[amino(phenyl)methyl]-8-bromo[1]benzofuro[3,2- d]pyrimidin-4(3H)-one **Activity = A, B and C** |
| **175** | 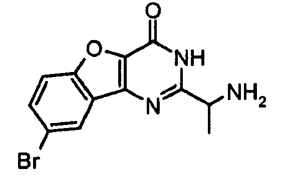 | 2-(1-aminoethyl)-8-bromo[1]benzofuro[3,2-d]pyrimidin-4(3H)-one **Activity = A, B and C** |
| **176** | 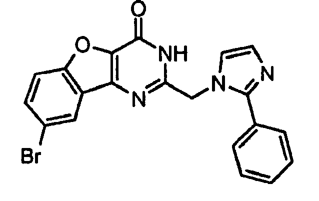 | 8-bromo-2-[(2-phenyl-1H-imidazol-1-yl)methyl][1]benzofuro[3, 2-d]pyrimidin-4(3H)-one **Activity = B and C** |
| **177** | 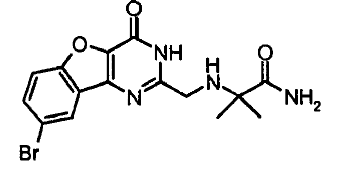 | N²-[(8-bromo-4-oxo-3,4-dihydro[1]benzofuro[3,2-d]pyrimidin-2-yl)methyl]-2-methylalaninamide **Activity = B and C** |
| ***178** | 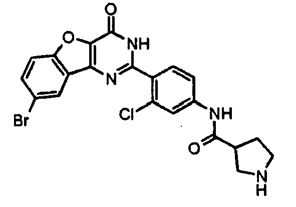 | N-[4-(8-bromo-4-oxo-3,4-dihydro[1]benzofuro[3,2-d]pyrimidin-2-yl)-3-chlorophenyl]pyrrolidine-3-carboxamide **Activity = A, B and C** |
| ***179** | 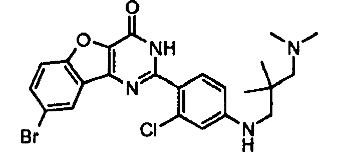 | 8-bromo-2-(2-chloro-4-{ [3-(dimethylamino)-2,2-dimethylpropyl]amino} ph enyl)[1]benzofuro[3,2-d]pyrimidin-4(3H)-one **Activity = B and C** |
| **180** | 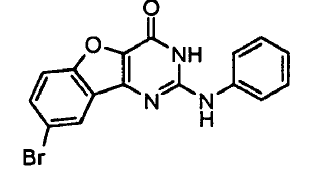 | 8-bromo-2-(phenylamino)[ 1 ]benzofur o[3,2-d]pyrimidin-4(3H)-one **Activity = B and C** |
| ***181** | 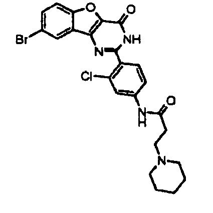 | N-[4-(8-bromo-4-oxo-3,4-dihydro[1]benzofuro[3,2-d]pyrimidin-2-yl)-3-chlorophenyl]-3-piperidin-1-ylpropanamide **Activity = A, B and C** |
| ***182** | 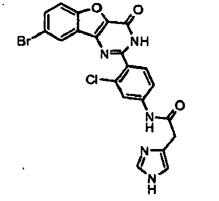 | N-[4-(8-bromo-4-oxo-3,4-dihydro[1]benzofuro[3,2-d]pyrimidin-2-yl)-3-chlorophenyl]-2-(1H-imidazol-4-yl)acetamide **Activity = A, B and C** |
| **183** | 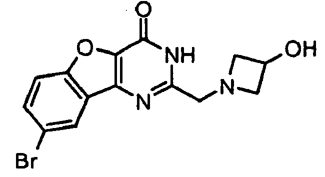 | 8-bromo-2-[(3-hydroxyazetidin-1-yl)methyl][ 1 ]benzofuro[3, 2-d]pyrimidin-4(3H)-one **Activity = A, B and C** |
| **184** | 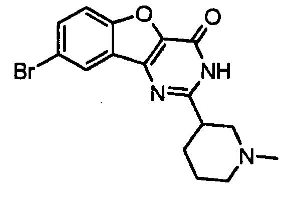 | 8-bromo-2-(1-methylpiperidin-3-yl)[1]benzofuro[3,2-d]pyrimidin-4(3H)-one **Activity = A, B and C** |
| **185** | 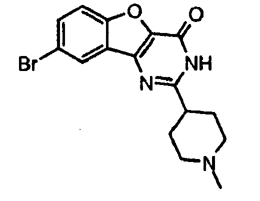 | 8-bromo-2-(1-methylpiperidin-4-yl)[1]benzofuro[3,2-d]pyrimidin-4(3H)-one **Activity** = **A, B and C** |
| **186** | 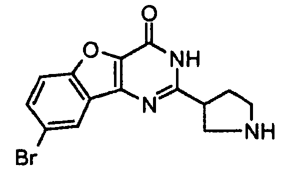 | 8-bromo-2-pyrrolidin-3-yl[1]benzofuro[3,2-d]pyrimidin-4(3H)-one **Activity** = **A, B and C** |
| **187** | 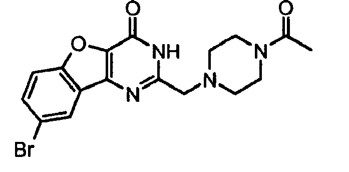 | 2-[(4-acetylpiperazin-1-yl)methyl]-8-bromo[1]benzofuro[3,2-d]pyrimidin-4(3H)-one **Activity** = **B and C** |
| ***188** | 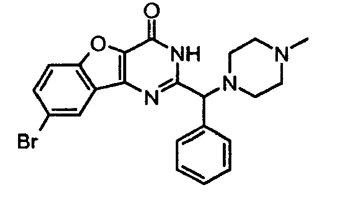 | 8-bromo-2-[(4-methylpiperazin-1-yl)(phenyl)methyl][1]benz ofuro[3,2-d]pyrimidin-4(3H)-one **Activity = B** |
| **189** | 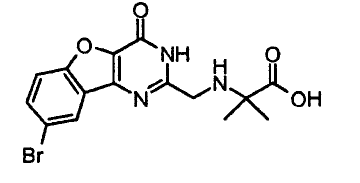 | N-[(8-bromo-4-oxo-3,4-dihydro[1]benzofuro[3,2-d]pyrimidin-2-yl)methyl]-2-methylalanine **Activity** = **A, B and C** |
| **190** | 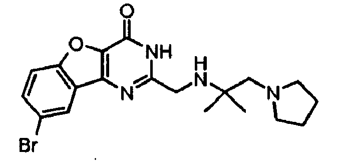 | 8-bromo-2-{[(1,1-dimethyl-2-pyrrolidin-1-ylethyl)amino]methyl}[1] benzofuro[3,2-d]pyrimidin-4(3H)-one **Activity = B and C** |
| **191** | 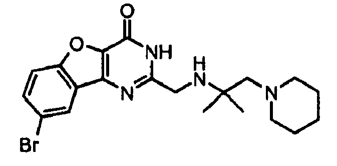 | 8-bromo-2-{[(1,1-dimethyl-2-piperidin-1-ylethyl)amino]methyl} [1] benzofuro[3,2-d]-pyrimidin-4(3H)-one **Activity = A, B and C** |
| **192** | 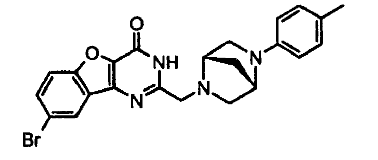 | 8-bromo-2-{[(1S,4S)-5-(4-methylphenyl)-2,5-diazabicyclo[2.2.1]hept-2-yl]methyl}[1]benzofuro[3, 2-d]pyrimidin-4(3H)-one **Activity = B and C** |
| **193** | 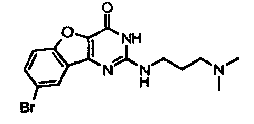 | 8-bromo-2-{[3-(dimethylamino)propyl]a mino}[1]benzofuro[3,2-d]pyrimidin-4(3H)-one **Activity = B and C** |
| **194** | 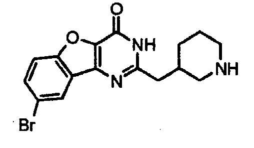 | 8-bromo-2-(piperidin-3-ylmethyl)[1]benzofuro[3,2 -d]pyrimidin-4(3H)-one **Activity = A, B and C** |
| **195** | 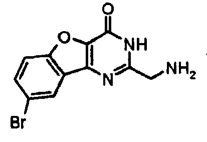 | 2-(aminomethyl)-8-bromo[1]benzofuro[3,2-d]pyrimidin-4(3H)-one **Activity = A, B and C** |
| **196** | 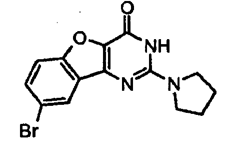 | 8-bromo-2-pyrrolidin-1-yl[1]benzofuro[3,2-d]pyrimidin-4(3H)-one **Activity** = **B and C** |
| ***197** | 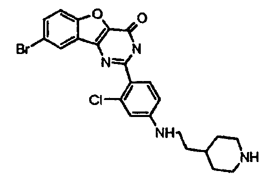 | 8-bromo-2-{2-chloro-4-[(2-piperidin-4-ylethyl)amino]phenyl}[1] benzofuro[3,2-d]pyrimidin-4(3H)-one **Activity** = **A, B and C** |
| **198** | 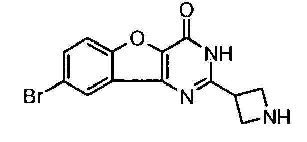 | 2-azetidin-3-yl-8-bromo[1]benzofuro[3,2-d]pyrimidin-4(3H)-one **Activity = A, B and C** |
| **199** | 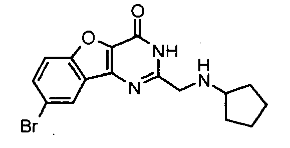 | 8-bromo-2-[(cyclopentylamino)methy 1][1]benzofuro[3,2-d]pyrimidin-4(3H)-one **Activity** = **A, B and C** |
| **200** | 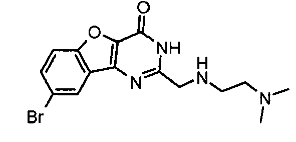 | 8-bromo-2-({[2-(dimethylamino)ethyl]ami no}methyl)[1]benzofuro[3 ,2-d]pyrimidin-4(3H)-one **Activity = B** |
| **201** | 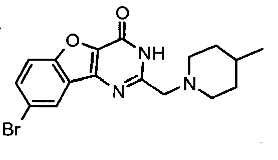 | 8-bromo-2-[(4-methylpiperidin-1-yl)methyl][1]benzofuro[3, 2-d]pyrimidin-4(3H)-one **Activity** = **A, B and C** |
| **202** | 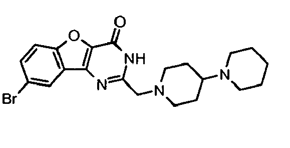 | 2-(1,4'-bipiperidin-1'-ylmethyl)-8-bromo[1]benzofuro[3,2-d]pyrimidin-4(3H)-one **Activity = B** |
| **203** | 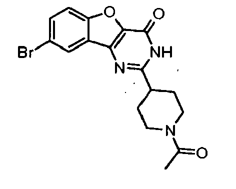 | 2-(1 -acetylpiperidin-4-yl)-8-bromo[1]benzofuro[3,2-d]pyrimidin-4(3H)-one **Activity = B and C** |
| **204** | 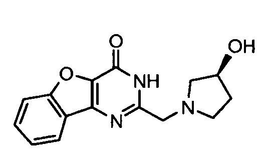 | 2-{[(3S)-3-hydroxypyrrolidin-1-yl]methyl}[1]benzofuro[3, 2-d]pyrimidin-4(3H)-one **Activity = B and C** |
| ***205** | 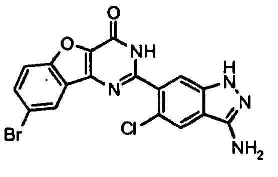 | 2-(3-amino-5-chloro-1H-indazol-6-yl)-8-bromo[1]benzofuro[3,2-d]pyrimidin-4(3H)-one **Activity = A, B and C** |
| **206** | 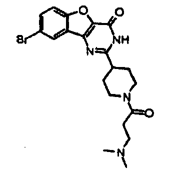 | 8-bromo-2-[1-(N,N-dimethyl-beta-alanyl)piperidin-4-yl][1]benzofuro[3,2-d]pyrimidin-4(3H)-one **Activity = B and C** |
| **207** | 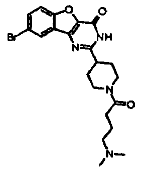 | 8-bromo-2-{1-[4-(dimethylamino)butanoyl] piperidin-4-yl}[1]benzofuro[3,2-d]pyrimidin-4(3H)-one **Activity = B and C** |
| **208** | 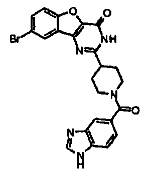 | 2-[1-(1H-benzimidazol-5-ylcarbonyl)piperidin-4-yl]-8-bromo[1]benzofuro[3,2-d]pyrimidin-4(3H)-one **Activity = B and C** |
| **209** | 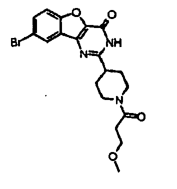 | 8-bromo-2-{1-[3-(methyloxy)propanoyl]pip eridin-4-yl}[1]benzofuro[3,2-d]pyrimidin-4(3H)-one **Activity** = **B and C** |
| **210** | 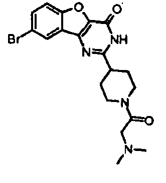 | 8-bromo-2-[1-(N,N-dimethylglycyl)piperidin-4-yl][1]benzofuro[3,2-d]pyrimidin-4(3H)-one **Activity = B and C** |
| ***211** | 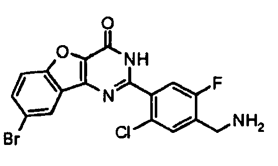 | 2-[4-(aminomethyl)-2-chloro-5-fluorophenyl]-8-bromo[1]benzofuro[3,2-d]pyrimidin-4(3H)-one **Activity = B and C** |
| **212** | 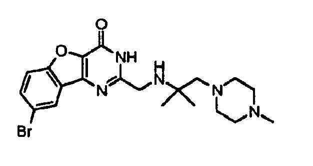 | 8-bromo-2-({[1,1-dimethyl-2-(4-methylpiperazin-1-yl)ethyl]amino} methyl)[1] benzofuro[3,2-d]pyrimidin-4(3H)-one **Activity = B and C** |
| **213** | 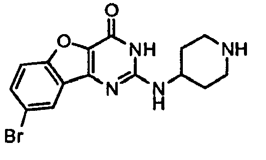 | 8-bromo-2-(piperidin-4-ylamino)[1]benzofuro[3,2-d]pyrimidin-4(3H)-one **Activity = B and C** |
| **214** | 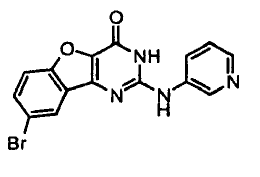 | 8-bromo-2-(pyridin-3-ylamino)[1]benzofuro[3,2-d]pyrimidin-4(3H)-one **Activity = A, B and C** |
| **215** | 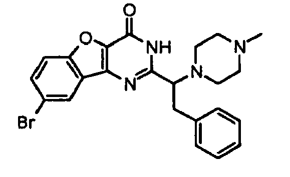 | 8-bromo-2-[1-(4-methylpiperazin-1-yl)-2-phenylethyl][1]benzofuro[ 3,2-d]pyrimidin-4(3H)-one **Activity** = **B** |
| ***216** | 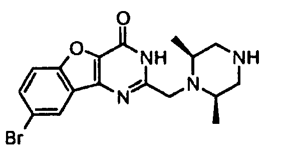 | 8-bromo-2-{[(2R,6S)-2,6-dimethylpiperazin-1-yl]methyl} [1]benzofuro[3, 2-d]pyrimidin-4(3H)-one **Activity = A, B and C** |
| ***217** | 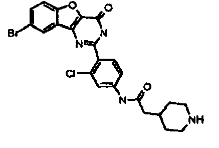 | N-[4-(8-bromo-4-oxo-3,4-dihydro[1]benzofuro[3,2-d]pyrimidin-2-yl)-3-chlorophenyl]-2-piperidin-4-ylacetamide **Activity = A, B and C** |
| **218** | 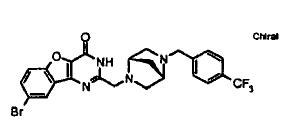 | 8-bromo-2-{[(1S,4S)-5-{[4-(trifluoromethyl)phenyl]m ethyl}-2,5-diazabicyclo[2.2.1]hept-2-yl]methyl}[1]benzofuro[3, 2-d]pyrimidin-4(3H)-one **Activity = B and C** |
| ***219** | 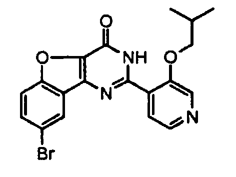 | 8-bromo-2-{3-[(2-methylpropyl)oxy]pyridin-4-yl}[1]benzofuro[3,2- d]pyrimidin-4(3H)-one **Activity = B** |
| ***220** | 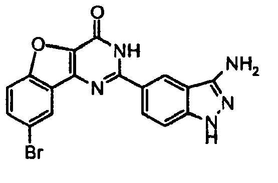 | 2-(3-amino-1H-indazol-5-yl)-8-bromo[1]benzofuro[3,2-d]pyrimidin-4(3H)-one **Activity = A, B and C** |
| **221** | 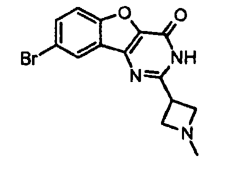 | 8-bromo-2-(1-methylazetidin-3-yl)[1]benzofuro[3,2-d]pyrimidin-4(3H)-one **Activity = A, B and C** |
| ***222** | 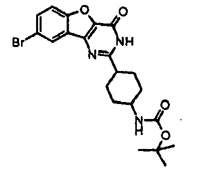 | 1,1-dimethylethyl [4-(8-bromo-4-oxo-3,4-dihydro[1]benzofuro[3,2-d]pyrimidin-2-yl)cyclohexyl]carbamate **Activity = B and C** |
| ***223** | 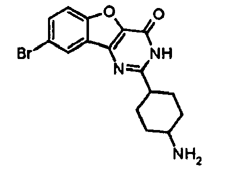 | 2-(4-aminocyclohexyl)-8-bromo[1]benzofuro[3,2-d]pyrimidin-4(3H)-one **Activity = A, B and C** |
| **224** | 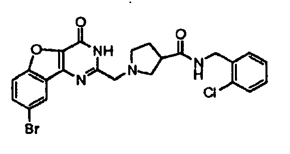 | 1-[(8-bromo-4-oxo-3,4-dihydro[1]benzofuro[3,2-d]pyrimidin-2-yl)methyl]-N-[(2-chlorophenyl)methyl]pyrr olidine-3-carboxamide **Activity = A, B and C** |
| **225** | 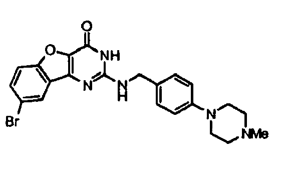 | 8-bromo-2-({[4-(4-methylpiperazin-1-yl)phenyl]methyl} amino)[ 1]benzofuro[3,2-d]pyrimidin-4(3H)-one **Activity = B** |
| **226** | 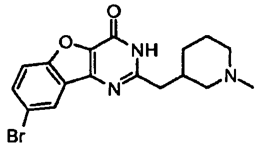 | 8-bromo-2-[(1-methylpiperidin-3-yl)methyl][1]benzofuro[3, 2-d]pyrimidin-4(3H)-one **Activity = A, B and C** |
| **227** | 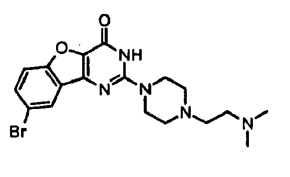 | 8-bromo-2-{4-[2-(dimethylamino)ethyl]pipe razin-1-yl}[1]benzofuro[3,2-d]pyrimidin-4(3H)-one **Activity = B and C** |
| **228** | 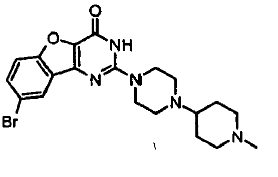 | 8-bromo-2-[4-(1-methylpiperidin-4-yl)piperazin-1-yl][1]benzofuro[3,2-d]pyrimidin-4(3H)-one **Activity = B and C** |
| **229** | 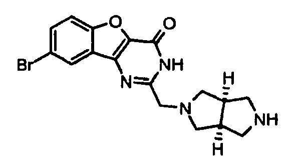 | 8-bromo-2-[(3aR,6aS)-hexahydropyrrolo[3,4-c]pyrrol-2(1H)-ylmethyl][1]benzofuro[3,2 -d]pyrimidin-4(3H)-one **Activity = B and C** |
| **230** | 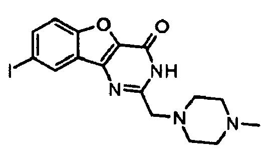 | 8-iodo-2-[(4-methylpiperazin-1-yl)methyl][1]benzofuro[3, 2-d]pyrimidin-4(3H)-one **Activity = B and C** |
| ***231** | 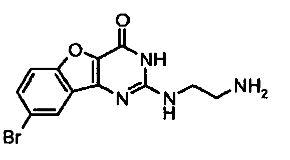 | 2-[(2-aminoethyl)amino]-8-bromo[1]benzofuro[3,2-d]pyrimidin-4(3H)-one **Activity = A, B and C** |
| **232** | 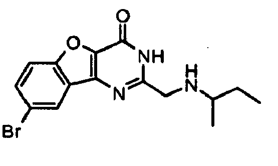 | 8-bromo-2-{[(1-methylpropyl)amino]meth yl}[1]benzofuro[3,2-d]pyrimidin-4(3H)-one **Activity = A, B and C** |
| **233** | 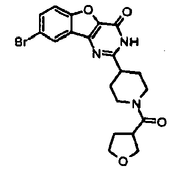 | 8-bromo-2-[1-(tetrahydrofuran-3-ylcarbonyl)piperidin-4-yl][1]benzofuro[3,2-d]pyrimidin-4(3H)-one **Activity = B and C** |
| **234** | 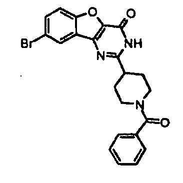 | 8-bromo-2-[1-(phenylcarbonyl)piperidin -4-yl][1]benzofuro[3,2-d]pyrimidin-4(3H)-one **Activity = B and C** |
| **235** | 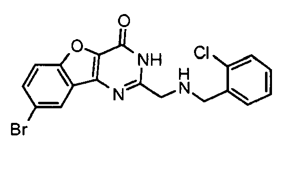 | 8-bromo-2-({[(2-chlorophenyl)methyl]amin o}methyl)[1]benzofuro[3, 2-d]pyrimidin-4(3H)-one **Activity = A, B and C** |
| **236** | 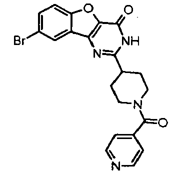 | 8-bromo-2-[1-(pyridin-4-ylcarbonyl)piperidin-4-yl][1]benzofuro[3,2-d]pyrimidin-4(3H)-one **Activity = B and C** |
| **237** | 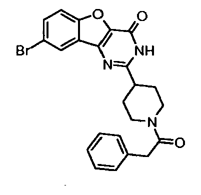 | 8-bromo-2-[1-(phenylacetyl)piperidin-4-yl][1]benzofuro[3,2-d]pyrimidin-4(3H)-one **Activity = B and C** |
| **238** | 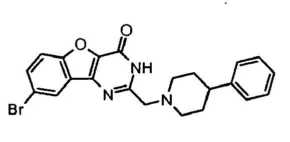 | 8-bromo-2-[(4-phenylpiperidin-1-yl)methyl][1]benzofuro[3, 2-d]pyrimidin-4(3H)-one **Activity = B and C** |
| **239** | 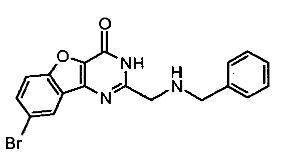 | 8-bromo-2-{[(phenylmethyl)amino]m ethyl}[1]benzofuro[3,2-d]pyrimidin-4(3H)-one **Activity = B and C** |
| **240** | 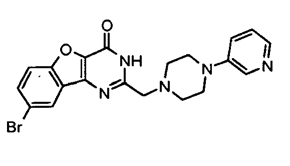 | 8-bromo-2-[(4-pyridin-3-ylpiperazin-1-yl)methyl][1]benzofuro[3, 2-d]pyrimidin-4(3H)-one **Activity = B and C** |
| ***241** | 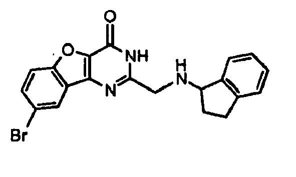 | 8-bromo-2-[(2,3-dihydro-1H-inden-1-ylamino)methyl][1]benzof uro[3,2-d]pyrimidin-4(3H)-one **Activity = B and C** |
| **242** | 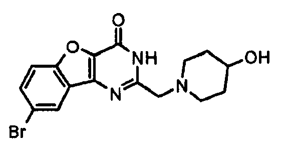 | 8-bromo-2-[(4-hydroxypiperidin-1-yl)methyl][1]benzofuro[3, 2-d]pyrimidin-4(3H)-one **Activity = A, B and C** |
| ***243** | 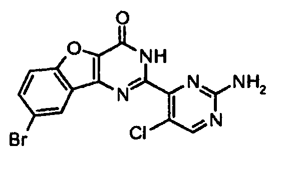 | 2-(2-amino-5-chloropyrimidin-4-yl)-8-bromo[1]benzofuro[3,2-d]pyrimidin-4(3H)-one **Activity = B and C** |
| **244** | 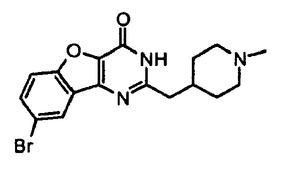 | 8-bromo-2-[(1-methylpiperidin-4-yl)methyl][1]benzofuro[3, 2-d]pyrimidin-4(3H)-one **Activity = B and C** |
| **245** | 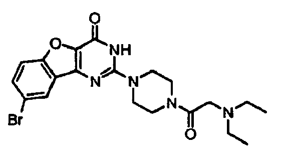 | 8-bromo-2-[4-(N,N-diethylglycyl)piperazin-1-yl][1]benzofuro[3,2-d]pyrimidin-4(3H)-one **Activity = B and C** |
| **246** | 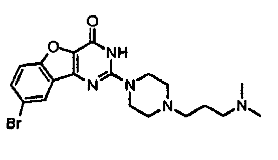 | 8-bromo-2-{4-[3-(dimethylamino)propyl]pi perazin-1-yl}[1]benzofuro[3,2-d]pyrimidin-4(3H)-one **Activity = B and C** |
| **247** | 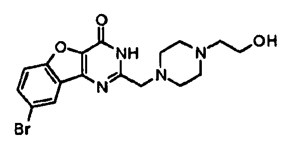 | 8-bromo-2-{[4-(2-hydroxyethyl)piperazin-1-yl]methyl}[1]benzofuro[3, 2-d]pyrimidin-4(3H)-one **Activity = B and C** |
| ***248** | 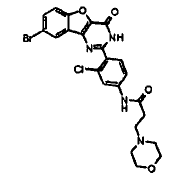 | N-[4-(8-bromo-4-oxo-3,4-dihydro[1]benzofuro[3,2-d]pyrimidin-2-yl)-3-chlorophenyl]-3-morpholin-4-ylpropanamide **Activity = A, B and C** |
| ***249** | 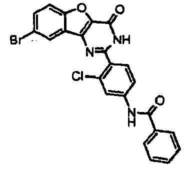 | N-[4-(8-bromo-4-oxo-3,4-dihydro[1]benzofuro[3,2-d]pyrimidin-2-yl)-3-chlorophenyl]benzamide **Activity = B and C** |
| ***250** | 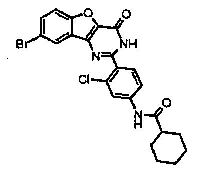 | N-[4-(8-bromo-4-oxo-3,4-dihydro[1]benzofuro[3,2-d]pyrimidin-2-yl)-3-chlorophenyl]cyclohexane carboxamide **Activity = B and C** |
| ***251** | 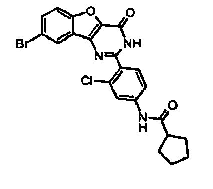 | N-[4-(8-bromo-4-oxo-3,4-dihydro[1]benzofuro[3,2-d]pyrimidin-2-yl)-3-chlorophenyl]cyclopentan ecarboxamide **Activity = B and C** |
| **252** | 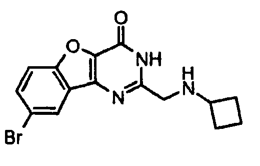 | 8-bromo-2-[(cyclobutylamino)methyl ][1]benzofuro[3,2-d]pyrimidin-4(3H)-one **Activity = A, B and C** |
| **253** | 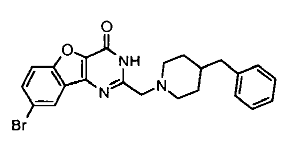 | 8-bromo-2-{[4-(phenylmethyl)piperidin-1-yl]methyl}[1]benzofuro[3, 2-d]pyrimidin-4(3H)-one **Activity = B and C** |
| **254** | 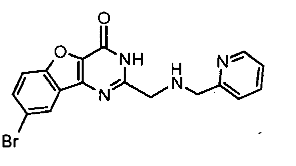 | 8-bromo-2-{[(pyridin-2-ylmethyl)amino]methyl}[ 1]benzofuro[3,2-d]pyrimidin-4(3H)-one **Activity = B and C** |
| **255** | 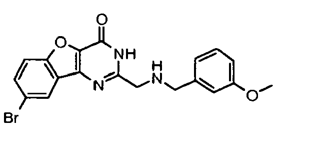 | 8-bromo-2-[({[3-(methyloxy)phenyl]methyl }amino)methyl][1]benzof uro[3,2-d]pyrimidin-4(3H)-one **Activity = B and C** |
| **256** | 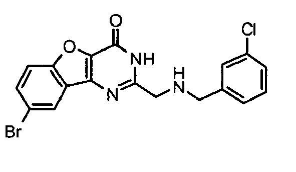 | 8-bromo-2-({[(3-chlorophenyl)methyl]amin o}methyl)[1]benzofuro[3, 2-d]pyrimidin-4(3H)-one **Activity = B and C** |
| **257** | 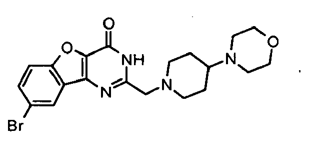 | 8-bromo-2-[(4-morpholin-4-ylpiperidin-1-yl)methyl][1]benzofuro[3, 2-d]pyrimidin-4(3H)-one **Activity = B and C** |
| **258** | 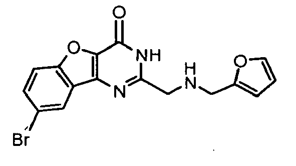 | 8-bromo-2-{[(furan-2-ylmethyl)amino]methyl}[ 1]benzofuro[3,2-d]pyrimidin-4(3H)-one **Activity = B and C** |
| **259** | 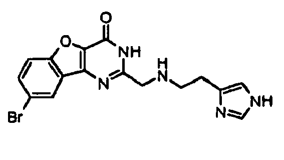 | 8-bromo-2-({[2-(1H-imidazol-4-yl)ethyl]amino}methyl)[1] benzofuro[3,2-d]pyrimidin-4(3H)-one **Activity = A, B and C** |
| **260** | 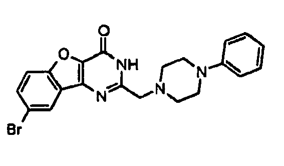 | 8-bromo-2-[(4-phenylpiperazin-1-yl)methyl][1]benzofuro[3, 2-d]pyrimidin-4(3H)-one **Activity = B and C** |
| **261** | 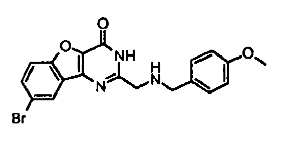 | 8-bromo-2-[({[4-(methyloxy)phenyl]methyl }amino)methyl][1]benzof uro[3,2-d]pyrimidin-4(3H)-one **Activity = B and C** |
| **262** | 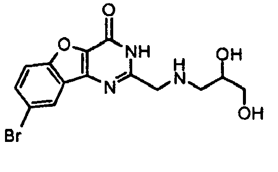 | 8-bromo-2-{[(2,3-dihydroxypropyl)amino]m ethyl}[1]benzofuro[3,2-d]pyrimidin-4(3H)-one **Activity = A, B and C** |
| ***263** | 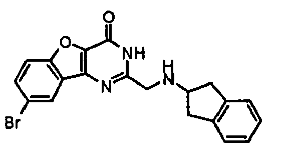 | 8-bromo-2-[(2,3-dihydro-1H-inden-2-ylamino)methyl][1]benzof uro[3,2-d]pyrimidin-4(3H)-one **Activity = B and C** |
| **264** | 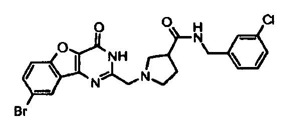 | 1-[(8-bromo-4-oxo-3,4-dihydro[1]benzofuro[3,2-d]pyrimidin-2-yl)methyl]-N-[(3-chlorophenyl)methyl]pyrr olidine-3-carboxamide **Activity = A, B and C** |
| ***265** | 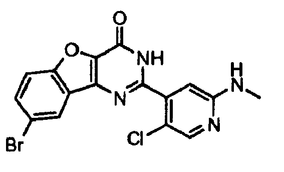 | 8-bromo-2-[5-chloro-2-(methylamino)pyridin-4-yl][1]benzofuro[3,2-d]pyrimidin-4(3H)-one **Activity = A, B and C** |
| **266** | 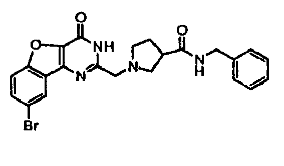 | 1-[(8-bromo-4-oxo-3,4-dihydro[1]benzofuro[3,2-d]pyrimidin-2-yl)methyl]-N-(phenylmethyl)pyrrolidine -3-carboxamide **Activity =** A, **B and C** |
| **267** | 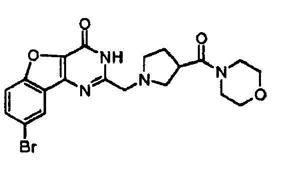 | 8-bromo-2-{[3-(morpholin-4-ylcarbonyl)pyrrolidin-1-yl]methyl}[1]benzofuro[3, 2-d]pyrimidin-4(3H)-one **Activity = B and C** |
| **268** | 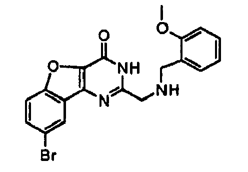 | 8-bromo-2-[({[2-(methyloxy)phenyl]methyl }amino)methyl][1]benzof uro[3,2-d]pyrimidin-4(3H)-one **Activity = B and C** |
| **269** | 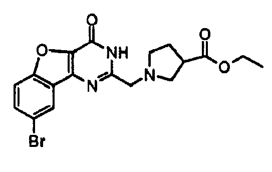 | ethyl 1-[(8-bromo-4-oxo-3,4-dihydro[1]benzofuro[3,2-d]pyrimidin-2-yl)methyl]pyrrolidine-3-carboxylate **Activity = A, B and C** |
| **270** | 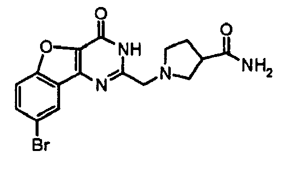 | 1-[(8-bromo-4-oxo-3,4-dihydro[1]benzofuro[3,2-d]pyrimidin-2-yl)methyl]pyrrolidine-3-carboxamide **Activity** = **A, B and C** |
| **271** | 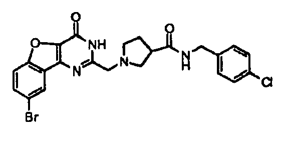 | 1-[(8-bromo-4-oxo-3,4-dihydro[1]benzofuro[3,2-d]pyrimidin-2-yl)methyl]-N-[(4-chlorophenyl)methyl]pyrr olidine-3-carboxamide **Activity = B and C** |
| **272** | 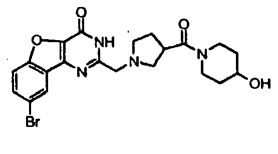 | 8-bromo-2-({3-[(4-hydroxypiperidin-1-yl)carbonyl]pyrrolidin-1-yl}methyl)[1]benzofuro[3, 2-d]pyrimidin-4(3H)-one **Activity = B and C** |
| **273** | 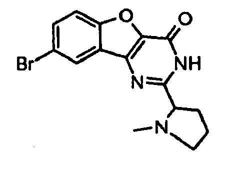 | 8-bromo-2-(1-methylpyrrolidin-2-yl)[1]benzofuro[3,2-d]pyrimidin-4(3H)-one **Activity = A, B and C** |
| ***274** | 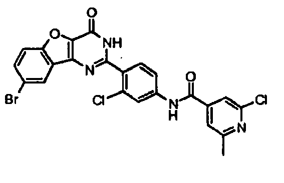 | N-[4-(8-bromo-4-oxo-3,4-dihydro[1]benzofuro[3,2-d]pyrimidin-2-yl)-3-chlorophenyl]-2-chloro-6-methylpyridine-4-carboxamide **Activity = B and C** |
| ***275** | 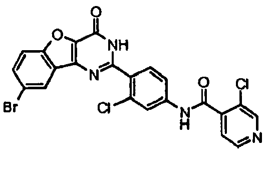 | N-[4-(8-bromo-4-oxo-3,4-dihydro[1]benzofuro[3,2-d]pyrimidin-2-yl)-3-chlorophenyl]-3-chloropyridine-4-carboxamide **Activity = A, B and C** |
| ***276** | 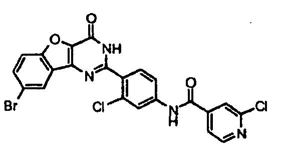 | N-[4-(8-bromo-4-oxo-3,4-dihydro[1]benzofuro[3,2-d]pyrimidin-2-yl)-3-chlorophenyl]-2-chloropyridine-4-carboxamide **Activity** = **A, B and C** |
| **277** | 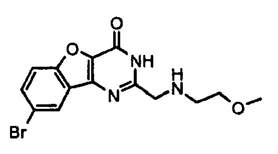 | 8-bromo-2-({[2-(methyloxy)ethyl]amino} methyl)[1]benzofuro[3,2-d]pyrimidin-4(3H)-one **Activity = A, B and C** |
| **278** | 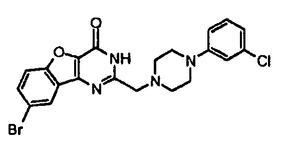 | 8-bromo-2-{[4-(3-chlorophenyl)piperazin-1-yl]methyl}[1]benzofuro[3, 2-d]pyrimidin-4(3H)-one **Activity = B** |
| ***279** | 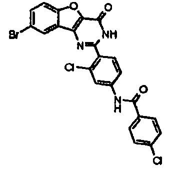 | N-[4-(8-bromo-4-oxo-3,4-dihydro[1]benzofuro[3,2-d]pyrimidin-2-yl)-3-chlorophenyl]-4-chlorobenzamide **Activity = B and C** |
| ***280** | 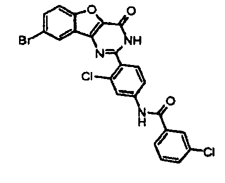 | N-[4-(8-bromo-4-oxo-3,4-dihydro[1]benzofuro[3,2-d]pyrimidin-2-yl)-3-chlorophenyl]-3-chlorobenzamide **Activity = B and C** |
| ***281** | 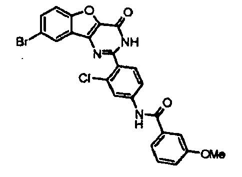 | N-[4-(8-bromo-4-oxo-3,4-dihydro[1]benzofuro[3,2-d]pyrimidin-2-yl)-3-chlorophenyl]-3-(methyloxy)benzamide **Activity = B and C** |
| ***282** | 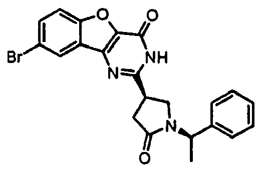 | 8-bromo-2-{(3R)-5-oxo-1-[(1R)-1-phenylethyl]pyrrolidin-3-yl}[1]benzofuro[3,2-d]pyrimidin-4(3H)-one **Activity = B and C** |
| ***283** | 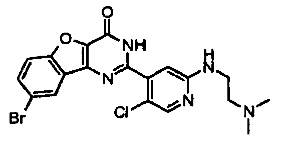 | 8-bromo-2-(5-chloro-2-{[2-(dimethylamino)ethyl]ami no}pyridin-4-yl)[1]benzofuro[3,2-d]pyrimidin-4(3H)-one **Activity = A, B and C** |
| ***284** | 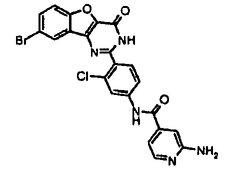 | 2-amino-N-[4-(8-bromo-4-oxo-3,4- dihydro[1]benzofuro[3,2-d]pyrimidin-2-yl)-3-chlorophenyl]pyridine-4-carboxamide **Activity = B and C** |
| ***285** | 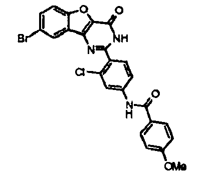 | N-[4-(8-bromo-4-oxo-3,4-dihydro[1]benzofuro[3,2-d]pyrimidin-2-yl)-3-chlorophenyl]-4-(methyloxy)benzamide **Activity = B and C** |
| **286** | 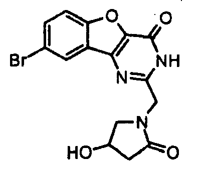 | 8-bromo-2-[(4-hydroxy-2-oxopyrrolidin-1-yl)methyl][1]benzofuro[3, 2-d]pyrimidin-4(3H)-one **Activity = B and C** |
| **287** | 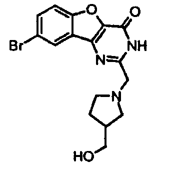 | 8-bromo-2-{[3-(hydroxymethyl)pyrrolidin -1-yl]methyl}[1]benzofuro[3, 2-d]pyrimidin-4(3H)-one **Activity = A, B and C** |
| **288** | 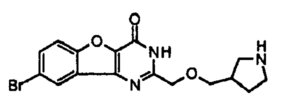 | 8-bromo-2-{[(pyrrolidin-3-ylmethyl)oxy]methyl} [1]b enzofuro[3,2-d]pyrimidin-4(3H)-one **Activity = B and C** |
| **289** | 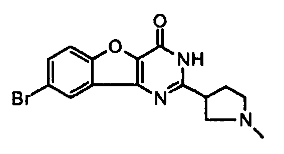 | 8-bromo-2-(1-methylpyrrolidin-3-yl)[1]benzofuro[3,2-d]pyrimidin-4(3H)-one **Activity = A, B and C** |
| **290** | 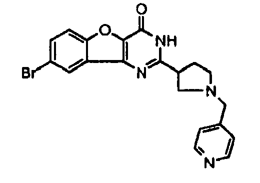 | 8-bromo-2-[1-(pyridin-4-ylmethyl)pyrrolidin-3-yl][1]benzofuro[3,2-d]pyrimidin-4(3H)-one **Activity = A, B and C** |
| ***291** | 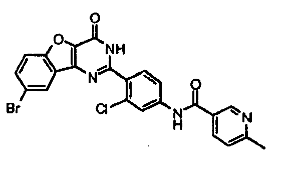 | N-[4-(8-bromo-4-oxo-3,4-dihydro[1]benzofuro[3,2-d]pyrimidin-2-yl)-3-chlorophenyl]-6-methylpyridine-3-carboxamide **Activity = A, B and C** |
| ***292** | 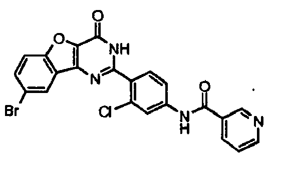 | N-[4-(8-bromo-4-oxo-3,4-dihydro[1]benzofuro[3,2- d]pyrimidin-2-yl)-3-chlorophenyl]pyridine-3-carboxamide **Activity = B and C** |
| ***293** | 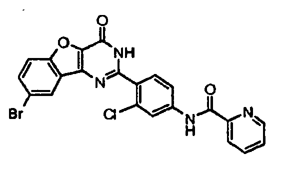 | N-[4-(8-bromo-4-oxo-3,4-dihydro[1]benzofuro[3,2-d]pyrimidin-2-yl)-3-chlorophenyl]pyridine-2-carboxamide **Activity = A, B and C** |
| ***294** | 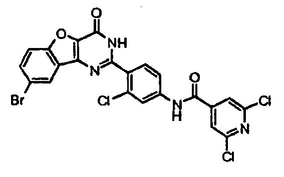 | N-[4-(8-bromo-4-oxo-3,4-dihydro[1]benzofuro[3,2-d]pyrimidin-2-yl)-3-chlorophenyl]-2,6-dichloropyridine-4-carboxamide **Activity = A, B and C** |
| **295** | 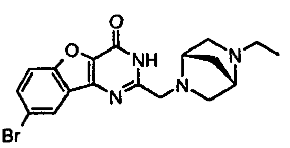 | 8-bromo-2-{[(1S,4S)-5-ethyl-2,5-diazabicyclo[2.2.1]hept-2-yl]methyl}[1]benzofuro[3, 2-d]pyrimidin-4(3H)-one **Activity = B and C** |
| ***296** | 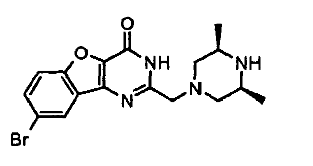 | 8-bromo-2-{[(3R,5S)-3,5-dimethylpiperazin-1-yl]methyl}[1]benzofuro[3, 2-d]pyrimidin-4(3H)-one **Activity = B** |
| ***297** | 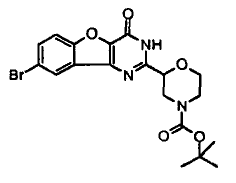 | 1,1-dimethylethyl 2-(8-bromo-4-oxo-3,4-dihydro[1]benzofuro[3,2-d]pyrimidin-2-yl)morpholine-4-carboxylate **Activity = B and C** |
| **298** | 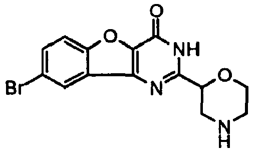 | 8-bromo-2-morpholin-2-yl[1]benzofuro[3,2-d]pyrimidin-4(3H)-one **Activity = A, B and C** |
| ***299** | 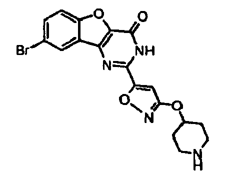 | 8-bromo-2-[3-(piperidin-4-yloxy)isoxazol-5-yl][1]benzofuro[3,2-d]pyrimidin-4(3H)-one **Activity = B and C** |
| ***300** | 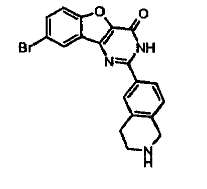 | 8-bromo-2-(1,2,3,4-tetrahydroisoquinolin-6-yl)[1]benzofuro[3,2-d]pyrimidin-4(3H)-one **Activity = B and C** |
| ***301** | 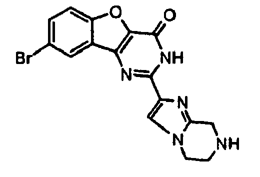 | 8-bromo-2-(5,6,7,8-tetrahydroimidazo[1,2-a]pyrazin-2-yl)[1]benzofuro[3,2-d]pyrimidin-4(3H)-one **Activity = B** |
| ***302** | 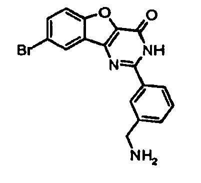 | 2-[3-(aminomethyl)phenyl]-8-bromo[1]benzofuro[3,2-d]pyrimidin-4(3H)-one **Activity = A, B and C** |
| ***303** | 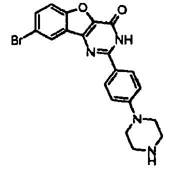 | 8-bromo-2-(4-piperazin-1-ylphenyl)[1]benzofuro[3,2 -d]pyrimidin-4(3H)-one **Activity = B and C** |
| **304** | 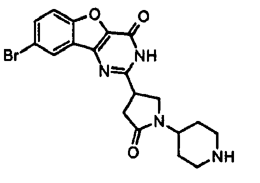 | 8-bromo-2-(5-oxo-1-piperidin-4-ylpyrrolidin-3-yl)[1]benzofuro[3,2- d]pyrimidin-4(3H)-one **Activity = B and C** |
| **305** | 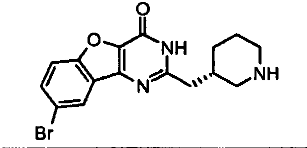 | 8-bromo-2-[(3S)-piperidin-3-ylmethyl][1]benzofuro[3,2 -d]pyrimidin-4(3H)-one **Activity = A, B and C** |
| **306** | 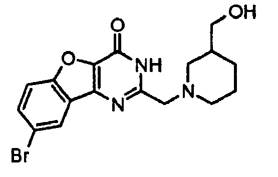 | 8-bromo-2-{[3-(hydroxymethyl)piperidin-1-yl]methyl} [1]benzofuro[3, 2-d]pyrimidin-4(3H)-one **Activity = B and C** |
| **307** | 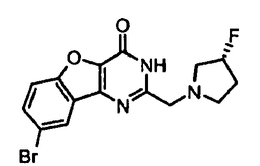 | 8-brumo-2-{[(3R)-3-fluoropyrrolidin-1-yl]methyl}[1]benzofuro[3, 2-d]pyrimidin-4(3H)-one **Activity = A, B and C** |
| **308** | 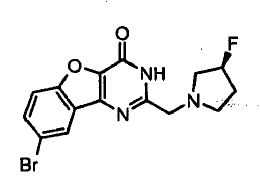 | 8-bromo-2-{[(3S)-3-fluoropyrrolidin-1-yl]methyl}[1]benzofuro[3, 2-d]pyrimidin-4(3H)-one **Activity = A, B and C** |
| **309** | 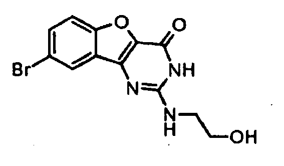 | 8-bromo-2-[(2-hydroxyethyl)amino][1]be nzofuro[3,2-d]pyrimidin-4(3H)-one **Activity = B and C** |
| ***310** | 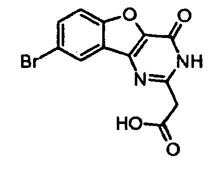 | (8-bromo-4-oxo-3,4-dihydro[1]benzofuro[3,2-d]pyrimidin-2-yl)acetic acid **Activity = B and C** |
| **311** | 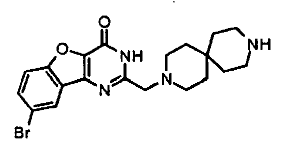 | 8-bromo-2-(3,9-diazaspiro[5.5]undec-3-ylmethyl)[1]benzofuro[3,2 -d]pyrimidin-4(3H)-one **Activity = A, B and C** |
| ***312** | 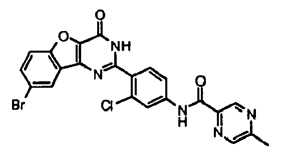 | N-[4-(8-bromo-4-oxo-3,4-dihydro[1]benzofuro[3,2-d]pyrimidin-2-yl)-3-chlorophenyl]-5-methylpyrazine-2-carboxamide **Activity = A, B and C** |
| ***313** | 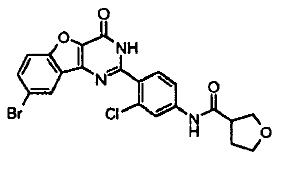 | N-[4-(8-bromo-4-oxo-3,4-dihydro[1]benzofuo[3,2-d]pyrimidin-2-yl)-3-chlorophenyl]tetrahydrofu ran-3-carboxamide **Activity = A, B and C** |
| ***314** | 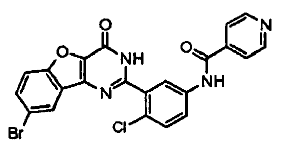 | N-[3-(8-bromo-4-oxo-3,4-dihydro[1]benzofuro[3,2-d]pyrimidin-2-yl)-4-chlorophenyl]pyridine-4-carboxamide **Activity = B and C** |
| **315** | 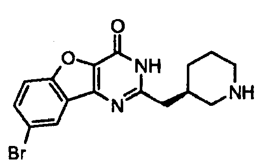 | 8-bromo-2-[(3R)-piperidin-3-ylmethyl][1]benzofuro[3,2 -d]pyrimidin-4(3H)-one **Activity = A, B and C** |
| ***316** | 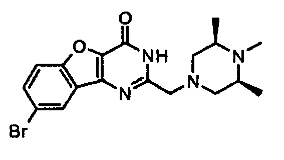 | 8-bromo-2-{[(3R,5S)-3,4,5-trimethylpiperazin-1-yl]methyl}[1]benzofuro[3, 2-d]pyrimidin-4(3H)-one **Activity = B** |
| **317** | 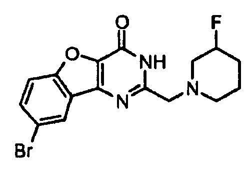 | 8-bromo-2-[(3-fluoropiperidin-1-yl)methyl][1]benzofuro[3, 2-d]pyrimidin-4(3H)-one **Activity = A, B and C** |
| **318** | 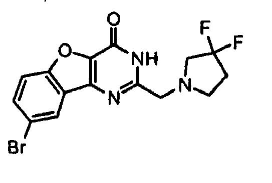 | 8-bromo-2-[(3,3-difluoropyrrolidin-1-yl)methyl][1]benzofuro[3, 2-d]pyrimidin-4(3H)-one **Activity = B and C** |
| ***319** | 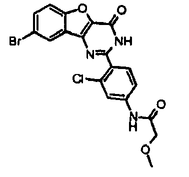 | N-[4-(8-bromo-4-oxo-3,4-dihydro[1]benzofuro[3,2-d]pyrimidin-2-yl)-3-chlorophenyl]-2-(methyloxy)acetamide **Activity = A, B and C** |
| ***320** | 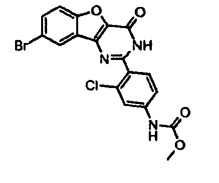 | methyl [4-(8-bromo-4-oxo-3,4-dihydro[1]benzofuro[3,2-d]pyrimidin-2-yl)-3-chlorophenyl]carbamate **Activity = A, B and C** |
| ***321** | 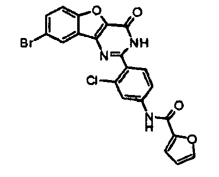 | N-[4-(8-bromo-4-oxo-3,4-dihydro[1]benzofuro[3,2-d]pyrimidin-2-yl)-3-chlorophenyl]furan-2-carboxamide **Activity = A, B and C** |
| ***322** | 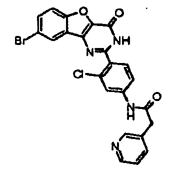 | N-[4-(8-bromo-4-oxo-3,4-dihydro[1]benzofuro[3,2-d]pyrimidin-2-yl)-3-chlorophenyl]-2-pyridin-3-ylacetamide **Activity = A, B and C** |
| ***323** | 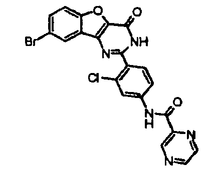 | N-[4-(8-bromo-4-oxo-3,4-dihydro[1]benzofuro[3,2-d]pyrimidin-2-yl)-3-chlorophenyl]pyrazine-2-carboxamide **Activity = A, B and C** |
| ***324** | 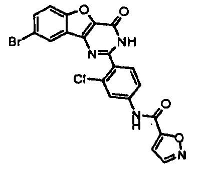 | N-[4-(8-bromo-4-oxo-3,4-dihydro[1]benzofuro[3,2-d]pyrimidin-2-yl)-3-chlorophenyl]isoxazole-5-carboxamide **Activity = A, B and C** |
| ***325** | 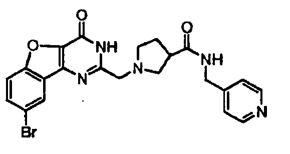 | 1-[(8-bromo-4-oxo-3,4-dihydro[1]benzofuro[3,2-d]pyrimidin-2-yl)methyl]-N-(pyridin-4-ylmethyl)pyrrolidine-3-carboxamide **Activity = B and C** |
| ***326** | 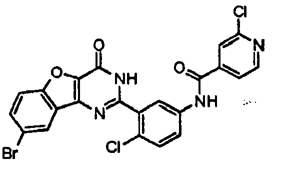 | N-[3-(8-bromo-4-oxo-3,4-dihydro[1]benzofuro[3,2-d]pyrimidin-2-yl)-4-chlorophenyl]-2-chloropyridine-4-carboxamide **Activity = B and C** |
| ***327** | 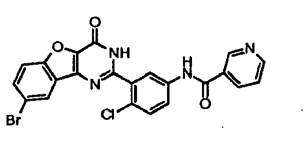 | N-[3-(8-bromo-4-oxo-3,4-dihydro[1]benzofuro[3,2-d]pyrimidin-2-yl)-4-chlorophenyl]pyridine-3-carboxamide **Activity = B** |
| **328** | 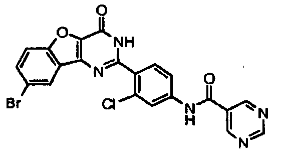 | N-[4-(8-bromo-4-oxo-3,4-dihydro[1]benzofuro[3,2-d]pyrimidin-2-yl)-3-chlorophenyl]pyrimidine-5-carboxamide **Activity = A, B and C** |
| **329** | 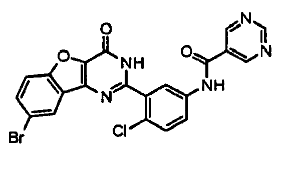 | N-[3-(8-bromo-4-oxo-3,4-dihydro[1]benzofuro[3,2-d]pyrimidin-2-yl)-4-chlorophenyl]pyrimidine-5-carboxamide **Activity = B and C** |
| **330** | 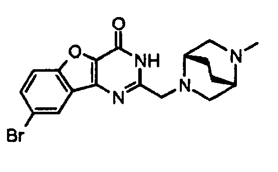 | 8-bromo-2-{[(1S,4S)-5-methyl-2,5-diazabicyclo[2.2.2]oct-2-yl]methyl}[1]benzofuro[3, 2-d]pyrimidin-4(3H)-one **Activity = A, B and C** |
| **331** | 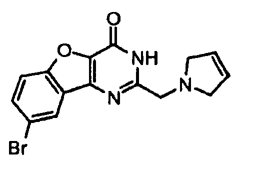 | 8-bromo-2-((2,5-dihydro-1H-pyrrol-1-yl)methyl)benzofuro[3,2-d]pyrimidin-4(3H)-one **Activity = A, B and C** |
| **332** | 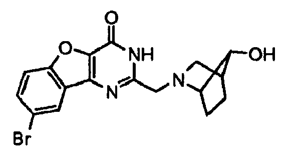 | 8-bromo-2-((7-hydroxy-2-azabicyclo[2.2.]heptan-2-yl)methyl)benzofuro[3,2-d]pyrimidin-4(3H)-one **Activity = A, B and C** |
| ***333** | 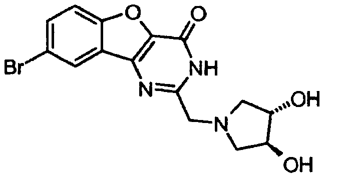 | 8-bromo-2-(((3S,4S)-3,4-dihydroxypyrrolidin-1-yl)methyl)benzofuro[3,2-d]pyrimidin-4(3H)-one **Activity = A, B and C** |
| ***334** | 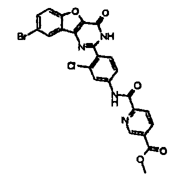 | methyl 6-({[4-(8-bromo-4-oxo-3,4-dihydro[1]benzofuro[3,2-d]pyrimidin-2-yl)-3-chlorophenyl]amino}carb onyl)pyridine-3-carboxylate **Activity = B and C** |
| ***335** | 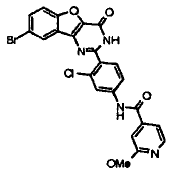 | N-[4-(8-bromo-4-oxo-3,4-dihydro[1]benzofuro[3,2-d]pyrimidin-2-yl)-3-chlorophenyl]-2-(methyloxy)pyridine-4-carboxamide **Activity = B and C** |
| ***336** | 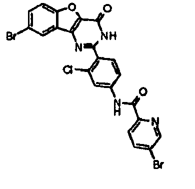 | 5-bromo-N-[4-(8-bromo-4-oxo-3,4-dihydro[1]benzofuro[3,2-d]pyrimidin-2-yl)-3-chlorophenyl]pyridine-2-carboxamide **Activity = B and C** |
| ***337** | 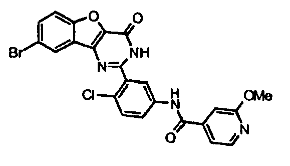 | N-[3-(8-bromo-4-oxo-3,4-dihydro[1]benzofuro[3,2-d]pyrimidin-2-yl)-4-chlorophenyl]-2-(methyloxy)pyridine-4-carboxamide **Activity = B and C** |
| ***338** | 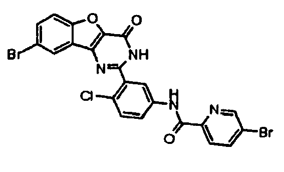 | 5-bromo-N-[3-(8-bromo-4-oxo-3,4-dihydro[1]benzofuro[3,2-d]pyrimidin-2-yl)-4-chlorophenyl]pyridine-2-carboxamide **Activity = B** |
| ***339** | 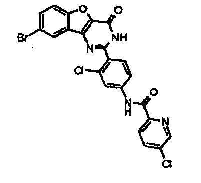 | N-[4-(8-bromo-4-oxo-3,4-dihydro[1]benzofuro[3,2-d]pyrimidin-2-yl)-3-chlorophenyl]-5-chloropyridine-2-carboxamide **Activity = B and C** |
| ***340** | 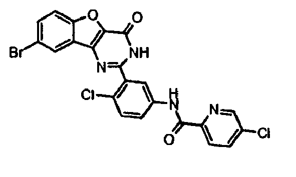 | N-[3-(8-bromo-4-oxo-3,4-dihydro[1]benzofuro[3,2-d]pyrimidin-2-yl)-4-chlorophenyl]-5-chloropyridine-2-carboxamide **Activity = B and C** |
| ***341** | 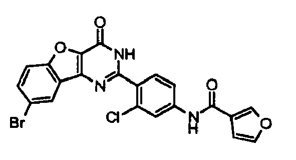 | N-[4-(8-bromo-4-oxo-3,4-dihydro[1]benzofuro[3,2-d]pyrimidin-2-yl)-3-chlorophenyl]furan-3-carboxamide **Activity = B and C** |
| ***342** | 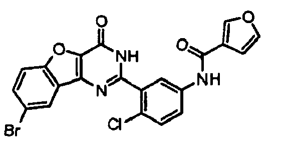 | N-[3-(8-bromo-4-oxo-3,4-dihydro[1]benzofuro[3,2-d]pyrimidin-2-yl)-4-chlorophenyl]furan-3-carboxamide **Activity = B and C** |
| ***343** | 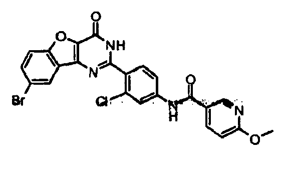 | N-[4-(8-bromo-4-oxo-3,4-dihydro[1]benzofuro[3,2-d]pyrimidin-2-yl)-3-chlorophenyl]-6-(methyloxy)pyridine-3-carboxamide **Activity = A, B and C** |
| **344** | 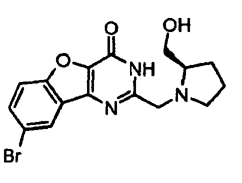 | 8-bromo-2-{[(2R)-2-(hydroxymethyl)pyrrolidin -1-yl]methyl}[1]benzofuro[3, 2-d]pyrimidin-4(3H)-one **Activity = A, B and C** |
| ***345** | 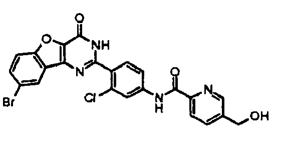 | N-[4-(8-bromo-4-oxo-3,4-dihydro[1]benzofuro[3,2-d]pyrimidin-2-yl)-3-chlorophenyl]-5-(hydroxymethyl)pyridine-2-carboxamide **Activity = B and C** |
| **346** | 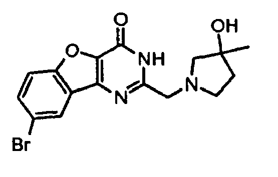 | 8-bromo-2-[(3-hydroxy-3-methylpyrrolidin-1-yl)methyl][1]benzofuro[3, 2-d]pyrimidin-4(3H)-one **Activity = A, B and C** |
| ***347** | 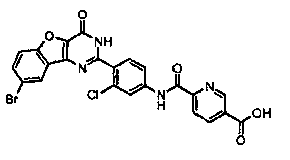 | 6-({[4-(8-bromo-4-oxo-3,4-dihydro[1]benzofuro[3,2-d]pyrimidin-2-yl)-3-chlorophenyl]amino} carb onyl)pyridine-3-carboxylic acid **Activity = A, B and C** |
| ***348** | 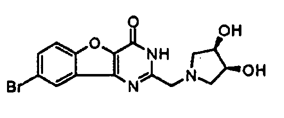 | 8-bromo-2-{[(3R,4S)-3,4-dihydroxypyrrolidin-1-yl]methyl} [1]benzofuro[3, 2-d]pyrimidin-4(3H)-one **Activity = A, B and C** |
| ***349** | 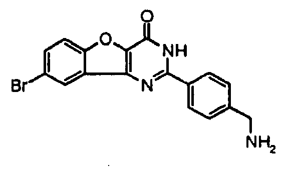 | 2-[4-(aminomethyl)phenyl]-8-bromo[1]benzofuro[3,2-d]pyrimidin-4(3H)-one **Activity = A, B and C** |
| ***350** | 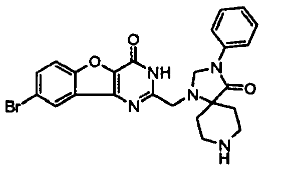 | 8-bromo-2-[(4-oxo-3-phenyl-1,3,8-triazaspiro[4.5]dec-1-yl)methyl][1]benzofuro[3, 2-d]pyrimidin-4(3H)-one **Activity = B and C** |
| ***351** | 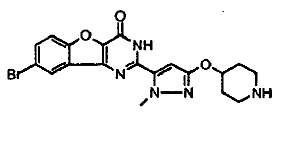 | 8-bromo-2-[1-methyl-3-(piperidin-4-yloxy)-1H-pyrazol-5-yl][1]benzofuro[3,2-d]pyrimidin-4(3H)-one **Activity = B and C** |
| ***352** | 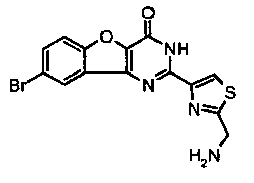 | 2-[2-(aminomethyl)-1,3-thiazol-4-yl]-8-bromo[1]benzofuro[3,2-d]pyrimidin-4(3H)-one **Activity = B and C** |
| ***353** | 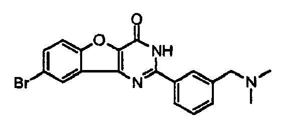 | 8-bromo-2-{3-[(dimethylamino)methyl]p henyl} [1]benzofuro[3,2-d]pyrimidin-4(3H)-one **Activity = A, B and C** |
| ***354** | 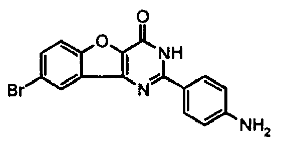 | 2-(4-aminophenyl)-8-bromo[1]benzofuro[3,2-d]pyrimidin-4(3H)-one **Activity = B and C** |
| ***355** | 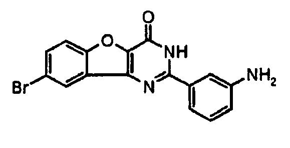 | 2-(3-aminophenyl)-8-bromo[1]benzofuro[3,2-d]pyrimidin-4(3H)-one **Activity = B and C** |
| ***356** | 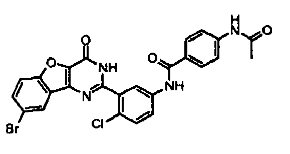 | 4-(acetylamino)-N-[3-(8-bromo-4-oxo-3,4-dihydro[1]benzofuro[3,2-d]pyrimidin-2-yl)-4-chlorophenyl]benzamide **Activity = B and C** |
| ***357** | 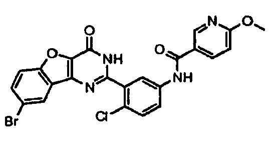 | N-[3-(8-bromo-4-oxo-3,4-dihydro[1]benzofuro[3,2-d]pyrimidin-2-yl)-4- chlorophenyl]-6(methyloxy)pyridine-3-carboxamide **Activity = B** |
| ***358** | 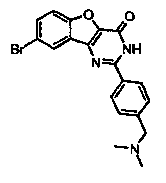 | 8-bromo-2-{4-[(dimethylamino)methyl]p henyl}[1]benzofuro[3,2-d]pyrimidin-4(3H)-one **Activity = B and C** |
| ***359** | 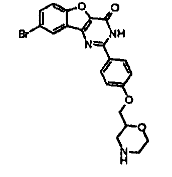 | 8-bromo-2-{4-[(morpholin-2-ylmethyl)oxy]phenyl} [1]b enzofuro[3,2-d]pyrimidin-4(3H)-one **Activity = B and C** |
| ***360** | 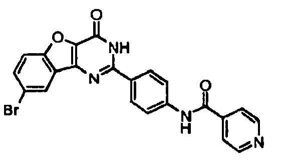 | N-[4-(8-bromo-4-oxo-3,4-dihydro[1]benzofuro[3,2-d]pyrimidin-2-yl)phenyl]pyridine-4-carboxamide **Activity = B and C** |
| ***361** | 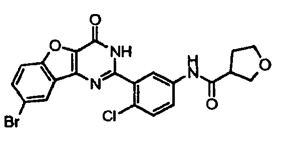 | N-[3-(8-bromo-4-oxo-3,4-dihydro[1]benzofuro[3,2-d]pyrimidin-2-yl)-4- chlorophenyl]tetrahydrofu ran-3-carboxamide **Activity = B and C** |
| ***362** | 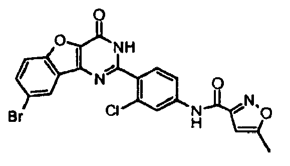 | N-[4-(8-bromo-4-oxo-3,4-dihydro[1]benzofuro[3,2-d]pyrimidin-2-yl)-3-chlorophenyl]-5-methylisoxazole-3-carboxamide **Activity = A, B and C** |
| ***363** | 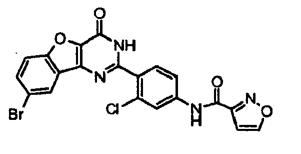 | N-[4-(8-bromo-4-oxo-3,4-dihydro[1]benzofuro[3,2-d]pyrimidin-2-yl)-3-chlorophenyl]isoxazole-3-carboxamide **Activity = A, B and C** |
| ***364** | 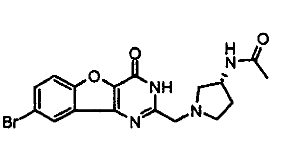 | N- {(3R)-1-[(8-bromo-4-oxo-3,4-dihydro[1]benzofuro[3,2-d]pyrimidin-2-yl)methyl]pyrrolidin-3-yl}acetamide **Activity = A, B and C** |
| ***365** | 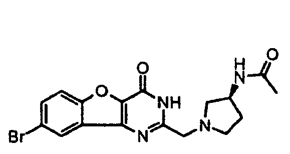 | N-{(3S)-1-[(8-bromo-4-oxo-3,4-dihydro[1]benzofuro[3,2-d]pyrimidin-2-yl)methyl]pyrrolidin-3-yl}acetamide **Activity = B and C** |
| **366** | 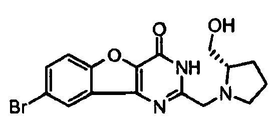 | 8-bromo-2-{[(2S)-2-(hydroxymethyl)pyrrolidin -1-yl]methyl}[1]benzofuro[3, 2-d]pyrimidin-4(3H)-one **Activity = A, B and C** |
| **367** | 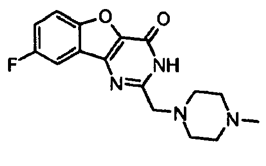 | 8-fluoro-2-[(4-methylpiperazin-1-yl)methyl] [1]benzofuro[3, 2-d]pyrimidin-4(3H)-one **Activity = B** |
| **368** | 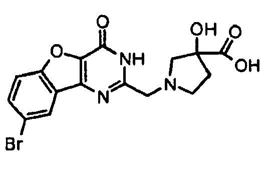 | 1-[(8-bromo-4-oxo-3,4-dihydro[1]benzofuro[3,2-d]pyrimidin-2-yl)methyl]-3-hydroxypyrrolidine-3-carboxylic acid **Activity = A, B and C** |
| **369** | 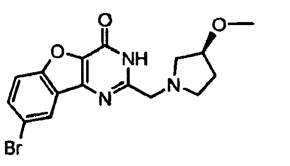 | 8-bromo-2-{[(3S)-3-(methyloxy)pyrrolidin-1-yl]methyl}[1]benzofuro[3, 2-d]pyrimidin-4(3H)-one **Activity = A, B and C** |
| **370** | 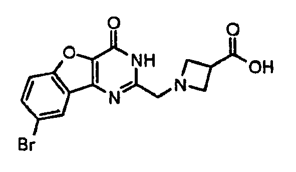 | 1-[(8-bromo-4-oxo-3,4-dihydro[1]benzofuro[3,2-d]pyrimidin-2-yl)methyl]azetidine-3-carboxylic acid **Activity = A, B and C** |
| ***371** | 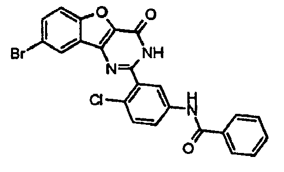 | N-[3-(8-bromo-4-oxo-3,4-dihydro[1]benzofuro[3,2-d]pyrimidin-2-yl)-4-chlorophenyl]benzamide **Activity = B and C** |
| ***372** | 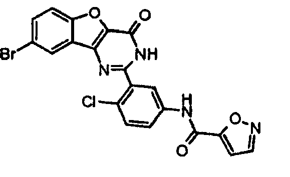 | N-[3-(8-bromo-4-oxo-3,4-dihydro[1]benzofuro[3,2-d]pyrimidin-2-yl)-4-chlorophenyl]isoxazole-5-carboxamide **Activity = B and C** |
| ***373** | 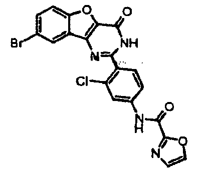 | N-[4-(8-bromo-4-oxo-3,4-dihydro[1]benzofuro[3,2-d]pyrimidin-2-yl)-3-chlorophenyl]-1,3-oxazole-2-carboxamide **Activity = A, B and C** |
| ***374** | 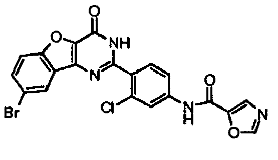 | N-[4-(8-bromo-4-oxo-3,4-dihydro[1]benzofuro[3,2-d]pyrimidin-2-yl)-3-chlorophenyl]-1,3-oxazole-5-carboxamide **Activity = A, B and C** |
| ***375** | 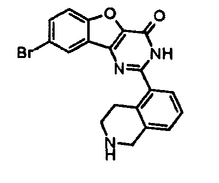 | 8-bromo-2-(1,2,3,4-tetrahydroisoquinolin-5-yl)[1]benzofuro[3,2-d]pyrimidin-4(3H)-one **Activity = A, B and C** |
| ***376** | 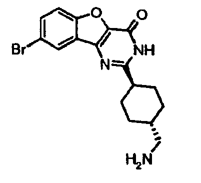 | 2-[trans-4-(aminomethyl)cyclohexyl] -8-bromo[1]benzofuro[3,2-d]pyrimidin-4(3H)-one **Activity = B and C** |
| ***377** | 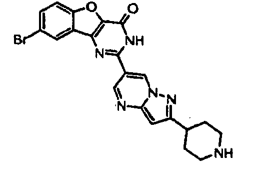 | 8-bromo-2-(2-piperidin-4-ylpyrazolo[1,5-a]pyrimidin-6-yl)[1]benzofuro[3,2-d]pyrimidin-4(3H)-one **Activity = A, B and C** |
| **378** | 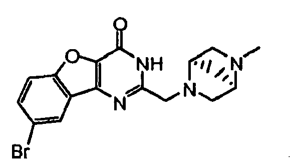 | 8-bromo-2-(((1R,4R)-5-methyl-2,5-diazabicyclo[2.2.1]heptan-2-yl)methyl)benzofuro[3,2-d]pyrimidin-4(3H)-one **Activity = B and C** |
| ***379** | 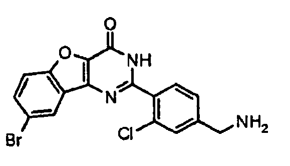 | 2-[4-(aminomethyl)-2-chlorophenyl]-8-bromo[1]benzofuro[3,2-d]pyrimidin-4(3H)-one **Activity = A, B and C** |
| ***380** | 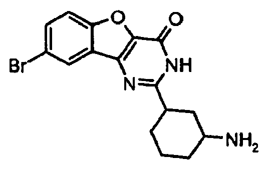 | 2-(3-aminocyclohexyl)-8-bromo[1]benzofuro[3,2-d]pyrimidin-4(3H)-one **Activity = A, B and C** |
| ***381** | 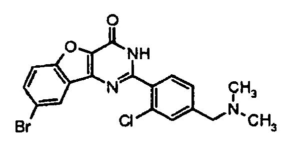 | 8-bromo-2-{2-chloro-4-[(dimethylamino)methyl]p henyl}[1]benzofuro[3,2-d]pyrimidin-4(3H)-one **Activity = A, B and C** |
| ***382** | 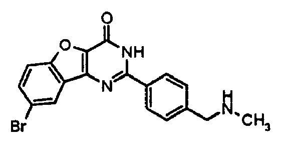 | 8-bromo-2-{4-[(methylamino)methyl]ph enyl}[1]benzofuro[3,2-d]pyrimidin-4(3H)-one **Activity = B and C** |
| ***383** | 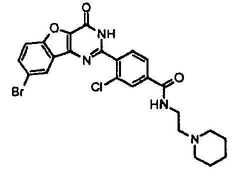 | 4-(8-bromo-4-oxo-3,4- dihydro[1]benzofuro [3,2-d]pyrimidin-2-yl)-3-chloro-N-(2-piperidin-1-ylethyl)benzamide **Activity = A, B and C** |
| ***384** | 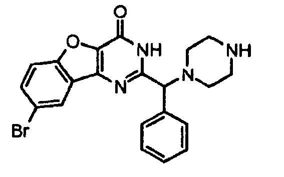 | 8-bromo-2-[phenyl(piperazin-1-yl)methyl][1]benzofuro[3, 2-d]pyrimidin-4(3H)-one **Activity = B** |
| ***385** | 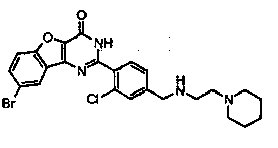 | 8-bromo-2-(2-chloro-4-{[(2-piperidin-1-ylethyl)amino]methyl} phe nyl)[1]benzofuro[3,2-d]pyrimidin-4(3H)-one **Activity = A, B and C** |
| ***386** | 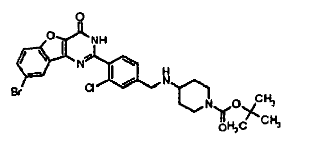 | 1,1 -dimethylethyl 4-({[4-(8-bromo-4-oxo-3,4-dihydro[1]benzofuro[3,2-d]pyrimidin-2-yl)-3-chlorophenyl]methyl} ami no)piperidine-1-carboxylate **Activity = A, B and C** |
| ***387** | 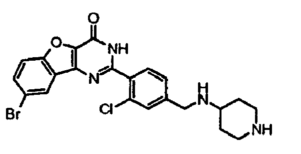 | 8-bromo-2-{2-chloro-4-[(piperidin-4-ylamino)methyl]phenyl}[1 ]benzofuro[3,2-d]pyrimidin-4(3H)-one **Activity = A, B and C** |
| **388** | 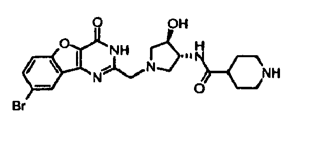 | N-{(3R,4R)-1-[(8-bromo-4-oxo-3,4-dihydro[1]benzofuro[3,2-d]pyrimidin-2-yl)methyl]-4-hydroxypyrrolidin-3-yl}piperidine-4-carboxamide **Activity = A, B and C** |
| ***389** | 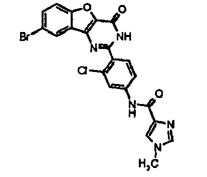 | N-[4-(8-bromo-4-oxo-3,4-dihydro[1]benzofuro[3,2-d]pyrimidin-2-yl)-3-chlorophenyl]-1-methyl-1H-imidazole-4-carboxamide **Activity = A, B and C** |
| ***390** | 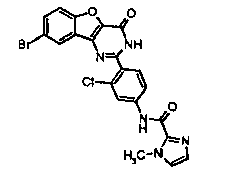 | N-[4-(8-bromo-4-oxo-3,4-dihydro[1]benzofuro[3,2-d]pyrimidin-2-yl)-3-chlorophenyl]-1-methyl1H-imidazole-2-carboxamide **Activity = A, B and C** |
| ***391** | 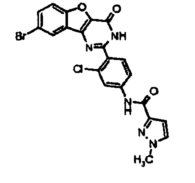 | N-[4-(8-bromo-4-oxo-3,4-dihydro[1]benzofuro[3,2-d]pyrimidin-2-yl)-3-chlorophenyl]-1-methyl-1H-pyrazole-3-carboxamide **Activity = A, B and C** |
| ***392** | 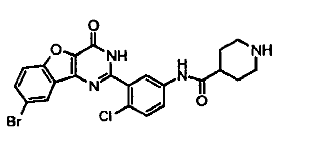 | N-[3-(8-bromo-4-oxo-3,4-dihydro[1]benzofuro[3,2-d]pyrimidin-2-yl)-4-chlorophenyl]piperidine-4-carboxamide **Activity = B** |
| ***393** | 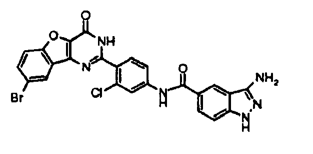 | 3-amino-N-[4-(8-bromo-4-oxo-3,4-dihydro[1]benzofuro[3,2- d]pyrimidin-2-yl)-3chlorophenyl]-1H-indazole-5-carboxamide **Activity = A, B and C** |
| ***394** | 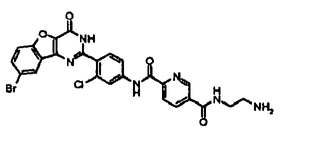 | N⁵-(2-aminoethyl)-N²-[4-(8-bromo-4-oxo-3,4-dihydro[I]benzofuro[3,2-d]pyrimidin-2-yl)-3-chlorophenyl]pyridine-2,5-dicarboxamide **Activity = A, B and C** |
| ***395** | 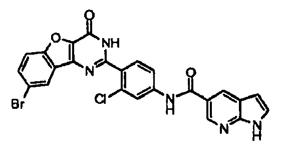 | N-[4-(8-bromo-4-oxo-3,4-dihydro[1]benzofuro[3,2-d]pyrimidin-2-yl)-3-chlorophenyl]-1H-pyrrolo[2,3-b]pyridine-5-carboxamide **Activity = A, B and C** |
| ***396** | 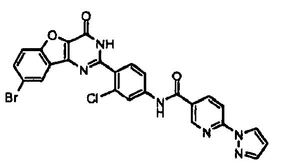 | N-[4-(8-bromo-4-oxo-3,4-dihydro[1]benzofuru[3,2-d]pyrimidin-2-yl)-3-chlorophenyl]-6-(1H-pyrazol-1-yl)pyridine-3-carboxamide **Activity = B and C** |
| ***397** | 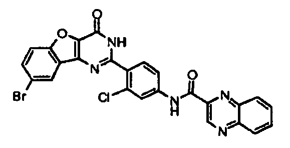 | N-[4-(8-bromo-4-oxo-3,4-dihydro[1]benzofuro[3,2-d]pyrimidin-2-yl)-3-chlorophenyl]quinoxaline-2-carboxamide **Activity = B and C** |
| **398** | 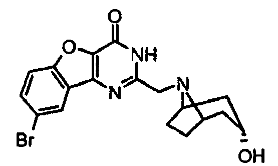 | 8-bromo-2-[(3-hydroxy-8-azabicyclo[3.2.1]oct-8-yl)methyl][1]benzofuro[3, 2-d]pyrimidin-4(3H)-one **Activity = A, B and C** |
| ***399** | 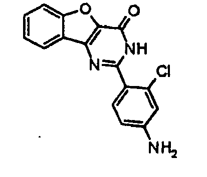 | 2-(4-amino-2-chlorophenyl)[1]benzofur o[3,2-d]pyrimidin-4(3H)-one **Activity = B** |
| **400** | 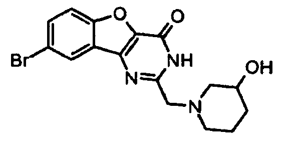 | 8-bromo-2-[(3-hydroxypiperidin-1-yl)methyl][1]benzofuro[3, 2-d]pyrimidin-4(3H)-one **Activity = A, B and C** |
| **401** | 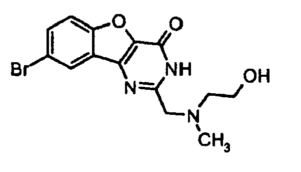 | 8-bromo-2-{[(2-hydroxyethyl)(methyl)ami no]methyl}[1]benzofuro[3 ,2-d]pyrimidin-4(3H)-one **Activity = A, B and C** |
| **402** | 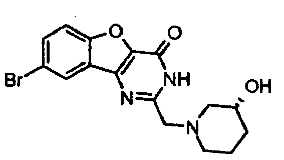 | 8-bromo-2-{[(3R)-3-hydroxypiperidin-1-yl]methyl}[1]benzofuro[3, 2-d]pyrimidin-4(3H)-one **Activity = B and C** |
| ***403** | 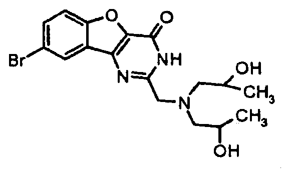 | 2-{[bis(2-hydroxypropyl)amino]met hyl}-8-bromo[1]benzofuro[3,2-d]pyrimidin-4(3H)-one **Activity = B and C** |
| **404** | 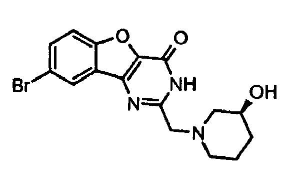 | 8-bromo-2-{[(3S)-3-hydroxypiperidin-1-yl]methyl}[1]benzofuro[3, 2-d]pyrimidin-4(3H)-one **Activity = A, B and C** |
| **405** | 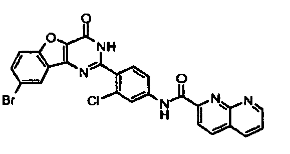 | N-[4-(8-bromo-4-oxo-3,4-dihydro[1]benzofuro[3,2-d]pyrimidin-2-yl)-3-chlorophenyl]-1,8-naphthyridine-2-carboxamide **Activity = B and C** |
| ***406** | 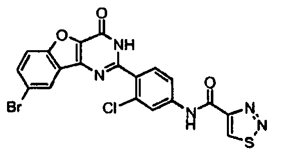 | N-[4-(8-bromo-4-oxo-3,4-dihydro[1]benzofuro[3,2-d]pyrimidin-2-yl)-3-chlorophenyl]-1,2,3-thiadiazole-4-carboxamide **Activity = A, B and C** |
| **407** | 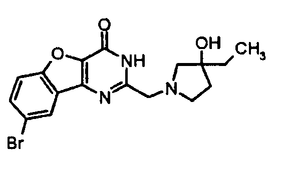 | 8-bromo-2-[(3-ethyl-3-hydroxypyrrolidin-1-yl)methyl][1]benzofuro[3, 2-d]pyrimidin-4(3H)-one **Activity = A, B and C** |
| **408** | 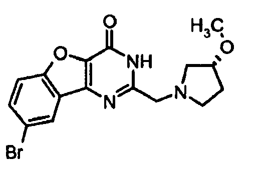 | 8-bromo-2-{[(3R)-3-(methyloxy)pyrrolidin-1-yl]methyl}[1]benzofuro[3, 2-d]pyrimidin-4(3H)-one **Activity = A, B and C** |
| **409** | 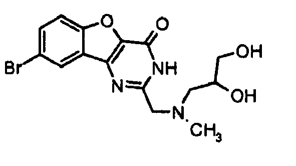 | 8-bromo-2-{[(2,3-dihydroxypropyl)(methyl) amino]methyl}[1]benzofu ro[3,2-d]pyrimidin-4(3H)-one **Activity = B and C** |
| **410** | 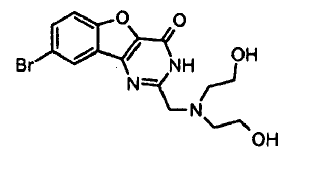 | 2-{[bis(2-hydroxyethyl)amino]meth yl}-8-bromo[1]benzofuro[3,2-d]pyrimidin-4(3H)-one **Activity = B and C** |
| ***411** | 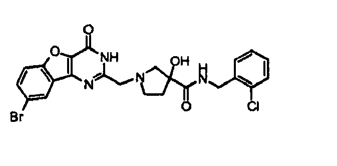 | 1-[(8-bromo-4-oxo-3,4-dihydro[1]benzofuro[3,2-d]pyrimidin-2-yl)methyl]-N-[(2-chlorophenyl)methyl]-3-hydroxypyrrolidine-3-carboxamide **Activity = B and C** |
| **412** | 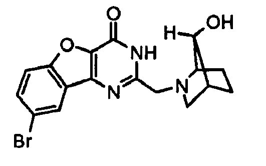 | 8-bromo-2-[(7-hydroxy-2-azabicyclo[2.2.]hept-2-yl)methyl] [1]benzofuro[3, 2-d]pyrimidin-4(3H)-one **Activity = A, B and C** |
| **413** | 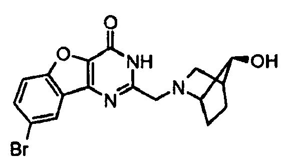 | 8-bromo-2-{[(7S)-7-hydroxy-2-azabicyclo[2.2.1]hept-2-yl]methyl}[1]benzofuro[3, 2-d]pyrimidin-4(3H)-one **Activity = A, B and C** |
| ***415** | 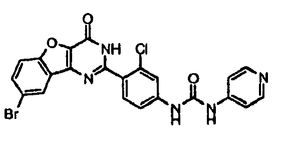 | 1-[4-(8-bromo-4-oxo-3,4-dihydro[1]benzofuro[3,2-d]pyrimidin-2-yl)-3-chlorophenyl]-3-pyridin-4-ylurea **Activity = B and C** |
| **417** | 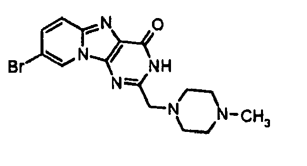 | 8-bromo-2-[(4-methylpiperazin-1-yl) methyl]pyrido[1,2-e]purin-4(3H)-one **Activity = B** |
| **418** | 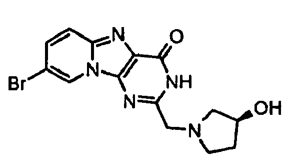 | 8-bromo-2-{[(3S)-3-hydroxypyrrolidin-1-yl]methyl}pyrido[ 1,2-e]purin-4(3H)-one **Activity = B** |
| **420** | 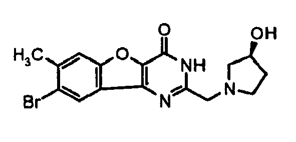 | 8-bromo-2-{[(3S)3-hydroxypyrrolidin-1-yl]methyl}-7- methyl[1]benzofuro[3,2-d]pyrimidin-4(3H)-one **Activity = A, B and C** |
| ***421** | 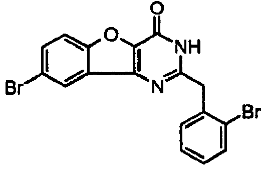 | 8-bromo-2-[(2-bromophenyl)methyl][1]b enzofuro[3,2-d]pyrimidin-4(3H)-one **Activity = A and B** |
| ***422** | 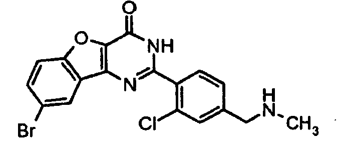 | 8-bromo-2-{2-chloro-4-[(methylamino)methyl]ph enyl}[1]benzofuro[3,2-d]pyrimidin-4(3H)-one **Activity = A, B and C** |
| ***423** | 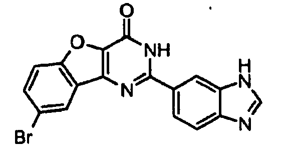 | 2--benzimidazol-6-yl)-8-bromo[1]benzofuro[3,2-d]pyrimidin-4(3H)-one **Activity = B** |
| **424** | 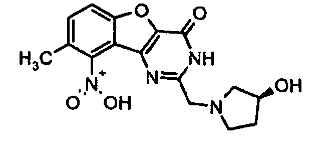 | hydroxy(2-{[(3S)-3- hydroxypyrrolidin-1-yl]methyl}-8-methyl-4-oxo-3,4- dihydro[1]benzofuro[3,2- d]pyrimidin-9-yl)oxoammonium **Activity = B and C** |
| **425** | 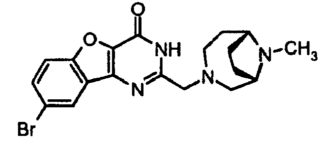 | 8-bromo-2-{[(1S,6R)-9-methyl-3,9-diazabicyclo[4.2.1]non-3-yl]methyl}[1]benzofuro[3, 2-d]pyrimidin-4(3H)-one **Activity = A, B and C** |
| **426** | 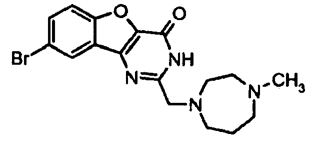 | 8-bromo-2-[(4-methyl-1,4-diazepan-1-yl)methyl][1]benzofuro[3, 2-d]pyrimidin-4(3H)-one **Activity = A, B and C** |
| ***427** | 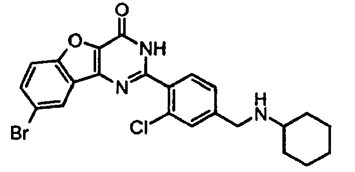 | 8-bromo-2-{2-chloro-4-[(cyclohexylamino)methyl ]phenyl}[1]benzofuro[3,2-d]pyrimidin-4(3H)-one **Activity = A, B and C** |
| **428** | 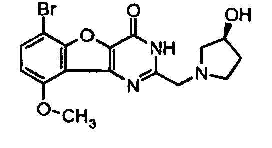 | 6-bromo-2-{[(3S)-3-hydroxypyrrolidin-1-yl]methyl}-9-(methyloxy)[1]benzofuro[ 3,2-d]pyrimidin-4(3H)-one **Activity = A,B and C** |
| **429** | 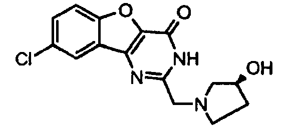 | 8-chloro-2-{[(3S)-3-hydroxypyrrolidin-1-yl]methyl}[1]benzofuro[3, 2-d]pyrimidin-4(3H)-one **Activity = A, B and C** |
| ***430** | 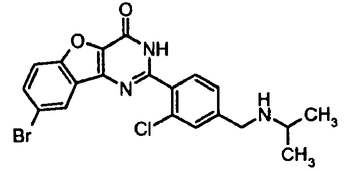 | 8-bromo-2-(2-chloro-4-{[(1- methylethyl)amino]methyl }phenyl)[1]benzofuro[3,2-d]pyrimidin-4(3H)-one **Activity = A, B and C** |
| **431** | 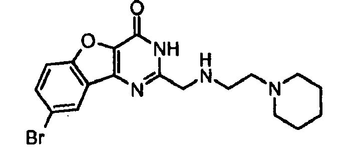 | 8-bromo-2-{[(2-piperidin-1-ylethyl)amino]methyl}[1] benzofuro[3,2-d]pyrimidin-4(3H)-one **Activity = A and B** |
| **432** | 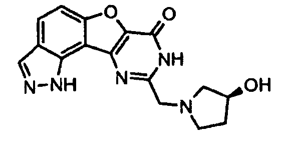 | 9-{[(3S)-3-hydroxypyrrolidin-1-yl]methyl}-1H-pyrimido[4',5':4,5]furo[2,3 -g]indazol-7(8H)-one **Activity** = **A, B and C** |
| **433** | 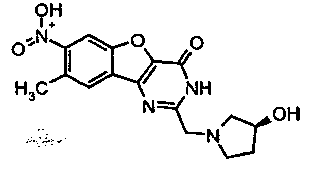 | hydroxy(2-{[(3S)-3-hydroxypyrrolidin-1-yl]methyl}-8-methyl-4-oxo-3,4-dihydro[1]benzofuro[3,2-d]pyrimidin-7-yl)oxoammonium **Activity = A, B and C** |
| **434** | 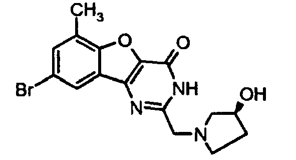 | 8-bromo-2-{[(3S)-3-hydroxypyrrolidin-1-yl]methyl}-6-methyl[1]benzofuro[3,2-d]pyrimidin-4(3H)-one **Activity = A, B and C** |
| ***435** | 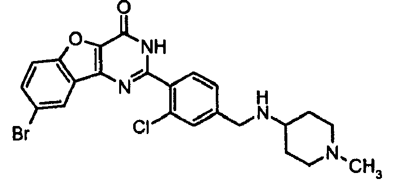 | 8-bromo-2-(2-chloro-4-{[(1-methylpiperidin-4-yl)amino]methyl}phenyl)[ 1]benzofuro[3,2-d]pyrimidin-4(3H)-one **Activity = A, B and C** |
| **436** | 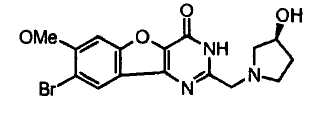 | 8-bromo-2-{[(3S)-3-hydroxypyrrolidin-1-yl]methyl}-7-(methyloxy)[1]benzofuro[ 3,2-d]pyrimidin-4(3H)-one **Activity = B and C** |
| **437** | 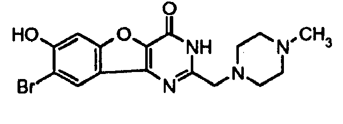 | 8-bromo-7-hydroxy-2-{[(3S)-3-hydroxypyrrolidin-1-yl]methyl}[1]benzofuro[3, 2-d]pyrimidin-4(3H)-one **Activity** = **A, B and C** |
| **438** | 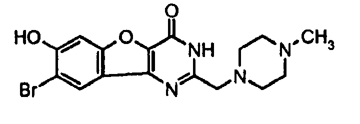 | 8-bromo-7-hydroxy-2-[(4-methylpiperazin-1-yl)methyl][1]benzofuro[3, 2-d]pyrimidin-4(3H)-one **Activity = A, B and C** |
| ***439** | 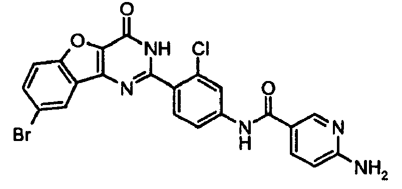 | 6-amino-N-[4-(8-bromo-4-oxo-3,4-dihydro[1]benzofuro[3,2-d]pyrimidin-2-yl)-3-chlorophenyl]pyridine-3-carboxamide **Activity = A, B and C** |
| **440** | 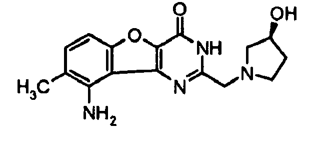 | 9-amino-2-{[(3S)-3-hydroxypyrrolidin-1-yl]methyl}-8-methyl[1]benzofuro[3,2-d]pyrimidin-4(3H)-one **Activity = A, B and C** |
| ***441** | 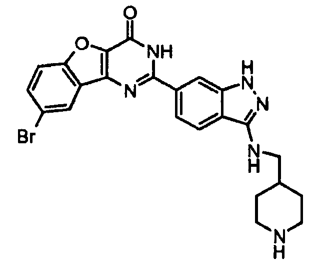 | 8-bromo-2-{3-[(piperidin--4-ylmethyl)amino]-1H-indazol-6-yl}[1]benzofuro[3,2-d]pyrimidin-4(3H)-one **Activity** = **B and C** |
| **442** | 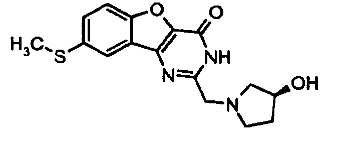 | 2-{[(3S)-3-hydroxypyrrolidin-1-yl]methyl}-8-(methylthio)[1]benzofuro[ 3,2-d]pyrimidin-4(3H)-one **Activity = A, B and C** |
| **443** | 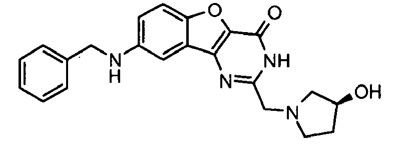 | 2-{[(3S)-3-hydroxypyrrolidin-1-yl]methyl}-8-[(phenylmethyl)amino] [1] benzofuro[3,2-d]pyrimidin-4(3H)-one **Activity = B and C** |
| **444** | 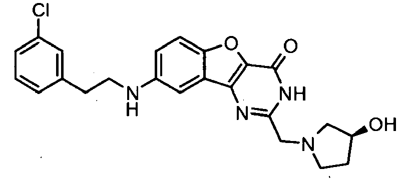 | 8-{[2-(3-chlorophenyl)ethyl]amino }-2-{[(3S)-3-hydroxypyrrolidin-1-yl]methyl}[1]benzofuro[3, 2-d]pyrimidin-4(3H)-one **Activity = A, B and C** |
| **445** | 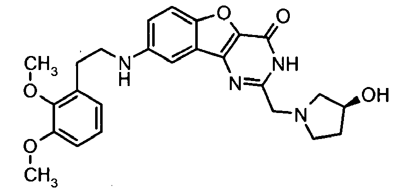 | 8-({2-[2,3-bis(methyloxy)phenyl]eth yl}amino)-2-{[(3S)-3- hydroxypyrrolidin-1-yl]methyl}[1]benzofuro[3, 2-d]pyrimidin-4(3H)-one **Activity = B and C** |
| **446** | 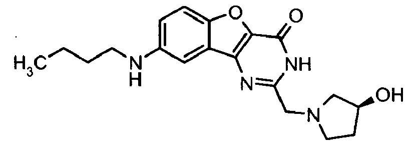 | 8-(butylamino)-2-{[(3S)-3-hydroxypyrrolidin-1-yl]methyl}[1]benzofuro[3, 2-d]pyrimidin-4(3H)-one **Activity = B and C** |
| **447** | 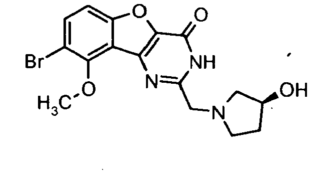 | 8-bromo-2-{[(3S)-3-hydroxypyrrolidin-1-yl]methyl}-9-(methyloxy)[1]benzofuro[3,2-d]pyrimidin-4(3H)-one **Activity = A, B and C** |
| **448** | 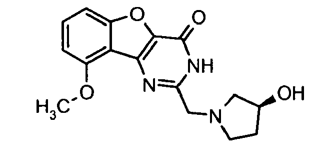 | 2-{[(3S)-3-hydroxypyrrolidin-1-yl]methyl}-9-(methyloxy)[1]benzofuro[ 3,2-d]pyrimidin-4(3H)-one **Activity = A, B and C** |
| **449** | 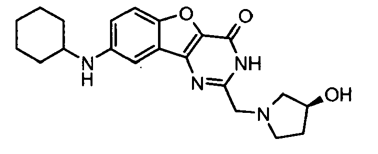 | 8-(cyclohexylamino)-2-{[(3S)-3-hydroxypyrrolidin-1-yl]methyl[1]benzofuro[3, 2-d]pyrimidin-4(3H)-one **Activity= B** |
| **450** | 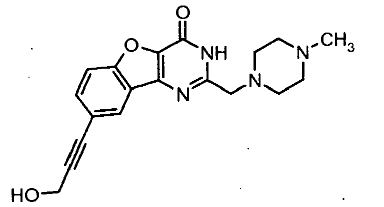 | 8-(3-hydroxyprop-1-yn-1-yl)-2-[(4-methylpiperazin-1-yl)methyl][1]benzofuro[3, 2-d]pyrimidin-4(3H)-one **Activity = B** |
| **451** | 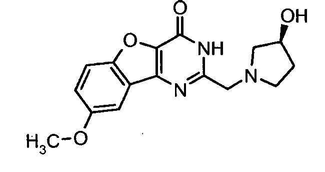 | 2-{[(3S)-3-hydroxypyrrolidin-1-yl]methyl}-8-(methyloxy)[1]benzofuro[ 3,2-d]pyrimidin-4(3H)-one **Activity = A, B and C** |
| **452** | 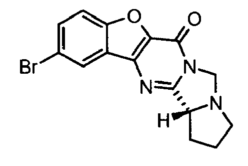 | (13bR)-11-bromo-1,2,3,13b-tetrahydro-7H-[1]benzofuro[3,2-d]pyrrolo[ 1',2':3,4]imidaz o[1,5-a]pyrimidin-7-one **Activity = A, B and C** |
| **453** | 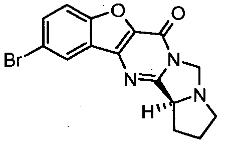 | (13bS)-11-bromo- 1,2,3,13b-tetrahydro-7H-[1]benzofuro[3,2-d]pyrrolo[1',2':3,4]imidaz o[1,5-a]pyrimidin-7-one **Activity = A, B and C** |
| **454** | 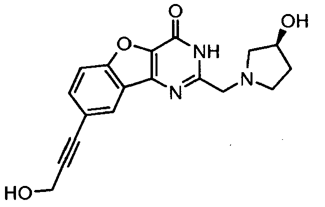 | 8-(3-hydroxyprop-1-yn-1-yl)-2-{[(3S)-3-hydroxypyrrolidin-1-yl]methyl[1]benzofuro[3, 2-d]pyrimidin-4(3H)-one **Activity = B and C** |
| **455** | 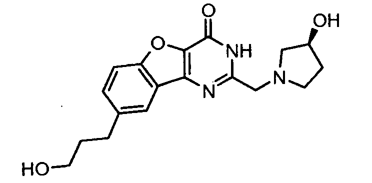 | 8-(3-hydroxypropyl)-2-{[(3S)-3-hydroxypyrrolidin-1-yl]methyl}[1]benzofuro[3, 2-d]pyrimidin-4(3H)-one **Activity = B and C** |
| **456** | 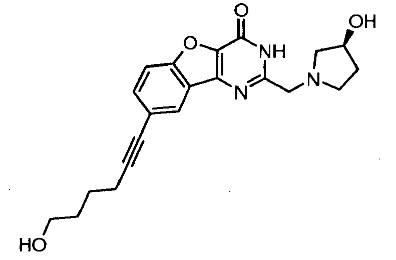 | 8-(6-hydroxyhex-1-yn-1-yl)-2-{[(35)-3-hydroxypyrrolidin-1-yl]methyl}[1]benzofuro[3, 2-d]pyrimidin-4(3H)-one **Activity = B and C** |
| **457** | 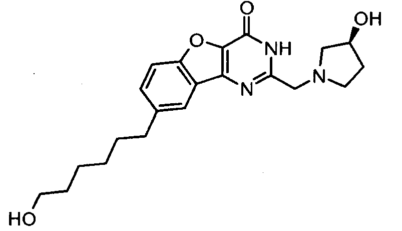 | 8-(6-hydroxyhexyl)-2-{[(3S)-3-hydroxypyrrolidin-1-yl]methyl}[1]benzofuro[3, 2-d]pyrimidin-4(3H)-one **Activity = B and C** |
| **458** | 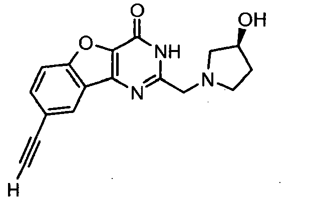 | 8-ethynyl-2-{[(3S)-3-hydroxypyrrolidin-1-yl]methyl}[1]benzofuro[3, 2-d]pyrimidin-4(3H)-one **Activity = A, B and C** |
| | | |
| **459** | 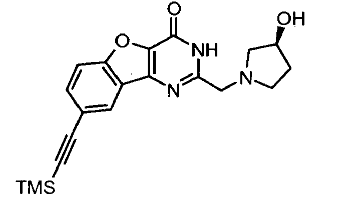 | 2-{[(3S)-3-hydroxypyrrolidin-1-yl]methyl}-8-[(trimethylsilyl)ethynyl][1 ]benzofuro[3,2-d]pyrimidin-4(3H)-one **Activity = A, B and C** |
| **460** | 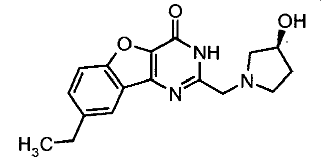 | 8-ethyl-2-{[(3S)-3-hydroxypyrrolidin-1-yl]methyl}[1]benzofuro[3, 2-d]pyrimidin-4(3H)-one **Activity = A, B and C** |
| **461** | 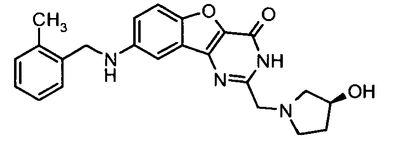 | 2-{[(3S)-3-hydroxypyrrolidin-1-yl]methyl}-8-{[(2-methylphenyl)methyl]ami no}[1]benzofuro[3,2-d]pyrimidin-4(3H)-one **Activity = B and C** |
| **462** | 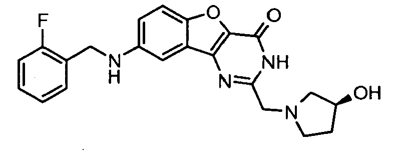 | 8-{[(2-fluorophenyl)methyl]amin o}-2-{[(3S)-3-hydroxypyrrolidin-1-yl]methyl}[1]benzofuro[3, 2-d]pyrimidin-4(3H)-one **Activity = B and C** |
| **463** | 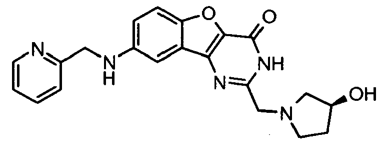 | 2-{[(3S)-3-hydroxypyrrolidin-1-yl]methyl}-8-[(pyridin-2-ylmethyl)amino] [1]benzof uro[3,2-d]pyrimidin-4(3H)-one **Activity = B and C** |
| **464** | 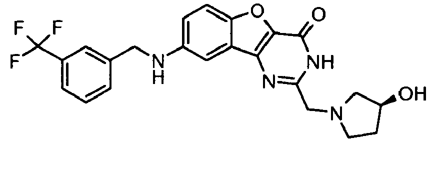 | 2-{[(3S)-3-hydroxypyrrolidin-1-yl]methyl}-8-({[3-(trifluoromethyl)phenyl]m ethyl}amino)[1]benzofuro [3,2-d]pyrimidin-4(3H)-one **Activity = B and C** |
| **465** | 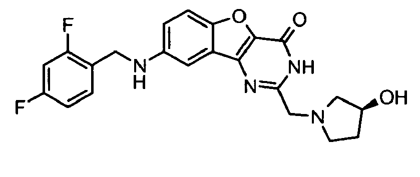 | 8-{[(2,4-difluorophenyl)methyl]am ino}-2-{[(3S)-3-hydroxypyrrolidin-1-yl]methyl[1]benzofuro[3, 2-d]pyrimidin-4(3H)-one **Activity = B and C** |
| **466** | 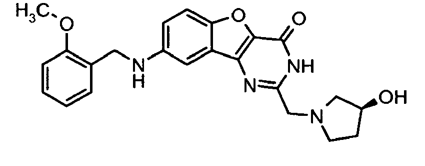 | 2-{[(3S)-3-hydroxypyrrolidin-1-yl]methyl}-8-({[2-(methyloxy)phenyl]methyl }amino)[1]benzofuro[3,2-d]pyrimidin-4(3H)-one **Activity = B and C** |
| **467** | 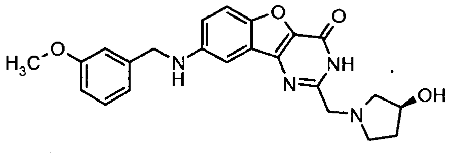 | 2-{[(3S)-3-hydroxypyrrolidin-1-yl]methyl}-8-({[3-(methyloxy)phenyl]methyl }amino)[1]benzofuro[3,2-d]pyrimidin-4(3H)-one **Activity = B and C** |
| **468** | 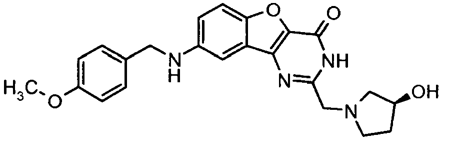 | 2-{[(3S)-3-hydroxypyrrolidin-1-yl]methyl}-8-({[4-(methyloxy)phenyl]methyl }amino)[1]benzofuro[3,2-d]pyrimidin-4(3H)-one **Activity = B and C** |
| **469** | 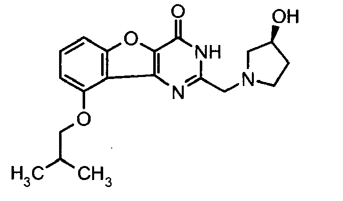 | 2-{[(3S)-3-hydroxypyrrolidin-1-yl]methyl}-9-[(2-methylpropyl)oxy] [1]benz ofuro[3,2-d]pyrimidin-4(3H)-one **Activity = A, B and C** |
| **470** | 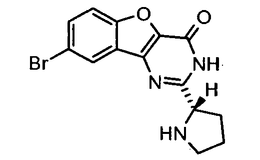 | 8-bromo-2-[(2S)-pyrrolidin-2-yl][1]benzofuro[3,2-d]pyrimidin-4(3H)-one **Activity = A, B and C** |
| **471** | 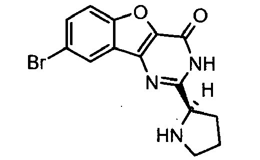 | 8-bromo-2-[(2R)-pyrrolidin-2-yl][1]benzofuro[3,2-d]pyrimidin-4(3H)-one **Activity = A, B and C** |
| **472** | 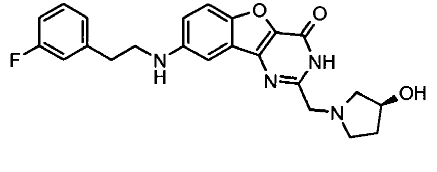 | 8-{[2-(3-fluorophenyl)ethyl]amino }-2-{[(3S)-3-hydroxypyrrolidin-1-yl]methyl}[1]benzofuro[3, 2-d]pyrimidin-4(3H)-one **Activity = B and C** |
| **473** | 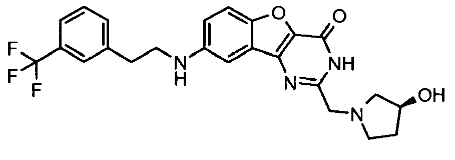 | 2-{[(3S)-3-hydroxypyrrolidin-1-yl]methyl}-8-({2-[3-(trifluoromethyl)phenyl]et hyl} amino)[1]benzofuro[3 ,2-d]pyrimidin-4(3H)-one **Activity = B and C** |
| **474** | 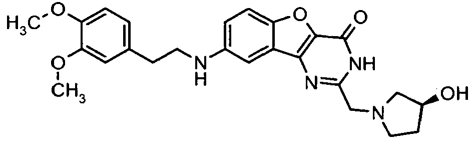 | 8-({2-[3,4-bis(methyloxy)phenyl]eth yl}amino)-2-{[(3S)-3-hydroxypyrrolidin-1-yl]methyl}[1]benzofuro[3, 2-d]pyrimidin-4(3H)-one **Activity = B and C** |
| **475** | 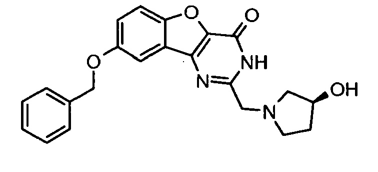 | 2-{[(3S)-3-hydroxypyrrolidin-1-yl]methyl}-8-[(phenylmethyl)oxy][1]be nzofuro[3,2-d]pyrimidin-4(3H)-one **Activity = B and C** |
| **476** | 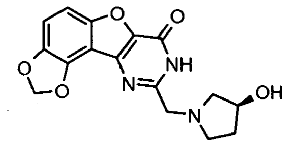 | 9-{[(3S)-3-hydroxypyrrolidin-1-yl]methyl}[1,3]dioxolo[4, 5][1]benzofuro[3,2-d]pyrimidin-7(8H)-one **Activity = A, B and C** |
| **477** | 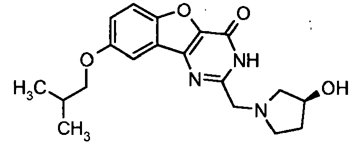 | 2-{[(3S)-3-hydroxypyrrolidin-1-yl]methyl}-8-[(2-methylpropyl)oxy][1]benz ofuro[3,2-d]pyrimidin-4(3H)-one **Activity = B and C** |
| **488** | 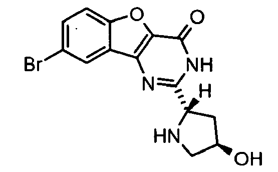 | 8-bromo-2-[(2S,4R)-4-hydroxypyrrolidin-2-yl][1]benzofuro[3,2-d]pyrimidin-4(3H)-one **Activity = A, B and C** |
| **489** | 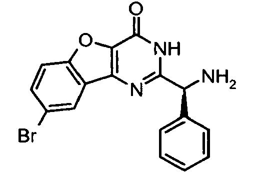 | 2-[(S)-amino(phenyl)methyl]-8-bromo[1]benzofuro[3,2-d]pyrimidin-4(3H)-one **Activity = A, B and C** |
| **490** | 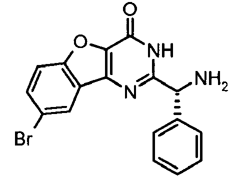 | 2-[(R)-amino(phenyl)methyl]-8-bromo[1]benzofuro[3,2-d]pyrimidin-4(3H)-one **Activity = A, B and C** |
| **491** | 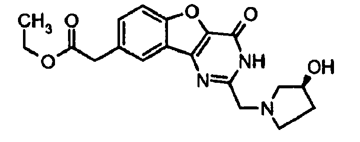 | ethyl (2-{[(3S)-3-hydroxypyrrolidin-1-yl]methyl-4-oxo,-3,4-dihydro[1]benzofuro[3,2-d]pyrimidin-8-yl)acetate **Activity = B and C** |
| **492** | 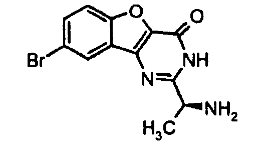 | 2-[(1S)-1-aminoethyl]-8-bromo[1]benzofuro[3,2-d]pyrimidin-4(3H)-one **Activity = A, B and C** |
| **493** | 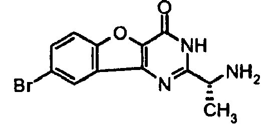 | 2-[(1R)-1-aminoethyl]-8-bromo[1]benzofuro[3,2-d]pyrimidin-4(3H)-one **Activity = A, B and C** |
| **494** | 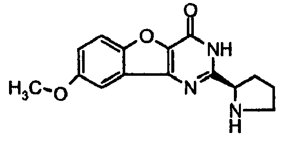 | 8-(methyloxy)-2-[(2R)-pyrrolidin-2-yl][1]benzofuro[3,2-d]pyrimidin-4(3H)-one **Activity = A, B and C** |
| **495** | 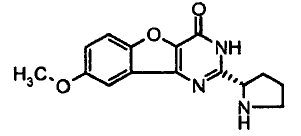 | 8-(methyloxy)-2-[(2S)-pyrrolidin-2-yl][1]benzofuro[3,2-d]pyrimidin-4(3H)-one **Activity = A, B and C** |
| **496** | 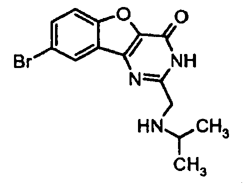 | 8-bromo-2-{[(1-methylethyl)amino]methyl }[1]benzofuro[3,2-d]pyrimidin-4(3H)-one **Activity = A, B and C** |
| ***497** | 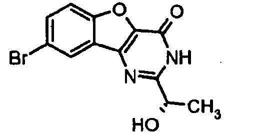 | 8-bromo-2-[{1S)-1-hydroxyethyl][1]benzofur o[3,2-d]pyrimidin-4(3H)-one **Activity = A, B and C** |
| **498** | 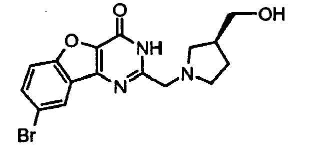 | 8-bromo-2-{[(3S)-3-(hydroxymethyl)pyrrolidin -1-yl]methyl} [1]benzofuro[3, 2-d]pyrimidin-4(3H)-one **Activity = A, B and C** |
| **499** | 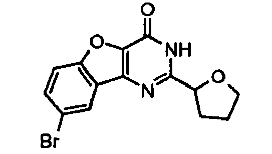 | 8-bromo-2-(tetrahydrofuran-2-yl)[1]benzofuro[3,2-d]pyrimidin-4(3H)-one **Activity = A, B and C** |
| **500** | 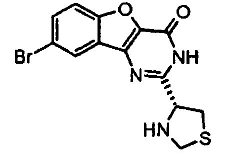 | 8-bromo-2-[(4R)-1,3-thiazolidin-4-yl][1]benzofuro[3,2-d]pyrimidin-4(3H)-one **Activity = A, B and C** |
| **501** | 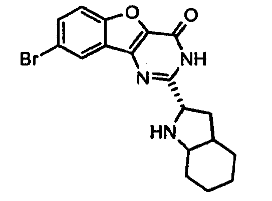 | 8-bromo-2-[(2S)-octahydro-1H-indol-2-yl][1]benzofuro[3,2-d]pyrimidin-4(3H)-one **Activity = A, B and C** |
| **502** | 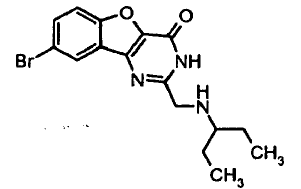 | 8-bromo-2-{[(1-ethylpropyl)amino]methyl }[1]benzofuro[3,2-d]pyrimidin-4(3H)-one **Activity = A, B and C** |
| **503** | 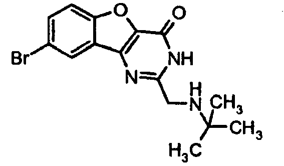 | 8-bromo-2-{[(1,1-dimethylethyl)amino]meth yl}[1]benzofuro[3,2-d]pyrimidin-4(3H)-one **Activity = A, B and C** |
| ***504** | 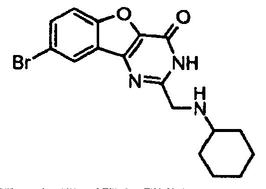 | 8-bromo-2-[(cyclohexylamino)methyl ][1]benzofuro[3,2-d]pyrimidin-4(3H)-one **Activity = A, B and C** |
| **505** | 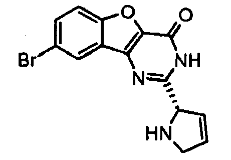 | 8-bromo-2-[(2S)-2,5-dihydro-H-pyrrol-2-yl][1]benzofuro[3,2-d]pyrimidin-4(3H)-one **Activity = A, B and C** |
| **506** | 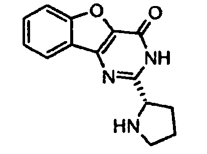 | 2-[(2S)-pyrrolidin-2-yl][1]benzofuro[3,2-d]pyrimidin-4(3H)-one **Activity = A, B and C** |
| **507** | 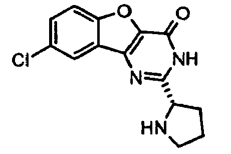 | 8-chloro-2-[(2S)-pyrrolidin-2-yl][1]benzofuro[3,2-d]pyrimidin-4(3H)-one **Activity = A, B and C** |
| **508** | 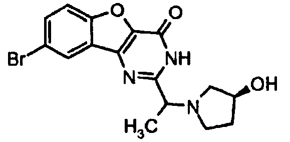 | 8-bromo-2-{1-[(3S)-3-hydroxypyrrolidin-1-yl]ethyl}[1]benzofuro[3,2-d]pyrimidin-4(3H)-one **Activity = A, B and C** |
| **509** | 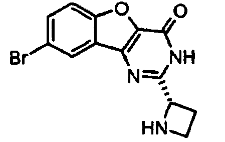 | 2-[(2S)-azetidin-2-yl]-8-bromo[1]benzofuro[3,2-d]pyrimidin-4(3H)-one **Activity = A, B and C** |
| ***510** | 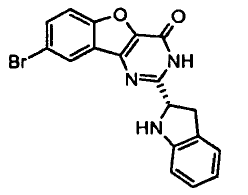 | 8-bromo-2-[(2S)-2,3-dihydro-1H-indol-2-yl][1]benzofuro[3,2-d]pyrimidin-4(3H)-one **Activity = A, B and C** |
| **511** | 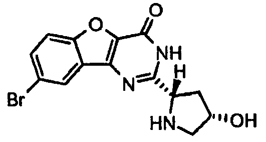 | 8-bromo-2-[(2S,4S)-4-hydroxypyrrolidin-2-yl][1]benzofuro[3,2-d]pyrimidin-4(3H)-one **Activity = A, B and C** |
| **512** | 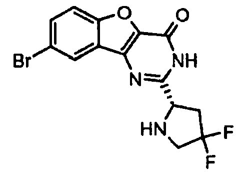 | 8-bromo-2-[(2S)-4,4-difluoropyrrolidin-2-yl][1]benzofuro[3,2-d]pyrimidin-4(3H)-one **Activity = A, B and C** |
| **513** | 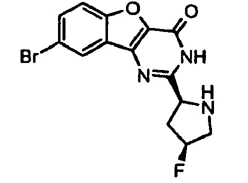 | 8-bromo-2-[(2S,4S)4-fluoropyrrolidin-2-yl][1]benzofuro[3,2-d]pyrimidin-4(3H)-one **Activity = A, B and C** |
| **514** | 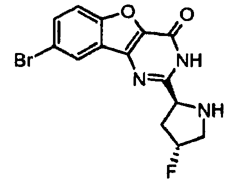 | 8-bromo-2-[(2S,4R)-4-fluoropyrrolidin-2-yl][1]benzofuro[3,2-d]pyrimidin-4(3H)-one **Activity = A, B and C** |
| ***515** | 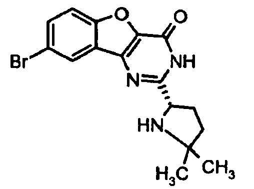 | 8-bromo-2-[(2S)-5,5-dimethylpyrrolidin-2-yl][1]benzofuro[3,2-d]pyrimidin-4(3H)-one **Activity = A, B and C** |
| **516** | 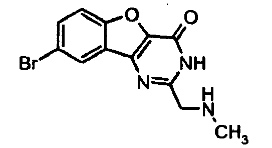 | 8-bromo-2-[(methylamino)methyl][1] benzofuro[3,2-d]pyrimidin-4(3H)-one **Activity = A, B and C** |
| **517** | 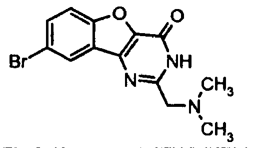 | 8-bromo-2-[(dimethylamino)methyl][ 1]benzofuro[3,2-d]pyrimidin-4(3H)-one **Activity = A, B and C** |
| **518** | 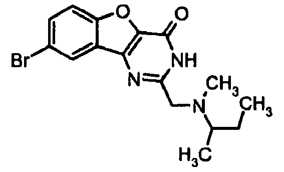 | 8-bromo-2-{[methyl(1-methylpropyl)amino]meth yl}[1]benzofuro[3,2-d]pyrimidin-4(3H)-one **Activity = A, B and C** |
| **519** | 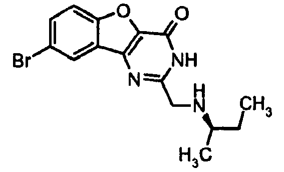 | 8-bromo-2-({[(1R)-1-methylpropyl]amino meth yl)[1]benzofuro[3,2-d]pyrimidin-4(3H)-one **Activity** = **A, B and C** |
| **520** | 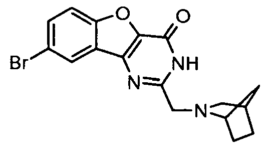 | 2-(2-azabicyclo[2.2.1]hept-2-ylmethyl)-8-bromo[1]benzofuro[3,2-d]pyrimidin-4(3H)-one **Activity = A, B and C** |
| **521** | 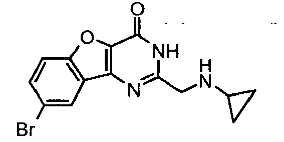 | 8-bromo-2-[(cyclopropylamino)methy 1][1]benzofuro[3,2-d]pyrimidin-4(3H)-one **Activity = A, B and C** |
| **522** | 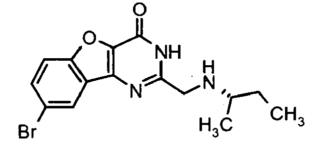 | 8-bromo-2-({[(1S)-1-methylpropyl]amino}meth yl)[1]benzofuro[3,2-d]pyrimidin-4(3H)-one **Activity = A, B and C** |
| **523** | 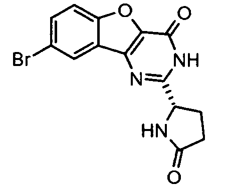 | 8-bromo-2-[(2S)-5-oxopyrrolidin-2 yl][1]benzofuro[3,2-d]pyrimidin-4(3H)-one **Activity = A, B and C** |
| **524** | 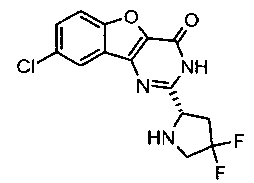 | 8-chloro-2-[(2S)-4,4-difluoropyrrolidin-2-yl][1]benzofuro[3,2-d]pyrimidin-4(3H)-one **Activity = A and B** |
| **525** | 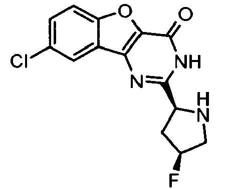 | 8-chloro-2-[(2S,4S)-4-fluoropyrrolidin-2-yl][1]benzofuro[3,2-d]pyrimidin-4(3H)-one **Activity = A and B** |
| **526** | 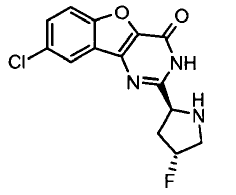 | 8-chloro-2-[(2S,4R)-4-fluoropyrrolidin-2-yl][1]benzofuro[3,2-d]pyrimidin-4(3H)-one **Activity = A, B and C** |
| **527** | 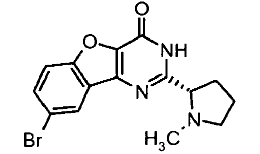 | 8-bromo-2-[(2S)-1-methylpyrrolidin-2-yl][1]benzofuro[3,2-d]pyrimidin-4(3H)-one **Activity = A, B and C** |
| **528** | 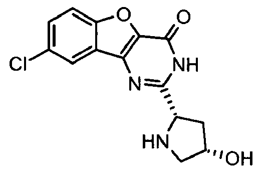 | 8-chloro-2-[(2S,4S)-4-hydroxypyrrolidin-2-yl][1]benzofuro[3,2-d]pyrimidin-4(3H)-one **Activity = A, B and C** |
| **529** | 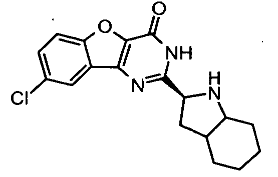 | 8-chloro-2-[(2S)-octahydro-1H-indol-2-yl][1]benzofuro[3,2-d]pyrimidin-4(3H)-one **Activity = A, B and C** |
| **530** | 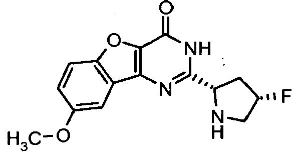 | 2-[(2S,4S)-4-fluoropyrrolidin-2-yl]-8-(methyloxy)[1]benzofuro[ 3,2-d]pyrimidin-4(3H)-one **Activity = A and B** |
| **531** | 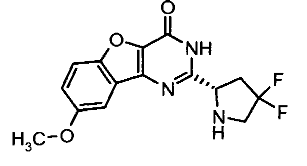 | 2-[(2S)-4,4-difluoropyrrolidin-2-yl]-8-(methyloxy)[1]benzofuro[ 3,2-d]pyrimidin-4(3H)-one **Activity = A, B and C** |
| **532** | 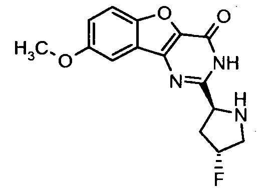 | 2-[(2S,4R)-4-fluoropyrrolidin-2-yl]-8-(methyloxy)[1]benzofuro[ 3,2-d]pyrimidin-4(3H)-one **Activity = A, B and C** |
| **533** | 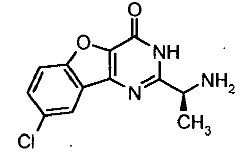 | 2-[(1S)-1-aminoethyl]-8-chloro[1]benzofuro[3,2-d]pyrimidin-4(3H)-one **Activity = A, B and C** |
| **534** | 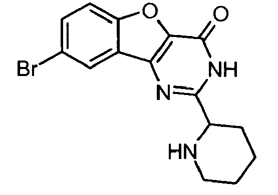 | 8-Bromo-2-piperidin-2-yl[1]benzofuro[3,2-d]pyrimidin-4(3H)-one Hydrochloride **Activity = A, B and C** |
| **535** | 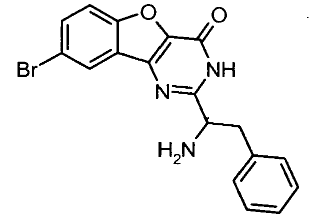 | 2-(1-amino-2-phenylethyl)-8-bromo[1]benzofuro[3,2-d]pyrimidin-4(3H)-one **Activity = A and B** |
| **536** | 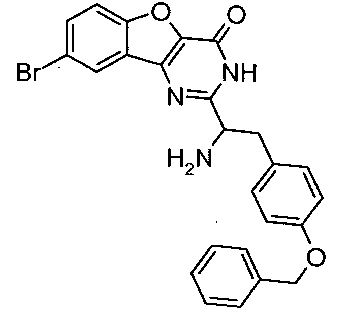 | 2-(1-amino-2-{4-[(phenylmethyl)oxy]pheny 1}ethyl)-8-bromo[1]benzofuro[3,2-d]pyrimidin-4(3H)-one **Activity = A and B** |
| **537** | 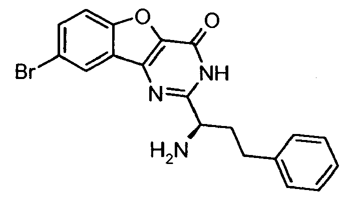 | 2-[(1S)-1-amino-3-phenylpropyl]-8-bromo[1]benzofuro[3,2-d]pyrimidin-4(3H)-one **Activity = A, B and C** |
| **538** | 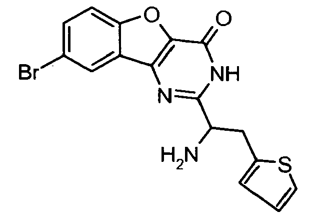 | 2-[1-amino-2-(2-thienyl)ethyl]-8-bromo[1]benzofuro[3,2-d]pyrimidin-4(3H)-one **Activity = A, B and C** |
| **539** | 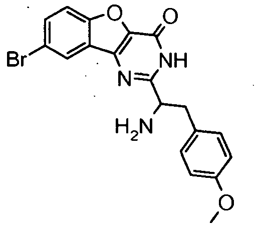 | 2-{1-amino-2-[4-(methyloxy)phenyl]ethyl} -8-bromo[1]benzofuro[3,2-d]pyrimidin-4(3H)-one **Activity = A and B** |
| **540** | 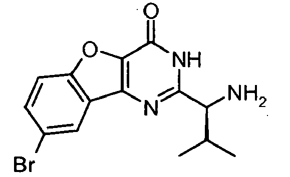 | 2-[(1S)-1-amino-2-methylpropyl]-8-bromo[1]benzofuro[3,2-d]pyrimidin-4(3H)-one **Activity = A, B and C** |
| **541** | 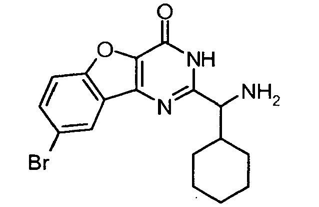 | 2-[amino(cyclohexyl)methyl ]-8-bromo[1]benzofuro[3,2-d]pyrimidin-4(3H)-one **Activity = A and B** |
| **542** | 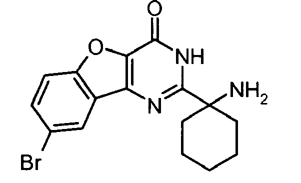 | 2-(1-aminocyclohexyl)-8-bromo[1]benzofuro[3,2-d]pyrimidin-4(3H)-one **Activity = A, B and C** |
| **543** | 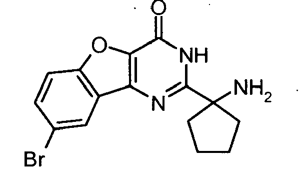 | 2-(1-aminocyclopentyl)-8-bromo[1]benzofuro[3,2-d]pyrimidin-4(3H)-one **Activity = A, B and C** |
| **544** | 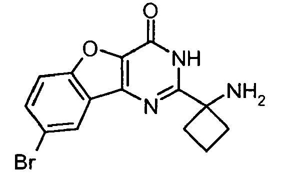 | 2-(1-aminocyclobutyl)-8-bromo[1]benzofuro[3,2-d]pyrimidin-4(3H)-one **Activity = A, B and C** |

**Activity A** in Table I is meant to mean the compound has a CDC7 IC₅₀ value of less than 10,000 nanomolar (nm).

**Activity B** in Table 1 is meant to mean the compound has a PIM IC₅₀ value of less than 10,000 nanomolar (nm).

**Activity C** in Table 1 is meant to mean the compound has a CK2 IC₅₀ value of less than 10,000 nanomolar (nm).

When the activity for any of the compounds in Table 1 lists more than one target for which the compound has activity against (as defined by Activity A, B and C above), then the compound in question is meant to be active against each of the targets that is listed next to the compound.

This dislcosure also relates to a pharmaceutical composition comprising a compound according to Formula 1, or a pharmaceutically acceptable salt thereof, and a pharmaceutically acceptable carrier, excipient, or diluent.

This dislcosure also relates to an in vitro method of inhibiting PIM, CDC7 or CK2 in a cell, comprising contacting a cell in which inhibition of PIM, CDC7 or CK2 is desired with a compound according to Formula I, or a pharmaceutically acceptable salt thereof.

This dislcosure also relates to an in vitro method of inhibiting PIM, CDC7 or CK2 in a cell, comprising contacting a cell in which inhibition of PIM, CDC7 or CK2 is desired with a pharmaceutical composition comprising a compound according to Formula I, or a pharmaceutically acceptable salt thereof, and a pharmaceutically acceptable carriers, excipient, or diluent.

Also discussed herein is a compound or composition for use in the treatment of a disease or condition, including those that involve PIM, CDC7 or CK2, comprising a compound according to Formula I, or a pharmaceutically acceptable salt thereof. Non-limiting examples of the disease or condition that can be treated include cancer such as ovarian cancer, pancreatic cancer, prostate cancer, hepatocellular carcinoma, lymphomas, leukemias, cervical cancer, breast cancer, colorectal cancer, non-small cell lung cancer (NSCL) or glioblastomas. The disease or condition that can be treated is selected from pancreatic cancer, prostate cancer, hepatocellular carcinoma, lymphomas, leukemias, colorectal cancer and non-small cell lung cancer. The disease or condition that can be treated is selected from pancreatic cancer, prostate cancer, hepatocellular carcinoma, lymphomas and leukemias. The disease or condition that can be treated is selected from colorectal cancer and non-small cell lung cancer.

This disclosure relates to a compound in Table 1 that has a CDC7 IC₅₀ value of less than 3000 nm, or a pharmaceutically acceptable salt of such a compound. The compounds in Table 1 that have a CDC7 IC₅₀ value of less than 3000 nm are compounds 1-3, 5, 13, 24, 28, 32-35, 40, 43, 45, 48, 49, 52, 60, 64, 100, 103, 107, 108-109, 111-112, 119, 121, 127-128, 130, 138, 140-144, 147-148, 153-155, 157-161, 164, 166-167, 169, 173-175, 178, 181, 182-186, 189, 191, 194-195, 197-199, 201, 205, 214, 216-217, 220-221, 223-224, 226, 231-232, 235, 248, 252, 259, 262, 264, 266, 269, 270, 273, 277, 283, 287, 289-290, 298, 302, 305, 307, 308, 311, 313, 315, 317, 319, 323, 324, 330-333, 344, 346, 348, 349, 353, 363-364, 366, 368-370, 373-375, 379-381, 383, 385-389, 395, 400-402, 404, 406-408, 412-413, 418, 420, 425-427, 429, 430-435, 436-439, 442, 448, 451-453, 458-460, 469-471, 476, 488-490, 492-509, 511-535, and 537-544.

This disclosure also relates to a compound in Table 1 that has a CDC7 IC₅₀ value of less than 100 nm, or a pharmaceutically acceptable salt of such a compound. The compounds in Table 1 that have a CDC7 IC₅₀ value of less than 100 nm are compounds 1-2, 5, 28, 49, 103, 108, 119, 128, 147, 157-158, 164, 166-167, 169, 173-175, 183-186, 191, 198-199, 201, 214, 216, 221, 223, 231-232, 242, 252, 259, 262, 269, 270, 273, 277, 287, 289, 298, 307, 308, 317, 331-333, 344, 346, 348, 364, 366, 368-369, 375, 379-380, 383, 400-402, 404, 407-408, 412-413, 421, 426, 429, 432, 437, 442, 452, 453, 458, 470-471, 476, 488-490, 492, 493, 495, 496, 498, 501-503 505 507-509, 511-534, and 540-544.

Also disclosed herein is a pharmaceutical composition for use in the treatment of a disease or condition, including those that involve PIM, CDC7 or CK2, comprising a pharmaceutical composition comprising a compound according to Formula I, or a pharmaceutically acceptable salt thereof, and a pharmaceutically acceptable carrier, excipient, or diluent. Non-limiting examples of the disease or condition that can be treated include cancer such as ovarian cancer, pancreatic cancer, prostate cancer, hepatocellular carcinoma, lymphomas, leukemias, cervical cancer, breast cancer, colorectal cancer, non-small cell lung cancer (NSCL) or glioblastomas. The disease or condition that can be treated is selected from pancreatic cancer, prostate cancer, hepatocellular carcinoma, lymphomas, leukemias, colorectal cancer and non-small cell lung cancer. The disease or condition that can be treated is selected from pancreatic cancer, prostate cancer, hepatocellular carcinoma, lymphomas and leukemias. The disease or condition that can be treated is selected from colorectal cancer and non-small cell lung cancer.

Another aspect of this dislcosure relates to a compound or composition for use in the treatment of a disease or condition, including those that involve PIM, CDC7 or CK2 comprising a compound according to Formula I, or a pharmaceutically acceptable salt thereof, in combination with radiation treatment and/or one or more therapeutic angents selected from Camptothecin, Topotecan, 9-Nitrocamptothecin, 9-Aminocamptothecin, Karenitecin, Irinotecan, Etoposide, Etoposide Phosphate, Teniposide, Amsacrine, Razoxane, Dexrazoxane, Mechlorethamine, Cyclophosphamide, Ifosfamide, Chlorambucil, Melphalan, Thiotepa, Trenimon, Triethylenemelamine, Rapamycin, Dianhydrogalactitol, Dibromodulcitol, Busulfan, dimethylsulfate, Chloroethylnitrosourea, BCNU, CCNU, Methyl-CCNU, Streptozotocin, Chlorozotocin, Prednimustine, Estramustine, Procarbazine, Dacarbazine, Hexamethylmelamine, Pentamethylmelamine, Temozolomide, Cisplatin, Carboplatin, Oxaliplatin, Bleomycin, Dactinomycin, Mithramycin, Mitomycin C, Daunorubicin, Doxorubicin, Epirubicin, Idarubicin, Methotrexate, Edatrexate, Trimethoprim, Nolatrexed, Raltitrexed, Hydroxyurea, 5-fluorouracil, Ftorafur, Capecitabine, Furtulon, Eniluracil, ara-C, 5-azacytidine, Gemcitabine, Mercaptopurine, Thioguanine, Pentostatin, antisense DNA, antisense RNA, an antisense DNA/RNA hybrid, a ribozyme, ultraviolet radiation, Vincristine, Vinblastine, Paclitaxel, Docetaxel, L-Asparaginase, a kinase inhibitor, Imatinib, Mitotane, Aminoglutethimide, Diethylstilbestrol, Ethinyl estradiol, Tamoxifen, Anastrozole, Testosterone propionate, Fluoxymesterone, ixabepilone, Flutamide, Leuprolide, Prednisone, Hydroxyprogesterone caproate, Medroxyprogesterone acetate, Megestrol acetate, Interferon-alfa, and Interleukin. In a more specific embodiment, the combination is with ixabepilone or etoposide.

### Abbreviations and Definitions

The following abbreviations and terms have the indicated meanings throughout:

| **Abbreviation** | **Meaning** |
|---|---|
| Ac | Acetyl |
| Br | Broad |
| °C | Degrees Celsius |
| c- | Cyclo |
| CBZ | CarboBenZoxy = benzyloxycarbonyl |
| D | Doublet |
| Dd | Doublet of doublet |
| Dt | doublet of triplet |
| DIPEA | *N,N*-diisopropylethylamine |
| DMF | *N,N*-dimethylformamide |
| DMSO | dimethyl sulfoxide |
| EI | Electron Impact ionization |
| Et | Ethyl |
| G | gram(s) |
| GC | gas chromatography |
| h or hr | hour(s) |
| HOAc | acetic acid |
| HOBt | Hydroxybenzotriazole |
| HPLC | high pressure liquid chromatography |
| L | liter(s) |
| M | molar or molarity |
| M | Multiplet |
| Me | Methyl |
| Mesyl | Methanesulfonyl |
| Mg | milligram(s) |
| MHz | megahertz (frequency) |
| Min | minute(s) |
| mL | milliliter(s) |
| mM | Millimolar |
| Mol | millimole(s) |
| Mol | mole(s) |
| MS | mass spectral analysis |
| MTBE | methyl t-butyl ether |
| N | normal or normality |
| NBS | *N*-bromosuccinimide |
| NCS | *N*-chlorosuccinimide |
| nM | Nanomolar |
| NMO | *N*-methylmorpholine oxide |
| NMR | nuclear magnetic resonance spectroscopy |
| PEG | polyethylene glycol |
| pEY | poly-glutamine, tyrosine |
| Ph | Phenyl |
| PhOH | Phenol |
| PfP | Pentafluorophenol |
| PfPy | Pentafluoropyridine |
| PPTS | Pyridinium p-toluenesulfonate |
| Py | Pyridine |
| PyBroP | bromo-tris-pyrrolidino-phosphonium hexafluorophosphate |
| Q | Quartet |
| RT | Room temperature |
| Sat'd | Saturated |
| S | Singlet |
| s- | Secondary |
| t- | Tertiary |
| t or tr | Triplet |
| TBDMS | t-butyldimethylsilyl |
| TES | Triethylsilyl |
| TFA | trifluoroacetic acid |
| THF | Tetrahydrofuran |
| TMOF | trimethyl orthoformate |
| TMS | Trimethylsilyl |
| Tosyl | *p*-toluenesulfonyl |
| Trt | Triphenylmethyl |
| uL | microliter(s) |
| uM | Micromole(s) or micromolar |

As used in the present specification, the following words and phrases are generally intended to have the meanings as set forth below, except to the extent that the context in which they are used indicates otherwise or they are expressly defined to mean something different.

The symbol "-" means a single bond, "=" means a double bond, "≡" means a triple bond, "-̅-̅-̅-̅" means a single or double bond. When a group is depicted removed from its parent formula, the " 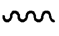 " symbol will be used at the end of the bond which was theoretically cleaved in order to separate the group from its parent structural formula.

When chemical structures are depicted or described, unless explicitly stated otherwise, all carbons are assumed to have hydrogen substitution to conform to a valence of four. For example, in the structure on the left-hand side of the schematic below there are nine hydrogens implied. The nine hydrogens are depicted in the righthand structure. Sometimes a particular atom in a structure is described in textual formula as having a hydrogen or hydrogens as substitution (expressly defined hydrogen), for example,
-CH₂CH₂-. It is understood by one of ordinary skill in the art that the aforementioned descriptive techniques are common in the chemical arts to provide brevity and simplicity to description of otherwise complex structures.

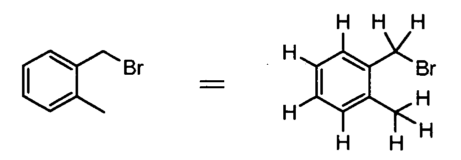

If a group "R" is depicted as "floating" on a ring system, as for example in the formula: 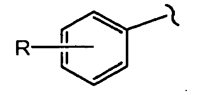 then, unless otherwise defined, a substituent "R" can reside on any atom of the ring system, assuming replacement of a depicted, implied, or expressly defined hydrogen from one of the ring atoms, so long as a stable structure is formed.

If a group "R" is depicted as floating on a fused ring system, as for example in the formulae: 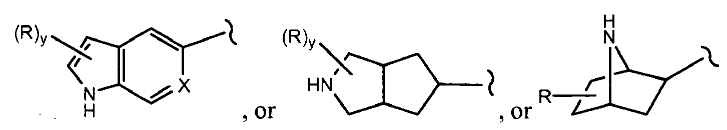 then, unless otherwise defined, a substituent "R" can reside on any atom of the fused ring system, assuming replacement of a depicted hydrogen (for example the -NH- in the formula above), implied hydrogen (for example as in the formula above, where the hydrogens are not shown but understood to be present), or expressly defined hydrogen (for example where in the formula above, "X" equals =CH-) from one of the ring atoms, so long as a stable structure is formed. In the example depicted, the "R" group can reside on either the 5-membered or the 6-membered ring of the fused ring system. In the formula depicted above, when y is 2 for example, then the two "R's" can reside on any two atoms of the ring system, again assuming each replaces a depicted, implied, or expressly defined hydrogen on the ring.

When a group "R" is depicted as existing on a ring system containing saturated carbons, as for example in the formula: 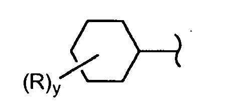 where, in this example, "y" can be more than one, assuming each replaces a currently depicted, implied, or expressly defined hydrogen on the ring; then, unless otherwise defined, where the resulting structure is stable, two "R's" can reside on the same carbon. A simple example is when R is a methyl group; there can exist a geminal dimethyl on a carbon of the depicted ring (an "annular" carbon). In another example, two R's on the same carbon, including that carbon, can form a ring, thus creating a spirocyclic ring (a "spirocyclyl" group) structure with the depicted ring as for example in the formula:

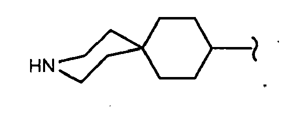

"Administration" and variants thereof (e.g., "administering" a compound) in reference to a compound of this disclosure (i.e., a compound of Formula I as described herein) means introducing the compound or a prodrug of the compound into the system of the animal in need of treatment. When a compound of this disclosure or prodrug thereof is provided in combination with one or more other active agents (e.g., surgery, radiation, and chemotherapy, etc.), "administration" and its variants are each understood to include concurrent and sequential introduction of the compound or prodrug thereof and other agents.

"Alkyl" is intended to include molecules having 1-12 carbons in size (C₁-C₁₂)alkyl, which can be straight chained or branched. For example, "C₆ alkyl" can refer to an *n*-hexyl, *iso*-hexyl, cyclobutylethyl, and the like. Alkyl is intended to include lower alkyl groups of from 1-6 carbons in size, such as methyl, ethyl, propyl, isopropyl, butyl, *s*-butyl, *t*-butyl, isobutyl, pentyl, hexyl and the like. Higher alkyl refers to alkyl groups containing more that six carbon atoms. An alkyl residue having a specific number of carbons is named, all geometric isomers having that number of carbons are intended to be encompassed; thus, for example, either "butyl" or "C₄ alkyl" is meant to include *n*-butyl, *sec*-butyl, isobutyl, *t*-butyl, and for example, "propyl" or "C₃ alkyl" each include *n*-propyl and isopropyl.

-(C₁-C₆)alkyl is a subset of alkyl groups that are from one to six carbon atoms in length, and can be straight chained or branched.

-(C₁-C₃)alkyl is a subset of alkyl groups that are from one to three carbon atoms in length, and can be straight chained or branched.

"alkenyl" is intended to be an alkyl that contains at least one double bond between two carbons. Non-limiting examplels of alkenyl include vinyl, allyl, isoprenyl, and the like.

"alkynyl" is intended to be an alkyl that contains at least one triple bond between two carbons.

"Cycloalkyl" means a non-aromatic mono- or multicyclic ring system comprising about 3 to about 14 carbon atoms. Non-limiting examples of monocyclic cycloalkyls include cyclopropyl, cyclopentyl, cyclohexyl, cycloheptyl and the like. Non-limiting examples of multicyclic cycloalkyls include 1-decalin, norbornyl, adamantyl and the like. Cycloalkyls can be fused or bridge ring systems or spirocyclic systems.

"-(C₃-C₆)cycloalkyl" is a subset of cycloalkyl and means a non-aromatic monocyclic ring system comprising from 3 to 6 carbon atoms.

"Alkoxy" or "alkoxyl" both refer to the group -O-alkyl, wherein the "alkyl" portion is as defined hereinabove. Examples include methoxy, ethoxy, propoxy, isopropoxy, and the like.

"Aryl" means a monovalent six- to fourteen-membered mono- or multicyclic ring, wherein the monocyclic ring is aromatic and at least one of the rings in the multicyclic ring is aromatic. A multicyclic ring that contains only one aryl ring is intended to be included within the definition of aryl. An aryl can also be six- to ten membered, or six membered. Representative non-limiting examples of aryl include phenyl, naphthyl, and the like.

"Arylalkyl" means a residue in which an aryl moiety, as defined above, is attached to a parent structure via one of an alkyl (i.e, alkylene, alkenylene, or alkynylene), wherein the "aryl" and "alkyl" portions are as defined herein. Examples include benzyl, phenethyl, phenylvinyl, phenylallyl and the like. The "alkyl" portion of the group can be one to ten carbons, and in another embodiment, one to six carbons; the latter can also be referred to as C₁₋₆ arylalkyl. When a group is referred to as or "-(C₁-C₆)alkylaryl," an aryl moiety is attached to a parent structure via an alkylene group. Examples include benzyl, phenethyl, and the like.

In some examples, as appreciated by one of ordinary skill in the art, two adjacent groups on an aromatic system can be fused together to form a ring structure. The fused ring structure can contain heteroatoms and can be optionally substituted with one or more groups. It should additionally be noted that saturated carbons of such fused groups (*i.e*. saturated ring structures) can contain two substitution groups.

"Fused-polycyclic" or "fused ring system" refers to a polycyclic ring system that contains bridged or fused rings; that is, where two rings have more than one shared atom in their ring structures. In this application, fused-polycyclics and fused ring systems includes non-aromatic and aromatic systems. Typically, but not necessarily, fused-polycyclics share a vicinal set of atoms, for example naphthalene or 1,2,3,4-tetrahydro-naphthalene. A spiro ring system is not a fused-polycyclic by this definition, but fused polycyclic ring systems of the compounds disclosed herein can themselves have spiro rings attached thereto via a single ring atom of the fused-polycyclic.

"Halogen" or "halo" both refer to fluorine, chlorine, bromine or iodine.

"Haloalkyl" (which includes alkyl, as defined herein, optionally substituted with up to 8 halo) and "haloaryl" refer generically to alkyl and aryl groups that are substituted with one or more halo, respectively. Non-limiting examples of "haloalkyl" include 3,3,3-trifluoro-1-methylpropyl, 2-methyl-1-(trifluoromethyl)propyl, -CH₂F, - CHCl₂ and -CF₃.

"Heteroatom" refers to O, S, N, or P. In another example, the heteroatom is O or N. In another example, the heteroatom is O. In another example, the heteroatom is N.

"Heterocyclyl" refers to a stable three- to fifteen-membered ring substituent that consists of carbon atoms and from one to five heteroatoms selected from the group consisting of nitrogen, phosphorus, oxygen and sulfur. For purposes of this disclosure, the heterocyclyl substituent can be a monocyclic, bicyclic or tricyclic ring system, which can include fused or bridged ring systems as well as spirocyclic systems. The terms "heterocycloalkyl" and "heteroaryl" are groups that are encompassed by the broader term "heterocyclyl." The nitrogen, phosphorus, carbon or sulfur atoms in the heterocyclyl group can be optionally oxidized to various oxidation states. In a specific example, the group -S(O)₀₋₂-, refers to -S- (sulfide), - S(O)- (sulfoxide), and -SO₂- (sulfone) respectively. For convenience, nitrogens, particularly but not exclusively, those defined as annular aromatic nitrogens, are meant to include their corresponding *N-*oxide form, although not explicitly defined as such in a particular example. Thus, for a compound of this disclosure having, for example, a pyridyl ring; the corresponding pyridyl-*N*-oxide is meant to be included as another compound of the disclosure. In addition, annular nitrogen atoms can be optionally quaternized; and the ring substituent can be partially or fully saturated or aromatic. Examples of heterocyclyl groups include, but are not limited to, azetidinyl, acridinyl, benzodioxolyl, benzodioxanyl, benzofuranyl, carbazoyl, cinnolinyl, dioxolanyl, indolizinyl, naphthyridinyl, perhydroazepinyl, phenazinyl, phenothiazinyl, phenoxazinyl, phthalazinyl, pteridinyl, purinyl, quinazolinyl, quinoxalinyl, quinolinyl, isoquinolinyl, tetrazoyl, tetrahydroisoquinolyl, piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, dihydropyridinyl, tetrahydropyridinyl, pyridinyl, pyrazinyl, pyrimidinyl, pyridazinyl, oxazolyl, oxazolinyl, oxazolidinyl, triazolyl, isoxazolyl, isoxazolidinyl, morpholinyl, thiazolyl, thiazolinyl, thiazolidinyl, isothiazolyl, quinuclidinyl, isothiazolidinyl, indolyl, isoindolyl, indolinyl, isoindolinyl, octahydroindolyl, octahydroisoindolyl, quinolyl, isoquinolyl, decahydroisoquinolyl, benzimidazolyl, thiadiazolyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, dioxaphospholanyl, oxadiazolyl, tetrahydrofuranyl, tetrahydroisoquinolinyl, and tetrahydroquinolinyl.

"Heterocycloalkyl" refers to a stable 4-12 membered monocyclic or multicyclic ring, wherein at least one of the rings contain at least one heteroatom and wherein there are no aromatic rings. Heterocycloalkyl is meant to include multicyclic rings wherein one ring contains a heteroatom and another ring does not contain a heteroatom.

"Heterocycloalkylalkyl" refers to a heterocycloalkyl, as defined herein, attached to the parent moiety through an "alkyl," as defined herein. One non-limiting example of heterocycloalkyl includes piperadinyl. Another non-limiting example of heterocycloalkyl includes piperazinyl. Another non-limiting example of heterocycloalkyl includes furanyl. Another non-limiting example of heterocycloalkyl includes pyrrolidinyl. Another non-limiting example of heterocycloalkyl includes morpholinyl.

"Amino" refers to -NH₂.

"Alkylamino" refers to -NH(alkyl), wherein "alkyl" portion is as defined above, and wherein the parent moiety is attached to the nitrogen atom.

"Dialkylamino" refers to -N(alkyl)₂, wherein the "alkyl" portiobs are as defined above, and wherein the parent moiety is attached to the nitrogen atom.

"Dialkylaminoalkyl" refers to -(alkyl)N(alkyl)₂, wherein the "alkyl" portions are as defined above. One such non-limiting example of "dialkylaminoalkyl" includes -CH₂C(CH₃)₂CH₂N(CH₃)₂.

"Aminoalkyl" refers to -(alkyl)NH₂, wherein "alkyl" is as defined above, and wherein the parent moiety is attached to the alkyl group. The amino group can be attached at any point along the alkyl group. Non-limiting examples of aminoalkyl include -CH₂NH₂, -CH₂CH₂NH₂, -C(CH₃)₂NH₂. and -CH(NH₂)CH₃.

"Heteroaryl" means a 5- to 12-membered, monocyclic aromatic heterocyclyl (where heterocyclyl is defined herein) or bicyclic heterocyclyl ring system (where at least one of the rings in the bicyclic system is aromatic) where the monocyclic ring and at least one of the rings in the bicyclic ring system contains one, two, three, four, or five heteroatom(s) selected from nitrogen, oxygen, phosphorous, and sulfur. The ring containing the heteroatom can be aromatic or non-aromatic. Representative examples include pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzdioxolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. Fused, bridged, and spiro moieties are also included within the scope of this definition. In another embodiment, the heteroaryl groups described herein are selected from thienyl, oxazolyl, furyl, pyrrolyl, imidazolyl, thiazolyl, pyridinyl, imidazolyl and pyrimidinyl.

"Carbonyl" refers to the group "-C(O)-", which is bivalent.

"Aminocarbonyl" refers to the group "-C(O)-NH₂," wherein the parent moiety is attached to the amino group.

"Alkoxycarbonyl" refers to the group "-C(O)alkoxy," wherein alkoxy is as defined above, and the parent moiety is attached to the carbonyl. A non-limiting example includes -C(O)-OC(CH₃)₃.

"Hydroxyalkynyl" refers to a group wherein the parent moiety is attached to the alkynyl group, as defined above, and a hydroxyl group is attached to the alkynyl. A non-limiting example includes 4-hydroxybut-1-yn-1-yl.

"Hydroxyalkyl" refers to a group wherein the paret moiety is attached to the alkyl group, and a hydroxyl group is attached to the alkyl, wherein the alkyl portion is as defined in the term "alkyl" herein

"Amino(imino)alkyl" refers to a group represented by -alkyl-C(=NH)-NH₂, wherein the alkyl portion is as defined in the term "alkyl" herein. A non-limiting example includes amino(imino)methyl.

"Dihydroxyalkyl" refers to a group wherein the parent moiety is attached to the alkyl group, and a two hydroxyl groups are attached to the alkyl, wherein the alkyl portion is as defined in the term "alkyl" herein.

"Alkylaminoalkyl" refers to -(alkyl)NH(alkyl), wherein the alkyl portion is as defined in the term "alkyl" herein.

"Alkylaminoalkylarnino" refers to -N(H)(alkyl)NH(alkyl), wherein the alkyl portion is as defined in the term "alkyl" herein.

"Aminoalkylamino" refers to -N(H)(alkyl)NH₂, wherein the alkyl portion is as defined in the term "alkyl" herein.

"Arylalkylamino" refers to -N(H)(alkyl)aryl, wherein the "alkyl" and "aryl" portions are as defined in the terms "alkyl" and "aryl" herein.

"Alkylsulfonylheterocycloalkylamino" refers to -N(H)-heterocycloalkyl-S(O)₂-alkyl, wherein the amino is attached to the parent moiety, wherein the "alkyl" and "heterocycloalkyl" portions are as defined herein.

"Cycloalkylalkylamino" refers to -N(H)-alkylcycloalkyl, wherein the amino is attached to the parent moiety, and wherein the "alkyl" and "cycloalkyl" portions are as defined herein.

"Dialkylaminoalkoxy" refers to -(alkoxy)N(alkyl)₂, wherein the "alkoxy" and "alkyl" portions are as defined herein. One such non-limiting example of "dialkylaminoalkoxy" includes dimethylaminoethyloxy represented by -O-(CH₂)₂N(CH₃)₂.

"Alkylsulfonylalkylamino" refers to -NH₂-alkyl-S(O)₂-alkyl, wherein the amino portion of this group is attached to the parent moieity, and wherein the "alkyl" portions are as defined above. A non-limiting example includes methylsulfonylethylamino.

"Aminocarbonylalkylaminoalkyl" refers to the group "-alkyl-N(H)-alkyl-C(O)-NH₂" wherein the parent moiety is attached to the alkyl group, and wherein the alkyl portions are as defined herein.

"Aminoalkylaminoalkyl" refers to the group "-alkyl-N(H)-alkyl-NH₂" wherein the parent moiety is attached to the alkyl group, and wherein the alkyl portions are as defined herein.

"Dialkylaminoalkylaminoalkyl" refers to the group -alkyl-N(H)-alkyl-N(alkyl)₂, wherein the parent moiety is attached to the alkyl group, and wherein the "alkyl" portions are as defined herein.

"Carboxyalkylaminoalkyl" refers to the group -alkyl-N(H)-alkyl-C(O)OH, wherein the parent moiety is attached to the alkyl group, and wherein the "alkyl" portions are as defined herein.

"Cycloakylaminoalkyl" refers to the group -alkyl-N(H)-alkyl-cycloalkyl, wherein the parent moiety is attached to the alkyl group, and wherein the "alkyl" and cycloalkyl portions are as defined herein.

"Dialkylaminoarylalkylaminoalkyl" refers to the group -alkyl-N(H)-alkylaryl-N(alkyl)₂, wherein the parent moiety is attached to the alkyl group, and wherein the "alkyl" and "aryl" portions are as defined herein.

"Heteroarylalkylaminoalkyl" refers to the group -alkyl-N(H)-alkyl-heteroaryl, wherein the parent moiety is attached to the alkyl group, and wherein the "alkyl" and "heteroaryl" portions are as defined herein.

"Heterocycloalkylarylalkylaminoalkyl" refers to the group -alkyl-N(H)-alkyl-aryl-heterocycloalkyl, wherein the parent moiety is attached to the alkyl group, and wherein the "alkyl" and "heterocycloalkyl" portions are as defined herein.

"Alkoxyalkylaminoalkyl" refers to the group -alkyl-N(H)-alkyl-O-alkyl, wherein the parent moiety is attached to the alkyl group, and wherein the "alkyl" portions are as defined herein.

"Hydroxymethylalkynyl" refers to the group -alkynyl-CH₂-OH, wherein the parent moiety is attached to the alkynyl group, and wherein the "alkynyl" portion is as defined herein.

"Heteroarylalkyl" refers to the group -alkyl-heteroaryl, wherein the parent moiety is attached to the alkyl group, and wherein the "alkyl" and heteroaryl portions are as defined herein.

"Heterocycloalkylaminoalkyl" refers to the group -alkyl-N(H)-alkyl-heterocylcoalkyl, wherein the parent moiety is attached to the alkyl group, and wherein the "alkyl" and "heterocycloalkyl" portions are as defined herein.

"Cycloalkylaminoalkyl" refers to the group -alkyl-N(H)-cycloalkyl, wherein the parent moiety is attached to the alkyl group, and wherein the "alkyl" and "cycloalkyl" portions are as defined herein.

"Heterocycloalkylalkylaminoalkyl" refers to the group -alkyl-N(H)-alkyl-heterocycloalkyl, wherein the parent moiety is attached to the alkyl group, and wherein the "alkyl" and "heterocycloalkyl" portions are as defined herein.

"Arylalkylaminoalkyl" refers to the group -alkyl-N(H)-alkyl-aryl, wherein the parent moiety is attached to the alkyl group, and wherein the "alkyl" and "aryl" portions are as defined herein.

"Alkylheterocycloalkyl" refers to the group -alkyl-heterocycoalkyl, wherein the parent moiety is attached to the alkyl group, and wherein the "alkyl" and "heterocycloalkyl" portions are as defined herein.

"Heteroarylaminoalkyl" refers to the group -alkyl-N(H)-heteroaryl, wherein the parent moiety is attached to the alkyl group, and wherein the "alkyl" and "heteroaryl" portions are as defined herein.

"Arylamino" refers to the group -N(H)-aryl, wherein the parent moiety is attached to the amino group, and wherein the "aryl" portion is as defined herein.

"Aryloxyalkyl" refers to the group -alkyl-O-aryl, wherein the parent moiety is attached to the alkyl group, and wherein the "alkyl" and "aryl" portions are as defined herein.

"Heteroarylalkyl" refers to the group -alkyl-heteroaryl, wherein the parent moiety is attached to the alkyl group, and wherein the " alkyl" and "heteroaryl" portions are as defined herein.

Alkylpiperazinylcarbonyl" refers to the group -C(O)-piperazinyl-alkyl, wherein the parent moiety is attached to the carbonyl group, and wherein the "alkyl" portion is as defined herein.

"Alkylheterocycloakylarylalkoxyalkyl" refers to the group -alkyl-O-alkyl-aryl-heterocycloalkyl-alkyl, wherein the parent moiety is attached to the alkyl group, and wherein the "aryl," "aryl" and "heterocycloalkyl" portions are as defined herein.

"Alkylheteroarylalkoxyalkyl" refers to the group -alkyl-O-alkyl-heteroaryl-alkyl, wherein the parent moiety is attached to the alkyl group, and wherein the "aryl" and "heteroaryl" portions are as defined herein.

"Dialkylaminoalkylamino" refers to the group -N(H)-alkyl-N(alkyl)₂, wherein the parent moiety is attached to the amino group, and wherein the "alkyl" portions are as defined herein.

"Heterocycloalkylallcylamino" refers to the group -N(H)-alkyl-heterocycloalkyl, wherein the parent moiety is attached to the amino group, and wherein the "alkyl" and "heterocycloalkyl" portions are as defined herein.

"Heteroarylamino" refers to the group -N(H)-heteroaryl, wherein the parent moiety is attached to the amino group, and wherein the "heteroaryl" portion is as defined herein.

"Heterocycloalkylalkoxyalkyl" refers to the group -alkyl-O-alkyl-heterocycloalkyl, wherein the parent moiety is attached to the alkyl group, and wherein the "alkyl" and heterocycloalkyl portions are as defined herein.

"Carboxyalkyl" refers to the group -alkyl-C(O)-OH, wherein the parent moiety is attached to the alkyl group, and wherein the "alkyl" portion is as defined herein.

"Heterocycloalkyloxyalkyl" refers to the group -alkyl-O-heterocycloalkyl, wherein the parent moiety is attached to the alkyl group, and wherein the "alkyl" and "heterocycloalkyl" portions are as defined herein.

"Alkoxycarbonylalkyl" refers to the group -alkyl-C(O)-O-alkyl, wherein the parent moiety is attached to the alkyl group, and wherein the "alkyl" portions are as defined herein.

"Arylalkoxy" refers to the group -alkyl-O-aryl, wherein the parent moiety is attached to the alkyl group, and wherein the "alkyl" and "aryl" portions are as defined herein.

"Spiro-heterocycloalkyl" refers to the group: 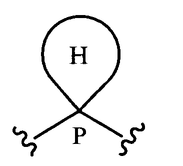 wherein H represents a heterocycloalkyl group and P represents the parent moiety, wherein the heterocycloalkyl portion is as defined herein.

"Arylalkylaminocarbonyl" refers to the group -C(O)-N(H)-alkyl-aryl, wherein the parent moiety is attached to the carbonyl group, and wherein the "alkyl" and "aryl" portions are as defined herein.

"Heterocycloalkylalkylamino" refers to the group -N(H)-alkyl-heterocycloalkyl, wherein the parent moiety is attached to the amino group, and wherein the "alkyl" and "heterocycloalkyl" portions are as defined herein.

"Dialkylaminoalkylcarbonyl" refers to the group -C(O)-alkyl-N(alkyl)₂, wherein the parent moiety is attached to the carbonyl group, and wherein the "alkyl" portions are as defined herein.

"Dialkylaminocarbonylalkyl" refers to the group -alkyl-C(O)-N(alkyl)₂, wherein the parent moiety is attached to the alkyl group, and wherein the "alkyl" portions are as defined herein.

"Heterocycloalkylcarbonyl" refers to the group -C(O)-heterocycloalkyl, wherein the parent moiety is attached to the carbonyl group, and wherein the "heterocycloalkyl" portion is as defined herein.

"Alkylthio" refers to the group -S-alkyl, wherein the parent moiety is attached to the thio group (-S-), and wherein the "alkyl" portion is as defined herein.

"Alkylsulfonyl" refers to the group -S(O)₂-alkyl, wherein the parent moiety is attached to the sulfonyl group [-S(O)₂-], and wherein the "alkyl" portion is as defined herein

"Alkylheterocycloalkyl" refers to the group -alkyl-heterocycloalkyl, wherein the parent moiety is attached to the alkyl group, and wherein the "alkyl" and "heterocycloalkyl" portions are as defined herein.

"Alkoxycarbonylheterocycloalkylaminoalkyl" refers to the group -alkyl-N(H)-heterocycloalkyl-C(O)-O-alkyl, wherein the parent moiety is attached to the alkyl group, and wherein the "alkyl" and "heterocycloalkyl" portions are as defined herein.

"Heterocycloalkylaminoalkyl" refers to the group -alkyl-N(H)-heterocycloalkyl, wherein the parent moiety is attached to the alkyl group, and wherein the "alkyl" and "heterocycloalkyl" portions are as defined herein.

"Aminoalkylaminocarbonyl" refers to the group -N(H)-alkyl-heterocycloalkyl, wherein the parent moiety is attached to the amino group, and wherein the "alkyl" and "heterocycloalkyl" portions are as defined herein.

"Heteroarylcarbonyl" refers to the group -C(O)-heteroaryl, wherein the parent moiety is attached to the carbonyl group, and wherein the "heteroaryl" portion is defined herein.

"Arylallcylcarbonyl" refers to the goup -C(O)-alkyl-aryl, wherein the parent moiety is attached to the carbonyl goup, and wherein the "alkyl" and "aryl" portions are as defined herein.

"Arylcarbonyl" refers to the group -C(O)-aryl, wherein the parent moiety is attached to the carbonyl group, and wherein the "aryl" portion is defined herein.

'Alkylcarbonyl" refers to the group -C(O)-alkyl, wherein the parent moiety is attached to the carbonyl group, and wherein the "alkyl" portion is defined herein.

"Alkoxyalkylcarbonyl" refers to the group -C(O)-alkyl-O-alkyl, wherein the parent moiety is attached to the carbonyl group, and wherein the "alkyl" portions are as defined herein.

"Spiro-cycloalkyl" refers to the group: 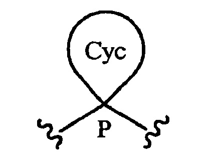 wherein Cyc represents a cycloalkyl group and P represents the parent moiety, wherein the cycloalkyl portion is as defined herein.

When a portion of term (such as the "alkyl" portion of "arylalkyl,") is referred to as being defined above or defined herein, this means that this portion has the same meaning as the definition of this term within this specification.

The phrases "the compounds in this disclosure," the compounds in the disclosure, the compounds disclosed herein, compounds of this disclosure, and similar phrases that contain both of the words "compounds" and "disclosure" are meant to mean compounds of Formula I and all of the options of Formula I described herein.

In the case where there is a point of attachment for a monovalent substituent, such as -CH₃, -NH₂, or -OH, the indication of where the point of attachment is is not necessary. That is, -CH₃ has the same meaning as CH₃, -NH₂ has the same meaning as NH₂, and -OH has the same meaning as OH.

In Table 1, where there appears to be an empty valence for oxygen or nitrogen for any of the compounds listed in this table, where the name of the structure requires that the empty valence is filled with hydrogen, it is assumed that the missing valence is filled with hydrogen for each of these cases.

When a group is referred to as "-(C₁-C₆)alkyl heterocyclyl" the heterocyclyl is attached to a parent structure via an alkyl group.

"Optional" or "optionally" means that the subsequently described event or circumstance can or can not occur, and that the description includes instances where said event or circumstance occurs and instances in which it does not. One of ordinary skill in the art would understand that with respect to any molecule described as containing one or more optional substituents, only sterically practical and/or synthetically feasible compounds are meant to be included. "Optionally substituted" means substituted or unsubstituted and refers to all subsequent modifiers in a term unless otherwise specified. So, for example, in the term "optionally substituted arylalkyl," both the "alkyl" portion and the "aryl" portion of the molecule can be substituted or unsubstituted.

Unless otherwise specified, the term "optionally substituted" applies to the chemical moiety immediately preceding it. For instance, if a variable group (such as R) is defined as aryl, optionally substituted alkyl", or cycloalkyl, then only the alkyl group is optionally substituted.

"Saturated bridged ring system" refers to a bicyclic or polycyclic ring system that is not aromatic. Such a system can contain isolated or conjugated unsaturation, but not aromatic or heteroaromatic rings in its core structure (but can have aromatic substitution thereon). For example, hexahydro-furo[3,2-b]furan, 2,3,3a,4,7,7a-hexahydro- H-indene, 7-aza-bicyclo[2.2.1]-heptane, and 1,2,3,4,4a,5,8,8a-octahydro-naphthalene are all included in the class "saturated bridged ring system.

"Spirocyclyl" or "spirocyclic ring" refers to a ring originating from a particular annular carbon of another ring. For example, as depicted below, a ring atom of a saturated bridged ring system (rings B and B'), but not a bridgehead atom, can be a shared atom between the saturated bridged ring system and a spirocyclyl (ring A) attached thereto. A spirocyclyl can be carbocyclic or heteroalicyclic.

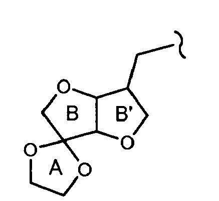

Some of the compounds of the disclosure can have imino, amino, oxo or hydroxy substituents off aromatic heterocyclyl systems. For purposes of this disclosure, it is understood that such imino, amino, oxo or hydroxy substituents can exist in their corresponding tautomeric form, *i.e.*, amino, imino, hydroxy or oxo, respectively.

"Animal" for the purposes of this disclosure includes humans (including patients receiving treatment) and other animals, particularly mammals, and other organisms. Thus, the methods are applicable to both human therapy and veterinary applications. In a preferred embodiment the patient is a mammal, and in a most preferred embodiment the patient is human.

"Kinase-dependent diseases or conditions" refer to pathologic conditions that depend on the activity of one or more protein kinases. Kinases either directly or indirectly participate in the signal transduction pathways of a variety of cellular activities including proliferation, adhesion, migration, differentiation and invasion. Diseases associated with kinase activities include tumor growth, the pathologic neovascularization that supports solid tumor growth, and associated with other diseases where excessive local vascularization is involved such as ocular diseases (diabetic retinopathy, age-related macular degeneration, and the like) and inflammation (psoriasis, rheumatoid arthritis, and the like).

While not wishing to be bound to theory, phosphatases can also play a role in "kinase-dependent diseases or conditions" as cognates of kinases; that is, kinases phosphorylate and phosphatases dephosphorylate, for example protein substrates. Therefore compounds of this disclosure, while modulating kinase activity as described herein, can also modulate, either directly or indirectly, phosphatase activity. This additional modulation, if present, can be synergistic (or not) to activity of compounds of this disclosure toward a related or otherwise interdependent kinase or kinase family. In any case, as stated previously, the compounds of this disclosure are useful for treating diseases characterized in part by abnormal levels of cell proliferation (*i.e*. tumor growth), programmed cell death (apoptosis), cell migration and invasion and angiogenesis associated with tumor growth.

"Therapeutically effective amount" is an amount of a compound of this disclosure, that when administered to a patient, ameliorates a symptom of the disease. The amount of a compound of this disclosure which constitutes a "therapeutically effective amount" will vary depending on the compound, the disease state and its severity, the age of the patient to be treated, and the like. The therapeutically effective amount can be determined routinely by one of ordinary skill in the art having regard to their knowledge and to this disclosure.

"Cancer" as referred to herein refers to cellular-proliferative disease states, including but not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinorna, glucagonoma, gastrinoma, carcinoid tumors, vipoma), small bowel (adenocarcinorna, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostrate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; and Adrenal lands: neuroblastoma. Thus, the term "cancerous cell" as provided herein, includes a cell afflicted by any one of the above-identified conditions.

A "pharmaceutically acceptable salt" of a compound means a salt that is pharmaceutically acceptable and that possesses the desired pharmacological activity of the parent compound. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985 or S. M. Berge, et al., "Pharmaceutical Salts," J. Pharm. Sci., 1977;66:1-19.

Examples of pharmaceutically acceptable acid addition salts include those formed with inorganic acids such as hydrochloric acid, hydrobromic acid, sulfuric acid, nitric acid, phosphoric acid, and the like; as well as organic acids such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, tartaric acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, p-toluenesulfonic acid, and salicylic acid and the like.

Examples of a pharmaceutically acceptable base addition salts include those formed when an acidic proton present in the parent compound is replaced by a metal ion, such as sodium, potassium, lithium, ammonium, calcium, magnesium, iron, zinc, copper, manganese, aluminum salts and the like. Preferable salts are the ammonium, potassium, sodium, calcium, and magnesium salts. Salts derived from pharmaceutically acceptable organic non-toxic bases include, but are not limited to, salts of primary, secondary, and *tert*iary amines, substituted amines including naturally occurring substituted amines, cyclic amines and basic ion exchange resins. Examples of organic bases include isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, tromethamine, *N*-methylglucamine, polyamine resins, and the like. Exemplary organic bases are isopropylamine, diethylamine, ethanolamine, trimethylamine, dicyclohexylamine, choline, and caffeine.

"Prodrug" refers to compounds that are transformed (typically rapidly) *in vivo* to yield the parent compound of the above formulae, for example, by hydrolysis in blood. Common examples include, but are not limited to, ester and amide forms of a compound having an active form bearing a carboxylic acid moiety. Examples of pharmaceutically acceptable esters of the compounds of this disclosure include, but are not limited to, alkyl esters (for example with between about one and about six carbons) the alkyl group is a straight or branched chain. Acceptable esters also include cycloalkyl esters and arylalkyl esters such as, but not limited to benzyl. Examples of pharmaceutically acceptable amides of the compounds of this disclosure include, but are not limited to, primary amides, and secondary and tertiary alkyl amides (for example with between about one and about six carbons). Amides and esters of the compounds of this disclosure can be prepared according to conventional methods. A thorough discussion of prodrugs is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

"Metabolite" refers to the break-down or end product of a compound or its salt produced by metabolism or biotransformation in the animal or human body; for example, biotransformation to a more polar molecule such as by oxidation, reduction, or hydrolysis, or to a conjugate (see Goodman and Gilman, "The Pharmacological Basis of Therapeutics" 8.sup.th Ed., Pergamon Press, Gilman et al.. (eds), 1990 for a discussion of biotransformation). As used herein, the metabolite of a compound of this disclosure or its salt can be the biologically active form of the compound in the body. In one example, a prodrug can be used such that the biologically active form, a metabolite, is released *in vivo.* In another example, a biologically active metabolite is discovered serendipitously, that is, no prodrug design *per se* was undertaken. An assay for activity of a metabolite of a compound of this disclosure is known to one of skill in the art in light of the present disclosure.

The compounds of this disclosure also include N-oxide derivatives and protected derivatives of compounds of Formula I. For example, when compounds of Formula I contain an oxidizable nitrogen atom, the nitrogen atom can be converted to an N-oxide by methods well known in the art. When compounds of Formula I contain groups such as hydroxy, carboxy, thiol or any group containing a nitrogen atom(s), these groups can be protected with a suitable "protecting group" or "protective group". A comprehensive list of suitable protective groups can be found in T.W. Greene, Protective Groups in Organic Synthesis, John Wiley & Sons, Inc. 1991. The protected derivatives of compounds of Formula I can be prepared by methods well known in the art.

"Treating" or "treatment" of a disease, disorder, or syndrome, as used herein, includes (i) preventing the disease, disorder, or syndrome from occurring in a human, i.e. causing the clinical symptoms of the disease, disorder, or syndrome not to develop in an animal that can be exposed to or predisposed to the disease, disorder, or syndrome but does not yet experience or display symptoms of the disease, disorder, or syndrome; (ii) inhibiting the disease, disorder, or syndrome, *i.e*., arresting its development; and (iii) relieving the disease, disorder, or syndrome, *i.e*., causing regression of the disease, disorder, or syndrome. As is known in the art, adjustments for systemic versus localized delivery, age, body weight, general health, sex, diet, time of administration, drug interaction and the severity of the condition can be necessary, and will be ascertainable with routine experimentation by one of ordinary skill in the art.

One of ordinary skill in the art would understand that certain crystallized, protein-ligand complexes, in particular HSP90-ligand complexes, and their corresponding x-ray structure coordinates can be used to reveal new structural information useful for understanding the biological activity of kinases as described herein. As well, the key structural features of the aforementioned proteins, particularly, the shape of the ligand binding site, are useful in methods for designing or identifying selective modulators of kinases and in solving the structures of other proteins with similar features. Such protein-ligand complexes, having compounds of this disclosure as their ligand component, are an aspect of this disclosure.

As well, one of ordinary skill in the art would appreciate that such suitable x-ray quality crystals can be used as part of a method of identifying a candidate agent capable of binding to and modulating the activity of kinases. Such methods can be characterized by the following aspects: a) introducing into a suitable computer program, information defining a ligand binding domain of a kinase in a conformation (*e.g*. as defined by x-ray structure coordinates obtained from suitable x-ray quality crystals as described above) wherein the computer program creates a model of the three dimensional structures of the ligand binding domain, b) introducing a model of the three dimensional structure of a candidate agent in the computer program, c) superimposing the model of the candidate agent on the model of the ligand binding domain, and d) assessing whether the candidate agent model fits spatially into the ligand binding domain. Aspects a-d are not necessarily carried out in the aforementioned order. Such methods can further entail: performing rational drug design with the model of the three-dimensional structure, and selecting a potential candidate agent in conjunction with computer modeling.

Additionally, one skilled in the art would appreciate that such methods can further entail: employing a candidate agent, so-determined to fit spatially into the ligand binding domain, in a biological activity assay for kinase modulation, and determining whether said candidate agent modulates kinase activity in the assay. Such methods can also include administering the candidate agent, determined to modulate kinase activity, to a mammal suffering from a condition treatable by kinase modulation, such as those described above.

Also, one skilled in the art would appreciate that compounds disclosed herein can be used in a method of evaluating the ability of a test agent to associate with a molecule or molecular complex comprising a ligand binding domain of a kinase. Such a method can be characterized by the following aspects: a) creating a computer model of a kinase binding pocket using structure coordinates obtained from suitable x-ray quality crystals of the kinase, b) employing computational algorithms to perform a fitting operation between the test agent and the computer model of the binding pocket, and c) analyzing the results of the fitting operation to quantify the association between the test agent and the computer model of the binding pocket.

### General Information on Administration

Administration can preferably be by the oral route. Administration of the compounds of this disclosure, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally (intravenous, intramuscular, or subcutaneous), topically, transdermally, intravaginally, intravesically, intracistemally, or rectally, in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages.

The compositions will include a conventional pharmaceutical carrier or excipient and a compound of this disclosure as the/an active agent, and, in addition, can include carriers and adjuvants, etc.

Adjuvants include preserving, wetting, suspending, sweetening, flavoring, perfuming, emulsifying, and dispensing agents. Prevention of the action of microorganisms can be ensured by various antibacterial and antifungal agents, for example, parabens, chlorobutanol, phenol, sorbic acid, and the like. It can also be desirable to include isotonic agents, for example sugars, sodium chloride, and the like. Prolonged absorption of the injectable pharmaceutical form can be brought about by the use of agents delaying absorption, for example, aluminum monostearate and gelatin.

If desired, a pharmaceutical, composition of the compounds in this disclosure can also contain minor amounts of auxiliary substances such as wetting or emulsifying agents, pH buffering agents, antioxidants, and the like, such as, for example, citric acid, sorbitan monolaurate, triethanolamine oleate, butylalted hydroxytoluene, etc.

The choice of formulation depends on various factors such as the mode of drug administration (e.g., for oral administration, formulations in the form of tablets, pills or capsules are preferred) and the bioavailability of the drug substance. Recently, pharmaceutical formulations have been developed especially for drugs that show poor bioavailability based upon the principle that bioavailability can be increased by increasing the surface area i.e., decreasing particle size. For example, U.S. Pat. No. 4,107,288 describes a pharmaceutical formulation having particles in the size range from 10 to 1,000 nm in which the active material is supported on a crosslinked matrix of macromolecules. U.S. Pat. No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

Compositions suitable for parenteral injection can comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

One preferable route of administration is oral, using a convenient daily dosage regimen that can be adjusted according to the degree of severity of the disease-state to be treated.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, cellulose derivatives, starch; alignates, gelatin, polyvinylpyrrolidone, sucrose, and gum acacia, (c) humectants, as for example, glycerol, (d) disintegrating agents, as for example, agar-agar, calcium carbonate, potato or tapioca starch, alginic acid, croscarmellose sodium, complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, magnesium stearate and the like (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms can also comprise buffering agents.

Solid dosage forms, as described above, can be prepared with coatings and shells, such as enteric coatings and others well known in the art. They can contain pacifying agents, and can also be of such composition that they release the active compound or compounds in a certain part of the intestinal tract in a delayed manner. Examples of embedded compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups, and elixirs. Such dosage forms are prepared, for example, by dissolving, dispersing, etc., a compound(s) of this disclosure, or a pharmaceutically acceptable salt thereof, and optional pharmaceutical adjuvants in a carrier, such as, for example, water, saline, aqueous dextrose, glycerol, ethanol and the like; solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide; oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan; or mixtures of these substances, and the like, to thereby form a solution or suspension.

Suspensions, in addition to the active compounds, can contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are, for example, suppositories that can be prepared by mixing the compounds of this disclosure with, for example, suitable non-irritating excipients or carriers such as cocoa butter, polyethyleneglycol or a suppository wax, which are solid at ordinary temperatures but liquid at body temperature and therefore, melt while in a suitable body cavity and release the active component therein.

Dosage forms for topical administration of a compound of this disclosure include ointments, powders, sprays, and inhalants. The active component is admixed under sterile conditions with a physiologically acceptable carrier and any preservatives, buffers, or propellants as can be required. Ophthalmic formulations, eye ointments, powders, and solutions are also contemplated for the comounds in this disclosure.

Compressed gases can be used to disperse a compound of this disclosure in aerosol form. Inert gases suitable for this purpose are nitrogen, carbon dioxide, etc.

Generally, depending on the intended mode of administration, the pharmaceutically acceptable compositions will contain about 1% to about 99% by weight of a compound(s) of this disclosure, or a pharmaceutically acceptable salt thereof, and 99% to 1% by weight of a suitable pharmaceutical excipient. In one example, the composition will be between about 5% and about 75% by weight of a compound(s) of this disclosure, or a pharmaceutically acceptable salt thereof, with the rest being suitable pharmaceutical excipients.

Actual methods of preparing such dosage forms are known, or will be apparent, to those skilled in this art; for example, see Remington's Pharmaceutical Sciences, 18th Ed., (Mack Publishing Company, Easton, Pa., 1990). The composition to be administered will, in any event, contain a therapeutically effective amount of a compound of this disclosure, or a pharmaceutically acceptable salt thereof, for treatment of a disease-state in accordance with the teachings of this disclosure.

The compounds of this disclosure, or their pharmaceutically acceptable salts, can be administered in a therapeutically effective amount which will vary depending upon a variety of factors including the activity of the specific compound employed, the metabolic stability and length of action of the compound, the age, body weight, general health, sex, diet, mode and time of administration, rate of excretion, drug combination, the severity of the particular disease-states, and the host undergoing therapy. The compounds of this disclosure can be administered to a patient at dosage levels in the range of about 0.1 to about 1,000 mg per day. For a normal human adult having a body weight of about 70 kilograms, a dosage in the range of about 0.01 to about 100 mg per kilogram of body weight per day is an example. The specific dosage used, however, can vary. For example, the dosage can depend on a number of factors including the requirements of the patient, the severity of the condition being treated, and the pharmacological activity of the compound being used. The determination of optimum dosages for a particular patient is well known to one of ordinary skill in the art.

The compositions will include a conventional pharmaceutical carrier or excipient and a compound of this disclosure as the/an active agent, and, in addition, can include other medicinal agents and pharmaceutical agents. Compositions of the compounds in this disclosure can be used in combination with anticancer and/or other agents that are generally administered to a patient being treated for cancer, e.g. surgery, radiation and/or chemotherapeutic agent(s). Chemotherapeutic agents that can be useful for administration in combination with compounds of Formula I in treating cancer include alkylating agents, platinum containing agents.

If formulated as a fixed dose, such combination products employ the compounds of this disclosure within the dosage range described above and the other pharmaceutically active agent(s) within its approved dosage range. Compounds of this disclosure can alternatively be used sequentially with known pharmaceutically acceptable agent(s) when a combination formulation is inappropriate.

Representative pharmaceutical formulations containing the compounds disclosed herein are described below.

### Synthetic Procedures

The compounds disclosed herein, or their pharmaceutically acceptable salts, can have asymmetric carbon atoms, oxidized sulfur atoms or quaternized nitrogen atoms in their structure.

The compounds disclosed herein and their pharmaceutically acceptable salts can exist as single stereoisomers, racemates, and as mixtures of enantiomers and diastereomers. The compounds disclosed herein can also exist as geometric isomers. All such single stereoisomers, racemates and mixtures thereof, and geometric isomers are intended to be within the scope of the compounds disclosed herein.

It is assumed that when considering generic descriptions of compounds of the disclosed herein for the purpose of constructing a compound, such construction results in the creation of a stable structure. That is, one of ordinary skill in the art would recognize that theoretically some constructs which would not normally be considered as stable compounds (that is, sterically practical and/or synthetically feasible, *supra*).

Methods for the preparation and/or separation and isolation of single stereoisomers from racemic mixtures or non-racemic mixtures of stereoisomers are well known in the art. For example, optically active (R)- and (S)- isomers can be prepared using chiral synthons or chiral reagents, or resolved using conventional techniques. Enantioiners (R- and S-isomers) can be resolved by methods known to one of ordinary skill in the art, for example by: formation of diastereoisomeric salts or complexes which can be separated, for example, by crystallization; via formation of diastereoisomeric derivatives which can be separated, for example, by crystallization, selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. It will be appreciated that where a desired enantiomer is converted into another chemical entity by one of the separation procedures described above, a further step can be required to liberate the desired enantiomeric form. Alternatively, specific enantiomer can be synthesized by asymmetric synthesis using optically active reagents, substrates, catalysts or solvents or by converting on enantiomer to the other by asymmetric transformation. For a mixture of enantiomers, enriched in a particular enantiomer, the major component enantiomer can be further enriched (with concomitant loss in yield) by recrystallization.

In addition, the compounds of this disclosure can exist in unsolvated as well as solvated forms with pharmaceutically acceptable solvents such as water, ethanol, and the like. In general, the solvated forms are considered equivalent to the unsolvated forms for the purposes of the compounds of this disclosure.

In addition, it is intended that the present disclosure cover compounds made either using standard organic synthetic techniques, including combinatorial chemistry or by biological methods, such as bacterial digestion, metabolism, enzymatic conversion, and the like.

The examples and scheme below depict the general synthetic procedure for the compounds disclosed herein. Synthesis of the compounds disclosed herein is not limited by these examples and schemes. One skilled in the art will know that other procedures can be used to synthesize the compounds disclosed herein, and that the procedures described in the examples and schemes is only one such procedure. In the descriptions below, one of ordinary skill in the art would recognize that specific reaction conditions, added reagents, solvents, and reaction temperatures can be modified for the synthesis of specific compounds that fall within the scope of this disclosure. All intermediate compounds described below, for which there is no description of how to synthesize such intermediates within these examples below, are commercially available compounds unless otherwise specified.

### EXAMPLES

### Instrumentation

IR spectra were collected by reflectance on a Perkin Elmer Spectrum^{™} 100 FT-IR. ¹H NMR were collected on a Varian 400 MHz with Mercury and Mercury consoles.

All variables in the Examples below (e.g., R₃ₐ, R₁₅, etc.) are described within the compounds within each of these examples unless otherwise specified. The variables in the examples below are not intended to change or limit the scope of the disclosure above or in the claims. When the variables within the examples below correspond to the same variables within the disclosure above or the claims (for instance, R₃ₐ), this does not intend to alter or change the meaning of the variables in this disclosure or in the claims. Compounds marked with an asterisk ("*") are not specifically recited in the appended claims but are useful for general information.

### EXAMPLE 1: (Scheme 1)

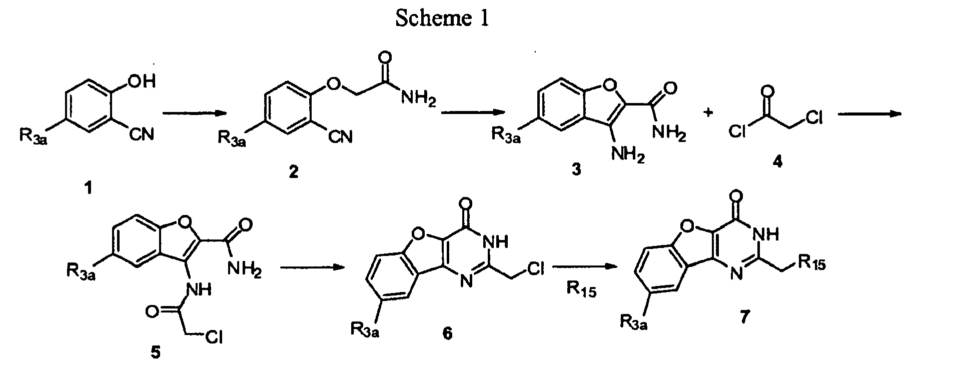

wherein R₃ₐ is as defined in the disclosure above and R₁₅ is described within the compounds within this example.

### 2-(4-bromo-2-cyanophenoxylacetamide 2

To a solution of 5-bromo-2-hydroxybenzonitrile **1** (10 g, 50.5 mmol) in 50 mL acetone was added potassium carbonate (13.8 g, 100 mmol) and 2-bromoacetamide (6.9 g, 50.5 mmol). The reaction mixture was heated to 60 °C for 18 hours. After cooling, the solids were filtered off and dissolved in a large excess of water (1000 mL). The white solid was collected and dried to afford 11.2 g (89%) of 2-(4-bromo-2-cyanophenoxy)acetamide **2** as a white solid. ¹H NMR (400 MHz, d₆-DMSO): 8.03 (d, 1H), 7.83 (dd, 1H), 7.53 (br s, 1 H), 7.45 (br s, 1H), 7.03 (d, 1H), 4.7 (s, 2H).

### 3-amino-5-bromobenzofuran-2-carboxamide 3

To a solution of KOH (8.0 g, 142 mmol) in 200 mL ethanol was added 2-(4-bromo-2-cyanophenoxy)acetamide **2** (11.2 g, 44 mmol) and heated to 80 °C for an hour. After cooling down to room temperature, the reaction mixture was poured into a large excess of water and the resultant solids were filtered off and dried to afford 8.9 g (79%) of 3-amino-5-bromobenzofuran-2-carboxamide 3 as a white solid. ¹H NMR (400 MHz, d₆-DMSO): 8.11 (d, 1H), 7.56 (dd, 1 H), 7.40 (d, 1H), 7.34 (br s, 2H), 6.04 (s, 2H).

### 5-bromo-3-(2-chloroacetamido)benzofuran-2-carboxamide 5

A solution of 3-amino-5-bromobenzofuran-2-carboxamide **3** (1.7 g, 6.72 mmol) in chloroacetyl chloride (10 mL) was heated to 40 °C for 30 minutes. The reaction mixture was quenched with saturated aqueous NaHCO₃ (100 mL) and extracted with ethyl acetate (2 x 150 mL). The combined organic phases were washed with brine, dried over Na₂SO₄ and concentrated *in vacuo* to afford 2.03 g (90%) of 5-bromo-3-(2-chloroacetamido)benzofuran-2-carboxamide **5** as a white solid. ¹H NMR (400 MHz, d₆-DMSO): 10.75 (s, 1H), 8.25 (br s, 1H), 8.23 (d, 1H), 8.0 (br s, 1H), 7.66 (dd, 1H), 7.59 (dd, 1H), 4.5 (s, 2H); MS (EI) for C₁₁H₈BrClN₂O₃: 331 (MH⁺).

### 8-bromo-2-(chloromethyl)benzofuro[3,2-d]pyrimidin-4(3H)-one 6

A solution of 5-bromo-3-(2-chloroacetamido)benzofuran-2-carboxamide **5** (2.0 g, 6.05 mmol) in 30 mL of 2N NaOH was heated to 40 °C for 20 minutes. The reaction mixture was brought to neutral pH with 1N HCl and extracted with ethyl acetate (3 x 50 mL). The combined organic phases were washed with brine, dried over Na₂SO₄ and concentrated *in vacuo.* The product was purified by SiO₂ flash chromatography (50:50 hexanes/ethyl acetate to 100% ethyl acetate) to afford 8-bromo-2-(chloromethyl)benzofuro[3,2-d]pyrimidin-4(3H)-one **6** (1.3 g, 63%) as a yellowish solid. ¹H NMR (400 MHz, d₆-DMSO): 13.4 (s, 1H), 8.23 (m, 1H), 7.85 (m, 2H), 4.66 (s, 2H); MS (EI) for C₁₁H₆BrClN₂O₂: 313 (MH⁺).

### (Compound 2)

8-bromo-2-(piperidin-1-1-yl)[1]benzofuro[3,2-d]pyrimidin-4(3H)-one To a solution of 8-bromo-2-(chloromethyl)[1]benzofuro[3,2-d]pyrimidin-4(3H)-one, **6**, (100 mg, 0.319 mmol) in 5 mL anhydrous ethanol was added piperidine (100 mg, 1.18 mmol). The reaction mixture was heated to 80 °C for 16 hours, cooled down and concentrated *in vacuo.* Recrystallization from hot ethanol afforded 8-bromo-2-(piperidin-1-ylmethyl)[1]benzofuro[3,2-d]pyrimidin-4(3H)-one (22 mg, 20%) as a white solid. ¹H NMR (400 MHz, d₆-DMSO): 8.21 (m, 1H), 7.81 (m, 2H), 3.50 (s, 2H), 2.50 (m, 4H, overlapped), 1.54 (m, 4H), 1.39 (m, 2H); MS (EI) for C₁₆H₁₆BrN₃O₂: 362 (MH⁺).

### (Compound 1)

### 8-bromo-2-(pyrrolidin-1-ylmethyl)benzofuro[3,2-d]pyrimidin-4(3H)-one

8-bromo-2-(pyrrolidin-1-ylmethyl)[1]benzofuro[3,2-d]pyrimidin-4(3H)-one was synthesized in a manner similar to **Example 1, Compound 2,** wherein piperidine was substituted with pyrrolidine. Purification by preparative HPLC, followed by concentration *in vacuo* and lyophilization afforded the title compound (112 mg, 50%) as a white solid. ¹H NMR (400 MHz, d₆-DMSO): 8.2 (m, 1H), 7.82 (m, 2H), 3.68 (s, 2H), 2.60 (m, 4H), 1.73 (m, 4H); MS (EI) for C₁₅H₁₄BrN₃O₂: 348 (MH⁺).

### (Compound 3)

### 8-bromo-2-f(4-methylpiperazin-1-yl)methyl][1]benzofuro[3,2-d]pyrimidin-4(3H)-one

8-bromo-2-[(4-methylpiperazin-1-yl)methyl][1]benzofuro[3,2-d]pyrimidin-4(3H)-one was synthesized in a manner similar to **Example 1, Compound 2,** wherein piperidine was substituted with N-Methylpiperazine. Purification by preparative HPLC resulting in 14 mg (18% Yield) of 8-bromo-2-[(4-methylpiperazin-1-yl)methyl][1]benzofuro[3,2-d]pyrimidin-4(3H)-one as the acetate salt. ¹ NMR (400MHz, d₆-DMSO + D₂): 8.26 (s, 1H), 7.82 (m, 2H), 7.98 (m, 1H), 4.0 (m, 4H), 3.60 (s, 2H), 2.61 (m, 4H) 2.34 (s, 3H), 1.89 (s, 2H); MS (EI) for C₁₆H₇BrN₄O₂: 377:379 (Bromine isotope MH⁺).

### (Compound 476) 9-; {[(3S)-3-hydroxypyrrolidin-1-yl]methyl}[1,3]dioxolo[4,5][1]benzo-uro3,2-d]pyrimidin-7(8H)-one

9-{[(3S)-3-hydroxypyrrolidin-1-yl]methyl}[1,3]dioxolo[4,5][1]benzofuro[3,2-d]pyrimidin-7(8H)-one was synthesized in a manner similar to **Example 1,** wherein 5-bromo-2-hydroxybenzonitrile was replaced by 5-hydroxybenzo[d][1,3]dioxole-4-carbonitrile at the start of the sequence and piperidine was substituted for S-(1)-3-pyrrolidinol in the final step. Purification of the desired compound was accomplished by preparative reverse phase chromatography to afford the title compound (¹H NMR (400 MHz, d₆-DMSO): 7.24 (AB, 2H), 6.24 (s, 2H), 4.19 (m, 1H), 3.64(m, 2H), 2.76 (m, 2H), 2.51 (m, 2H), 2.02 (m, 1H), 1.58 (m, 1H); MS (EI) for C₁₆Hₗ₅N₃O₅: 330.2 (MH⁺).

### (Compound 5)

### 8-chloro-2-(pyrrolidin-1-ylmethyl)[1]benzofuro[3,2-d]pyrimidin-4(3H)-one (HCl salt)

8-chloro-2-(pyrrolidin-1-ylmethyl)[1]benzofuro[3,2-d]pyrimidin-4(3H)-one was synthesized in a manner similar to **Example 1** wherein 8-bromo-2-(chloromethyl)benzofuro[3,2-d]pyrimidin-4(3H)-one **6** was substituted with 8-chloro-2-(chloromethyl)benzofuro[3,2-d]pyrimidin-4(3H)-one and piperidine was substituted with pyrrolidine. Purification by preparative HPLC (reverse-phase, acetonitrile/water by preparative HPLC (reverse-phase, acetonitrile/water with 10 mM ammonium acetate) and (reverse-phase, 0.1% TFA in acetonitrile/0.05% TFA in water) was followed by concentration *in vacuo* and lyophilization afforded the title compound (19.8mg, 11.7%).
¹H NMR (400 MHz, d₆-DMSO): 8.17 (d, 1H), 7.94 (d, 1H), 7.75 (d, 1H), 4.56 (s, 2H), 3.48-3.26 (m, 4H, overlapped), 2.01 (m, 4H); MS (EI) for C₁₅H₁₄ClN₃O₂: 304 (MH⁺).

### (Compound 7)

### 8-chloro-2-[(4-methylpiperazin-1-yl)methyl][1]benzofuro[3,2-d]pyrimidin-4(3H-one HCl salt)

8-chloro-2-[(4-methylpiperazin-1-yl)methyl][1]benzofuro[3,2-d]pyrimidin-4(3H)-one was synthesized in a manner similar to **Example 1, Compound 2**, wherein 8-bromo-2-(chloromethyl)benzofuro[3,2-d]pyrimidin-4(3H)-one **6** was substituted with 8-chloro-2-(chloromethyl)benzofuro[3,2-d]pyrimidin-4(3H)-one and piperidine was substituted with N-methylpiperazine. Purification by preparative HPLC (reverse-phase, acetonitrile/water with 10 mM ammonium acetate) and (reverse-phase, 0.1% TFA in acetonitrile/0.05% TFA in water) was followed by concentration *in vacuo* and lyophilization afforded the title compound (12 mg, 6.5%). ¹H NMR (400 MHz, d₆-DMSO): 8.08 (d, 1H), 7.91 (d, 1H), 7.72 (dd, 1H), 3.66 (s, 2H), 3.05 (m, 5H), 2.78 (s, 3H), 2.59 (m, 3H); MS (EI) for C₁₆H₁₇ClN₄O₂: 333 (MH⁺).

### *(Compound 11)

### 8-bromo-2-{[(2-chlorophenyl)aminolmethyl][1]benzofuro[3,2-d]pyrimidin-4(3H)-one

8-bromo-2-{[(2-chlorophenyl)amino]methyl}[1]benzofuro[3,2-d]pyrimidin-4(3H)-one was synthesized in a manner similar to **Example 1, Compound 2,** wherein piperidine was substituted with commercially available 2-Chloro-aniline. Precipitation from ethanol gave 47 mg (53% Yield) of 8-bromo-2-{[(2-chlorophenyl)amino]methyl} [1]benzofuro[3,2-d]pyrimidin-4(3H)-one. ¹H NMR (400MHz, d₆-DMSO): 13.05 (br s, 1H), 8.20 (s,1H), 7.82 (s, 2H), 7.31 (d, 1H), 7.19 (t, 1H), 6.75 (d, 1 H), 6.63 (t, 1H), 6.05 (br t, 1H), 4.41 (br d, 2H); MS (EI) for C₁₇H₁₁BrClN₃O₂:404: 406 (Bromine isotope MH⁺).

### *Compound 12)

### 8-bromo-2-{[(3-fluorophenyl)amino]methyl}[1]benzofuro[3,2-d]pyrimidin-4(3H)-one

8-bromo-2-{[(3-fluorophenyl)amino]methyl} [1]benzofuro[3,2-d]pyrimidin-4(3H)-one was synthesized in a manner similar to **Example 1**, **Compound 2,** wherein piperidine was substituted with 3-fluoroaniline. Purification by preparative HPLC, followed by concentration *in vacuo* and lyophilization afforded the title compound (9.8 mg, 8%) as a white solid. ¹H NMR (400 MHz, d₆-DMSO): 8.17 (m, 1H), 7.82 (m, 2H), 7.10 (dd, 1H), 6.49 (m, 1H), 6.35 (td, 1H), 4.32 (d, 2H)); MS (EI) for C₁₇H₁₁BrFN₃O₂: 388 (MH⁺).

### (Compound 13)

### 8-bromo-2-[(pyridin-3-ylamino)methyl][1]benzofuro[3,2-d]pyrimidin-4(3H)-one (acetate salt)

8-bromo-2-[(pyridin-3-ylamino)methyl][1]benzofuro[3,2-d]pyrimidin-4(3H)-one was synthesized in a manner similar to **Example 1, Compound 2,** wherein piperidine was substituted with 3-aminopyridine. Purification by preparative HPLC, followed by concentration *in vacuo* and lyophilization afforded the title compound (42 mg, 52%) as a white solid. ¹H NMR (400 MHz, d₆-DMSO): 8.19 (m, 1H), 8.13 (m, 1H), 7.71 (dd, 1H), 7.63 (m, 2H), 7.59 (dd, 1H), 6.62 (br s, 1H), 5.50 (s, 1H), 1.9 (s, 1H); MS (EI) for C₁₆H₁₁BrN₄O₂: 371 (MH⁺).

### *(Compound 16)

### 8-bromo-2-[(phenylamino)methyl][1]benzofuro[3,2-d]pyrimidin-4(3H)-one

8-bromo-2-[(phenylamino)methyl][1]benzofuro[3,2-d]pyrimidin4(3H)-one was synthesized in a manner similar to **Example 1, Compound 2,** wherein piperidine was substituted with aniline. Purification by preparative HPLC, followed by concentration *in vacuo* and lyophilization afforded the title compound (12 mg, 15%) as a white solid. ¹H NMR (400 MHz, d₆-DMSO): 8.19 (m, 1H), 7.82 (m, 2H), 7.11 (td, 2H), 6.67 (dd, 2H), 6.58 (t, 1H), 6.14 (m, 1H), 4.33 (d, 2H); MS (EI) for C₁₇H₁₂BrN₃O₂: 370 (MH⁺).

### *(Compound 19)

### 8-bromo-2- {[(2-fluorophenyl)amino]methyl}[1]benzofuro[3,2-d]pyrimidin-4(3H)-one

8-bromo-2- {[(2-fluorophenyl)amino]methyl} [1]benzofuro[3,2-d]pyrimidin-4(3H)-one was synthesized in a manner similar to **Example 1, Compound 2**, wherein piperidine was substituted with 2-fluoroaniline. Purification by preparative HPLC, followed by concentration *in vacuo* and lyophilization afforded the title compound (51 mg, 41%) as a white solid. ¹H NMR (400 MHz, d₆-DMSO): 8.17 (m, 1H), 7.82 (m, 2H), 7.07 (m, 1H), 6.94 (m, 1H), 6.74 (m, 1H), 6.62 (m, 1H), 6.00 (m, 1H), 4.39 (d, 2H) ; MS (EI) for C₁₇H₁₁BrFN₃O₂: 388 (MH⁺).

### (Compound 21)

### 8-bromo-2-[({[3-(dimethylamino)phenyl]methyl}amino)methyl][1]benzofuro[3,2-d]pyrimidin-4(3H)-one

8-bromo-2-[({[3-(dimethylamino)phenyl]methyl}amino)methyl][1]-benzofuro[3,2-d]pyrimidin-4(3H)-one was synthesized in a manner similar to **Example 1, Compound 2**, wherein piperidine was substituted with commercially available N-[3-(Aminomethyl)phenyl]-N,N-dimethylamine. Preparative HPLC gave 9 mg (10% Yield) of 8-bromo-2-[({[3-(dimethylamino)phenyl]methyl}amino)methyl][1]benzofuro[3,2-d]pyrimidin-4(3H)-one. ¹H NMR (400MHz, d₆-DMSO): 9.81 (br s, 2H), 8.20 (s, 1H), 7.85 (m, 2H), 7.31 (t, 1H), 7.05 (m, 1H), 6.91 (m, 2H), 4.29 (d, 2 H), 2.99 (s, 6H); MS (EI) for C₂₀H₁₉BrN₄O₂: 427:429 (Bromine isotope MH⁺).

### *(Compound 22)

### 8-bromo-2-{[(4-fluorophenyl)amino]methyl}[1]benzofuro[3,2-d]pyrimidin-4(3H)-one

8-bromo-2-{[(4-fluorophenyl)amino]methyl}[1]benzofuro[3,2-d]pyrimidin-4(3H)-one was synthesized in a manner similar to **Example 1, Compound 2,** wherein piperidine was substituted with 4-fluoroaniline. Purification by preparative HPLC, followed by concentration *in vacuo* and lyophilization afforded the title compound (9.2 mg, 7.5%) as a white solid. ¹H NMR (400 MHz, d₆-DMSO): 8.18 (m, 2H), 7.82 (m, 2H), 6.93 (m, 2H), 6.65 (m, 2H), 6.1 (m, 1H), 4.30 (d, 2H);
MS (EI) for C₁₇H₁₁BrFN₃O₂: 388 (MH⁺).

### (Compound 24)

### 8-bromo-2-(morpholin-4-ylmethyl)[1]benzofuro[3,2-d]pyrimidin-4(3H)-one

8-bromo-2-(morpholin-4-ylmethyl)[1]benzofuro[3,2-d]pyrimidin-4(3H)-one was synthesized in a manner similar to **Example 1, Compound 2,** wherein piperidine was substituted with morpholine. Purification via recrystallization from hot ethanol afforded the title compound (41 mg, 37%) as a white solid. ¹H NMR (400 MHz, d₆-DMSO): 12.73 (s, 1H), 8.2 (m, 1H), 7.81 (m, 2 H), 3.60 (m, 6H), 3.5 (s, 2H), 2.50 (m, 2H, overlapped); MS (EI) for C₁₅H₁₄BrN₃O₃: 364 (MH⁺).

### (Compound 74)

### 8-bromo-2-[({[2-(4-methylpiperazin-1-yl)phenyl]methyl}amino)methyl][1] benzofuro[3,2-d]pyrimidin-4(3H)-one

8-bromo-2-[({[2-(4-methylpiperazin-1-yl)phenyl]methyl}amino)methyl][1]-benzofuro[3,2-d]pyrimidin-4(3H)-one was synthesized in a manner similar to **Example 1, Compound 2,** wherein piperidine was substituted with (2-(4-methylpiperazin-1-yl)phenyl)methanamine. The reaction mixture was concentrated *in vacuo* and purified by preparative HPLC (reverse-phase, 0.1% TFA in acetonitrile/0.05% TFA in water). Concentration *in vacuo* and lyophilization afforded the title compound (5.6 mg, 3.6%). ¹H NMR (400 MHz, d₆-DMSO): 8.17 (m, 1H), 7.90 (m, 2H), 7.60 (m, 1H), 7.48 (m, 1H), 7.35 (m, 1H), 7.30 (m, 1H), 4.42 (m, 2H), 4.28 (m, 2H), 3.49 (m, 4H, overlapped), 3.01 (m, 4H), 2.82 (s, 3H). MS (EI) for C₂₃H₂₄BrN₅O₂: 482 (MH⁺).

### (Compound 84)

### 8-bromo-2-4{[4-(1-methylpiperidin-4-yl)piperazin-1-yl]methyl}[1]benzofuro[3,2-d]pyrimidin-4(3H)-one

8-bromo-2-{[4-(1-methylpiperidin-4-yl)piperazin-1-yl]methyl}[1]benzofuro[3,2-d]pyrimidin-4(3H)-one was synthesized in a manner similar to **Example 1, Compound 2,** wherein piperidine was substituted with 1-(1-Methylpiperidin-4-yl)piperazine. The product was precipitated from 1:1 DMSO:MeOH to afforded the title compound (90 mg, 61%) as a white solid. ¹H NMR (400 MHz, MeOD): 8.23 (d, 1H), 7.76 (m, 1H), 7.65 (d, 1H), 3.65 (s, 2H), 2.98 (m, 2H), 2.69 (br s, 4H), 2.32 (m, 4H), 2.16 (t, 2H), 1.92 (m, 2H), 1.60 (q, 2H); MS (EI) for C₂₁H₂₆BrN₅O₂: 460:462 (Bromine isoptope MH⁺).

### (Compound 133)

### 8-bromo-2-{[(3S)-3-hydroxypyrrolidin-1-yl]methyl}[1]benzofuro[3,2-d]pyrimidin-4(3H)-one

8-bromo-2-{[(3S)-3-hydroxypyrrolidin-1-yl]methyl}[1]benzofuro[3,2-d]pyrimidin-4(3H)-one was synthesized in a manner similar to **Example 1, Compound 2,** wherein piperidine was substituted with S-(1)-3-pyrrolidinol. Purification by flash chromatography afforded the title compound (69 mg, 55%). ¹H NMR (400 MHz, d₆-DMSO+ D₂O): 8.22 (s, 1H), 7.81 (s, 2H), 4.21 (m, 1H), 3.76(m, 2H), 2.86 (m, 2H), 2.61 (m, 2H), 2.08 (m, 1H), 1.65 (m, 1H); MS (EI) for C₁₅H₁₄BrN₃O₃: 364:366 (Bromine isotope MH⁺).

### (Compound 167)

### 2-(azetidin-1-ylmethyl)-8-bromo[1]benzofuro[3,2-d]pyrimidin-4(3H)-one

2-(azetidin-1-ylmethyl)-8-bromo[1]benzofuro[3,2-d]pyrimidin-4(3H)-one was synthesized in a manner similar to **Example 1, Compound 2**, wherein piperidine was substituted with azetidine. The reaction mixture was concentrated *in vacuo* and purified by preparative HPLC. Concentration *in vacuo* and lyophilization afforded the title compound (25 mg, 29%). ¹H NMR (400 MHz, d₆-DMSO): 8.18 (m, 1H), 7.80 (m, 2H), 3.65 (s, 2H), 3.38 (t, 4H), 2.05 (q, 2H). MS (EI) for C₁₄H₁₂BrN₃O₂: 334 (MH⁺).

### (Compound 191)

### 8-bromo-2-{[(1,1-dimethyl-2-piperidin-1-ylethyl)amino]methyl}[1]benzofuro[3,2-d]pyrimidin-4(3H)-one

8-bromo-2- {[(1,1-dimethyl-2-piperidin-1-ylethyl)amino]methyl}[1]-benzofuro[3,2-d]pyrimidin-4(3H)-one was synthesized in a manner similar to **Example 1, Compound 2,** wherein piperidine was substituted with 1,1-Dimethyl-2-piperidin-1-ylethyl)amine. Purification by preparative HPLC, followed by concentration *in vacuo* and lyophilization afforded the title compound (13 mg, 5%). ¹H NMR (400 MHz, d₆-DMSO): 8.10 (d, 1H), 7.77 (m, 2H), 3.84 (s, 2H), 2.33 (s, 2H), 1.58 (m, 4H), 1.38 (br s, 2H), 1.07 (br s,6H); MS (EI) for C₂₀H₂₅BrN₄O₂: 433:435 (Bromine isoptope MH⁺).

### (Compound 212)

### 8-bromo[-2-({[1,1-dimethyl-2-(4-methylpiperazin-1-yl)ethyl]amino}methyl)[1]benzofuro[3,2-d]pyrimidin-4(3H)-one

8-bromo-2-({[1,1-dimethyl-2-(4-methylpiperazin-1-yl)ethyl]amino}methyl)[1]benzofuro[3,2-d]pyrimidin-4(3H)-one was synthesized in a manner similar to **Example 1, Compound 2,** wherein piperidine was substituted with 2-Methyl-1-(4-methylpiperazine-1-yl)propane-2-amine. Purification by preparative HPLC, followed by concentration *in vacuo* and lyophilization afforded the title compound (68 mg, 25%). ¹H NMR (400 MHz, d₆-DMSO): 8.09 (d, 1H), 7.65 (m, 2H), 4.37 (s, 2H), 3.70 (br s, 2H), 3.42 (m, 2H), 3.14 (s, 3H), 1.67 (s,3H), 1.13 (s, 6H); MS (EI) for C₂₀H₂₆BrN₅O₂: 448:450 (Bromine isoptope MH⁺).

### (Compound 247)

### 8-bromo-2-{[4-(2-hydroxyethyl)piperazin-1-yl]methyl}[1]benzofuro[3,2-d]pyrimidin-4(3H)-one

8-bromo-2-{[4-(2-hydroxyethyl)piperazin-1-yl]methyl}[1]benzofuro[3,2-d]pyrimidin-4(3H)-one was synthesized in a manner similar to **Example 1, Compound 2,** wherein piperidine was substituted with N-(2-Hydroxyethyl)piperazine. Precipitation from EtOH, followed by rinsing with MeOH afforded the title compound (38 mg, 30%). ¹H NMR (400 MHz, d₆-DMSO): 8.21 (m, 1H), 7.83 (m, 2H), 3.51(s, 2H), 3.47 (t, 2H), 2.39 (m, 2H), 2.38 (t, 2H); MS (EI) for C₁₇H₁₉BrN₄O₃: 407:409 (Bromine isoptope MH⁺).

### *(Compound 51)

### 8-bromo-2-(2-hydroxyphenyl)[1]benzofuro[3,2-d]pyrimidin-4(3H)-one

The title compound was synthesized in a manner similar to **Example 1.** A solution of 3-amino-5-bromobenzofuran-2-carboxamide **3** (0.50g, 0.196 mmol) and 2-hydroxybenzaldehyde (3.92 mmol) in 6 mL anhydrous ethanol were combined and stirred at room temperature for 10 minutes. The resulting suspension was treated with concentrated hydrochloric acid (40 µL) and a precipitate formed immediately. The resulting slurry was diluted with additional anhydrous ethanol (10 ml) and the resulting slurry was heated at 80 °C for 16 hours. The resulting precipitate was filtered off and washed with ethyl acetate (2 x 50 ml) and methanol (2 x 5 ml) to give 8-bromo-2-(2-hydroxyphenyl)[1]benzofuro[3,2-d]pyrimidin-4(3H)-one. ¹H NMR (400 MHz, d₆-DMSO): 8.38 (s, 1H), 8.17 (dd, 1H), 7.88 (m, 2H), 7.42 (t, 1H), 7.0 (m, 1H); MS (EI) for C₁₆H₉BrlN₂O₃: 357 (MH⁺).

### (Compound 204)

### 2-{[(3S)-3-hydroxypyrrolidin-1-yl]methy}[1]benzofuro[3,2-d]pyrimidin-4(3H)-one

2-{[(3S)-3-hydroxypyrrolidin-1-yl]methyl}[1]benzofuro[3,2-d]pyrimidin-4(3H)-one was synthesized in a manner similar to **Example 1, Compound 2,** wherein 8-bromo-2-(chloromethyl)benzofuro[3,2-d]pyrimidin-4(3H)-one **6** was substituted with 2-(chloromethyl)benzofuro[3,2-d]pyrimidin-4(3H)-one and piperidine was substituted with S-(-)-3-hydroxypyrrolidine. Purification by preparative HPLC resulting in 130 mg (90% Yield) of 2-{[(3S)-3-hydroxypyrrolidin-1-yl]methyl}[1]benzofuro[3,2-d]pyrimidin-4(3H)-one. ¹H NMR (400 MHz, d₆-DMSO): 8.24 (s, 1H), 8.08 (d, 1H), 7.75-7.63 (m, 2H), 7.45 (dd, 1H), 4.53-4.50 (m, 1H), 4.21 (d, 1H), 4.19 (d, 1H), 3.40-3.30 (m, 1H), 3.20-3.05 (m, 3H), 2.32-2.22 (m, 1H), 2.0-1.95 (m, 1H); MS (EI) for C₁₅H₁₅N₃O₃: 286 (MH⁺).

### (Compound 311)

### 8-bromo-2-(3,9-diazaspiro[5.5]undec-3-ylmethyl)[1]benzofuro[3,2-d]pyrimidin-4(3H)-one

The compound was synthesized in a manner similar to **Example 1** wherein a solution of 8-bromo-2-(chloromethyl)[1]benzofuro[3,2-d]pyrimidin-4(3H)-one 6 (100 mg, 0.319 mmol) in 5 mL anhydrous ethanol was added 3,9-diaza-spiro[5,5]undecane-3-carboxylic acid *tert*-butyl ester (254 mg, 1 eq). The reaction mixture was heated to 80 °C for 16 hours, cooled down. 4 N HCl in dioxane (2 mL) was added. The reaction mixture was heated to 80 °C for 1 hour. The reaction was cooled and concentrated. The purification of the residue by preparative HPLC gave 8-bromo-2-(3,9-diazaspiro[5.5]undec-3-ylmethyl)[1]benzofuro[3,2-d]pyrimidin-4(3H)-one (90 mg) as a white solid. ¹H NMR (400 MHz, d₆-DMSO): 13.55 (s, 1H), 10.78 (s, 1H), 9.0 (s, 2H), 8.21 (m, 1H), 7.90 (m, 2H), 4.53 (s, 2H), 3.60-3.40 (m, 4H), 3.02 (s, 4H), 1.95-1.75 (m, 8H); MS (EI) for C₂₀H₂₃BrN₄O₂: 431 (MH⁺).

### (Compound 331)

### 8-bromo-2-(3-pyrrolin-1-ylmethyl)[1]benzofuro[3,2-d]pyrimidin-4(3H)-one

8-bromo-2-(3-pyrrolin-1-ylmethyl)[1]benzofuro[3,2-d]pyrimidin-4(3H)-one was synthesized in a manner similar to **Example 1, Compound 2,** wherein piperidine was substituted with 3-pyrroline. Purification by preparative HPLC (reverse-phase, acetonitrile/water with 0.01% ammonium acetate), followed by concentration *in vacuo* and lyophilization afforded the title compound as a white solid. ¹H NMR (400 MHz, d₆-DMSO): 8.20 (s, 1H), 7.82 (m, 2H), 5.83 (s, 1 H), 3.93 (s, 2H), 3.67 (s, 4H); MS (EI) for C₁₅H₁₂BrN₃O₂: 346 (MH⁺).

### (Compound 332)

8-bromo-2-((7-hydroxy-2-azabicyclo[2.2.1]heptan-2-yl)methyl)benzofuro[3,2-d]pyrimidin-4(3H)-one was synthesized in a manner similar to **Example 1, Compound 2,** wherein piperidine was substituted with anti-7-hydroxy-2-azabicyclo[2.2.1]heptane. Purification by preparative HPLC (reverse-phase, acetonitrile/water with 0.01% ammonium acetate), followed by concentration *in vacuo* and lyophilization afforded the title compound as a racemer. ¹H NMR (400 MHz, d₆-DMSO): 8.19 (s, 1H), 7.82 (m, 2H), 4.12 (s, 1H), 3.78 (d, 1H), 3.65 (d, 1H), 3.02 (m, 2H), 2.27 (d, 1H), 2.0 (m, 1H), 1.80 (m, 2H), 1.67 (m, 1H), 1.27 (m, 1H); MS (EI) for C₁₇H₁₆BrN₃O₃: 390 (MH⁺).

### *(Compound 333)

### 8-bromo-2-(((3S,4S)-3,4-dihydroxypyrrolidin-1-yl)methyl)benzofuro[3,2-d]pyrimidin-4(3H)-one

To a solution of 8-bromo-2-(chloromethyl)[1]benzofuro[3,2-d]pyrimidin-4(3H)-one 6 (**Example 1**) (142 mg, 0.45 mmol) in 5 mL anhydrous ethanol was added (3S, 4S)-3,4-bis[(*tert*-butyl-dimethylsilyl)oxy]pyrrolidine (150 mg, 0.45 mmol). The reaction mixture was heated to 80 °C for 16 hours. 2 N HCl aq. (2 mL) was added. The reaction mixture was further heated at 80 °C for 1 hour. The reaction was cooled and concentrated. The purification of the residue by preparative HPLC gave the title compound as a white solid. ¹H NMR (400 MHz, d₆-DMSO): 8.21 (s, 1H), 8.18 (s, 1H), 7.82 (m, 2H), 3.87 (m, 2H), 3.75 (dd, 2H), 2.99 (m, 2H), 2.45 (m, 2H); MS (EI) for C₁₅H₁₄BrN₃O₄: 380 (MH⁺).

### (Compound 344)

### 8-bromo-2-{[(2R)-2-(hydroxymethyl)pyrrolidin-1-yl]methyl}[1]benzofuro[3,2-d]pyrimidin-4(3H)-one

8-bromo-2-{[(2R)-2-(hydroxymethyl)pyrrolidin-1-yl]methyl}[1]benzofuro[3,2-d]pyrimidin-4(3H)-one was synthesized in a manner similar to **Example 1, Compound 2**, wherein piperidine was substituted with D-prolinol. Purification by preparative HPLC (reverse-phase, acetonitrile/water with 0.01% ammonium acetate), followed by concentration *in vacuo* and lyophilization afforded the title compound as a white solid. ¹H NMR (400 MHz, d₆-DMSO): 8.19 (s, 1H), 7.82 (m, 2H), 4.02 (d, 1 H), 3.57 (d, 1H), 3.48 (m, 2H), 2.95 (m, 2H), 2.70 (m, 1H), 2.37 (m, 1H), 1.92 (m, 1H), 1.67 (m, 1H), 1.27 (m, 1H); MS (EI) for C₁₆H₁₆BrN₃O₃: 378 (MH⁺).

### *(Compound 348)

### 8-bromo-2-{[cis-3,4-dihydroxypyrrolidin-1-yl]methyl}[1]benzofuro[3,2-d]pyrimidin-4(3H)-one

8-bromo-2-{[cis-3,4-dihydroxypyrrolidin-1-yl]methyl}[1]benzofuro[3,2-d]pyrimidin-4(3H)-one was synthesized in a manner similar to **Example 1, Compound 2**, wherein piperidine was substituted with cis-3,4-dihydroxypyrrolidine (prepared above). Purification by preparative HPLC (reverse-phase, acetonitrile/water with 0.01% ammonium acetate), followed by concentration *in vacuo* and lyophilization afforded the title compound as a white solid. ¹H NMR (400 MHz, d4-CDOD₃): 8.58 (s, 1H), 7.81 (d, 1H), 7.72 (d, 1H), 4.65 (s, 2H), 4.42 (m, 2H), 3.75 (m, 2H), 3.65 (m, 2H); MS (EI) for C₁₅H₁₄BrN₃O₄: 380 (MH⁺).

### *(Compound 364)

N-{(3R)-1-[(8-bromo-4-oxo-3,4-dihydro[1]benzofuro[3,2-d]pyrimidin-2-yl)methyl]pyrrolidin-3yl}acetamide N- {(3R)-1-[(8-bromo-4-oxo-3,4-dihydro[1]benzofuro[3,2-d]pyrimidin-2-yl)methyl]pyrrolidin-3-yl}acetamide was synthesized in a manner similar to **Example 1, Compound 2,** wherein piperidine was substituted with (3R)-(+)-3-acetamidopyrrolidine. Purification by preparative HPLC (reverse-phase, acetonitrile/water with 0.01% ammonium acetate), followed by concentration in *vacuo* and lyophilization afforded the title compound as a white solid. ¹H NMR (400 MHz, d6-DMSO): 7.40 (m, 1H), 6.98 (m, 1H), 6.85 (d, 1H), 3.60 (m, 1H), 3.07 (s, 2H), 2.20 (m, 2H), 1.95 (m, 2H), 1.50 (m, 1H), 1.09 (s, 3H), 0.99 (m, 1H); MS (EI) for C₁₇H₁₇BrN₄O₃: 405 (MH⁺).

### *(Compound 365)

### N-{(3S)-1-[(8-bromo-4-oxo-3,4-dihydro[1]benzofuro[3,2-d]pyrimidin-2-yl)methyl]pyrrolidin-3-yl}acetamide

N-{(3S)-1-[(8-bromo-4-oxo-3,4-dihydro[1]benzofuro[3,2-d]pyrimidin-2-yl)methyl]pyrrolidin-3-yl}acetamide was synthesized in a manner similar to **Example 1, Compound 2**, wherein piperidine was substituted with (3S)-(-)-3-acetamidopyrrolidine. Purification by preparative HPLC (reverse-phase, acetonitrile/water with 0.01 % ammonium acetate), followed by concentration in *vacuo* and lyophilization afforded the title compound as a white solid. ¹H NMR (400 MHz, d6-DMSO): 8.22 (s, 1H), 8.10 (m, 1H), 7.81 (m, 2H), 4.20 (m, 1H), 3.70 (dd, 2H), 2.80 (m, 2H), 2.40 (m, 2H), 2.10 (m, 1H), 1.80 (s, 3H), 1.58 (m, 1H); MS (EI) for C₁₇H₁₇BrN₄O₃: 405 (MH⁺).

### (Compound 369)

### 8-bromo-2-{[(3S)-3-(methyloxy)pyrrolidin-1-yl]methyl}[1]benzofuro[3,2-d]pyrimidin-4(3H)-one

8-bromo-2-{[(3S)-3-(methyloxy)pyrrolidin-1-yl]methyl}[1]benzofuro[3,2-d]pyrimidin-4(3H)-one was synthesized in a manner similar to **Example 1, Compound 2,** wherein piperidine was substituted with (S)-3-methoxypyrrolidine. Purification by preparative HPLC (reverse-phase, acetonitrile/water with 0.01% ammonium acetate), followed by concentration *in vacuo* and lyophilization afforded the title compound as a white solid. ¹H NMR (400 MHz, d6-DMSO): 8.22 (s, 1H), 7.81 (m, 2H), 3.93 (m, 1H), 3.65 (s, 2H), 3.17 (s, 3H), 2.82 (dd, 1 H), 2.70 (m, 1H), 2.58 (m, 2H), 2.0 (m, 1H), 1.68 (m, 1H); MS (EI) for C₁₆H₁₆BrN₃O₃: 378 (MH⁺).

### (Compound 408)

### 8-bromo-2-{[(3R)-3-(methyloxy)pyrrolidin-1-yl]methyl}[1]benzofuro[3,2-d]pyrimidin-4(3H)-one

8-bromo-2-{[(3R)-3-(methyloxy)pyrrolidin-1-yl]methyl}[1]benzofuro[3,2-d]pyrimidin-4(3H)-one was synthesized in a manner similar to **Example 1, Compound 2,** wherein piperidine was substituted with (3R)-(-)-N-*tert-*butoxycarbonyl-3-hydroxypyrrolidine. ¹H NMR (400 MHz, d6-DMSO): 8.22 (s, 1H), 7.81 (m, 2H), 3.93 (m, 1H), 3.65 (s, 2H), 3.17 (s, 3H), 2.82-2.65 (m, 2H), 2.58 (m, 2H), 1.95 (m, 1H), 1.68 (m, 1H); MS (EI) for C₁₆H₁₆BrN₃O₃: 378 (MH⁺).

### (Compound 370)

### 1-[(8-bromo-4-oxo-3,4-dihydro[1]benzofuro[3,2-d]pyrimidin-2-yl)methyl]azetidine-3-carboxylic acid

1-[(8-bromo-4-oxo-3,4-dihydro[1]benzofuro[3,2-d]pyrimidin-2-yl)methyl]azetidine-3-carboxylic acid was synthesized in a manner similar to **Example 1, Compound 2**, wherein piperidine was substituted with azetidine-3-carboxylic acid. Purification by preparative HPLC (reverse-phase, acetonitrile/water with 0.01 % ammonium acetate), followed by concentration *in vacuo* and lyophilization afforded the title compound as a white solid. ¹H NMR (400 MHz, d6-DMSO): 8.29 (s, 1H), 8.22 (s, 1H), 7.82 (m, 2H), 3.67-3.55 (m, 4H), 3.40 (t, 2H), 3.22 (m, 1H); MS (EI) for C₁₅H₁₂BrN₃O₄: 378 (MH⁺).

### (Compound 398)

### 8-bromo-2-[(3-hydroxy-8-azabicyclo[3,2,1]oct-8-yl)methyl][1]benzofuro[3,2-d]pyrimidin-4(3H)-one

8-bromo-2-[(3-hydroxy-8-azabicyclo[3.2.1]oct-8-yl)methyl][1]benzofuro[3,2-d]pyrimidin-4(3H)-one was synthesized in a manner similar to **Example 1, Compound 2,** wherein piperidine was substituted with 8-aza-bicyclo[3,2,1]octan-3-ol. Purification by preparative HPLC (reverse-phase, acetonitrile/water with 0.01 % ammonium acetate), followed by concentration in *vacuo* and lyophilization afforded the title compound as a white solid. ¹H NMR (400 MHz, d6-DMSO): 8.22 (m, 1H), 7.77 (m, 2H), 3.77-3.35 (m, 6H), 2.20-1.90 (m, 5H), 1.80-179 (m, 2H); MS (EI) for C₁₈H₁₈BrN₃O₃: 404 (MH⁺).

### (Compound 366)

### 8-bromo-2-{[(2S)-2-(hydroxymethyl)pyrrolidin-1-yl]methyl}[1]benzofuro[3,2-d]pyrimidin-4(3H)-one

8-bromo-2-{[(2S)-2-(hydroxymethyl)pyrrolidin-1-yl]methyl}[1]benzofuro[3,2-d]pyrimidin-4(3H)-one was synthesized in a manner similar to **Example 1, Compound 2,** wherein piperidine was substituted with L-prolinol. Purification by preparative HPLC (reverse-phase, acetonitrile/water with 0.01% ammonium acetate), followed by concentration *in vacuo* and lyophilization afforded the title compound as a white solid. ¹H NMR (400 MHz, d₆-DMSO): 8.21 (s, 1H), 7.82 (m, 2H), 4.02 (d, 1 H), 3.57 (d, 1H), 3.47 (dd, 1H), 3.37 (dd, 1H), 2.95 (m, 2H), 2.70 (m, 1H), 2.37 (m, 1H), 1.82 (m, 1H), 1.69 (m, 3H); MS (EI) for C₁₆H₁₆BrN₃O₃: 378 (MH⁺).

### (Compound 199)

### 8-bromo-2-[(cyclopentylamino)methyl][1]benzofuro[3,2-d]pycimidin-4(3H)-one

8-bromo-2-[(cyclopentylamino)methyl][1]benzofuro[3,2-d]pyrimidin-4(3H)-one was synthesized in a manner similar to **Example 1, Compound 2,** wherein piperidine was substituted with cyclopentylamine. Purification by preparative HPLC (reverse-phase, acetonitrile/water with 0.1% formic acid), followed by concentration *in vacuo* and lyophilization afforded the title compound (11.2 mg). ¹H NMR (400 MHz, d₆-DMSO): 8.51 (s, 1H), 8.14-8.16 (m, 1H), 7.76-7.79 (m, 2H), 3.79 (s, 2H), 3.12-3.19 (m, 1H), 1.74-1.83 (m, 2H), 1.60-1.64 (m, 2H), 1.36-1.53 (m, 4H). MS (EI) for C₁₆H₁₆BrN₃O₂: 362 (MH⁺).

### (Compound 200)

### 8-bromo-2-({[2-(dimethylamino)ethyl]amino}methyl)[1]benzofuro[3,2-d]pyrimidin-4(3H)-one

8-bromo-2-({[2-(dimethylamino)ethyl]amino}methyl)[1]benzofuro[3,2-d]pyrimidin-4(3H)-one was synthesized in a manner similar to **Example 1, Compound 2**, wherein piperidine was substituted with N,N-dimethylethylenediamine. Purification by preparative HPLC (reverse-phase, acetonitrile/water with 0.1 % formic acid), followed by concentration *in vacuo* and lyophilization afforded the title compound (7.3 mg). ¹H NMR (400 MHz, d₆-DMSO): 8.23 (s, 1H), 8.00-8.03 (m, 1H), 7.64-7.70 (m, 1H), 4.37 (s, 2H), 3.41-3.50 (m, 2H), 3.23 (s, 6H), 3.04-3.10 (m, 2H). MS (EI) for C₁₅H₁₇BrN₄O₂: 365 (MH⁺).

### (Compound 201)

### 8-bromo-2-[(4-methylpiperidin-1-yl)methyl][1]benzofuro[3,2-d]pyrimidin-4(3H)-one

8-bromo-2-[(4-methylpiperidin-1-yl)methyl][1]benzofuro[3,2-d]pyrimidin-4(3H)-one was synthesized in a manner similar to **Example 1, Compound 2**, wherein piperidine was substituted with 4-methylpiperidine. Purification by preparative HPLC (reverse-phase, acetonitrile/water with 0.1 % formic acid), followed by concentration *in vacuo* and lyophilization afforded the title compound (26 mg). ¹H NMR (400 MHz, d₆-DMSO): 8.21 (s, 1H), 7.82 (s, 2H), 3.50 (s, 2H), 2.81-2.92 (m, 2H), 2.06-2.17 (m, 2H), 1.52-1.62 (m, 2H), 1.33 (s, 1H), 1.11-1.26 (m, 2H), 0.89 (d, 3H). MS (EI) for C₁₇H₁₈BrN₃O₂: 376 (MH⁺).

### (Compound 202)

### 2-(1,4'-bipiperidin-1'-ylmethyl)-8-bromo[1]benzofuro[3,2-d]pyrimidin-4(3H)-one

2-(1,4'-bipiperidin-1'-ylmethyl)-8-bromo[1]benzofuro[3,2-d]pyrimidin-4(3H)-one was synthesized in a manner similar to **Example 1, Compound 2,** wherein piperidine was substituted with 4-piperidinopiperidine. Purification by preparative HPLC (reverse-phase, acetonitrile/water with 0.1 % formic acid), followed by concentration *in vacuo* and lyophilization afforded the title compound (27 mg). ¹H NMR (400 MHz, d₆-DMSO): 8.20-8.23 (m, 1H), 7.80-7.85 (m, 2H), 3.51 (s, 4H), 2.93 (d, 2H), 2.23-2.35 (m, 1H), 2.07-2.18 (m, 2H), 1.68 (d, 2H), 1.45-1.58 (m, 6H), 1.34-1.42 (m, 2H). MS (EI) for C₂₁H₂₅BrN₄O₂: 445 (MH⁺).

### (Compound 232)

### 8-bromo-2-{[(1-methylpropyl)amino]methyl}[1]benzofuro[3,2-d]pyrimidin-4(3H)-one

8-bromo-2-{[(1-methylpropyl)amino]methyl}[1]benzofuro[3,2-d]pyrimidin-4(3H)-one was synthesized in a manner similar to **Example 1, Compound 2,** wherein piperidine was substituted with sec-butylamine. Purification by preparative HPLC (reverse-phase, acetonitrile/water with 0.1 % formic acid), followed by concentration *in vacuo* and lyophilization afforded the title compound (19.2 mg). ¹H NMR (400 MHz, d₆-DMSO): 8.24 (s, 1H), 8.16 (s, 1H), 7.80 (m, 1H), 3.78-3.87 (m, 2H), 2.61-2.74 (m, 1H), 1.47-1.60 (m, 1H), 1.27-1.41 (m, 2H), 1.02-1.10 (m, 3H), 0.84-0.90 (m, 3H). MS (EI) for C₁₅H₁₆BrN₃O₂: 350 (MH⁺).

### (Compound 235)

### 8-bromo-2-({[(2-chlorophenyl)methyl]amino}methyl)[1]benzofuro[3,2-d]pyrimidin-4(3H)-one

8-bromo-2-({[(2-chlorophenyl)methyl]amino}methyl)[1]benzofuro[3,2-d]pyrimidin-4(3H)-one was synthesized in a manner similar to **Example 1, Compound 2**, wherein piperidine was substituted with 2-chlorobenzylamine. Purification by preparative HPLC (reverse-phase, acetonitrile/water with 0.1% formic acid), followed by concentration *in vacuo* and lyophilization afforded the title compound (17.8 mg). ¹H NMR (400 MHz, d₆-DMSO): 8.18-8.22 (m, 1H), 7.80-7.86 (m, 2H), 7.54-7.59 (m, 1H), 7.38-7.42 (m, 1H), 7.22-7.35 (m, 2H), 3.86 (s, 2H), 3.80 (s, 2H). MS (EI) for C₁₈H₁₃BrClN₃O₂: 418 (MH⁺).

### (Compound 238)

### 8-bromo-2-[(4-phenylpiperidin-1-yl)methyl][1]benzofuro[3,2-d]pyrimidin-4(3H)-one

8-bromo-2-[(4-phenylpiperidin-1-yl)methyl][1]benzofuro[3,2-d]pyrimidin-4(3H)-one was synthesized in a manner similar to **Example 1, Compound** , wherein piperidine was substituted with 4-phenylpiperidine. Purification by preparative HPLC (reverse-phase, acetonitrile/water with 0.1% formic acid), followed by concentration *in vacuo* and lyophilization afforded the title compound (33.8 mg). ¹H NMR (400 MHz, d₆-DMSO): 11.35 (s, 1H), 8.65 (s, 1H), 8.20-8.24 (m, 1H), 7.80-7.86 (m, 2H), 7.14-7.36 (m, 4H), 3.59 (s, 2H), 2.97-3.06 (m, 2H), 2.22-2.36 (m, 2H), 1.90-1.97 (m, 1H), 1.65-1.83 (m, 4H). MS (EI) for C₂₂H₂₀BrN₃O₂:438 (MH⁺).

### (Compound 240)

### 8-bromo-2-[(4-pyridin-3-ylpiperazin-1-yl)methyl][1]benzofuro[3,2-d]pyrimidin-4(3H)-one

8-bromo-2-[(4-pyridin-3-ylpiperazin-1-yl)methyl][1]benzofuro[3,2-d]pyrimidin-4(3H)-one was synthesized in a manner similar to **Example 1, Compound 2**, wherein piperidine was substituted with 1-pyridin-3-yl piperazine. Purification by preparative HPLC (reverse-phase, acetonitrile/water with 0.1 % formic acid), followed by concentration *in vacuo* and lyophilization afforded the title compound (15.4 mg). ¹H NMR (400 MHz, d₆-DMSO): 8.30 (s, 1H), 8.21 (s, 1H), 7.96-8.01 (m, 1H), 7.79-7.85 (m, 2H), 7.28-7.34 (m, 1H), 7.18-7.24 (m, 1H), 3.60 (s, 2H), 3.22 (s, 4H), 2.69 (s, 4H). MS (EI) for C₂₀H₁₈BrN₅O₂: 440 (MH⁺).

### *(Compound 241)

### 8-bromo-2-[(2,3-dihydro-1H-inden-1-ylamino)methyl][1]benzoruro[3,2-d]pyrimidin-4(3H)-one

8-bromo-2-[(2,3-dihydro-1H-inden-1-ylamino)methyl][1]benzofuro[3,2-d]pyrimidin-4(3H)-one was synthesized in a manner similar to **Example 1, Compound 2**, wherein piperidine was substituted with 1-aminoindan. Purification by preparative HPLC (reverse-phase, acetonitrile/water with 0.1% formic acid), followed by concentration *in vacuo* and lyophilization afforded the title compound (20.0 mg). ¹H NMR (400 MHz, d₆-DMSO): 8.21-8.24 (m, 1H), 7.79-7.85 (m, 2H), 7.41-7.46 (m, 1H), 7.17-7.26 (m, 3H), 4.20-4.26 (m, 1H), 3.84 (s, 2H), 2.89-2.99 (m, 1H), 2.64-2.80 (m, 1H), 2.21-2.35 (m, 1H), 1.74-1.87 (m, 1H). MS (EI) for C₂₀H₁₆BrN₃O₂:410 (MH⁺).

### (Compound 242)

### 8-bromo-2-[(4-hydroxypiperidin-1-yl)methyl][1]benzofuro[3,2-d]pyrimidin-4(3H)-one

8-bromo-2-[(4-hydroxypiperidin-1-yl)methyl][1]benzofuro[3,2-d]pyrimidin-4(3H)-one was synthesized in a manner similar to **Example 1,** 158 **Compound 2**, wherein piperidine was substituted with 4-hydroxy piperidine. Purification by preparative HPLC (reverse-phase, acetonitrile/water with 0.1% formic acid), followed by concentration *in vacuo* and lyophilization afforded the title compound (13.7 mg). ¹H NMR (400 MHz, d₆-DMSO): 8.20-8.26 (m, 1H), 7.79-7.85 (m, 2H), 3.50 (s, 2H), 2.71-2.81 (m, 1H), 2.17-2.27 (m, 2H), 1.67-1.76 (m, 2H), 1.35-1.49 (m, 2H), 0.96-1.02 (m, 2H). MS (EI) for C₁₆H₁₆BrN₃O₃: 378 (MH⁺).

### (Compound 252)

### 8-bromo-2-[(cyclobutylamino)methyl][1]benzofuro[3,2-d]pyrimidin-4(3H)-one

8-bromo-2-[(cyclobutylamino)methyl][1]benzofuro[3,2-d]pyrimidin-4(3H)-one was synthesized in a manner similar to **Example 1, Compound 2**, wherein piperidine was substituted with cyclobutylamine. Purification by preparative HPLC (reverse-phase, acetonitrile/water with 0.1% formic acid), followed by concentration *in vacuo* and lyophilization afforded the title compound (9.3 mg). ¹H NMR (400 MHz, d₆-DMSO): 8.31 (s, 1H), 8.17-8.19 (m, 1H), 7.78-7.83 (m, 2H), 3.69 (s, 2H), 3.23-3.32 (m, 1H), 1.99-2.10 (m, 2H), 1.67-1.79 (m, 2H), 1.46-1.66 (m, 2H). MS (EI) for C₁₅H₁₄BrN₃O₂: 348 (MH⁺).

### (Compound 253)

### 8-bromo-2-{[4-(phenylmethyl)piperidin-1-yl]methyl}[1]benzofuro[3,2-d]pyrimidin-4(3H)-one

8-bromo-2-{[4-(phenylmethyl)piperidin-1-yl]methyl}[1]benzofuro[3,2-d]pyrimidin-4(3H)-one was synthesized in a manner similar to **Example 1, Compound 2**, wherein piperidine was substituted with 4-benzyl piperidine. Purification by preparative HPLC (reverse-phase, acetonitrile/water with 0.1 % formic acid), followed by concentration *in vacuo* and lyophilization afforded the title compound (22.1 mg). ¹H NMR (400 MHz, d₆-DMSO): 8.31 (s, 1H), 8.19-8.21 (m, 1H), 7.78-7.85 (m, 2H), 7.23-7.31 (m, 2H), 7.11-7.20 (m, 3H), 3.49 (s, 2H), 2.86 (d, 2H), 2.06 (t, 2H), 1.43-1.58 (m, 3H), 1.18-1.30 (m, 2H). MS (EI) for C₂₃H₂₂BrN₃O₂: 452 (MH⁺).

### (Compound 254)

### 8-bromo-2-{[(pyridin-2-ylmethyl)amino]methyl}[1]benzofuro[3,2-d]pyrimidin-4(3H)-one

8-bromo-2-{[(pyridin-2-ylmethyl)amino]methyl}[1]benzofuro[3,2-d]pyrimidin-4(3H)-one was synthesized in a manner similar to **Example 1, Compound 2**, wherein piperidine was substituted with 2-picolylamine. Purification by preparative HPLC (reverse-phase, acetonitrile/water with 0.1% formic acid), followed by concentration *in vacuo* and lyophilization afforded the title compound (22.4 mg). ¹H NMR (400 MHz, d₆-DMSO): 8.49-8.52 (m, 1H), 8.18-8.21 (m, 1H), 7.80-7.85 (m, 2H), 7.71-7.78 (m, 1H), 7.45 (d, 1H), 7.22-7.27 (m, 1H), 3.90 (s, 2H), 3.83 (s, 2H). MS (EI) for C₁₇H₁₃BrN₄O₂: 385 (MH⁺).

### (Compound 255)

### 8-bromo-2-[({[3-(methyloxy)phenyl]methyl}amino)methyl][1]benzofuro[3,2-d]pyrimidin-4(3H)-one

8-bromo-2-[({[3-(methyloxy)phenyl]methyl}amino)methyl][1]benzofuro[3,2-d]pyrimidin-4(3H)-one was synthesized in a manner similar to **Example 1, Compound 2**, wherein piperidine was substituted with 3-methoxybenzylamine. Purification by preparative HPLC (reverse-phase, acetonitrile/water with 0.1 % formic acid), followed by concentration *in vacuo* and lyophilization afforded the title compound (20.7 mg). ¹H NMR (400 MHz, d₆-DMSO): 8.22 (s, 1H), 8.18-8.21 (m, 1H), 7.80-7.85 (m, 2H), 7.19-7.25 (m, 1H), 6.90-6.96 (m, 2H), 6.76-6.84 (m, 2H), 3.76 (s, 2H), 3.74 (s, 2H), 3.72 (s, 3H). MS (EI) for C₁₉H₁₆BrN₃O₃: 414 (MH⁺).

### (Compound 256)

### 8-bromo-2-({[(3-chlorophenyl)methyl]amino}methyl)[1]benzofuro[3,2-d]pyrimidin-4(3H)-one

8-bromo-2-({[(3-chlorophenyl)methyl]amino}methyl)[1]benzofuro[3,2-d]pyrimidin-4(3H)-one was synthesized in a manner similar to **Example 1, Compound 2**, wherein piperidine was substituted with 3-chlorobenzylamine. Purification by preparative HPLC (reverse-phase, acetonitrile/water with 0.1% formic acid), followed by concentration *in vacuo* and lyophilization afforded the title compound (25.3 mg). ¹H NMR (400 MHz, d₆-DMSO): 8.18-8.21 (m, 2H), 7.80-7.85 (m, 2H), 7.46 (s, 1H), 7.25-7.35 (m, 3H), 3.78 (s, 2H), 3.75 (s, 2H). MS (EI) for C₁₅H₁₃BrClN₃O₂: 418 (MH⁺).

### (Compound 257)

### 8-bromo-2-[(4-morpholin-4-ylpiperidin-1-yl)methyl][1]benzofuro[3,2-d]pyrimidin-4(3H)-one

8-bromo-2-[(4-morpholin-4-ylpiperidin-1-yl)methyl][1]benzofuro[3,2-d]pyrimidin-4(3H)-one was synthesized in a manner similar to **Example 1, Compound 2**, wherein piperidine was substituted with 4-morpholino piperidine. Purification by preparative HPLC (reverse-phase, acetonitrile/water with 0.1 % formic acid), followed by concentration *in vacuo* and lyophilization afforded the title compound (16.3 mg). ¹H NMR (400 MHz, d₆-DMSO): 8.26 (s, 1H), 8.18-8.21 (m, 1H), 7.80-7.85 (m, 2H), 3.53-3.59 (m, 7H), 2.92 (d, 2H), 2.39-2.48 (m, 3H), 2.06-2.19 (m, 3H), 1.74 (d, 2H), 1.37-1.49 (m, 2H). MS (EI) for C₂₀H₂₃BrN₄O₃: 447 (MH⁺).

### (Compound 258)

### 8-bromo-2-{[(furan-2-ylmethyl)amino]methyl}[1]benzofuro[3,2-d]pyrimidin-4(3H)-one

8-bromo-2-{[(furan-2-ylmethyl)amino]methyl}[1]benzofuro[3,2-d]pyrimidin-4(3H)-one was synthesized in a manner similar to **Example 1, Compound 2,** wherein piperidine was substituted with furfurylamine. Purification by preparative HPLC (reverse-phase, acetonitrile/water with 0.1 % formic acid), followed by concentration *in vacuo* and lyophilization afforded the title compound (9.7 mg). ¹H NMR (400 MHz, d₆-DMSO): 8.35 (s, 1H), 8.18-8.21 (m, 1H), 7.80-7.83 (m, 2H), 7.53-7.57 (m, 1H), 6.34-6.37 (m, 1H), 6.25-6.28 (m, 1H), 3.76 (s, 2H), 3.74 (s, 2H). MS (EI) for C₁₆H₁₂BrN₃O₃: 374 (MH⁺).

### (Compound 259)

### 8-bromo-2-({[2-(1H-imidazol-4-yl)ethyl]amino}methyl)[1]benzofuro[3,2-d]pyrimidin-4(3H)-one

8-bromo-2-({[2-(1H-imidazol-4-yl)ethyl]amino}methyl)[1]benzofuro[3,2-d]pyrimidin-4(3H)-one was synthesized in a manner similar to **Example 1, Compound 2**, wherein piperidine was substituted with histamine. Purification by preparative HPLC (reverse-phase, acetonitrile/water with 0.1% formic acid), followed by concentration *in vacuo* and lyophilization afforded the title compound (3.0 mg). ¹H NMR (400 MHz, d₆-DMSO): 8.20 (s, 1H), 8.15-8.17 (m, 1H), 7.78-7.83 (m, 2H), 7.54 (d, 1H), 6.81 (s, 1H), 3.84 (s, 2H), 2.86 (t, 2H), 2.66-2.73 (m, 2H). MS (EI) for C₁₆H₁₄BrN₅O₂: 388 (MH⁺).

### (Compound 260)

### 8-bromo-2-[(4-phenylpiperazin-1-yl)methyl][1]benzofuro[3,2-d]pyrimidin-4(3H)-one

8-bromo-2-[(4-phenylpiperazin-1-yl)methyl][1]benzofuro[3,2-d]pyrimidin-4(3H)-one was synthesized in a manner similar to **Example 1, Compound 2**, wherein piperidine was substituted with 1-phenyl piperazine. Purification by preparative HPLC (reverse-phase, acetonitrile/water with 0.1% formic acid), followed by concentration *in vacuo* and lyophilization afforded the title compound (46.2 mg). ¹H NMR (400 MHz, d₆-DMSO): 8.21-8.23 (m, 1H), 7.80-7.85 (m, 2H), 7.20 (t, 2H), 6.93 (d, 2H), 6.77 (t, 1H), 3.60 (s, 2H), 3.13-3.18 (m, 4H), 2.65-2.70 (m, 4H). MS (EI) for C₂₁H₁₉BrN₄O₂: 439 (MH⁺).

### (Compound 261)

### 8-bromo-2-[({[4-(methyloxy)phenyl]methyl}amino)methyl][1]benzofuro[3,2-d]pyrimidin-4(3H)-one

8-bromo-2-[({[4-(methyloxy)phenyl]methyl}amino)methyl][1]benzofuro[3,2-d]pyrimidin-4(3H)-one was synthesized in a manner similar to **Example 1, Compound 2**, wherein piperidine was substituted with 4-methoxybenzylamine. Purification by preparative HPLC (reverse-phase, acetonitrile/water with 0.1 % formic acid), followed by concentration *in vacuo* and lyophilization afforded the title compound (22.5 mg). ¹H NMR (400 MHz, d₆-DMSO): 8.18-8.20 (m, 1H), 8.15 (s, 1H), 7.80-7.85 (m, 2H), 7.28 (d, 2H), 6.87 (d, 2H), 3.75 (s, 2H), 3.72 (s, 3H). MS (EI) for C₁₉H₁₆BrN₃O₃: 414 (MH⁺).

### (Compound 262)

### 8-bromo-2-{[(2,3-dihydroxypropyl)amino]methyl}[1]benzofuro[3,2-d]pyrimidin-4(3H)-one

8-bromo-2-{[(2,3-dihydroxypropyl)amino]methyl}[1]benzofuro[3,2-d]pyrimidin-4(3H)-one was synthesized in a manner similar to **Example 1, Compound 2**, wherein piperidine was substituted with (±) 3-amino-1,2-propanediol. Purification by preparative HPLC (reverse-phase, acetonitrile/water with 0.1 % formic acid), followed by concentration *in vacuo* and lyophilization afforded the title compound (27.9 mg). ¹H NMR (400 MHz, d₆-DMSO): 8.24 (s, 1H), 8.17-8.20 (m, 1H), 7.78-7.84 (m, 2H), 3.75-3.83 (m, 2H), 3.57 (s, 1H), 3.27-3.39 (m, 2H), 2.66-2.75 (m, 2H). MS (EI) for C₁₄H₁₄BrN₃O₄: 368 (MH⁺).

### *(Compound 263)

### 8-bromo-2-[(2,3-dihydro-1H-inden-2-ylamino)methyl][1]benzofuro[3,2-d]pyrimidin-4(3H)-one

8-bromo-2-[(2,3-dihydro-1H-inden-2-ylamino)methyl][1]benzofuro[3,2-d]pyrimidin-4(3H)-one was synthesized in a manner similar to **Example 1, Compound 2**, wherein piperidine was substituted with 2-aminoindan. Purification by preparative HPLC (reverse-phase, acetonitrile/water with 0.1% formic acid), followed by concentration *in vacuo* and lyophilization afforded the title compound (19.9 mg). ¹H NMR (400 MHz, d₆-DMSO): 8.19-8.21 (m, 1H), 7.78-7.84 (m, 2H), 7.16-7.22 (m, 2H), 7.09-7.14 (m, 2H), 3.84 (s, 2H), 3.55-3.66 (m, 1H), 3.04-3.12 (m, 2H), 2.71-2.80 (m, 2H). MS (EI) for C₂₀H₁₆BrN₃O₂:410 (MH⁺).

### (Compound 268)

### 8-bromo-2-[({[2-(methyloxy)phenyl]methyl}amino)methyl][1]benzofuro[3,2-d]pyrimidin-4(3H)-one

8-bromo-2-[({[2-(methyloxy)phenyl]methyl} amino)methyl][1]benzofuro[3,2-d]pyrimidin-4(3H)-one was synthesized in a manner similar to **Example 1, Compound 2**, wherein piperidine was substituted with 2-methoxybenzylamine. Purification by preparative HPLC (reverse-phase, acetonitrile/water with 0.1% formic acid), followed by concentration *in vacuo* and lyophilization afforded the title compound (10 mg). ¹H NMR (400 MHz, d₆-DMSO): 8.18 (m, 1H), 7.81 (s, 2H), 7.33 (d, 1H), 7.20 (t, 1H), 6.94 (d, 1H), 6.88 (t, 1H), 3.79 (s, 3H), 3.77 (s, 2H), 3.72 (s, 2H). MS (EI) for C₁₉H₁₆BrN₃O₃:415 (MH⁺).

### (Compound 277)

### 8-bromo-2-({[2-(methyloxy)ethyl]amino}methyl)[1]benzofuro[3,2-d]pyrimidin-4(3H)-one

8-bromo-2-({[2-(methyloxy)ethyl]amino}methyl)[1]benzofuro[3,2-d]pyrimidin-4(3H)-one was synthesized in a manner similar to **Example 1, Compound 2**, wherein piperidine was substituted with 2-methoxyethylamine. Purification by preparative HPLC (reverse-phase, acetonitrile/water with 0.1% formic acid), followed by concentration *in vacuo* and lyophilization afforded the title compound (20.2 mg). NMR (400 MHz, d₆-DMSO): 8.24 (s, 1H), 8.17-8.19 (m, 1H), 7.79-7.84 (m, 2H), 3.78 (s, 2H), 3.39-3.44 (m, 3H), 2.71-2.77 (m, 2H). MS (EI) for C₁₄H₁₄BrN₃O₃: 352 (MH⁺).

### (Compound 278)

### 8-bromo-2-{[4-(3-chlorophenyl)piperazin-1-yl]methyl}[1]benzofuro[3,2-d]pyrimidin-4(3H)-one

8-bromo-2-{[4-(3-chlorophenyl)piperazin-1-yl]methyl}[1]benzofuro[3,2-d]pyrimidin-4(3H)-one was synthesized in a manner similar to **Example 1, Compound 2**, wherein piperidine was substituted with 1-(3-chlorophenyl)piperazine. Purification by preparative HPLC (reverse-phase, acetonitrile/water with 0.1% formic acid), followed by concentration *in vacuo* and lyophilization afforded the title compound (34.6 mg). NMR (400 MHz, d₆-DMSO): 8.22-8.23 (m, 1H), 7.80-7.86 (m, 2H), 7.20 (t, 1H), 6.87-6.95 (m, 2H), 6.75-6.79 (m, 1H), 3.60 (s, 2H), 3.16-3.24 (m, 4H), 2.61-2.70 (m, 4H). MS (EI) for C₂₁H₁₈BrClN₄O₂: 473 (MH⁺).

### (Compound 431)

### 8-bromo-2-{[(2-piperidin-1-ylethyl)amino]methyl}[1]benzofuro[3,2-d]pyrimidin-4(3H)-one

8-bromo-2- {[(2-piperidin-1-ylethyl)amino]methyl}[1]benzofuro[3,2-d]pyrimidin-4(3H)-one was synthesized in a manner similar to **Example 1, Compound 2**, wherein piperidine was substituted with 1-(2-aminoethyl)piperidine piperidine. ¹H NMR (400 MHz, d6-DMSO): 8.07 (s, 1H), 7.72 (m, 2H), 3.77 (s, 2H), 2.73 (t, 2H), 2.39 (t, 2H), 2.33 (m, 4H), 1.51 (m, 4H), 1.36 (m, 2H). MS (EI) for C18 H21 Br N4 O2: 406 MH+).

### EXAMPLE 2: (Scheme 1 except via Microwave promoted displacement)

### (Compound 4)

### 8-bromo-2-[({[4-(4-methylpiperazin-1-yl)phenyl]methyl}amino)methyl][1]benzofuro[3,2-d]pyrimidin-4(3H)-one (acetate salt)

To a solution of 8-bromo-2-(chloromethyl)benzofuro[3,2-d]pyrimidin-4(3H)-one **6** (70 mg, 0.22 mmol) in 1.5 mL anhydrous DMF was added (4-(4-methylpiperazin-1-yl)phenyl)methanamine (50 mg, 0.22 mmol) and Cs₂CO₃ (143 mg, 0.44 mmol). The reaction mixture was heated to 85 °C at 150 W for 10 minutes in a CEM-Discover microwave reactor. The reaction mixture was filtered and concentrated *in vacuo.* Purification by preparative HPLC, followed by concentration *in vacuo* and lyophilization afforded the title compound (18.2 mg, 17%) as a white solid. ¹H NMR (400 MHz, d₆-DMSO): 8.16 (m, 1H), 7.79 (m, 2H), 7.19 (d, 2H), 6.86 (d, 2H), 3.72 (s, 2H), 3.68 (s, 2H), 3.07 (m, 4H), 2.50 (m, 4H), 2.21 (s, 3H), 1.90 (s, 3H); MS (EI) for C₂₃H₂₄BrN₅O₂: 482 (MH).

### *(Compounds 8)

### 8-bromo-2-[(piperidin-4-ylamino)methyl][1]benzofuro[3,2-d]pyrimidin-4(3H)-one

8-bromo-2-[(piperidin-4-ylamino)methyl][1]benzofuro[3,2-d]pyrimidin-4(3H)-one was synthesized in a manner similar to **Example 2,** wherein (4-(4-methylpiperazin-1-yl)phenyl)methanamine was substituted with tert-butyl 4-aminopiperidine-1-carboxylate. Purification by preparative HPLC, followed by concentration *in vacuo* and lyophilization afforded tert-butyl 8-bromo-2-[(piperidin-4-ylamino)methyl][1]benzofuro[3,2-d]pyrimidin-4(3H)-one-carboxylate. Tert-butyl 8-bromo-2-[(piperidin-4-ylamino)methyl][1]benzofuro[3,2-d]pyrimidin-4(3H)-one-carboxylate was taken in 4 mL methanol and 2mL of 4N HCl/dioxanes. After 18 hours, the reaction mixture was concentrated *in vacuo* and purified by preparative HPLC, followed by concentration *in vacuo* and lyophilization to afford the title compound (5 mg, 6%) as a white solid. ¹H NMR (400 MHz, d₆-DMSO): 8.12 (m, 1H), 7.74 (m, 2H), 3.75 (s, 2H), 2.64 (m, 3H), 2.54 (m, 2H), 1.9 (s, 3H), 1.85 (m, 2H), 1.30 (m, 2H); MS (EI) for C₁₆H₁₇BrN₄O₂: 377 (MH⁺).

### (Compound 20)

### 1-[(8-bromo-4-oxo-3,4-dihydro[1]benzofuro[3,2-d]pyrimidin-2-yl)methyl]-N-[3-(dimethylamino)propyl]prolinamide (HCl salt)

1-[(8-bromo-4-oxo-3,4-dihydro[1]benzofuro[3,2-d]pyrimidin-2-yl)methyl]-N-[3-(dimethylamino)propyl]prolinamide was synthesized in a manner similar to **Example 2,** wherein (4-(4-methylpiperazin-1-yl)phenyl)methanamine was substituted with N-(3-(dimethylamino)propyl)pyrrolidine-2-carboxamide. The crude reaction mixture was purified by preparative HPLC (reverse-phase, acetonitrile/water with 10 mM ammonium acetate) and (reverse-phase, 0.1% TFA in acetonitrile/0.05% TFA in water) to afford the title compound (3.7 mg, 4.5%) as a white solid. ¹H NMR (400 MHz, d₆-DMSO): 8.5 (br s, 1H), 8.19 (m, 1H), 7.87 (m, 2H), 3.36-3.12 (s, 2 H, overlapped), 3.21-3.05 (m, 7 H), 2.71 (s, 6H), 1.77 (m, 6H); MS (EI) for C₂₁H₂₆BrN₅O: 476 (MH⁺).

### (Compound 6)

### 8-bromo-2-(1H-imidazol-1-ylmethyl)[1]benzofuro[3,2-d]pyrimidin-4(3H)-one

8-bromo[-2-(1H-imidazol-1-ylmethyl)[1]benzofuro[3,2-d]pyrimidin-4(3H)-one was synthesized in a manner similar to **Example 2**, wherein (4-(4-methylpiperazin-1-yl)phenyl)methanamine was substituted with imidazole. Purification by preparative HPLC resulting in 14 mg (13% Yield) of 8-bromo-2-(1H-imidazol-1-ylmethyl)[1]benzofuro[3,2-d]pyrimidin-4(3H)-one as the HCl salt. ¹H NMR (400MHz, d₆-DMSO+D₂O): 9.22 (s, 1H), 8.01 (s, 1H), 7.85 (m, 3H), 7.78 (s, 1H), 5.61 (s, 2H); MS (EI) for C₁₄H₉BrN₄O₂: 345:347 (Bromine isotope MH⁺).

### (Compound 9)

### 8-bromo-2-({4-[3-(dimethylamino)propyl]piperazin-1-yl}methyl)[1]benzofuro[3,2-d]pyrimidin-4(3H)-one

8-bromo-2-({4-[3-(dimethylamino)propyl]piperazin-1-yl}methyl)[1]benzofuro[3,2-d]pyrimidin-4(3H)-one was synthesized in a manner similar to **Example 2**, wherein (4-(4-methylpiperazin-1-yl)phenyl)methanamine was substituted with commercially available 1-(3-dimethylaminopropyl)-piperazine. Purification by preparative HPLC resulting in 54 mg (37% Yield) of 8-bromo-2-({4-[3-(dimethylamino)propyl]piperazin-1-yl}methyl)[1]benzofuro[3,2-d]pyrimidin-4(3H)-one as the HCl salt. ¹H NMR (400MHz, d₆-DMSO + D₂O): 8.20 (s, 1H), 7.90 (s,2H), 3.44 (m, 6H), 3.05 (m, 6H), 2.78 (s, 6H), 1.98 (m, 2 H); MS (EI) for C₂₀H₂₆BrN₅O₂: 448 : 450 (Bromine isotope MH⁺).

### (Compound 10)

### 8-bromo-2-[({[2-(dimethylamino)phenyl]methyl}amino)methyl][1]benzofuro[3,2-d]pyrimidin-4(3H)-one

8-bromo-2-[({[2-(dimethylamino)phenyl]methyl}amino)methyl][1]-benzofuro[3,2-d]pyrimidin-4(3H)-one was synthesized in a manner similar to **Example 2**, wherein (4-(4-methylpiperazin-1-yl)phenyl)methanamine was substituted with commercially available N-[2-(aminomethyl) phenyl]-N,N-dimethylamine. Purification by preparative HPLC resulting in 27 mg (24% Yield) of 8-bromo-2-[({[2-(dimethylamino)phenyl]methyl}amino)methyl][1]benzofuro[3,2-d]pyrimidin-4(3H)-one as the HCl salt. ¹H NMR (400MHz, d₆-DMSO + D₂O): 8.22 (s, 1H), 7.86 (m,2H), 7.50 (m, 4H), 7.28 (t, 1H), 4.55 (s, 2H), 4.32 (s, 2 H), 2.79 (s, 6H); MS (EI) for C₂₀H₁₉BrN₄O₂: 427 : 429 (Bromine isotope MH⁺).

### (Compound 14)

### 8-bromo-2-[({[3-(4-methylpiperazin-1-yl)phenyl]methyl}amino)-methyl][1]benzofuro[3,2-d]pyrimidin-4(3H)-one

8-bromo-2-[({[3-(4-methylpiperazin-1-yl)phenyl]methyl}amino)methyl][1]benzofuro[3,2-d]pyrimidin-4(3H)-one was synthesized in a manner similar to **Example 2**, wherein (4-(4-methylpiperazin-1-yl)phenyl)methanamine was substituted with commercially available 1-[3-(4-methyl piperazin-1-yl)phenyl]methanamine. Preparative HPLC purification gave 27 mg (25% Yield) of 8-bromo-2-[({[3-(4-methylpiperazin-1-yl)phenyl]methyl}amino)methyl][1]benzofuro[3,2-d]pyrimidin-4(3H)-one. ¹H NMR (400MHz, d₆-DMSO): 11.21 (br s, 1H), 10.20 (br s,2H), 8.20 (s, 1H), 7.89 (m, 2H), 7.34 (m, 2H), 7.08 (d, 2 H), 4.30 (d, 4H), 3.89 (d, 2H), 3.50 (d, 2H), 3.19 (m, 4H), 2.81 (d, 3H), 2.48 (s, 3H); MS (EI) for C₂₃H₂₄BrN₅O₂: 482:484 (Bromine isotope MH⁺).

### (Compound 17)

### 8-bromo-2-{[3-(dimethylamino)pyrrolidin-1-yl]methyl}[1]benzofuro[3,2-d]pyrimidin-4(3H)-one

8-bromo-2-{[3-(dimethylamino)pyrrolidin-1-yl]methyl}[1]benzofuro[3,2-d]pyrimidin-4(3H)-one was synthesized in a manner similar to **Example 2**, wherein (4-(4-methylpiperazin-1-yl)phenyl)methanamine was substituted with commercially available 3-(dimethylamino)pyrrolidine. Preparative HPLC purification gave 32 mg (37% Yield) of 8-bromo-2-{[3-(dimethylamino)pyrrolidin-1-y1]methyl} [1]benzofur[3,2-d]pyrimidin-4(3H)-one.¹H NMR (400MHz, d₆-DMSO + D₂O): 8.30 (s, 1H), 7.85 (s,2H), 4.19 (s, 2H), 3.40 (m, 4H), 3.15 (m, 1H), 2.81 (s, 6 H), 2.38 (m, 1H), 2.19 (m, 1H); MS (EI) for C₁₇H₁₉BrN₄O₂: 391:393 (Bromine isotope MH⁺).

### *(Compound 18)

### 8-bromo-2-{[(2-chlorophenyl)(methyl)amino]methyl}[1]benzofuro[3,2-d]pyrimidin-4(3H)-one

8-bromo-2-{[(2-chlorophenyl)(methyl)amino]methyl}[1]benzofuro[3,2-d]pyrimidin-4(3H)-one was synthesized in a manner similar to **Example 2**, wherein (4-(4-methylpiperazin-1-yl)phenyl)methanamine was substituted with commercially available 2-chloro-N-Methyl aniline. Precipitation gave 15 mg (37% Yield) of 8-bromo-2-{[(2-chlorophenyl)(methyl)amino]methyl}[1]benzofuro[3,2-d]pyrimidin-4(3H)-one. ¹H NMR (400MHz, d₆-DMSO): 12.64 (s, 1H), 8.15 (s, 1H), 7.82 (m, 2H), 7.42 (d, 1H),7.38 (d, 1H), 7.31 (t, 1H), 7.15 (td, 1H), 4.36 (s, 2 H), 2.92 (s, 3H); MS (EI) for C₁₈H₁₃BrClN₃O₂: 418:420 (Bromine isotope MH⁺).

### (Compound 25)

### 2-{4-(8-bromo-4-oxo-3,4-dihydro[1]benzouro[3,2-d]pyrimidin-2-yl)methyl]piperazin-1-yl}-N,N-dimethylacetamide

2-4-[(8-bromo-4-oxo-3,4-dihydro[1]benzofuro[3,2-d]pyrimidin-2-yl)methyl]piperazin-1-yl}-N,N-dimethylacetamide was synthesized in a manner similar to **Example 2**, wherein (4-(4-methylpiperazin-1-yl)phenyl)methanamine was substituted with commercially available [piperazino-N, N-dimethyl amide. Preparative HPLC gave 40 mg (41% Yield) of 2-{4-[(8-bromo-4-oxo-3,4-dihydro[1]benzofuro[3,2-d]pyrimidin-2-yl)methyl]piperazin-1-yl}-N,N-dimethylacetamide. ¹H NMR (400MHz, d₆-DMSO): 8.21 (s, 1H), 7.80 (m,2H), 3.55 (br s, 4H), 3.15 (s, 3H), 3.00 (s, 3H), 2.82 (s, 3H), 2.40 (br s, 8 H), 1.90 (s, 2H); MS (EI) for C₁₉H₂₂BrN₅O₃: 448:450 (Bromine isotope MH⁺)_{.}

### (Compound 26)

### 8-bromo-2-{[4-(N,N-diethylglycyl)piperazin-1-yl]methyl}[1]benzofuro[3,2-d]pyrimidin-4(3H)-one

8-bromo-2-{[4-(N,N-diethylglycyl)piperazin-1-yl]methyl}[1]benzofuro[3,2-d]pyrimidin-4(3H)-one was synthesized in a manner similar to **Example 2**, wherein (4-(4-methylpiperazin-1-yl)phenyl)methanamine was substituted with 2-(diethylamino)-1-(piperazin-1-yl)ethanone. Preparative HPLC gave 40 mg (41% Yield) of 8-bromo-2-{[4-(N,N-diethylglycyl)piperazin-1-yl]methyl}[1]benzofuro[3,2-d]pyrimidin-4(3H)-one. ¹H NMR (400MHz, d₆-DMSO): 8.35 (s, 1H), 7.85 (m,2H), 4.43 (br s, 2H), 4.29 (br s, 2H), 3.90 (br s, 2H), 3.79 (br s, 2H), 3.43 (m, 4H), 3.20 (m, 4 H), 1.25 (t, 6H); MS (EI) for C₂₁H₂₆BrN₅O₃: 476:478 (Bromine isotope MH⁺).

### *(Compound 75)

### 8-bromo-2-({[4-(4-methylpiperazin-1-yl)phenyl]amino}methyl)[1]benzofuro[3,2-d]pyrimidin-4(3H)-one

8-bromo-2-({[4-(4-methylpiperazin-1-yl)phenyl]amino}methyl)[1]benzofuro-[3,2-d]pyrimidin-4(3H)-one was synthesized in a manner similar to **Example 2**, wherein (4-(4-methylpiperazin-1-yl)phenyl)methanamine was substituted with 4-(4-methylpiperazino)aniline. Purification by preparative, followed by concentration *in vacuo* and lyophilization afforded the title compound (14 mg, 23%) as a white solid. ¹H NMR (400 MHz, d₆-DMSO): 8.21 (s, 1H), 7.85 (s, 2H), 6.88 (d, 2H), 6.71 (d, 2H), 4.32 (s, 2H), 3.22 (br q, 4H), 2.90 (br t, 4H), 2.80 (d, 3H); MS (EI) for C₂₂H₂₂BrN₅0₂: 468.1:470.1 (Bromine isoptope MH⁺).

### *(Compound 76)

### 8-bromo-2-({[3-(dimethylamino)phenyl]amino}methyl)[1]benzofuro[3,2-d]pyrimidin-4(3H)-one

8-bromo-2-({[3-(dimethylamino)phenyl]amino}methyl)[1]benzofuro[3,2-d]pyrimidin-4(3H)-one was synthesized in a manner similar to **Example 2**, wherein (4-(4-methylpiperazin-1-yl)phenyl)methanamine was substituted with N,N-dimethyl-m-phenylene diamine HCl. Purification by preparative HPLC (reverse-phase, acetonitrile/water with 0.01 % ammonium acetate), followed by concentration *in vacuo* and lyophilization afforded the title compound (6 mg, 12%) as a white solid. ¹H NMR (400 MHz, d₆-DMSO): 8.20 (s, 1H), 7.81 (s, 2H), 6.88 (t, 1H), 6.09 (s, 1H), 6.02 (m, 2H), 5.90 (br s, 1H), 4.29 (br s, 2H), 2.80 (s, 6H); MS (EI) for C₁₉H₁₇BrN₄O₂:412:414 (Bromine isoptope MH⁺).

### (Compound 81)

### 8-bromo-2-({4-[2-(dimethylamino)ethyl]piperazin-1-yl}methyl)[1]benzofuro[3,2-d]pyrimidin-4(3H)-one

8-bromo-2-({4-[2-(dimethylamino)ethyl]piperazin-1-yl}methyl)[1]benzofuro-[3,2-d]pyrimidin-4(3H)-one was synthesized in a manner similar to **Example 2**, wherein (4-(4-methylpiperazin-1-yl)phenyl)methanamine was substituted with N,N-dimethyl-2-(piperazin-1-yl)ethanamine. The reaction mixture was concentrated *in vacuo* and purified by preparative HPLC. Concentration *in vacuo* and lyophilization afforded the title compound (27.5 mg, 22%). ¹H NMR (400 MHz, d₆-DMSO): 8.20 (m, 1H), 7.85 (m, 2H), 3.56 (m, 4H), 3.15 (m, 4H), 2.74 (s, 6H), 2.67 (m, 3H), 2.57 (m, 3H). MS (EI) for C₁₉H₂₄BrN₅O₂: 434.1 (MH⁺).

### (Compound 82)

### 8-bromo-2-{[4-(2-morpholin-4-ylethyl)piperazin-1-yl]methyl}[1]benzofuro[3,2-d]pyrimidin-4(3H)-one

8-bromo-2-{[4-(2-morpholin-4-ylethyl)piperazin-1-yl]methyl}[1]benzofuro[3,2-d]pyrimidin-4(3H)-one was synthesized in a manner similar to **Example 2**, wherein (4-(4-methylpiperazin-1-yl)phenyl)methanamine was substituted with 4-(2-(piperazin-1-yl)ethyl)morpholine. The reaction mixture was concentrated *in vacuo* and purified by preparative HPLC. Concentration *in vacuo* and lyophilization afforded the title compound (15 mg, 31.5%). ¹H NMR (400 MHz, d₆-DMSO): 8.2 (m, 1H), 7.81 (m, 2H), 3.53 (m, 6 H), 3.50 (s, 2H), 2.38 (m, 14 H). MS (EI) for C₂₁H₂₆BrN₅O₃: 476.3 (MHz).

### (Compound 100)

### 8-bromo-2-[(2-methyl-1H-imidazol-1-yl)methyl][1]benzofuro[3,2-d]pyrimidin-4(3H)-one

8-bromo-2-[(2-methyl-1H-imidazol-1-yl)methyl][1]benzofuro[3,2-d]pyrimidin-4(3H)-one was synthesized in a manner similar to **Example 2**, wherein (4-(4-methylpiperazin-1-yl)phenyl)methanamine was substituted with 2-methyl imidazole. Purification by preparative HPLC afforded the title compound (15 mg, 13%) as a white solid. ¹H NMR (400 MHz, d6-DMSO): 8.05 (s, 1H), 7.79 (s, 2H), 7.16 (s, 1H), 6.76 (s, 2H), 5.12 (s, 2H), 2.36 (s, 3H), 1.91 (s, 3H); MS (EI) for C₁₅H₁₁BrN₄O₂: 359:361 (Bromine isoptope MH⁺).

### (Compound 103)

### 1-[(8-bromo-4-oxo-3,4-dihydro[1]benzofuro[3,2-d]pyrimidin-2-yl)methyl]pyrrolidine-3-carboxylic acid

1-[(8-bromo-4-oxo-3,4-dihydro[1]benzofuro[3,2-d]pyrimidin-2-yl)methyl]pyrrolidine-3-carboxylic acid was synthesized in a manner similar to **Example 2**, wherein (4-(4-methylpiperazin-1-yl)phenyl)methanamine was substituted with pyrrolidine-3-carboxylic acid. Purification by preparative HPLC (reverse-phase, acetonitrile/water with 0.01% ammonium acetate), followed by concentration *in vacuo* and lyophilization afforded the title compound (7 mg, 5.6%). ¹H NMR (400 MHz, d₆-DMSO): 8.19 (m, 1H), 7.78 (m, 2H), 3.65 (m, 2H), 2.90 (m, 2H), 2.78 (m, 1H), 2.66 (m, 2H), 1.96 (m, 2H). MS (EI) for C₁₆H₁₄BrN₃O₄:392 (MH⁺).

### (Compound 104)

### 8-bromo-2-[({4-(dimethylamino)phenyl]methyl}amino)methyl][1]benzofuro[3,2-d]pyrimidin-4(3H)-one

8-bromo-2-[({[4-(dimethylamino)phenyl]methyl}amino)methyl][1]benzofuro-[3,2-d]pyrimidin-4(3H)-one was synthesized in a manner similar to **Example 2**, wherein (4-(4-methylpiperazin-1-yl)phenyl)methanamine was substituted with 4-(aminomethyl)-N,N-dimethylaniline. The reaction mixture was concentrated *in vacuo* and purified by preparative HPLC. Concentration *in vacuo* and lyophilization afforded the title compound (3.9 mg, 3%). ¹H NMR (400 MHz, d₆-DMSO): 8.17 (m, 1H), 7.80 (m, 2H), 7.17 (d, 2H), 6.65 (d, 2H), 3.74 (s, 2H), 3.68 (s, 2H), 2.84 (s, 6H). MS (EI) for C₂₀H₁₉BrN₄O₂: 427 (MH⁺).

### (Compound 368)

### 1-[(8-bromo-4-oxo-3,4-dihydro[1]benzofuro[3,2-d]pyrimidin-2-yl)methyl]-3-hydroxypyrrolidine-3-carboxylic acid

1-[(8-Bromo-4-oxo-3,4-dihydro[1]benzofuro[3,2-d]pyrimidin-2-yl)methyl]-3-hydroxypyrrolidine-3-carboxylic acid was synthesized in a manner similar to was synthesized in a manner similar to **Example 2** wherein (4-(4-methylpiperazin-1-yl)phenyl)methanamine was substituted with 3-hydroxy-3-pyrrolidinecarboxylic acid. ¹H NMR (400 MHz, d₆-DMSO): 8.21 (m, 1H), 7.78 (m, 2H), 3.68 (dd, 2H), 3.00 (d, 1H), 2.90-2.62 (m, 2H), 2.45 (m, 1H), 2.20-2.03 (m, 1H), 1.90-1.82 (m, 1H); MS (EI) for C₁₆H₁₄BrN₃O₅: 408 (MH⁺).

### (Compound 108)

### 2-{[(3S)-3-aminopyrrolidin-1-yl]methyl}-8-bromo[1]benzofuro[3,2-d]pyrimidin-4(3H)-one

2-{[(3S)-3-aminopyrrolidin-1-yl]methyl}-8-bromo[1]benzofuro[3,2-d]pyrimidin-4(3H)-one was synthesized in a manner similar to **Example 2**, wherein (4-(4-methylpiperazin-1-yl)phenyl)methanamine was substituted with (s)-(-)-3-(Boc amino) pyrrolidine. Purification by flash chromatography (60:40 EtOAc:Hexanes to 100% EtOAc) afforded the Boc protected title compound. The intermediate was then taken up in 1.0 mL MeOH and 0.8 mL 4N HCl/dioxane and stirred at RT for 5 hr. The precipitate was filtered and rinsed with MeOH 3X to afford the title compound as an HCl salt (39 mg, 34%). ¹H NMR (400 MHz, d6-DMSO): 8.29 (s, 1H), 7.88 (s, 2H), 4.60 (br s, 2H), 4.01 (br s, 1H), 2.45 (m, 1H), 2.15 (m, 1H); MS (EI) for C₁₅H₁₅BrN₄O₂: 363:365 (Bromine isoptope MH⁺).

### (Compound 109)

### 2-{[(3R)-3-aminopyrrolidin-1-yl]methyl}-8-bromo[1]benzofuro[3,2-d]pyrimidin-4(3H)-one

2-{[(3R)-3-aminopyrrolidin-1-yl]methyl}-8-bromo[1]benzofuro[3,2-d]pyrimidin-4(3H)-one was synthesized in a manner similar to **Example 2**, wherein (4-(4-methylpiperazin-1-yl)phenyl)methanamine was substituted with (R)-(-)-3-(Boc amino) pyrrolidine. Purification by flash chromatography (60:40 EtOAc:Hexanes to 100% EtOAc) afforded the Boc protected title compound. The intermediate was then taken up in 1.0 mL MeOH and 0.8 mL 4N HCl/dioxane and stirred at RT for 5 hr. The precipitate was filtered and rinsed with MeOH 3X to afford the title compound as an HCl salt (39 mg, 34%). ¹H NMR (400 MHz, d6-DMSO): 8.29 (s, 1H), 7.88 (s, 2H), 4.60 (br s, 2H), 4.01 (br s, 1H), 2.45 (m, 1H), 2.15 (m, 1H); MS (EI) for C₁₅H₁₅BrN₄O₂: 363:365 (Bromine isoptope MH⁺).

### (Compound 113)

### 8-bromo-2-({4-[2-(1H-imidazol-1-yl)ethyl]perazin-1-yl}methyl)[1]benzofuro[3,2-d]pyrimidin-4(3H)-one

8-bromo-2-({4-[2-(1H-imidazol-1-yl)ethyl]piperazin-1-yl}methyl)[1]benzofuro[3,2-d]pyrimidin-4(3H)-one was synthesized in a manner similar to **Example 2,** wherein (4-(4-methylpiperazin-1-yl)phenyl)methanamine was substituted with 1-(2-Imidazol-1-yl ethyl)-piperazine. Purification by preparative HPLC afforded the title compound (63 mg, 43%). ¹H NMR (400 MHz, d6-DMSO): 8.21 (s, 1H), 7.80 (m, 2H), 7.68 (s, 1H), 7.21 (s, 1H), 6.90 (s, 1H), 4.11 (m, 2H), 3.58 (s, 2H), 2.66 (m, 2H), 2.44 (m, 2H), 1.93 (s, 1H); MS (EI) for C₂₀H₂₁BrN₆O₂: 457:459 (Bromine isoptope MH⁺).

### (compound 114)

### 8-bromo-2-[(4,4-difluoropiperidin-1-yl)methyl][1]benzofuro[3,2-d]pyrimidin-4(3H)-one

8-bromo-2-[(4,4-difluoropiperidin-1-yl)methyl][1]benzofuro[3,2-d]pyrimidin-4(3H)-one in a manner similar to **Example 2**, wherein (4-(4-methylpiperazin-1-yl)phenyl)methanamine was substituted with 4,4-difluoropiperidine. The reaction mixture was concentrated *in vacuo* and purified by preparative HPLC to afford the title compound (15 mg, 15%). ¹H NMR (400 MHz, d₆-DMSO): 8.21 (m, 1H), 7.83 (m, 2H), 3.63 (s, 2H), 2.66 (m, 4H), 2.0 (m, 4H). MS (EI) for C₁₆H₁₄BrF₂N₃O₂: 398 (MH⁺).

### (Compound 118)

### 8-bromo-2-{[3-(methylamino)pyrrolidin-1-yl]methyl}[1]benzofuro[3,2-d]pyrimidin-4(3H)-one

8-bromo-2-{[3-(methylamino)pyrrolidin-1-yl]methyl}[1]benzofuro[3,2-d]pyrimidin-4(3H)-one was synthesized in a manner similar to **Example 2**, wherein (4-(4-methylpiperazin-1-yl)phenyl)methanamine was substituted with 3-(N-tert-butoxycarbonyl-N-methyl amino)pyrrolidine. Purification by flash chromatography (60:40 EtOAc:Hexanes to 100% EtOAc) afforded the Boc protected title compound. The intermediate was then taken up in 3.0 mL MeOH and 2.0 mL 4N HCI/dioxane and stirred at RT for 4 hr. The precipitate was filtered and rinsed with MeOH 3X to afford the title compound as an HCl salt (103 mg, 43%). ¹H NMR (400 MHz, d6-DMSO): 8.25 (s, 1H), 7.88 (s, 2H), 3.85 (br s, 2H), 3.44 (s, 3H), 2.60 (m, 4H); MS (EI) for C₁₆H₁₇BrN₄O₂: 379:381 (Bromine isoptope MH⁺).

### (Compound 149)

### 8-bromo-2-[(2-ethyl-1H-imidazol-1-yl)methyl][1]benzofuro[3,2-d]pyrimidin-4(3H)-one

8-bromo-2-[(2-ethyl-1H-imidazol-1-yl)methyl][1]benzofuro[3,2-d]pyrimidin-4(3H)-one was synthesized in a manner similar to **Example 2**, wherein (4-(4-methylpiperazin-1-yl)phenyl)methanamine was substituted with 2-ethyl imidazole. Purification by preparative HPLC afforded the title compound (16 mg, 13%) as a white solid. ¹H NMR (400 MHz, d6-DMSO): 8.04 (s, 1H), 7.83 (m, 2H), 7.21 (s, 1H), 6.85 (s, 1H), 5.19 (s, 2H), 2.74 (q, 2H), 1.23 (t, 3H); MS (EI) for C₁₆H₁₃BrN₄O₂₁ 373:375 (Bromine isoptope MH⁺).

### (Compound 150)

### 8-bromo-2-[(4-ethylpiperazin-1-yl)methyl][1]benzofuro[3,2-d]pyrimidin-4(3H)-one

8-bromo-2-[(4-ethylpiperazin-1-yl)methyl][1]benzofuro[3,2-d]pyrimidin-4(3H)-one was synthesized in a manner similar to **Example 2,** wherein (4-(4-methylpiperazin-1-yl)phenyl)methanamine was substituted with 1-ethylpiperazine. Purification by preparative HPLC, followed by concentration *in vacuo* and lyophilization afforded the title compound (65 mg, 53%) as a white solid. ¹H NMR (400 MHz, d₆-DMSO): 8.21 (s, 1H), 7.81 (m, 2H), 3.46 (s, 2H), 3.06 (br s, 4H), 2.45 (m, 4H), 2.32 (q, 2H), 1.90 (s, 2H), 0.973 (t, 3H); MS (EI) for C₁₇H₁₉BrN₄O₂: 391:393 (Bromine isoptope MH⁺).

### (Compound 151)

8-bromo-2-({4-[2-(methyloxy)ethyl]piperazin-1-yl}methyl)[1]benzofuro[3,2-d]pyrimidin-4(3H)-one 8-bromo-2-({4-[2-(methyloxy)ethyl]piperazin-1-yl}methyl)[1]benzofuro[3,2-d]pyrimidin-4(3H)-one was synthesized in a manner similar to **Example 2**, wherein (4-(4-methylpiperazin-1-yl)phenyl)methanamine was substituted with 1-(2-methoxyethyl)-piperazine. Purification by preparative HPLC, followed by concentration *in vacuo* and lyophilization afforded the title compound (72 mg, 55%). ¹H NMR (400 MHz, d₆-DMSO): 8.19 (s, 1H), 7.80 (s, 2H), 3.39 (s, 2H), 3.37 (br s, 8H), 3.18 (s, 3H), 2.42 (t, 4H); MS (EI) for C₁₈H₂₁BrN₄O₃: 421:423 (Bromine isoptope MH⁺).

### (Compound 165)

### 8-bromo-2-{[(pyridin-4-ylmethyl)amino]methyl}[1]benzofuro[3,2-d]pyrimidin-4(3H)-one

8-bromo-2-{[(pyridin-4-ylmethyl)amino]methyl}[1]benzofuro[3,2-d]pyrimidin-4(3H)-one was synthesized in a manner similar to **Example 2**, wherein (4-(4-methylpiperazin-1-yl)phenyl)methanamine was substituted with 4-Amino methyl pyridine. Purification by preparative HPLC, followed by concentration *in vacuo* and lyophilization afforded the title compound (21 mg, 17%).¹H NMR (400 MHz, d₆-DMSO): 8.48 (d, 2H), 8.19 (s, 1H), 7.82 (m, 2H), 7.38 (d, 2H), 3.81 (s, 2H), 3.76 (s, 2H); MS (EI) for C₁₇H₁₃BrN₄O₂: 385:387 (Bromine isoptope MH⁺).

### (Compound 166)

### 8-bromo-2-{[(2-pyridin-4-ylethyl)amino]methyl}[1]benzofuro[3,2-d]pyrimidin-4(3H)-one

8-bromo-2-{[(2-pyridin-4-ylethyl)amino]methyl}[1]benzofuro[3,2-d]pyrimidin-4(3H)-one was synthesized in a manner similar to **Example 2**, wherein (4-(4-methylpiperazin-1-yl)phenyl)methanamine was substituted with 4-(2-aminoethyl)pyridine. Purification by preparative HPLC, followed by concentration *in vacuo* and lyophilization afforded the title compound (14 mg, 11 %). ¹H NMR (400 MHz, d₆-DMSO): 8.74 (d, 2H), 8.28 (s, 1H), 7.87 (s, 2H), 7.83 (d, 2H), 4.32 (s, 2H), 3.54 (t, 2H), 3.30 (t, 2H); MS (EI) for C₁₈H₁₅BrN₄O₂: 399:401 (Bromine isoptope MH⁺).

### (Compound 177)

### N-2-[(8-bromo-4-oxo-3,4-dihydro[1]benzofuro[3,2-d]pyrimidin-2-yl)methyl]-2-methylaninamide

N-2-[(8-bromo-4-oxo-3,4-dihydro[1]benzofuro[3,2-d]pyrimidin-2-yl)methyl]-2-methylalaninamide was synthesized in a manner similar to **Example 2,** wherein (4-(4-methylpiperazin-1-yl)phenyl)methanamine was substituted with methyl-α-aminoisobutyrate. Purification by preparative HPLC, afforded methyl 2-((8-bromo-4-oxo-3,4-dihydrobenzofuro[3,2-d]pyrimidin-2-yl)methylamino)-2-methylpropanoate which was then taken up in 2 mL of 7M ammonia in methanol and heated to 100 °C overnight in a sealed vessel. The resulting precipitate was filtered and dried affording the title compound (7.0mg, 47%). ¹H NMR (400 MHz, d₆-DMSO + D₂O): 8.25 (s, 1H), 7.83 (m, 2H), 3.73 (s, 2H), 1.31 (s, 6H); MS (EI) for C₁₅H₁₅BrN₄O₃ : 379:381 (Bromine isoptope MH⁺).

### (Compound 189)

### N-[(8-bromo-4-oxo-3,4-dihydro[1]benzofuro[3,2-d]pyrimidin-2-yl)methyl] methylalanine

N-[(8-bromo-4-oxo-3,4-dihydro[1]benzofuro[3,2-d]pyrimidin-2-yl)methyl]-2-methylalanine was synthesized in a manner similar to **Example 2,** wherein (4-(4-methylpiperazin-1-yl)phenyl)methanamine was substituted with methyl-α-aminoisobutyrate. Purification by preparative HPLC, afforded methyl 2-((8-bromo-4-oxo-3,4-dihydrobenzofuro[3,2-d]pyrimidin-2-yl)methylamino)-2-methylpropanoate which was then taken up in 1.0 mL of 2N LiOH and heated to 50 °C for 1.5 hr. The reaction was acidified to pH 4 with 1N HCl and extracted with EtOAc, dried with Na₂SO₄ and concentrated to afford the title compound (3.0mg, 20%). ¹H NMR (400 MHz, d₆-DMSO + D₂O) 8.25 (s, 1H), 7.86 (m, 2H), 4.15 (m, 2H), 1.43 (s, 6H); MS (EI) for C₁₅H₁₄BrN₃O₄: 380:382 (Bromine isoptope MH⁺).

### (Compound 190)

### 8-bromo-2-{[(1,1-dimethyl-2-pyrrolidin-1-ylethyl)amino]methyl}[1]benzofuro[3,2-d]pyrimidin-4(3H)-one

8-bromo-2-{[(1,1-dimethyl-2-pyrrolidin-1-ylethyl)amino]methyl}[1]benzofuro[3,2-d]pyrimidin-4(3H)-one was synthesized in a manner similar to **Example 2**, wherein (4-(4-methylpiperazin-1-yl)phenyl)methanamine was substituted with 1,1-dimethyl-2-pyrrolidin-1-ylethyl)amine. Purification by preparative HPLC, followed by concentration *in vacuo* and lyophilization afforded the title compound (58mg, 23%). ¹H NMR (400 MHz, d₆-DMSO): 8.10 (d, 1H), 7.75 (m, 2H), 3.70 (s, 2H), 2.69 (br s, 2H), 2.56 (s, 1H), 2.48 (br s, 2H), 1.88 (br s, 1H), 1.76 (br s, 4H), 1.11 (s, 6H); MS (EI) for C₁₉H₂₃BrN₄O₂: 419:421 (Bromine isoptope MH⁺).

### (Compound 307)

### 8-bromo-2-{[(3R)-3-fluoropyrrolidin-1-yl]methyl}[1]benzofuro[3,2-d]pyrimidin-4(3H)-one

8-bromo-2-{[(3R)-3-fluoropyrrolidin-1-yl]methyl}[1]benzofuro[3,2-d]pyrimidin-4(3H)-one was synthesized in a manner similar to **Example 2**, wherein (4-(4-methylpiperazin-1-yl)phenyl)methanamine was substituted with R-(-)-3-fluoropyrrolidine HCl. Purification by silica gel chromatography afforded the title compound (18mg, 16%). ¹H NMR (400 MHz, d₆-DMSO): 8.21 (d, 1H), 7.83 (m, 2H), 5.29 (t, 0.5H), 5.15 (t, 0.5H), 4.12 (m, 1H), 3.69 (s, 2H), 2.88 (m, 3H), 2.15 (m, 1H), 1.90 (m, 1H); MS (EI) for C₁₅H₁₃BrFN₃O₂: 366:367 (Bromine isoptope MH⁺).

### (Compound 308)

### 8-bromo-2-{[(3S)-3-fluoropyrrolidin-1-yl]methyl}[1]benzofuro[3,2-d]pyrimidin-4(3H)-one

8-bromo-2-{[(3S)-3-fluoropyrrolidin-1-yl]methyl}[1]benzofuro[3,2-d]pyrimidin-4(3H)-one was synthesized in a manner similar to **Example 2**, wherein (4-(4-methylpiperazin-1-yl)phenyl)methanamine was substituted with S-(-)-3-fluoropyrrolidine HCl. Purification by silica gel chromatography afforded the title compound (45mg, 40%). ¹H NMR (400 MHz, d₆-DMSO): 8.21 (d, 1H), 7.83 (m, 2H), 5.29 (t, 0.5H), 5.15 (t, 0.5H), 4.12 (m, 1H), 3.69 (s, 2H), 2.88 (m, 3H), 2.15 (m, 1H), 1.90 (m, 1H); MS (EI) for C₁₅H₁₃BrFN₃O₂: 366:367 (Bromine isoptope MH⁺).

### (Compound 317)

### 8-bromo-2-[(3-fluoropiperidin-1-yl)methyl][1]benzofuro][3,2-d]pyrimidin-4(3H)-one

8-bromo-2-[(3-fluoropiperidin-1-yl)methyl][1]benzofuro[3,2-d]pyrimidin-4(3H)-one was synthesized in a manner similar to **Example 2**, wherein (4-(4-methylpiperazin-1-yl)phenyl)methanamine was substituted with 3-fluoropiperidine HCl. Purification by silica gel chromatography twice afforded the title compound (18 mg, 40%). ¹H NMR (400 MHz, d₆-DMSO): 8.21 (m, 1H), 7.83 (m, 2H), 4.74 (m, 0.5H), 4.61 (m, 0.5H), 3.58 (s, 2H), 2.09 (m, 1H), 2.51 (m, 2H), 1.80 (m, 2H), 1.50 (m, 2H); MS (EI) for C₁₆H₁₅BrFN₃O₂: 380:382 (Bromine isoptope MH⁺).

### (Compound 318)

### 8-bromo-2-[(3,3-difluoropyrrolidin-1-yl)methyl][1]benzofuro[3,2-d]pyrimidin-4(3H)-one

8-bromo-2-[(3,3-difluoropyrrolidin-1-yl)methyl][1]benzofuro[3,2-d]pyrimidin-4(3H)-one was synthesized in a manner similar to **Example 2**, wherein (4-(4-methylpiperazin-1-yl)phenyl)methanamine was substituted with 3,3-difluoroazetidine HCl. Purification by silica gel chromatography twice afforded the title compound (21 mg, 17%). ¹H NMR (400 MHz, d₆-DMSO): 8.21 (m, 1H), 7.83 (m, 2H), 3.71 (s, 2H), 3.08 (t, 2H), 2.88 (t, 2H), 2.28 (heptet, 2H); MS (EI) for C₁₅H₁₂BrF₂N₃O₂: 380:382 (Bromine isoptope MH⁺).

### EXAMPLE 3: (Scheme 1- generating ethers as a product)

### (Compound 15)

### 8-bromo-2-[(phenyloxy)methyl][1]benzofuro[3,2-d]pyrimidin-4(3H)-one

To a solution of 8-bromo-2-(chloromethyl)benzofuro[3,2-d]pyrimidin-4(3H)-one **6** (111 mg, 0.35 mmol) in 5mL anhydrous DMF was added phenol (200 mg, 2.12 mmol) and K₂CO₃ (292 mg, 2.12 mmol). The reaction mixture was heated to 80 °C for 24 hours, after which it was cooled down and partitioned between water and ethyl acetate. The aqueous layer was extracted with ethyl acetate (2 x 50 mL) and the combined organic layers were washed with brine, dried over Na₂SO₄ and concentrated *in vacuo.* Purification by preparative HPLC, followed by concentration *in vacuo* and lyophilization afforded the title compound (4.8 mg, 4%) as a white solid. ¹H NMR (400 MHz, d₆-DMSO): 8.21 (m, 1H), 7.83 (m, 2H), 7.33 (m, 2H), 7.07 (m, 2H), 7.00 (m, 1H), 5.09 (s, 2H); MS (EI) for C₁₇H₁₁BrN₂O₃: 371 (MH⁺).

### *(Compound 102)

### 8-bromo-2-[({[4-(4-methylpiperazin-1-yl)phenyl]methyl}oxy)methyl][1]benzofuro[3,2-d]pyrimidin-4(3H)-one

8-bromo-2-[({[4-(4-methylpiperazin-1-yl)phenyl]methyl}oxy)methyl][1]benzofuro[3,2-d]pyrimidin-4(3H)-one was synthesized in a manner similar to **Example 3, Compound 15**, wherein phenol was substituted with [4-(4-methylpiperazine-lyl) phenyl] methanol. Purification by preparative HPLC afforded the title compound (5 mg, 3%) as a white solid. ¹H NMR (400 MHz, d6-DMSO): 8.01 (s, 1H), 7.67 (m, 2H), 7.23 (d, 2H), 7.00 (d, 2H), 5.10 (t, 1H), 4.53 (s, 2H), 4.39 (d, 2H), 3.93 (m, 2H), 3.75 (m, 2H), 3.52 (m, 2H), 3.29 (s, 3H); MS (EI) for C₂₃H₂₃BrN₄O₃: 483:485 (Bromine isoptope MH⁺). 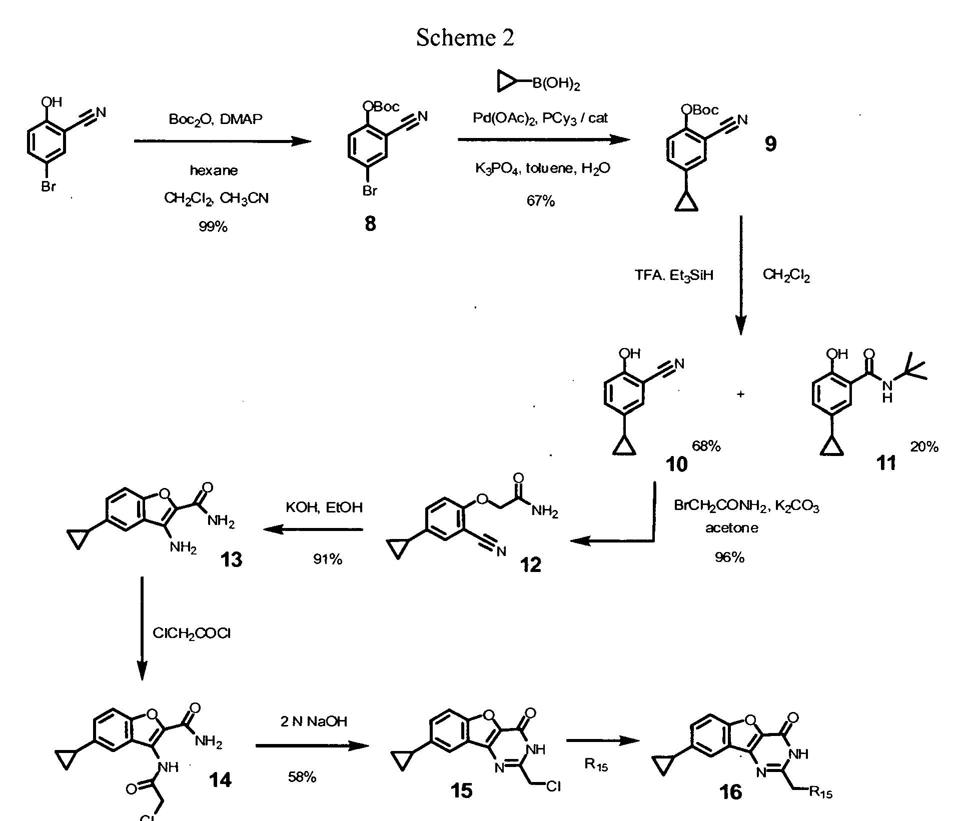 wherein R₁₅ is described within the compounds within this example.

### EXAMPLE 4: (Scheme 2)

### 4-Bromo-2-cyanophenyl tert-butyl carbonate 8

A 250-mL round bottom flask was charged with 4-bromo-2-cyanophenol (3.29 g, 16.64 mmol), di*tert*-butyl dicarbonate (3.99 g, 18.28 mmol), and DMAP (101 mg, 0.83 mmol). *n*-Hexane (50 mL), dichloromethane (10 mL) and acetonitrile (10 mL) were added at room temperature and under a positive pressure of nitrogen. The mixture was briefly heated at 50 °C until all material went into solution, then stirred for another 20 min at room temperature. The reaction was judged to have reached completion by TLC. The mixture was diluted with EtOAc (150 mL), washed with 1 N NaHSO₄ (20 mL), water (20 mL) and brine (10 mL). The organic layer was dried over MgSO₄ After purification by flash chromatography (9:1 hexane/EtOAc), the title compound **(8)** was obtained as colorless oil (4.89 g), 99% yield.
¹H NMR (400 MHz, CDCl₃) δ 7.78 (d, *J*= 2.5 Hz, 1H), 7.72 (dd, *J*= 2.5, *J*= 11 Hz, 1H), 7.24 (d, *J=* 9 Hz, 1H), 1.58 (s, 9H); ¹³C NMR (100 MHz, CDCl₃) δ 151.9, 150.3, 137.4, 135.8, 124.7, 119.1, 113.9, 109.2, 85.9, 53.7. MS (ESI+) for C₁₂H₁₂BrNO₃: 299 (MH⁺).

### tert-Butyl 2-cyano-4-cyclopropylphenyl carbonate 9

A mixture of 4-bromo-2-cyanophenyl ter-t-butyl carbonate (**8**, 1.366 g, 4.58 mmol), cyclopropyl boronic acid (531 mg, 6.18 mmol), [Pd(OAc)₂](165 mg, 0.245 mmol), tricyclohexylphosphine (148 mg, 0.528 mmol), and K₃PO₄ (3.86 g, 18.2 mmol) in toluene (10 mL) and water (1 mL) was heated at 110 °C for 3 h. The mixture was cooled to room temperature and poured onto water, extracted twice with ethyl acetate. The combined organic layers were washed with water and brine, and dried over MgSO₄. After purification by flash chromatography (85:15 hexane/EtOAc), the title compound **(9)** was obtained as yellowish oil (671 mg), 56% yield.
¹H NMR (400 MHz, CDCl₃) δ 7.33 (d, *J*= 2.5 Hz, 1H), 7.29 (dd, *J*= 2.5, *J*= 8 Hz, 1H), 7.20 (d, *J*= 8 Hz, 1H), 1.91 (m, 1H), 1.58 (s, 9H), 1.03 (m, 2H), 0.70 (m, 2H); ¹³C NMR (100 MHz, CDCl₃) δ 151.1, 150.4, 143.0, 131.7, 130.5, 122.8, 115.4, 107.1, 85.2, 27.8, 15.0, 9.8; MS (ESI+) for C₁₅H₁₇NO₃: 260 (MH⁺).

### 5-Cyclopropyl-2-hydroxybenzonitrile 10

A mixture of *tert*-butyl 2-cyano-4-cyclopropylphenyl carbonate (**9**, 671 mg, 2.59 mmol) in dichloromethane (20 mL), trifluoroacetic acid (4 mL) and triethylsilane (2.5 mL) was stirred at room temperature for 16 h. Volatiles were removed in vacuo and replaced with same volume of fresh solvent and reagents. The mixture was heated at 45 °C for 1 h, then concentrated under reduced pressure. After purification by flash chromatography (8:2 hexane/EtOAc), the title compound **(10)** was obtained as yellowish oil (280 mg), 68% yield, along with N-*tert*-butyl-5-cyclopropyl-2-hydroxybenzamide (**11**, 119 mg) as side-product, 20% yield.
¹H NMR (400 MHz, CDCl₃) δ 7.17 (m, 1H), 7.14 (m, 1H), 6.94 (d, *J=* 9 Hz, 1H), 1.82 (m, 1H), 0.93 (m, 2H), 0.60 (m, 2H); ¹³C NMR (100 MHz, CDCl₃) δ 157.4, 136.4, 132.8, 130.2, 116.7,99.2,21.4, 14.6; MS (ESI+) for C₁₀H₉NO: 160 (MH⁺).

### 2-(2-Cyano-4-cyclopropylphenoxy)acetamide 12

A 100-mL round bottom flask was charged with 5-cyclopropyl-2-hydroxybenzonitrile (**11**, 280 mg, 1.76 mmol), bromoacetamide (263 mg, 1.91 mmol), potassium carbonate (545 mg, 3.94 mmol) and acetone (20 mL). The mixture was heated at 65 °C for 18 h under nitrogen. The reaction was judged to have reached completion by LC-MS. The mixture was diluted with EtOAc (150 mL), washed with water (20 mL) and brine (10 mL). The organic layer was dried over MgSO₄. After removal of solvents under reduced pressure, the title compound **(12)** was obtained as a white solid (366 mg), 96% yield, which was deemed pure enough for the following treatment.
¹H NMR (400 MHz, CDCl₃) δ 7.31 (m, 2H), 6.91 (m, 1H), 4.55 (s, 2H), 3.64 (br s, 2H), 1.89 (m, 1H), 1.01 (m, 2H), 0.66 (m, 2H); ¹³C NMR (100 MHz, CDCl₃) δ 170.6, 156.9, 138.7, 132.7, 131.0, 116.5, 112.7, 101.8,67.5, 14.5, 9.1; MS (ESI+) for C₁₂H₁₂N₂O₂: 217 (MH⁺).

### 3-Amino-5-cyclopropylbenzofuran-2-carboxamide 13

To a solution of 2-(2-cyano-4-cyclopropylphenoxy)acetamide (**12**, 366 mg, 1.69 mmol) in ethanol (15 mL), a solution of KOH (322 mg, 5.74 mmol) in ethanol (10 mL) was added at 80 °C. Reaction was judged complete (TLC, LC-MS) after 1 h. The mixture was cooled to room temperature and diluted with ethyl acetate (100 mL), then washed with water (30 mL), 1M pH=8 phosphate buffer. The combined aqueous layers were extracted again with ethyl acetate (50 mL) and chloroform (2x 40 mL). The combined organic layers were washed with brine (2x), and dried over MgSO₄. After purification by flash chromatography (96:4 dichloromethane/methanol), the title compound **(13)** was obtained as a solid (324 mg), 91 % yield.
¹H NMR (400 MHz, CDCl₃) δ 7.32 (s, 1H), 7.24 (d, *J*= 8 Hz, 1H), 7.17 (dd, *J*= 8, *J*= 2 Hz, 1H), 6.4 (br s, 2H), 5.25 (br s, 2H), 2.00 (m, 1H), 0.99 (m, 2H), 0.70 (m, 2H); ¹³C NMR (100 MHz, CDCl₃) δ 164.4, 151.7, 138.1, 136.6, 126.9, 122.3, 116.4, 11.4, 15.1, 8.8; MS (ESI+) for C₁₂H₁₂N₂O₂: 217 (MH⁺).

### 3-(2-Chloroacetamido)-5-cyclopropylbenzofuran-2-carboxamide 14

A solution of 3-amino-5-cyclopropylbenzofuran-2-carboxamide **13** (183 mg, 0.846 mmol) in chloroacetyl chloride (3 mL) was heated to 40 °C for 1 h. The reaction mixture was quenched with saturated aqueous NaHCO₃ (60 mL) and extracted with chloroform (3 x 50 mL). The combined organic phases were washed with water, dried over MgSO₄ and concentrated *in vacuo* to afford the title compound **(14)** as a white solid (308 mg), contaminated by chloroacetic acid.
¹H NMR (400 MHz, CDCl₃, spiked with CD₃OD) δ 8.10 (s, 1H), 7.32 (m, 1H), 7.20 (dd, *J=* 8, *J*= 2 Hz, 1H), 6.8 (br s, 1H), 4.09 (s, 2H), 2.03 (m, 1H), 0.99 (m, 2H), 0.74 (m, 2H); ¹³C NMR (100 MHz, CDCl₃ spiked with CD₃OD) δ 169.8, 164.8, 152.3, 139.5, 127.3, 123.2, 121.6, 111.5, 41.3, 15.6, 9.3; MS (ESI+) for C₁₄H₁₃ClN₂O₃: 293 (MH⁺).

### 2-(Chloromethyl)-8-cyclopropylbenzofuro[3,2-d]pyrimidin-4(3H)-one 15

A solution of 3-(2-chloroacetamido)-5-cyclopropylbenzofuran-2-carboxamide **14** (0.846 mmol) in 10 mL of 2N NaOH was heated to 40 °C for 15 min. The reaction mixture was brought to acidic pH with 1N NaHSO₄ and extracted with ethyl acetate (2 x 50 mL). The combined organic phases were washed with water, brine, and dried over MgSO₄. After purification by flash chromatography (97:3 to 94:6 dichloromethane/methanol), the title compound **(15)** was obtained as a solid (135 mg), 58% yield over two steps.
¹H NMR (400 MHz, CDCl₃, spiked with CD₃OD) δ 7.74 (s, 1H), 7.55 (d, *J*= 8 Hz, 1H), 7.39 (dd, *J*= 8, *J*= 2 Hz, 1H), 4.61 (s, 2H), 2.08 (m, 1H), 1.05 (m, 2H), 0.79 (m, 2H); MS (ESI+) for C₁₄H₁₁ClN₂O₂: 275 (MH⁺).

### (Compound 23)

### 8-Cyclopropyl-2-[(4-methylpiperazin-1-yl)methyl][1]benzofuro[3,2-d]pyrimidin-4(3H)-one

To a solution of 2-(chloromethyl)-8-cyclopropylbenzofuro[3,2-*d*]pyrimidin-4(3*H*)-one **15** (135 mg, 0.49 mmol) in 8 mL anhydrous ethanol was added 4-methylpiperazine (125 mg, 1.25 mmol). The reaction mixture was heated to 80 °C for 16 hours, cooled down and concentrated *in vacuo.* After purification by flash chromatography (93:6:1 dichloromethane/methanol/28% (w/w) ammonium hydroxide), the title compound was obtained as a solid (140 mg), 84% yield. ¹H NMR (400 MHz, CDCl₃) δ 7.66 (s, 1H), 7.51 (d, *J*= 8 Hz, 1H), 7.36 (dd, *J*= 8, *J*= 2 Hz, 1H), 3.69 (s, 2H), 2.68 (br s, 4H), 2.53 (br s, 4H), 2.33 (s, 3H), 2.04 (m, 1H), 1.03 (m, 2H), 0.77 (m, 2H); ¹³C NMR (100 MHz, CDCl₃) δ 155.9, 155.1, 153.2, 144.7, 140.5, 138.7, 129.1, 122.5, 117.5, 112.6, 60.3, 55.1, 53.5, 46.1, 15.6, 9.7; MS (ESI+) for C₁₉H₂₂N₄O₂: 339 (MH⁺). 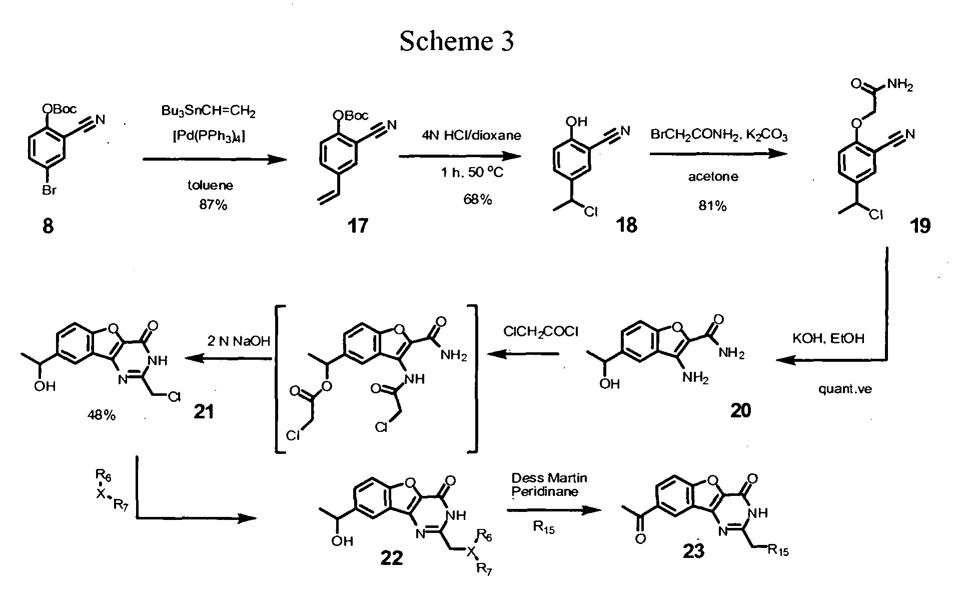 wherein X, R₆, R₇ and R₁₅ are described within the compounds within this example.

### EXAMPLE 5: (Scheme 3)

### tert-Butyl 2-cyano-4-vinylphenyl carbonate 17

A mixture of 4-bromo-2-cyanophenyl *tert*-butyl carbonate (**8**, 878 mg, 2.94 mmol), tributylvinylstannane (1.155 g, 3.64 mmol) and [Pd(PPh₃)₄] (72 mg, 0.062 mmol) in toluene (25 mL) was heated at 110 °C for 6 h. The mixture was cooled to room temperature and filtered through Celite^{®}. The clear solution was concentrated under reduced pressure. After purification by flash chromatography (85: 15 hexane/EtOAc), the title compound **(17)** was obtained as colorless oil (625 mg), 87% yield.
¹H NMR (400 MHz, CDCl₃) δ 7.66 (d, *J*= 2.5 Hz, 1H), 7.62 (dd, *J=* 2.5, *J=* 8 Hz, 1H), , 7.28 (d, *J*= 8 Hz, 1 H), 6.66 (dd, *J*= 11, *J*= 17 Hz, 1H), 5.76 (d, *J*= 11 Hz, 1H), 5.37 (d, *J*= 17 Hz, 1H), 1.58 (s, 9H); ¹³C NMR (100 MHz, CDCl₃) δ 151.9, 150.8, 136.3, 134.2, 131.8, 130.9, 123.2, 116.9, 115.2, 107.6, 85.5, 27.8; MS (ESI+) for C₁₄H₁₅NO₃: 246 (MH⁺).

### 5-(1-Chloroethyl)-2-hydroxybenzonitrile 18

A mixture of *tert*-butyl 2-cyano-4-vinylphenyl carbonate (**17**, 597 mg, 2.44 mmol) was treated with 4 N HCl in dioxane (6 mL) at 50°C for 90 min. The reaction mixture was quenched with saturated aqueous NaHCO₃ (40 mL) and extracted with EtOAc (2 x 50 mL). The combined organic phases were washed with water and brine, dried over MgSO₄ After purification by flash chromatography (8:2 hexane/EtOAc), the title compound **(18)** was obtained as yellowish oil (301 mg), 68% yield.
¹H NMR (400 MHz, CD₃OD) δ 7.48 (d, *J*= 2.5 Hz, 1H), 7.45 (dd, *J*= 2.5, *J*= 8 Hz, 1H), 6.91 (d, *J* = 8 Hz, 1H), 4.75 (q, *J*= 6 Hz, 1H), 1.39 (d, *J*= 6 Hz, 3H); ¹³C NMR (100MHz, CD₃OD) δ 159.5, 138.1, 132.1, 130.0, 116.9, 115.9, 99.0, 68.5, 24.2; MS (ESI+) for C₉H₈ClNO: 182 (MH⁺).

### 2-(4-(1-Chloroethyl)-2-cyanophenoxy)acetamide 19

A 100-mL round bottom flask was charged with 5-(1-chloroethyl)-2-hydroxybenzonitrile (**18**, 301 mg, 1.66 mmol), bromoacetamide (252 mg, 1.83 mmol), potassium carbonate (486 mg, 3.52 mmol) and acetone (20 mL). The mixture was heated at 65 °C for 24 h under nitrogen. The reaction was judged to have reached completion by LC-MS. The mixture was diluted with EtOAc (150 mL), washed with water (20 mL) and brine (10 mL). The organic layer was dried over MgSO₄. After removal of solvents under reduced pressure, the title compound **(19)** was obtained as a white solid (322 mg), 81 % yield, which was deemed pure enough for the following treatment.
¹H NMR (400 MHz, CD₃OD) δ 7.65 (m, 1H), 7.62 (m, 1H), 7.08 (d, *J*= 8 Hz, 1H), 4.81 (q, *J*= 6 Hz, 1H), 4.67 (s, 2H), 1.40 (d, *J*= 6 Hz, 3H); ¹³C NMR (100 MHz, CD₃OD) δ 171.5, 158.7, 140.8, 132.0, 130.6, 116.1, 112.8, 101.6, 68.2, 67.4, 24.3; MS (ESI+) for C₁₁H₁₁ClN₂O₂: 239 (MH⁺).

### 3-Amino-5-(1-hydroxyethyl)benzofuran-2-carboxamide 20

To a solution of 2-(4-(1-chloroethyl)-2-cyanophenoxy)acetamide (**19**, 322 mg, 1.35 mmol) in ethanol (40 mL), a solution of KOH (223 mg, 3.97 mmol) in ethanol (20 mL) was added at 50 °C. The mixture was heated at 75 °C. Reaction was judged complete (TLC, LC-MS) after 1.5 h. The mixture was cooled to room temperature and diluted with ethyl acetate (100 mL), then washed with water (30 mL), 1 M pH=8 phosphate buffer. The combined aqueous layers were extracted again with ethyl acetate (50 mL) and chloroform (2x 40 mL). The combined organic layers were washed with brine (2x), and dried over MgSO₄. After purification by flash chromatography (96:4 dichloromethane/methanol), the title compound **(20)** was obtained as a solid (324 mg), quantitative yield.
¹H NMR (400 MHz, CD3OΔ) δ 7.74 (m, 1H), 7.47 (m, 1H), 7.35 (d, *J*=8 Hz, 1H), 4.93 (q, *J*= 6 Hz, 1H), 1.48 (d, *J*= 6 Hz, 3H); MS (ESI+) for C₁₁H₁₂N₂O₃: 221 (MH⁺).

### 2-(Chloromethyl)-8-(1-hydroxyethyl)benzofuro[3,2-d]pyrimidin-4(3H)-one 21

A solution of 3-amino-5-(1-hydroxyethyl)benzofuran-2-carboxamide (**20,** 324 mg, 1.47 mmol) in chloroacetyl chloride (3 mL) was heated to 40 °C for 1 h. The reaction mixture was quenched and vigorously stirred for 15 min with saturated aqueous NaHCO₃ (60 mL), extracted with EtOAc (2 x 50 mL) and chloroform (2 x 50 mL). The combined organic phases were washed with water, dried over MgSO₄ and concentrated *in vacuo* to afford the intermediate, 1-(2-carbamoyl-3-(2-chloroacetamido)benzofuran-5-yl)ethyl 2-chloroacetate, as a white solid (1.005 g), contaminated by chloroacetic acid. A solution of this material, which did not undergo further purification, in 8 mL of 2 N NaOH was heated to 40 °C for 10 min, then stirred at room temperature for another 20 min. The reaction mixture was brought to acidic pH with 1N NaHSO₄ and extracted with ethyl acetate (2 x 50 mL) and dichloromethane (50 mL). The combined organic phases were washed with water, brine, and dried over MgSO₄. After purification by flash chromatography (97:3 to 94:6 dichloromethane/methanol), the title compound **(21)** was obtained as a solid (197 mg), 48% yield over two steps.
¹H NMR (400 MHz, 3:1 CD₃OD/CDCl₃) δ 8.07 (m, 1H), 7.69 (m, 1H), 7.64 (d, *J*= 8 Hz, 1H), 5.02 (q, *J*= 6 Hz, 1H), 4.63 (s, 2H), 1.53 (d, *J=* 6 Hz, 3H); ¹³C NMR (100 MHz, 3:1 CD₃OD/CDCl₃) δ 156.6, 153.8, 143.4, 138.9, 128.3, 122.0, 118.0, 112.6, 69.4, 42.2, 24.9; MS (ESI+) for C₁₃H₁₁ClN₂O₃: 279 (MH⁺).

### *(Compound 27) 8-(1-Hydroxyethyl)-2-[(4-methylpiperazin-1-yl)methyl)[1]benzofuro 3,2-d]pyrimidin-4(3H)-one

To a solution of 2-(chloromethyl)-8-(1-hydroxyethyl)benzofuro[3,2-*d*]pyrimidin-4(3*H*)-one (**21,** 197 mg, 0.70 mmol) in 15 mL anhydrous ethanol was added 4-methylpiperazine (192 mg, 1.92 mmol). The reaction mixture was heated to 80 °C for 16 hours, cooled down and concentrated *in vacuo.* After purification by flash chromatography (90:8.5:1.5 dichloromethane/methanol/28% (w/w) ammonium hydroxide), the title compound was obtained as a solid (205 mg), 86% yield.
¹H NMR (400 MHz, 3:1 CD₃OD/CDCl₃) δ 8.06 (m, 1H), 7.67 (dd, *J*= 2.5, *J*= 8 Hz, 1H), 7.62 (d, *J*= 8 Hz, 1H), 5.02 (q, *J=* 6 Hz, 1H), 3.68 (s, 2H), 2.69 (br, 4H), 2.59 (br, 4H), 2.33 (s, 3H), 1.53 (d, *J*= 6 Hz, 3H); ¹³C NMR (100 MHz, 3:1 CD₃OD/CDCl₃) δ 156.6, 156.1, 154.5, 144.5, 143.2, 138.7, 128.1, 122.1, 118.1, 112.5, 69.4, 60.4, 54.6, 52.6, 45.1, 25.0; MS (ESI+) for C₁₈H₂₂N₄O₃: 343 (MH⁺).

### (Compound 29)

### 8-Acetyl-2-[(4-methylpiperazin-1-yl)methyl][1]benzofuro[3,2-d]pyrimidin-4(3H)-one

To a solution of **Compound 27** (54 mg, 0.158 mmol) in 5 mL anhydrous dichloromethane was added anhydrous NMP (0.2 mL) and Dess-Martin periodinane (120 mg, 0.28 mmol). The reaction mixture was stirred at room temperature for 2 hours. The reaction mixture was quenched with saturated aqueous NaHCO₃ (10 mL) and extracted with EtOAc (2x 40 mL) and chloroform (2 x 30 mL). The combined organic phases were washed with water, dried over MgSO₄. After purification by flash chromatography (88:10:2 dichloromethane/methanol/28% (w/w) ammonium hydroxide), the title compound was obtained as a solid (56 mg), quantitative yield. ¹H NMR (400 MHz, 3:1 CD₃OD/CDCl₃) δ 8.77 (m, 1H), 8.30 (dd, *J*= 2.5, *J*= 8 Hz, 1H), 7.81 (m, 1H), 3.72 (s, 2H), 2.75 (br, 8H), 2.74 (s, 3H), 2.47 (s, 3H); MS (ESI+) for C₁₈H₂₀N₄O₃: 341 (MH⁺). 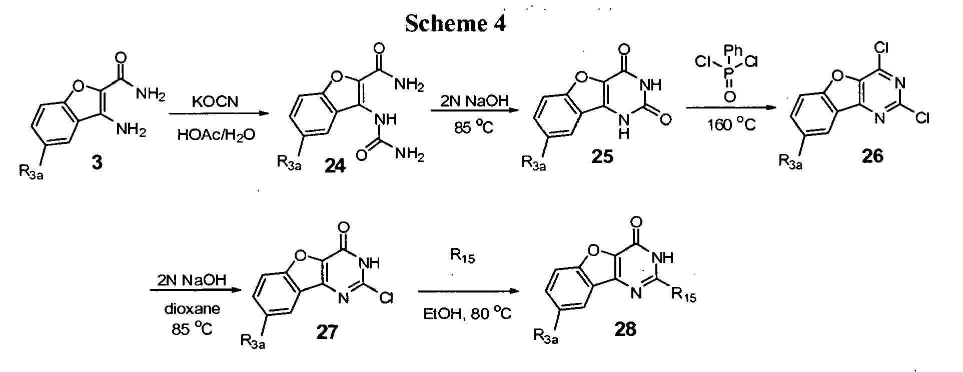 wherein R₃ₐ is as defined in the disclosure above and R₁₅ is described within the compounds within this example.

### EXAMPLE 6: (Scheme 4)

### 5-bromo-3-ureidobenzofuran-2-carboxamide 24

To a suspension of 3-amino-5-bromobenzofuran-2-carboxamide (1.0 g, 3.95 mmol) in 40 mL acetic acid and 20 mL water was added sodium cyanate (1.1 g, 18.6 mmol). The reaction was stirred for 48 hours at room temperature, diluted with water (100 mL) and extracted with ethyl acetate (2 x 150 mL). The combined organic phases were washed with brine, dried over Na₂SO₄ and concentrated *in vacuo.* The product was purified by SiO₂ flash chromatography (20:80 hexanes/ethyl acetate to 20:80 methanol/ethyl acetate to afford 5-bromo-3-ureidobenzofuran-2-carboxamide (0.7 g, 60%) as a white solid. ¹H NMR (400 MHz, d₆-DMSO): 9.03 (s, 1H), 8.37 (d, 1H), 8.05 (br s, 1H), 7.79 (br s, 1H), 7.60 (dd, 1H), 7.5 (d, 1H), 6.7 (br s, 2H) ); MS (EI) for C₁₀H₈BrN₃O₃: 298.1 (MH⁺).

### 8-bromobenzofuro[3,2-d]pyrimidine-2,4(1H,3H)-dione 25

A suspension of 5-bromo-3-ureidobenzofuran-2-carboxamide (**24**) (0.7 g, 2.35 mmol) in 20 mL aqueous 2N NaOH was heated to 95 °C for 2 hours. The reaction mixture was cooled to room temperature and acidified with 1N HCl (50 mL). The precipitate was washed with a large quantity of water (500 mL) and dried to afford 8-bromobenzofuro[3,2-d]pyrimidine-2,4(1H,3H)-dione (0.5 g, 76%) as a white solid. ¹H NMR (400 MHz, d₆-DMSO): 8.15 (m, 1H), 7.76 (m, 2H); MS (EI) for C₁₀H₅BrN₂O₃: 281.1 (MH⁺).

### 8-bromo-2,4-dichlorobenzofuro[3,2-d]pyrimidine 26

A suspension of 8-bromo-2,4-dichlorobenzofuro[3,2-d]pyrimidine (**25**) (0.820 g, 2.35 mmol) in 2 mL of phenylphosphonic dichloride was heated to 160 °C for 2 hours. After cooling down to room temperature, the reaction was poured on ice-water and extracted with ethyl acetate (2 x 150 mL). The combined organic phases were washed with brine, dried over Na₂SO₄ and concentrated *in vacuo.* The product was purified by SiO₂ flash chromatography (90:10 hexanes/ethyl acetate) to afford 8-bromo-2,4-dichlorobenzofuro[3,2-d]pyrimidine (0.372 g, 48%) as a yellow solid. ¹H NMR (400 MHz, d₆-DMSO): 8.54 (m, 1H), 8.05 (m, 2H).

### 8-bromo-2-chlorobenzofuro[3,2-d]pyrimidin-4(3H)-one 27

To a solution of 8-bromo-2,4-dichlorobenzofuro[3,2-d]pyrimidine (**26**) (0.372 g, 1.12 mmol) in 5 mL dioxane was added 2N NaOH (2 mL) and heated to 85 °C for 10 minutes. The reaction mixture was cooled to room temperature, acidified with 1N HCl (10 mL) and extracted with ethyl acetate (2 x 100 mL). The combined organic phases were washed with brine, dried over Na₂SO₄ and concentrated *in vacuo* to afford 8-bromo-2-chlorobenzofuro[3,2-d]pyrimidin-4(3H)-one (0.338 g, quant.). ¹H NMR (400 MHz, d₆-DMSO): 8.24 (m, 1H), 7.85 (m, 2H); MS (EI) for C₁₀H₄BrClN₂O₂: 297 (M-H).

### (Compound 115)

### 8-bromo-2-(4-methylpiperazin-1-yl)[1]benzofuror3,2-d]pyrimidin-4(3H)-one

To a solution of 8-bromo-2-chlorobenzofuro[3,2-d]pyrimidin-4(3H)-one (27) (53 mg, 0.18 mmol) in 2 mL ethanol was added N-methylpiperazine (180 mg, 1.80 mmol) and heated to 80 °C for 14 hours. The reaction mixture was concentrated *in vacuo* and purified by preparative HPLC. Concentration *in vacuo* and lyophilization afforded 8-bromo-2-(4-methylpiperazin-1-yl)[1]benzofuro[3,2-d]pyrimidin-4(3H)-one (23.7 mg, 36%). ¹H NMR (400 MHz, d₆-DMSO): 8.05 (m, 1H), 7.72 (m, 2H), 3.65 (m, 4H), 2.40 (m, 4H), 2.22 (s, 3H); MS (EI) for C₁₅H₁₅BrN₄O₂:363 (MH⁺).

### (Compound 180)

### 8-bromo-2-(phenylamino)[1]benzofuro[3,2-d]pyrimidin-4(3H)-one

8-bromo-2-(phenylamino)[1]benzofuro[3,2-d]pyrimidin-4(3H)-one was synthesized in a manner similar to **Example 6, Compound 115,** wherein N-methylpiperazine was substituted with aniline. The product was filtered off, washed with cold EtOH and dried *in vacuo* to afford the title compound (53.4 mg, 75%). ¹H NMR (400 MHz, d₆-DMSO): 11.25 (s, 1H), 8.84 (m, 1H), 8.18 (m, 1H), 7.76 (m, 4H), 7.39 (m, 2H), 7.08 (m, 1H). MS (EI) for C₁₆H₁₀BrN₃O₂: 356.1 (MH⁺).

### (Compound 193)

### 8-bromo-2-{[3-(dimethylamino)propyl]amino}[1]benzofuro[3,2-d]pyrimidin-4(3H)-one

8-bromo-2-{[3-(dimethylamino)propyl]amino}[1]benzofuro[3,2-d]pyrimidin-4(3H)-one was synthesized in a manner similar to **Example 6, Compound 115,** wherein N-methylpiperazine was substituted with N,N-dimethylpropane-1,3-diamine. Purification by preparative HPLC was followed by concentration *in vacuo* and lyophilization to afford the title compound as an acetate salt (20.8 mg, 28%). ¹H NMR (400 MHz, d₆-DMSO): 7.95 (m, 1H), 7.67 (m, 2H), 3.33 (m, 2H), 2.29 (t, 2H), 1.84 (s, 3H), 1.67 (m, 2H). MS (EI) for C₁₅H₁₇BrN₄O₂: 363 (M-H).

### (Compound 196)

### 8-bromo-2-pyrrolidin-1-yl[1]benzofuro[3,2-d]pyrimidin-4(3H)-one

8-bromo-2-pyrrolidin-1-yl[1]benzofuro[3,2-d]pyrimidin-4(3H)-one was synthesized in a manner similar to **Example 6, Compound 115,** wherein N-methylpiperazine was substituted with pyrrolidine. Purification by preparative HPLC was followed by concentration *in vacuo* and lyophilization to afford the title compound (7.7 mg, 13.7%). ¹H NMR (400 MHz, d₆-DMSO): 8.00 (m, 1H), 7.70 (m, 2H), 3.52 (m, 4H), 1.91 (m, 4H). MS (EI) for C₁₄H₁₂BrN₃O₂: 334 (MH⁺).

### (Compound 213)

### 8-bromo-2-(piperidin-4-ylamino)[1]benzofuro(3,2-d]pyrimidin-4(3H)-one

t-butyl-4-(8-bromo-4-oxo-3,4-dihydrobenzofuro[3,2-d]pyrimidin-2-ylamino)piperidine-1-carboxylate was synthesized in a manner similar to **Example 6, Compound 115,** wherein N-methylpiperazine was substituted with tert-butyl 4-aminopiperidine-1-carboxylate (HCl salt) and Cs₂CO₃ was employed as a base. After heating at 100 °C for 48 hours, the reaction mixture was cooled to room temperature, diluted with water and extracted with ethyl acetate (2 x 50 mL). The combined organic phases were washed with brine, dried over Na₂SO₄ and concentrated *in vacuo* to afford tert-butyl 4-(8-bromo-4-oxo-3,4-dihydrobenzofuro[3,2-d]pyrimidin-2-ylamino)piperidine-1-carboxylate. To a suspension of the crude material in methanol (4 mL) was added 4N HCl/dioxane (2 mL) and stirred at room temperature for 12 hours. The reaction mixture was concentrated *in vacuo* and purified by preparative HPLC. Concentration *in vacuo* and lyophilization afforded the title compound (12 mg, 13%). ¹H NMR (400 MHz, d₆-DMSO): 7.99 (m, 1H), 7.70 (m, 2H), 3.95 (m, 1H), 3.08 (m, 2H), 2.72 (m, 2H), 1.97 (m, 2H), 1.46 (m, 2H). MS (EI) for C₁₅H₁₅BrN₄O₂: 363 (MH⁺).

### (Compound 214)

### 8-bromo-2-(pyridin-3-ylamino)[1]benzofuro[3,2-d]pyrimidin-4(3H)-one

8-bromo-2-(pyridin-3-ylamino)[1]benzofuro[3,2-d]pyrimidin-4(3H)-one was synthesized in a manner similar to **Example 6, Compound 115,** wherein N-methylpiperazine was substituted with pyridin-3-amine. The product was filtered off and dried *in vacuo* to afford the title compound (33 mg, 40%).
¹H NMR (400 MHz, d₆-DMSO): 9.35 (s, 1H), 9.15 (m, 1H), 8.11 (s, 1H), 7.82 (m, 1H), 7.74 (m, 3H), 6.77 (s, 1H). MS (EI) for C₁₅H₉BrN₄O₂: 357.1 (MH⁺).

### (Compound 225)

### 8-bromo-2-({[4-(4-methylpiperazin-1-yl)phenyl]methyl}amino)[1]benzofuro[3,2-d]pyrimidin-4(3H)-one

8-bromo-2-({[4-(4-methylpiperazin-1-yl)phenyl]methyl}amino)[1]benzofuro[3,2-d]pyrimidin-4(3H)-one was synthesized in a manner similar to **Example 6, Compound 115,** wherein N-methylpiperazine was substituted with (4-(4-methylpiperazin-1-yl)phenyl)methanamine and Cs₂CO₃ was used as a base. The reaction mixture was concentrated *in vacuo* and purified by preparative HPLC. Concentration *in vacuo* and lyophilization afforded the title compound (12.4 mg, 13%). ¹H NMR (400 MHz, d₆-DMSO): 8.02 (m, 1H), 7.73 (m, 2H), 7.33 (m, 2H), 6.99 (m, 2H), 4.9 (m, 2H), 3.82 (m, 2H), 3.52 (m, 2H, overlapped), 3.38 (s, 3H, overlapped), 2.85 (m, 4H). MS (EI) for C₂₂H₂₂BrN₅O₂: 467.9 (MH⁺).

### (Compound 227)

### 8-bromo-2-{4-[2-(dimethylamino)ethyl]piperazin-1-yl}[1]benzofuro[3,2-d]pyrimidin-4(3H)-one

8-bromo-2-{4-[2-(dimethylamino)ethyl]piperazin-1-yl}[1]benzofuro[3,2-d]pyrimidin-4(3H)-one was synthesized in a manner similar to **Example 6, Compound 115,** wherein N-methylpiperazine was substituted with N,N-dimethyl-2-(piperazin-1-yl)ethanamine. The reaction mixture was concentrated *in vacuo* and purified by preparative HPLC. Concentration *in vacuo* and lyophilization afforded the title compound (23.6 mg, 24 %). ¹H NMR (400 MHz, d₆-DMSO): 8.02 (m, 1H), 7.69 (m, 2H), 3.62 (m, 4H), 2.49 (m, 4H), 2.44 (m, 4H), 2.2 (s, 6H). MS (EI) for C₁₈H₂₂BrN₅O₂: 420 (MH⁺).

### (Compound 228)

### 8-bromo-2-[4-(1-methylpiperidin-4-yl)piperazin-1yl][1]benzofuro[3,2-d]pyrimidin-4(3H)-one

8-bromo-2-[4-(1-methylpiperidin-4-yl)piperazin-1-yl][1]benzofuro[3,2-d]pyrimidin-4(3H)-one was synthesized in a manner similar to **Example 6, Compound 115,** wherein N-methylpiperazine was substituted with 1-(1-methylpiperidin-4-yl)piperazine. The reaction mixture was concentrated *in vacuo* and purified by preparative HPLC. Concentration *in vacuo* and lyophilization afforded the title compound (57 mg, 60%). ¹H NMR (400 MHz, d₆-DMSO): 8.03 (m, 1H), 7.71 (m, 2H), 3.63 (m, 4H), 2.83 (m, 2H), 2.55 (m, 4H), 2.51 (m, 2H, overlapped), 2.16 (s, 3H), 1.87 (m, 1H), 1.74 (m, 2H), 1.42 (m, 2H). MS (EI) for C₂₀H₂₄BrN₅O₂: 445.9 (MH⁺).

### (Compound 245)

### 8-bromo-2-[4-(N,N-diethylglycyl)piperazin-1-yl][1]benzofuro[3,2-d]pyrimidin-4(3H)-one

8-bromo-2-[4-(N,N-diethylglycyl)piperazin-1-yl][1]benzofuro[3,2-d]pyrimidin-4(3H)-one 8-bromo-2-[4-(1-methylpiperidin-4-yl)piperazin-1-yl][1]benzofuro[3,2-d]pyrimidin-4(3H)-one was synthesized in a manner similar to **Example 6, Compound 115,** wherein N-methylpiperazine was substituted with 2-(diethylamino)-1-(piperazin-1-yl)ethanone. The reaction mixture was concentrated *in vacuo* and purified by preparative HPLC reverse-phase, 0.1% TFA in acetonitrile/0.05% TFA in water). Concentration *in vacuo* and lyophilization afforded the title compound (15.3 mg, 16%). ¹H NMR (400 MHz, d₆-DMSO): 8.06 (m, 1H), 7.76 (m, 2H), 4.30 (m, 2H), 3.75 (m, 4H), 3.66 (m, 2H), 3.54 (m, 2H), 3.14 (m, 4H), 1.22 (t, 6H). MS (EI) for C₂₀H₂₄BrN₅O₃: 461.9 (MH⁺).

### (Compound 246)

### 8-bromo-2-{4-[3-(dimethylamino)propyl]piperazin-1-yl}[1]benzofuro[3,2-d]pyrimidin-4(3H)-one

8-bromo-2-{4-[3-(dimethylamino)propyl]piperazin-1-yl}[1]benzofuro[3,2-d]pyrimidin-4(3H)-one was synthesized in a manner similar to **Example 6, Compound 115,** wherein N-methylpiperazine was substituted with N,N-dimethyl-3-(piperazin-1-yl)propan-1-amine. The reaction mixture was concentrated *in vacuo* and purified by preparative HPLC.
Concentration *in vacuo* and lyophilization afforded the title compound (23 mg, 24%). ¹H NMR (400 MHz, d₆-DMSO): 8.02 (m, 1H), 7.70 (m, 2H), 3.63 (m, 4H), 2.44 (m, 4H), 2.32 (m, 4H), 2.20 (s, 6H), 1.61 (m, 2H). MS (EI) for C₁₉H₂₄BrN₅O₂: 433.9 (MH⁺), 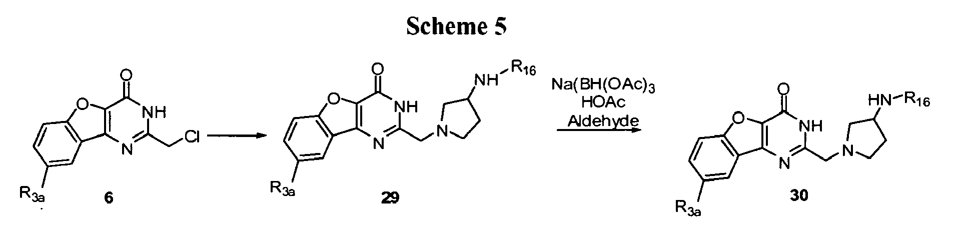 wherein R₃ₐ is as defined in the disclosure above and R₁₆ is hydrogen or piperidin-4-ylmethyl)amino]pyrrolidin-1-yl.

### EXAMPLE 7: (Scheme 5)

### (Compound 28)

### 2-[(3-aminopyrrolidin-1-yl)methyl]-8-bromo[1]benzofuro[3,2-d]pyrimidin-4(3H)-one HCl salt

To a solution of 8-bromo-2-(chloromethyl)benzofuro[3,2-d]pyrimidin-4(3H)-one 6 (300 mg, 0.96 mmol) in 2.0 mL anhydrous DMF was added 3-(tert-butoxy carbonyl amino)pyrrolidine (178 mg, 0.96 mmol) and Cs₂CO₃ (311 mg, 0.96 mmol). The reaction mixture was heated to 80 °C at 150 W for 10 minutes in a CEM-Discover microwave reactor. The reaction mixture was filtered and concentrated *in vacuo.* Purification by flash chromatography (60:40 ethyl acetate:hexanes to 100% ethyl acetate afforded the Boc protected title compound as a solid. The pure intermediate was then dissolved in MeOH (3.0mL) and 4M HCl in Dioxanes (2.0 mL) and stirred at room temperature for 6 h. The resulting precipitate was filtered and rinsed with MeOH 3X to afforded the title compound (163 mg, 47%) as a light tan solid. ¹H NMR (400 MHz, d₆-DMSO+D₂O): 8.32 (s, 1H), 7.79 (m, 2H), 4.40 (m, 3H), 4.00 (m, 1H), 3.60 (m, 4H), 3.19 (m, 1H), 2.48 (m, 1H), 2.09 (m, 1H); MS (EI) for C₁₅H₁₅BrN₄O₂: 363 (MH⁺).

### (Compound 110)

### 8-bromo-2-({3-[(piperidin-4-ylmethyl)amino]pyrrolidin-1-yl}methyl)[1]benzofuro[3,2-d]pyrimidin-4(3H)-one

To a solution of **Compound 28** (30 mg, 0.083 mmol) in 2.0 mL anhydrous 1,2-DCE was added 4-formyl-piperidine-1-carboxylic acid t-butyl ester (19 mg, 0.091 mmol) and acetic acid (0.1 mL). The reaction mixture was stirred at ambient temp. Before adding sodium triacetoxy borohydride (52 mg, 0.249 mmol). After overnight stirring at room temperature, an additional 1.0 eq of sodium triacetoxy borohydride was added and stirred until the reaction went to completion. The reaction was quenched with EtOAc/ H₂O, partitioned, dried with Na₂SO₄, concentrated and purified via flash chromatography to afford the Boc protected title compound as a solid. The pure intermediate was then dissolved in MeOH (1.0mL) and 4M HCl in Dioxanes (0.7 mL) and stirred at room temperature for 2 h. The reaction was then concentrated, dissolved in H₂O and lyophilized 4X to afforded the title compound (40mg, 90%). ¹H NMR (400 MHz, d₆-DMSO+D₂O): 8.34 (s, 1H), 7.87 (m, 2H), 4.55 (br s, 2H), 3.25 (m, 4H), 2.83 (m, 3H), 1.79 (m, 5H), 1.29 (m, 3H); MS (EI) for C₂₁H₂₆BrN₅O₂: 460:462 (Bromine isotope, MH⁺).

### EXAMPLE 8

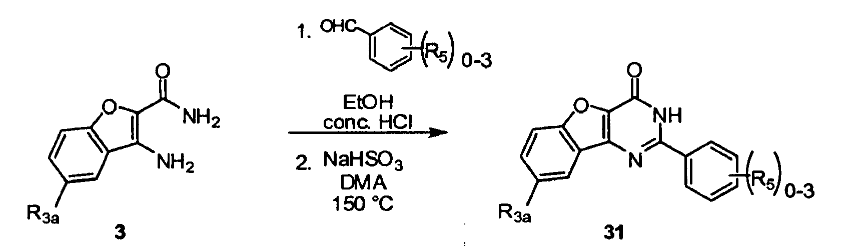

wherein R₃ₐ is as defined in the disclosure above and R₅ is described within the compounds within this example.

### *(Compounds 30)

### 8-Bromo-2-(2-chlorophenyl)[1]benzofuro[3,2-d]pyrimidin-4(3H)-one

A solution of 3-amino-5-bromobenzofuran-2-carboxamide **3** (200 mg, 0.78 mmol) and 2-chlorobenzaldehyde (175 µL, 1.55 mmol) in 3 mL anhydrous ethanol was heated to 80 °C. Concentrated hydrochloric acid (20 µL) was added and a precipitate formed immediately. The precipitate was filtered, washed with 1 mL ethanol and air dried to give 190 mg of imine. The precipitate was suspended in 3 mL of dimethylacetamide and heated to 150 °C for 2 hr. Sodium bisulfite (80 mg, 0.76 mmol) was added and the reaction was heated at 150 °C for another 45 min. After cooling, the reaction mixture was filtered and the filtrate was diluted with 5 mL of water. The precipitate was filtered, washed with 1 mL of methanol and dried under vacuum to give 19 mg of 8-bromo-2-(2-chlorophenyl)[1]benzofuro[3,2-d]pyrimidin-4(3H)-one. ¹H NMR (400 MHz, d₆-DMSO): 13.34 (m, 1H), 8.19 (d, 1H), 7.82 (d, 1H), 7.79 (dd, 1H), 7.63 (dd, 1H), 7.59 (dd, 1H), 7.54 (m, 1H), 7.46 (m, 1H); MS (EI) for C₁₆H₈BrClN₂O₂: 375 (MH⁺).

### *(Compound 36)

### 8-Bromo-2-(2,6-dichlorophenyl)[1]benzofuro[3,2-d]pyrimidin-4(3H)-one

8-Bromo-2-(2,6-dichlorophenyl)[1]benzofuro[3,2-d]pyrimidin-4(3H)-one was synthesized in a manner similar to **Example 8,** wherein 2-chlorobenzaldehyde was substituted with 2,6-dichlorobenzaldehyde. ¹H-NMR (400MHz, d₆-DMSO): 13.64 (s, 1H), 8.29 (s br, 1H), 7.88 (m, 2H), 7.67 (m, 3H). MS (EI) for C₁₆H₇BrCl₂N₂O₂: 410.8 (MH⁺). 192

### *(Compound 37)

### 8-Bromo-2-(2,5-dichlorophenyl)[1]benzofuro[3,2-d]pyrimidin-4(3H)-one

8-Bromo-2-(2,5-dichlorophenyl)[1]benzofuro[3,2-d]pyrimidin-4(3H)-one was synthesized in a manner similar to **Example 8,** wherein 2-chlorobenzaldehyde was substituted with 2,5-dichlorobenzaldehyde. ¹H-NMR (400MHz, d₆-DMSO): 13.47 (s, 1H), 8.25 (d, 1H), 7.87 (s, 1H), 7.85 (m, 2H), 7.68 (d, 2H). MS (EI) for C₁₆H₇BrCl₂N₂O₂: 410.7 (MH⁺).

### *(Compound 38)

### 8-Bromo-2-(2-bromophenyl)[1]benzofuro[3,2-d]pyrimidin-4(3H)-one

8-Bromo-2-(2-bromophenyl)[1]benzofuro[3,2-d]pyrimidin-4(3H)-one was synthesized in a manner similar to **Example 8,** wherein 2-chlorobenzaldehyde was substituted with 2-bromobenzaldehyde. ¹H NMR (400 MHz, *d₆*-DMSO): 13.38 (br s, 1H), 8.23 (d, 1H), 7.85 (s, 1H), 7.84 (d, 1H), 7.81 (d, 1H), 7.65 (dd, 1H), 7.54 (td, 1H), 7.50 (td, 1H); MS (EI) for C₁₆H₈Br₂N₂O₂: 421 (MH⁺).

### *(Compound 39)

### 8-bromo-2-(2-chloro-6-fluorophenyl)[1]benzofuro[3,2-d]pyrimidin-4(3H)-one

8-bromo-2-(2-chloro-6-fluorophenyl)[1]benzofuro[3,2-d]pyrimidin-4(3H)-one was synthesized in a manner similar to **Example 8,** wherein 2-chlorobenzaldehyde was substituted with 2-chloro-6-fluorobenzaldehyde. ¹H NMR (400 MHz, *d₆*-DMSO): 13.32 (br s, 1H), 8.25 (d, 1H), 7.87 (s, 1H), 7.86 (d, 1H), 7.81 (td, 1H), 7.65 (m, 1H), 7.41(q, 2H); MS (EI) for C₁₆H₇BrCl FN₂O₂: 394 (MH⁺).

### *(Compound 40)

### 8-bromo-2-(2-iodoohenyl)[1]benzofuro[3,2-d]pyrimidin-4(3H)-one

8-bromo-2-(2-iodophenyl)[1]benzofuro[3,2-d]pyrimidin-4(3H)-one was synthesized in a manner similar to **Example 8,** wherein 2-chlorobenzaldehyde was substituted with 2-iodobenzaldehyde. ¹H NMR (400 MHz, d₆-DMSO): 13.35 (s, 1H), 8.26 (d, 1H), 8.01 (d, 1H), 7.87 (m, 2H), 7.59 (m, 2H), 7.32 (m, 1H); MS (EI) for C₁₆H₈BrIN₂O₂: 467 (MH⁺).

### *(Compound 41)

### 8-bromo-2-[2-chloro-4-(dimethylamino)phenyl][1]benzofuro[3,2-d]pyrimidin-4(3H)-one

8-bromo-2-[2-chloro-4-(dimethylamino)phenyl][1]benzofuro[3,2-d]pyrimidin-4(3H)-one was synthesized in a manner similar to **Example 8,** wherein 2-chlorobenzaldehyde was substituted with 2-chloro-4-(dimethylamino)benzaldehyde. ¹H NMR (400 MHz, d₆-DMSO): 13.05 (s, 1H), 8.20 (d, 1H), 7.82 (m, 2H), 7.46 (d, 1H), 6.77 (m, 2H), 2.85 (s, 6H); MS (EI) for C₁₈H₁₃BrClN₃O₂: 420 (MH⁺).

### *(Compound 42)

### 8-bromo-2-(2-chloro-4-fluorophenyl)[1]benzofuro[3,2-d]pyrimidin-4(3H)-one

8-bromo-2-(2-chloro-4-fluorophenyl)[1]benzofuro[3,2-d]pyrimidin-4(3H)-one was synthesized in a manner similar to **Example 8,** wherein 2-chlorobenzaldehyde was substituted with 2-chloro-4-fluorobenzaldehyde. ¹H NMR (400 MHz, d₆-DMSO): 13.45 (s, 1H), 8.25 (d, 1H), 7.86 (m, 2H), 7.77 (m, 1H), 7.68 (m, 1H), 7.43 (m, 1H); MS (EI) for C₁₆H₇BrClN₂O₂: 395 (MH⁺).

### *(Compound 43)

### 8-bromo-2-(3-bromopyridin-4-yl)[1]benzofuro[3,2-d]pyrimidin-4(3H)-one

8-bromo-2-(3-bromopyridin-4-yl)[1]benzofuro[3,2-d]pyrimidin-4(3H)-one was synthesized in a manner similar to **Example 8,** wherein 2-chlorobenzaldehyde was substituted with 3-bromo-4-pyridinecarboxaldehyde.
¹H NMR (400 MHz, d₆-DMSO): 13.55 (s, 1H), 8.96 (s, 1H), 7.76 (d, 1H), 8.27 (s, 1H), 7.88 (m, 2H), 7.75 (d, 1H); MS (EI) for C₁₅H₇Br₂N₃O₂: 422 (MH⁺).

### *(Compound 44)

### 8-bromo-2-(2-chloro-5-fluorophenyl)[1]benzofuro[3,2-d]pyrimidin-4(3H)-one

8-bromo-2-(2-chloro-5-fluorophenyl)[1]benzofuro[3,2-d]pyrimidin-4(3H)-one was synthesized in a manner similar to **Example 8,** wherein 2-chlorobenzaldehyde was substituted with 2-chloro-5-fluorobenzaldehyde was substituted. ¹H NMR (400 MHz, d₆-DMSO): 13.47 (s, 1H), 8.26 (d, 1H), 7.85-7.90 (m, 3H), 7.69 (d, 2H); MS (EI) for C₁₆H₇BrClFN₂O₂: 393 (MH⁺).

### *(Compound 52)

### 8-bromo-2-(2,4-dichlorophenyl)[1]benzofuro[3,2-d]pyrimidin-4(3H)-one

8-bromo-2-(2,4-dichlorophenyl)[1]benzofuro[3,2-d]pyrimidin-4(3H)-one was synthesized in a manner similar to **Example 8,** wherein 2-chlorobenzaldehyde was substituted with 2,4-dichlorobenzaldehyde. ¹H NMR (400 MHz, d₆-DMSO): 13.45 (s, 1H), 8.24 (s, 1H), 7.86 (m, 3H), 7.73 (d, 1H), 7.62 (dd, 1H); MS (EI) for C₁₆H₇BrCl₂N₂O₂: 411 (MH⁺).

### *(Compound 55)

### 8-bromo-2-(2,3-dichlorophenyl)[1]benzofuro[3,2-d]pyrimidin-4(3H)-one

8-bromo-2-(2,4-dichlorophenyl)[1]benzofuro[3,2-d]pyrimidin-4(3H)-one was synthesized in a manner similar to **Example 8**, wherein 2-chlorobenzaldehyde was substituted with 2,3-dichlorobenzaldehyde. ¹H NMR (400 MHz, d₆-DMSO): 13.47 (s, 1H), 8.25 (s, 1H), 7.86 (m, 3H), 7.69 (d, 1H), 7.57 (t, 1H); MS (EI) for C₁₆H₇BrCl₂N₂O₂: 411 (MH⁺).

### *(Compound 87)

### 2-(2-chloro-6-fluorophenyl)-8-cyclopropyl[1]benzofuro[3,2-d]pyrimidin-4(3H)-one

The title compound was synthesized in a manner similar to **Example 8,** wherein 3-amino-5-bromobenzofuran-2-carboxamide was substituted with 3-amino-5-cyclopropylbenzofuran-2-carboxamide (whose preparation is described in **Example 4)** and 2-chlorobenzaldehyde with 2-chloro-6-fluorobenzaldehyde. After purification by flash chromatography (91:8:1 dichloromethane/methanol/28% (w/w) ammonium hydroxide), the title compound was obtained as a solid (89 mg), 36% yield.
¹H NMR (400 MHz, 3:1 CD₃OD/CDCl₃) δ 7.79 (m, 1H), 7.61 (m, 2H), 7.45 (m, 2H), 7.31 (m, 1H), 3.32 (m, 1 H), 2.08 (m, 1H), 1.04 (m, 2H), 0.77 (m, 2H); ¹⁹F NMR (376 MHz, 3:1 CD₃OD/CDCl₃) δ -113.5 (dd, *J*= 5.3, *J*= 9.4 Hz); MS (ESI+) for C₁₉H₁₂ClFN₂O₂: 355 (MH⁺).

### *(Compound 64)

### 8-bromo-2-[2-chloro-3-(methyloxy)phenyl][1]benzofuro[3,2-d]pyrimidin-4(3H)-one

8-bromo-2-[2-chloro-3-(methyloxy)phenyl][1]benzofuro[3,2-d]pyrimidin-4(3H)-one was synthesized in a manner similar to **Example 8,** wherein 2-chlorobenzaldehyde was substituted with 2-chloro-3-methoxybenzaldehyde. 1H NMR (400 MHz, d6-DMSO): 13.39 (s, 1H), 8.24 (s, 1H), 7.87 (m, 2H), 7.48 (m, 1H), 7.36 (d, 1H), 7.24 (d, 1H), 3.94 (s, 3H). MS (EI) for C17 H10 Br Cl N2 03 : 407 (MH+).

### *(Compound 65)

### 8-bromo-2-[2-(trifluoromethyl)phenyl][1]benzofuro[3,2-d]pyrimidin-4(3H)-one

8-bromo-2-[2-(trifluoromethyl)phenyl][1]benzofuro[3,2-d]pyrimidin-4(3H)-one was synthesized in a manner similar to **Example 8,** wherein 2-chlorobenzaldehyde was substituted with 2-trifluoromethylbenzaldehyde. 1H NMR (400 MHz, d6-DMSO): 13.45 (s, 1H), 8.16 (s, 1H), 7.81 (m, 6H). MS (EI) for Cl7 H8 Br F3 N2 O2: 410 (MH+).

### *(Compound 66)

### 8-bromo-2-[2-bromo-4,5-bis(methyloxy)pheny][1]benzofuro[3,2-d]pyrimidin-4(3H)-one

8-bromo-2-[2-bromo-4,5-bis(methyloxy)phenyl][1]benzofuro[3,2-d]pyrimidin-4(3H)-one was synthesized in a manner similar to **Example 8,** wherein 2-chlorobenzaldehyde was substituted with 2-bromo-4,5-dimethoxybenzaldehyde. 1H NMR (400 MHz, d6-DMSO): 13.29 (s, 1H), 8.26 (s, 1H), 7.87 (m, 2H), 7.30 (d, 2H), 3.87 (s, 3H), 3.80 (s, 3H). MS (EI) for C18 H12 Br2 N2 04: 481 (MH+).

### *(Compound 67)

### 8-bromo-2-[2-fluoro-5-(methyloxy)phenyl[1]benzofuro[3,2-d]pyrimidin-4(3H)-one

8-bromo-2-[2-fluoro-5-(methyloxy)phenyl][1]benzofuro[3,2-d]pyrimidin-4(3H)-one was synthesized in a manner similar to **Example 8,** wherein 2-chlorobenzaldehyde was substituted with 2-fluoro-5-methoxybenzaldehyde. 1H NMR (400 MHz, d6-DMSO): 13.30 (s, br, 1H), 8.24 (s, 1H), 7.85 (m, 2H), 7.32 (m, 2H), 7.16 (m, 1H), 3.80 (s, 3H). MS (EI) for C17 H10 Br F N2 O3: 390 (MH+).

### *(Compound 68)

### 8-bromo-2-[2-chloro-3,4-bis(methyloxy)pheny][1]benzofuro[3,2-d]pyrimidin-4(3H)-one

8-bromo-2-[2-chloro-3,4-bis(methyloxy)phenyl][1]benzofuro[3,2-d]pyrimidin-4(3H)-one was synthesized in a manner similar to **Example 8,** wherein 2-chlorobenzaldehyde was substituted with 2-chloro-3,4-dimethoxybenzaldehyde. 1H NMR (400 MHz, d6-DMSO): 13.28 (s, 1H), 8.24 (s, 1H), 7.86 (m, 2H), 7.42 (d, 1H), 7.21 (d, 1H), 3.92 (s, 3H), 3.81 (s, 3H). MS (EI) for C18 H12 Br Cl N2 O4: 437 (MH+).

### *(Compound 69)

### 8-bromo-2-(5-chloro-2-thienyl)[1]benzofuro[3,2-d]pyrimidin-4(3H)-one

8-bromo-2-(5-chloro-2-thienyl)[1]benzofuro[3,2-d]pyrimidin-4(3H)-one was synthesized in a manner similar to **Example 8,** wherein 2-chlorobenzaldehyde was substituted with 5-chloro-2-thiophenecarboxaldehyde. 1H NMR (400 MHz, d6-DMSO): 13.37 (s, 1H), 8.18 (s, 1H), 8.06 (d, 1H), 7.84 (m, 2H), 7.29 (d, 1H). MS (EI) for C14 H6 Br Cl N2 02 S: 383 (MH+).

### *(Compound 70)

### 8-bromo-2-(2,6-difluorophenyl)[1]benzofuro[3,2-d]pyrimidin-4(3H)-one

8-bromo-2-(2,6-difluorophenyl)[1]benzofuro[3,2-d]pyrimidin-4(3H)-one was synthesized in a manner similar to **Example 8,** wherein 2-chlorobenzaldehyde was substituted with 2,6-dichlorobenzaldehyde. 1H NMR (400 MHz, d6-DMSO): 13.67 (s, 1H), 8.27 (s, 1H), 7.89 (m, 2H), 7.71 (m, 1H), 7.30 (m, 2H). MS (EI) for C16 H7 Br F2 N2 O2: 378 (MH+).

### *(Compound 107)

### 8-bromo-2-(3,5-dichloropyridin4-yl)[1]benzofuro[3,2-d]pyrimidin-4(3H)-one

8-bromo-2-(3,5-dichloropyridin-4-yl)[1]benzofuro[3,2-d]pyrimidin-4(3H)-one was synthesized in a manner similar to **Example 8,** wherein 2-chlorobenzaldehyde was substituted with 3,5-dichloroisonicotinaldehyde. 1H NMR (400 MHz, d6-DMSO): 13.81 (s, 1H), 8.91 (s, 2H), 8.30 (s, 1H), 7.90 (m, 2H). MS (EI) for C15 H6 Br Cl2 N3 O2: 412 (MH+).

### *(Compound 57)

### 8-Bromo-2-pyridin-2-yl[1]benzofuro[3,2-d]pyrimidin-4(3H)-one.

8-Bromo-2-pyridin-2-yl[1]benzofuro[3,2-d]pyrimidin-4(3H)-one was synthesized manner similar to **Example 8,** wherein 2-chlorobenzaldehyde was substituted with pyridine-2-carboxaldehyde. ¹H NMR (400 MHz, d₆-DMSO): 12.41 (broad s, 1H), 8.77 (d, 1H), 8.47 (d, 1H), 8.30 (s, 1H), 8.16 (dd, 1H), 7.88 (d, 1H), 7.86 (d, 1H), 7.65 (dd, 1H). MS (EI) for C₁₅ H₈ Br N₃ O₂: 343 (MH⁺).

### *(Compound 58)

### 8-Bromo-2-(2-fluorophenyl)[1]benzofuro[3,2-d]pyrimidin-4(3H)-one

8-Bromo-2-(2-fluorophenyl)[1]benzofuro[3,2-d]pyrimidin-4(3H)-one was synthesized in a manner similar to **Example 8,** wherein 2-chlorobenzaldehyde was substituted with 2-fluorobenzaldehyde. ¹H NMR (400 MHz, d₆-DMSO): 12.42 (broad s, 1H), 7.96 (s, 1H), 7.89 (d, 1H), 7.62 (d, 1H), 7.61 (d, 1H), 7.53 (dd, 1H), 7.46 (s, 1H), 7.35 (dd, 1H). MS (EI) for C₁₆ H₈ Br F N₂ O₂: 360 (MH⁺).

### *(Compound 59)

### 8-bromo-2-(2-thienyl)[1]benzofuror3,2-d]pyrimidin-4(3H)-one

8-Bromo-2-(2-thienyl)[1]benzofuro[3,2-d]pyrimidin-4(3H)-one was synthesized in a manner similar to **Example 8,** wherein 2-chlorobenzaldehyde was substituted with thiophene-2-carboxaldehyde. ¹H NMR (400 MHz, d₆-DMSO): 12.39 (broad s, 1H), 8.23 (d, 1H), 7.85 (s, 1H), 7.84 (d, 1H), 7.69 (d, 1H), 7.46 (d, 1H), 7.17 (dd, 1H). MS (EI) for C₁₄ H₇ Br N₂ O₂ S: 348 (MH⁺).

### *(Compound 53)

### methyl 4-(4-oxo-3,4-dihydro[1]benzofuro[3,2-d]pyrimidin-2-yl)benzoate

Methyl 4-(4-oxo-3,4-dihydro[1]benzofuro[3,2-d]pyrimidin-2-yl)benzoate was synthesized in the same manner as **Example 8** wherein 2-chlorobenzaldehyde was replaced with methyl 4-formylbenzoate, and 3-amino-5-bromobenzofuran-2-carboxamide 3 was replaced with 3-amino-benzofuran-2-carboxamide. 1H NMR (400 MHz, d6-DMSO): 13.32 (s, 1H), 8.30 (d, 2H), 8.11 (m, 3H), 7.86 (d, 1H), 7.71 (m, 1H), 7.53 (m, 1H), 3.90 (s, 3H). MS (EI) for C₁₈H₁₂N₂O₄: 321 (MH⁺).

### *(Compound 63)

### 2-(2-chlorophenyl)-8-methyl[1]benzofuro[3,2-d]pyrimidin-4(3H)-one

2-(2-chlorophenyl)-8-methyl[1]benzofuro[3,2-d]pyrimidin-4(3H)-one was synthesized in the same manner as **Example 8** wherein 3-amino-5-bromobenzofuran-2-carboxamide **3** was replaced with 3-amino-5-methyl-benzofuran-2-carboxamide. 3-Amino-5-methyl-benzofuran-2-carboxamide was synthesized in the same manner as **Example 1** wherein 5-bromo-2-hydroxybenzonitrile **1** was replaced with 5-methyl-2-hydroxybenzonitrile. 5-methyl-2-hydroxybenzonitrile was synthesized in the same manner as **Example AAG1** wherein 5-bromo-2-hydroxy-3-methylbenzaldehyde was replaced with 2-hydroxy-5-methylbenzaldehyde. 1H NMR (400 MHz, d6-DMSO): 13.28 (s, 1H), 7.85 (s, 1H), 7.76 (d, 1H), 7.70 (dd, 1H), 7.65 (m, 1H), 7.60 (m, 1H), 7.53 (m, 2H), 2.48 (s, 3H). MS (EI) for C₁₇H₁₁ClN₂O₂: 312 (MH+).

### *(Compound 123)

### 8-bromo-2-(3-methyl-1H-indazol-5-yl)[1]benzofuro[3,2-d]pyrimidin-4(3H)-one

8-bromo-2-(3-methyl-1H-indazol-5-yl)[1]benzofuro[3,2-d]pyrimidin-4(3H)-one was synthesized in a manner similar to **Example 8,** wherein 2-chlorobenzaldehyde was substituted with 3-methyl-1H-indazole-5-carbaldehyde (see **Note 1** for synthesis). ¹H-NMR (400MHz, d₆-DMSO): 12.93 (s, 1H), 8.69 (s br, 1H), 8.26 (m, 2H), 7.83 (s, 2H), 7.58 (d, 1H), 2.57 (s, 3H). MS (EI) for C₁₈H₁₁BrN₄O₂: 396 (MH⁺).

**Note 1:** 3-methyl-1H-indazole-5-carbaldehyde and 1H-indazole-5-carbaldehyde were synthesized according to a patent published by Piatnitski, Evgueni; Kiselyov, Alexander. Heteroaryl aminophenyl ketone derivatives and their preparation and use as kinase inhibitors, e.g., in the treatment of neoplastic diseases. PCT Int. Appl. (2005), 58 pp. CODEN: PIXXD2 WO 2005000813 Al 20050106 CAN 142:114055 AN 2005:14374 CAPLUS

### *(Compound 170)

### 2-(3-methyl-1H-indazol-5-yl)-9-(methyloxy)[1]benzofuro[3,2-d]pyrimidin-4(3H)-one

2-(3-methyl-1H-indazol-5-yl)-9-(methyloxy)[1]benzofuro[3,2-d]pyrimidin-4(3H)-one was synthesized in a manner similar to **Example 8,** wherein 3-methyl-1H-indazole-5-carbaldehyde (see **Note 1** for synthesis) was substituted with 2-chlorobenzaldehyde and **Example 1** wherein 6-methoxy-2-hydroxybenzonitrile was substituted with 5-bromo-2-hydroxybenzonitrile **1**. ¹H-NMR (400MHz, d₆-DMSO): 12.66 (s, 1H), 8.65 (s br, 1H), 8.43 (d, 1H), 7.45 (m, 2H), 7.25 (d, 1H), 6.89 (d, 1H), 4.04 (s, 3H), 2.56 (s, 3H). MS (EI) for C₁₉H₁₄N₄O₃: 347 (MH⁺).

### *(Compound 171)

### 2-(3-amino-1H-indazol-5-yl)-9-(methyloxy)[1]benzofuro[3,2-d]pyrimidin-4(3H)-one

2-(3-amino-1H-indazol-5-yl)-9-(methyloxy)[1]benzofuro[3,2-d]pyrimidin-4(3H)-one was synthesized in a manner similar to **Example 8,** wherein 2-chlorobenzaldehyde was substituted with 2-fluoro-5-formylbenzonitrile, and **Example 1**, wherein 5-bromo-2-hydroxybenzonitrile (1) was substituted with 6-methoxy-2-hydroxybenzonitrile. Subsequently, resulting crude 2-fluoro-5-(9-methoxy-4-oxo-3,4-dihydrobenzofuro[3,2-d]pyrimidin-2-yl)benzonitrile was dissolved in DMSO (2mL) and hydrazine hydrate (1 mL). The reaction mixture was heated to 110 °C for 16h in a sealed vessel. Upon cooling, the mixture was concentrated *in vacuo* and dissolved in methanol. Formation of product was confirmed by LC/MS and the product was purified by preparatory HPLC (reverse-phase, acetonitrile/water with 0.1% formic acid) to yeild the title compound. ¹H-NMR (400MHz, d₆-DMSO): 8.64 (s br, 1H), 8.10 (d, 1H), 7.62 (t, 1H), 7.52 (d, 1H), 7.40 (d, 1H), 7.05 (d, 1H), 4.04 (s, 3H). MS (EI) for C₁₈H₁₃N₅O₃: 348 (MH⁺).

### *(Compound 220)

### 2-(3-amino-1H-indazol-5-yl)-8-bromo[1]benzofuro[3,2-d]pyrimidin-4(3H)-one

2-(3-amino-1H-indazol-5-yl)-8-bromo[1]benzofuro[3,2-d]pyrimidin-4(3H)-one was synthesized in a manner similar to **Example 8** wherein 2-chlorobenzaldehyde was substituted with 2-fluoro-5-formylbenzonitrile. Subsequently, resulting crude 2-fluoro-5-(8-bromo-4-oxo-3,4-dihydrobenzofuro[3,2-d]pyrimidin-2-yl)benzonitrile was dissolved in DMSO (2mL) and hydrazine hydrate (1 mL). The reaction mixture was heated to 110 °C for 16h in a sealed vessel. Upon cooling the mixture was concentrated *in vacuo* and dissolved in methanol. Formation of product was confirmed by LC/MS and the product was purified by preparatory HPLC (reverse-phase, acetonitrile/water with 0.1% formic acid) to yeild the title compound. ¹H-NMR (400MHz, d₆-DMSO): 11.7 (br s, 1H), 8.63 (s br, 1H), 8.28 (s, 1H), 8.20 (m, 1H), 8.10 (d, 1H), 7.79 (m, 2H), 7.30 (d, 1H). MS (EI) for C₁₇H₁₀BrN₅O₂: 396 (MH⁺).

### EXAMPLE 9

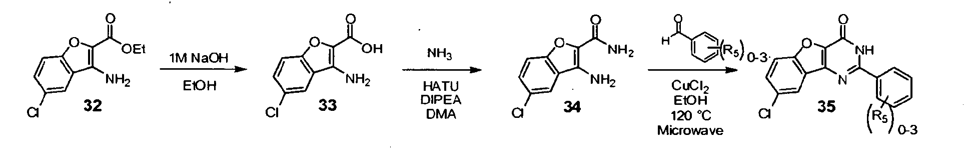

wherein R₅ is described within the compounds within this example. 3-Amino-5-chlorobenzofuran-2-carboxylic acid **33**

Ethyl 3-amino-5-chlorobenzofuran-2-carboxylate **32** (1.00 g, 4.17 mmol) was suspended in a solution of 15 mL ethanol and 15 mL of 1.0 M sodium hydroxide. The suspension was heated at 65°C overnight. After cooling, the clear solution was acidified to pH 3 with concentrated hydrochloric acid. The precipitate was filtered, washed with 2 mL water and dried under vacuum to give 753 mg of white solid. MS (EI) for C₉H₆ClNO₃: 212 (MH⁺).

### 3-Amino-5-chlorobenzofuran-2-carboxamide 34

3-Amino-5-chlorobenzofuran-2-carboxylic acid **33** (250 mg, 1.18 mmol) and diisopropylethylamine (515 µL, 2.95 mmol) were dissolved in 10 mL of dimethylacetamide. HATU (560 mg,1.25 mmol) was added and the reaction was stirred for 30 min at room temperature. Ammonia gas was bubbled through the reaction mixture for 1 min. After stirring for another 30 min, the reaction mixture was diluted with 20 mL of ethyl acetate. The reaction mixture was washed three times with 10 mL portions of water and once with 10 mL of saturated aqueous sodium chloride. The organic fraction was separated, dried with magnesium sulfate and concentrated under vacuum to give 220 mg of 3-amino-5-chlorobenzofuran-2-carboxamide. MS (EI) for C₉H₇ClN₂O₂: 211 (MH⁺).

### *(Compound 31)

### 8-Chloro-2-(2-chlorophenyl)[1]benzofuror3,2-d]pyrimidin-4(3H)-one

A suspension of 3-amino-5-chlorobenzofuran-2-carboxamide **34** (150 mg, 0.71 mmol), 2-chlorobenzaldehyde (90 µL, 0.80 mmol) and copper(II) chloride (95 mg, 0.71) in 2.5 mL anhydrous ethanol was heated for 20 min at 120°C in a microwave reactor. The solvent was concentrated under vacuum. The residue was taken up in 3 mL of dimethylformamide, filtered and purified by preparative HPLC to give 20 mg of 8-chloro-2-(2-chlorophenyl)[1]benzofuro[3,2-d]pyrimidin-4(3H)-one. ¹H NMR (400 MHz, d₆-DMSO): 13.34 (m, 1H), 8.06 (d, 1H), 7.88(d, 1H), 7.67 (dd, 1H), 7.63 (dd, 1H), 7.58 (dd, 1H), 7.53 (m, 1H), 7.46 (m, 1H); MS (EI) for C₁₆H₈Cl₂N₂O₂: 331 (MH⁺).

### *(Compound 85)

### 8-bromo[-2-(1H-imidazol-2-yl)[1]benzofuro[3,2-d]pyrimidin-4(3H)-one

8-bromo-2-(1H-imidazol-2-yl)[1]benzofuro[3,2-d]pyrimidin-4(3H)-one was synthesized in a manner similar to **Example 9, Compound 31,** wherein 2-chlorobenzaldehyde was substituted with 1H-imidazole-2-carbaldehyde, and 3-amino-5-bromobenzofuran-2-carboxamide was substituted for 3-amino-5-chlorobenzofuran-2-carboxamide. ¹H NMR (400 MHz, d₆-DMSO): 8.18 (s, 1H), 7.81 (m, 2H), 7.28 (m, 2H); MS (EI) for C₁₃H₇BrN₄O₂: 332 (MH⁺).

### *(Compound 86)

### 8-bromo-2-(1,3-thiazol-2-yl)[1]benzofuro[3,2-d]pyrimidin-4(3H)-one

8-bromo-2-(1,3-thiazol-2-yl)[1]benzofuro[3,2-d]pyrimidin-4(3H)-one was synthesized in a manner similar to **Example 9,** wherein thiazole-2-carbaldehyde replaced 2-chlorobenzaldehyde and 3-amino-5-bromobenzofuran-2-carboxamide replaced 3-amino-5-chlorobenzofuran-2-carboxamide. ¹H NMR (400 MHz, d₆-DMSO): 8.17 (s, 1H), 7.89 (s, 1H), 7.67 (m, 3H); MS (EI) for C₁₃H₆BrN₃O₂S: 349 (MH⁺).

### *(Compound 77)

### 8-bromo-2-(2-ethylphenyl)[1]benzofuro[3,2-d]pyrimidin-4(3H)-one

8-bromo-2-(2-ethylphenyl)[1]benzofuro[3,2-d]pyrimidin-4(3H)-one was synthesized in a manner similar to **Example 9**, wherein 3-amino-5-bromobenzofuran-2-carboxamide replaced 3-amino-5-chlorobenzofuran-2-carboxamide, and 2-ethylbenzaldehyde replaced 2-chlorobenzaldehyde. 1H NMR (400 MHz, d6-DMSO): 13.20 (s, br, 1H), 8.20 (s, 1H), 7.85 (m, 2H), 7.48 (m, 2H), 7.40 (d, 1H), 7.34 (m, 1H), 2.74 (m, 2H), 1.12 (t, 3H). MS (EI) for C18 H13 BrN2 O2: 370 (MH+).

### *(Compound 78)

### 8-bromo-2-[2-bromo-5-(methyloxy)phenyl][1]benzofuro[3,2-d]pyrimidin-4(3H)one

8-bromo-2-[2-bromo-5-(methyloxy)phenyl][1]benzofuro[3,2-d]pyrimidin-4(3H)-one was synthesized in a manner similar to **Example 9**, wherein 3-amino-5-bromobenzofuran-2-carboxamide replaced 3-amino-5-chlorobenzofuran-2-carboxamide, and 2-bromo-5-methoxybenzaldehyde replaced 2-chlorobenzaldehyde. 1H NMR (400 MHz, d6-DMSO): 13.37 (s, br, 1H), 8.23 (s, 1H), 7.85 (m, 2H), 7.57 (m, 1H), 7.26 (s, 1H), 7.10 (m, 1H), 3.80 (s, 3H). MS (EI) for C17 H10 Br2 N2 O3: 451 (MH+).

### *(Compound 79)

### 8-bromo-2-[2-chloro-4-(methyloxy)phenyl][1]benzofuro[3,2-d]pyrimidin-4(3H)-one

8-bromo-2-[2-chloro-4-(methyloxy)phenyl][1]benzofuro[3,2-d]pyrimidin-4(3H)-one was synthesized in a manner similar to **Example 9**, wherein 3-amino-5-bromobenzofuran-2-carboxamide replaced 3-amino-5-chlorobenzofuran-2-carboxamide, and 2-chloro-4-methoxybenzaldehyde replaced 2-chlorobenzaldehyde. 1H NMR (400 MHz, d6-DMSO): 13.30 (s, 1H), 8.25 (s, 1H), 7.87 (d, 2H), 7.63 (d, 1H), 7.22 (s, 1H), 7.09 (dd, 1H), 3.86 (s, 3H). MS (EI) for C17 H10 Br Cl N2 O3: 407 (MH+).

### EXAMPLE 10

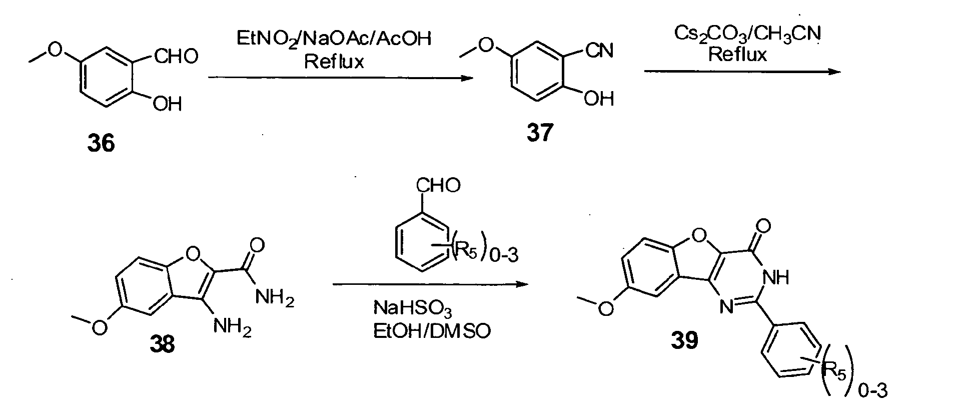

wherein R₅ is described within the compounds within this example. 2-Hydroxy-5-methoxybenzonitrile **37**

To a suspension of sodium acetate (10.8 g, 131.7 mmol) in 10 mL of acetic acid was added 2-hydroxy-5-methoxybenzaldehyde **36** (10.0 g, 65.72 mmol) and nitroethane (4.7 mL, 65.8 mmol). The mixture was refluxed overnight, cooled to room temperature, then diluted with ethyl acetate and water (200 mL each). The organic layer was separated. The aqueous layer was extracted with ethyl acetate (2X50 mL). The combined organic was washed with saturated sodium bicarbonate solution (3X100 mL), brine (100 mL), dried over anhydrous sodium sulfate, and concentrated to a smaller volume. 1.2 g of crystalline powder was collected by filtration after standing overnight, washed with 20 mL of tert-butyl methyl ether. The filtrate was purified with a silica gel column (5% to 25% ethyl acetate in hexanes), 6.3 g of yellowish powder was obtained as the desired product. ¹H NMR (400 MHz, d₆-DMSO): 10.20 (s, 1H), 6.93 - 6.96 (m, 3H), 3.75 (s, 3H); MS (EI) for C₈H₇NO₂: 150 (MH⁺).

### 3-Amino-5-methoxybenzofura -2-carboxamide 38

To a suspension of 2-hydroxy-5-methoxybenzonitrile **37** (6.3 g, 42.3 mmol), cesium carbonate (20.6 g, 63.45 mmol) in 70 mL of acetonitrile was added 2-chloroacetamide (4.2 g, 44.42 mmol) and potassium iodide (2 g, 12.0 mmol). The suspension was heated to 80 °C overnight, then cooled to room temperature. The suspension was filtered through a Celite pat and washed with 150 mL of ethyl acetate. The filtrate was concentrated, the solid residue was suspended in 20 mL of methanol, and filtered again, washed with 10 mL of methanol. 3.9 g of yellowish crystalline powder was collected as the desired product. ¹H NMR (400 MHz, d₆-DMSO): 7.39 (s, 1H), 7.30 (d, 1H), 7.10 (br, 2H), 6.98 (d, 2H), 5.90 (s, 2H), 3.78 (s, 3H); MS (EI) for C₁₀H₁₀N₂O₃: 207 (MH⁺).

### *(Compound 32)

### 2-(2-Chlorophenyl)-8-(methyloxy)[1]benzofuro[3,2-d]pyrimidin-4(3H)-one

To a suspension of 3-amino-5-methoxybenzofuran-2-carboxamide **38** (1.0 g, 4.85 mmol) and 2-chlorobenzaldehyde (1.7 g, 9.70 mmol) in 20 mL anhydrous ethanol was added 2 drops of concentrated HCl. The mixture was stirred overnight at room temperature, and then 20 mL DMSO was added. The reaction mixture was heated to 150 °C while the flask was opened to the air. Sodium bisulfite (2.5 g, 24.25 mmol) was added in several portions. The mixture was cooled to room temperature after 5 h, diluted with 100 mL of water, stirred for 0.5 h. The aqueous suspension was filtered, washed with 20 mL of water, dried in the air. 1.3 g of yellow-brown powder was collected as the desired product. ¹H NMR (400 MHz, d₆-DMSO): 13.28 (s, 1H), 7.80 (d, 1H), 7.72 (d, 1H), 7.58 - 7.64 (m, 2H), 7.52 (m, 2H), 7.28 (d, 1H), 3.82 (s, 3H); MS (EI) for C₁₇H₁₁ClN₂O₃: 327 (MH⁺).

### *(Compound 71)

### 2-(2-chlorophenyl)-7-hydroxy[1]benzofuro[3,2-d]pyrimidin-4(3H)-one

2-(2-chlorophenyl)-7-hydroxy[1]benzofuro[3,2-d]pyrimidin-4(3H)-one was synthesized in the same manner as **Example 10** wherein 2-hydroxy-5-methoxybenzaldehyde was replaced with 2-hydroxy-4-methoxybenzaldehyde. The resulting 2-(2-chlorophenyl)-7-methoxy[1]benzofuro[3,2-d]pyrimidin-4(3H)-one (160 mg, 0.49 mmol) was dissolved in 20 mL of Dichloroethane and BBr₃Me₂S (0.3 g, 1 mmol) was added. The reaction mixture was heated to reflux for 8h, cooled down to room temperature and 5 mL of water was added. The organic phase was washed with brine, dried over Na₂SO₄ and concentrated *in vacuo* to afford 142 mg (93%) of the title compound. 1H NMR (400 MHz, d6-DMSO): 13.22 (s, br, 1H), 10.41 (s, br, 1H), 7.83 (d, 1H), 7.68 (m, 1H), 7.63 (m, 1H), 7.59 (m, 1H), 7.51 (m, 1H), 7.13 (d, 1H), 6.98 (dd, 1H). MS (EI) for C16H9ClN203: 314 (MH+).

### *(Compound 73)

### 2-(2-chlorophenyl)-8-hydroxy[1]benzofuro[3,2-d]pyrimidin-4(3H)-one

2-(2-chlorophenyl)-8-hydroxy[1]benzofuro[3,2-d]pyrimidin-4(3H)-one was synthesized in the same manner as in **Example 10**. The resulting 2-(2-Chlorophenyl)-8-(methyloxy)[l]benzofuro[3,2-d]pyrimidin-4(3H)-one **(Compound 32)** (160 mg, 0.49 mmol) was dissolved in 20 mL of Dichloroethane and BBr₃Me₂S (0.3 g, 1 mmol) was added. The reaction mixture was heated to reflux for 8h, cooled down to room temperature and 5 mL of water was added. The organic phase was washed with brine, dried over Na₂SO₄ and concentrated *in vacuo* to afford 135 mg (90%) of the title compound.
1H NMR (400 MHz, d6-DMSO): 13.22 (s, 1H), 9.75 (m, 1H), 7.59 (m, 4H), 7.24 (m, 1H), 7.08 (m, 1H), 5.76 (m, 1H). MS (EI) for C16H9ClN2O3: 314 (MH+).

### *(Compound 83)

### 2-(2-chlorophenyl)-9-(methyloxy)[1]benzofuro[3,2-d]pyrimidin-4(3H)-one

2-(2-chlorophenyl)-9-(methyloxy)[1]benzofuro[3,2-d]pyrimidin-4(3H)-one was synthesized in the same manner as **Example 10** wherein 2-hydroxy-5-methoxybenzaldehyde was replaced with 2-hydroxy-6-methoxylbenzaldehyde. 1H NMR (400 MHz, d6-DMSO): 13.28 (s, 1H), 7.68 (dd, 1H), 7.62 (m, 3H), 7.52 (m, 2H), 7.41 (d, 1H), 7.01 (d, 1H), 3.95 (s, 3H). MS (EI) for C17H11ClN2O3: 328 (MH+).

### *(Compound 124)

### 2-(2-chloro-4-nitrophenyl)-8-(methyloxy)[1]benzofuro[3,2-d]pyrimidin-4(3H)-one

2-(2-Chloro-4-nitrophenyl)-8-(methyloxy)[1]benzofuro[3,2-d]pyrimidin-4(3H)-one was synthesized in the same manner as **Example 10** wherein 2-chlorobenzaldehyde was replaced with 2-chloro-4-nitrobenzaldehyde. 1H NMR (400 MHz, d6-DMSO): 13.52 (s, 1H), 8.50 (s, 1H), 8.37 (d, 1H), 8.03 (d, 1H), 7.81 (d, 1H), 7.53 (s, 1H), 7.29 (d, 1H), 3.86 (s, 3H). MS (EI) for C17H10ClN3O5: 373 (MH+).

### *(Compound 129)

### 2-(4-amino-2-chlorophenyD-8-(methyloxy)[1]benzofuro[3,2-d]pyrimidin-4(3H)-one

2-(4-amino-2-chlorophenyl)-8-(methyloxy)[1]benzofuro[3,2-d]pyrimidin-4(3H)-one was synthesized in the same manner as in **Example 10** and wherein 2-chlorobenzaldehyde was replaced with 2-chloro-4-nitrobenzaldehyde. 1H NMR (400 MHz, d6-DMSO): 13.26 (s, br, 1H), 7.77 (d, 1H), 7.48 (m, 1H), 7.37 (m, 1H), 7.26 (m, 1H), 6.77 (s, 1H), 6.66 (m, 1H), 3.86 (s, 3H). MS (EI) for C17H12ClN3O3: 343 (MH+).

### *(Compound 137)

### 1,1-dimethylethyl-4-[({3-chloro-4-[8-(methyloxy)-4-oxo-3,4-dihydro[1]benzofuro[3,2-d]pyrimidin-2-yl]phenyl}amino)methyl]piperidine-1-carboxylate

1,1-dimethylethyl 4-[({3-chloro-4-[8-(methyloxy)-4-oxo-3,4-dihydro[1]benzofuro[3,2-d]pyrimidin-2-yl]phenyl} amino)methyl]piperidine-1-carboxylate was synthesized in the same manner as in **Example 10** wherein 2-chlorobenzaldehyde was replaced with 2-chloro-4-nitrobenzaldehyde. 1H NMR (400 MHz, d6-DMSO): 7.35 (d, 1H), 7.57 (m, 2H), 7.22 (m, 3H), 6.64 (m, 2H), 4.12 (m, 1H), 3.91 (s, 3H), 3.10 (d, 2H), 2.71 (m, 2H), 1.78 (m, 4H), 1.46 (s, 9H), 1.21 (m, 2H). MS (EI) for C28H31ClN4O5: 540 (MH+).

### *(Compound 138)

### 2-{2-chloro-4-[(piperidin-4-ylmethyl)amino]phenyl}-8-(methyloxy)[1]benzofuro[3,2-d]pyrimidin-4(3H)-one

2-{2-chloro-4-[(piperidin-4-ylmethyl)amino]phenyl}-8-(methyloxy)[1]benzofuro[3,2-d]pyrimidin-4(3H)-one was synthesized in the same manner as in **Example 10** wherein 2-chlorobenzaldehyde was replaced with 2-chloro-4-nitrobenzaldehyde. 1H NMR (400 MHz, d6-DMSO): 8.93 (br s, 1H), 8.66 (br s, 1H), 7.74 (d, 1H), 7.43 (d, 1H), 7.36 (d, 1H), 7.23 (dd, 1H), 6.70 (d, 1H), 6.63 (dd, 1H), 4.10 (m, 2H), 3.71 (s, 3H), 3.23 (m, 2H), 2.99 (m, 2H), 2.83 (m, 2H), 1.82 (m, 3H), 1.37 (m, 2H). MS (EI) for C23H23ClN4O3: 440 (MH+).

### *(Compound 144)

### 2-(2-chloro-4-fluorophenyl)-8-(methyloxy)[1]benzofuro[3,2-d]pyrimidin-4(3H)-one

2-(2-chloro-4-fluorophenyl)-8-(methyloxy)[1]benzofuro[3,2-d]pyrimidin-4(3H)-one was synthesized in the same manner as **Example 10** wherein 2-chlorobenzaldehyde was replaced with 2-chloro-4-fluorobenzaldehyde.
1H NMR (400 MHz, d6-DMSO): 13.32 (s, 1H), 7.79 (m, 2H), 7.68 (dd, 1H), 7.52 (d, 1H), 7.42 (m, 1H), 7.28 (dd, 1H), 3.86 (s, 3H). MS (EI) for C17H10ClFN2O3: 346 (MH+).

### *(Compound 145)

### 2-(2-chloro-5-nitrophenyl)-8-(methyloxy)[1]benzofuro[3,2-d]pyrimidin-4(3H)-one

2-(2-chloro-5-nitrophenyl)-8-(methyloxy)[1]benzofuro[3,2-d]pyrimidin-4(3H)-one was synthesized in the same manner as **Example 10** wherein 2-chlorobenzaldehyde was replaced with 2-chloro-5-nitrobenzaldehyde. 1 H NMR (400 MHz, d6-DMSO): 13.45 (s, 1H), 8.63 (d, 1H), 8.43 (dd, 1H), 7.97 (d, 1H), 7.81 (d, 1H), 7.53 (d, 1H), 7.29 (dd, 1H), 3.86 (s, 3H). MS (EI) for C17H10ClN3O5: 373 (MH+).

### *(Compound 152)

### 2-(3-chloropyridin-4-yl)-8-(methyloxy)[1]benzofuro[3,2-d]pyrimidin-4(3H)-one

2-(3-chloropyridin-4-yl)-8-(methyloxy)[1]benzofuro[3,2-d]pyrimidin-4(3H)-one was synthesized in the same manner as **Example 10** wherein 2-chlorobenzaldehyde was replaced with 3-chloroisonicotinaldehyde.
1H NMR (400 MHz, d6-DMSO): 10.84 (s, 1H), 8.75 (d, 2H), 8.09 (s, 1H), 7.78 (m, 2H), 7.52 (m, 1H), 7.37 (s, 1H), 7.12 (m, 1H), 3.78 (s, 3H). MS (EI) for C16H10ClN3O3: 329 (MH+).

### EXAMPLE 11

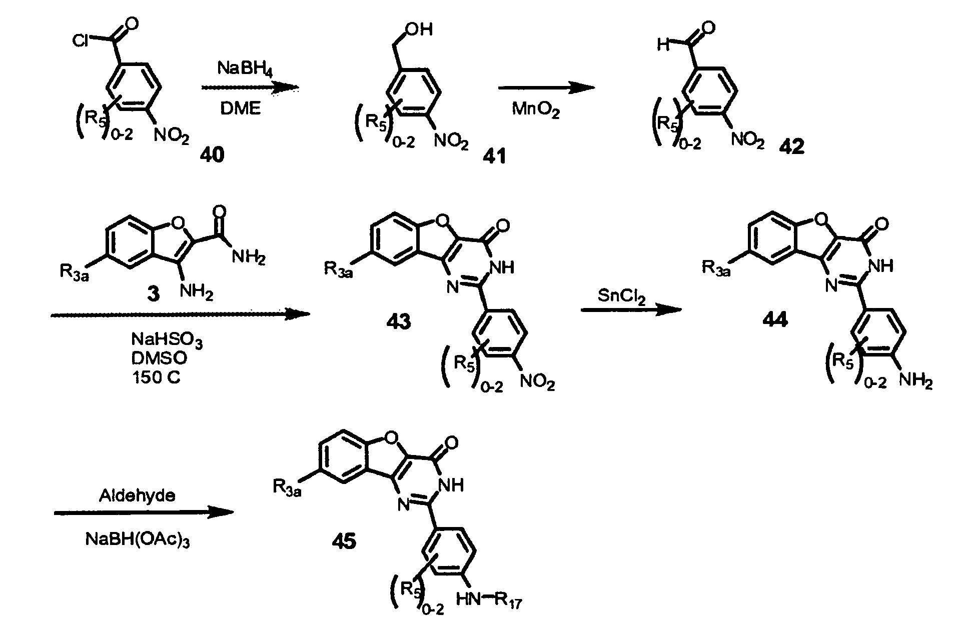

wherein R₃ₐ is as defined in the disclosure, R₁₅ is described within the compounds within this example, and R₁₇ is hydrogen, heterocycloalkylalkyl or dialkylaminoalkyl.

### 2-Chloro-4-nitrobenzaldehyde

To a suspension of NaBH₄ (2.1g, 56.6mmol) in 1,2-dimethoxyethane (30 mL) was added 2-chloro-4-nitrobenzoyl chloride (5g, 22.8 mmol) at 0C, and the reaction mixture was stirred at RT for 2h. The mixture was concentrated in vacuo. Water was added to the residue and the mixture was extracted with EtOAc. The organic layer was washed with 1N HCl, 1N NaOH, water, brine, and dried over MgSO4, and concentrated on rotary evaporator. The residue was dissolved in 50 mL of Acetone and activated Mn02 (10 g) was added. The reaction mixture was stirred for 18h at RT. The slurry was filtered and concentrated down. Purification by column chromatography resulted in 1.2 g (29%) of 2-chloro-4-nitrobenzaldehyde.

### *(Compound 33)

### 8-Bromo-2-(2-chloro-4-nitrophenyl)[1]benzofuro[3,2-d]pyrimidin-4(3H)-one.

A mixture of 2-chloro-4-nitrobenzaldehyde (1.2 g, 6.4mmol), compound 3 (0.7g, 2.7mmol), NaHSO₃ (2g, 19 mmol) in 10 mL of DMSO was heated to 150 °C for 5h. Upon completion, the reaction mixture was cooled down to RT, and 10 mL of water was added. The precipitate was filtered off, resulting in 1g (88%) of 8-bromo-2-(2-chloro-4-nitrophenyl)[1]benzofuro[3,2-d]pyrimidin-4(3H)-one. The product was submitted to the next step without further purification. ¹H NMR (400 MHz, d₆-DMSO): 13.62 (s, 1H), 8.52 (d, 1H), 8.37 (dd, 1H), 8.27 (d, 1H), 8.02 (d, 1H), 7.89 (m, 2H); MS (EI) for C₁₆H₇BrClN₃O₄: 422 (MH⁺).

### *(Compound 34)

### 2-(4-Amino-2-chlorophenyl)-8-bromo[1]benzofuro[3,2-d]pyrimidin-4(3H)-one.

A mixture of 8-bromo-2-(2-chloro-4-nitrophenyl)[1]benzofuro[3,2-d]pyrimidin-4(3H)-one (0.4 g, 0.95 mmol), Tin(II) chloride dihydrate (3g, 13.3 mmol), Methanol (5mL), EtOAc (20mL), and water (1mL) was heated to 80 °C for 1h. The resulting slurry was concentrated down on the rotary evaporator, and extracted with EtOAc/ water mixture. The organic layer was dried over magnesium sulfate, and concentrated down, resulting in 2-(4-amino-2-chlorophenyl)-8-bromo[1]benzofuro[3,2-d]pyrimidin-4(3H)-one that was submitted to the next step without further purification. The aliquot of the product was purified by preparative HPLC. ¹H NMR (400 MHz, d₆-DMSO): 8.19 (m, 1H), 7.83 (m, 2H), 7.33 (d, 1H), 6.69 (m, 1H), 6.58 (m, 1H), 5.86 (m, 2H); MS (EI) for C₁₆H₉BrClN₃O₂: 391 (MH⁺).

### *(Compound 35)

### 8-bromo-2-{2-chloro-4-[(piperidin-4-ylmethyl)amino]phenyl}[1]benzofuro[3,2-d]pyrimidin-4(3H)-one

To the solution of 2-(4-amino-2-chlorophenyl)-8-bromo[1]benzofuro[3,2-d]pyrimidin-4(3H)-one (o.3g, 0.76 mmol) in 10mL of 1:1 Dichloromethane/Dimethylformamide was added *tert*-butyl 4-formylpiperidine-1-carboxylate (0.5 g, 2.3mmol), and sodium triacetoxyborohydride (1g, 4.7mmol) at room temperature. The reaction mixture was heated to 60 °C for 1 h, then it was concentrated under reduced pressure, re-dissolved in 10 mL of MeOH. To the resulting slurry was added 2 mL of 4 N HCl in Dioxane. The reaction mixture was heated to 50 °C for 1h, concentrated under reduced pressure. The residue was purified by preparative HPLC, resulting in 11 mg of 8-bromo-2-{2-chloro-4-[(piperidin-4-ylmethyl)amino]phenyl}[1]benzofuro[3,2-d]pyrimidin-4(3H)-one.
¹H NMR (400 MHz, d₆-DMSO): 8.10 (m, 1H), 7.74 (m, 2H), 7.34 (d, 1H), 6.64 (m, 1 H), 6.58 (dd, 1H), 6.27 (m, 1H), 3.07 (m, 2H), 2.94 (m, 2H), 2.55 (m, 2H), 1.75 (m, 2H), 1.15 (m, 2H); MS (EI) for C₁₆C₂₂H₂₀BrClN₄O₂ 488(MH⁺).

### *(Compound 139)

### 8-bromo-2- {2-chloro-4-[(1H-imidazol-4-ylmethyl)amino]phenyl}[1]benzofuro[3,2-d]pyrimidin-4(3H)-one

8-bromo-2-{2-chloro-4-[(1H-imidazol-4-ylmethyl)amino]phenyl}-[1]benzofuro[3,2-d]pyrimidin-4(3H)-one was synthesized in a manner similar to **Example 11** wherein 1H-imidazole-4-carbaldehyde replaced tert-butyl 4-formylpiperidine-1-carboxylate. ¹H NMR (400 MHz, d₆-DMSO): 8.17 (d, 2H), 7.83 (t, 2H), 7.60 (s, 1H), 7.37 (d, 1H), 6.99 (s, 1H), 6.75 (m, 3H), 4.22 (s, 2H); MS (EI) for C₂₀H₁₃BrClN₅O₂: 472
(MH⁺).

### *(Compound 154)

### 8-bromo-2-{2-chloro-4-[(pyrrolidin-3-ymethyl)amino]phenyl}[1]benzofuro[3,2-d]pyrimidin-4(3H)-one

8-bromo-2-{2-chloro-4-[(pyrrolidin-3-ylmethyl)amino]phenyl}[1]benzofuro[3,2-d]pyrimidin-4(3H)-one was synthesized in a manner similar to **Example 11** wherein tert-butyl 3-formylpyrrolidine-1-carboxylate replaced tert-butyl 4-formylpiperidine-1-carboxylate. ¹H NMR (400 MHz, d₆-DMSO): 8.39 (s, 1H), 8.16 (s, 1H), 7.9 (m, 2H), 7.37 (d, 1H), 6.7 (s, 1H), 6.63 (d, 1H), 6.48 (m, 1H), 3.28 (m, 3H), 3.11 (m, 3H), 2.88 (m, 2H), 2.04 (m, 1H), 1.62 (m, 1H); MS (EI) for C₂₁H₁₈BrClN₄O₂: 475 (MH⁺).

### *(Compound 155)

### 8-bromo-2-{2-chloro-4-[(2-piperidin-3-ylethyl)amino]phenyl}[1]benzofuro[3,2-d]pyrimidin-4(3H)-one

8-bromo-2-{2-chloro-4-[(2-piperidin-3-ylethyl)amino]phenyl}[1]benzofuro[3,2-d]pyrimidin-4(3H)-one was synthesized in a manner similar to **Example 11**, wherein tert-butyl 3-(2-oxoethyl)piperidine-1-carboxylate replaced tert-butyl 4-formylpiperidine-1-carboxylate. ¹H NMR (400 MHz, d₆-DMSO): 8.39 (s, 1H), 8.15 (s, 1H), 7.80 (m, 2H), 7.37 (d, 1H), 6.67 (s, 1H), 6.60 (d, 1H), 6.32 (s, 1H), 3.15 (m, 6H), 2.67 (t, 1H), 1.74 (m, 3H), 1.38 (m, 3H); MS (EI) for C₂₃H₂₂BrClN₄O₂: 503 (MH⁺).

### *(Compound 179)

### 8-bromo-2-(2-chloro-4-{[3-(dimethylamino)-2,2-dimethylpropyl]amino}pheny)[1]benzofuro[3,2-d]pyrimidin-4(3H)-one

8-bromo-2-(2-chloro-4- {[3-(dimethylamino)-2,2-dimethylpropyl]amino}phenyl)[1]benzofuro[3,2-d]pyrimidin-4(3H)-one was synthesized in a manner similar to **Example 11**, wherein 3-(dimethylamino)-2,2-dimethylpropanal replaced tert-butyl 4-formylpiperidine-1-carboxylate. ¹H NMR (400 MHz, d₆-DMSO): 8.33 (s, 1H), 8.19 (s, 1H), 7.83 (m, 2H) 7.37 (d, 1H), 6.71 (m, 2H), 6.27 (s, 1H), 2.94 (s, 2H), 2.24 (s, 6H), 2.19 (s, 2H), 0.93 (s, 6H); MS (EI) C₂₃H₂₄BrClN₄O₂: 505 (MH⁺).

### *(Compound 136)

### 8-bromo-2-(2-chloro-4-{[4-(dimethylamino)butyl]amino}phenyl)[1]benzofuro[3,2-d]pyrimidin-4(3H)-one

8-bromo-2-(2-chloro-4-{[4-(dimethylamino)butyl]amino}phenyl)[1]benzofuro[3,2-d]pyrimidin-4(3H)-one was synthesized in a manner similar to **Example 11**, wherein 4-dimethylamino-butyraldehyde replaced tert-butyl 4-formylpiperidine-1-carboxylate. 1H NMR (400 MHz, d6-DMSO): 8.23 (s, br, 1H), 8.13 (s, 1H), 7.77 (m, 2H), 7.30 (d, 1H), 6.61 (s, 1H), 6.55 (dd, 1H), 3.02 (m, 2H), 2.22 (m, 2H), 2.10 (s, 6H), 1.47 (m, 4H). MS (EI) for C22 H22 Br Cl N4 O2: 491 (MH+).

### *(Compound 159)

### 8-bromo-2-{2-chloro-4-[(piperidin-3-ylmethyl)amino]phenyl}[1]benzofuro[3,2-d]pyrimidin-4(3H)-one

8-bromo-2-{2-chloro-4-[(piperidin-3-ylmethyl)amino]phenyl}[1]benzofuro[3,2-d]pyrimidin-4(3H)-one was synthesized in a manner similar to **Example 11**, wherein tert-butyl 3-formylpiperidine-1-carboxylate replaced tert-butyl 4-formylpiperidine-1-carboxylate. 1H NMR (400 MHz, d6-DMSO): 8.29 (s, 1H), 8.10 (s, 1H), 7.74 (m, 2H), 7.31 (d, 1H), 6.63 (s, 1H), 6.56 (d, 1H), 6.40 (m, 1H), 3.13 (m, 2H), 3.03 (m, 2H), 2.94 (m, 2H), 2.58 (m, 1H), 1.77 (m, 1H), 1.66 (m, 1H), 1.45 (m, 1H), 1.40 (m, 1H). MS (EI) for C22 H20 Br Cl N4 O2: 489 (MH+).

### *(Compound 197)

### 8-bromo-2-{2-chloro-4-[(2-piperidin-4-ylethyl)amino]phenyl}[1]benzofuro[3,2-d]pyrimidin-4(3H)-one

8-bromo-2-{2-chloro-4-[(2-piperidin-4-ylethyl)amino]phenyl}[1]benzofuro[3,2-d]pyrimidin-4(3H)-one was synthesized in a manner similar to **Example 11**, wherein tert-butyl 4-(2-oxoethyl)piperidine-1-carboxylate replaced tert-butyl 4-formylpiperidine-1-carboxylate. 1H NMR (400 MHz, d6-DMSO): 9.10 (s, br, 1H), 8.89 (s, br, 1H), 8.22 (s, 1H), 7.86 (m, 2H), 7.41 (d, 1H), 6.76 (s, 1H), 6.69 (d, 1H), 3.25 (d, 2H), 3.13 (m, 2H), 2.82 (m, 2H), 2.02 (d, 2H), 1.69 (m, 1H), 1.53 (m, 2H), 1.39 (m, 2H). MS (EI) for C23 H22 Br Cl N4 O2: 503 (MH+).

### EXAMPLE 11B: Example 11+ coupling

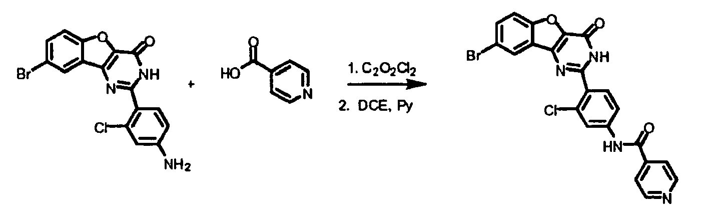

### *(Compound 140)

### N-[4-(8-bromo-4-oxo-3,4-dihydro[1]benzofuro[3,2-d]pyrimidin-2-yl)-3-chlorophenyl]piperidine-3-carboxamide

N-[4-(8-bromo-4-oxo-3,4-dihydro[1]benzofuro[3,2-d]pyrimidin-2-yl)-3-chlorophenyl]piperidine-3-carboxamide was synthesized in a manner similar to **Example 11+coupling**, wherein 1-(tert-butoxycarbonyl)piperidine-3-carboxylic acid replaced 3-dimethylpropionic acid. The resulting product was dissolved 2 mL of 4 N HCl in Dioxane and 4 mL of EtOAc. The mixture was heated to 50 °C for 1h, concentrated under reduced pressure. The residue was purified by preparative HPLC, resulting in the title compound. ¹H NMR (400 MHz, d₆-DMSO): 13.35 (s, 1H), 10.76 (s, 1H), 8.23 (s, 1H), 7.99 (s, 1H), 7.88 (m, 2H), 7.64 (m, 2H), 3.19 (m, 1H), 3.06 (m, 1H), 2.94 (m, 2H), 2.08 (m, 1H), 1.75 (m, 4H); MS (EI) for C₂₂H₁₈BrClN₄O₃: 503 (MH⁺).

### (Compound 115)

### N-[4-(8-bromo-4-oxo-3,4-dihydro[1]benzofuro[3,2-d]pyrimidin-2-yl)-3-chlorophenyl]piperidine-4-carboxamide

N-[4-(8-bromo-4-oxo-3,4-dihydro[1]benzofuro[3,2-d]pyrimidin-2-yl)-3-chlorophenyl]piperidine-4-carboxamide was synthesized in a manner similar to **Example 11+coupling**, wherein 1-(tert-butoxycarbonyl)piperidine-4-carboxylic acid replaced 3-dimethylpropionic acid. The resulting product was dissolved 2 mL of 4 N HCl in Dioxane and 4 mL of EtOAc. The mixture was heated to 50 °C for 1h, concentrated under reduced pressure. The residue was purified by preparative HPLC, resulting in the title compound. ¹H NMR (400 MHz, d₆-DMSO): 10.40 (s, 1H), 8.35 (s, 1H), 8.15 (s, 1H), 7.93 (s, 1H), 7.78 (m, 2H), 7.57 (m, 2H), 3.25 (d, 3H), 2.82 (t, 2H), 1.92 (m, 2H), 1.75 (m, 2H); MS (EI) for C₂₂H₁₈BrClN₄O₃: 503 (MH⁺).

### (Compound 132)

### N-[4-(8-bromo-4-oxo-3,4-dihydro[1]benzofuro[3,2-d]pyrimidin-2-yl)-3-chlorophenyl]pyrrolidine-3-carboxamide

N-[4-(8-bromo-4-oxo-3,4-dihydro[1]benzofuro[3,2-d]pyrimidin-2-yl)-3-chlorophenyl]pyrrolidine-3-carboxamide was synthesized in a manner similar to **Example 11+coupling**, wherein 1-(tert-butoxycarbonyl)pyrrolidine-3-carboxylic acid replaced 3-dimethylpropionic acid. The resulting product was dissolved 2 mL of 4 N HCl in Dioxane and 4 mL of EtOAc. The mixture was heated to 50 °C for 1 h, concentrated under reduced pressure. The residue was purified by preparative HPLC, resulting in the title compound. ¹H NMR (400 MHz, d₆-DMSO): 10.51 (s, 1H), 8.34 (s, 1H), 8.16 (s, 1H), 7.90 (s,1H), 7.78 (m, 2H), 7.58 (m, 2H), 3.41 (m, 2H), 3.20 (m, 6H), 2.02 (m, 1H); MS (EI) for C₂₁H₁₆BrClN₄O₃: 489 (MH⁺).

### *(Compound 127)

### N-[4-(8-bromo-4-oxo-3.4-dihydro[1]benzofuro[3,2-d]pyrimidin-2-yl)-3-chlorophenyl]-N'2',N'2'-dimethylglycinamide

N-[4-(8-bromo-4-oxo-3,4-dihydro[1]benzofuro[3,2-d]pyrimidin-2-yl)-3-chlorophenyl]-N'2',N'2'-dimethylglycinamide was synthesized in a manner similar to **Example 11 + Coupling**, wherein dimethylaminoacetyl chloride replaced 3-dimethylaminopropionic acid chloride. 1H NMR (400 MHz, d6-DMSO): 10.03 (s, 1H), 8.25 (s, 1H), 8.07 (s, 1H), 7.91 (s, 1H), 7.70 (m, 2H), 7.61 (dd, 1H), 7.49 (d, 1H), 3.06 (s, 2H), 2.23 (s, 6H). MS (EI) for C20 H16 Br Cl N4 O3: 477 (MH+).

### *(Compound 143)

### N-[4-(8-bromo-4-oxo-3,4-dihydro[1]benzofuro[3,2-d]pyrimidin-2-yl)-3-chlorophenyl]pyridine-4-carboxamide

N-[4-(8-bromo-4-oxo-3,4-dihydro[1]benzofuro[3,2-d]pyrimidin-2-yl)-3-chlorophenyl]pyridine-4-carboxamide was synthesized in a manner similar to **Example 11 + Coupling**, wherein isonicotinoyl chloride replaced 3-dimethylaminopropionic acid chloride. 1H NMR (400 MHz, d6-DMSO): 10.87 (s, 1H), 8.84 (d, 2H), 8.24 (s, 1H), 8.12 (s, 1H), 7.91 (d, 2H), 7.86 (m, 3H), 7.71 (d, 1H). MS (EI) for C22 H12 Br Cl N4 03: 497 (MH+).

### (Compound 160)

### N-[4-(8-bromo-4-oxo-3,4-dihydro[1]benzofuro[3,2-d]pyrimidin-2-yl)-3-chlorophenyl]-4-(dimethylamino)butanamide

N-[4-(8-bromo-4-oxo-3,4-dihydro[1]benzofuro[3,2-d]pyrimidin-2-yl)-3-chlorophenyl]-4-(dimethylamino)butanamide was synthesized in a manner similar to **Example 11 + Coupling**, wherein 4-dimethylaminobutyric acid replaced 3-dimethylaminopropionic acid. 1H NMR (400 MHz, d6-DMSO): 10.40 (s, 1H), 8.22 (m, 2H), 7.99 (s, 1H), 7.86 (m, 2H), 7.61 (m, 2H), 2.44 (m, 4H), 2.30 (s, 6H), 1.79 (m, 2H). MS (EI) for C22 H20 Br Cl N4 03: 505 (MH+).

### *(Compound 162)

### N-[4-(8-bromo-4-oxo-3,4-dihydro[1]benzofuro[3,2-d]pyrimidin-2-yl)-3-chlorophenyl]-3-(1H-imidazol-4-yl)propanamide

N-[4-(8-bromo-4-oxo-3,4-dihydro[1]benzofuro[3,2-d]pyrimidin-2-yl)-3-chlorophenyl]-3-(1H-imidazol-4-yl)propanamide was synthesized in a manner similar to **Example 11 + Coupling**, wherein 3-(imidazol-4-yl)propionic acid replaced 3-dimethylaminopropionic acid. 1H NMR (400 MHz, d6-DMSO): 10.34 (s, 1H), 8.21 (s, 2H), 8.12 (s, 1H), 7.89 (s, 1H), 7.75 (m, 2H), 7.51 (m, 2H), 7.46 (s, 1H), 6.72 (s, 1H), 2.77 (m, 2H), 2.61 (m, 2H). MS (EI) for C22 H15 Br Cl N5 O3: 514 (MH+).

### *(Compound 181)

### N-[4-(8-bromo-4-oxo-3,4-dihydro[1]benzofuro[3,2d]pyrimidin-2-yl)-3-chlorophenyl]-3-piperidin-1-ylpropanamide

N-[4-(8-bromo-4-oxo-3,4-dihydro[1]benzofuro[3,2-d]pyrimidin-2-yl)-3-chlorophenyl]-3-piperidin-1-ylpropanamide was synthesized in a manner similar to **Example 11 + Coupling**, wherein 1-piperidinepropionic acid replaced 3-dimethylaminopropionic acid. 1H NMR (400 MHz, d6-DMSO): 10.56 (s, 1H), 8.23 (dd, 1H), 8.20 (s, 1H), 7.97 (d, 1H), 7.86 (m, 2H), 7.62 (d, 1H), 7.56 (dd, 1H), 2.66 (m, 2H), 2.54 (m, 2H), 2.44 (m, 4H), 1.52 (m, 4H), 1.40 (m, 2H). MS (EI) for C24 H22 Br Cl N4 03: 531 (MH+).

### *(Compound 182)

### N-[4-(8-bromo-4-oxo-3,4-dihydro[1]benzofuro[3,2-d]pyrimidin-2-yl)-3-chlorophenyl]-2-(1H-imidazol-4-yl)acetamide

N-[4-(8-bromo-4-oxo-3,4-dihydro[1]benzofuro[3,2-d]pyrimidin-2-yl)-3-chlorophenyl]-2-(1H-imidazol-4-yl)acetamide was synthesized in a manner similar to **Example 11 + Coupling**, wherein 4-imidazoleacetic acid replaced 3-dimethylaminopropionic acid. 1H NMR (400 MHz, d6-DMSO): 10.55 (s, 1H), 8.29 (s, 1H), 8.19 (s, 1H), 7.99 (s, 1H), 7.85 (m, 2H), 7.61 (m, 3H), 6.97 (s, 1H), 3.64 (s, 2H). MS (EI) for C21 H13 Br Cl N5 O3: 500 (MH+).

### (Compound 248)

### N-[4-(8-bromo-4-oxo-3,4-dihydro[1]benzofuro[3,2-d]pyrimidin-2-yl)-3-chlorophenyl]-3-morpholin-4-ylpropanamide

N-[4-(8-bromo-4-oxo-3,4-dihydro[1]benzofuro[3,2-d]pyrimidin-2-yl)-3-chlorophenyl]-3-morpholin-4-ylpropanamide was synthesized in a manner similar to **Example 11 + Coupling**, wherein 3-morpholin-4-yl-propionic acid replaced 3-dimethylaminopropionic acid. 1H NMR (400 MHz, d6-DMSO): 10.43 (s, 1H), 8.27 (s, 1H), 8.17 (s, 1H), 7.98 (s, 1H), 7.86 (m, 2H), 7.60 (m, 2H), 3.58 (t, 4H), 2.65 (t, 2H), 2.53 (t, 2H), 2.41 (t, 4H). MS (EI) for C23 H20 Br Cl N4 O4: 533 (MH+).

### *(Compound 249)

### N-[4-(8-bromo-4-oxo-3,4-dihydro[1]benzofuro[3,2-d]pyrimidin-2-yl)-3-chlorophenyl]benzamide

N-[4-(8-bromo-4-oxo-3,4-dihydro[1]benzofuro[3,2-d]pyrimidin-2-yl)-3-chlorophenyl]benzamide was synthesized in a manner similar to **Example 11 + Coupling**, wherein benzoyl chloride replaced 3-dimethylaminopropionic acid chloride. 1H NMR (400 MHz, d6-DMSO): 13.39 (s, 1H), 10.67 (s, 1H), 8.26 (s, 1H), 8.16 (s, 1H), 8.01 (d, 2H), 7.88 (m, 3H), 7.63 (m, 4H). MS (EI) for C23 H13 Br Cl N3 O3: 496 (MH+).

### *(Compound 250)

### N-[4-(8-bromo-4-oxo-3,4-dihydro[1]benzofuro[3,2-d]pyrimidin-2-yl)-3-chlorophenyl]cyclohexanecarboxamide

N-[4-(8-bromo-4-oxo-3,4-dihydro[1]benzofuro[3,2-d]pyrimidin-2-yl)-3-chlorophenyl]cyclohexanecarboxamide was synthesized in a manner similar to **Example 11 + Coupling**, wherein cyclohexanecarbonyl chloride replaced 3-dimethylaminopropionic acid chloride. 1H NMR (400 MHz, d6-DMSO): 13.34 (s, br, 1H), 10.25 (s, 1H), 8.23 (s, 1H), 7.95 (s, 1H), 7.85 (m, 2H), 7.60 (s, 2H), 2.37 (m, 1H), 1.80 (m, 4H), 1.32 (m, 6H). MS (EI) for C23 H19 Br Cl N3 03: 502 (MH+).

### *(Compound 251)

### N-[4-(8-bromo-4-oxo-3,4-dihydro[1]benzofuro[3,2-d]pyrimidin-2-yl)-3-chlorophenyl]cyclopentanecarboxamide

N-[4-(8-bromo-4-oxo-3,4-dihydro[1]benzofuro[3,2-d]pyrimidin-2-yl)-3-chlorophenyl]cyclopentanecarboxamide was synthesized in a manner similar to **Example 11 + Coupling**, wherein cyclopentanecarbonyl chloride replaced 3-dimethylaminopropionic acid chloride. 1H NMR (400 MHz, d6-DMSO): 13.33 (s, br, 1H), 10.30 (s, 1H), 8.23 (s, 1H), 7.99 (s, 1H), 7.85 (m, 2H), 7.61 (s, 2H), 2.81 (m, 1H), 1.88 (m, 2H), 1.71 (m, 4H), 1.57 (m, 2H). MS (EI) for C22 H17 Br Cl N3 O3: 488 (MH+).

### *(Compound 279)

### N-[4-(8-bromo-4-oxo-3,4-dihydro[1]benzofuro[3,2-d]pyrimidin-2-yl)-3-chlorophenyl]-4-chlorobenzamide

N-[4-(8-bromo-4-oxo-3,4-dihydro[1]benzofuro[3,2-d]pyrimidin-2-yl)-3-chlorophenyl]-4-chlorobenzamide was synthesized in a manner similar to **Example 11 + Coupling,** wherein 4-chlorobenzoyl chloride replaced 3-dimethylaminopropionic acid chloride. 1H NMR (400 MHz, d6-DMSO): 10.67 (s, 1H), 8.21 (s, 1H), 8.11 (s, 1H), 8.03 (d, 2H), 7.84 (m, 3H), 7.67 (m, 3H). MS (EI) for C23 H12 Br Cl2 N3 O3: 530 (MH+).

### *(Compound 280)

### N-[4-(8-bromo-4-oxo-3,4-dihydro[1]benzofuro[3,2-d]pyrimidin-2-yl)-3-chlorophenyl]-3-chlorobenzamide

N-[4-(8-bromo-4-oxo-3,4-dihydro[1]benzofuro[3,2-d]pyrimidin-2-yl)-3-chlorophenyl]-3-chlorobenzamide was synthesized in a manner similar to **Example 11 + Coupling**, wherein 3-chlorobenzoly chloride replaced 3-dimethylaminopropionic acid chloride. 1H NMR (400 MHz, d6-DMSO): 10.70 (s, 1H), 8.22 (s, 1H), 8.10 (s, 1H). 8.06 (s, 1H), 7.96 (d, 1H), 7.84 (m, 3 H), 7.70, (m, 2H), 7.61 (m, 1H). MS (EI) for C23 H12 Br Cl2 N3 O3: 530 (MH+).

### *(Compound 281)

### N-[4-(8-bromo-4-oxo-3,4-dihydro[1]benzofuro[3,2-d]pyrimidin-2-yl)-3-chlorophenyl]-3-(methyloxy)benzamide

N-[4-(8-bromo-4-oxo-3,4-dihydro[1]benzofuro[3,2-d]pyrimidin-2-yl)-3-chlorophenyl]-3-(methyloxy)benzamide was synthesized in a manner similar to **Example 11 + Coupling**, wherein 3-methoxybenzoyl chloride replaced 3-dimethylaminopropionic acid chloride. 1H NMR (400 MHz, d6-DMSO): 13.39 (s, 1H), 10.62 (s, 1H), 8.26 (s, 1H), 8.14 (s, 1H), 7.88 (m, 3H), 7.70 (d, 1H), 7.58 (d, 1H), 7.51 (m, 2H), 7.21 (d, 1H), 3.86 (s, 3H). MS (EI) for C24 H15 Br Cl N3 O4: 526 (MH+).

### *(Compound 285)

### N-[4-(8-bromo-4-oxo-3,4-dihydro[1]benzofuro[3,2-d]pyrimidin-2-yl)-3-chlorophenyl]-4-(methyloxy)benzamide

N-[4-(8-bromo-4-oxo-3,4-dihydin[1]benzofuro[3,2-d]pyrimidin-2-yl)-3-chlorophenyl]-4-(methyloxy)benzamide was synthesized in a manner similar to **Example 11 + Coupling**, wherein 4-methoxybenzoly chloride replaced 3-dimethylaminopropionic acid chloride. 1H NMR (400 MHz, d6-DMSO): 13.36 (s, br, 1H), 10.46 (s, 1H), 8.24 (s, 1H), 8.13 (s, 1H), 8.01 (d, 2H), 7.87 (m, 3H), 7.67 (d, 1H), 7.11 (d, 2H), 3.87 (s, 3H). MS (EI) for C24 H15 Br Cl N3 O4: 526 (MH+).

### *(Compound 319)

### N-[4-(8-bromo-4-oxo-3,4-dihydro[1]benzofuro[3,2-d]pyrimidin-2-yl)-3-chlorophenyl]-2-(methyloxy)acetamide

N-[4-(8-bromo-4-oxo-3,4-dihydro[1]benzofuro[3,2-d]pyrimidin-2-yl)-3-chlorophenyl]-2-(methyloxy)acetamide was synthesized in a manner similar to **Example 11 + Coupling**, wherein methoxyacetyle chloride replaced 3-dimethylaminopropionic acid chloride. 1H NMR (400 MHz, d6-DMSO): 10.21 (s, 1H), 8.23 (s, 1H), 8.03 (s, 1H), 7.85 (m, 2H), 7.75 (dd, 1H), 7.63 (d, 1H), 4.07 (s, 2H), 3.40 (s, 3H). MS (EI) for C19 H13 Br Cl N3 O4: 464 (MH+).

### *(Compound 320)

### methyl [4-(8-bromo-4-oxo-3,4-dihydro[1]benzofuro[3.2-d]pyrimidin-2-yl)-3-chlorophenyl]carbamate

methyl [4-(8-bromo-4-oxo-3,4-dihydro[1]benzofuro[3,2-d]pyrimidin-2-yl)-3-chlorophenyl]carbamate was synthesized in a manner similar to **Example 11 + Coupling**, wherein methyl chloroformate replaced 3-dimethylaminopropionic acid chloride. 1H NMR (400 MHz, d6-DMSO): 13.30 (s, br, 1H), 10.16 (s, 1H), 8.24 (s, 1H), 7.86 (m, 2H), 7.76 (s, 1H), 7.62 (d, 1H), 7.52 (dd, 1H), 3.72 (s, 3H). MS (EI) for C18 H11 Br Cl N3 O4:4450 (MH+).

### *(Compound 321)

### N-[4-(8-bromo-4-oxo-3,4-dihydro[1]benzofuro[3,2-d]pyrimidin-2-yl)-3-chlorophenyl]furan-2-carboxamide

N-[4-(8-bromo-4-oxo-3,4-dihydro[1]benzofuro[3,2-d]pyrimidin-2-yl)-3-chlorophenyl]furan-2-carboxamide was synthesized in a manner similar to **Example 11 + Coupling**, wherein 3-chlorobenzoyl chloride replaced 3-dimethylaminopropionic acid chloride. 1H NMR (400 MHz, d6-DMSO): 13.33 (s, br, 1H), 10.59 (s, 1H), 8.24 (s, 1H), 8.11 (s, 1H), 8.01 (s, 1H), 7.87 (m, 3H), 7.67 (d, 1H), 7.43 (d, 1H), 6.77 (s, 1H). MS (EI) for C21 H11 Br Cl N3 O4: 486 (MH+).

### *(Compound 322)

### N-[4-(8-bromo-4-oxo-3,4-dihydro[1]benzofuro[3,2-d]pyrimidin-2-yl)-3-chlorophenyl]-2-pyridin-3-ylacetamide

N-[4-(8-bromo-4-oxo-3,4-dihydro[1]benzofuro[3,2-d]pyrimidin-2-yl)-3-chlorophenyl]-2-pyridin-3-ylacetamide was synthesized in a manner similar to **Example 11 + Coupling**, wherein 2-(pyridin-3-yl)acetic acid replaced 3-dimethylaminopropionic acid. 1H NMR (400 MHz, d6-DMSO): 10.67 (s, 1H), 8.54 (s, 1H), 8.48 (d, 1H), 8.22 (d, 1H), 7.94 (s, 1H), 7.80 (m, 3H), 7.60 (m, 2H), 7.39 (m, 1H), 3.78 (s, 2H). MS (EI) for C23 H14 Br Cl N4 O3: 511 (MH+).

### *(Compound 323)

### N-[4-(8-bromo-4-oxo-3,4-dihydro[1]benzofuro[3,2-d]pyrimidin-2-yl)-3-chlorophenyl]pyrazine-2-carboxamide

N-[4-(8-bromo-4-oxo-3,4-dihydro[1]benzofuro[3,2-d]pyrimidin-2-yl)-3-chlorophenyl]pyrazine-2-carboxamide was synthesized in a manner similar to **Example 11 + Coupling**, wherein pyrazine-2-carboxylic acid replaced 3-dimethylaminopropionic acid. 1H NMR (400 MHz, d6-DMSO): 13.40 (s, br, 1H), 11.21 (s, 1H), 9.38 (s, 1H), 8.98 (s, 1H), 8.86 (s, 1H), 8.26 (dd, 2H), 8.04 (dd, 1H), 77.86 (m, 2H), 7.71 (d, 1H). MS (EI) for C21 H11 Br Cl N5 03: 498 (MH+).

### *(Compound 324)

### N-[4-(8-bromo-4-oxo-3,4-dihydro[1]benzofuro[3,2-d]pyrimidin-2-yl)-3-chlorophenyl]isoxazole-5-carboxamide

N-[4-(8-bromo-4-oxo-3,4-dihydro[1]benzofuro[3,2-d]pyrimidin-2-yl)-3-chlorophenyl]isoxazole-5-carboxamide was synthesized in a manner similar to **Example 11 + Coupling**, wherein isoxazole-5-carboxylic acid replaced 3-dimethylaminopropionic acid. 1H NMR (400 MHz, d6-DMSO): 11.16 (s, 1H), 8.89 (s, 1H), 8.22 (s, 1H), 8.09 (s, 1H), 7.84 (m, 3H), 7.70 (d, 1H), 7.35 (s, 1H). MS (EI) for C20 H10 Br Cl N4 O4: 487 (MH+).

### *(Compound 334)

### methyl 6-({[4-(8-bromo-4-oxo-3,4-dihydro[1]benzofuro[3,2-d]pyrimidin-2-yl)-3-chlorophenyl]amino}carbonyl)pyridne-3-carboxylate

methyl 6-({[4-(8-bromo-4-oxo-3,4-dihydro[1]benzofuro[3,2-d]pyrimidin-2-yl)-3-chlorophenyl]amino}carbonyl)pyridine-3-carboxylate was synthesized in a manner similar to **Example 11 + Coupling**, wherein 5-(methoxycarbonyl)picolinic acid replaced 3-dimethylaminopropionic acid. 1H NMR (400 MHz, d6-DMSO): 11.21 (s, 1H), 9.22 (s, 1H), 8.58 (dd, 1H), 8.33(d, 1H), 8.27 (s, 1H), 8.19 (s, 1H), 8.03 (dd, 1H), 7.81 (m, 2H), 7.67 (d, 1H), 3.97 (s, 3H). MS (EI) for C24 H14 Br Cl N4 O5: 555 (MH+).

### *(Compound 335)

### N-[4-(8-bromo-4-oxo-3,4-dihydro[1]benzofuro[3,2-d]pyrimidin-2-yl)-3-chlorophenyl]-2-(methyloxy)pyridine-4-carboxamide

N-[4-(8-bromo-4-oxo-3,4-dihydro[1]benzofuro[3,2-d]pyrimidin-2-yl)-3-chlorophenyl]-2-(methyloxy)pyridine-4-carboxamide was synthesized in a manner similar to **Example 11 + Coupling**, wherein 2-methoxyisonicotinic acid replaced 3-dimethylaminopropionic acid. 1H NMR (400 MHz, d6-DMSO): 13.39 (s, br, 1H), 10.81 (s, 1H), 8.40 (d, 1H), 8.25 (s, 1H), 8.12 (s, 1H), 7.88 (m, 3H), 7.71 (d, 1H), 7.48 (dd, 1H), 7.36 (s, 1H), 3.95 (s, 3H). MS (EI) for C23 H 14 Br Cl N4 O4: 527 (MH+).

### *(Compounds 336)

### 5-bromo-N-[4-(8-bromo-4-oxo-3,4-dihydro[1]benzofuro[3,2-d]pyrimidin-2-yl)-3-chlorophenyl]pyridine-2-carboxamide

5-bromo-N-[4-(8-bromo-4-oxo-3,4-dihydro[1]benzofuro[3,2-d]pyrimidin-2-yl)-3-chlorophenyl]pyridine-2-carboxamide was synthesized in a manner similar to **Example 11 + Coupling,** wherein 5-bromopicolinic acid replaced 3-dimethylaminopropionic acid. 1H NMR (400 MHz, d6-DMSO): 13.36 (s, br, 1H), 11.16 (s, 1H), 8.91 (s, 1H), 8.37 (dd, 1H), 8.27 (dd, 2H), 8.13 (d, 1H), 8.05 (dd, 1H), 7.87 (m, 2H), 7.70 (d, 1H). MS (EI) for C22 H11 Br2 Cl N4 03: 576 (MH+).

### *(Compound 339)

### N-[4-(8-bromo-4-oxo-3.4-dihydro[1]benzofuro[3,2-d]pyrimidin-2-yl)-3-chlorophenyl]-5-chloropyridine-2-carboxamide

N-[4-(8-bromo-4-oxo-3,4-dihydro[1]benzofuro[3,2-d]pyrimidin-2-yl)-3-chlorophenyl]-5-chloropyridine-2-carboxamide was synthesized in a manner similar to **Example 11 + Coupling**, wherein 5-chloropicolinic acid replaced 3-dimethylaminopropionic acid. 1H NMR (400 MHz, d6-DMSO): 13.37 (s, br, 1H), 11.16 (s, 1H), 8.84 (s, 1H), 8.30 (s, 1H), 8.23 (m, 3H), 8.06 (d, 1H), 7.87 (m, 2H), 7.70 (d, 1H). MS (EI) for C22 H11 Br Cl2 N4 03: 531 (MH+).

### *(Compound 373)

### N-[4-(8-bromo-4-oxo-3,4-dihydro[1]benzofuro[3,2-d]pyrimidin-2-yl)-3-chlorophenyl]-1,3-oxazole-2-carboxamide

N-[4-(8-bromo-4-oxo-3,4-dihydro[1]benzofuro[3,2-d]pyrimidin-2-yl)-3-chlorophenyl]-1,3-oxazole-2-carboxamide was synthesized in a manner similar to **Example 11 + Coupling**, wherein isoxazole-2-carboxylic acid replaced 3-dimethylaminopropionic acid. 1H NMR (400 MHz, d6-DMSO): 13.37 (s, 1H), 11.28 (s, 1H), 8.46 (s, 1H), 8.23 (s, 1H), 8.14 (s, 1H), 7.93 (dd, 1H), 7.85 (m, 2H), 7.68 (d, 1H), 7.60 (s, 1H). MS (EI) for C20 H10 Br Cl N4 O4: 487 (MH+).

### *(Compound 389)

### N-14-(8-bromo-4-oxo-3,4-dihydro[1]benzofuro[3,2-d]pyrimidin-2-yl)-3-chlorophenyl]-1-methyl-1H-imidazole-4-carboxamide

N-[4-(8-bromo-4-oxo-3,4-dihydro[1]benzofuro[3,2-d]pyrimidin-2-yl)-3-chlorophenyl]-1-methyl-1H-imidazole-4-carboxamide was synthesized in a manner similar to **Example 11 + Coupling**, wherein 1-methyl-1H-imidazole-4-carboxylic acid replaced 3-dimethylaminopropionic acid. 1H NMR(400 MHz, d6-DMSO): 10.35 (s, 1H), 8.25 (dd, 2H), 7.97 (dd, 1H), 7.88 (m, 3H), 7.83 (s, 1H), 7.63 (d, 1H), 3.77 (s, 3H). MS (EI) for C21 H13 Br Cl N5 O3: 500 (MH+).

### *(Compound 390)

### N-[4-(8-bromo-4-oxo-3,4-dihydro[1]benzofuro[3,2-d]pyrimidin-2-yl)-3-chlorophenyl]-1-methyl-1H-imidazole-2-carboxamide

N-[4-(8-bromo-4-oxo-3,4-dihydro[1]benzofuro[3,2-d]pyrimidin-2-yl)-3-chlorophenyl]-1-methyl-1H-imidazole-2-carboxamide was synthesized in a manner similar to **Example 11 + Coupling** wherein 1-methyl-1H-imidazole-2-carboxylic acid replaced 3-dimethylaminopropionic acid. 1H NMR (400 MHz, d6-DMSO): 13.33 (s, br, 1H), 10.83 (s, 1H), 8.23 (dd, 2H), 7.96 (dd, 1H), 7.86 (m, 2H), 7.65 (d, 1H), 7.52 (s, 1H), 7.14 (s, 1H), 4.02 (s, 3H). MS (EI) for C21 H13 Br Cl N5 O3: 500 (MH+).

### *(Compound 391)

### N-[4-(8-bromo-4-oxo-3,4-dihydro[1]benzofuro[3,2-d]pyimidin-2-yl)-3-chlorophenyl]-1-methyl-1H-pyrazole-3-carboxamide

N-[4-(8-bromo-4-oxo-3,4-dihydro[1]benzofuro[3,2-d]pyrimidin-2-yl)-3-chlorophenyl]-1-methyl-1H-pyrazole-3-carboxamide was synthesized in a manner similar to **Example 11 + Coupling** wherein 1-methyl-1H-pyrazole-3-carboxylic acid replaced 3-dimethylaminopropionic acid. 1H NMR (400 MHz, d6-DMSO): 13.31 (s, br, 1H), 10.56 (s, 1H), 8.22 (d, 2H), 7.95 (d, 1H), 7.90 (m, 1H), 7.86 (m, 2H), 7.64 (d, 1H), 6.81 (s, 1H), 4.00 (s, 3H). MS (EI) for C21 H13 Br Cl N5 O3: 500 (MH+).

### *(Compound 135)

### N-[4-(8-bromo-4-oxo-3,4-dihydro[1]benzofuro[3,2-d]pyrimidin-2-yl)-3-chlorophenyl]-N'3'N'3'-dimethyl-beta-alaninamide

To a solution of 3-dimethylaminopropionic acid (108 mg, 0.7 mmol) in dichloromethane (5 mL) was added oxalyl chloride (245 µL, 2.8 mmol) followed by catalytic amount (2 drops) of dimethylformamide. The reaction mixture was stirred at room temperature for 1 hour. The solvent was concentrated *in vacuo* to give solid 3-dimethylaminopropionic acid chloride. The resulting product was used without further purification. A solution of 2-(4-amino-2-chlorophenyl)-8-bromo[1]benzofuro[3,2-d]pyrimidin-4(3H)-one (200 mg, 0.47 mmol) in pyridine (3 mL) was added to a suspension of 3-dimethylaminopropionic acid chloride (0.7 mmol) in dichloromethane (2 mL). The reaction was stirred at room temperature for 15 minutes. The solvent was concentrated under reduced pressure and the residue was taken up in 3 mL of dimethylformamide. Purification by preparative HPLC (reverse-phase, acetonitrile/water with 0.1% formic acid) gave 23 mg (10%) of N-[4-(8-bromo-4-oxo-3,4-dihydro[1]benzofuro[3,2-d]pyrimidin-2-yl)-3-chlorophenyl]-N'3',N'3'-dimethyl-beta-alaninamide. 1H NMR (400 MHz, d6-DMSO): 10.48 (s, 1H), 8.23 (s, 1H), 7.97 (s, 1H), 7.85 (m, 2H), 7.60 (m, 2H), 2.62 (m, 2H), 2.53 (m, 2H), 2.22 (s, 6H). MS. (EI) for C₂₁ H₁₈ Br Cl N₄ O₃: 491 (MH+).

### (Compound 218)

### N-[4-(8-bromo-4-oxo-3,4-dihydro[1]benzofuro[3,2-d]pyrimidin-2-yl)-3-chlorophenyl]-2-chloro-6-methylpyridine-4-carboxamide

N-[4-(8-bromo-4-oxo-3,4-dihydro[1]benzofuro[3,2-d]pyrimidin-2-yl)-3-chlorophenyl]-2-chloro-6-methylpyridine-4-carboxamide was synthesized in a manner similar to **Example 11 + coupling,** wherein 3-dimethylaminopropionic acid was substituted with 2-chloro-6-methyl isonicotinic acid. Purification by preparative HPLC (reverse-phase, acetonitrile/water with 0.1% formic acid), followed by concentration *in vacuo* and lyophilization afforded the title compound (27.9 mg). NMR (400 MHz, d₆-DMSO): 13.36 (s, 1H), 10.88 (s, 1H), 8.25 (d, 1H), 8.09 (s, 1H), 7.83-7.90 (m, 3H), 7.78 (s, 1H), 7.73 (s, 1H), 7.71 (s, 1H), 2.58 (s, 3H). MS (EI) for C₂₃H₁₃BrClN₄O₃ : 544 (MH⁺).

### *(Compound 275)

### N-[4-(8-bromo-4-oxo-3,4-dihydro[1]benzofuro[3,2-d]pyrimidin-2-yl)-3-chlorophenyl]-3-chloropyridine-4-carboxamide

N-[4-(8-bromo-4-oxo-3,4-dihydro[1]benzofuro[3,2-d]pyrimidin-2-yl)-3-chlorophenyl]-3-chloropyridine-4-carboxamide was synthesized in a manner similar to **Example 11 + coupling,** wherein 3-dimethylaminopropionic acid was substituted with 3-chloroisonicotinic acid. Purification by preparative HPLC (reverse-phase, acetonitrile/water with 0.1% formic acid), followed by concentration *in vacuo* and lyophilization afforded the title compound (17.2 mg). NMR (400 MHz, d₆-DMSO): 13.39 (s, 1H), 11.13 (s, 1H), 8.84 (s, 1H), 8.72 (d, 1H), 8.24 (s, 1H), 8.04 (s, 1H), 7.82-7.90 (m, 2H), 7.75 (d, 1H), 7.72 (s, 2H). MS (EI) for C₂₂H₁₁BrCl₂N₄O₃: 530 (MH⁺).

### *(Compound 276)

### N-[4-(8-bromo-4-oxo-3,4-dihydro[1]benzofuro[3,2-d]pyrimidin-2yl)-3-chlorophenyl]-2-chloropyridine-4-carboxamide

N-[4-(8-bromo-4-oxo-3,4-dihydro[1]benzofuro[3,2-d]pyrimidin-2-yl)-3-chlorophenyl]-2-chloropyridine-4-carboxamide was synthesized in a manner similar to **Example 11 + coupling,** wherein 3-dimethylaminopropionic acid was substituted with 2-chloroisonicotinic acid. Purification by preparative HPLC (reverse-phase, acetonitrile/water with 0.1% formic acid), followed by concentration *in vacuo* and lyophilization afforded the title compound (11.3 mg). NMR (400 MHz, d₆-DMSO): 13.39 (s, 1H), 10.93 (s, 1H), 8.67 (d, 1H), 8.25 (s, 1H), 8.11 (s, 1H), 8.06 (s, 1H), 7.83-7.93 (m, 4H), 7.73 (d, 1H). MS (EI) for C₂₂H₁₁BrCl₂N₄O₃: 530 (MH⁺).

### *(Compound 291)

### N-[4-(8-bromo-4-oxo-3,4-dihydro[1]benzofuro[3,2-d]pyrimidin-2-yl)-3-chlorophenyl]-6-methylpyridine-3-carboxamide

N-[4-(8-bromo-4-oxo-3,4-dihydro[1]benzofuro[3,2-d]pyrimidin-2-yl)-3-chlorophenyl]-6-methylpyridine-3-carboxamide was synthesized in a manner similar to **Example 11 + coupling**, wherein 3-dimethylaminopropionic acid was substituted with 6-methylnicotinic acid. Purification by preparative HPLC (reverse-phase, acetonitrile/water with 0.1% formic acid), followed by concentration *in vacuo* and lyophilization afforded the title compound (37.0 mg). NMR (400 MHz, d₆-DMSO): 13.39 (s, 1H), 10.72 (s, 1H), 9.04 (d, 1H), 8.25-8.27 (m, 1H), 8.24 (d, 1H), 8.13 (d, 1H), 7.84-7.91 (m, 3H), 7.71 (d, 1H), 7.47 (d, 1H), 2.58 (s, 3H). MS (EI) for C₂₃H₁₄BrClN₄O₃: 509 (MH⁺).

### *(Compound 292)

### N-[4-(8-bromo4-oxo-3,4-dihydro[1]benzofuro[3,2-d]pyrimidin-2-yl)-3-chlorophenyl]pyridine-3-carboxamide

N-[4-(8-bromo-4-oxo-3,4-dihydro[1]benzofuro[3,2-d]pyrimidin-2-yl)-3-chlorophenyl]pyridine-3-carboxamide was synthesized in a manner similar to **Example 11 + coupling**, wherein 3-dimethylaminopropionic acid was substituted with nicotinic acid. Purification by preparative HPLC (reverse-phase, acetonitrile/water with 0.1 % formic acid), followed by concentration *in vacuo* and lyophilization afforded the title compound (7.5 mg). NMR (400 MHz, d₆-DMSO): 10.83 (s, 1H), 9.16 (d, 1H), 8.80-8.82 (m, 1H), 8.33-8.37 (m, 1H), 8.22 (d, 1H), 8.12 (d, 1H), 7.95 (s, 1H), 7.80-7.88 (m, 3H), 7.69 (d, 1H), 7.60-7.64 (m, 1H). MS (EI) for C₂₂H₁₂BrClN₄O₃: 495 (MH⁺).

### *(Compound 293)

### N-[4-(8-bromo-4-oxo-3,4-dihydro[1]benzofuro[3,2-d]pyrimidin-2-yl)-3-chlorophenyl]pyridine-2-carboxamide

N-[4-(8-bromo-4-oxo-3,4-dihydro[1]benzofuro[3,2-d]pyrimidin-2-yl)-3-chlorophenyl]pyridine-2-carboxamide was synthesized in a manner similar to **Example 11 + coupling**, wherein 3-dimethylaminopropionic acid was substituted with picolinic acid. Purification by preparative HPLC (reverse-phase, acetonitrile/water with 0.1% formic acid), followed by concentration *in vacuo* and lyophilization afforded the title compound (33.8 mg). NMR (400 MHz, d₆-DMSO): 13.36 (s, 1H), 11.10 (s, 1H), 8.79 (d, 1H), 8.31 (d, 1H), 8.16-8.26 (m, 2H), 8.03-8.15 (m, 2H), 7.81-7.90 (m, 2H), 7.66-7.76 (m, 2H). MS (EI) for C₂₂H₁₂BrClN₄O₃: 495 (MH⁺).

### *(Compounds 294)

### N-[4-8-bromo-4-oxo-3,4-dihydro[1]benzofuro[3,2-d]pyrimidin-2-yl)-3-chlorophenyl]-2,6-dichloropyridine-4-carboxamide

N-[4-(8-bromo-4-oxo-3,4-dihydro[1]benzofuro[3,2-d]pyrimidin-2-yl)-3-chlorophenyl]-2,6-dichloropyridine-4-carboxamide was synthesized in a manner similar to **Example 11 + coupling**, wherein 3-dimethylaminopropionic acid was substituted with 2,6-dichloroisonicotinic acid. Purification by preparative HPLC (reverse-phase, acetonitrile/water with 0.1% formic acid), followed by concentration *in vacuo* and lyophilization afforded the title compound (10.4 mg). NMR (400 MHz, d₆-DMSO): 10.50 (s, 1H), 8.12-8.25 (m, 2H), 7.80-7.91 (m, 2H), 7.67 (d, 1H), 7.51-7.56 (m, 1H), 7.38 (t, 1H), 7.14-7.18 (m, 1H). MS (EI) for C₂₂H₁₀BrCl₃N₄O₃: 564 (MH⁺).

### *(Compounds 312)

### N-[4-(8-bromo-4-oxo-3,4-dihydro[1]benzofuro[3,2-d]pyrimidin-2-yl)-3-chlorophenyl]-5-methylpyrazine-2-carboxamide

N-[4-(8-bromo-4-oxo-3,4-dihydro[1]benzofuro[3,2-d]pyrimidin-2-yl)-3-chlorophenyl]-5-methylpyrazine-2-carboxamide was synthesized in a manner similar to **Example 11 + coupling**, wherein 3-dimethylaminopropionic acid was substituted with 5-methylpyrazine-2-carboxylic acid. Purification by preparative HPLC (reverse-phase, acetonitrile/water with 0.1% formic acid), followed by concentration *in vacuo* and lyophilization afforded the title compound (17.0 mg). NMR (400 MHz, d₆-DMSO): 11.11 (s, 1H), 9.21 (s, 1H), 8.75 (s, 1H), 8.17-8.28 (m, 2H), 8.01-8.06 (m, 1H), 7.80-7.89 (m, 1H), 7.69 (d, 1H), 2.66 (s, 3H). MS (EI) for C₂₂H₁₃BrClN₅O₃: 510 (MH⁺).

### *(Compound 313)

### N-[4-(8-bromo-4-oxo-3,4-dihydro[1]benzofuro[3,2-d]pyrimidin-2-yl)-3-chlorophenyl]tetrahydrofuran-3-carboxamide

N-[4-(8-bromo-4-oxo-3,4-dihydro[1]benzofuro[3,2-d]pyrimidin-2-yl)-3-chlorophenyl]tetrahydrofuran-3-carboxamide was synthesized in a manner similar to **Example 11 + coupling**, wherein 3-dimethylaminopropionic acid was substituted with tetrahydro-3-furoic acid. Purification by preparative HPLC (reverse-phase, acetonitrile/water with 0.1% formic acid), followed by concentration *in vacuo* and lyophilization afforded the title compound (35.0 mg). NMR (400 MHz, d₆-DMSO): 13.30 (s, 1H), 10.45 (s, 1H), 8.24 (d, 1H), 7.98 (d, 1H), 7.82-7.89 (m, 2H), 7.57-7.66 (m, 2H), 3.96 (t, 1H), 3.69-3.83 (m, 3H), 3.14-3.24 (m, 1H), 2.05-2.16 (m, 2H). MS (EI) for C₂₁H₁₅BrClN₃O₄: 488 (MH⁺).

### *(Compound 328)

### N-[4-8-bromo-4-oxo-3,4-dihydro[1]benzofuro[3,2-d]pyrimidin-2-yl)-3-chlorophenyl]pyrimidine-5-carboxamide

N-[4-(8-bromo-4-oxo-3,4-dihydro[1]benzofuro[3,2-d]pyrimidin-2-yl)-3-chlorophenyl]pyrimidine-5-carboxamide was synthesized in a manner similar to **Example 11 + coupling,** wherein 3-dimethylaminopropionic acid was substituted with 5-pyrimidine carboxylic acid. Purification by preparative HPLC (reverse-phase, acetonitrile/water with 0.1 % formic acid), followed by concentration *in vacuo* and lyophilization afforded the title compound (9.9 mg). NMR (400 MHz, d₆-DMSO): 13.41 (s, 1H), 10.96 (s, 1H), 9.41 (s, 1H), 9.36 (s, 2H), 8.25 (d, 1H), 8.11 (d, 1H), 7.83-7.91 (m, 3H), 7.73 (d, 1H). MS (EI) for C₂₁H₁₁,BrClN₅O₃: 496 (MH⁺).

### *(Compound 341)

### N-[4-(8-bromo-4-oxo-3,4-dihydro[1]benzofuro[3,2-d]pyrimidin-2-yl)-3-chlorophenyl]furan-3-carboxamide

N-[4-(8-bromo-4-oxo-3,4-dihydro[1]benzofuro[3,2-d]pyrimidin-2-yl)-3-chlorophenyl]furan-3-carboxamide was synthesized in a manner similar to **Example 11 + coupling**, wherein 3-dimethylaminopropionic acid was substituted with 3-furoic acid. Purification by preparative HPLC (reverse-phase, acetonitrile/water with 0.1 % formic acid), followed by concentration *in vacuo* and lyophilization afforded the title compound (10.0 mg). NMR (400 MHz, d₆-DMSO): 10.28 (s, 1H), 8.46 (s, 1H), 8.19-8.23 (m, 1H), 8.06 (d, 1H), 7.78-7.88 (m, 4H), 7.76 (d, 1H), 7.04 (s, 1H). MS (EI) for C₂₁H₁₁BrClN₃O₄: 484 (MH⁺).

### *(Compound 343)

### N-[4-(8-bromo-4-oxo-3,4-dihydro[1]benzofuro[3,2-d]pyrimidin-2-yl)-3-chlorophenyl]-6-(methyloxy)pyridine-3-carboxamide

N-[4-(8-bromo-4-oxo-3,4-dihydro[1]benzofuro[3,2-d]pyrimidin-2-yl)-3-chlorophenyl]-6-(methyloxy)pyridine-3-carboxamide was synthesized in a manner similar to **Example 11 + coupling**, wherein 3-dimethylaminopropionic acid was substituted with 6-methoxynicotinic acid. Purification by preparative HPLC (reverse-phase, acetonitrile/water with 0.1 % formic acid), followed by concentration *in vacuo* and lyophilization afforded the title compound (36.8 mg). NMR (400 MHz, d₆-DMSO): 10.61 (s, 1H), 8.82 (d, 1H), 8.28(d, 1H), 8.20 (d, 1H), 8.10 (s, 1H), 7.78-7.85 (m, 3H), 7.65-7.70 (m, 1H), 6.98 (d, 1H), 3.95 (s, 3H). MS (EI) for C₂₃H₁₄BrClN₄O₄: 525 (MH⁺).

### *(Compounds 396)

### N-[4-(8-bromo-4-oxo-3,4-dihydro[1]benzofuro[3,2-d]pyrimidin-2-yl)-3-chlorophenyl]-6-(1H-pyrazol-1-yl)pyridine-3-carboxamide

N-[4-(8-bromo-4-oxo-3,4-dihydro[1]benzofuro[3,2-d]pyrimidin-2-yl)-3-chlorophenyl]-6-(1H-pyrazol-1-yl)pyridine-3-carboxamide was synthesized in a manner similar to **Example 11 + coupling**, wherein 3-dimethylaminopropionic acid was substituted with 6-(1H-pyrazol-1-yl) nicotinic acid. Purification by preparative HPLC (reverse-phase, acetonitrile/water with 0.1 % formic acid), followed by concentration *in vacuo* and lyophilization afforded the title compound (6.0 mg). NMR (400 MHz, d₆-DMSO): 10.82 (s, 1H), 9.07 (d, 1H), 8.74(d, 1H), 8.54-8.58 (m, 1H), 8.24 (d, 1H), 8.08-8.15 (m, 2H), 7.93 (d, 1H), 7.81-7.89 (m, 3H), 7.71 (d, 1H), 6.65-6.68 (m, 1H). MS (EI) for C₂₅H₁₄BrClN₆O₃: 561 (MH⁺).

### *(Compound 397)

### N-[4-(8-bromo-4-oxo-3,4-dihydro[1]benzofuro[3,2-d]pyrimidin-2-yl)-3-chlorophenyl]quinoxaline-2-carboxamide

N-[4-(8-bromo-4-oxo-3,4-dihydro[1]benzofuro[3,2-d]pyrimidin-2-yl)-3-chlorophenyl]quinoxaline-2-carboxamide was synthesized in a manner similar to **Example 11 + coupling**, wherein 3-dimethylaminopropionic acid was substituted with 2-quinoxaline carboxylic acid. Purification by preparative HPLC (reverse-phase, acetonitrile/water with 0.1 % formic acid), followed by concentration *in vacuo* and lyophilization afforded the title compound (17.0 mg). NMR (400 MHz, d₆-DMSO): 11.28 (s, 1H), 9.59 (s, 1H), 8.31-8.37 (m, 2H), 8.23-8.29 (m, 2H), 8.03-8.14 (m, 3H), 7.82-7.91 (m, 3H), 7.73-7.77 (m, 1H). MS (EI) for C₂₅H₁₃BrClN₅O₃: 546 (MH⁺).

### *(Compound 405)

### N-[4-(8-bromo-4-oxo-3,4-dihydro[1]benzofuro[3,2-d]pyrimidin-2-yl)-3-chlorophenyl]-1,8-naphthyridine-2-carboxamide

N-[4-(8-bromo-4-oxo-3,4-dihydro[1]benzofuro[3,2-d]pyrimidin-2-yl)-3-chlorophenyl]-1,8-naphthyridine-2-carboxamide was synthesized in a manner similar to **Example 11 + coupling**, wherein 3-dimethylaminopropionic acid was substituted with 1,8-naphthyridine-2-carboxylic acid. Purification by preparative HPLC (reverse-phase, acetonitrile/water with 0.1% formic acid), followed by concentration *in vacuo* and lyophilization afforded the title compound (9.7 mg). NMR (400 MHz, d₆-DMSO): 11.25 (s, 1H), 9.27-9.30 (m, 1H), 8.79 (d, 1H), 8.63-8.69 (m, 1H), 8.39 (d, 2H), 8.33 (s, 1H), 8.24 (d, 3H), 8.05-8.12 (m, 1H), 7.77-7.87 (m, 3H), 7.70 (d, 1H), 6.65 (s, 1H). MS (EI) for C₂₅H₁₃BrClN₅O₃: 546 (MH⁺).

### *(Compound 406)

### N-[4-(8-bromo4-oxo-3,4-dihydro)[1]benzofuro[3,2-d]pyrimidin-2-yl)-3-chloropheny]-1,2,3-thiadiazole-4-carboxamide

N-[4-(8-bromo-4-oxo-3,4-dihydro[1]benzofuro[3,2-d]pyrimidin-2-yl)-3-chlorophenyl]-1,2,3-thiadiazole-4-carboxamide was synthesized in a manner similar to **Example 11 + coupling,** wherein 3-dimethylaminopropionic acid was substituted with 1,2,3-thiadiazole-4-carboxylic acid. Purification by preparative HPLC (reverse-phase, acetonitrile/water with 0.1% formic acid), followed by concentration *in vacuo* and lyophilization afforded the title compound (9.7 mg). NMR (400 MHz, d₆-DMSO): 11.29 (s, 1H), 9.84 (s, 1H), 8.13-8.17 (m, 2H), 7.89-7.94 (m, 1H), 7.73-7.80 (m, 2H), 7.63 (d, 1H), 6.57 (s, 1H). MS (EI) for C₁₉H₉BrClN₅O₃S: 546 (MH⁺).

### *(Compound 362)

### N-[4-(8-bromo-4-oxo-3 ,4-dihydro[1]benzofuro[3,2-d]pyrimidin-2-yl)-3-chlorophenyl]-5-methylisoxazole-3-carboxamide

N-[4-(8-bromo-4-oxo-3,4-dihydro[1]benzofuro[3,2-d]pyrimidin-2-yl)-3-chlorophenyl]-5-methylisoxazole-3-carboxamide was synthesized in a manner similar to **Example 11 + coupling,** wherein 3-dimethylaminopropionic acid was substituted with 5-methylisoxazole-3-carboxylic acid. Purification by preparative HPLC (reverse-phase, acetonitrile/water with 0.1% formic acid), followed by concentration *in vacuo* and lyophilization afforded the title compound. NMR (400 MHz, d₆-DMSO): 11.07 (s, 1H), 8.25 (s, 1H), 8.12 (s, 1H), 7.91 (m, 3H), 7.70 (d, 1H), 6.72 (s, 1H), 2.53 (s, 3H). MS (EI) for C₂₁H₁₂BrClN₄O₄: 500 (MH⁺).

### *(Compound 363)

### N-[4-(8-bromo-4-oxo-3,4-dihydro[1]benzofuro[3,2-d]pyrimidin-2-yl)-3-chlorophenyl]isoxazole-3-carboxamide

N-[4-(8-bromo-4-oxo-3,4-dihydro[1]benzofuro[3,2-d]pyrimidin-2-yl)-3-chlorophenyl]isoxazole-3-carboxamide was synthesized in a manner similar to **Example 11 + coupling**, wherein 3-dimethylaminopropionic acid was substituted with isoxazole-3-carboxylic acid. Purification by preparative HPLC (reverse-phase, acetonitrile/water with 0.1% formic acid), followed by concentration *in vacuo* and lyophilization afforded the title compound. NMR (400 MHz, d₆-DMSO): 11.16 (s, 1H), 9.22 (d, 1H), 8.25 (s, 1H), 8.12 (s, 1H), 7.87 (m, 3H), 7.71 (d, 1H), 7.09 (d, 1H). MS (EI) for C₂₀H₁₀BrClN₄O₄: 486 (MH⁺).

### *(Compound 374)

### N-[4-(8-bromo-4-oxo-3,4-dihydro[1] benzofuro[3,2-d]pyrimidin-2-yl)-3-chlorophenyl]-1,3-oxazole-5-carboxamide

N-[4-(8-bromo-4-oxo-3,4-dihydro[1]benzofuro[3,2-d]pyrimidin-2-yl)-3-chlorophenyl]-1,3-oxazole-5-carboxamide was synthesized in a manner similar to **Example 11 + coupling**, wherein 3-dimethylaminopropionic acid was substituted with oxazole-5-carboxylic acid. Purification by preparative HPLC (reverse-phase, acetonitrile/water with 0.1% formic acid), followed by concentration *in vacuo* and lyophilization afforded the title compound. NMR (400 MHz, d₆-DMSO): 13.38 (br s, 1H), 10.83 (s, 1H), 8.73 (s, 1H), 8.25 (d, 1H), 8.08 (m, 2H), 7.90 (m, 3H), 7.72 (d, 1H). MS (EI) for C₂₀H₁₀BrClN₄O₄: 486 (MH⁺).

### *(Compound 395)

### N-[4-(8-bromo-4-oxo-3,4-dihydro[1]benzofuro[3,2-d]pyrimidin-2-yl)-3-chtorophenyl]-1H-pyrrolo[2,3-b]pyridine-5-carboxamide

'N-[4-(8-bromo-4-oxo-3,4-dihydro[1]benzofuro[3,2-d]pyrimidin-2-yl)-3-chlorophenyl]-1H-pyrrolo[2,3-b]pyridine-5-carboxamide was synthesized in a manner similar to **Example 11 + coupling**, wherein 3-dimethylaminopropionic acid was substituted with 1H-pyrrolo[2,3-b]pyridine-6-carboxylic acid. Purification by preparative HPLC (reverse-phase, acetonitrile/water with 0.1% formic acid), followed by concentration *in vacuo* and lyophilization afforded the title compound. C₂₄H₁₃BrClN₅O₃: 534 (MH⁺).

### *(Compound 62)

### N-[4-(8-bromo-4-oxo-3,4-dihydro[1]benzofuro[3,2-d]pyrimidin-2-yl)-3-methylphenyl]-N'2',N'2'-dimethylglycinamide

N-[4-(8-bromo-4-oxo-3,4-dihydro[1]benzofuro[3,2-d]pyrimidin-2-yl)-3-methylphenyl]-N'2',N'2'-dimethylglycinamide was synthesized in a manner similar to **Example 11 + coupling**, wherein 3-dimethylaminopropionic acid was substituted with N'N'-dimethylglycine, and 2-(4-amino-2-chlorophenyl)-8-bromo[1]benzofuro[3,2-d]pyrimidin-4(3H)-one was substituted with 2-(4-amino-2-methylphenyl)8-bromo[1]benzofuro[3,2-d]pyrimidin-4(3H)-one. Purification by preparative HPLC (reverse-phase, acetonitrile/water with 0.1% formic acid), followed by concentration *in vacuo* and lyophilization afforded the title compound. NMR (400 MHz, d₆-DMSO): 8.26 (s, 1H), 8.19 (s, 1H), 7.81 (br s, 2H), 7.65 (br s, 2H), 7.49 (d, 1H), 7.09 (br s, 1H), 2.51 (br s, 11H). MS (EI) for C₂₁H₁₉BrN₄O₃: 457 (MH⁺).

### *(Compound 393)

### '3-amino-N-[4-(8-bromo-4-oxo-3,4-dihydro[1]benzofuro[3,2-d]pyrimidin-2-yl)-3-chlorophenyl]-1H-indazole-5-carboxamide

'3-amino-N-[4-(8-bromo-4-oxo-3,4-dihydro[1]benzofuro[3,2-d]pyrimidin-2-yl)-3-chlorophenyl]-1H-indazole-5-carboxamide was synthesized was synthesized in a manner similar to **Example 11 + coupling** wherein 3-dimethylaminopropionic acid was substituted with 2-fluoro-5-formylbenzonitrile. Subsequently, resulting crude N-(3-(8-bromo-4-oxo-3,4-dihydrobenzofuro[3,2-d]pyrimidin-2-yl)-4-chlorophenyl)-3-cyano-4-fluorobenzamide was dissolved in DMSO (2mL) and hydrazine hydrate (1 mL). The reaction mixture was heated to 110 °C for 16h in a sealed vessel. Upon cooling the mixture was concentrated *in vacuo* and dissolved in methanol. Formation of product was confirmed by LC/MS and the product was purified by preparatory HPLC (reverse-phase, acetonitrile/water with 0.1% formic acid) to yeild the title compound. ¹H-NMR (400MHz, d₆-DMSO): 11.78 (br s, 1H), 10.54 (s, 1H), 8.46 (s, 1H), 8.24 (s, 1H). 8.14 (s, 1H), 7.83 (m, 4H), 7.67 (d, 1H). 7.31 (d, 1H), 5.64 (s, 2H). MS (EI) for C₂₄H₁₄BrClN₆O₃ 549 (MH⁺).

### EXAMPLE 12

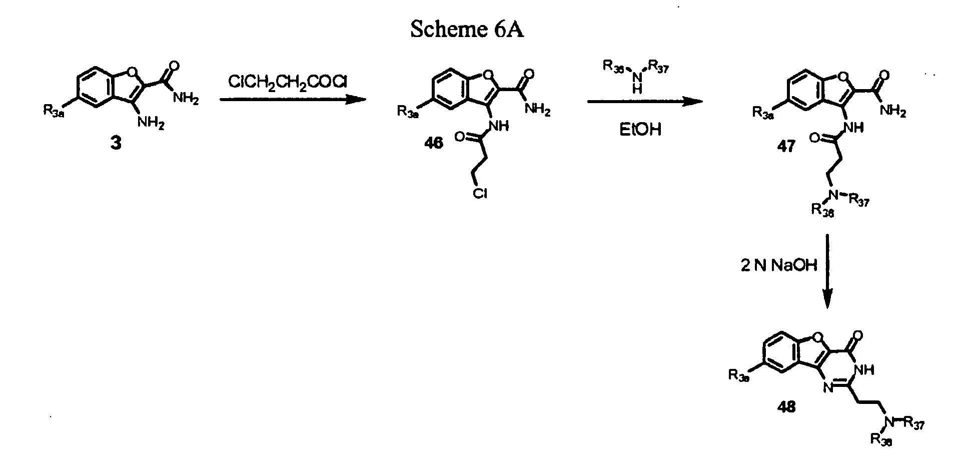

wherein R₃ₐ is as defined in the disclosure above and R₃₆ and R₃₇ are described within the compounds within this example.

### 5-Bromo-3-(3-chloropropanamido)benzofuran-2-carboxamide 46

The title compound was synthesized in a manner similar to **Example 1**, wherein chloroacetyl chloride was substituted with chloropropanoyl chloride. After purification by flash chromatography (50:45:5 hexane/ethyl acetate/methanol), the title compound was obtained as an oil (459 mg), quantitative yield.
¹H NMR (400 MHz, d₆-DMSO) δ 10.26 (br s, 1H), 8.14 (br s, 1H), 8.06 (d, *J*= 2.5 Hz, 1 H), 7.92 (br s, 1H), 7.64 (dd, *J*= 2.5, *J=* 9 Hz, 1H), 7.58 (d, *J* 9 Hz, 1 H), 3.92 (t, *J=* 6 Hz, 1H), 3.76 (t, *J*= 6 Hz, 1H), 2.98 (t, *J*= 6 Hz, 1 H), 2.72 (t, *J*= 6 Hz, 1 H); MS (ESI+) for C₁₂H₁₀BrClN₂O₃: 346(MH⁺).

### (Compound 105)

### 2-(2-Chloro-6-fluorophenyl)-8-cyclopropyl[1]benzofuro[3,2-d]pyrimidin-4(3H)-one

To a solution of 5-bromo-3-(3-chloropropanamido)benzofuran-2-carboxamide (**46**, 106 mg, 0.307 mmol) in 10 mL anhydrous ethanol was added pyrrolidine (80 µL, 0.95 mmol). The reaction mixture was heated to 80 °C for 17 h, then concentrated to dryness under reduced pressure. The residual crude material was diluted with absolute ethanol (10 mL); 2 N NaOH (3 ml) was added, and the mixture was heated at 40 °C for 1 h. After purification by flash chromatography (90:8.5:1.5 dichloromethane/methanol/28% (w/w) ammonium hydroxide), the title compound was obtained as a solid (89 mg), 79% yield (over two steps).
¹H NMR (400 MHz, CDCl₃) δ 12.11 (br s, 1H), 8.12 (d, *J*= 2.5 Hz, 1H), 7.62 (dd, *J*= 2.5, *J*= 9 Hz, 1H), 7.48 (d, *J*= 9 Hz, 1H), 3.06 (m, 4H), 2.82 (m, 4H), 1.96 (m, 4H); ¹³C NMR (100 MHz, CDCl₃) δ 159.1, 155.5, 154.2, 143.3, 139.4, 132.5, 124.7, 124.4, 117.1, 114.6, 53.6, 52.8, 31.7, 23.8; MS (ESI+) for C₁₆H₁₆BrN₃O₂: 364 (MH⁺).

### (Compound 164)

### 8-bromo-2-{[(3R)-3-hydroxypyrrolidin-1-yl]methyl}[1]benzofuro[3,2-d]pyrimidin-4(3H)-one

8-bromo-2-{[(3R)-3-hydroxypyrrolidin-1-yl]methyl}[1]benzofuro[3,2-d]pyrimidin-4(3H)-one was synthesized in a manner similar to **Example 12**, wherein chloropropanoyl chloride was substituted with chloroacetyl chloride and pyrrolidine was substituted with R-(+)-3-pyrrolidinol. Purification by flash chromatography afforded the title compound (53 mg, 46%). ¹H NMR (400 MHz, d₆-DMSO + D₂O): 8.22 (s, 1H), 7.81 (s, 2H), 4.21 (m, 1H), 3.76(m, 2H), 2.86 (m, 2H), 2.61 (m, 2H), 2.08 (m, 1H), 1.65 (m, 1H); MS (EI) for C₁₅H₁₄BrN₃O₃: 421:423(Bromine isoptope MH⁺).

### (Compound 176)

### 8-bromo-2-[(2-phenyl-1H-imidazol-1-yl)methyl][1]benzofuro[3,2-d]pyrimidin-4(3H)-one

8-bromo-2-[(2-phenyl-1H-imidazol-1-yl)methyl][1]benzofuro[3,2-d]pyrimidin-4(3H)-one was synthesized in a manner similar to **Example 12**, wherein chloropropanoyl chloride was substituted with chloroacetyl chloride and pyrrolidine was substituted with 2-Phenylimidazole. Purification by preparative HPLC, followed by concentration *in vacuo* and lyophilization afforded the title compound (21mg, 8%). ¹H NMR (400 MHz, d₆-DMSO): 8.02 (s, 1H), 7.82 (m, 2H), 7.65 (m, 2H), 7.44 (m, 4H), 7.07 (s, 1H), 5.29 (s, 2H); MS (EI) for C₂₀H₁₃BrN₄O₂: 421:423(Bromine isoptope MH⁺).

### (Compound 378)

### 8-bromo-2-(((1R,4R)-5-methyl-2,5-diazabicyclo[2.2.1]heptan-2-yl)methyl)benzofuro[3,2-d]pyrimidin-4(3H)-one

8-bromo-2-(((1R,4R)-5-methyl-2,5-diazabicyclo[2.2.1]heptan-2-yl)methyl)benzofuro[3,2-d]pyrimidin-4(3H)-one was synthesized in a manner similar to **Example 12,** wherein chloropropanoyl chloride was substituted with chloroacetyl chloride and pyrrolidine was substituted with (1R,4R)-2-methyl-2,5-diazabicyclo[2.2.1]heptane and triethyl amine. (1*R*,4*R*)-2-methyl-2,5-diazabicyclo[2.2.1]heptane was synthesized according to a procedure published in J. Org. Chem, 1990, vol. 55, 1684-1687. To a solution of the resulting 5-bromo-3-(2-((1R,4R)-5-methyl-2,5-diazabicyclo[2.2.1]heptan-2-yl)acetamido)benzofuran-2-carboxamide (140 mg, 0.34 mmol) in ethanol (5 mL) was added an aqueous solution of 2N NaOH (5 mL) and the resulting mixture was heated to 50 °C for 4 hours. The reaction mixture was concentrated *in vacuo* and brought to pH 9 and extracted with chloroform (3 x 50 mL). The combined organic phases were washed with brine, dried over Na₂SO₄ and concentrated *in vacuo.* Purification by preparative HPLC afforded the title compound (38.4 mg, 29%). ¹H NMR (400 MHz, d₆-DMSO): 8.18 (m, 1H), 7.80 (m, 2H), 3.72 (m, 2H), 3.40 (m, 1H), 3.21 (m, 1H), 2.75 (m, 3H), 2.56 (m, 1H), 2.30 (s, 3H), 1.67 (m, 2H). MS (EI) for C₁₇H₁₇BrN₄O₂: 388.9 (MH⁺).

### *(Compound 159)

### 8-bromo-2-(piperazin-1-ylmethyl)[1]benzofuro[3,2-d]pyrimidin-4(3H)-one (HCl salt)

8-bromo-2(piperazin-1-ylmethyl)[1]benzofuro[3,2-d]pyrimidin-4(3H)-one was synthesized in a manner similar to **Example 12**, wherein chloropropanoyl chloride was substituted with chloroacetyl chloride and pyrrolidine was substituted with N-Boc-piperazine. The pure material was given by deprotection with 4N-HCl in dioxane. ¹H NMR (400 MHz, CD₃OD): 8.25 (q, 1H), 7.80 (dd, H), 7.70 (dd, 1H), 3.91 (s, 2 H), 3.37 (br, 4 H), 3.25 (br, 4H); MS (EI) for C₁₅H₁₅BrN₄O₂: 363 (MH⁺).

### (Compound 183)

### 8-bromo-2-[(3-hydroxyazetidin-1-yl)methyl][1]benzofuro[3,2-d]pyrimidin-4(3H)-one

8-bromo-2-[(3-hydroxyazetidin-1-yl)methyl][1]benzofuro[3,2-d]pyrimidin-4(3H)-one was synthesized in a manner similar to **Example 12**, wherein chloropropanoyl chloride was substituted with chloroacetyl chloride and pyrrolidine was substituted with azetidin-3-ol. The crude product was purified by SiO₂ flash chromatography to afford the desired product. ¹H NMR (400 MHz, CD₃OD): 8.2 (q, 1H), 7.75 (dd, 1H), 7.63 (dd, 1H), 4.55 (q, 1H), 4.15 (m, 2H), 4.03 (s, 2H), 3.55 (m, 2 H); MS (EI) for C₁₄H₁₂BrN₃O₃: 350 (MH⁺).

### (Compound 187)

### 2-[(4-acetylpiperazin-1-yl)methyl]-8-bromo[1]benzofuro[3,2-d]pyrimidin-4(3H)-one

2-[(4-acetylpiperazin-1-yl)methyl]-8-bromo[1]benzofuro[3,2-d]pyrimidin-4(3H)-one was synthesized in a manner similar to **Example 12**, wherein chloropropanoyl chloride was substituted with chloroacetyl chloride and pyrrolidine was substituted with N-Boc-piperazine. The pure material was given by deprotection with 4N-HCl in dioxane and followed by acetylation with acetic anhydride in DIEA. ¹H NMR (400 MHz, CD₃OD): 8.23 (s, 1H), 7.78 (d, 1H), 7.68 (d, 1H), 3.66 (m, 4H), 3.60 (m, 2H), 2.59(m, 4H), 2.10 (s, 3H); MS (EI) for C₁₇H₁₇BrN4O₃: 405 (MH⁺).

### (Compound 306)

### 8-bromo-2-{[3-(hydroxymethyl)piperidin-1-yl]methyl} [1]benzofuro[3,2-d]pyrimidin-4(3H)-one

8-bromo-2-{[3-(hydroxymethyl)piperidin-1-yl]methyl}[1]benzofuro[3,2-d]pyrimidin-4(3H)-one was synthesized in a manner similar to **Example 12,** wherein chloropropanoyl chloride was substituted with chloroacetyl chloride and pyrrolidine was substituted with piperidin-4-ylmethanol. Purification by preparative HPLC (reverse-phase, acetonitrile/water with 0.01% ammonium acetate), followed by concentration *in vacuo* and lyophilization afforded the title compound as a solid (48% yield). ¹H NMR (400 MHz, CD₃OD): 8.21 (s, 1H), 7.77 (d, 1H), 7.66 (d, 1H), 3.77 (d, 2H), 3.49 (m, 1H), 3.40 (m, 1H), 3.14 (d, 1H), 3.03 (d, 1H), 2.41 (t, 1H), 2.20 (t, 1H), 1.92 (m, 1H), 1.76 (m, 3H), 1.1 (m, 1H); MS (EI) for C₁₇H₁₈BrN₃O₃: 392 (MH⁺).

### (Compound 367)

### 8-Fluoro-2-[(4-methylpiperazin-1-yl)methyl][1]benzofuro[3,2-d]pyrimidin-4(3H)-one

The title compound was synthesized in a manner similar to **Example 12,** wherein 3-amino-5-bromobenzofuran-2-carboxamide was substituted with 3-amino-5-fluorobenzofuran-2-carboxamide, which in turn was synthesized in a manner similar to **Example 1**, wherein 5-bromo-2-hydroxybenzonitrile was substituted with 5-fluoro-2-hydroxybenzonitrile. After purification by flash chromatography (91:8:1 dichloromethane/methanol/28% (w/w) ammonium hydroxide), the title compound was obtained as a solid (89 mg), 17% yield.
¹H NMR (400 MHz, 1:1 CD₃OD/CDCl₃) δ 7.75 (dd, 1H), 7.66 (dd, 1H), 7.39 (dt, 1H), 4.59 (s, 2H), 3.71 (s, 3H), 2.72 (m, 4H), 2.39 (s, 4H); MS (ESI+) for C₁₆H₁₇FN₄O₂: 317 (MH⁺).

### (Compound 230)

### 8-Iodo-2-[(4-methylpiperazin-1-yl)methyl][1]benzofuro[3,2-d]pyrimidin-4(3,H)-one

The title compound was synthesized in a manner similar to **Example 12**, wherein 3-(2-chloropropanamido)-5-bromobenzofuran-2-carboxamide was substituted with 3-(2-chloroacetamido)-5-iodobenzofuran-2-carboxamide (5). After purification by flash chromatography (91:8:1 dichloromethane/methanol/28% (w/w) ammonium hydroxide), the title compound was obtained as a solid (200 mg), 69% yield.
¹H NMR (400 MHz, CD₃OD) δ 8.42 (d, 1H), 7.95 (dd, 1H), 7.57 (dd, 1H), 3.76 (s, 2H), 3.51 (br, 3H), 3.20 (m, 5H), 2.92 (s, 3H); MS (ESI+) for C₁₆H₁₆BrN₃O₂: 425 (MH⁺).

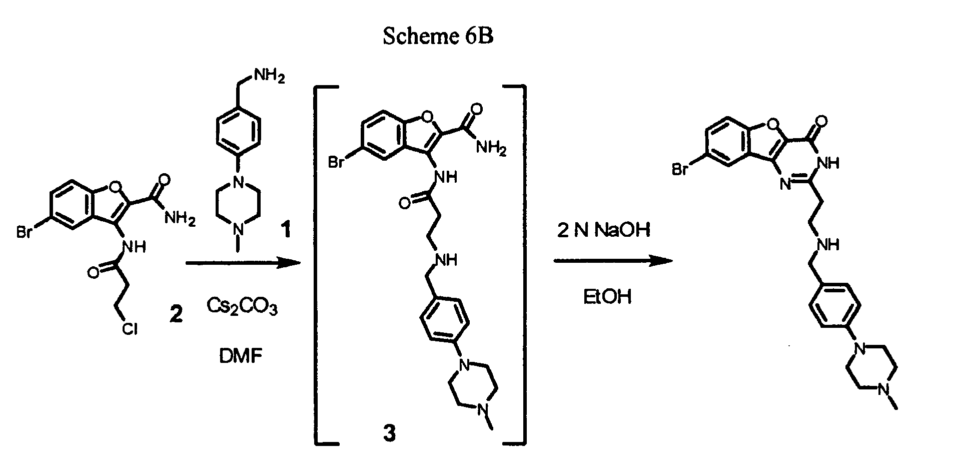

### *(Compound 129)

### 8-Bromo-2-[2-({[4-(4-methylpiperazin-1-yl)phenyl]methyl}amino)ethyl][1]benzofuro[3,2-d]pyrimidin-4(3H)-one

Intermediate 5-bromo-3-[3-[4-(4-methylpiperazin-1-yl)benzylamino]propanamido]benzofuran-2-carboxamide (**3**) was synthesized in a manner similar to **Example 2**, wherein 8-bromo-2-(chloromethyl)benzofuro[3,2-*d*]pyrimidin-4(3*H*)-one was substituted with 5-bromo-3-(3-chloropropanamido)benzofuran-2-carboxamide (**46**, prepared as described in **Example 12**). Compound **3** was not isolated pure, but directly treated with 2 N NaOH in ethanol, in a similar manner as described in **Example 12**. After purification by flash chromatography (88:10:2 dichloromethane/methanol/28% (w/w) ammonium hydroxide), the title compound was obtained as an impure oil (34 mg), 44% yield over two steps. Purification by preparative HPLC (reverse-phase, 0.1% TFA in acetonitrile/0.05% TFA in water), followed by concentration *in vacuo* and lyophilization afforded the title compound (22 mg, 28%) as a white solid.
¹H NMR (400 MHz, CD₃OD) δ 8.20 (d, *J*= 2.5 Hz, 1H), 7.80 (dd, *J*= 2.5, *J=* 9 Hz, 1H), 7.69 (d, *J*= 9 Hz, 1H), 7.51 (d, *J*= 8 Hz, 1H), 7.14 (d, *J*= 8 Hz, 1H), 4.31 (s, 2H), 3.95 (m, 2H), 3.63 (m, 2H), 3.59 (t, *J*= 6 Hz, 2H), 3.26 (m, 2H), 3.22 (t, *J*= 6 Hz, 2H), 3.08 (m, 2H), 2.97 (s, 3H); MS (ESI+) for C₂₄H₂₆BrN₅O₂: 497 (MH⁺).

### EXAMPLE 13:

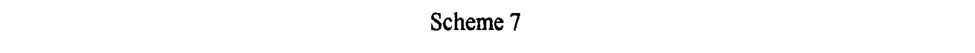

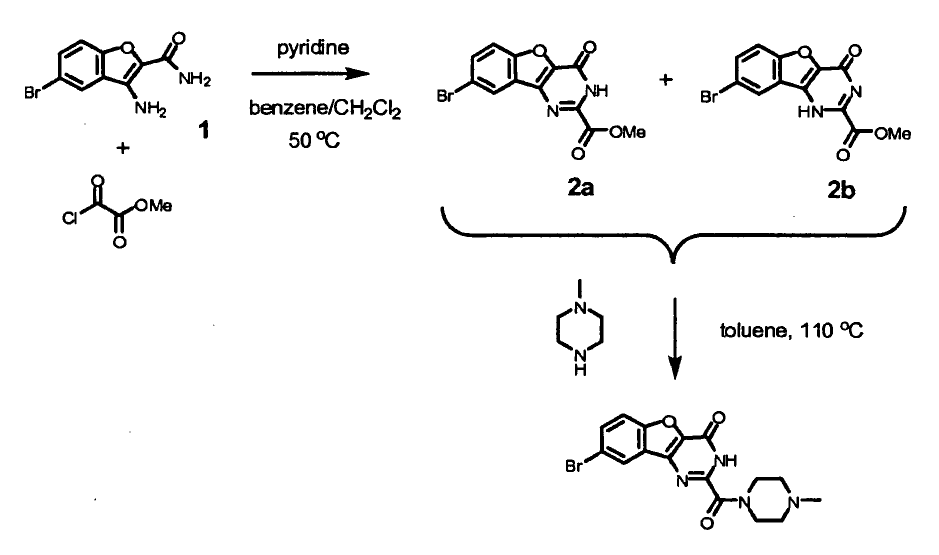

### *(Compound 106)

### 8-Bromo-2-[(4-methylpiperazin-1-yl)carbonyl][1]benzofuro[3,2-d]pyrimidin-4(3H)-one

To a solution of 3-amino-5-bromobenzofuran-2-carboxamide (213 mg, 0.835 mmol) in benzene (3 mL) and pyridine (0.2 mL), methyl oxalyl chloride (315 mg, 2.59 mg) was added at room temperature. An equal volume of CH₂Cl₂ (about 3 mL) was added. The mixture was heated to 50°C in sealed tube for 3 h, then filtered through Celite^{®} and concentrated to dryness under reduced pressure. After purification by flash chromatography (90:9:1 dichloromethane/methanol/28% (w/w) ammonium hydroxide), intermediate compound **2a** (methyl 8-bromo-4-oxo-3,4-dihydrobenzofuro[3,2*-d*]pyrimidine-2-carboxylate) and tautomer **2b** (methyl 8-bromo-4-oxo-1,4-dihydrobenzofuro[3,2*-d*]pyrimidine-2-carboxylate) were obtained as inseparable mixture (195 mg, 0.60 mmol; 72% yield), which could only be partially characterized analytically [MS (ESI+) for C₁₂H₇BrN₂O₄: 324 (MH⁺)]. To a solution of the obtained mixture of compounds **2a** and **2b** in anhydrous toluene (10 mL) was added *N-*methylpiperazine (123 mg, 1.23 mmol). The reaction mixture was heated to 110 °C for 2 hours, cooled down and concentrated *in vacuo.*
After purification by flash chromatography (88:10:2 dichloromethane/methanol/28% (w/w) ammonium hydroxide), the title compound was obtained as a solid (126 mg), 54% yield.
¹H NMR (400 MHz, CDCl₃) δ 10.13 (br s, 1H), 7.95 (d, *J*= 1.7 Hz, 1H), 7.62 (dd, *J*= 2, *J*= 9 Hz, 1H), 7.41 (d, *J*= 9 Hz, 1H), 4.22 (m, 2H), 3.75 (m, 2H), 2.52 (m, 4H), 2.34 (s, 3H); ¹³C NMR (100 MHz, CDCl₃) δ 158.9, 153.2, 132.5, 127.2, 124.3, 123.2, 120.6, 117.6, 114.1, 111.1, 55.5, 54.8, 46.8, 46.1, 44.1; MS (ESI+) for C₁₆H₁₅BrN₄O₃: 392 (MH⁺).

### EXAMPLE 14:

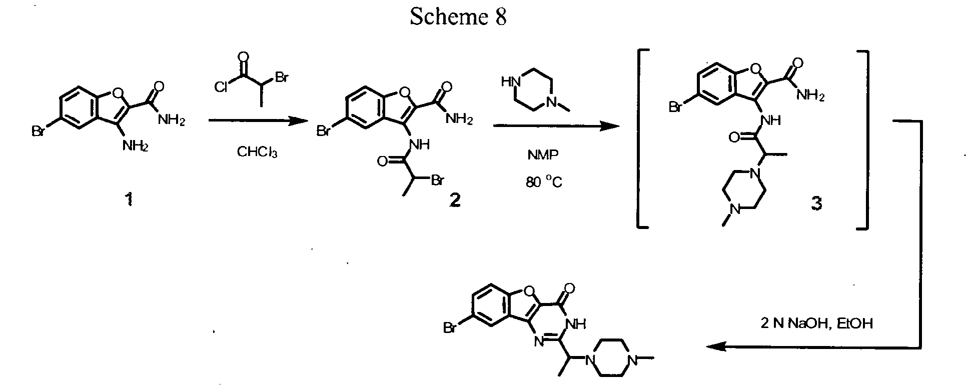

### 5-Bromo-3-(2-bromopropanamido)benzofuran-2-carboxamide (2)

The title compound was synthesized in a manner similar to **Example 1**, wherein chloroacetyl chloride was substituted with 2-bromopropanoyl chloride. After purification by flash chromatography (91:8:1 dichloromethane/methane/28% (w/w) ammonium hydroxide), the title compound was obtained as an oil (179 mg), quantitative yield.
¹H NMR (400 MHz, d₆-DMSO) δ 8.24 (br s, 1 H), 8.10 (m, 1H), 7.94 (br s, 1H), 7.65 (dd, *J*= 2.5, *J=* 9 Hz, 1H), 7.59 (d, *J*= 9 Hz, 1H), 5.00 (q, *J*= 6.5 Hz, 1H), 1.82 (d, *J*= 6.5 Hz, 3H); MS (ESI+) for C₁₂H₁₀Br₂N₂O₃: 391 (MH⁺).

### (Compound 117)

### 8-Bromo-2-[1-(4-methylpiperazin-1-yl)ethyl][1]benzofuro[3 2-d]pyrimidin-4(3H)-one

The title compound was synthesized in a manner similar to **Example 12** wherein pyrrolidine was substituted with *N-*methylpiperazine and ethanol with *N-*methylpyrrolidinone (NMP). The reaction in the second step was run at 80 °C for 18 h. After purification by flash chromatography (95:4.5:0.5 to 90:9:1 dichloromethane/methanol/28% (w/w) ammonium hydroxide), the title compound was obtained as a solid (41 mg), 51 % (over two steps).
¹H NMR (400 MHz, CDCl₃, spiked with CD₃OD) δ 8.24 (d, *J*= 2.5 Hz, 1H), 7.71 (dd, *J=* 2.5, *J*= 9 Hz, 1H), 7.56 (d, *J*= 9 Hz, 1H), 3.72 (q, *J*= 7 Hz, 1H), 3.59 (br s, 2H), 2.71 (m, 2H), 2.59 (m, 4 H), 2.34 (s, 3H), 1.50 (d, *J*= 7 Hz, 3H); ¹³C NMR (100 MHz, CDCl₃ spiked with CD₃OD) δ 159.8, 156.0, 153.4, 144.0, 133.0, 124.7, 124.4, 117.5, 114.7, 110.2, 62.9, 55.1, 45.8, 22.3, 14.3, MS (ESI+) for C₁₇H₁₉BrN₄O₂: 392 (MH⁺).

### EXAMPLE 15:

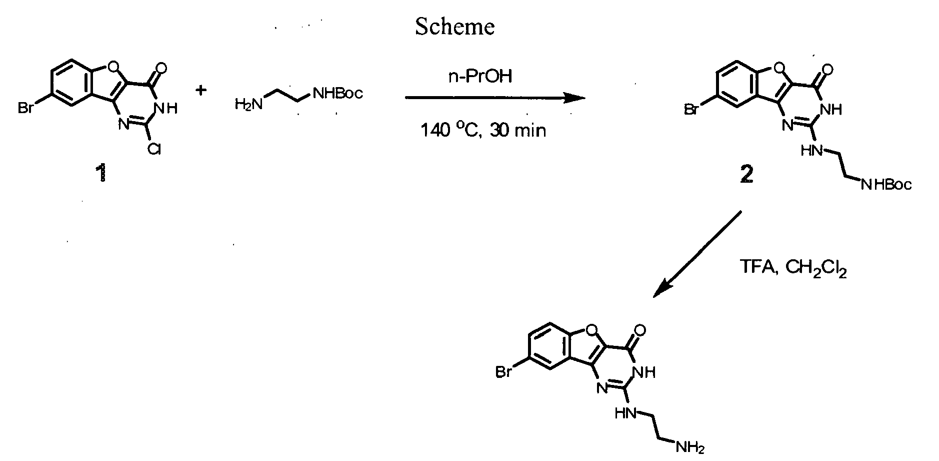

*tert*-Butyl 2-(8-bromo-4-oxo-3,4-dihydrobenzofuro[3,2*-d*]pyrimidin-2-ylamino)ethylcarbamate (**2**)

### (Compound 309)

### 8-Bromo-2-[(2-hydroxyethyl)[amino][1]benzofuro[3,2-d]pyrimidin-4(3H)-one

The title compound was synthesized in a manner similar to **Example 15** wherein *N*-methylpiperazine was substituted with ethanolamine, and ethanol with n-propanol. Purification by preparative HPLC (reverse-phase, 0.1 % TFA in acetonitrile/0.05% TFA in water), followed by concentration *in vacuo* and lyophilization afforded the title compound (5 mg, 9%) as a white solid.
FT-IR (solid): 3270, 30.90, 2950, 1699, 1669, 1602, 1309, 1205, 1120, 1064 cm⁻¹; ¹H NMR (400 MHz, CD₃OD) δ 8.01 (d, 1H), 7.72 (d, 1H), 7.71 (d, 1H), 6.57 (m, 1H), 5.15 (br t, 1H), 3.57 (dd, 2H), 2.85 (t, 2H); MS (ESI+) for C₁₂H₁₀BrN₃O₃: 325 (MH⁺).

### EXAMPLE 16:

### (1S,4S)-tert-Butyl 5-[(8-bromo-4-oxo-3,4-dihydrobenzofuro[3,2-d]pyrimidin-2-yl)methyl]-2,5-diazabicyclo[2,2,1]heptane-2-carboxylate

In a manner similar to **Example 1**, a solution of 8-bromo-2-(chloromethyl)benzofuro[3,2*-d*]pyrimidin-4(3*H*)-one (161 mg, 0.513 mmol), (1*S*,4*S*)-*tert*-butyl 2,5-diazabicyclo[2.2.1]heptane-2-carboxylate (132 mg, 0.666 mmol) and diisopropylethylamine (150 µL, 0.84 mmol) in absolute ethanol (10 mL) was heated to 80°C for 24 h. After cooling to room temperature, the reaction mixture was diluted with ethyl acetate and washed with aq. NaHCO₃. The aqueous layer was further extracted with ethyl acetate and dichloromethane. The combined organic phases were washed with brine and dried over MgSO₄. After purification by flash chromatography (91:8:1 dichloromethane/methanol/28% (w/w) ammonium hydroxide), the title compound was obtained as an oil (162 mg), 58% yield.
¹H NMR (400 MHz, 1:1 CDCl₃/CD₃OD) δ 8.21 (d, *J*= 2.2 Hz, 1H), 7.75 (dd, *J*= 2.5, *J*= 9 Hz, 1H), 7.62 (d, *J*= 9 Hz, 1H), 4.40 (d, *J*= 11 Hz, 1H), 3.67 (d, *J*= 10 Hz, 1H), 3.59 (d, *J*= 11 Hz, 1H), 3.28 (t, *J*= 9 Hz, 1H), 3.06 (t, *J*= 9 Hz, 1H), 2.81 (dd, *J*= 10, *J*= 34 Hz, 1H), 2.01 (d, *J*= 9 Hz, 1H), 1.85 (t, *J*= 11 Hz, 1H), 1.49 (s, 9H); MS (ESI+) for C₂₁H₂₃BrN₄O₄: 476 (MH⁺).

### * (Compound 137)

### 8-Bromo-2-[(1S,4S)-2,5-diazabicyclo[2.2.1]hept-2-ylmethyl][1]benzofuro[3,2-d]pyrimidin-4(3H)-one

(1*S*,4*S*)-*tert*-Butyl-5-((8-bromo-4-oxo-3,4-dihydrobenzofuro[3,2-*d*]pyrimidin-2-yl)methyl)-2,5-diazabicyclo[2.2.1]heptane-2-carboxylate (157 mg, 0.33 mmol) was dissolved in CH₂Cl₂ (8 mL) and treated with TFA (1.5 mL) and triethylsilane (1 mL) at room temp for 4 h. After concentration under reduced pressure and azeotropic distillation with toluene, the residual crude mixture was purified by preparative HPLC (reverse-phase, 0.1% TFA in acetonitrile/0.05% TFA in water), followed by concentration *in vacuo* and lyophilization to afford the TFA salt of the title compound (149 mg) as a white solid, 92% yield.
¹H NMR (400 MHz, CD₃OD) δ 8.08 (d, *J*= 2.2 Hz, 1H), 7.68 (dd, *J*= 2.5, *J*= 9 Hz, 1H), 7.54 (d, *J*= 9 Hz, 1H), 4.63 (d, *J*= 20 Hz, 2H), 4.56 (d, *J*= 20 Hz, 2H), 3.95 (d, *J*= 13 Hz, 1H), 3.87 (d, *J*= 13 Hz, 1H), 3.75 (m, 1H), 3.60 (m, 1H), 2.61 (d, *J*= 12 Hz, 1H), 2.28 (d, *J*= 12 Hz, 1H); ¹³C NMR (100 MHz, CD₃OD) δ 166.2, 155.7, 153.6, 142.5, 139.2, 133.0, 124.0, 123.8, 117.3, 114.4, 63.3, 57.5, 57.0, 54.6, 33.7; MS (ESI+) for C₁₆H₁₅BrN₄O₂: 376 (MH⁺).

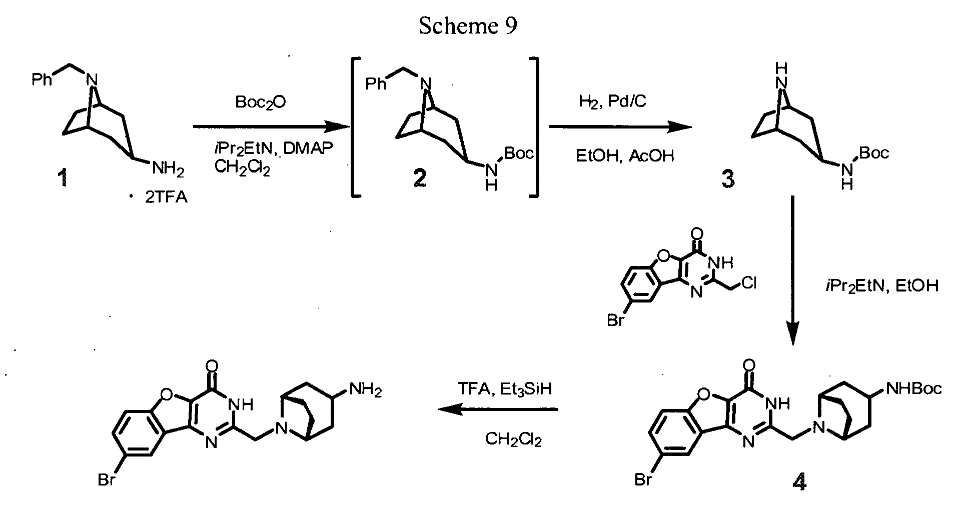

### tert-Butyl 8-azabicyclo[3.2.1]octan-3-ylcarbamate (3)

A 50 mL round bottom flask was charged with 8-benzyl-8-azabicyclo[3.2.1]octan-3-amine (202 mg, 0.61 mmol), DMAP (7 mg, 0.06 mmol), di-*tert*-butyldicarbonate (147 mg, 0.67 mmol), dichloromethane (10 mL) and DIEA (0.22 mL, 1.3 mmol). After stirring for 4 h at room temperature, the reaction mixture was concentrated to dryness under reduced pressure, without isolation of intermediate *tert*-butyl 8-benzyl-8-azabicyclo[3.2.1]octan-3-ylcarbamate **(2).** The obtained crude solid material was transferred in a Parr vessel and diluted with ethanol (10 mL) and acetic acid (1.5 mL). 10% (w/w) Pd/C was added (300 mg). The hydrogenation was carried out at 40 psi. The reaction was completed after 6 h (TLC). The mixture was filtered through Celite^{®}. The obtained clear solution was concentrated in vacuo and azeotroped with toluene. After purification by flash chromatography (88:10:2 dichloromethane/methanol/28% (w/w) ammonium hydroxide), the title compound was obtained as an oil (106 mg), 77% yield over two steps.
¹H NMR (400 MHz, CDCl₃) δ 3.82 (br s, 1H), 3.52 (br s, 2H), 2.09 (br s, 2H), 2.05 (m, 2H), 1.89 (m, 3H), 1.70 (d, *J*= 14 Hz, 2H), 1.44 (s, 9H); ¹³C NMR (100 MHz, CDCl₃) δ 155.5, 53.6,43.2, 37.9, 29.2, 28.6; MS (ESI+) for C₁₂H₂₂N₂O₂: 227 (MH⁺).

### tert-Butyl 8-[(8-bromo-4-oxo-3,4-dihydrobenzofuro[3,2-d]pyrimidin-2-yl)methyl]-8-azabicyclo[3.2.1]octan-3-ylcarbamate (4)

The title compound was synthesized in a manner similar to **Example 16** wherein (1*S*,4*S*)-*tert*-butyl 2,5-diazabicyclo[2.2.1]heptane-2-carboxylate was substituted with *tert*-butyl 8-azabicyclo[3.2.1]octan-3-ylcarbamate (3). After purification by flash chromatography (91:8:1 dichloromethane/methanol/28% (w/w) ammonium hydroxide), the title compound was obtained as an oil (76 mg), 71 % yield. ¹H NMR (400 MHz, CDCl₃) δ 8.13 (d, *J*= 2.2 Hz, 1H), 7.73 (dd, *J*= 2.5, *J*= 9 Hz, 1H), 7.59 (d,*J*= 9 Hz, 1H), 4.50 (br s, 2H), 4.42 (br s, 2H), 3.82 (t,*J*= 8 Hz, 1H), 3.30 (m, 1H), 2.97 (m, 2H), 2.61 (m, 2H), 2.26-2.34 (m, 4H), 1.79 (d, *J*= 14 Hz, 2H), 1.45 (s, 9H); ¹³C NMR (100 MHz, CDCl₃) δ 156.6, 156.0, 155.6, 152.8, 143.9, 139.4, 132.9, 124.5, 117.4, 114.8, 59.9, 55.7, 53.7, 42.2, 37.2, 20.7, 26.5; MS (ESI+) for C₂₃H₂₇BrN₄O₄: 504 (MH⁺).

### (Compound 131)

### 2- {[(1R,5S)-3-Amino-8-azabicyclo[3.2.1]oct-8-yl]methyl}-8-bromo[1]benzofuro[3,2-d]pyrimidin-4(3H)-one

The title compound was synthesized in a manner similar to **Example 16** and obtained as a solid (TFA salt, 75 mg), 96% yield.
¹H NMR (400 MHz, CD₃OD) δ 8.15 (d, *J*= 2.2 Hz, 1H), 7.68 (dd, *J*= 2.5, *J*= 9 Hz, 1H), 7.54 (d, *J*= 9 Hz, 1H), 4.87 (br d, *J*= 6 Hz, 1H), 3.86 (br s, 1H), 3.66 (s, 2H), 3.22 (br s, 2H), 2.26 (m, 2H), 2.14 (m, 2H), 1.97 (m, 3H), 1.79 (d, *J*= 14 Hz, 2H), 1.45 (s, 9H); MS (ESI+) for C₁₈H₁₉BrN₄O₂: 404 (MH⁺).

### (Compound 132)

### 2-{[(1R,5S)-3-amino-8-azabicyclo[3.2.1]oct-8-yl]methyl}-8-bromo[1]benzofuro[3,2-d]pyrimidin-4(3H)-one

The title compound was synthesized in a manner similar to **Example 16**, wherein (1*S*,4*S*)-*tert*-butyl 2,5-diazabicyclo[2.2.1]heptane-2-carboxylate was substituted with (*S*)-octahydropyrrolo[1,2-*a*]pyrazine. After purification by flash chromatography (90:9:1 dichloromethane/methanol/28% (w/w) ammonium hydroxide), the title compound was obtained as an oil (54 mg), 42% yield.
¹H NMR (400 MHz, CDCl₃) δ 8.17 (d, *J*= 2.5 Hz, 1H), 7.68 (dd, *J*= 2.5, *J*= 9 Hz, 1H), 7.54 (d, *J*= 9 Hz, 1H), 3.73 (d, *J*= 4 Hz, 2H), 3.09 (dd,*J*= 10, *J*= 18 Hz, 2H), 2.98 (d, *J*= 10 Hz, 1H), 2.83 (d,*J*= 10 Hz, 1H), 2.59 (m, 1H), 2.36 (m, 1H), 2.11-2.27 (m, 3H), 1.71-1.91 (m, 3H), 1.41 (m, 1H), 1.13 (m, 1H); ¹³C NMR (100 MHz, CDCl₃) δ 156.0, 155.9, 153.0, 144.0, 139.4, 133.0, 124.6, 124.5, 117.5, 114.7, 62.8, 60.0, 58.5, 53.5, 53.0, 51.6, 27.7, 21.5; MS (ESI+) for C₁₈H₁₉BrN₄O₂: 404 (MH⁺).

### EXAMPLE 17:

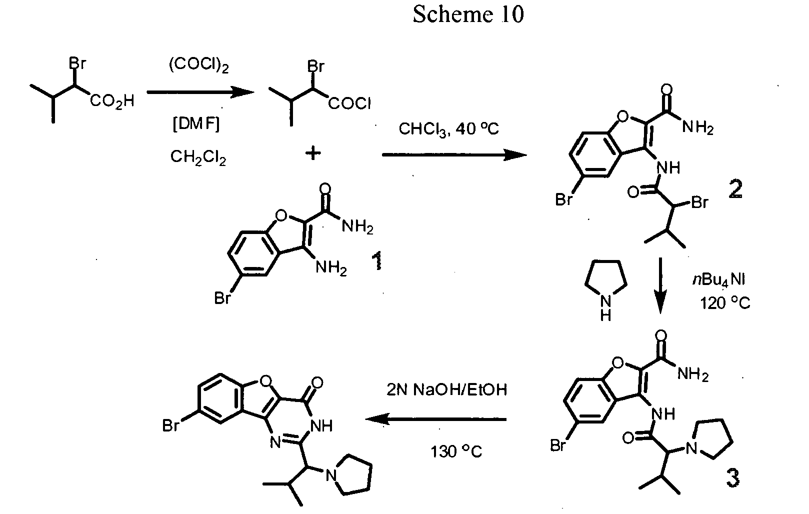

### 5-Bromo-3-(2-bromo-3-methylbutanamido)benzofuran-2-carboxamide (2)

To a solution of 2-bromo-3-methyl butanoic acid (1.07 g, 5.91 mmol) in anhydrous CH₂Cl₂ (10 mL), a droplet of DMF and oxalyl chloride (1.5 mL, 17 mmol) were added at room temperature under nitrogen atmosphere. After stirring for 16 h, the reaction mixture was concentrated under reduced pressure and azeotroped with toluene. To the obtained residue, 3-amino-5-bromobenzofuran-2-carboxamide (1, 186 mg, 0.73 mmol) and chloroform were added. The mixture was heated to 40°C for 3 h. The mixture was poured over aqueous saturated NaHCO₃ (120 mL) and the organic phase was separated. The aqueous layer was further extracted with CHCl₃ (2x 80 mL) . The combined organic layers were dried over MgSO₄, then concentrated in vacuo. After purification by flash chromatography (91:8:1 dichloromethane/methanol/28% (w/w) ammonium hydroxide), the title compound was obtained as an oil (198 mg), 65% yield.
¹H NMR (400 MHz, CDCl₃ δ 10.64 (br s, 1H), 8.66 (d, *J*= 3 Hz, 1H), 7.56 (dd, *J*= 3, *J*=9Hz, 1 H), 7.31 (d, *J*= 9 Hz, 1H), 6.41 (br s, 1H), 5.80 (br s, 1H), 4.37 (d, *J*= 6.5 Hz, 1H), 2.46 (sept, *J*= 6.5 Hz, 1H), 1.14 (dd, *J*= 3, *J*= 6.5 Hz, 1H); ¹³C NMR (100 MHz, CDCl₃) δ 167.3, 162.6,152.4, 133.0, 131.8, 129.7, 127.9, 123.4, 116.7,113.4, 59.5, 33.1, 20.9, 19.5; MS (ESI+) for C₁₄H₁₄Br₂N₂O₃: 419 (MH⁺).

### (Compound 153)

### 8-Bromo-2-(2-methyl-1-pyrrolidin-1-ylpropyl)[1]benzofuro[3,2-d]pyrimidin-4(3H)-one

5-Bromo-3-(2-bromo-3-methylbutanamido)benzofuran-2-carboxamide (2, 85 mg, 0.203 mmol), pyrrolidine (2 mL) and tetrabutylammonium iodide (58 mg, 0.16 mmol) were placed in a test tube and heated to 120 °C for 15 min in a CEM-Discover microwave reactor. The reaction mixture, containing 5-bromo-3-(3-methyl-2-(pyrrolidin-1-yl)butanamido)benzofuran-2-carboxamide (3), was concentrated under reduced pressure and azeotroped with acetonitrile. The residual material was diluted with absolute ethanol (5 mL). 2 N NaOH (1 mL) was added, the mixture was heated to 130 °C for 30 min in a CEM-Discover microwave reactor. After concentration under reduced pressure, the crude reaction mixture was partitioned between water and ethyl acetate. The aqueous phase was further extracted with ethyl acetate and with chloroform (7 x 50 mL). The combined organic layers were dried over MgSO₄, then concentrated in vacuo. After purification by flash chromatography (91:8:1 dichloromethane/methanol/28% (w/w) ammonium hydroxide), the title compound was obtained as a solid (71 mg), 40% yield over two steps.
¹H NMR (400 MHz, CD₃OD) δ 8.17 (m, 1H), 7.70 (dd, *J*= 2.2, J*=* 9 Hz, 1H), 7.61 (dd, *J*= 1, *J*= 9 Hz, 1H), 3.46 (m, 5H), 3.12 (s, 2H), 2.14 (m, 5H), 1.44 (s, 6H); MS (ESI+) for C₁₈H₂₀BrN₃O₂: 391 (MH⁺).

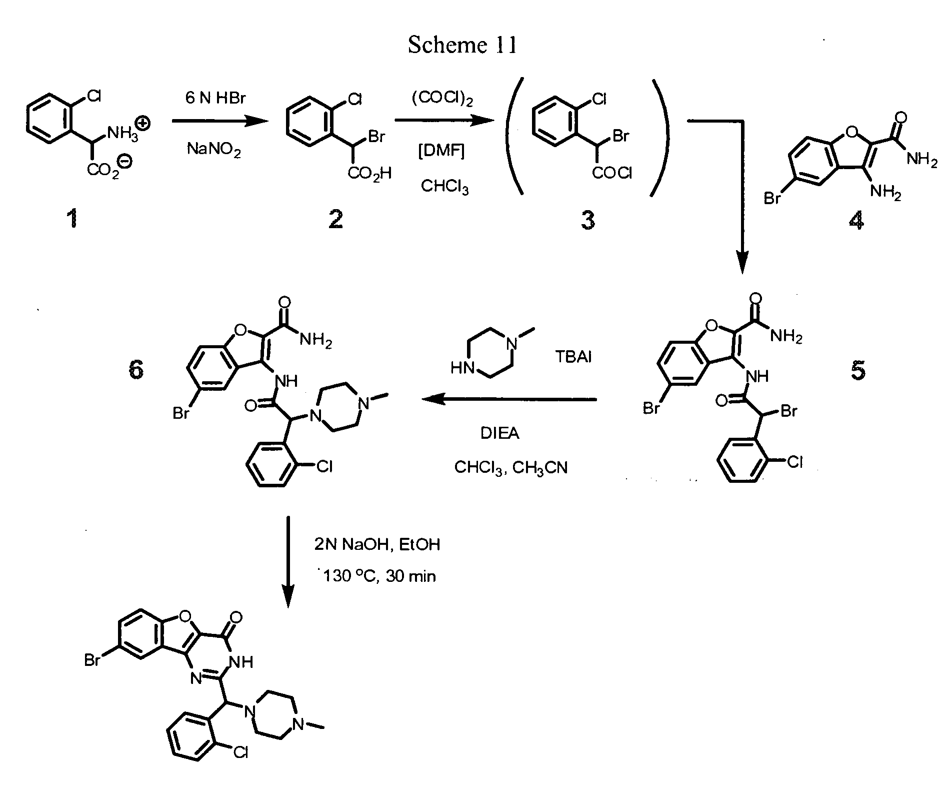

### 2-Bromo-2-(2-chlorophenyl)acetic acid (2)

A 100-mL round bottom flask was charged with 2-amino-2-(2-chlorophenyl)acetic acid (2.22 g, 11.9 mmol) and 6 N HBr (40 mL). Sodium nitrite (1.57 g, 22.8 mmol) was added portionwise at 0 °C over 1 h 20 min. After 6 h, the reaction mixture was poured onto water and extracted with 3:1 Et₂O/EtOAc (3x 80 mL). The combined organic layers were washed with water, sodium thiosulfate (2 x 50 mL), water, brine, dried over MgSO₄. After purification by flash chromatography (75:25:0.5:0.35 hexanes/ethyl acetate/acetic acid/methanol), the title compound was obtained as a solid (2.161 g), 73% yield over two steps.
¹H NMR (400 MHz, CDCl₃) δ 11.58 (br s, 1H), 7.77 (m, 1H), 7.40 (m, 1H), 7.31 (m, 2H), 5.94 (s, 1H); ¹³C NMR (100 MHz, CDCl₃) δ 174.0, 133.5, 133.2, 131.3, 130.0, 130.0, 127.9, 42.7.

### 5-Bromo-3-(2-bromo-2-(2-chlorophenyl)acetamido)benzofuran-2-carboxamide (5)

The title compound was synthesized in a manner similar to **Example 17**, wherein 2-bromo-3-methyl butanoic acid was substituted with 2-bromo-2-(2-chlorophenyl)acetic acid. After purification by flash chromatography (95:4.5:0.5 dichloromethane/methanol/28% (w/w) ammonium hydroxide), the title compound was obtained as a solid (357 mg), 87% yield.
¹H NMR (400 MHz, *d*₆-DMSO) δ 11.08 (br s, 1H), 8.29 (br d, 1H), 8.15 (m, 1H), 8.01 (br d, 1H), 7.83 and 7.72 (m, 1H), 7.65-7.69 (m, 1H), 7.62 (m, 1H), 7.51-7.59 (m, 1H), 7.45 (m, 2H), 6.45 (s, 1H); MS (ESI+) for C₁₇H₁₁Br₂ClN₂O₃: 487 (MH⁺).

### 5-Bromo-3-(2-(2-chlorophenyl)-2-(4-methylpiperazin-1-yl)acetamido)benzofuran-2-carboxamide (6)

The title compound was synthesized in a manner similar to **Example 17**, wherein 5-bromo-3-(2-bromo-3-methylbutanamido)benzofuran-2-carboxamide was substituted with 5-bromo-3-(2-bromo-2-(2-chlorophenyl)acetamido)benzofuran-2-carboxamide and pyrrolidine with N-methylpiperazine and diisopropylethylamine (1.12 equiv); chloroform (20 mL/mmol) and acetonitrile (20 mL/mmol) were also added. After purification by flash chromatography (95:4.5:0.5 to 90:9:1 dichloromethane/methanol/28% (w/w) ammonium hydroxide), the title compound was obtained as a solid (192 mg), quantitative yield.
¹H NMR (400 MHz, CDCl₃) δ 11.44 (s, 1H), 8.71 (d, *J*= 2.5 Hz, 1H), 7.47 (m, 3H), 7.28 (m, 3H), 6.59 (br s, 1H), 6.36 (br s, 1H), 5.00 (s, 1H), 2.74 (m, 8H), 2.41 (s, 3H); ¹³C NMR (100 MHz, CDCl₃) δ 169.7, 162.6, 152.3, 135.7, 133.1, 132.4, 131.6, 130.8, 130.4, 129.9, 129.8, 127.5, 127.3, 123.7, 116.4, 113.3, 70.2, 54.1, 50.7, 45.6; MS (ESI+) for C₂₂H₂₂BrClN₄O₃: 506 (MH⁺).

### *(Compound 156)

### 8-Bromo-2-[(2-chlorophenyl)(4-methylpiperazin-1-yl)methyl[1]benzofuro[3,2-d]pyrimidin-4(3H)-one

The title compound was synthesized in a manner similar to **Example 17**, wherein 5-bromo-3-(3-methyl-2-(pyrrolidin-1-yl)butanamido)benzofuran-2-carboxamide was substituted 5-bromo-3-(2-(2-chlorophenyl)-2-(4-methylpiperazin-1-yl)acetamido)benzofuran-2-carboxamide. After purification by flash chromatography (91:8:1 dichloromethane/methanol/28% (w/w) ammonium hydroxide), the title compound was obtained as a solid (71 mg), 40% yield.
¹H NMR (400 MHz, *d*₆-DMSO) δ 8.10 (m, 1H), 7.97 (d, *J*= 8 Hz, 1H), 7.81 (m, 2H), 7.44 (m, 2H), 7.33 (m, 1H), 5.07 (s, 1H), 2.31-2.48 (m, 8H), 2.17 (s, 3H); MS (ESI+) for C₂₂H₂₀BrClN₄O₂: 489 (MH⁺).

### *(Compound 68)

### 2-(4-amino-2-methylphenyl)-8-bromo[1]benzofuro[3,2-d]pyrimidin-4(3H)-one

2-(4-amino-2-methylphenyl)-8-bromo[1]benzofuro[3,2-d]pyrimidin-4(3H)-one was synthesized in a manner similar to **Scheme 11**, wherein 2-chloro-4-nitrobenzaldehyde was substituted with 2-methyl-4-nitrobenzaldehyde. NMR (400 MHz, d₆-DMSO): 8.18 (s, 1H), 7.80 (s, 2H), 7.24 (d, 1H), 6.47 (m, 2H), 5.49 (s, 2H), 2.49 (s, 3H). MS (EI) for C₁₇H₁₂BrN₃O₂: 371 (MH⁺).

### *(Compound 72)

### 8-bromo-2-{2-methyl-4-[(piperidin-4-ylmethyl)amino]pheny}[1]benzofuro[3,2-d]pyrimidin-4(3H)-one

8-bromo-2-{2-methyl-4-[(piperidin-4-ylmethyl)amino]phenyl}[1]benzofuro[3,2-d]pyrimidin-4(3H)-one was synthesized in a manner similar to **Scheme 11**, wherein 2-(4-amino-2-chlorophenyl)-8-bromo[1]benzofuro[3,2-d]pyrimidin-4(3H)-one was substituted with 2-(4-amino-2-methylphenyl)-8-bromo[1]benzofuro[3,2-d]pyrimidin-4(3H)-one. NMR (400 MHz, d₆-DMSO): 8.13 (s, 1H), 7.77 (s, 2H), 7.33 (d, 1H), 6.47 (m, 2H), 6.04 (m, 1H), 3.06 (d, 2H), 2.96 (t, 2H), 2.55 (m, 2H), 2.40 (s, 3H), 1.73 (m, 3H), 1.17 (m, 2H). MS (EI) for C₂₃H₂₃BrN₄O₂: 468 (MH⁺).

### *(Compound 61)

### 8-bromo-2-{2-methyl-4-[(tetrahydrofuran-3-ylmethyl)amino]phenyl}[1]benzofuro[3,2-d]pyrimidin-4(3H)-one was synthesized

8-bromo-2-{2-methyl-4-[(tetrahydrofuran-3-ylmethyl)amino]phenyl} [1]benzofuro[3,2-d]pyrimidin-4(3H)-one was synthesized in a manner similar to **Scheme 11**, wherein 2-(4-amino-2-chlorophenyl)-8-bromo[1]benzofuro[3,2-d]pyrimidin-4(3H)-one was substituted with 2-(4-amino-2-methylphenyl)-8-bromo[1]benzofuro[3,2-d]pyrimidin-4(3H)-one, and *tert*-butyl 4-formylpiperidine-1-carboxylate was substituted with tetrahydrofuran-3-carbaldehyde. NMR (400 MHz, d₆-DMSO): 8.16 (s, 1H), 7.79 (s, 2H), 7.34 (d, 1H), 6.50 (s, 2H), 6.14 (m, 1H), 3.75 (m, 2H), 3.64 (dd, 1H), 3.47 (dd, 1H), 3.05 (t, 2H), 2.39 (s, 3H), 1.99 (m, 1H), 1.90 (br s, 1H), 1.59 (m, 1H). MS (EI) for C₂₂H₂₀BrN₃O₃: 455 (MH⁺).

### EXAMPLE 18:

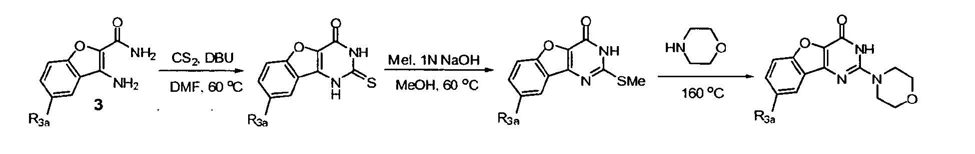

wherein R₃ₐ is as described in the disclosure above.

### 8-bromo-2-thioxo-2,3-dihydrobenzofuro[3,2-d]pyrimidin-4(1H)-one

To a solution of 3-amino-5-bromobenzofuran-2-carboxamide (0.500 g, 1.97 mmol) was added DBU (0.6 g, 3.94 mmol) and CS₂ (4.25 g, 56.0 mmol). The mixture was heated to 60 °C for 12 hours. The reaction was cooled down to room temperature and acidified with 1N HCl (50 mL). The precipitate was filtered off and washed with a large amount of water to afford 695 mg of the title compound (quantitative). ¹H NMR (400 MHz, d₆-DMSO): 13.54 (s, 1H), 12.87 (s, 1H), 8.34 (m, 1H), 7.81 (m, 2H). MS (EI) for C₁₀H₅BrN₂O₂S: 295 (M-H).

### 8-bromo-2-(methylthio)benzofuro[3,2-d]pyrimidin-4(3H)-one

To a solution of 8-bromo-2-thioxo-2,3-dihydrobenzofuro[3,2-d]pyrimidin-4(1H)-one (0.53 g, 1.80 mmol) was added aqueous 1N NaOH (1.80 mL) and iodomethane (0.25 g, 1.80 mmol). The reaction mixture was heated to 40 °C for 90 minutes and then concentrated *in vacuo.* After addition of 100 mL of water, the precipitate was filtered off and dried to afford the title compound (440 mg, 79%). ¹H NMR (400 MHz, d₆-DMSO): 8.17 (m, 1H), 7.81 (m, 2H), 2.64 (s, 3H).

### (Compound 147)

### 8-bromo-2-morpholin-4-yl][1]benzofuro[3,2-d]pyrimidin-4(3H)-one

A solution of 8-bromo-2-(methylthio)benzofuro[3,2-d]pyrimidin-4(3H)-one (51 mg, 0.165 mmol) in 1 mL morpholine was heated to 160 °C for 64 hours. The reaction mixture was cooled down to room temperature and partitioned between water and ethyl acetate. After extraction with ethyl acetate (2 x 50 mL), the combined organic phases were washed with brine, dried over Na₂SO₄ and concentrated *in vacuo* to afford the title compound (14.5 mg, 25%). ¹H NMR (400 MHz, d₆-DMSO): 8.05 (m, 1H), 7.73 (m, 2H), 3.69 (m, 4H), 3.63 (m, 4H). MS (EI) for C₁₄H₁₂BrN₃O₃: 350 (MH⁺).

### EXAMPLE 19:

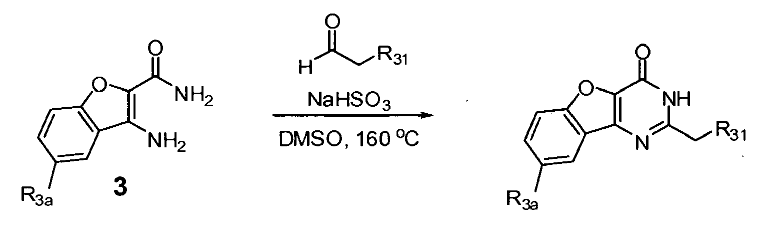

wherein R₃ₐ is as described in the disclosure above and R₃₁ is as described in the compounds within this example.

### (Compound 122)

### 8-bromo-2-(piperidin-4-ylmethyl)[1]benzofuro[3,2-d]pyrimidin-4(3H)-one

To a suspension of 3-amino-5-bromobenzofuran-2-carboxamide (100 mg, 0.39 mmol) in DMSO (3 mL) was added tert-butyl 4-(2-oxoethyl)piperidine-1-carboxylate (177 mg, 0.78 mmol) and NaHSO₃ (121 mg, 1.17 mmol). The reaction mixture was heated to 160 °C for 2 hours, cooled to room temperature and partitioned between water and ethyl acetate. The reaction was extracted with ethyl acetate (2 x 150 mL) and the combined organic phases were washed with brine, dried over Na₂SO₄ and concentrated *in vacuo.* To a solution of the crude product in MeOH (2 mL) was added 4 N HCl/dioxane (2 mL) and stirred for 12 hours at room temperature. The reaction mixture was concentrated *in vacuo* and purified by preparative HPLC to afford the title compound (14.3 mg, 10%). ¹H NMR (400 MHz, d₆-DMSO): 8.14 (m, 1H), 7.75 (m, 2H), 3.00 (m, 2H), 2.59 (m, 4H, overlapped), 2.02 (m, 1H), 1.62 (m, 2H), 1.23 (m, 2H). MS (EI) for C₁₆H₁₆BrN₃O₂: 362.2 (MH⁺).

### (Compound 168)

### 2-{1-(2-aminoethyl)piperidin-4-yl]methyl}-8-bromo[1]benzofuro[3,2-d]pyrimidin-4(3H)-one

To a solution of 8-bromo-2-(piperidin-4-ylmethyl)[1]benzofuro[3,2-d]pyrimidin-4(3H)-one **(Compound 122)** (25 mg, 0.066 mmol) in dichloroethane (3 mL) was added tert-butyl 2-oxoethylcarbamate (25 mg, 0.132 mmol), NaBH(OAc)₃ and acetic acid (300 µL) and stirred at room temperature for 12 hours. The reaction mixture was quenched with saturated aqueous NaHCO₃ (50 mL) and extracted with dichloromethane (2 x 150 mL), The combined organic phases were washed with brine, dried over Na₂SO₄ and concentrated *in vacuo.* To a solution of the crude product in MeOH (2 mL) was added 4 N HCl/dioxane (2 mL) and stirred for 12 hours at room temperature. The reaction mixture was concentrated *in vacuo* and purified by preparative HPLC to afford the title compound (10.3 mg, 39%). ¹H NMR (400 MHz, d₆-DMSO): 8.15 (m, 1H), 7.77 (m, 2H), 2.81 (m, 3H), 2.66 (m, 3H), 2.57 (m, 2H), 2.31 (m, 2H), 1.84 (m, 1H), 1.59 (m, 2H), 1.29 (m, 2H). MS (EI) for C₁₈H₂₁BrN₄O₂: 405.2 (MH⁺).

### (Compound 194)

### 8-bromo-2-(piperidin-3-ylmethyl)[1]benzofuro[3,2-d]pyrimidin-4(3H)-one

8-bromo-2-(piperidin-3-ylmethyl)[1]benzofuro[3,2-d]pyrimidin-4(3H)-one was synthesized in a manner similar to **Example 19**, wherein tert-butyl 4-(2-oxoethyl)piperidine-1-carboxylate was substituted with tert-butyl 3-(2-oxoethyl)piperidine-1-carboxylate. The reaction mixture was concentrated *in vacuo* and purified by preparative HPLC to afford the title compound (64 mg, 22%). ¹H NMR (400 MHz, d₆-DMSO): 8.13 (m, 1H), 7.77 (m, 2H), 2.99 (m, 2H), 2.56 (m, 2H), 2.38 (m, 2H), 2.07 (m, 1H), 1.73 (m, 1H), 1.61 (m, 1H), 1.43 (m, 1H), 1.15 (m, 1H). MS (EI) for C₁₆H₁₆BrN₃O₂: 361.9 (MH⁺).

### (Compound 226)

### 8-bromo-2-[(1-methylpiperidin-3-yl)methyl][1]benzofuro[3,2-d]pyrimidin-4(3H)-one

To a solution of 8-bromo-2-(piperidin-3-ylmethyl)benzofuro[3,2-d]pyrimidin-4(3H)-one **(Compound 194)** (150 mg, 0.42 mmol) in 5 mL of water was added formaldehyde (37% wt in water) and formic acid (2 mL). The reaction mixture was heated to reflux for 5 hours, cooled down, basified to pH 9 with aqueous 1N NaOH and extracted with chloroform (3 x 50 mL). The combined organic phases were washed with brine, dried over Na₂SO₄ and concentrated *in vacuo.* The crude material was purified by preparative HPLC to afford the title compound (42.5 mg, 27%). ¹H NMR (400 MHz, d₆-DMSO): 8.19 (m, 1H), 7.81 (m, 2H), 2.67 (m, 2H), 2.60 (m, 2H), 2.11 (s, 3H), 2.09 (m, 1H, overlappped), 1.84 (m, 1H), 1.63 (m, 3H), 1.43 (m, 1H), 0.94 (m, 1H). MS (EI) for C₁₇H₁₈BrN₃O₂: 376 (MH⁺).

### (Compound 244)

### 8-bromo-2-[(1-methylpiperidin-4-yl)methyl][1]benzofuro[3,2-d]pyrimidin-4(3H)-one

To a solution of 8-bromo-2-(piperidin-4-ylmethyl)benzofuro[3,2-d]pyrimidin-4(3H)-one **(Compound 122)** (100 mg, 0.27 mmol) in 5 mL of water was added formaldehyde (37% wt in water) and formic acid (2 mL). The reaction mixture was heated to reflux for 5 hours, cooled down, basified to pH 9 with aqueous 1N. NaOH and extracted with chloroform (3 x 50 mL). The combined organic phases were washed with brine, dried over Na₂SO₄ and concentrated *in vacuo.* The crude material was purified by preparative HPLC to afford the title compound (18.3 mg, 17.6%). ¹H NMR (400 MHz, d₆-DMSO): 8.19 (m, 1H), 7.80 (m, 2H), 2.72 (m, 2H), 2.60 (m, 2H), 2.12 (s, 3H), 1.80 (m, 3H), 1.58 (m, 2H), 1.25 (m, 2H). MS (EI) for C₁₇H₁₈BrN₃O₂: 376 (MH⁺).

### (Compound 305)

### 8-bromo-2-(3S)-piperidin-3-ylmethyl][1]benzofuro[3,2-d]pyrimidin-4(3H)-one

8-bromo-2-[(3S)-piperidin-3-ylmethyl][1]benzofuro[3,2-d]pyrimidin-4(3H)-one was synthesized in a manner similar to **Example 19**, wherein tert-butyl 4-(2-oxoethyl)piperidine-1-carboxylate was substituted with (S)-tert-butyl 3-(2-oxoethyl)piperidine-1-carboxylate. The reaction mixture was concentrated *in vacuo.* and purified by preparative HPLC to afford the title compound (16 mg, 3.7%). ¹H NMR (400 MHz, d₆-DMSO): 8.14 (m, 1H), 7.77 (m, 2H), 2.99 (m, 2H), 2.58 (m, 2H), 2.38 (m, 3H), 1.74 (m, 1H), 1.63 (m, 1H), 1.42 (m, 1H), 1.17 (m, 1H). MS (EI) for C₁₆H₁₆BrN₃O₂: 362 (MH⁺).

### (Compound 315)

### 8-bromo-2-[(3R)-piperidin-3-ylmethyl][1]benzofuro[3,2-d]pyrimidin-4(3H)-one

8-bromo-2-[(3R)-piperidin-3-ylmethyl][1]benzofuro[3,2-d]pyrimidin-4(3H)-one was synthesized in a manner similar to **Example 19**, wherein tert-butyl 4-(2-oxoethyl)piperidine-1-carboxylate was substituted with (R)-tert-butyl 3-(2-oxoethyl)piperidine-1-carboxylate. The reaction mixture was concentrated *in vacuo* and purified by preparative HPLC to afford the title compound (7.6 mg, 2%). ¹H NMR (400 MHz, d₆-DMSO): 8.14 (m, 1H), 7.84 (m, 2H), 3.23 (m, 2H), 2.64 (m, 2H), 2.33 (m, 3H), 1.78 (m, 2H), 1.59 (m, 1H), 1.28 (m, 1H). MS (EI) for C₁₆H₁₆BrN₃O₂: 362 (MH⁺).

### EXAMPLE 20

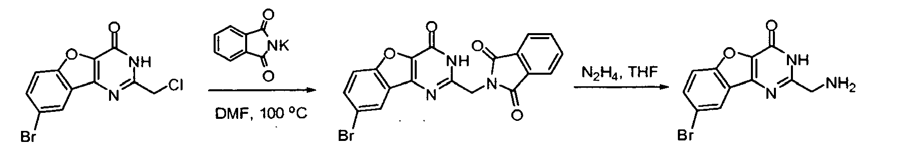

### (Compound 195)

### 2-(aminomethyl)-8-bromo[1]benzofuro[3,2-d]pyrimidin-4(3H)-one

To a solution of 8-bromo-2-(chloromethyl)benzofuro[3,2-d]pyrimidin-4(3H)-one (510 mg, 1.63 mmol) in DMF (8 mL) was added potassium 1,3-dioxoisoindolin-2-ide (452 mg, 2.44 mmol). The reaction mixture was heated to 100 °C for an hour, cooled down to room temperature, diluted with water and extracted with choroform (3 x 100 mL). The combined organic phases were washed with brine, dried over Na₂SO₄, concentrated *in vacuo* and purified by SiO₂ flash chromatography (50:50 to 30:70 hexanes/ethyl acetate) to afford 2-((8-bromo-4-oxo-3,4-dihydrobenzofuro[3,2-d]pyrimidin-2-yl)methyl)isoindoline-1,3-dione (160 mg, 25%). To a solution of 2-((8-bromo-4-oxo-3,4-dihydrobenzofuro[3,2-d]pyrimidin-2-yl)methyl)isoindoline-1,3-dione (160 mg, 0.378 mol) in THF (4 mL) was added hydrazine monohydrate (0.6 mL) and stirred at room temperature for 12 hours. The reaction mixture was concentrated *in vacuo* and purified by preparative HPLC to afford the title compound (9.2 mg, 8.3%). ¹H NMR (400 MHz, d₆-DMSO): 8.08 (m, 1H), 7.72 (m, 2H), 3.80 (m, 2H). MS (EI) for C₁₁H₈BrN₃O₂: 292 (M-H).

### EXAMPLE 21

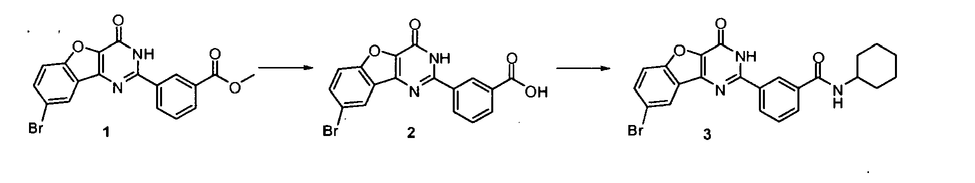

### (Compound 46)

### 3-(8-bromo-4-oxo-3,4-dihydro[1]benzofuro[3,2-d]pyrimidin-2-yl)-N-cyclohexylbenzamide 3 methyl 3-(8-bromo-4-oxo-3,4-dihydrobenzofuro[3,2-d]pyrimidin-2-yl)benzoate 1

Methyl 3-(8-bromo-4-oxo-3,4-dihydrobenzofuro[3,2-d]pyrimidin-2-yl)benzoate was synthesized in the same manner as **Example 8** wherein 2-chlorobenzaldehyde was replaced with methyl-3-formylbenzoate. Filtration of solids afforded 180 mg (45%) of title compound 1.

### 3-(8-bromo-4-oxo-3,4-dihydrobenzofuro[3,2-d]pyrimidin-2-yl)benzoic acid 2

To a solution of methyl 3-(8-bromo-4-oxo-3,4-dihydrobenzofuro[3,2-d]pyrimidin-2-yl)benzoate **1** (280 mg, 0.7 mmol) in 1:1 tetrahydrofuran:water (8 mL) and methanol (0.7 mL) was added lithium hydroxide (75 mg, 1.8 mmol) and stirred at room temperature for 2 h. The reaction mixture was concentrated *in vacuo.* The concentrate was dissolved in water and acidified with concentrated HCl. The solids were filtered and dried to afford 58 mg (20%) of 3-(8-bromo-4-oxo-3,4-dihydrobenzofuro[3,2-d]pyrimidin-2-yl)benzoic acid **2.**

### *(Compound 92)

### 3-(8-bromo-4-oxo-3,4-dihydro[1]benzofuro[3,2-d]pyrimidin-2-yl)-N-cyclohexylbenzamide

To a solution of 3-(8-bromo-4-oxo-3,4-dihydrobenzofuro[3,2-d]pyrimidin-2-yl)benzoic acid 2 (58 mg, 0.15 mmol) in dichloromethane (5 mL) was added cyclohexylamine (30 mg, 0.3 mmol), 1-(3-dimethylaminopropyl)-3-ethylcarbodiimide hydrochloride (32 mg, 0.17 mmol), HOBt (25 mg, 0.17 mmol), N-methylmorpholine (60 mg, 0.6 mmol) and stirred for 18 h at room temperature. The reaction was quenched with water and extracted with ethyl acetate (2 x 100 ml). The combined organic phases were washed with aqueous K₂CO₃, brine, dried over Na₂SO₄ and concentrated *in vacuo* to afford 29 mg of crude product. Purification by preparative HPLC (reverse-phase, acetonitrile/water with 0.1 % formic acid), followed by concentration *in vacuo* and lyophilization afforded the title compound (2.0 mg, 2 %). NMR (400 MHz, d₆-DMSO): 8.67 (s, 1H), 8.39 (d, 1H), 8.32 (d, 1H), 8.21 (s, 1H), 8.17 (s, 1H), 7.86 (d, 1H), 7.71 (s, 1H), 7.53 (t, 1H), 6.56-6.64 (s, br, 1H), 3.75-3.85 (s, br, 2H), 1.82-1.91 (m, 2H), 1.72-1.80 (m, 1H), 1.59-1.64 (m, 1H), 1.09-1.43 (m, 4H). MS (EI) for C₂₃H₂BrN₃O₂:466 (MH⁺).

### EXAMPLE 22

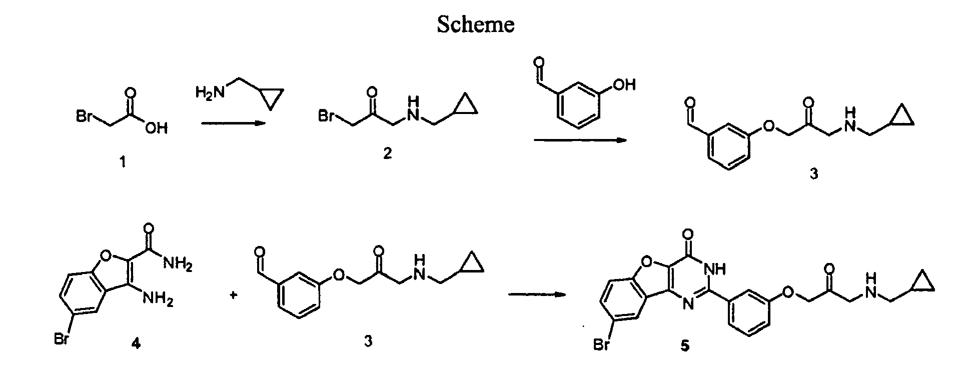

### 1-bromo-3-(cyclopropylmethylamino)propan-2-one 2

To a solution of aminomethylcyclopropane (466 mg, 6.5 mmol) in acetonitrile (20 mL) and diisopropylethylamine (2.2 mL) was added bromoacetic acid (1.1 g, 7.9 mmol) and HATU (2.9 g, 7.6 mmol). The reaction mixture was stirred 15 h and the solids were filtered. The filtrate was concentrated *in vacuo* to afford 430 mg (35%) of crude 1-bromo-3-(cyclopropylmethylamino)propan-2-one **2.**

### 3-(3-(cyclopropylmethylamino)-2-oxopropoxy)benzaldehyde 3

To a solution of 1-bromo-3(cyclopropylmethylamino)propan-2-one **2** (290 mg, 1.5 mmol) in acetone (20 mL) at 0°C was added 3-hydroxybenzaldehyde (185 mg, 1.5 mmol) and cesium carbonate (2.0 g, 6.1 mmol) and stirred for 3 h at room temperature. The reaction mixture was concentrated *in vacuo,* dissolved in ethyl acetate. The product was then purified by silica gel column using 1:1 hexanes:ethyl acetate as eluent, followed by concentration *in vacuo* and lyophilization to afford 240 mg (68%) of 3-(3-(cyclopropylmethylamino)-2-oxopropoxy)benzaldehyde **3.**

### *(Compound 98)

### 2- {[3-(8-bromo-4-oxo-3,4-dihydro [1]benzofuro[3,2-d]pyrimidin-2-yl)phenyl]oxy} -N-(cyclopropylmethyl)acetamide

2-{[3-(8-bromo-4-oxo-3,4-dihydro[1]benzofuro[3,2-d]pyrimidin-2-yl)phenyl]oxy}-N-(cyclopropylmethyl)acetamide was synthesized in a similar manner to **Example 8** wherein 2-chlorobenzaldehyde was replaced with 3-(3-(cyclopropylmethylamino)-2-oxopropoxy)benzaldehyde **3.** Purification by preparative HPLC (reverse-phase, acetonitrile/water with 0.1% formic acid), followed by concentration *in vacuo* and lyophilization afforded the title compound (3.9 mg). NMR (400 MHz, d₆-DMSO): 8.33 (s, 1H), 8.23-8.28 (m, 1H), 8.12 (s, 1H), 7.93-7.98 (m, 2H), 7.68 (s, 2H), 7.30-7.39 (m, 1H), 4.54 (s, 2H), 2.69-3.09 (m, 2H), 0.90-1.06 (m, 1H), 0.32-0.46 (m, 2H), 0.13-0.24 (m, 2H). MS (EI) for C₂₂H₁₈BrN₃O₄: 468 (MH⁺).

### *(Compound 99)

### 3-(8-bromo-4-oxo-3,4-dihydro[1]benzofuro[3,2-d]pyrimidin-2-yl)-N-(phenylmethyl)benzamide

3-(8-bromo-4-oxo-3,4-dihydro[1]benzofuro[3,2-d]pyrimidin-2-yl)-N-(phenylmethyl)benzamide was synthesized in a similar manner to **Example 21** wherein cyclohexylamine was replaced with benzylamine. Purification by preparative HPLC (reverse-phase, acetonitrile/water with 0.1 % formic acid), followed by concentration *in vacuo* and lyophilization afforded the title compound (2.8 mg). NMR (400 MHz, d₆-DMSO): 13.02-13.24 (s, br, 1H), 9.13-9.20 (m, 1H), 8.68 (s, 1H), 8.26-8.36 (m, 2H), 8.03-8.15 (m, 2H), 7.79-7.91 (m, 2H), 7.64-7.71 (m, 1H), 7.21-7.40 (m, 4H), 4.49-4.56 (m, 2H). MS (EI) for C₂₄H₁₆BrN₃O₃ 474 (MH⁺).

### *(Compound 101)

### 3-(8-bromo-4-oxo-3,4-dihydro[1]benzofuro[3,2-d]pyrimidin-2-yl)-N-(2-methylpropyl)benzamide

3-(8-bromo-4-oxo-3,4-dihydro[1]benzofuro[3,2-d]pyrimidin-2-yl)-N-(2-methylpropyl)benzamide was synthesized in a similar manner to **Example 21** wherein cyclohexylamine was replaced with isobutylamine. Purification by preparative HPLC (reverse-phase, acetonitrile/water with 0.1% formic acid), followed by concentration *in vacuo* and lyophilization afforded the title compound (3.5 mg). NMR (400 MHz, d₆-DMSO): 8.65 (s, 1H), 8.55-8.60 (m, 1H), 8.35 (d, 1H), 8.24 (s, 1H), 8.17 (s, 1H), 7.95 (d, 1H), 7.76-7.81 (m, 2H), 7.57-7.62 (m, 1H), 3.09-3.18 (m, 2H), 1.83-1.94 (m, 1H), 0.93 (d, 6H). MS (EI) for C₂₁H₁₈BrN₃O₃:440 (MH⁺).

### EXAMPLE 23

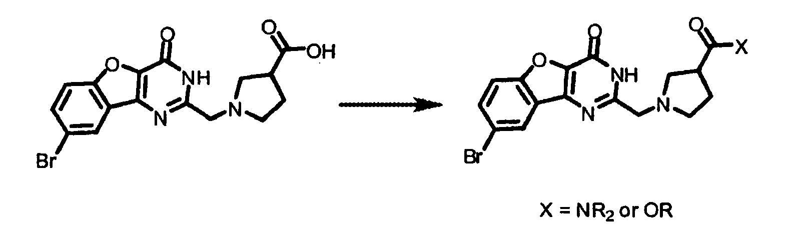

wherein R₂ and R are as described in the compounds within this example.

### (Compound 224)

### 1-[(8-bromo-4-oxo-3,4-dihydro[1]benzofuro[3,2-d]pyrimidin-2-yl)methyl]-N-[(2-chlorophenyl)methyl]pyrrolidine-3-carboxamide

To a solution of 1-[(8-bromo-4-oxo-3,4-dihydro[1]benzofuro[3,2-d]pyrimidin-2-yl)methyl]pyrrolidine-3-carboxylic acid **(Compound 103)** (40 mg, 0.1 mmol) in dimethylacetamide (2 mL) and diisopropylethylamine (130 mg, 1 mmol) was added 2-chlorobenzylamine (140 mg, 1 mmol) followed by HATU (380 mg, 1 mmol). The reaction mixture was stirred at room temperature for 2h. Formation of product was confirmed by LC/MS and the product was purified by preparatory HPLC (reverse-phase, acetonitrile/water with 0.1 % NH₄OAc/AcOH) to yield 9 mg (17%) of the title compound. NMR (400 MHz, d₆-DMSO): 8.39 (t, 1H), 8.21 (t, 1H), 7.83 (m, 2H), 7.42 (d, 1H), 7.28 (m, 2H), 4.32 (d, 2H), 3.68 (s, 2H), 2.94 (m, 2H), 2.75 (m, 1H), 2.67 (m, 1H), 2.61 (m, 1H), 1.99 (m, 2H). MS (EI) for C₂₃ H₂₀ Br Cl N₄ O₃: 515 (MH⁺).

### (Compound 264)

### 1-[(8-bromo-4-oxo-3,4-dihydro[1]benzofuro[3,2-d]pyrimidin-2-yl)methyl]-N-[(3-chlorophenyl)methyl]pyrrolidine-3-carboxamide

1-[(8-bromo-4-oxo-3,4-dihydro[1]benzofuro[3,2-d]pyrimidin-2-yl)methyl]-N-[(3-chlorophenyl)methyl]pyrrolidine-3-carboxamide was synthesized in a manner similar to **Example 22** wherein 2-chlorobenzylamine was substituted with 3-chlorobenzylamine. Purification by preparative HPLC (reverse-phase, acetonitrile/water with 0.1 % formic acid), followed by concentration *in vacuo* and lyophilization afforded the title compound (15.4 mg). NMR (400 MHz, d₆-DMSO): 13.35 (s, 1H), 10.48-10.53 (s, br, 1H), 8.78 (s, 1H), 8.17-8.30 (m, 1H), 7.85-7.93 (m, 2H), 7.31-7.40 (m, 2H), 7.25 (d, 1H), 4.50-4.69 (m, 2H), 4.33 (d, 2H), 4.07 (s, 2H), 3.88 (s, 2H), 2.89 (s, 1H), 1.98-2.35 (m, 2H). MS (EI) for C₂₃H₂₀BrClN₄O₃: 515 (MH⁺).

### (Compound 266)

### 1[(8-bromo-4-oxo-3,4-dihydro[1]benzofuro[3,2-d]pyrimidin-2-yl)methyl]-N-(phenylmethyl)pyrrolidine-3-carboxamide

1-[(8-bromo-4-oxo-3,4-dihydro[1]benzofuro[3,2-d]pyrimidin-2-yl)methyl]-N-(phenylmethyl)pyrrolidine-3-carboxamide was synthesized in a manner similar to **Example 22** wherein 2-chlorobenzylamine was substituted with benzylamine. Purification by preparative HPLC (reverse-phase, acetonitrile/water with 0.1 % formic acid), followed by concentration *in vacuo* and lyophilization afforded the title compound (64 mg). NMR (400 MHz, d₆-DMSO): 8.37 (t, 1H), 8.21 (s, 1H), 7.83 (m, 2H), 7.29 (m, 2H), 7.21 (m, 2H), 4.27 (d, 2H), 3.67 (s, 2H), 2.91 (m, 2H), 2.75 (m, 1H), 2.62 (m, 2H), 1.99 (m, 2H). MS (EI) for C₂₃ H₁₀ Br N₄ O₃: 482 (MH⁺).

### (Compound 267)

### 8-bromo-2-{[3-(morpholin-4-ylcarbonyl)pyrrolidin-1-yl]methyl}[1]benzofuro[3,2-d]pyrimidin-4(3H)-one

8-bromo-2-{[3-(morpholin-4-ylcarbonyl)pyrrolidin-1-yl]methyl}[1]benzofuro[3,2-d]pyrimidin-4(3H)-one was synthesized in a manner similar to **Example 22** wherein 2-chlorobenzylamine was substituted with morpholine. Purification by preparative HPLC (reverse-phase, acetonitrile/water with 0.1 % formic acid), followed by concentration *in vacuo* and lyophilization afforded the title compound (27 mg). NMR (400 MHz, d₆-DMSO): 8.13 (s, 1H), 7.83 (m, 2H), 3.69 (s, 2H), 3.53 (br s, 8H), 2.91 (m, 2H), 2.75 (m, 2H), 2.59 (m, 1H), 1.99 (m, 2H). MS (EI) for C₂₀ H₂, Br N₄ O₄: 462 (MH⁺).

### (Compound 271)

### 1-[(8-bromo-4-oxo-3,4-dihydro[1]benzofuro[3,2-d]pyrimidin-2-yl)methyl]-N-[(4-chlorophenyl)methyl]pyrrolidine-3-carboxamide

1-[(8-bromo-4-oxo-3,4-dihydro[1]benzofuro[3,2-d]pyrimidin-2-yl)methyl]-N-[(4-chlorophenyl)methyl]pyrrolidine-3-carboxamide was synthesized in a manner similar to **Example 22** wherein 2-chlorobenzylamine was substituted with 4-chlorobenzylamine. Purification by preparative HPLC (reverse-phase, acetonitrile/water with 0.1 % formic acid), followed by concentration *in vacuo* and lyophilization afforded the title compound (3 mg). NMR (400 MHz, d₆-DMSO): 8.42 (t, 1H), 8.20 (t, 1H), 7.82 (m, 2H), 7.34 (d, 2H), 7.22 (d, 2H), 4.25 (d, 2H), 3.66 (s, 2H), 2.90 (m, 2H), 2.75 (m, 1H), 2.67 (m, 2H), 1.94 (m, 2H). MS (EI) for C₂₃ H₂₀ Br Cl N₄ O₃: 515 (MH⁺).

### (Compound 272)

### 8-bromo-2-({3-[(4-hydroxypiperidin-1-yl)carbonyl]pyrrolidin-1-yl}methyl)[1]benzofuro[3,2-d]pyrimidin-4(3H)-one

8-bromo-2-({3-[(4-hydroxypiperidin-1-yl)carbonyl]pyrrolidin-1-yl}methyl)[1]benzofuro[3,2-d]pyrimidin-4(3H)-one was synthesized in a manner similar to **Example 22** wherein 2-chlorobenzylamine was substituted with 4-hydroxypiperidine. Purification by preparative HPLC (reverse-phase, acetonitrile/water with 0.1 % formic acid), followed by concentration *in vacuo* and lyophilization afforded the title compound (25 mg). NMR (400 MHz, d₆-DMSO): 8.21 (m, 1H), 8.14 (m, 1H), 7.83 (m, 2H), 3.91 (s, 2H), 3.69 (br s, 2H), 3.14 (m, 2H), 2.97 (m, 3H), 2.75 (m, 2H), 2.60 (m, 1H), 1.94 (m, 2H), 1.66 (m, 2H), 1.24 (m, 2H). MS (EI) for C₂₁ H₂₃ Br N₄ O₄: 476 (MH⁺).

### *(Compound 325)

### 1-[(8-bromo-4-oxo-3,4-dihydro[1]benzofuro[3,2-d]pyrimidin-2-yl)methyl]-N-(pyridin-4-ylmethyl)pyrrolidine-3-carboxamide

1-[(8-bromo-4-oxo-3,4-dihydro[1]benzofuro[3,2-d]pyrimidin-2-yl)methyl]-N-(pyridin-4-ylmethyl)pyrrolidine-3-carboxamide was synthesized in a manner similar to **Example 22** wherein 2-chlorobenzylamine was substituted with pyridin-4-ylmethanamine. Purification by preparative HPLC (reverse-phase, acetonitrile/water with 0.1% formic acid), followed by concentration *in vacuo* and lyophilization afforded the title compound (3 mg). NMR (400 MHz, d₆-DMSO): 8.58 (t, 1H), 8.45 (d, 2H), 8.16 (m, 1H), 7.78 (d, 2H), 7.20 (d, 2H), 4.28 (d, 2H), 3.64 (s, 2H), 2.91 (m, 2H), 2.71 (m, 3H), 1.98 (m, 2H). MS (EI) for C₂₂ H₂₀ Br N₅ O₃: 483 (MH⁺).

### *(Compound 411)

### '1-[(8-bromo-4-oxo-3,4-dihydro[1]benzofuro[3,2-d]pyrimidin-2-yl)methyl]-N-[(2-chlorophenyl)methyl]-3-hydroxypyrrolidine-3-carboxamide

'1-[(8-bromo-4-oxo-3,4-dihydro[1]benzofuro[3,2-d]pyrimidin-2-yl)methyl]-N-[(2-chlorophenyl)methyl]-3-hydroxypyrrolidine-3-carboxamide was synthesized in a manner similar to **Example 22** wherein 1-[(8-bromo-4-oxo-3,4-dihydro[1]benzofuro[3,2-d]pyrimidin-2-yl)methyl]pyrrolidine-3-carboxylic acid **(Compound 103)** was substituted with 1-[(8-bromo-4-oxo-3,4-dihydro[1]benzofuro[3,2-d]pyrimidin-2-yl)methyl]-3-hydroxypyrrolidine-3-carboxylic acid **(Compound 414)**. Purification by preparative HPLC (reverse-phase, acetonitrile/water with 0.1% formic acid), followed by concentration *in vacuo* and lyophilization afforded the title compound (12 mg). NMR (400 MHz, d₆-DMSO): 8.46 (t, 1H), 8.21 (d, 2H), 7.84 (m, 2H), 7.43 (dd, 1H), 7.27 (m, 3H), 4.35 (d, 2H), 3.75 (q, 2H), 2.99 (q, 1H), 2.93 (d, 1H), 2.84 (d, 1H), 2.59 (q, 1H), 2.30 (m, 1H), 1.87 (m, 1H). MS (EI) for C₂₃ H₂₀ BrCl N₄ O₄: 532 (MH⁺).

### EXAMPLE 23B

### (Compound 269)

### Ethyl 1-[(8-bromo-4-oxo-3,4-dihydro[1]benzofuro[3,2-d]pyrimidin-2-yl)methyl]pyrrolidine-3-carboxylate

To a solution of 1-[(8-bromo-4-oxo-3,4-dihydro[1]benzofuro[3,2-d]pyrimidin-2-yl)methyl]pyrrolidine-3-carboxylic acid **(Compound 103)** (200 mg, 0.5 mmol) in ethanol (3 mL) was added 2 drops of conc. sulfuric acid and the reaction mixture was stirred at 85 °C for 3h. Formation of product was confirmed by LC/MS and the product was purified by preparatory HPLC (reverse-phase, acetonitrile/water with 0.1% NH₄OAc/AcOH) to yield the title compound. NMR (400 MHz, d₆-DMSO): 8.20 (s, 1H), 7.81 (s, 2H), 4.05 (q, 2H), 3.37 (s, 2H), 3.04 (m, 1H), 2.90 (t, 1H), 2.75 (m, 1H), 2.67 (m, 2H), 1.99 (m, 2H), 1.17 (t, 3H). MS (EI) for C₁₈ H₁₈ Br N₃ O₄: 421 (MH⁺).

### (Compound 270)

### 1-[(8-bromo-4-oxo-3,4-dihydro[1]benzofuro[3,2-d]pyrimidin-2-yl)methyl]pyrrolidine-3-carboxamide

To a solution of ethyl 1-[(8-bromo4-oxo-3,4-dihydro[1]benzofuro[3,2-d]pyrimidin-2-yl)methyl]pyrrolidine-3-carboxylate (100 mg, 0.23 mmol) in ethanol (1 mL) was added 5 mL of 4N NH₃ in MeOH and the reaction mixture was stirred at 95 °C in a sealed vessel for 16h. Formation of product was confirmed by LC/MS and the product was purified by preparatory HPLC (reverse-phase, acetonitrile/water with 0.1 % NH₄OAc/AcOH) to yield 64 mg (70%) of the title compound. NMR (400 MHz, d₆-DMSO): 8.19 (s, 1H), 7.80 (s, 2H), 7.35 (s, 1H), 6.85 (s, 1H), 3.63 (s, 2H), 2.82 (m, 2H), 2.67 (m, 3H), 1.89 (m, 2H). MS (EI) for C₁₆ H₁₅ Br N₄ O₃: 392 (MH⁺).

### EXAMPLE 24

### *(Compound 314)

### N-[3-(8-bromo-4-oxo-3,4-dihydro[1]benzofuro[3,2-d]pyrimidin-2-yl)-4-chlorophenyl]pyridine-4-carboxamide

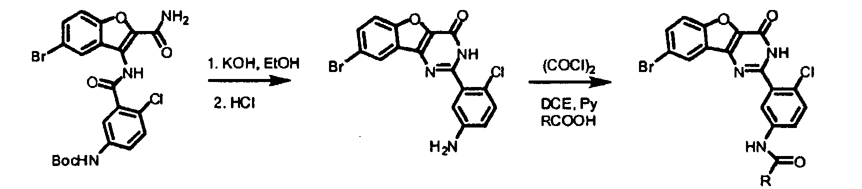

wherein R is as described within the compounds within this example.

### tert-Butyl 3-(5-bromo-2-carbamoylbenzofuran-3-ylcarbamoyl)-4-chlorophenylcarbamate

tert-Butyl 3-(5-bromo-2-carbamoylbenzofuran-3-ylcarbamoyl)-4-chlorophenylcarbamate was synthesized in a manner similar to **(Compound 198)** wherein 5-(tert-butoxycarbonylamino)-2-chlorobenzoic acid (CNH Technologies Inc.) was substituted with Boc-3-azetidine carboxylic acid. The crude material from this reaction was submitted to the next step without any further purification. ¹H NMR (400 MHz, d₆-DMSO):10.73 (br s, 1H), 9.76 (br s, 1H), 8.30 (s, 1H), 8.26 (s,1H), 8.00 (br s, 1H), 7.88 (s, 2H), 7.70 (dd, 1H), 7.63 (d, 1H), 7.57 (dd, 1H), 7.49 (d, 1H), 1.48 (s,9H). MS (EI) for C₂₁ H₁₉ Br Cl N₃ O₅ : 509 (MH⁺).

### 2-(5-Amino-2-chlorophenyl)-8-bromo[1]benzofuro[3,2-d]pyrimidin-4(3H)-one

tert-Butyl 3-(5-bromo-2-carbamoylbenzofuran-3-ylcarbamoyl)-4-chlorophenylcarbamate (15.92 mmoles, 8.1 g), was dissolved in ethanol (200 ml) and treated with aqueous 1M potassium hydroxide solution (60 mmoles, 60ml). The reaction mixture was then refluxed for 18 hours, allowed to cool to ca. 60 °C, and acidified to pH =1, by the drop wise addition of concentrated hydrochloric acid, which resulted in the formation of a white precipitate. The resulting suspension was then diluted with additional ethanol (100ml) and heated to 100 °C for a further 8 hours. The reaction mixture was then allowed to cool and the resulting solid was filtered off, washed with cold ethanol and dried under reduced pressure to give 4.97 of 2-(5-amino-2-chlorophenyl)-8-bromo[1]benzofuro[3,2-d]pyrimidin-4(3H)-one as its hydrochloride salt. ¹H NMR (400 MHz, d₆-DMSO):13.44 (br s, 1H), 8.25 (7.89 (dd, 1H), 7.85 (dd, 1H), 7.56 (d, 1H), 7.35 (s, 1H), 7.28 (dd, 1H), 6.82 (br s, 3H).MS (EI) for C16 H9 Br Cl N3 O2: 391: (MH⁺).

### N-[3-(8-bromo-4-oxo-3,4-dihydro[1]benzofuro[3,2-d]pyrimidin-2-yl)-4-chlorophenyl]pyridine-4-carboxamide

N-[3-(8-bromo-4-oxo-3,4-dihydro[1]benzofuro[3,2-d]pyrimidin-2-yl)-4-chlorophenyl]pyridine-4-carboxamide was synthesized in a manner similar to **Example 11** and **Example 11 + coupling**, wherein 3-dimethylaminopropionic acid was substituted with isonicotinic acid and 2-(4-amino-2-chlorophenyl)-8-bromo[1]benzofuro[3,2-d]pyrimidin-4(3H)-one was substituted with 2-(5-amino-2-chlorophenyl)-8-bromo[1]benzofuro[3,2-d]pyrimidin-4(3H)-one. Purification by preparative HPLC (reverse-phase, acetonitrile/water with 0.1% formic acid), followed by concentration *in vacuo* and lyophilization afforded the title compound (50.0 mg). NMR (400 MHz, d₆-DMSO): 13.41 (s, 1H), 11.02 (s, 1H), 8.87 (d, 2H), 8.26 (s, 1H), 8.12 (d, 1H), 8.00 (d, 2H), 7.83-7.94 (m, 3H), 7.72 (d, 1H). MS (EI) for C₂₂H₁₂BrClN₄O₃: 495 (MH⁺).

### *(Compound 326)

### N-[3-(8-bromo-4-oxo-3,4-dihydro[1]benzofuro[3,2-d]pyrimidin-2-yl)-4-chlorophenyl]-2-chloropyridine-4-carboxamide

N-[3-(8-bromo-4-oxo-3,4-dihydro[1]benzofuro[3,2-d]pyrimidin-2-yl)-4-chlorophenyl]-2-chloropyridine-4-carboxamide was synthesized in a manner similar to **Example 24 (Compound 314)** wherein isonicotinic acid was substituted with 2-chloroisonicotinic acid. Purification by preparative HPLC (reverse-phase, acetonitrile/water with 0.1% formic acid), followed by concentration *in vacuo* and lyophilization afforded the title compound (19.5 mg). NMR (400 MHz, d₆-DMSO): 10.84 (s, 1H), 8.62-8.66 (m, 1H), 8.18 (d, 1H), 8.02-8.07 (m, 2H), 7.92-7.96 (m, 1H), 7.88-7.91 (m, 1H), 7.77-7.85 (m, 2H), 7.61 (d, 1H), 6.61 (s, 1H). MS (EI) for C₂₂H₁₁BrCl₂N₄O₃: 530 (MH⁺).

### *(Compound 329)

### N-[3-(8-bromo-4-oxo-3,4-dihydro[1]benzofuro[3,2-d]pyimidin-2-yl)-4-chlorophenyl]pyrimidine-5-carboxamide

N-[3-(8-bromo-4-oxo-3,4-dihydro[1]benzofuro[3,2-d]pyrimidin-2-yl)-4-chlorophenyl]pyrimidine-5-carboxamide was synthesized in a manner similar to **Example 24** wherein isonicotinic acid was substituted with 5-pyrimidine carboxylic acid. Purification by preparative HPLC (reverse-phase, acetonitrile/water with 0.1% formic acid), followed by concentration *in vacuo* and lyophilization afforded the title compound (13.8 mg). NMR (400 MHz, d₆-DMSO): 10.92 (s, 1H), 9.30 (d, 2H), 8.22-8.24 (m, 1H), 8.09-8.12 (m, 1H), 7.94-7.98 (m, 1H), 7.81-7.90 (m, 2H), 7.64-7.68 (m, 1H), 6.61 (s, 1H). MS (EI) for C₂₁H₁₁BrClN₅O₃: 496 (MH⁺).

### *(Compound 342)

### N-[3-(8-bromo-4-oxo-3,4-dihydro[1]benzofuro[3,2-d]pyrimidin-2-yl)-4-chlorophenyl]furan-3-carboxamide

N-[3-(8-bromo-4-oxo-3,4-dihydro[1]benzofuro[3,2-d]pyrimidin-2-yl)-4-chlorophenyl]furan-3-carboxamide was synthesized in a manner similar **Example 24** wherein wherein isonicotinic acid was substituted with 3-furoic acid. Purification by preparative HPLC (reverse-phase, acetonitrile/water with 0.1% formic acid), followed by concentration *in vacuo* and lyophilization afforded the title compound (12.0 mg). NMR (400 MHz, d₆-DMSO): 10.22 (s, 1H), 8.43-8.43 (m, 1H), 8.20 (d, 1H), 8.00 (d, 1H), 7.90-7.95 (m, 1H), 7.78-7.85 (m, 3H), 7.60 (d, 1H), 6.95 (s, 1H). MS (EI) for C₂₁H₁₁BrClN₃O₄:484 (MH⁺).

### *(Compound 356)

### 4-(acetylaminol-N-[3-(8-bromo-4-oxo-3,4-dihydro[1]benzofuro[3,2-d]pyrimidin-2-yl)-4-chlorophenyl]benzamide

4-(acetylamino)-N-[3-(8-bromo-4-oxo-3,4-dihydro[1]benzofuro[3,2-d]pyrimidin-2-yl)-4-chlorophenyl]benzamide was synthesized in a manner similar to **Example 24** wherein wherein isonicotinic acid was substituted with 4-acetamidobenzoic acid. Purification by preparative HPLC (reverse-phase, acetonitrile/water with 0.1% formic acid), followed by concentration *in vacuo* and lyophilization afforded the title compound (6.9 mg). NMR (400 MHz, d₆-DMSO): 10.42 (s, 1H), 10.27 (s, 1H), 8.22 (d, 1H), 8.12 (d, 1H), 7.92-7.98 (m, 3H), 7.81-7.88 (m, 2H), 7.70-7.75 (m, 2H), 7.59 (d, 1H), 6.59 (s, 1H), 2.09 (s, 3H). MS (EI) for C₂₅H₁₆BrClN₄O₄: 551 (MH⁺).

### *(Compounds 357)

### N-[3-(8-bromo-4-oxo-3,4-dihydro[1]benzofuro[3,2-d]pyrimidin-2-yl)-4-chloropheny]l-6-(methyloxy)pyridine-3-carboxamide

N-[3-(8-bromo-4-oxo-3,4-dihydro[1]benzofuro[3,2-d]pyrimidin-2-yl)-4-chlorophenyl]-6-(methyloxy)pyridine-3-carboxamide was synthesized in a manner similar **Example 24** wherein isonicotinic acid was substituted with 6-methoxynicotinic acid. Purification by preparative HPLC (reverse-phase, acetonitrile/water with 0.1% formic acid), followed by concentration *in vacuo* and lyophilization afforded the title compound (29.6 mg). NMR (400 MHz, d₆-DMSO): 13.43 (s, 1H), 10.58 (s, 1H), 8.81 (d, 1H), 8.23-8.27 (m, 2H), 8.11 (d, 1H), 7.95-7.99 (m, 1H), 7.82-7.91 (m, 2H), 7.63 (d, 1H), 6.98 (d, 1H). MS (EI) for C₂₃H₁₄BrClN₄O₄: 525 (MH⁺).

### *(Compound 327)

### N-[3-(8-bromo-4-oxo-3,4-dihydro[1]benzofuro[3,2-d]pyrimidin-2-yl)-4-chlorophenyl]pyridine-3-carboxamide

N-[3-(8-bromo-4-oxo-3,4-dihydro[1]benzofuro[3,2-d]pyrimidin-2-yl)-4-chlorophenyl]pyridine.-3-carboxamide was synthesized in a manner similar to **Example 24** wherein isonicotinic acid was substituted with nicotinic acid. Purification by preparative HPLC (reverse-phase, acetonitrile/water with 0.1 % formic acid), followed by concentration *in vacuo* and lyophilization afforded the title compound (6.9 mg). NMR (400 MHz, d₆-DMSO): 10.77 (s, 1H), 9.13 (s, 1H), 8.78 (d, 1H), 8.31 (dt, 1H), 8.24 (d, 1H), 8.14 (d, 1H), 7.97 (dd, 1H), 7.88 (d, 1H), 7.84 (dd, 1H), 7.64 (d, 1H), 7.58 (dd, 1H). MS (EI) for C₂₂H₁₂BrClN₄O₃: 496 (MH⁺).

### *(Compound 360)

### N-[4-(8-bromo-4-oxo-3,4-dihydro[1]benzofuro[3,2-d]pyrimidin-2-yl)phenyl]pyridine-4-carboxamide

N-[4-(8-bromo-4-oxo-3,4-dihydro[1]benzofuro[3,2-d]pyrimidin-2-yl)phenyl]pyridine-4-carboxamide was synthesized in a manner similar to **Example 24** wherein isonicotinic acid was substituted with nicotinic acid, and 5-(tert-butoxycarbonylamino)-2-chlorobenzoic acid was substituted with 5-(tert-butoxycarbonylamino)-benzoic acid. Purification by preparative HPLC (reverse-phase, acetonitrile/water with 0.1% formic acid), followed by concentration *in vacuo* and lyophilization afforded the title compound (6.2 mg). NMR (400 MHz, d₆-DMSO): 13.13 (s, 1H), 10.81 (s, 1H), 8.81 (d, 1H), 8.22-8.30 (m, 2H), 7.96-8.01 (m, 2H), 7.81-7.93 (m, 3H), 6.57 (s, 1H). MS (EI) for C₂₂H₁₃BrN₄O₃: 461 (MH⁺).

### *(Compound 361)

### N-[3-(8-bromo-4-oxo-3,4-dihydro[1]benzofuro[3,2-d]pyrimidin-2-yl)-4-chlorophenyl]tetrahydrofuran-3-carboxamide

N-[3-(8-bromo-4-oxo-3,4-dihydro[1]benzofuro[3,2-d]pyrimidin-2-yl)-4-chlorophenyl]tetrahydrofuran-3-carboxamide was synthesized in a manner similar to **Example 24** wherein isonicotinic acid was substituted with tetrahydro-3-furoic acid. Purification by preparative HPLC (reverse-phase, acetonitrile/water with 0.1% formic acid), followed by concentration *in vacuo* and lyophilization afforded the title compound (34.3 mg). NMR (400 MHz, d₆-DMSO): 13.39 (s, 1H), 10.39 (s, 1H), 8.24 (d, 1H), 7.98 (d, 1H), 7.83-7.91 (m, 2H), 7.72-7.77 (m, 1H), 7.57 (d, 1H), 3.94 (t, 1H), 3.67-3.81 (m, 3H), 2.01-2.16 (m, 2H). MS (EI) for C₂₁H₁₅BrClN₃O₄: 488 (MH⁺).

### *(Compounds 392)

### N-[3-(8-bromo-4-oxo-3,4-dihydro[1]benzofuro[3,2-d]pyrimidin-2-yl)-4-chlorophenyl]piperidine-4-carboxamide

'N-[3-(8-bromo-4-oxo-3,4-dihydro[1]benzofuro[3,2-d]pyrimidin-2-yl)-4-chlorophenyl]piperidine-4-carboxamide was synthesized in a manner similar to **Example 24** wherein 3-amino-5-bromobenzofuran-2-carboxamide 3 was substituted with 2-(5-Amino-2-chlorophenyl)-8-bromo[1]benzofuro[3,2-d]pyrimidin-4(3H)-one. Purification by preparative HPLC (reverse-phase, acetonitrile/water with 0.1% formic acid), followed by concentration *in vacuo* and lyophilization afforded the title compound (34.3 mg). NMR (400 MHz, d₆-DMSO): 10.3 (s, 1H), 8.11 (d, 1H), 7.75 (m, 2H), 7.70 (m, 1H), 7.44 (d, 1H), 3.25-3.36 (m, 3H), 2.83 (m, 2H), 1.88 (m, 2H), 1.75 (m, 2H). MS (EI) for C₂₂H₁₈BrClN₄O₃: 501 (MH⁺).

### *(Compound 337)

### N-[3-(8-bromo-4-oxo-3,4-dihydro[1]benzofuro[3,2-d]pyrimidin-2-yl)-4-chlorophenyl]-2-(methyloxy)pyridine-4-carboxamide

N-[3-(8-bromo-4-oxo-3,4-dihydro[1]benzofuro[3,2-d]pyrimidin-2-yl)-4-chlorophenyl]-2-(methyloxy)pyridine-4-carboxamide was synthesized in a manner similar to **Example 24** wherein 2-methoxyisonicotinic acid replaced isonicotinic acid. 1H NMR (400 MHz, d6-DMSO): 13.45 (s, br, 1H), 10.78 (s, 1H), 8.37 (d, 1H), 8.25 (s, 1H), 8.14 (d, 1H), 7.98 (dd, 1H), 7.88 (m, 2H), 7.66 (d, 1H), 7.46 (dd, 1H), 7.34 (s, 1H), 3.91 (s, 3H). MS (EI) for C23 H14 Br Cl N4 O4: 527 (MH+).

### *(Compounds 338)

### 5-bromo-N-[3-(8-bromo-4-oxo-3,4-dihydro[1]benzofuro[3,2-d]pyrimidin-2-yl)-4-chlorophenyl]pyridine-2-carboxamide

5-bromo-N-[3-(8-bromo-4-oxo-3,4-dihydro[1]benzofuro[3,2-d]pyrimidin-2-yl)-4-chlorophenyl]pyridine-2-carboxamide was synthesized in a manner similar to **Example 24** wherein 5-bromopicolinic acid replaced isonicotinic acid. 1H NMR (400 MHz, d6-DMSO): 13.47 (s, br, 1H), 11.08 (s, 1H), 8.90 (s, 1H), 8.35 (m, 2H), 8.26 (s, 1H), 8.09 (d, 2H), 7.88 (m, 2H), 7.64 (d, 1H). MS (EI) for C22 H11 Br2 Cl N4 O3: 576 (MH+).

### *(Compounds 340)

### N-[3-(8-bromo-4-oxo-3,4-dihydro[1]benzofuro[3,2-d]pyrimidin-2-yl)-4-chlorophenyl]-5-chloropyridine-2-carboxamide

N-[3-(8-bromo-4-oxo-3,4-dihydro[1]benzofuro[3,2-d]pyrimidin-2-yl)-4-chlorophenyl]-5-chloropyridine-2-carboxamide was synthesized in a manner similar to **Example 24** wherein 5-chloropicolinic acid replaced isonicotinic acid I H NMR (400 MHz, d6-DMSO): 13.48 (s, br, 1H), 11.07 (s, 1H), 8.81 (s, 1H), 8.35 (s, 1H), 8.26 (s, 1H), 8.22 (d, 1H), 8.17 (d, 1H), 8.09 (d, 1H), 7.88 (m, 2H), 7.64 (d, 1H). MS (EI) for C22 H11 Br C12 N4 O3: 531 (MH+).

### *(Compounds 371)

### N-[3-(8-bromo-4-oxo-3,4-dihydro[1]benzofuro[3,2-d]pyrimidin-2-yl)-4-chlorophenyl]benzamide

N-[3-(8-bromo-4-oxo-3,4-dihydro[1]benzofuro[3,2-d]pyrimidin-2-yl)-4-chlorophenyl]benzamide was synthesized in a manner similar to **Example 24** wherein benzoyl chloride replaced isonicotinoyl chloride. 1H NMR (400 MHz, d6-DMSO):13.47 (s, 1H), 10.62 (s, 1H), 8.25 (s, 1H), 8.17 (s, 1H), 7.98 (m, 3H), 7.87 (m, 2H), 7.63 (m, 2H), 7.56 (m, 2H). MS (EI) for C23 H13 Br Cl N3 03: 496 (MH+).

### *(Compound 372)

### N-[3-(8-bromo-4-oxo-3,4-dihydro[1]benzofuro[3,2-d]pyrimidin-2-yl)-4-chlorophenyl]isoxazole-5-carboxamide

N-[3-(8-bromo-4-oxo-3,4-dihydro[1]benzofuro[3,2-d]pyrimidin-2-yl)-4-chlorophenyl]isoxazole-5-carboxamide was synthesized in a manner similar to **Example 24** wherein isoxazole-5-carboxylic acid replaced isonicotinic acid. 1H NMR (400 MHz, d6-DMSO): 11.07 (s, 1H), 8.85 (d, 1H), 8.21 (s, 1H), 8.10 (d, 1H), 7.95 (dd, 1H), 7.84 (m, 2H), 7.64 (d, 1H), 7.31 (d, 1H). MS (EI) for C20 H10 Br Cl N4 O4: 487 (MH+).

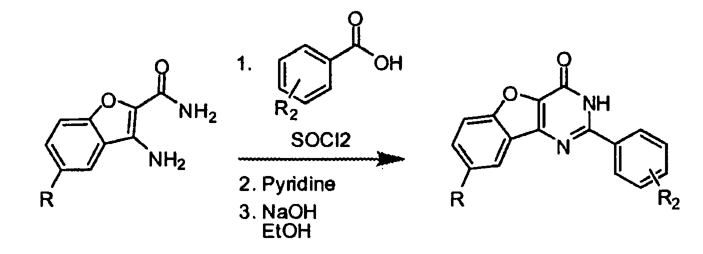

### EXAMPLE 25

### *(Compound 128)

### 8-bromo-2-[2-chloro-4-(methylsulfonyl)phenyl][1]benzofuro[3,2-d]pyrimidin-4(3H)-one

2-chloro-4-(methylsulfonyl)benzoic acid (300 mg, 1.28 mmol) was dissolved in 5 ml of thionyl chloride and the reaction mixture was heated to 70 °C for 1 hour. The reaction mixture was concentrated down under reduced pressure, and the white solid was dissolved in 5 ml of pyridine, and 3-amino-5-bromobenzofuran-2-carboxamide **3** (315 mg, 1.24 mmol) was added at room temperature. The reaction was stirred at room temperature for 1 hr, followed by addition of 10 ml of MeOH. The resulting precipitate was filtered off and dissolved in 3 ml of EtOH. The reaction was stirred for 12 hours at 120 °C in a sealed pressure tube. The reaction was cooled and then neutralized with 1M HCl until the pH 7. The resulting precipitate was filtered and washed with 5 ml of H₂O. The product was purified by preparatory HPLC (reverse-phase, acetonitrile/water with 0.1% NH₄OAc/AcOH) to yield 34.7 mg (12%) of the title compound. ¹H NMR (400 MHz, d₆-DMSO): 13.49 (s, 1H), 8.25 (d, 1H), 8.21 (d, 1H), 8.07 (dd, 1H), 8.00 (d, 1H), 7.89 (m, 2H), 3.41 (s, 3H); MS (EI) for C₁₇H₁₀BrClN₂O₄S: 455 (MH⁺).

### *(Compound 219)

### 8-bromo-2-{3-[(2-methylpropyl)oxy]pyridin-4-yl}[1]benzofuro[3,2-d]pyrimidin-4(3H)-one

8-bromo-2-{3-[(2-methylpropyl)oxy]pyridin-4-yl}[1]benzofuro[3,2-d]pyrimidin-4(3H)-one was synthesized in a manner similar to **Example 11,** wherein 3-isobutoxyisonicotinic acid **(Note A)** replaced 2-chloro-4-(methylsulfonyl)benzoic acid. ¹H-NMR (400MHz, d₆-DMSO): 13.02 (s, 1H), 8.58 (s, 1H), 8.38 (d, 1H), 8.23 (s, 1H), 7.86 (m, 2H), 7.66 (d, 1H), 4.00 (d, 2H), 1.99 (m, 1H), 0.93 (d, 6H). MS (EI) for C₁₉H₁₆BrN₃O₃: 415 (MH⁺).

### Note A: 3-isobutoxyisonicotinic acid

Sodium metal (7.3 g, 0.32 mol) was added in small portions to 2-methyl-1-propanol (145 mL, 0.63 mol) at 80 °C over 30 min period, and the reaction mixture was stirred for an additional 3 h at 80 °C. Subsequently, a solution of 3-chloroisonicotinic acid (10g, 63 mmol) in 5 mL of DMSO was added to the reaction mixture at 80 °C. The resulting slurry was heated to 120 °C for an additional 16h, then cooled down to room temperature, concentrated down to half volume under the reduced pressure, and filterred. The filtrate was concentrated down to half volume under the reduced pressure, and filterred again. The combined solids were mixed with 10 mL of MeOH and 1 mL of water and acidified with concentrated HCl at 0 °C to pH = 7. The resulting precipitate was filterred out and dried under vacuum, resulting in 10 g (81%) of 3-isobutoxyisonicotinic acid. ¹H-NMR (400MHz, d6-DMSO): 8.47 (s, 1H), 8.23 (d, 1H), 7.46 (d, 1H), 3.91 (d, 2H), 2.04 (m, 1H), 0.94 (d, 6H). MS (EI) for C₁₀ H₁₃NO₃: 196 (MH+).

### *(Compound 148)

### 8-bromo-2-(4-chloropyridin-3-yl)[1]benzofuro[3,2-d]pyrimidin-4(3H)-one

8-bromo-2-(4-chloropyridin-3-yl)[1]benzofuro[3,2-d]pyrimidin-4(3H)-one was synthesized in a manner similar to **Example 25** wherein 4-chloronicotinic acid replaced 2-chloro-4(methylsulfonyl)benzoic acid. 1H NMR (400 MHz, d6-DMSO): 13.55 (s, br, 1H), 8.89 (s, 1H), 8.70 (d, 1H), 8.25 (s, 1H), 7.75 (m, 3H). MS (EI) for C15 H7 Br Cl N3 O2: 378 (MH+).

### *(Compound 282)

### 8-bromo-2-{(3R)-5-oxo-1-[(1R)-1-phenylethyl]pyrrolidin-3-yl}[1]benzofuro[3,2-d]pyrimidin-4(3H)-one

8-bromo-2-{(3R)-5-oxo-1-[(1R)-1-phenylethyl]pyrrolidin-3-yl}[1]benzofuro[3,2-d]pyrimidin-4(3H)-one was synthesized in a manner similar to **Example 25** wherein (1'R, 3R)-1-(1'-phenylethyl)-5-oxo-3-pyrrolidine carboxylic acid replaced 2-chloro-4-(methylsulfonyl)benzoic acid. ¹H NMR (400 MHz, d₆-DMSO): 8.18 (s, 1H), 7.82 (s, 2H), 7.41-7.25 (m, 5H), 5.35-5.29 (m, 1H), 3.80-3.60 (m, 2H), 3.42-3.35 (m, 1H), 2.85-2.70 (m, 2H), 1.52 (d, 2H); MS (EI) for C₂₂H₁₈BrN₃O₃: 452 (MH⁺).

### (Compound 423)

### 2-(1H-benzimidazol-6-yl)-8-bromo[1]benzofuro[3,2-d]pyrimidin-4(3H)-one

2-(1H-benzimidazol-6-yl)-8-bromo[1]benzofuro[3,2-d]pyrimidin-4(3H)-one was synthesized in a manner similar to **Example 25** wherein benzimidazole-5-carboxylic acid replaced 2-chloro-4(methylsulfonyl)benzoic acid. ¹H NMR (400 MHz, d6-DMSO): 12.77 (s, 1H), 12.68 (s, 1H), 8.48 (s, 1H), 8.32 (m, 1H), 8.22 (m, 1H), 8.07 (m, 1H), 7.76 (m, 2H), 7.65 (m, 1H). MS (EI) for C17 H9 Br N4 O2: 382 (MH+).

### Example 26

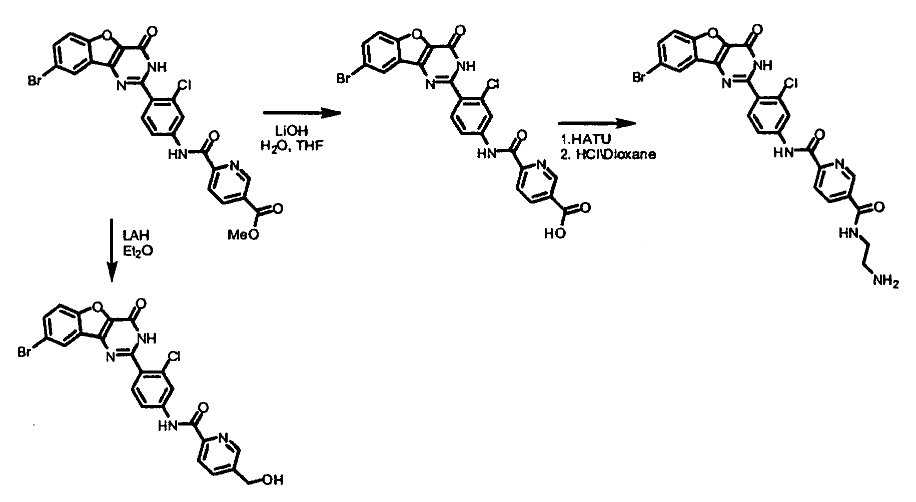

### *(Compound 345)

### N-[4-(8-bromo-4-oxo-3,4-dihyrdro[1]benzofuro[3,2-d]pyrimidin-2-yl)-3-chlorophenyl]-5-(hydroxymethyl)pyridine-2-carboxamide

To a solution of methyl 6-({[4-(8-bromo-4-oxo-3,4-dihydro[1]benzofuro[3,2-d]pyrimidin-2-yl)-3-chlorophenyl]amino}carbonyl)pyridine-3-carboxylate **(Compound 334)** (20 mg, 0.036 mmol) in diethyl ether (5 mL) was added 0.5 mL of LiAlH₄ 1 N solution in THF (0.25 mmol) and the reaction mixture was stirred at RT for 16 h. The resulting slurry was quenched with 1N HCl (2 mL) and the product was purified by preparatory HPLC (reverse-phase, acetonitrile/water with 0.1% formic acid) to yeild 3mg (16%) of the title compound. ¹H NMR (400 MHz, d₆-DMSO): 10.96 (s, 1H), 8.71 (s, 1H), 8.17 (m, 3H), 7.96 (m, 2H), 7.74 (m, 2H), 7.59 (d, 1H), 6.60 (s, 1H), 5.56 (t, 1H), 4.69 (d, 2H); MS (EI) for C₂₃H₁₄BrClN₄O₄: 526 (MH⁺).

### *(Compound 347)

### 6-({[4-(8-bromo-4-oxo-3,4-dihydro[1]benzofuro[3,2-d]pyrimidin-2-yl)-3-chlorophenyl]amino}carbonyl)pyridine-3-carboxylic acid

To a solution of methyl 6-({[4-(8-bromo-4-oxo-3,4-dihydro[1]benzofuro[3,2-d]pyrimidin-2-yl)-3-chlorophenyl]amino} carbonyl)pyridine-3-carboxylate **(Compound 334)** (180 mg, 0.32 mmol) in THF (2 mL), MeOH (0.5 mL), and H₂O (1 mL) was added 0.1 g of LiOH and the reaction mixture was stirred at RT for 16 h. The resulting slurry was acidified with 1N HCl (2 mL) to pH 6. The product was filtered out and submitted to the next step without further purification. The portion of the product was purified by preparatory HPLC (reverse-phase, acetonitrile/water with 0.1% formic acid) to yeild the title compound. ¹H NMR (400 MHz, d₆-DMSO): 11.24 (s, 1H), 9.18 (s, 1H), 8.49 (d, 1H), 8.29 (m, 3H), 8.08 (dd, 1H), 7.88 (m, 2H), 7.70 (d, 1H); MS (EI) for C₂₃H₁₂BrClN₄O₅: 541 (MH⁺).

### *(Compound 394)

### 'N'5'-(2-aminoethyl)-N'2'-[4-8-bromo-4-oxo-3,4-dihydro[1]benzofuro[3,2-d]pyrimidin-2-yl)-3-chlorophenyl]pyridine-2,5-dicarboxamide

To a solution of 6-({[4-(8-bromo-4-oxo-3,4-dihydro[1]benzofuro[3,2-d]pyrimidin-2-yl)-3-chlorophenyl]amino}carbonyl)pyridine-3-carboxylic acid **(Compound 347)** (50mg, 0.09 mmol) in DMA (5 mL), and diisopropylethylamine (0.5 mL) was added tert-butyl 2-aminoethylcarbamate (75 mg, 0.45 mmol) followed by HATU (170 mg, 0.45 mmol). The reaction mixture was stirred at RT for 16 h, then concentrated down under reduced pressure and re-dissolved in 5 mL of MeOH, and 5 mL of 4N HCl in Dioxane was added. The resulting mixture was stirred at RT for an additional 6 h and concentrated under reduced pressure. The product was purified by preparatory HPLC (reverse-phase, acetonitrile/water with 0.1% formic acid) to yeild the title compound. ¹H NMR (400 MHz, d₆-DMSO): 11.03 (s, 1H), 9.14 (s, 1H), 8.97 (s, 1H), 8.45 (d, 1H), 8.27 (d, 1H), 8.19 (s, 1H), 8.11 (s, 1H), 7.95 (d, 1H), 7.73 (m, 2H), 7.60 (d, 1H), 6.60 (s, 1H), 6.52 (s, 1H), 3.17 (m, 2H), 2.87 (m, 2H); MS (EI) for C₂₅H₁₈BrClN₆O₄: 582 (MH⁺).

### Example 26B

### (Compound 369)

### 1-[4-(8-bromo-oxo-3,4-dihydro[1]benzofuro[32-d]pyrimidin-2-yl)-3-chlorophenyl]-3-pyridin-4-ylurea

To a solution of 2-(4-amino-2-chlorophenyl)-8-bromo[1]benzofuro[3,2-d]pyrimidin-4(3H)-one (0.2g, 0.47 mmol) in pyridine (5 mL) was added 4-Nitrophenyl chloroformate (0.1 g, 0.5 mmol). The reaction mixture was stirred at room temperature for 1h. The solvent was concentrated under reduced pressure. The residue was taken up in 1 mL of dimethylformamide, and 4-aminopyridine (0.23 g, 2.5 mmol) in 1 mL of dichloromethane was added. The resulting mixture was heated to 100 °C at 150 W for 10 minutes in a CEM-Discover microwave reactor. The solvent was concentrated under reduced pressure and the residue was purified by preparative HPLC (reverse-phase, acetonitrile/water with 0.1% formic acid) to yeild the title compound. 1H NMR (400 MHz, d6-DMSO): 13.30 (s, 1H), 10.58 (s, 1H), 9.99 (s, 1H), 8.59 (d, 2H), 8.24 (s, 1H), 7.85 (m, 5H), 7.67 (d, 1H), 7.53 (d, 1H). MS (EI) for C₂₂ H₁₃ Br Cl N₅ O₃: 511 (MH+).

### (Compound 420)

### 8-bromo-2-{[(3S)-3-hydropyrrolidin-1-yl]methyl}-7-methyl[1]benzofuro[3,2-d]pyrimidin-4(3H)-one

8-bromo-2-{[(3S)-3-hydroxypyrrolidin-1-yl]methyl}-7-methyl[1]benzofuro[3,2-d]pyrimidin-4(3H)-one was synthesized in a manner similar to **Example 1** wherein 4-methyl-5-bromo-2-hydroxybenzonitrile replaced 5-bromo-2-hydroxybenzonitrile 1. 8-Bromo-2-{[(3S)-3-hydroxypyrrolidin-1-yl]methyl}-7-methyl[1]benzofuro[3,2-d]pyrimidin-4(3H)-one as mono HCl salt ¹H NMR (400 MHz, d₆-DMSO): 13.27 (br s, 1H), 10.71 (br s, 1H), 8.21 (s, 1H), 7.89 (s, 1H), 5.51 (s, 2H), 4.52 (s, 3H), 3.74 (s, 2H), 3.37 (br s, 2H), 1.88-2.37 (m, 3H); MS (EI) for C₁₆H₁₆BrN₃O₃: 379 (MH⁺).

### 4-methyl-5-bromo-2-hydroxybenzonitrile

To a solution of BCl₃ (1M in dichloromethane, Aldrich, 12 mL, 12 mmol) was added a solution of 4-bromo-3-methyl-phenol (1.87 g, 10 mmol) in 35 mL of dichloroethane, CH₃SCN (0.88g, 12 mmol), and AlCl₃ (1.33g, 10 mmol) at 0 °C. The mixture was heated to 80 °C for 4h under stirring. The reaction mixture was cooled down to room temperature and poured onto ice-4 N NaOH (33 mL), and heated to 80 °C for additional 30 min. Upon completion, the layers were separated, and the aqueous layer was acidified with 6N HCl and extracted with ether. The ether layer was washed with H2O and dried over MgSO4. Concentration under reduced pressure and re-crystallization from EtOAc/ Hexanes resulted in 0.95 g (47%) of the title compound. MS (EI) for C₈H₆BrNO: 202 (MH⁺)

### EXAMPLE 27

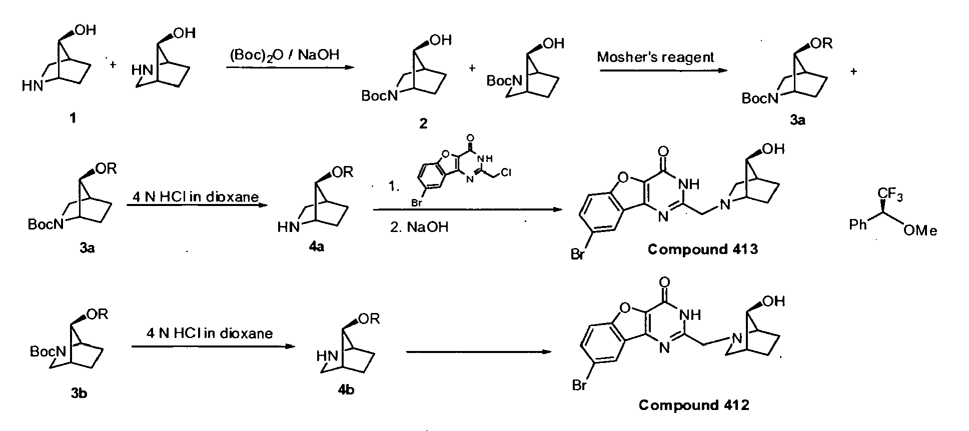

A solution of anti -7-hydroxy-2-azabicyclo[2.2.1]heptane **1** (0.75 g, 6.60 mmol, purchased from Tyger) and di-*tert*-butyldicarbonate (2.17 g, 9.90 mmol) in dioxane/ 1N NaOH (2:1, 75 mL) was stirred vigorously at room temperature for 24 hours. The solvent was evaporated and the residue was partitioned between DCM and water. The organic layer was dried and concentrated. The residue oil was purified by silica chromatography (hexanes/ EtOAc 3:2) to obtain **2** (racemic mixture). ¹H NMR (400 MHz, CDCl₃): 4.18 (s, 1H), 3.95 (m, 1H), 3.35 (m, 1H), 3.00 (dd, 1H), 2.30 (m, 1H), 1.95 (m, 2H), 1.75 (m, 1H), 1.65 (m, 1H), 1.45 (s, 9H).

To a stirred solution of compound **2** (0.43 g, 2.0 mmol) in DCM (10 mL) at 0 °C was added 4-dimethylaminopyridine (2.0 mmol) followed by (R)-(-)-α-methyl- α-trifluoromethyl-phenylacetyl chloride (504 mg) over 2 min. The mixture was warmed to room temp and stirred overnight. Then the reaction mixture was quenched with water, extracted with EtOAc, washed with brine, dried and concentrated. The residue was purified by silica chromatography, eluted with hexanes/THF (9:1) which first gave **3a** (280 mg), and further elution provided compound **3b** (260 mg).
Compound 3a: 1H NMR (400 MHz, CDCl₃): 7.53 (m, 2H), 7.42 (m, 3H), 5.05 (d, 1H), 4.20 (d, 1H), 3.53 (s, 3H), 3.40 (m, 1H), 3.08 (dd, 1H), 2.60 (m, 2H), 1.80-1.60 (m, 3H), 1.45 (m, 9H).
Compound 3b: ¹H NMR (400 MHz, CDCl₃): 7.52 (m, 2H), 7.41 (m, 3H), 5.03 (d, 1H), 4.20 (d, 1H), 3.55 (s, 3H), 3.41 (m, 1H), 3.07 (dd, 1H), 2.60 (s, 2H), 1.84-1.60 (m, 3H), 1.43 (m, 9H).

### (Compound 413)

### 8-bromo-2-{[(7S)-7-hydroxy-2-azabicyclo[2.2.1]hept-2-yl]methyl}[1]benzofuro[3,2-d]pyrimidin-4(3H)-one

To a solution of compound 3a (150 mg, 0.35 mmol) in EtOAc (10 mL) was added 4 N HCl in dioxane (3 mL) at 0 °C. The reaction mixture was allowed to warm up to room temperature and stirred 19 hours. The reaction was concentrated. The residue was dissolved in ethanol (15 mL); 8-bromo-2-(chloromethyl)[1]benzofuro[3,2-d]pyrimidin-4(3H)-one **6** was added in one portion, followed by sodium bicarbonate (300 mg). The reaction mixture was heated to 80°C for 4 hours. Then the reaction was cooled, 1 N NaOH (3 mL) was added. The reaction mixture was stirred at room temperature for 3 hours. Once the hydrolysis was complete, the reaction was neutralized with 1N HCl. The reaction mixture was concentrated in vacuo and purified by preparative HPLC to give the final product. ¹H NMR (400 MHz, d₆-DMSO): 8.19 (s, 1H), 7.82 (m, 2H), 4.45 (s, 1H), 4.18 (s, 2H), 3.55 (m, 2H), 3.38 (m, 1H), 2.96 (d, 1H), 2.35 (s, 1H), 2.05 (m, 2H), 1.60 (m, 1H); MS (EI) for C₁₇H₁₆BrN₃O₃: 390 (MH⁺).

### (Compound 412)

### 8-bromo-2-[(7-hydroxy-2-azabicyclo[2.2.1]hept-2-yl)methyl][1]benzofuro[3,2-d]pyrimidin-4(3H)-one

8-bromo-2-[(7-hydroxy-2-azabicyclo[2.2.1]hept-2-yl)methyl][1]benzofuro[3,2-d]pyrimidin-4(3H)-one was synthesized in a manner similar to **Compound 413,** wherein 3a was substituted with 3b. Purification by preparative HPLC (reverse-phase, acetonitrile/water with 0.01 % ammonium acetate), followed by concentration *in vacuo* and lyophilization afforded the title compound as a white solid. ¹H NMR (400 MHz, CD₃OD): 8.15 (s, 1H), 7.65 (m, 2H), 7.35 (m 1H), 4.45 (s, 1H), 4.15 (s, 2H), 3.55 (m, 2H), 3.39 (m, 1H), 2.95 (d, 1H), 2.35 (s, 1H), 2.05 (m, 2H), 1.55 (m, 1H); MS (EI) for C₁₇H₁₆BrN₃O₃: 390 (MH⁺).

### EXAMPLE 28

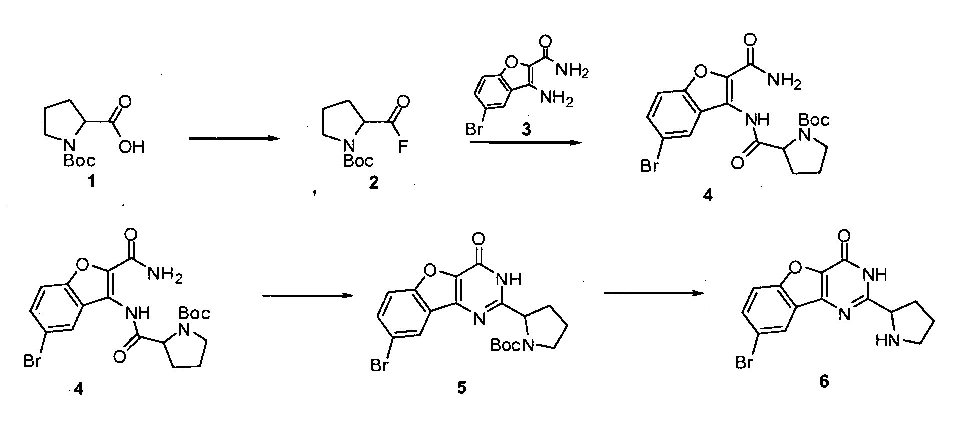

tert-butyl 2-(fluorocarbonyl)pyrrolidine-1-carboxylate **2**

To a solution of 1-(tert-butoxycarbonyl)pyrrolidine-2-carboxylic acid **1** (6.0 g, 27.9 mmol) in dichloromethane (50 mL) at 0°C was added pyridine (2.3 mL, 27.9 mmol) and cyanuric fluoride (3.77 g, 31 mmol). The reaction mixture was allowed to warm to room temperature while stirring for 2 h. The reaction was quenched with water (10mL), and the resulting mixture was filtered through celite and washed with dichloromethane (200 mL)). The layers were separated, the organic layer was washed with water (100 mL), brine (100 mL), dried over MgSO₄ and concentrated under reduced pressure to afford tert-butyl 2-(fluorocarbonyl)pyrrolidine-1-carboxylate 2. ¹H NMR (400 MHz, CDCl₃): 4.23-4.60 (m, 1H), 3.36-3.63 (m, 2H), 1.85-2.43 (m, 4H), 1.42-1.51 (m, 9H).

### tert-butyl 2-(5-bromo-2-carbamoylbenzofuran-3-ylcarbamoyl)pyrrolidine-1-carboxylate 3

To a solution of 3-amino-5-bromobenzofuran-2-carboxamide **3 (Example 1)** (1.5 g, 5.9 mmol) in pyridine (1.5 mL, 17.7 mmol) at 0°C was added a solution of tert-butyl 2-(fluorocarbonyl)pyrrolidine-1-carboxylate **2** (1.4 g, 6.5 mmol) in dichloromethane (50 mL) over 15 minutes. The reaction mixture was removed from the ice bath and was stirred over night at room temperature. DMAP (1.0 g, 8.1 mmol) was added and the reaction was again stirred over night at room temperature. The reaction mixture was quenched with water and extracted with ethyl acetate (2 x 150 mL). The combined organic phases were washed with brine, dried over MgSO₄ and concentrated under reduced pressure to afford tert-butyl 2-(5-bromo-2-carbamoylbenzofuran-3-ylcarbamoyl)pyrrolidine-1-carboxylate **4.** The product was submitted to the next step without further purification. MS (EI) for C₁₉H₂₂BrN₃O₅: 452 (MH⁺).

### tert-butyl 2-(8-bromo-4-oxo-3,4-dihydrobenzofuro[3,2-d]pyrimidin-2-yl)pyrrolidine-1-carboxylate 5

A solution of crude tert-butyl 2-(5-bromo-2-carbamoylbenzofuran-3-ylcarbamoyl)pyrrolidine-1-carboxylate **4** in ethanol (10 mL) and potassium hydroxide (10%) in water (5 mL) was heated to 80°C for 3h. The reaction mixture was cooled down to 0 °C and neutralized to pH 7 with conc. HCl. The precipitate was filtered, washed with ethyl acetate/hexanes and methanol, and dried to afford 1.7 g of tert-butyl 2-(8-bromo-4-oxo-3,4-dihydrobenzofuro[3,2-d]pyrimidin-2-yl)pyrrolidine-1-carboxylate **5** (1.7 g, 66% over 2 steps). MS (EI) for C₁₉H₂₀BrN₃O₄: 434 (MH⁺).

### (Compound 169)

### 8-bromo-2-pyrrolidin-2-yl[1]benzofuro[3,2-d]pyrimidin-4(3H)-one 6

A solution of tert-butyl 2-(8-bromo-4-oxo-3,4-dihydrobenzofuro[3,2-d]pyrimidin-2-yl)pyrrolidine-1-carboxylate **5** (1.7 g, 3.9 mmol) in methanol (10 mL) and 4N HCl in dioxane (5 mL) was stirred overnight at room temperature. The reaction mixture was filtered, resulting in 0.69 g (67%) of the title compound. ¹H NMR (400 MHz, d₆-DMSO): 10.48-11.50 (br, s, 1H), 8.17 (s, 1H), 7.76-7.87 (m, 2H), 4.78 (s, 1H), 3.43-3.63 (m, 2H), 2.42 (s, 1H), 1.91-2.21 (m, 4H); MS (EI) for C₁₄H₁₂BrN₃O₂: 334 (MH⁺).

### (Compound 175)

### 2-(1-aminoethyl)-8-bromo[1]benzofuro[3,2-d]pyrimidin-4(3H)-one

2-(1-aminoethyl)-8-bromo[1]benzofuro[3,2-d]pyrimidin-4(3H)-one was synthesized in a manner similar to **Example 28** wherein 1-(tert-butoxycarbonyl)pyrrolidine-2-carboxylic acid was substituted with 2-(tert-butoxycarbonylamino)propanoic acid. ¹H NMR (400 MHz, d₆-DMSO): 13.47 (s, 1H), 8.78 (s, 2H), 8.09 (s, 1H), 7.84-7.93 (m, 2H), 4.47 (s, 1H), 1.60 (s, 3H); MS (EI) for C₁₂H₁₀BrN₃O₂: 308 (MH⁺).

### (Compound 186)

### 8-bromo-2-pyrrolidin-3-yl[1]benzofuro[3,2-d]pyrimidin-4(3H)-one

8-bromo-2-pyrrolidin-3-yl[1]benzofuro[3,2-d]pyrimidin-4(3H)-one was synthesized in a manner similar to **Example 28** wherein 1-(tert-butoxycarbonyl)pyrrolidine-2-carboxylic acid was substituted with 1-(tert-butoxycarbonyl)pyrrolidine-3-carboxylic acid. ¹H NMR (400 MHz, d₆-DMSO): 13.17 (s, 1H), 9.75 (s, 1H), 9.33 (s, 1H), 8.27-8.30 (m, 1H), 7.79-7.86 (m, 2H), 3.25-3.84 (m, 4H), 2.31-2.45 (m, 1H), 2.15-2.28(m, 1H), 2.09 (s, 1H), 1.04 (s, 1H); MS (EI) for C₁₄H₁₂BrN₃O₂: 334 (MH⁺).

### (Compound 513)

### 8-bromo-2-[(2S,4S)-4-fluoropyrrolidin-2-yl][1]benzofuro[3,2-d]pyrimidin-4(3H)-one (2S,4S)-tert-bulyl 2-(5-bromo-2-carbamoylbenzofuran-3-ylcarbamoyl)-4-fluoropyrrolidine-1-carboxylate

The intermediate (2S,4S)-tert-butyl 2-(5-bromo-2-carbamoylbenzofuran-3-ylcarbamoyl)-4-fluoropyrrolidine-1-carboxylate was synthesized in a manner similar to 1, 1-dimethylethyl 3-(5-bromo-2-carbamoylbenzofuran-3-ylcarbamoyl)azetidine-1-carboxylate 4-(tert-butoxycarbonylamino), wherein (2S,4S)-1-(tert-butoxycarbonyl)-4-fluoropyrrolidine-2-carboxylic acid (commercially available from Omega Chem) replaced Boc-3-azetidine carboxylic acid. The crude material from this reaction was submitted to the next step without any further purification. MS (EI) for C₁₉H₂₁ Br F N₃ O₅ : 471.3 (MH⁺).

### (2S,4S)-tert-butyl 2-(8-bromo-4-oxo-3,4-dihydrobenzofuro[3,2-d]pyrimidin-2-yl)-4-fluoropyrrolidine-1-carboxylate

(2S,4S)-tert-butyl2-(8-bromo-4-oxo-3,4-dihydrobenzofuro[3,2-d]pyrimidin-2-yl)-4-fluoropyrrolidine-1-carboxylate was synthesized in a similar manner as to 1, 1-dimethylethyl 4-(8-bromo-4-oxo-3,4-dihydrobenzofuro[3,2-d]pyrimidin-2-yl)piperidine-1-carboxylate wherein (2S,4S)-tert-butyl 2-(5-bromo-2-carbamoylbenzofuran-3-ylcarbamoyl)-4-fluoropyrrolidine-1-carboxylate_was substituted for 1,1-dimethylethyl 4-(5-bromo-2-carbamoylbenzofuran-3-ylcarbamoyl)piperidine-1-carboxylate. MS (EI) for C₁₉ H₁₉ Br F N₃ O₄ : 453.3 (MH⁺).

### (Compound 513)

### 8-bromo-2-[(2S,4S)-4-fluoropyrrolidin-2-yl][1]benzofuro[3,2-d]pyrimidin-4-(3H)-one

8-bromo-2-[(2S,4S)-4-fluoropyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2S,4S)-tert-butyl 2-(8-bromo-4-oxo-3,4-dihydrobenzofuro[3,2-d]pyrimidin-2-yl)-4-fluoropyrrolidine-1-carboxylate. ¹H NMR (400 MHz, d₆-DMSO): 8.21 (s, 1H), 7.88 (m, 2H), 5.50 (d, 1H), 4.98 (dd, 1H), 3.80 (m, 1H), 3.69 (m, 1H), 2.82 (m, 2H), 2.61 (m, 1H). MS (EI) for C₁₄ H₁₁ Br F N₃ O₂ : 353.3 (MH⁺).

### (Compound 525)

### 8-chloro-2-[(2S,4S)-4-fluoropyrrolidin-2-yl][1]benzofuro[3,2-d]pyrimidin-4(3H)-one (2S,4S)-tert-butyl 2-(2-carbamoyl-5-chlorobenzofuran-3-ylcarbamoyl)-4-fluoropyrrolidine-1-carboxylate

The intermediate (2S,4S)-tert-butyl 2-(2-carbamoyl-5-chlorobenzofuran-3-ylcarbamoyl)-4-fluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2S,4S)-1-(tert-butoxycarbonyl)-4-fluoropyrrolidine-2-carboxylic acid (commercially available from Omega Chem) replaced Boc-3-azetidine carboxylic acid. The crude material from this reaction was submitted to the next step without any further purification. MS (EI) for C₁₉ H₂₁ Cl F N₃O₅: 426.8 (MH⁺).

### (2S,4S)-tert-butyl 2-(8-chloro-4-oxo-3,4-dihydrobenzofuro[3,2-d]pyrimidin-2-yl)-4-fluoropyrrolidine-1-carboxylate

(2S,4S)-tert-butyl 2-(8-chloro-4-oxo-3,4-dihydrobenzofuro[3,2-d]pyrimidin-2-yl)-4-fluoropyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2S,4S)-tert-butyl 2-(2-carbamoyl-5-chlorobenzofuran-3-ylcarbamoyl)-4-fluoropyrrolidine-1-carboxylate was substituted for 1,1-dimethylethyl 4-(5-bromo-2-carbamoylbenzofuran-3-ylcarbamoyl)piperidine-1-carboxylate. MS (EI) for C₁₉ H₁₉ Cl F N₃ O₄: 408.8 (MH⁺).

### (Compound 525)

### 8-chloro-2-[(2S,4S)-4-fluoropyrrolidin-2-yl][1]benzofuro[3,2-d]pyrimidin-4(3H)-one

8-chloro-2-[(2S,4S)-4-fluoropyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2S,4S)-tert-butyl 2-(8-chloro-4-oxo-3,4-dihydrobenzofuro[3,2-d]pyrimidin-2-yl)-4-fluoropyrrolidine-1-carboxylate. ¹H NMR (400 MHz, d₆-DMSO): 8.06 (d, 1H), 7.95 (d, 1H), 7.76 (dd, 1H), 5.50 (d, 1H), 4.97 (dd, 1H), 3.80 (m, 1H), 3.63 (m, 1H), 2.81 (m, 2H), 2.57 (m, 1H). MS (EI) for C₁₄H₁₁ClFN₃O₂: 308.5 (MH⁺).

### (Compound 526)

### 8-chloro-2-[(2S,4R)-4-fluoropyrrolidin-2-yl][1]benzofuro[3,2-d]pyrimidin-4(3H)-one (2S,4R)-tert-butyl 2-(2-carbamoyl-5-chlorobenzofuran-3-ylcarbamoyl)-4-fluoropyrrolidine-1-carboxylate

The intermediate (2S,4R)-tert-butyl 2-(2-carbamoyl-5-chlorobenzofuran-3-ylcarbamoyl)-4-fluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2S,4S)-1-(tert-butoxycarbonyl)-4-fluoropyrrolidine-2-carboxylic acid (commercially available from Omega Chem) replaced Boc-3-azetidine carboxylic acid. The crude material from this reaction was submitted to the next step without any further purification. MS (EI) for C₁₉ H₂₁ Cl F N₃ O₅ : 426.8 (MH⁺).

### (2S,4R)-tert-butyl 2-(8-chloro-4-oxo-3,4-dihydrobenzofuro[3,2-d]pyrimidin-2-yl)-4-fluoropyrrolidine-1-carboxylate

(2S,4R)-tert-butyl2-(8-chloro-4-oxo-3,4-dihydrobenzofuro[3,2-d]pyrimidin-2-yl)-4-fluoropyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2S,4R)-tert-butyl 2-(2-carbamoyl-5-chlorobenzofuran-3-ylcarbamoyl)-4-fluoropyrrolidine-1-carboxylate replaced 1,1-dimethylethyl 4-(5-bromo-2-carbamoylbenzofuran-3-ylcarbamoyl)piperidine-1-carboxylate. MS (EI) for C₁₉ H₁₉ Cl F N₃ O₄ : 408.8 (MH⁺).

### (Compound 526)

### 8-chloro-2-[(2S,4R)-4-fluoropyrrolidin-2-yl][1]benzofuro[3,2-d]pyrimidin-4(3H)-one

8-chloro-2-[(2S,4R)-4-fluoropyrrolidin-2-yl][1]benzofuro[3,2-d]pyrimidin-4(3H)-one was synthesized in a similar manner to 8-bromo-2-piperidin-4-yl[1]]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2S,4R)-tert-butyl 2-(8-chloro-4-oxo-3,4-dihydrobenzofuro[3,2-d]pyrimidin-2-yl)-4-fluoropyrrolidine-1-carboxylate.
¹H NMR (400 MHz, d₆-DMSO): 8.06 (d, 1H), 7.95 (d, 1H), 7.76 (dd, 1H), 5.60 (d, 1H), 4.97 (dd, 1H), 3.81 (m, 1H), 3.67 (m, 1H), 2.82 (m, 1H), 2.47 (m, 2H). MS (EI) for C₁₄ H₁₁ ClF N₃O₂: 308.6 (MH⁺).

### (Compound 530)

### 2-[(2S,4S)-4-fluoropyrrolidin-2-yl]-8-(methyloxy)[1]benzofuro[3,2-d]pyrimidin-4(3H)-one

### (2S,4S)-tert-butyl2-(2-carbamoyl-5-methoxybenzofuran-3-ylcarbamoyl)-4-fluoropyrrolidine-1-carboxylate

The intermediate (2S,4S)-tert-butyl 2-(2-carbamoyl-5-methoxybenzofuran-3-ylcarbamoyl)-4-fluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2S,4S)-1-(tert-butoxycarbonyl)-4-fluoropyrrolidine-2-carboxylic acid (commercially available from Omega Chem) replaced Boc-3-azetidine carboxylic acid. The crude material from this reaction was submitted to the next step without any further purification. MS (EI) for C₂₀ H₂₄ F N₃ O₆ : 422.4 (MH⁺).

### (2S,4S)-tert-butyl 4-fluoro-2-(8-methoxy-4-oxo-3,4-dihydrobenzofuro[3,2-d]pyrimidin-2-yl)pyrrolidine-1-carboxylate

(2S,4S)-tert-butyl4-fluoro-2-(8-methoxy-4-oxo-3,4-dihydrobenzofuro[3,2-d]pyrimidin-2-yl)pyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2S,4S)-tert-butyl 2-(2-carbamoyl-5-methoxybenzofuran-3-ylcarbamoyl)-4-fluoropyrrolidine-1-carboxylate replaced 1,1-dimethylethyl 4-(5-bromo-2-carbamoylbenzofuran-3-ylcarbamoyl)piperidine-1-carboxylate. MS (EI) for C₂₀ H₂₂ F N₃ O₅ : 404.2 (MH⁺).

### (Compound 530)

### 2-[(2S,4S)-4-fluoropyrrolidin-2-yl]-8-(methyloxy)[1]benzofuro[3,2-d]pyrimidin-4(3H)-one

2-[(2S,4S)-4-fluoropyrrolidin-2-yl]-8-(methyloxy)[1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2S,4S)-tert-butyl 4-fluoro-2-(8-methoxy-4-oxo-3,4-dihydrobenzofuro[3,2-d]pyrimidin-2-yl)pyrrolidine-1-carboxylate. ¹H NMR (400 MHz, d₆-DMSO): 7.66 (d, 1H), 7.42 (d, 1H), 7.18 (dd, 1H), 5.21 (d, 1H), 3.84 (s, 3H), 3.27 (dd, 2H), 3.01 (dd, 1H), 2.23 (m, 1H), 1.84 (m, 2H). MS (EI) for C₁₅ H₁₄ F N₃ O₃: 304.0 (MH⁺).

### (Compound 500)

### 8-bromo-2-[(4R)-1,3-thiazolidin-4-yl][1]benzofuro[3,2-d]pyrimidin-4(3H)-one (R)-tert-butyl 4-(5-bromo-2-carbamoylbenzofuran-3-ylcarbamoyl)thiazolidine-3-carboxylate

The intermediate (R)-tert-butyl 4-(5-bromo-2-carbamoylbenzofuran-3-ylcarbamoyl)thiazolidine-3-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (R)-3-(tert-butoxycarbonyl)thiazolidine-4-carboxylic acid (commercially available from Chem-Impex International) replaced Boc-3-azetidine carboxylic acid. The crude material from this reaction was submitted to the next step without any further purification. MS (EI) for C₁₈ H₂₀ BrN3 O₅ S: 471.4 (MH⁺)..

### (R)-tert -butyl 4-(8-bromo-4-oxo-3,4-dihydrobenzofuro[3,2-d]pyrimidin-2-yl)thiazolidine-3-carboxylate

(R)-tert-butyl 4-(8-bromo-4-oxo-3,4-dihydrobenzofuro[3,2-d]pyrimidin-2-yl)thiazolidine-3-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (R)-tert-butyl 4-(5-bromo-2-carbamoylbenzofuran-3-ylcarbamoyl)thiazolidine-3-carboxylate replaced 1,1-dimethylethyl 4-(5-bromo-2-carbamoylbenzofuran-3-ylcarbamoyl)piperidine-1-carboxylate. MS (EI) for C₁₈ H₁₈ Br N₃ O₄ S: 453.3 (MH⁺).

### (Compound 500)

### 8-bromo-2-[(4R)-1,3-thiazolidin-4-yl][1]benzofuro[3,2-d]pyrimidin-4(3H)-one

8-bromo-2-[(4R)-1,3-thiazolidin-4-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (R)-tert-butyl 4-(8-bromo-4-oxo-3,4-dihydrobenzofuro[3,2-d]pyrimidin-2-yl)thiazolidine-3-carboxylate. ¹H NMR (400 MHz, d₆-DMSO): 8.21 (s, 1H), 7.88 (m, 2H), 4.90 (m, 2H), 4.57 (m, 2H), 4.40 (m, 2H). MS (EI) for C₁₃H₁₀ Br N₃ O₂ S: 353.3 (MH⁺).

### (Compound 514)

### 8-bromo-2-[(2S,4R)-4-fluoropyrrolidin-2-yl][1]benzofuro[3,2-d]pyrimidin-4(3H)-one (2R,4S)-tert-butyl 2-(5-bromo-2-carbamoylbenzofuran-3-ylcarbamoyl)-4-fluoropyrrolidine-]-carboxylate

The intermediate (2R,4S)-tert-butyl 2-(5-bromo-2-carbamoylbenzofuran-3-ylcarbamoyl)-4-fluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2S,4R)-1-(tert-butoxycarbonyl)-4-fluoropyrrolidine-2-carboxylic acid (N-t-BOC-trans-4-Fluoro-L-Proline, OmegaChem Inc.) replaced Boc-3-azetidine carboxylic acid. The crude material from this reaction was submitted to the next step without any further purification. MS (EI) for C₁₉ H₂₁ B rF N₃ O₅: 471.3 (MH⁺).

### (2R,4S)-tert-butyl2-(8-bromo-4-oxo-3,4-dihydrobenzofuro[3,2-d]pyrimidin-2-yl)-4-fluoropyrrolidine-1-carboxylate

(2R,4S)-tert-Butyl 2-(8-bromo-4-oxo-3,4-dihydrobenzofuro[3,2-d]pyrimidin-2-yl)-4-fluoropyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2R,4S)-tert-butyl 2-(5-bromo-2-carbamoylbenzofuran-3-ylcarbamoyl)-4-fluoropyrrolidine-1-carboxylate replaced 1,1-dimethylethyl 4-(5-bromo-2-carbamoylbenzofuran-3-ylcarbamoyl)piperidine-1-carboxylate. MS (EI) for C₁₉ H₁₉ Br F N₃ O₄: 452.2 (MH⁺).

### 8-bromo-2-[(2S,4R)-4-fluoropyrrolidin-2-yl][1]benzofuro[3,2-d]pyrimidin-4(3H)-one

8-bromo-2-[(2S,4R)-4-fluoropyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2R,4S)-tert-butyl 2-(8-bromo-4-oxo-3,4-dihydrobenzofuro [3,2-d]pyrimidin-2-yl)-4-fluoropyrrolidine-1-carboxylate. ¹H NMR (400 MHz, d₆-DMSO): 8.21 (s, 1H), 7.89 (m, 2H), 5.61 (d, 1H), 4.98 (m, 1H), 3.73 (m, 2H), 2.84 (m, 1H), 2.45 (m; H). MS (EI) for C₁₄ H₁₁ Br F N3 O₂ 352.0 (MH⁺).

### (Compound 505)

### 8-bromo-2-[(2S)-2,5-dihydro-1H-pyrrol-2-yl][1]benzofuroro[3,2-d]pyrimidin-4(3H)-one

### (S)-tert-butyl 2-(5-bromo-2-carbamoylbenzofuran-3-ylcarbamoyl)-2,5-dihydro-1H-pyrrole-1-carboxylate

The intermediate (S)-tert-butyl 2-(5-bromo-2-carbamoylbenzofuran-3-ylcarbamoyl)-2,5-dihydro-1H-pyrrole-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (S)-1-(tert-butoxycarbonyl)-2,5-dihydro-1H-pyrrole-2-carboxylic acid (BOC-3,4, dehydro-L-proline, NeoMPS) replaced Boc-3-azetidine carboxylic acid. The crude material from this reaction was submitted to the next step without any further purification. MS (EI) for C₁₉ H₂₀ Br N₃ O₅: 451.3 (MH⁺).

### (S)-tert-butyl 2-(8-bromo-4-oxo-3,4-dihydrobenzofuro[3,2-d]pyrimidin-2-yl)-2,5-dihydro-1H-pyrrole-1-carboxylate

(S)-tert-butyl 2-(8-bromo-4-oxo-3,4-dihydrobenzofuro[3,2-d]pyrimidin-2-yl)-2,5-dihydro-1H-pyrrole-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (S)-tert-butyl 2-(2-(aminomethyl)-5-bromobenzofuran-3-ylcarbamoyl)-2,5-dihydro-1H-pyrrole-1-carboxylate replaced 1,1-dimethylethyl 4-(5-bromo-2-carbamoylbenzofuran-3-ylcarbamoyl)piperidine-1-carboxylate. MS (EI) for C₁₉ H₁₈ Br N₃ O₄ : 433 (MH⁺).

### 8-bromo-2-(2S)-2,5-dihydro-1H-pyrrol-2-yl][1]benzofuro[3,2-d]pyrimidin-4(3H)-one

8-bromo-2-[(2S)-2,5-dihydro-1H-pyrrol-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (S)-tert-butyl 2-(8-bromo-4-oxo-3,4-dihydrobenzofuro[3,2-d]pyrimidin-2-yl)-2,5-dihydro-1H-pyrrole-1-carboxylate.¹H NMR (400 MHz, d₆-DMSO): 8.22 (s, 1H), 7.88 (m, 2H), 6.16 (d, 1H), 5.54 (s, 1H), 4.31 (d, 1H), 4.14 (d, 1H). MS (EI) for C₁₄H₁₀BrN₃ O₂: 331.9 (MH⁺).**(Compound** 527)

### 8-Bromo-2-[(2S)-1-methylpyrrolidin-2-yl][1]benzofuro[3,2-d]pyrimidin-4(3H)-one hydrochloride

### N-[2-(aminocarbonyl)-5-bromo-1-benzofuran-3-yl]-L-prolinamide hydrochloride

1,1-Dimethylethyl (2*S*)-2-({[2-(aminocarbonyl)-5-bromo-1-benzofuran-3-yl]amino}carbonyl)pyrrolidine-1-carboxylate (intermediate from Compound 470 synthesis) (1000 mg, 2.22 mmol) was suspended in dioxane (4 mL). A solution of 4 M hydrochloric acid in dioxane (8 mL) was added and the reaction was stirred for 7 hr at room temperature. The reaction mixture was filtered and the precipitate was washed with dioxane (2 mL). 580 mg of product was isolated after drying in air. MS (EI) for C₁₄H₁₄BrN₃O₃: 352 (MH⁺).

### N-[2-(aminocarbonyl)-5-bromo-1-benzofuran-3-yl]-1-methyl-L-porolinamide

*N-*[2-(aminocarbonyl)-5-bromo-1-benzofuran-3-yl]-L-prolinamide hydrochloride (580 mg, 1.49 mmol) was dissolved in dimethylformamide (8 mL). 37% Aqueous formaldehyde (1.0 mL) was added followed by sodium triacetoxyborohydride (630 mg, 2.97 mmol). The reaction mixture was stirred for 10 min at room temperature, then 1M hydrochloric acid was added to lower the pH to 2. The reaction mixture was diluted with water (10 mL) and 1 M sodium hydroxide was added to increase the pH to 8. The precipitate was filtered off, washed with water (5 mL) and air-dried to give 215 mg of white powder. MS (EI) for C₁₅H₁₆BrN₃O₃: 366 (MH⁺).

### 8-Bromo-2-[(2S)-1-methylpyrrolidin-2-yl][1]benzofuro[3,2-d]pyrimidin-4(3H)-one hydrochloride

*N-*[2-(Aminocarbonyl)-5-bromo-1-benzofuran-3-yl]-1-methyl-L-prolinamide was suspended in ethanol (3.6 mL). Sodium hydroxide (1.0 M, 1.8 mL) was added and the reaction mixture was heated at 80 °C for 8 hr. After cooling to room temperature, the reaction mixture was acidified with 1 M hydrochloric acid to pH 3. The precipitate was filtered off and washed with water (2 mL) and acetonitrile (1 mL). 86 mg of product was isolated after drying under air. ¹H NMR (400 MHz, d₆-DMSO): 13.49 (br s, 1H), 10.11 (br s, 1H), 8.20 (s, 1H), 7.89 (d, 1H), 7.85 (dd, 1H), 4.55 (m, 1H), 3.74 (m, 1H), 2.97 (s, 3H), 2.65 (m, 1H), 2.1-2.0 (m, 3H). MS (EI) for C₁₅H₁₄BrN₃O₂: 348 (MH⁺).

### (Compound 49)

### 2-(1-Amino-1-methylethyl)-8-chloror[1]benzofuro[3,2-d]pyrimidin-4(3H)-one 9H-Fluoren-9-ylmethyl (2-{[2-(aminocarbonyl)-5-chloro-1-benzofuran-3-yl]amino}-1,1-dimethyl-2-oxoethyl)carbamate

*N-*{[(9*H*-Fluoren-9-ylmethyl)oxy]carbonyl}-2-methylalanine (15.0 g, 46.1 mmol) was suspended in dichloromethane (200 ml). The reaction was stirred at room temperature overnight. The mixture was concentrated to dryness under vacuum. The residue was taken up in dichloromethane (25 mL) and added to another solution of 3-amino-5-chloro-1-benzofuran-2-carboxamide (1950 mg, 9.26 mmol) in pyridine (50 mL). The reaction mixture was stirred at room temperature overnight. The precipitate was filtered off and washed with pyridine (5 mL) and ethyl acetate (2 mL) to give 6.4 g of crude product. ¹H NMR (400 MHz, d₆-DMSO): 10.59 (s, 1H), 8.29 (d, 1H), 8.16 (s, 1H), 8.01 (s, 1H), 7.91 (s, 1H), 7.86 (d, 2H), 7.71 (d, 2H), 7.60 (d, 1H), 7.51 (dd, 1H), 7.38 (m, 2H), 7.28 (m, 1H), 4.26 (m, 2H), 4.21 (m, 1H), 1.44 (s, 6H). MS (EI) for C₂₆H₂₄ClN₃O₅: 518 (MH⁺).

### 2-(1-Amino-1-methylethyl)-8-chloro[1]benzofuro[3,2-d]pyrimidin-4(3H)-one

9*H*-Fluoren-9-ylmethyl (2-{[2-(aminocarbonyl)-5-chloro-1-benzofuran-3-yl]amino}-1,1-dimethyl-2-oxoethyl)carbamate (crude , 9.3 mmol) was suspended in ethanol (50 mL) and sodium hydroxide (2.0 M, 25 mL) was added. The reaction was stirred at 100 °C overnight. After cooling, the reaction mixture was diluted with water (25 mL) and washed twice with ethyl acetate (25 mL). The aqueous fraction was acidified with concentrated hydrochloric acid to pH 6. The precipitate was filtered off and washed with water (2 mL) and acetonitrile (2 mL). 625 mg of white solid was isolated after drying under vacuum. ¹H NMR (400 MHz, d₆-DMSO): 7.93 (s, 1H), 7.87 (d, 1H), 7.68 (dd, 1H), 1.67 (s, 6H). MS (EI) for C₁₃H₁₂ClN₃O₂: 278 (MH⁺).

### Synthesis of 3-Amino-5-chloro-1-benzofuran-2-carboxamide

### 5-Chloro-2-hydroxybenzaldehyde oxime

5-Chlorosalicylaldehyde (**1**) (10 g, 63.90 mmol) was stirred in isopropyl alcohol (30 mL) to form a thick slurry. 50% Aqueous hydroxylamine (10 mL, 151 mmol), was added to the reaction mixture over the course of 5 minutes, then the suspension was heated to 90°C. After 4 h, the insoluble material dissolved and the reaction mixture was stirred at 90°C overnight. The reaction was allowed to cool to room temperature, which caused a white precipitate to form. The reaction mixture was poured into ice water (100 ml crushed ice, 50 ml water). The white slurry was stirred continually for 5 minutes and was allowed to stand for 1 hour with occasional stirring. The solid was isolated by filtration, washed with cold water (4 x 5 mL) and dried under reduced pressure for 24 h to give 10.87 g (99% yield, >95% purity) of 5-chloro-2-hydroxybenzaldehyde oxime. The material was used without further purification. ¹H NMR (400 MHz, d₆-DMSO): 11.49 (br s,1H), 10.29 (br s, 1H), 8.25 (s, 1H), 7.49 (d, 1H), 7.23 (dd, 1H), 6.86 (d, 1H). MS (EI) for C₇H₆ClNO₂: 172 (MH⁺).

### 5-Chloro-2-hydroxybenzonitrile

5-Chloro-2-hydroxybenzaldehyde oxime (6.11 g, 35.60 mmol) was dissolved in anhydrous N,N-dimethylformamide (3000 mL) under a nitrogen atmosphere. The reaction mixture was cooled in an ice bath to lower the internal temperature to -10°C. Phosphorus oxychloride (8.30 mL, 89 mmol) was added dropwise to the cold reaction mixture over 6 h to maintain the temperature below 0°C. The reaction mixture was allowed to stir overnight as the cold bath slowly warmed to room temperature. The resulting slurry was then poured into a stirred mixture of ice water (100 ml crushed ice, 50 ml water). The aqueous suspension was allowed to stand at room temperature overnight. The solid was collected by filtration, washed with cold water (4 x 10 mL) and dried under reduced pressure for 24 h to give 4.64 g of 5-chloro-2-hydroxybenzonitrile (85% yield, >90% purity). The material was used without further purification. ¹H NMR (400 MHz, d₆-DMSO): 8.28 (s, 1H), 7.53 (s, 1H), 7.22 (d, 1H), 6.91 (d, 1H). MS (EI) for C₇H₄ClNO: 154 (MH⁺).

### 2-(4-Chloro-2-cyanophenoxy)acetamide

5-Chloro-2-hydroxybenzonitrile (4.60 g, 30 mmol) and 2-chloroacetamide (4.03 g, 43.10 mol) were dissolved in anhydrous N, N-dimethylacetamide 50 mL). Cesium carbonate (12.70 g, 38.9 ,mol) was added in portions over 5 min and the reaction mixture was stirred at 80°C overnight. After cooling to room temperature, the reaction mixture was poured into a mixture of ice water (100 mL crushed ice, 50 mL water). The solid was isolated by filtration, washed with cold water (4 x 10 mL) and air dried for 48 hours to give 5.36 g (85% yield, >90% purity) of 2-(4-chloro-2-cyanophenoxy)acetamide. The material was used without further purification. ¹H NMR (400 MHz, d₆-DMSO): 7.91 (dd, 1H), 7.69 (dd, 1H), 7.51 (br s, 1H), 7.44 (br s, 1H), 7.07 (d, 1H), 4.68 (s, 2H). MS (EI) for C₉H₇ClN₂O₂: 211 (MH⁺).

### 3-Amino-5-chloro-1-benzofuran-2-carboxamide

Potassium hydroxide (1.83 g, 32.6 mmol) was dissolved in isopropyl alcohol (40 mL) at 50°C. The solution was diluted with more isopropyl alcohol (10 mL), then 2-(4-chloro-2-cyanophenoxy)acetamide (3.43 g, 16.3 mol) was added followed by more isopropyl alcohol (40 mL). The suspension was stirred at 90°C for 8 hours. After cooling to room temperature, the suspension was poured into a stirred mixture of ice water (100 ml ice, 50 mL water) and allowed to stand for 1 h. The precipitate was collected by filtration, washed with cold water (4 x 10 mL) and air dried for 72 h to give 309 g (90% yield, >95% purity) of 3-amino-5-chloro-1-benzofuran-2-carboxamide. The material was used without further purification. ¹H NMR (400 MHz, d₆-DMSO): 7.94 (d, 1H), 7.42 (d, 2H), 7.34 (br s, 2H), 6.01 (br s, 2H). MS (EI) for C₉H₇ClN₂O₂: 211 (MH⁺).

### (Compound 511)

### 8-bromo-2-[(2S,4S)-4-hydroxypyrrolidin-2-yl][1]benzofuro[3,2-d]pyrimidin-4(3H)-one

### (2R,4S)-tert-butyl 2-(5-bromo-2-carbamoylbenzofuran-3-ylcarbamoyl)-4-hydroxypyrrolidine-1-carboxylate

The intermediate (2*R*,4*S*)-*tert*-butyl 2-(5-bromo-2-carbamoylbenzofuran-3-ylcarbamoyl)-4-hydroxypyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein *N-*boc-cis-4-hydroxypyrrolidine-2-carboxylic acid (commercially available from Omega Chem, Canada) replaced Boc-3-azetidine carboxylic acid. The crude material from this reaction was submitted to the next step without any further purification. MS (EI) for C₁₉H₂₂BrN₃O₆: 469 (MH⁺).

### (2S,4S)-tert-butyl 2-(8-bromo-4-oxo-3,4-dihydrobenzofuro[3,2-d]pyrimidin-2-yl)-4-hydroxypyrrolidine-1-carboxylate

(2*S*,4*S*)-*tert*-butyl2-(8-bromo-4-oxo-3,4-dihydrobenzofuro[3,2-d]pyrimidin-2-yl)-4-hydroxypyrrolidine-1-carboxylate_was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2*R*,4*S*)-*tert*-butyl 2-(5-bromo-2-carbamoylbenzofuran-3-ylcarbamoyl)-4-hydroxypyrrolidine-1-carboxylate replaced 1,1 -dimethylethyl 4-(5-bromo-2-carbamoylbenzofuran-3-ylcarbamoyl)piperidine-1-carboxylate. MS (EI) for C₁₉H₂₀BrN₃O₅: 451 (MH⁺).

### 8-bromo-2-[(2S,4S)-4-hydroxypyrrolidin-2-yl][1]benzofuro[3,2-d]pyrimidin-4(3H)-one

8-bromo-2-[(2S,4S)-4-hydroxypyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2*S*,4*S*)-*tert*-butyl 2-(8-bromo-4-oxo-3,4-dihydrobenzofuro[3,2*-d*]pyrimidin-2-yl)-4-hydroxypyrrolidine-1-carboxylate. ¹H NMR (400 MHz, d₆-DMSO): 10.56 (s, br, 1H), 9.18 (s, br, 1H), 8.21 (m, 1H), 7.88 (M, 2H), 5.45 (s, br, 1H), 4.84 (m, 1H), 4.48 (m, 1H), 3.35 (m, 2H), 2.64 (m, 1H), 2.22 (m, 1H). MS (EI) for C₁₄H₁₂BrN₃O₃: 352 (MH⁺).

### (Compound 512)

### 8-bromo-2-[(2S)-4,4-difluoropyrrolidin-2-yl][1]benzofuro[3,2-d]pyrimidin-4(3H)-one (R)-tert-butyl 2-(5-bromo-2-carbamoylbenzofuran-3-ylcarbamoyl)-4,4 difluoropyrrolidine-1-carboxylate

The intermediate (R)-tert-butyl 2-(5-bromo-2-carbamoylbenzofuran-3-ylcarbamoyl)-4,4-difluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (R)-1-(tert-butoxycarbonyl)-4,4-difluoropyrrolidine-2-carboxylic acid (commercially available from OMEGACHEM, INC.) replaced Boc-3-azetidine carboxylic acid. The crude material from this reaction was submitted to the next step without any further purification. MS (EI) for C₁₉H₂₀BrF₂N₃O₅: 489.3 (MH⁺).

### (R)-tert-butyl 2-(8-bromo-4-oxo-3,4-dihydrobenzofuro[3,2-d]pyrimidin-2-yl)-4,4-difluoropyrrolidine-1-carboxylate

(R)-tert-butyl2-(8-bromo-4-oxo-3,4-dihydrobenzofuro[3,2-d]pyrimidin-2-yl)-4,4-difluoropyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (R)-tert-butyl 2-(5-bromo-2-carbamoylbenzofuran-3-ylcarbamoyl)-4,4-difluoropyrrolidine-1-carboxylate replaced 1,1 -dimethylethyl 4-(5-bromo-2-carbamoylbenzofuran-3-ylcarbamoyl)piperidine-1-carboxylate. MS (EI) for C₁₉H₁₈BrF₂N₃O₄: 471.3 (MH⁺).

### 8-bromo-2-[(2S)-4,4-difluoropyrrolidin-2-yl][1]benzofuro[3,2-d]pyrimidin-4(3H)-one

8-bromo-2-[(2S)-4,4-difluoropyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (R)-tert-butyl 2-(8-bromo-4-oxo-3,4-dihydrobenzofuro[3,2-d]pyrimidin-2-yl)-4,4-difluoropyrrolidine-1-carboxylate. ¹H NMR (400 MHz, d₆-DMSO): 8.22 (s, 1H), 7.88 (m, 2H), 5.14 (t, 1H), 4.03 (m, 1H), 3.89 (m, 1H), 3.13 (m, 1H), 2.93 (m, 1H). MS (EI) for C₁₄H₁₀BrF₂N₃O₂: 371.2 (MH⁺).

### (Compound 524)

### 8-chloro-2-[(2S)-4,4-difluoropyrrolidin-2-yl][1]benzofuro13,2-d]pyrimidin-4(3H)-one (R)-tert-butyl 2-(2-carbamoyl-5-chlorobenzofuran-3-ylcarbamoyl)-4,4-difluoropyrrolidine-1-carboxylate

The intermediate (R)-tert-butyl 2-(2-carbamoyl-5-chlorobenzofuran-3-ylcarbamoyl)-4,4-difluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (R)-1-(tert-butoxycarbonyl)-4,4-difluoropyrrolidine-2-carboxylic acid (commercially available from OMEGACHEM, INC.) replaced Boc-3-azetidine carboxylic acid. The crude material from this reaction was submitted to the next step without any further purification. MS (EI) for C₁₉H₂₀ClF₂N₃O₅: 444.1 (MH⁺).

### (R)-tert-butyl 2-(8-chloro-4-oxo-3,4-dihydrobenzofuro[3,2-d]pyrimidin-2-yl)-4,4-difluoropyrrolidine-1-carboxylate

(R)-tert-butyl2-(8-chloro-4-oxo-3,4-dihydrobenzofuro[3,2-d]pyrimidin-2-yl)-4,4-difluoropyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (R)-tert-butyl 2-(2-carbamoyl-5-chlorobenzofuran-3-ylcarbamoyl)-4,4-difluoropyrrolidine-1-carboxylate replaced 1,1-dimethylethyl 4-(5-bromo-2-carbamoylbenzofuran-3-ylcarbamoyl)piperidine-1-carboxylate. MS (EI) for C₁₉H₁₈ClF₂N₃O₄: 426.1 (MH⁺).

### 8-chloro-2-[(2S]-4,4-difluoropyrrolidin-2-yl][1]benzofuro[3,2-d]pyrimidin-4(3H)-one

8-chloro-2-[(2S)-4,4-difluoropyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (R)-tert-butyl 2-(8-chloro-4-oxo-3,4-dihydrobenzofuro[3,2-d]pyrimidin-2-yl)-4,4-difluoropyrrolidine-1-carboxylate. ¹H NMR (400 MHz, d₆-DMSO): 8.09 (s, 1H), 7.95 (s, 1H), 7.77 (d, 1H), 5.12 (t, 1H), 3.95 (m, 2H), 3.12 (m, 1H), 2.93 (m, 1H). MS (EI) for C₁₄H₁₀ClF₂N₃O₂: 326.0 (MH⁺).

### (Compound 528)

### 8-chloro-2-[(2S,4S)-4-hydroxypyrrolidin-2-yl][1]benzofuro[3,2-d]pyrimidin-4(3H)-one

### (2S,4S)-tert-butyl 2-(2-carbamoyl-5-chlorobenzofuran-3-ylcarbamoyl)-4-hydroxypyrrolidine-1-carboxylate

The intermediate (2S,4S)-tert-butyl 2-(2-carbamoyl-5-chlorobenzofuran-3-ylcarbamoyl)-4-hydroxypyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2S,4S)-4-hydroxypyrrolidine-2-carboxylic acid (commercially available from OMEGACHEM, INC.) replaced Boc-3-azetidine carboxylic acid. The crude material from this reaction was submitted to the next step without any further purification. MS (EI) for C₁₉H₂₂ClN₃O₆: 424.1 (MH⁺).

### (2S,4S)-tert-butyl 2-(8-chloro-4-oxo-3,4-dihydrobenzofuro[3,2-d]pyrimidin-2-yl)-4-hydroxynyrrolidine-1-carboxylate

(2S,4S)-tert-butyl 2-(8-chloro-4-oxo-3,4-dihydrobenzofuro[3,2-d]pyrimidin-2-yl)-4-hydroxypyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2S,4S)-tert-butyl 2-(2-carbamoyl-5-chlorobenzofuran-3-ylcarbamoyl)-4-hydroxypyrrolidine-1-carboxylate replaced 1,1-dimethylethyl 4-(5-bromo-2-carbamoylbenzofuran-3-ylcarbamoyl)piperidine-1-carboxylate. MS (EI) for C₁₉H₂₀ClN₃O₅: 406.1 (MH⁺).

### 8-chloro-2-[(2S,4S)-4-hydroxypyrrolidin-2-yl][1]benzofuro[3,2-d]pyrimidin-4(3H)-one

8-chloro-2-[(2S,4S)-4-hydroxypyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2S,4S)-tert-butyl 2-(8-chloro-4-oxo-3,4-dihydrobenzofuro[3,2-d]pyrimidin-2-yl)-4-hydroxypyrrolidine-1-carboxylate. ¹H NMR (400 MHz, d₆-DMSO): 8.01 (s, 1H), 7.83 (d, 1H), 7.64 (d, 1H), 4.33 (s, 1H), 3.47 (m, 1H), 3.06 (m, 2H), 2.43 (m, 1H), 2.01 (m, 1H). MS (EI) for C₁₄H₁₂ClN₃O₃: 306.1 (MH⁺).

### (Compound 529)

### 8-chloro-2-[(2S)-octahydro-1H-indol-2-yl][1]benzofuro[3,2-d]pyrimidin-4(3H)-one (2S)-tert-butyl 2-(2-carbamoyl-5-chlorobenzofuran-3-ylcarbamoyl)octahydro-1H-indole-1-carboxylate

The intermediate (2S)-tert-butyl 2-(2-carbamoyl-5-chlorobenzofuran-3-ylcarbamoyl)octahydro-1H-indole-1-carboxylate was synthesized in a manner similar to 1,1 -dimethylethyl 3-(5-bromo-2-carbamoylbenzofuran-3-ylcarbamoyl)azetidine-1-carboxylate 4-(tert-butoxycarbonylamino), wherein (2S)-1-(tert-butoxycarbonyl)octahydro-1H-indole-2-carboxylic acid (commercially available from OMEGACHEM, INC.) replaced Boc-3-azetidine carboxylic acid. The crude material from this reaction was submitted to the next step without any further purification. MS (EI) for C₂₃H₂₈CN₃O₅: 462.2 (MH⁺).

### (2S)-tert-butyl 2-(8-chloro-4-oxo-3,4-dihydrobenzofuro[3,2-d]pyrimidin-2-yl)octahydro-1H-indole-1-carboxylate

(2S)-tert-butyl 2-(8-chloro-4-oxo-3,4-dihydrobenzofuro[3,2-d]pyrimidin-2-yl)octahydro-1H-indole-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2S)-tert-butyl 2-(2-carbamoyl-5-chlorobenzofuran-3-ylcarbamoyl)octahydro-1H-indole-1H-carboxylate replaced 1,1-dimethylethyl 4-(5-bromo-2-carbamoylbenzofuran-3-ylcarbamoyl)piperidine-1-carboxylate. MS (EI) for C₂₃H₂₆ClN₃O₄: 444.2 (MH⁺).

### 8-chloro-2-[(2S)-octahydro-1H-indol-2-yl][1]benzofuro[3,2-d]pyrimidin-4(3H)-one

8-chloro-2-[(2S)-octahydro-1H-indol-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2S)-tert-butyl 2-(8-chloro-4-oxo-3,4-dihydrobenzofuro[3,2-d]pyrimidin-2-yl)octahydro-1H-indole-1-carboxylate. ¹H NMR (400 MHz, d₆-DMSO): 8.07 (s, 1H), 7.92 (d, 1H), 7.73 (d, 1H), 4.76 (t, 1H), 3.70 (m, 1H), 2.48 (m, 1H), 2.78 (m, 1H), 1.92 (s, 1H), 1.64 (m, 4H), 1.34 (m, 4H). MS (EI) for C₁₈H₁₈ClN₃O₂: 344.1 (MH⁺).

### (Compound 531)

### 2-[(2S)-4,4-difluoropyrrolidin-2-yl]-8-(methyloxy)[1]benzofuro[3,2-d]pyrimidin-4(3H)-one

### (R)-tert-butyl2-(2-carbamoyl-5-methoxybenzofuran-3-ylcarbamo)-4,4-difluoropyrrolidine-1-carboxylate

The intermediate (R)-tert-butyl 2-(2-carbamoyl-5-methoxybenzofuran-3-ylcarbamoyl)-4,4-difluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (R)-1-(tert-butoxycarbonyl)-4,4-difluoropyrrolidine-2-carboxylic acid (commercially available from OMEGACHEM, INC.) replaced Boc-3-azetidine carboxylic acid. The crude material from this reaction was submitted to the next step without any further purification. MS (EI) for C₂₀H₂F₂N₂O₆: 440.2 (MH⁺).

### (R)-tert-butyl 4,4-difluoro-2-(8-methoxy-4-oxo-3,4-dihydrobenzofuro[3,2-d]pyrimidin-2-yl)pyrrolidine-1-carboxylate

(R)-tert-butyl4,4-difluoro-2-(8-methoxy-4-oxo-3,4-dihydrobenzofuro[3,2-d]pyrimidin-2-yl)pyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein (R)-tert-butyl 2-(2-carbamoyl-5-methoxybenzofuran-3-ylcarbamoyl)-4,4-difluoropyrrolidine-1-carboxylate replaced 1,1 -dimethylethyl 4-(5-bromo-2-carbamoylbenzofuran-3-ylcarbamoyl)piperidine-1-carboxylate. MS (EI) for C₂₀H₂₁F₂N₃O₅: 422.1 (MH⁺).

### 2-[(2S)-4,4-difluoropyrrolidin-2-yl]-8-(methyloxy)[1]benzofuro[3,2-d]pyrimidin-4(3H)-one

2-[(2S)-4,4-difluoropyrrolidin-2-yl]-8-(methyloxy)[1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (R)-tert-butyl 4,4-difluoro-2-(8-methoxy-4-oxo-3,4-dihydrobenzofuro[3,2-d]pyrimidin-2-yl)pyrrolidine-1-carboxylate. ¹H NMR (400 MHz, d₆-DMSO): .80(d, 1H), 7.46 (d, 1H), 7.30 (dd, 1H), 5.14 (t, 1H), 4.03 (m, 1H), 3.89 (m, 1H), 3.13 (m, 1H), 2.93 (m, 1H). MS (EI) for C₁₅H₁₃F₂N₃O₃: 322.2 (MH⁺).

### (Compound 532)

### 2-[(2S,4R)-4-fluoropyrrolidin-2-yl]-8-methyloxy)[1]benzofuro[3,2-d]pyrimidin-4(3H)-one

### (2R,4S)-tert-butyl 2-(2-carbamoyl-5-methoxybenzofuran-3-ylcarbamoyl)-4-fluoropyrrolidine-1-carboxylate

The intermediate (2R,4S)-tert-butyl 2-(2-carbamoyl-5-methoxybenzofuran-3-ylcarbamoyl)-4-fluoropyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2R,4S)-1-(tert-butoxycarbonyl)-4-fluoropyrrolidine-2-carboxylic acid (commercially available from OMEGACHEM, INC.) replaced Boc-3-azetidine carboxylic acid. The crude material from this reaction was submitted to the next step without any further purification. MS (EI) for C₂₀H₂₄FN₃O₆: 422.2 (MH⁺).

### (2R,4S)-tert-butyl 4-fluoro-2-(8-methoxy-4-oxo-3,4-dihydrobenzofuro[3,2-dlpyrimidin-2-yl)pyrrolidine-1-carboxylate

(2R,4S)-tert-butyl4-fluoro-2-(8-methoxy-4-oxo-3,4-dihydrobenzofuro[3,2-d]pyrimidin-2-yl)pyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (2R,4S)-tert-butyl 2-(2-carbamoyl-5-methoxybenzofuran-3-ylcarbamoyl)-4-fluoropyrrolidine-1-carboxylate was substituted for 1,1-dimethylethyl 4-(5-bromo-2-carbamoylbenzofuran-3-ylcarbamoyl)piperidine-1-carboxylate. MS (EI) for C₂₀H₂₂FN₃O₅: 404.1(MH⁺).

### 2-[(2S,4R)-4-fluoropyrrolidin-2-yl]-8-(methyloxy)[1]benzofuro[3,2-d]pyrimidin-4(3H)-one

2-[(2S,4R)-4-fluoropyrrolidin-2-yl]-8-(methyloxy)[1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with (2R,4S)-tert-butyl 4-fluoro-2-(8-methoxy-4-oxo-3,4-dihydrobenzofuro[3,2-d]pyrimidin-2-yl)pyrrolidine-1-carboxylate. ¹H NMR (400 MHz, d₆-DMSO): NMR (400 MHz, d₆-DMSO): 7.80(d, 1H), 7.46(d, 1H), 7.30(dd, 1H), 5.6 (d, 1H), 4.95 (m, 1H), 3.88 (s, 3H), 3.71 (m, 2H), 2.84 (m, 1H), 2.43 (m, 1H). MS (EI) for C₁₅ H₁₄ F N₃ O₃: 304.3 (MH⁺).

### (Compound 494)

### 8-(methyloxy)-2-[(2S)-pyrrolidin-2-yl][1benzofuro[3,2-d]pyrimidin-4(3H)-one 1,1-dimethylethyl (2S)-2-({[2-(aminocarbonyl)-5-(methyloxy)-1-benzofuran-3-yl]amino}carbonyl)pyrrolidine-1-carboxylate

The intermediate 1,1-dimethylethyl (2*S*)-2-({[2-(aminocarbonyl)-5-(methyloxy)-1-benzofuran-3-yl]amino}carbonyl)pyrrolidine-1-carboxylate_was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein N-BOC-*L-*proline (commercially available from ChemImpex) replaced Boc-3-azetidine carboxylic acid. The crude material from this reaction was submitted to the next step without any further purification. MS (EI) for C₂₀H₂₅N₃O₆: 404 (MH⁺).

### 1,1-dimethylethyl (2S)-2-[8-(methyloxy)-4-oxo-3,4-dihydro[1]benzofuro[3,2-d]pyrimidin-2-yl]pyrrolidine-1-carboxylate

1,1-dimethylethyl (2*S*)-2-[8-(methytoxy)-4-oxo-3,4-dihydro[1]benzofuro[3,2*-d]*pyrimidin-2-yl]pyrrolidine-1-carboxylate was synthesized in a similar manner as to 1,1-dimethylethyl 4-(8-bromo-4-oxo-3,4-dihydrobenzofuro[3,2-d]pyrimidin-2-yl)plperidine-1-carboxylate wherein 1,1-dimethylethyl (2*S*)-2-({[2-(aminocarbonyl)-5-(methyloxy)-1-benzofuran-3-yl]amino}carbonyl)pyrrolidine-1-carboxylate was substituted for 1,1-dimethylethyl 4-(5-bromo-2-carbamoylbenzofuran-3-ylcarbamoyl)piperidine-1-carboxylate. ¹H NMR (400 MHz, d₆-DMSO): not collected. MS (EI) for C₂₀H₂₃N₃O₅: 386 (MH⁺).

### 8-(methyloxy)-2-[(2S)-pyrrolidin-2-yl][1]benzofuro[3,2-d]pyrimidin-4(3H)-one

8-(methyloxy)-2-[(2*S*)-pyrrolidin-2-yl][1]benzofuro[3,2*-d*]pyrimidin-4(3*H*)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl (2*S*)-2-[8-(methyloxy)-4-oxo-3,4-dihydro[1]benzofuro[3,2-*d*]pyrimidin-2-yl]pyrrolidine-1-carboxylate. ¹H NMR (400 MHz, d₆-DMSO): 13.45 (b s, 1H), 7.79 (d, 1H), 7.48 (b d, 1H), 7.29 (dd, 1H), 4.77 (app t, 1H), 3.88 (s, 3H), 3.49 (m, 2H), 2.47 (m, 1H), 2.06 (m, 3H). MS (EI) for C₁₅H₁₅N₃O₃ : 286 (MH⁺).

### (Compound 495)

### 8-(methyloxy)-2-[(2R)-pyrrolidin-2-yl][1]benzofuro[3,2-d]pyrimidin-4(3H)-one 1,1-dimethylethyl (2R)-2-({[2-(aminocarbonyl)-5-(methyloxy)-1-benzofuran-3-yl]amino}carbonyl)pyrrolidine-1-carboxylate

The intermediate 1,1-dimethylethyl (2*R*)-2-({[2-(aminocarbonyl)-5-(methyloxy)-1-benzofuran-3-yl]amino}carbonyl)pyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein *N-*BOC-D-proline (commercially available from ChemImpex) replaced Boc-3-azetidine carboxylic acid. The crude material from this reaction was submitted to the next step without any further purification. MS (EI) for C₂₀H₂₅N₃O₆: 404 (MH⁺).

### 1,1-dimethylethyl (2R)-2-[8-(methyloxy)-4-oxo-3,4-dihydro[1]benzofuro[3,2-d]pyrimidin-2-yl]pyrrolidine-1-carboxylate

1,1-dimethylethyl (2*R*)-2-[8-(methyloxy)-4-oxo-3,4-dihydro[1]benzofuro[3,2*-d*]pyrimidin-2-yl]pyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl (2*R*)-2-({[2-(aminocarbonyl)-5-(methyloxy)-1-benzofuran-3-yl]amino}carbonyl)pyrrolidine-1-carboxylate was substituted for 1,1-dimethylethyl 4-(5-bromo-2-carbamoylbenzofuran-3-ylcarbamoyl)piperidine-1-carboxylate. ¹H NMR (400 MHz, d₆-DMSO): not collected. MS (EI) for C₂₀H₂₃N₃O₅: 386 (MH⁺).

### 8-(methyloxy)-2-[(2R)-pyrrolidin-2-yl][1]benzofuro[3,2-d]pyrimidin-4(3H)-one

8-(methyloxy)-2-[(2*R*)-pyrrolidin-2-yl][1]benzofuro[3,2*-d*]pyrimidin-4(3*H*)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl (2*R*)-2-[8-(methyloxy)-4-oxo-3,4-dihydro[1]benzofuro[3,2*-d*]pyrimidin-2-yl]pyrrolidine-1-carboxylate. ¹H NMR (400 MHz, d₆-DMSO): 13.45 (b s, 1H), 7.8 (d, 1H), 7.48 (b d, 1H), 7.29 (dd, 1H), 4.77 (b s, 1H), 3.87 (s, 3H), 3.48 (m, 2H), 2.46 (m, 1H), 2.09 (m, 3H). MS (EI) for C₁₅H₁₅N₃O₃: 286 (MH⁺).

### (Compound 508)

### 8-bromo-2-{1-[(3S)-3-hydroxypyrrolidin-1-yl]ethyl}[1]benzofuro[3,2-d]pyrimidin-4(3H)-one

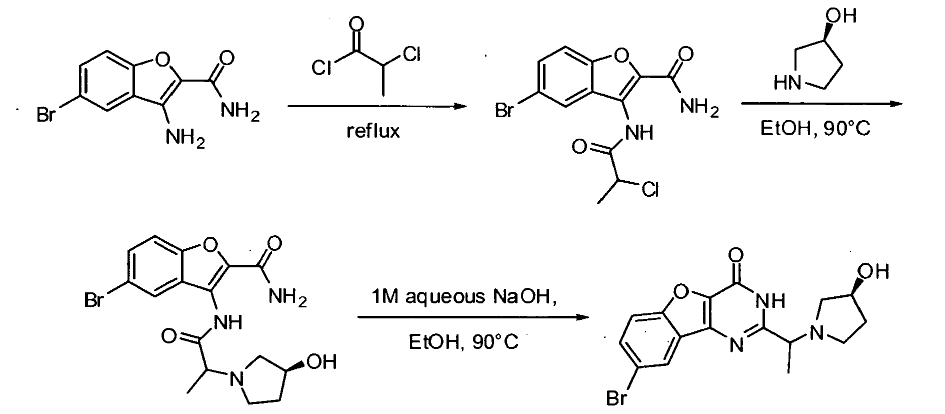

### 5-bromo-3-[(2-chloropropanoyl)amino]-1-benzofuran-2-carboxamide

A solution of 3-amino-5-bromobenzofuran-2-carboxamide 3 (1.02 g, 3.92 mmol) in 2-chloropropionyl chloride (10 mL) was heated to reflux overnight. The reaction mixture was cooled and concentrated to give a brown solid. The brown solid was triturated with ethyl acetate to afford 0.956 g of crude 5-bromo-3-[(2-chloropropanoyl)amino]-1-benzofuran-2-carboxamide as an off-white solid. This material was carried on without further purification. MS (EI) for C₁₂H₁₀BrClN₂O₃: 345 (M⁺).

### 5-bromo-3-({2-[(3S)-3-hydroxypyrrolidin-1-yl]propanoyl}amino)-1-benzofuran-2-carboxamide

To a solution of 5-bromo-3-[(2-chloropropanoyl)amino]-1-benzofuran-2-carboxamide (956 mg, 2.77 mmol) in 14 mL anhydrous ethanol was added (*S*)-3-hydroxypyrrolidine (0.7 mL, 8.31 mmol). The reaction mixture was heated to 90 °C for 5 hours, cooled down and concentrated *in vacuo.* Crude 5-bromo-3-({2-[(3*S*)-3-hydroxypyrrolidin-1-yl]propanoyl}amino)-1-benzofuran-2-carboxamide was carried on without further purification. MS (EI) for C₁₆H₁₈BrN₃O₄: 396 (M⁺).

### 8-bromo-2-{1-[(3S)-3-hydroxypyrrolidin-1-yl]ethyl}[1]benzofuro[3,2-d]pyrimidin-4(3H)-one

To a crude solution of 5-bromo-3-({2-[(3*S*)-3-hydroxypyrrolidin-1-yl]propanoyl}amino)-1-benzofuran-2-carboxamide (2.77 mmol) in 14 mL of ethanol was added 1M aqueous NaOH (8.5mL) and heated to 120 °C overnight. The reaction mixture was brought to pH ~ 4 with 1N HCl and concentrated. Purification by preparative HPLC (reverse-phase, 0.1 % TFA in acetonitrile/0.05% TFA in water), followed by concentration *in vacuo* and lyophilization afforded 8-bromo-2-{1-[(3*S*)-3-hydroxypyrrolidin-l-yl]ethyl}[1]benzofuro[3,2*-d*]pyrimidin-4(3*H*)-one (372.5 mg, 35%) as a tan solid. ¹H NMR (400 MHz, d₆-DMSO): 8.17 (m, 1H), 7.78 (m, 2H), 4.19 (b s , 1H), 3.62 (m, 1H), 2.84 (m, 1H), 2.65 (m, 2H), 2.45 (m, 1H), 1.98 (m, 1H), 1.59 (m, 1H), 1.41 (app dd, 3H); MS (EI) for C₁₆H₁₆BrN₃O₃: 378 (M⁺).

### (Compound 509)

### 2-[(2S)-azetidin-2-yl]-8-bromo[1]benzofuro[3,2-d]pyrimidin-4(3H)-one 1,1-dimethylethyl (2S)-2-({[2-(aminocarbonyl)-5-bromo-1-benzofuran-3-yl]amino}carbonyl)azetidine-1-carboxylate

The intermediate 1,1-dimethylethyl (2*S*)-2-({[2-(aminocarbonyl)-5-bromo-1-benzofuran-3-yl]amino}carbonyl)azetidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein *N-*BOC-3-azetidine carboxylic acid (commercially available from ChemImpex) replacedBoc-3-azetidine carboxylic acid. The crude material from this reaction was submitted to the next step without any further purification. MS (EI) for C₁₈H₂₀BrN₃O₅: 438 (M⁺).

### 1,1-dimethylethyl (2S)-2-(8-bromo-4-oxo-3,4-dihydro[1]benzofuro[3,2-d]pyrimidin-2-yl)azetidine-1-carboxylate

1,1-dimethylethyl (2*S*)-2-(8-bromo-4-oxo-3,4-dihydro[1]benzofuro[3,2-d]pyrimidin-2-yl)azetidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl (2*S*)-2-({[2-(aminocarbonyl)-5-bromo-1-benzofuran-3-yl]amino carbonyl)azetidine-1-carboxylate replaced 1,1-dimethylethyl 4-(5-bromo-2-carbamoylbenzofuran-3-ylcarbamoyl)piperidine-1-carboxylate. ¹H NMR (400 MHz, d₆-DMSO): not collected. MS (EI) for C₁₈H₁₈BrN₃O₄: 420 (M⁺).

### 2-[(2S)-azetidin-2-yl]-8-bromo[1]benzofuro[3,2-d]pyrimidin-4(3H)-one

2-[(2*S*)-azetidin-2-yl]-8-bromo[1]benzofuro[3,2*-d*]pyrimidin-4(3*H*)-one was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate replaced 1,1-dimethylethyl (2*S*)-2-(8-bromo-4-oxo-3,4-dihydro[1]benzofuro[3,2*-d*]pyrimidin-2-yl)azetidine-1-carboxylate. ¹H NMR (400 MHz, d₆-DMSO): 10.03 (b s, 1H), 8.23 (b d, 1H), 7.89 (m, 2H), 5.25 (app t, 1H), 4.08 (app q, 1H), 3.95 (app q, 1H), 2.81 (app q, 2H). MS (EI) for C₁₃H₁₀BrN₃O₂: 320 (M⁺).

### *(Compound 510)

### 8-bromo-2-[(2S)-2,3-dihydro-1H-indol-2-yl][1]benzofuro[3,2-d]pyrimidin-4(3H)-one 1,1-dimethylethyl (2S)-2-({[2-(aminocarbonyl)-5-bromo-1-benzofuran-3-yl]amino}carbonyl)-2,3-dihydro-1H-indole-1-carboxylate

The intermediate 1,1-dimethylethyl (2*S*)-2-({[2-(aminocarbonyl)-5-bromo-1-benzofuran-3-yl]amino}carbonyl)-2,3-dihydro-1*H*-indole-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein (2*S*)-1- {[(1,1-dimethylethyl)oxy]carbonyl}-2,3-dihydro-1*H*-indole-2-carboxylic acid (commercially available from ChemImpex) replaced Boc-3-azetidine carboxylic acid. The crude material from this reaction was submitted to the next step without any further purification. MS (EI) for C₂₃H₂₂BrN₃O₅: 501 (MH⁺)_{.}

### 1,1-dimethylethyl(2S)-2-(8-bromo-4-oxo-3,4-dihydro[1]benzofuro[3,2-d]pyrimidin-2-yl)-2,3-dihydro-1H-indole-1-carboxylate

1,1-dimethylethyl (2*S*)-2-(8-bromo-4-oxo-3,4-dihydro[1]benzofuro[3,2-d]pyrimidin-2-yl)-2,3-dihydro-1*H*-indole-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl (2*S*)-2-({[2-(aminocarbonyl)-5-bromo-1-benzofuran-3-yl]amino}carbonyl)-2,3-dihydro-1*H-*indole-1-carboxylate replaced 1,1-dimethylethyl 4-(5-bromo-2-carbamoylbenzofuran-3-ylcarbamoyl)piperidine-1-carboxylate. ¹HNMR (400 MHz, d₆-DMSO): not collected. MS (EI) for C₂₃H₂₀BrN₃O₄: 482 (M⁺).

### 8-bromo-2-[(2S)-2,3-dihydro-1H-indol-2-yl][1]benzofuro[3,2-d]pyrimidin-4(3H)-one

8-bromo-2-[(2*S*)-2,3-dihydro-1*H*-indol-2-yl][1]benzofuro[3,2-*d*]pyrimidin-4(3*H*)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl (2*S*)-2-(8-bromo-4-oxo-3,4-dihydro[1]benzofuro[3,2*-d*]pyrimidin-2-yl)-2,3-dihydro-1*H*-indole-1-carboxylate. ¹H NMR (400 MHz, d₆-DMSO): 8.16 (m, 1H), 7.85 (m, 2H), 7.11 (m, 2H), 6.78 (m, 2H), 5.03 (app t, 1H), 4.94 (b s, 1H), 3.45 (m, 2H). MS (EI) for C₁₈H₁₂BrN₃O₂: 382 (M⁺).

### (Compound 523)

### 8-bromo-2-[(2S)-5-oxopyrrolidin-2-yl][1]benzofuro[3,2-d]pyrimidin-4(3H)-one 1,1-dimethylethyl (2S)-2-({[2-(aminocarbonyl)-5-bromo-1-benzofuran-3-yl]amino}carbonyl)-5-oxopyrrolidine-1-carboxylate

The intermediate 1,1-dimethylethyl (2*S*)-2-({[2-(aminocarbonyl)-5-bromo-1-benzofuran-3-yl]amino}carbonyl)-5-oxopyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), wherein 1-{[(1,1-dimethylethyl)oxy]carbonyl}-5-oxo-L-proline (commercially available from ChemImpex) replaced Boc-3-azetidine carboxylic acid. The crude material from this reaction was submitted to the next step without any further purification. MS (EI) for C₁₉H₂₀BrN₃O₆: 466 (M⁺).

### N-[2-(aminocarbonyl)-5-bromo-1-benzofuran-3-yl]-5-oxo-L-prolinamide

*N-*[2-(aminocarbonyl)-5-bromo-1-benzofuran-3-yl]-5-oxo-L-prolinamide was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl (2*S*)-2-({[2-(aminocarbonyl)-5-bromo-1-benzofuran-3-yl]amino}carbonyl)-5-oxopyrrolidine-1-carboxylate. ¹H NMR (400 MHz, d₆-DMSO): not collected. MS (EI) for C₁₄H₁₂BrN₃O₄: 366 (M⁺).

### 8-bromo-2-[(2S)-5-oxopyrrolidin-2-yl][1]benzofuro[3,2-d]pyrimidin-4(3H)-one

8-bromo-2-[(2,5)-5-oxopyrrolidin-2-yl][1]benzofuro[3,2*-d*]pyrimidin-4(3*H*)-one was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein *N-*[2-(aminocarbonyl)-5-bromo-1-benzofuran-3-yl]-5-oxo-L-prolinamide replaced 1,1-dimethylethyl 4-(5-bromo-2-carbamoylbenzofuran-3-ylcarbamoyl)piperidine-1-carboxylate. ¹H NMR (400 MHz, d₆-DMSO): not collected. MS (EI) for C₁₄H₁₀Br N₃O₃: 348 (M⁺).

### EXAMPLE 29

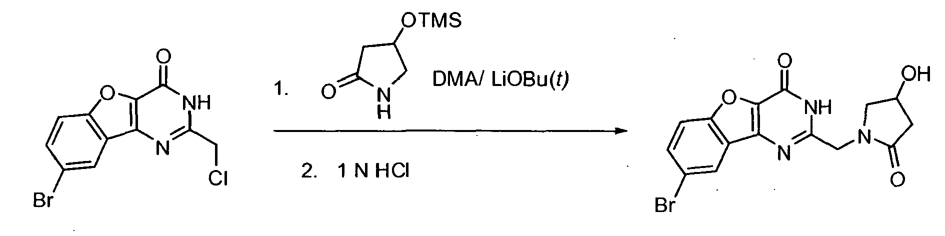

### (Compound 286)

### 8-bromo-2-[(4-hydroxy-2-oxopyrrolidin-1-yl)methyl][1]benzofuro[3,2-d]pyrimidin-4(3H)-one

To a solution of 4-(trimethylsilyloxy)pyrrolidin-2-one (166 mg, 1.6 mmol, reference: synthesis, 1978, 614-617) in DMA (5mL) was added lithium *tert*-butoxide (128 mg, 1.6 mmol) at rt. The recation mixture was stirred at rt for 20 min, 8-bromo-2-(chloromethyl)benzofuro[3,2-d]pyrimidin-4(3H)-one 6 (200 mg, 0.64 mmol) was added to the solution. The mixture was stirred for 3 hours. 1 N aquous HCl solution (5 mL) was added, and the reaction was stirred 2 hours at rt and concentrated. Purification by preparative HPLC afforded 8-bromo-2-[(4-hydroxy-2-oxopyrrolidin-1-yl)methyl][1]benzofuro[3,2-d]pyrimidin-4(3H)-one (60 mg). ¹H NMR (400 MHz, d₆-DMSO): 8.20 (s, 1H), 7.82 (s, 2H), 4.61 (d, 1H), 4.45-4.37 (m, 2H), 3.86-3.76 (m, 1H), 3.35 (dd, 1H), 2.70 (dd, 1H), 2.19 (dd, 1H); MS (EI) for C₁₅H₁₂BrN₃O₄: 379 (MH⁺).

### (Compound 287)

### 8-bromo-2-{[3-(hydroxymethyl)pyrrolidin-1-yl]methyl}[1]benzofuro[3,2-d]pyrimidin-4(3H)-one

To a solution of 3-hydroxymethyl-pyrroldine-1-carboxylic acid tert-butyl ester (201 mg, 1 mmol) in EtOAc (5 mL) was added 4 N HCl in dioxane (2 mL). The reaction mixture was stirred at room temp overnight and concentrated *in vacuo.* The residue was dissolved in ethanol (5 mL), followed by 8-bromo-2-(chloromethyl)benzofuro[3,2-d]pyrimidin-4(3H)-one 6 (1 mmol) and triethylamine (1 ml). The mixture was heated at 50 °C for 5 hours and concentrated. The residue was purified by preparative HPLC to afford 8-bromo-2-{[3-(hydroxymethyl)pyrrolidin-1-yl]methyl}[1]benzofuro[3,2-d]pyrimidin-4(3H)-one (142 mg). ¹H NMR (400 MHz, d₆-DMSO): 8.22 (s, 1H), 7.82 (s, 2H), 3.90 (s, 2H), 3.40-3.30 (m, 3H), 3.00-2.80 (m, 2H), 2.65-2.60 (m, 1H), 2.40-2.30 (m, 1H), 1.95-1.85 (m, 1H), 1.55-1.46 (m, 1H);MS (EI) for C₁₇H₁₆BrN₃O₃: 390 (MH⁺); MS (EI) for C₁₆H₁₆BrN₃O₃: 378 (MH⁺).

### (Compound 288)

### 8-bromo-2-{[(pyrrolidin-3-ylmethyl)oxy]methyl}[1]benzofuro[3,2-d]pyrimidin-4(3H)-one

To a solution of 3-hydroxymethyl-pyrroldine-1-carboxylic acid tert-butyl ester (201 mg, 1 mmol) in DMF (5 mL) was added NaH (50 mg, 60% in oil). After stirring at rt for 10 min, 8-bromo-2-(chloromethyl)benzofuro[3,2-d]pyrimidin-4(3H)-one 6 (155 mg, 0.5 mmol) was added in one portion. The reaction mixture was stirred at rt for 1 hour. 4 N HCl in dioxane (2 mL) was added, the reaction was heated to 50 °C for 2 hours. The reaction mixture was filtered, the filtrate was concentrated. Purification of the residue by preparative HPLC afforded 128 mg of 8-bromo-2-{[(pyrrolidin-3-ylmethyl)oxy]methyl}[1]benzofuro[3,2-d]pyrimidin-4(3H)-one. ). ¹H NMR (400 MHz, d₆-DMSO): 8.37 (s, 1H), 8.17 (s, 1H), 7.79 (s, 2H), 4.45 (dd, 2H), 3.58 (d, 2H), 3.27-3.18 (m, 2H), 3.16-3.09 (m, 2H), 2.62-2.58 (m, 1H), 2.09-2.95 (m, 1H), 1.78-1.69 (m, 1H); MS (EI) for C₁₇H₁₆BrN₃O₃: 390 (MH⁺); MS (EI) for C₁₆H₁₆BrN₃O₃: 378 (MH⁺).

### EXAMPLE 30

### (Compound 346)

### 8-bromo-2-[(3-hydroxy-3-methylpyrrolidin-1-yl)methyl][1]benzofuro[3,2-d]pyrimidin-4(3H)-one

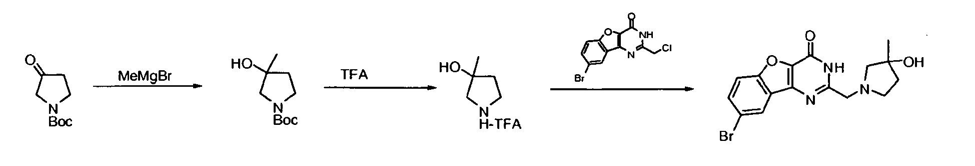

### Preparation of 3-methylpyrrolidin-3-ol

To a solution of *N-tert*-butoxycarbonyl-3-pyrrolidinone (1.0 g, 5.4 mmol) in THF (10 mL) was added methylmagnesium bromide (3 M in ether, 6 mL) dropwise at 0 °C. The reaction mixture was allowed to warm up to rt and stirred another 2 hours. The reaction was quenched with water (2 mL). The reaction mixture was partitioned between EtOAc and water. The organic layer was washed with brine, dried and concentrated. The residue was dissoved in TFA (3 mL), stirred at rt for 3 hours and concentrated to a black solid, which was used to the preparation of 8-bromo-2-[(3-hydroxy-3-methylpyrrolidin-1-yl)methyl][1]benzofuro[3,2-d]pyrimidin-4(3H)-one without further purification.

To a solution of 8-bromo-2-(chloromethyl)[1]benzofuro[3,2-d]pyrimidin-4(3H)-one **6** (100 mg, 0.32 mmol) in 5 mL anhydrous ethanol was added crude 3-hydroxypyrrolidine-3-carboxylic acid (500 mg, excess, prepared above) and triethylamine (2 mL). The reaction mixture was heated to 80 °C overnight. The reaction was cooled and concentrated. The purification of the residue by preparative HPLC gave the title compound as a white solid. ¹H NMR (400 MHz, d₆-DMSO): 8.21 (m, 1H), 7.82 (m, 2H), 3.78 (dd, 2H), 2.92 (dd, 1H), 2.65 (d, 1H), 2.55 (m, 2H), 1.79 (t, 1H), 1.24 (s, 3H); MS (EI) for C₁₆H₁₄BrN₃O₃: 378 (MH⁺).

### Preparation of cis-3,4-dihydroxypyrrolidine:

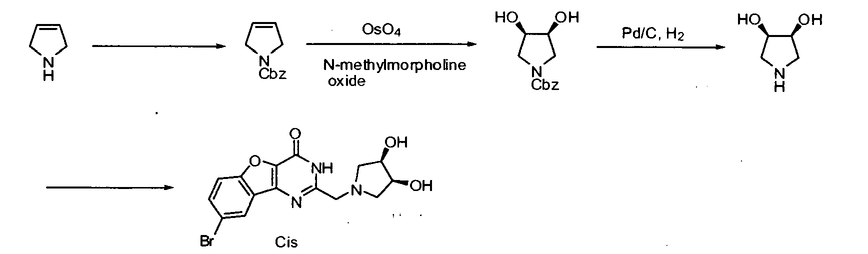

cis-3,4-dihydroxypyrrolidine

To a solution of benzyl chloroformate (3.4 g, 20 mmol) in DCM (10 mL) was added 3-pyrroline (1.46 g) dropwise at) 0°C. After stirring overnight at rt, the reaction mixture was washed with 0.5 N HCl and saturate aq. NaHCO₃ solution. The organic layer was dried and concentrated to give 1-(benzyloxycarbarbonyl)-3-pyrroline (2.5 g) as a white solid. ¹H NMR (400 MHz, CDCl₃): 7.42-7.28 (m, 5H), 5.80 (m, 2H), 5.17 (s, 2H), 4.20 (m, 4H).

1-(benzyloxycarbarbonyl)-3-pyrroline (1.02 g, 5mmol) was dissolved in THF (20 mL) and treated with OsO₄ (2 mL, 4% in water) followed by N-methylmorpholine N-oxide (585 mg, 5 mmol). After 5 hours, LC-MS indicated the reaction is complete. The solvent was evoporated, the residue was dissolved in EtOAc and washed with diluted Na₂SO₃ solution, saturated NaHCO₃, brine and dried. Concentration and purification by silica gel chromatography gave 630 mg of 1-(benzyloxycarbarbonyl)-cis-3,4-dihydroxypyrrolidine. ¹H NMR (400 MHz, CDCl₃): 7.40-7.28 (m, 5H), 5.16 (s, 2H), 4.27 (m, 2H), 3.65 (m, 2H), 3.42 (m, 2H), 2.63 (m, 2H).

A round-bottomed flask was charged with 1-(benzyloxycarbarbonyl)-cis-3,4-dihydroxypyrrolidine (600 mg), ethanol (30 mL), Pd/C (10% in weight, wet, 100 mg) and hydrogen balon. The reaction was stirred at rt overnight. LC-MS indicated the starting material was dissappeared. The reaction mixture was filtered, concentrated. The residue was used to next step without further purification. ¹H NMR (400 MHz, CDCl₃): 4.21 (m, 2H), 3.60 (m, 2H), 3.21 (m, 2H), 2.80 (m, 2H).

### (Compound 407)

### 8-bromo-2-[(3-ethyl-3-hydroxypyrrolidin-1-yl)methyl][1]benzofuroro[3,2-d]pyrimidin-4(3H)-one

8-bromo-2-[(3-ethyl-3-hydroxypyrrolidin-1-yl)methyl][1]benzofuro[3,2-d]pyrimidin-4(3H)-one was synthesized in a manner similar to **Compound 346,** wherein methylmagnesium bromide was substituted with ethylmagnesium bromide. The purification of the residue by preparative HPLC gave the title compound as a white solid. ¹H NMR (400 MHz, CDOl₃): 8.21 (s, 1H), 7.78 (d, 1H), 7.75 (d, 1H), 4.06 (dd, 2H), 3.35 (m, 1H), 3.05-2.95 (m, 2H), 2.90 (d, 1H), 2.08-1.91 (m 2H), 1.70 (q, 2H), 0.95 (t, 3H); MS (EI) for C₁₇H₁₈BrN₃O₃: 392 (MH⁺).

### EXAMPLE 31

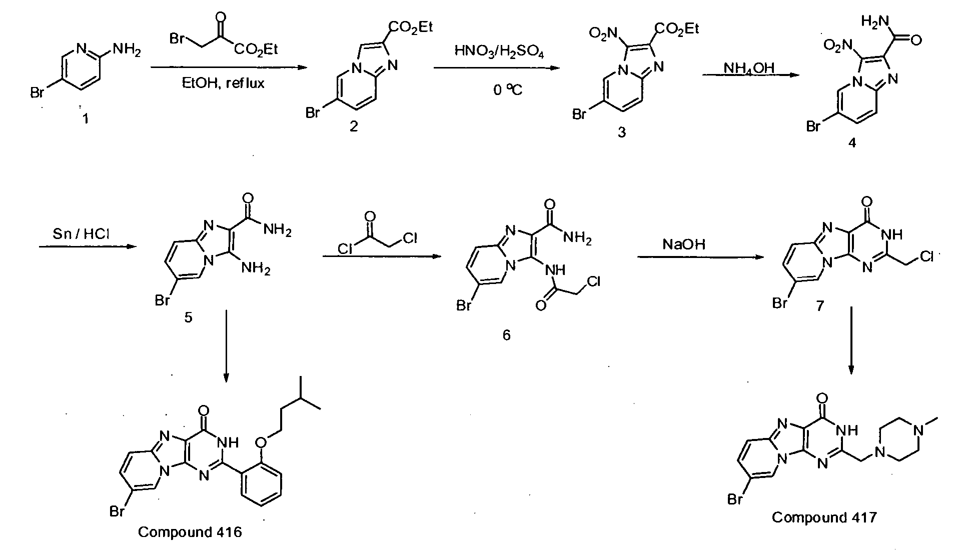

Ethyl 6-bromoimidazo[1,2-a]pyridine-2-carboxylate, **2,** was synthesized according to the reference: J. Org. Chem. 1965, 2403-2407.

Ethyl 6-bromo-3-nitroimidazo[1,2-a]pyridine-2-carboxylate, **3,** was **synthesized** according to the reference: J. Org. Chem. 1981, 46, 1026-1030. ¹H NMR (400 MHz, CDCl₃): 9.55 (s, 1H), 7.80 (s, 1H), 7.25 (s, 1H), 4.58 (q, 2H), 1.45 (t, 3H).

### 6-bromo-3-nitroimidazo[1,2-a]pyridine-2-carboxamide 4

To a solution of ethyl 6-bromo-3-nitroimidazo[1,2-a]pyridine-2-carboxylate, 3, (3.13 g, 10 mmol) in THF (40 mL) was added concentrated ammonium hydroxide (100 mL). The reaction was stirred at rt for 4 days. The suspension was filtered to give 6-bromo-3-nitroimidazo[1,2-a]pyridine-2-carboxamide (2.2 g, 78% yield) as a pale yellow solid. ¹H NMR (400 MHz, CDCl₃): 9.40 (s, 1H), 8.18 (s, 1H), 8.03 (m, 3H).

### 3-amino-6-bromoimidazo[1,2-a]pyridine-2-carboxamide 5

To hydrobronic acid (24 mL) cooled to -10 °C was added tin (3.23 g, 26.2 mmol). 6-bromo-3-nitroimidazo[1,2-a]pyridine-2-carboxamide (3.1 g, 10.8 g) was added partionwise to avoid a temperature of more than 5 °C. The mixture was stirred at 0°C for 1 hour, allowed to stand at rt and stirred another 1 hour. The suspension was filtered, the filtrate was made basic with half saturated sodium carbonate. The suspension was stirred for a few min., then filtered to give 3-amino-6-bromoimidazo[1,2-a]pyridine-2-carboxamide as pale yellow solid (6.0 g).¹H NMR (400 MHz, d6-DMSO): 8.90 (s, 1H), 7.80 (m, 2H)

### *(Compound 419)

### 8-bromo-2-(chloromethyl)pyrido[1,2-e]purin-4(3H)-one

To a solution of 3-amino-6-bromoimidazo[1,2-a]pyridine-2-carboxamide (1.0 g) in DMA (10 mL) was added 2-chloroacetyl chloride (20 mL) at rt. The reaction mixture was heated to 40 °C for 5 hours. LC-MS indicated that the reaction is complete. The reaction was concentrated to remove 2-chloroacetyl chloride *in vacuo.* To the remaining DMA solution was added 1 N NaOH (20 mL). After stirring at 40 °C for 1 hours, the reaction was complete. The reaction was cooled and neutralized with 3 N HCl to PH 5-6, then extracted with EtOAc. The organic layer was separated and dried. The residue was used to next stpe without purification. ¹H NMR (400 MHz, d6-DMSO): 10.63 (s, 1H), 87.42 (s, 1H), 7.75 (s, 1H), 7.60 (m, 1H), 7.50 (m, 2H), 4.45 (s, 2H).

### (Compound 417)

### 8-bromo-2-[(4-methylipiperazin-1-yl)methyl]pyridor[1,2-e]purin4(3H)-one

To a solution of 8-bromo-2-(chloromethyl)pyrido[1,2-e]purin-4(3H)-one **(Compound 419)** (100 mg, 0.10mmol) in 3 mL anhydrous ethanol was added 1-methylpiperazine (3 eq.). The reaction mixture was heated to 80 °C for 2 hours, cooled down and concentrated *in vacuo.* Purification by preparative HPLC resulting in 8-bromo-2-[(4-methylpiperazin-1-yl)methyl]pyrido[1,2-e]purin-4(3H)-one as a solid. ¹H NMR (400 MHz, d₆-DMSO): 8.85 (s, 1H), 8.27 (s, 2H), 7:70 (d, 1H), 7.60 (d, 1H), 3.60 (m, 4H), 2.35 (m, 4H), 2.17 (s, 3H); MS (EI) for C₁₅H₁₇BrN₆O: 377 (MH⁺).

### (Compound 418)

### 8-bromo-2-{(3S)-3-hydroxyrrolidin-1-yl]methyl}pyrido[1,2-e]purin-(3H)-one

8-bromo-2-{[(3S)-3-hydroxypyrrolidin-1-yl]methyl}pyrido[1,2-e]purin-4(3H)-one was synthesized in a manner similar to Compound 417, wherein 1-methylpiperazine was substituted with S-(-)3-hydroxypyrrolidine. Preparative HPLC purification gave the title compound as a white solid. ¹H NMR (400MHz, d₆-DMSO): 8.82 (s, 1H), 8.25 (s, 1H), 7.68 (d, 1H), 7.59 (d, 1H), 4.19 (s, 2H), 3.70 (m, 1H), 2.78 (m, 2H), 2.00 (m, 2 H), 1.80 (m, 2H); MS (EI) for C₁₄H₁₄BrN₅O₂: 364 (MH⁺).

### *(Compounds 416)

### 8-bromo-2-{2-[(3-methylbutyl)oxy]phenyl}pyrido[1,2-e]purin-4(3H)-one

A solution of 3-amino-6-bromoimidazo[1,2-a]pyridine-2-carboxamide (200 mg, 0.75 mmol) and 2-(isopentyloxy)benzaldehyde (200 mg) in 6 mL anhydrous ethanol and 10 mL DMA was heated at 80 °C overnight. Then sodium bisulfite (300) and DMSO (3 mL) was added and the reaction was heated at 150 °C for 2 days. After cooling, the reaction mixture was filtered and the filtrate was purified by preparative HPLC to give the title compound as a white solid. ¹H NMR (400 MHz, d₆-DMSO): 12.20 (s, 1H), 8.95 (s, 1H), 7.98 (d, 1H), 7.68 (d, 1H), 7.60 (dd, 1H), 7.57 (m, 1H), 7.23 (d, 1H), 7.10 (t, 1H), 4.15 (t, 2H), 1.80-1.65 (m, 3H), 0.90 (s, 6H); MS (EI) for C₂₀H₁₉BrC1N₄O₂: 327 (MH⁺).

### (Compound 450)

### 8-(3-hydroxypron-1-yn-1-yl)-2-[(4-methylpiperazin-1-yl)methyl][1]benzofuro[3,2-d]pyrimidin-4(3H)-one

8-(3-hydroxyprop-1-yn-1-yl)-2-[(4-methylpiperazin-1-yl)methyl][1]benzofuro[3,2-d]pyrimidin-4(3H)-one was synthesized in a manner similar to **Example 31** wherein 8-bromo-2-[(4-methylpiperazin-1-yl)methyl][1]benzofuro[3,2-d]pyrimidin-4(3H)-one **Compound 3** was substituted with 8-bromo-2-{[(3S)-3-hydroxypyrrolidin-1-yl]methyl}[1]benzofuro[3,2-d]pyrimidin-4(3H)-one . ¹H NMR (400 MHz, d6-DMSO): 7.98 (s, 1H), 7.79 (m. 1H), 7.64 (m, 1H), 4.32 (s, 2H), 3.48 (s, 2H), 2.31 (m, 4H), 2.12 (s, 3H), 1.82 (m, 6H). MS (EI) for C19 H20 N4 03: 353 (MH+).

### (Compound 456)

### 8-(6-hydroxyhex-1-yl-1-yl)-2- {(3S)-3-hydroxylyrrolidin-1-yl]methyl}[1]benzofuro[3.2-d]pyrimidin-4(3H)-one

8-(6-hydroxyhex-1-yn-1-yl)-2-{[(3S)-3-hydroxypyrrolidin-1-yl]methyl}[1]benzofuro[3,2-d]pyrimidin-4(3H)-one was synthesized in a manner similar to **Example 31** wherein hex-5-yn-1-ol was substituted with propargyl alcohol. ¹H NMR (400 MHz, d6-DMSO): 7.96 (m, 1H), 7.79 (m, 1H), 7.62 (m, 1H), 4.21 (s, 1H), 3.68 (m, 2H), 3.46 (m, 2H), 2.80 (m, 2H), 2.54 (m, 2H), 2.47 (m, 2H), 2.01 (m, 1H), 1.61 (m, 5H). MS (EI) for C21 H23 N3 O4: 382 (MH+).

### (Compound 458)

### 8-ethynyl-2-{[(3S)-3-hydroxypyrrolidin-1-yl]methyl}[1]benzofuro[3,2-d]pyrimidin-4(3H)-one

8-ethynyl-2-{[(3S)-3-hydroxypyrrolidin-1-yl]methyl}[1]benzofuro[3,2-d]pyrimidin-4(3H)-one was synthesized in a manner similar to **Example 31** wherein trimethylsilylacetylene was substituted with propargyl alcohol. ¹H NMR (400 MHz, d6-DMSO): 8.08 (s, 1H), 7.82 (d, 1H), 7.71 (d, 1H), 4.26 (s, 1H), 4.19 (s, 1H), 3.67 (m, 2H), 2.80 (m, 2H), 2.53 (m, 2H), 2.01 (m, 1H), 1.59 (m, 1H). MS (EI) for C17 H15 N3 O3: 310 (MH+).

### (Compound 459)

### 2-{[(3S)-3-hydroxypyrrolidin-1-yl]methyl}-8-[(trimethylsilyl)ethynyl[1]benzofuro[3,2]-pyrimidin-4(3H)-one

2-{[(3S)-3-hydroxypyrrolidin-1-yl]methyl}-8-[(trimethylsilyl)ethynyl][1]benzofuro[3,2-d]pyrimidin-4(3H)-one was synthesized in a manner similar to **Example 31** wherein trimethylsilylacetylene was substituted with propargyl alcohol. ¹H NMR (400 MHz, d6-DMSO): 7.78 (s, 1H), 7.56 (d, 1H), 7.44 (d, 1H), 3.93 (s, 1H), 3.41 (s, 2H), 2.54 (m, 2H), 2.28 (m, 2H), 1.75 (m, 1H), 1.33 (m, 1H), 0.00 (s, 9H). MS (EI) for C20 H23 N3 03 Si: 383 (MH+).

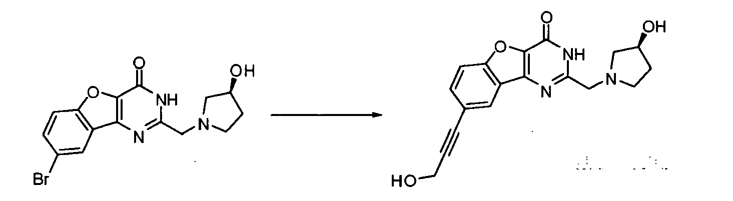

### (Compound 454)

### 8-(3-hydroxyprop-1-yn-1-yl)-2-{[(3S)-3-hydroxypyrrolidin-1-yl]methyl}[1]benzofuro[3,2-d]pyrimidin-4(3H)-one

To a solution of 8-bromo-2-{[(3S)-3-hydroxypyrrolidin-1-yl]methyl [1]benzofuro[3,2-d]pyrimidin-4(3H)-one (50 mg, 0.14 mmol) in *tert-*butanol (1.5 mL) and water (0.3 mL) was added Cs₂CO₃ (67 mg, 0.21 mmol), propargyl alcohol (40 µL, 0.69 mmol), [1,1'-bis(di-tert-butylphosphino)ferrocene] Palladium dichloride (17.9 mg, 0.03 mmol), and Cu(OTf) toluene complex (71 mg, 0.14 mmol). The reaction mixture was heated at 120 °C in the microwave for 10 minutes. Upon cooling, the reaction mixture was filtered through a PL-Thiol MP SPE cartridge and rinsed with 3 mL MeOH. The filtrate was concentrated *in vacuo* and the residue re-suspended in 1.5 mL MeOH. This suspension was filtered through a 0.2 micron syringe filter and purified by preparatory HPLC (reverse phase, acetonitrile/water with 0.1 % NH₄OAc/AcOH), followed by concentration *in vacuo.* and lyophilization to afford 23 mg (24%) of the title compound. ¹H NMR (400 MHz, d6-DMSO): 7.99 (s, 1H), 7.81 (m, 1H), 7.66 (m, 1H), 4.34 (s, 2H), 4.20 (s, 1H), 3.68 (s, 2H), 2.80 (m, 2H), 2.55 (m, 2H), 2.01 (m, 1H), 1.60 (m, 1H). MS (EI) for C18 H17 N3 04: 340 (MH+).

### Example 32

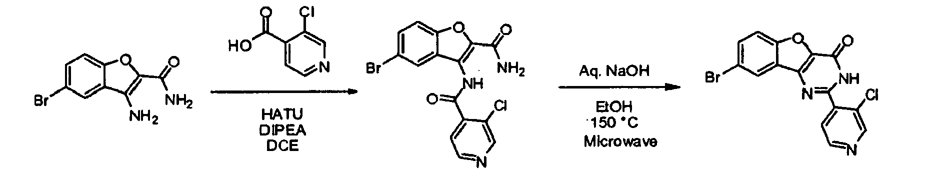

### N-(5-bromo-2-carbamoylbenzofuran-3-yl)-3-chloroisonicotinamide

To a solution of 3-Chloro-4-pyridinecarboxylic acid (594 mg, 3.77 mmol) in dichloromethane (8 mL), dimethylacetamide (2 mL) and diisopropylethylamine (1168 µL, 7.07 mmol) was added HATU (1433 mg, 3.77 mmol) followed by 3-amino-5-bromobenzofuran-2-carboxamide (400 mg, 1.57 mmol). The reaction was heated to 50 °C overnight. The reaction mixture was cooled to room temperature. The precipitate was filtered, rinsed with ethyl acetate and dried under vacuum to give 460 mg (74%) of N-(5-bromo-2-carbamoylbenzofuran-3-yl)-3-chloroisonicotinamide. 1H NMR (400 MHz, d6-DMSO): 10.93 (s, 1H), 8.83 (s, 1H), 8.73 (d, 1H), 8.23 (s, br, 1H), 8.16 (s, 1H), 7.96 (s, br, 1H), 7.80 (d, 1H), 7.67 (m, 2H). MS (EI) for C 15 H9 Br Cl N3 03: 396 (MH+).

### *(Compound 112)

### 8-bromo-2-(3-chloropyridin-4-yl)[1]benzofuro[3,2-d]pyrimidin-4(3H)-one

A suspension of N-(5-bromo-2-carbamoylbenzofuran-3-yl)-3-chloroisonicotinamide (260 mg, 0.66 mmol) and 2.6 M aqueous NaOH (609 µL, 1.58 mmol) in 3 mL anhydrous ethanol was heated for 20 min at 150 °C in a microwave reactor. The reaction mixture was diluted with 12 mL of water and acidified with 1M HCl. Precipitate was filtered, rinsed with ethanol, and stirred in 15 mL of ethanol at 50 °C for 30 minutes. It was then filtered, rinsed with ethanol, and dried under vacuum to give 190 mg (73%) of 8-bromo-2-(3-chloropyridin-4-yl)[1]benzofuro[3,2-d]pyrimidin-4(3H)-one. 1H NMR (400 MHz, d6-DMSO): 13.60 (s, 1H), 8.88 (s, 1H), 8.74 (d, 1H), 8.26 (s, 1H), 7.88 (m, 2H), 7.77 (d, 1H). MS (EI) for C 15 H7 Br Cl N3 O2: 378 (MH+).

### (Compound 460)

### 8-ethyl-2-{[(3S)-3-hydroxypyrrolidin-1-yl]methyl} [1]benzofuro[3,2-d]pyrimidin-4(3H-one

8-ethyl-2-{[(3S)-3-hydroxypyrrolidin-1-yl]methyl}[1]benzofuro[3,2-d]pyrimidin-4(3H)-one was synthesized in a manner similar to **Example 32** wherein trimethylsilylacetylene was substituted with propargyl alcohol. ¹H NMR (400 MHz, d6-cdCl₃):7.88 (s, 1H), 7.61 (d, 1H), 7.44 (d, 1H), 4.45 (t, 1H), 4.00 (d, 1H), 3.85 (d, 1H), 3.73 (m, 1H), 3.18 (m, 1H), 3.03 (d, 1H), 2.77 (m, 3H), 2.45 (m, 1H), 2.33 (m, 1H), 1.99 (m, 1H), 1.32 (t, 3H), 1.25 (t, 1H). MS(EI) for C17 H19 N3 O3: 314 (MH+).

### (Compound 492)

### 2-[(1S)-1-aminoethyl]-8-bromo 1]benzofuro[3,2-d]pyrimidin-4(3H)-one

2-[(1S)-1-aminoethyl]-8-bromo[1]benzofuro[3,2-d]pyrimidin-4(3H)-one was synthesized in a manner similar to **Example 32** wherein Boc-L-alanine was substituted with 2-chloro-4(methylsulfonyl)benzoic acid. ¹H NMR (400 MHz, d6-DMSO): 8.05 (s, 1H), 7.77 (m, 2H), 4.07 (m, 1H), 1.46 (d, 3H). MS (EI) for C12 H10 Br N3 O2: 309 (MH+).

### (Compound 457)

### 8-(6-hydroxyhexyl)-2-{[(3S)-3-hydroxypyrrolidin-1-yl]methyl}[1]benzofuro[3,2-d]pyrimidin-4(3H)-one

8-(6-hydroxyhexyl)-2-{[(3S)-3-hydroxypyrrolidin-1-yl]methyl} [1]benzofuro[3,2-d]pyrimidin-4(3H)-one was synthesized in a manner similar to **Example 32** wherein hex-5-yn-1-ol_ was substituted with propargyl alcohol. ¹H NMR (400 MHz, d6-DMSO): 7.81 (m, 1H), 7.69 (m, 1H), 7.49 (m, 1H), 4.19 (s, 1H), 3.66 (m, 2H), 3.35 (m, 4H), 2.75 (m, 4H), 2.00 (s, 1H), 1.62 (m, 3H), 1.39 (m, 2H), 1.30 (m, 4H). MS (EI) for C21 H27 N3 O4: 386 (MH+).

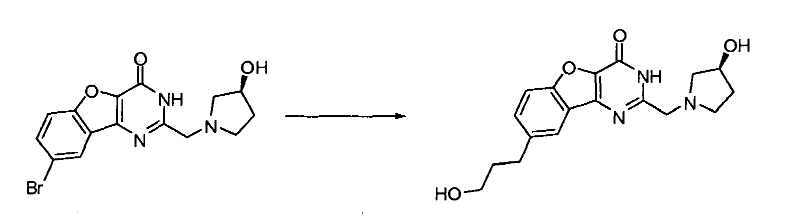

### (Compound 455)

### 8-(3-hydroxypropyl)-2- {[(3S)-3-hydroxynyrrolidin-1-yl]methyl}[1]benzofuro[3,2-d]pyrimidin-4(3H)-one

To a solution of 8-bromo-2-{[(3S)-3-hydroxypyrrolidin-1-yl]methyl}[1]benzofuro[3,2-d]pyrimidin-4(3H)-one (50 mg, 0.14 mmol) in *tert-*butanol (1.5 mL) and water (0.3 mL) was added Cs₂CO₃ (67 mg, 0.21 mmol), propargyl alcohol (40 µL, 0.69 mmol), [1,1'-bis(di-tert-butylphosphino)ferrocene] Palladium dichloride (17.9 mg, 0.03 mmol), and Cu(OTf) toluene complex (71 mg, 0.14 mmol). The reaction mixture was heated at 120 °C in the microwave for 10 minutes. Upon cooling, the reaction mixture was transferred to a hydrogenation vessel with the addition of a scope of Pd/C. The reaction was connected to parr shaker and shook overnight. The reaction mixture was filtered through a PL-Thiol MP SPE cartridge and rinsed with 3 mL MeOH. The filtrate was concentrated *in vacuo* and the residue re-suspended in 1.5 mL MeOH. This suspension was filtered through a 0.2 micron syringe filter and purified by preparatory HPLC (reverse phase, acetonitrile/water with 0.1 % NH₄OAc/AcOH), followed by concentration *in vacuo* and lyophilization to afford 13 mg (14%) of the title compound. ¹H NMR (400 MHz, d6-DMSO): 7.82 (m, 1H), 7.70 (m, 1H), 7.49 (m, 1H), 4.19 (s, 1H), 3.66 (m, 2H), 3.43 (m, 2H), 2.78 (m, 4H), 2.20 (s, 1H), 1.84 (m, 2H), 1.77 (m, 2H), 1.58 (s, 1H). MS (EI) for C18 H21 N3 O4: 344 (MH+).

### Example 33

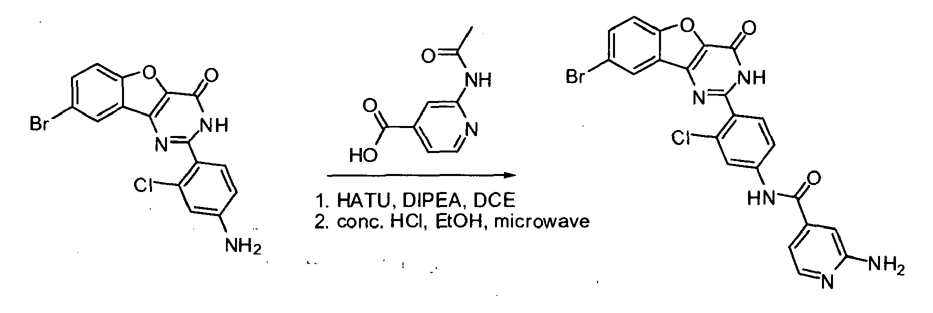

### *(Compounds 284)

### 2-amino-N-[4-(8-bromo-4-oxo-3,4-dihydro[1]benzofuro[3,2-d]pyrimidin-2-yl)-3-chlorophenyl]pyridine-4-carboxamide

To a solution of 2-acetamidoisonicotinic acid (368 mg, 2.04 mmol) in dimethylacetamide (5 mL) and diisopropylethylamine (675 µL, 4.08 mmol) was added 2-(4-amino-2-chlorophenyl)-8-bromo[1]benzofuro[3,2-d]pyrimidin-4(3H)-one (200 mg, 0.51 mmol) followed by HATU (776 mg, 2.04 mmol). The reaction mixture was heated at 50 °C overnight. The reaction mixture was cooled to room temperature, diluted with 50 mL of ice water. The precipitate was filtered and dried under vacuum to give 180 mg (64%) of 2-acetamido-N-(4-(8-bromo-4-oxo-3,4-dihydrobenzofuro[3,2-d]pyrimidin-2-yl)-3-chlorophenyl)isonicotinamide. The resulting product was used without further purification. A suspension of 2-acetamido-N-(4-(8-bromo-4-oxo-3,4-dihydrobenzofuro[3,2-d]pyrimidin-2-yl)-3-chlorophenyl)isonicotinamide (180 mg, 0.32 mmol) and concentrated HCl (200 µL) in 4 mL anhydrous ethanol was heated for 20 minutes at 120 °C in a microwave reactor. The reaction mixture was neutralized with diisopropylethylamine and purified by preparative HPLC (reverse-phase, acetonitrile/water with 0.01% formic acid) to give 40 mg (15%) of 2-amino-N-[4-(8-bromo-4-oxo-3,4-dihydro[1]benzofuro[3,2-d]pyrimidin-2-yl)-3-chlorophenyl]pyridine-4-carboxamide. 1H NMR (400 MHz, d6-DMSO): 10.67 (s, 1H), 8.23 (d, 2H), 8.09 (m, 2H), 7.84 (m, 3H), 7.67 (d, 1H), 6.96 (d, 1H), 6.90 (s, 1H), 6.29 (s, 2H). MS (EI) for C22 H13 Br Cl N5 03: 512 (MH+).

### EXAMPLE 34

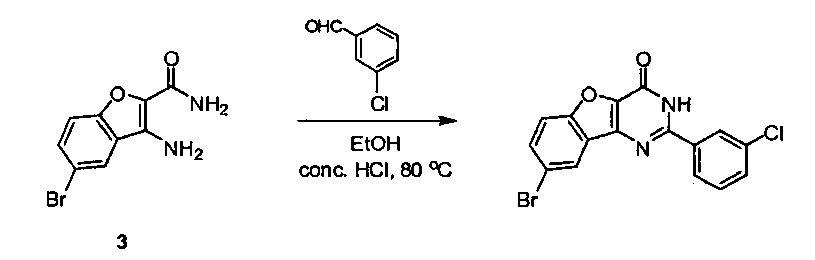

### *(Compound 93)

### 8-Bromo-2-(3-chlorophenyl)[1]benzofurro[3,2-d]pyrimidin-4(3H)-one

A solution of 3-amino-5-bromobenzofuran-2-carboxamide 3 (0.50g , 0.1.96 mmol) and 3-chlorobenzaldehyde (607 µL, 3.92 mmol) in 6 mL anhydrous ethanol were combined and stirred at room temperature for 10 minutes. The resulting suspension was treated with concentrated hydrochloric acid (40 µL) and a precipitate formed immediately. The resulting slurry was diluted with additional anhydrous ethanol (10 ml) and the resulting slurry was heated at 80°C for 16 hours. The resulting precipitate was filtered off and washed with ethyl acetate (2 x 50 ml) and methanol (2 x 5 ml) to give 76 mg of 8-bromo-2-(3-chlorophenyl)[1]benzofuro[3,2-d]pyrimidin-4(3H)-one. ¹H NMR (400 MHz, d₆-DMSO): 13.38 (br s, 1H), 8.23 (d, 1H), 7.91 (dd, 1H), 7.85 (s, 1H), 7.84 (d, 1H), 7.71 (dd, 1H), 7.52 (dd, 1H), 7.46 (d, 1H). MS (EI) for C₁₆ H₈ Br Cl N₂ O₂: 377 (MH⁺).

### *(Compound 94)

### 8-Bromo-2-(4-chlorophenyl)[1]benzofuro[3,2-d]pyrimidin-4(3H)-one

8-Bromo-2-(4-chlorophenyl)[1]benzofuro[3,2-d]pyrimidin-4(3H)-one was synthesized in a manner similar to **Example 34** wherein 4-chlorobenzaldehyde was substituted with 3-chlorobenzaldehyde. ¹H NMR (400 MHz, d₆-DMSO): 13.27 (br s, 1H), 8.27 (s, 1H), 8.11 (d, 2H), 7.86 (d, 2H), 7.80 (s, 1H), 7.78 (s, 1H). MS (EI) for C₁₆ H₈ Br Cl N₂ O₂: 377 (MH⁺).

### *(Compound 95)

### 8-Bromo-2-(4-bromophenyl)[1]benzofuro[3,2-d]pyrimidin-4(3H)-one

8-Bromo-2-(4-bromophenyl)[1]benzofuro[3,2-d]pyrimidin-4(3H)-one was synthesized in a manner similar to Example 34 wherein 4-bromobenzaldehyde was substituted with 3-chlorobenzaldehyde. ¹H NMR (400 MHz, d₆-DMSO): 13.27 (br s, 1H), 8.27 (s, 1H), 8.23 (d, 2H), 7.92 (d, 2H), 7.80 (s, 1H), 7.78 (s, 1H). MS (EI) for C₁₆ H₈ Br₂ N₂ O₂: 421 (MH⁺).

### EXAMPLE 35

### *(Compounds 96)

### 10-(2-Chlorophenyl)naphtho[1',2',4,5]furo[3,2-d]pyrimidin-8(9H)-one

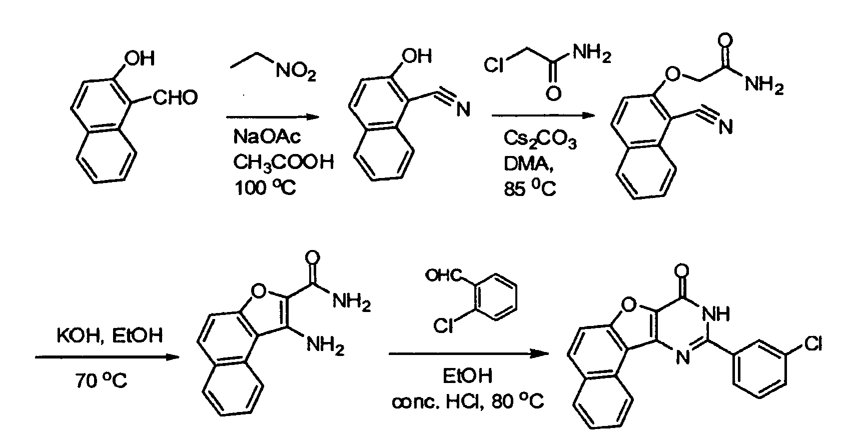

### 2-Hydroxy-1-naphthonitrile

2-Hydroxy-1-naphthonitrile was synthesized in a manner similar to 2-hydroxy-5-methoxybenzonitrile (**Example 10**) wherein 2-hydroxynaphthalene-1-carboxaldehyde was substituted with 2-hydroxy-5-methoxybenzaldehyde. ¹H NMR (400 MHz, d₆-DMSO): 11.40 (br s, 1H), 8.10 (d, 1H)m 7.95 (d, 1H), 7.88 (d, 1H), 7.68 (t, 1H), 7.46 (t, 1H), 7.20 (d, 1H). MS (EI) for C₁₁ H₇ NO: 170 (MH⁺).

### 2-(1-Cyanonaphthalen-2-yloxy)acetamide

2-(1-Cyanonaphthalen-2-yloxy)acetamide was synthesized in a manner similar to 2-(4-bromo-2-cyanophenoxy)acetamide **2,** wherein 2-hydroxy-1-naphthonitrile
was substituted with 5-bromo-2-hydroxybenzonitrile **1.** ¹H NMR (400 MHz, d₆-DMSO): 8.87 (s, 1H). 8.27 (d, 1H), 8.05 (d, 1H), 7.98 (d, 1H), 7.74 (t, 1H), 7.58 (br s , 1H), 7.54 (t, 1H), 7.48 (brs, 1H), 7.42 (d,1H). MS (EI) for C₁₃ H₁₀N₂ O₂: 227 (MH⁺).

### 1-Aminonaphtho[2,1-b]furan-2-carboxamide

1-Aminonaphtho[2,1-b]furan-2-carboxamide was synthesized in a manner similar to 3-amino-5-bromobenzofuran-2-carboxamide **3** wherein 2-(1-cyanonaphthalen-2-yloxy)acetamide was substituted with 2-(4-bromo-2-cyanophenoxy)acetamide **2.** ¹H NMR (400 MHz, d₆-DMSO): 8.64 (d, 1H), 8.36 (d, 1H), 7.85 (br s, 2H), 7.67 (dd, 2H), 7.65 (d, 1H), 7.56 (s, 1H), 7.36 (br s, 2H). MS (EI) for C₁₃ H₁₀ N₂ O₂: 227 (MH⁺).

### *(Compound 96)

### 10-(2-Chlorophenyl)naphtho[1',2':4,5]furo[3,2-d]pyrimidin-8(9H)-one

10-(2-Chlorophenyl)naphtho[1',2':4,5]furo[3,2-d]pyrimidin-8(9H)-one was synthesized in a manner similar to 8-bromo-2-(3-chlorophenyl)[1]benzofuro[3,2-d]pyrimidin-4(3H)-one, **Example 35** wherein 1-aminonaphtho[2,1-b]furan-2-carboxamide was substituted with 3-amino-5-bromobenzofuran-2-carboxamide 3, and 2-chlorobenzaldehyde was substituted with 4-bromobenzaldehyde. ¹NMR(400 MHz, d₆-DMSO): 10.72 (s, 1H), 8.31 (d, 1H), 8.23 (s, 1H), 7.98 (s, 1H), 7.79 (dd, 1H), 7.68 (dd, 1H), 7.66 (d, 1H), 7.58 (m, 1H), 7.52 (m, 1H). MS (EI) for C₂₀ H₁₁ Cl N₂ O₂: 348 (MH⁺).

### EXAMPLE 36

### (Compound 157)

### 8-Bromo-2-piperidin-4-yl[1]benzofuro[3,2-d]pyrimidin-4(3H)-one

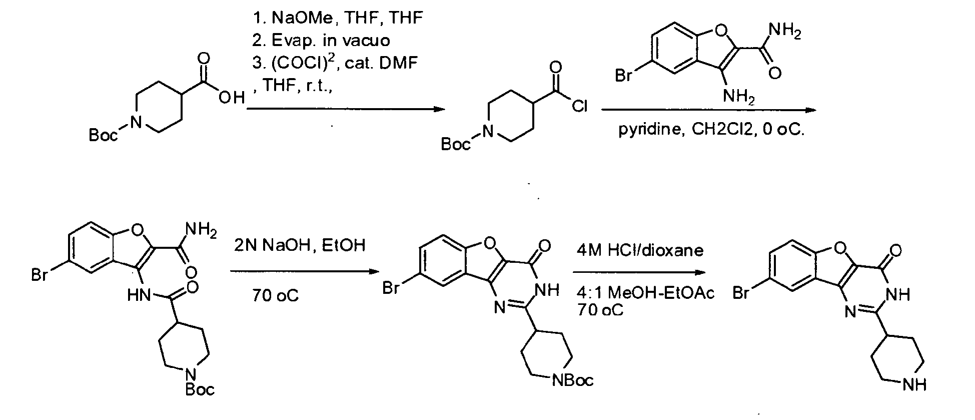

### 1,1-Dimethylethyl 4-(chlorocarbonyl)piperidine-1-carboxylate

Sodium methoxide (0.706 g, 13.08 mmoles) was added in one lot to a stirred solution of N-Boc-isonipecotic acid (3 g, 13.08 mmoles, commercially available from Chem-Impex International, Inc,) in anhydrous THF (40 ml). The resulting suspension was stirred for 1 hour at room temperature, concentrated at reduced pressure and the resulting solid was suspended in anhydrous dichloromethane (20 ml). 3 drops of anhydrous DMF was added to this stirred suspension followed by the drop-wise addition of oxalyl chloride (13.08 mmoles, 1.66 g, 1.0 uL. Upon completion of addition, the reaction mixture was stirred for 2 hours, concentrated under reduced pressure, and the resulting residue of 1,1-dimethylethyl 4-(chlorocarbonyl)piperidine-1-carboxylate was diluted with anhydrous dichloromethane (40 ml) and used directly in the next step without any further purification.

### 1,1-Dimethylethyl 4-(5-bromo-2-carbamoylbenzofuran-3-ylcarbamoyl)piperidine-1-carboxylate

The previously made solution of 1,1-dimethylethyl 4-(chlorocarbonyl)piperidine-1-carboxylate (13.08 mmole) in anhydrous dichloromethane (40 ml) was added to a solution of 3-amino-5-bromobenzofuran-2-carboxamide 3 (11.77 mmoles, 2.99 g) and anhydrous pyridine (39.24 mmoles, 0.978 g, 2.12 ml) in anhydrous dichloromethane (100 ml)m maintaining the temperature between 0 - 5°C. The reaction mixture was then transferred to a separatory funnel and washed with 1M hydrochloric acid (100 ml), water (100 ml), saturated aqueous sodium bicarbonate solution (100 ml) and saturated sodium chloride solution (100 ml). The dichloromethane solution was dried over anhydrous magnesium sulfate, filtered and evaporated under reduced pressure to give a 3.45 g of white solid as crude 1,1-dimethylethyl 4-(-5-bromo-2-carbamoylbenzofuran-3-ylcarbamoyl)piperidine-1-carboxylate which was used in the next step without any further purification. ¹H NMR (400 MHz, d₆-DMSO):10.22 (s, 1H), 8.20 (d, 1H), 8.18 (br s, 1H), 7.65 (br s ,1H), 7.64 (d, 1H), 7.56 (d, 1H), 4.06 (m. 2H), 2.89 (m, 1H), 2.86 (m, 2H), 1.90 (d, 2H), 1.69 (m, 2H), 1.42 (s, 9H). MS (EI) for C₂₀ H₂₄ Br N₃ O₅: 467 (MH⁺).

### 1,1-Dimethylethyl 4-(8-bromo-4-oxo-3,4-dihydrobenzofuro[3,2-d]pyrimidin-2-yl)pperidine-1-carboxylate

Aqueous 1M sodium hydroxide (3 equivalents, 22.5 ml) was added to a stirred solution of the crude 1,1-dimethylethyl 4-(5-bromo-2-carbamoylbenzofuran-3-ylcarbamoyl) piperidine-1-carboxylate (7.33 mmoles, 3.42 g) and ethanol (100 ml). The resulting stirred solution was the heated to 75 °C for 3 hours. The reaction mixture was allowed to cool to room temperature and acidified with ice cold 1 M hydrochloric acid (pH < 3) was slowly added which resulted in the formation of a white precipitate. The slurry was stirred for 30 minutes. The precipitate was then filtered off, washed with water (50 ml), ethyl acetate (2 x 25ml) and dried under reduced pressure to give 2.78 g of 1,1-dimethylethyl 4-(8-bromo-4-oxo-3,4-dihydrobenzofuro[3,2-d]pyrimidin-2-yl)piperidine-1-carboxylate which was used in the next step without any further purification. ¹H NMR (400 MHz, d₆-DMSO): 12.89 (br s, 1H), 8.18 (s, 1H), 7.81 (s, 2H), 4.06 (m. 2H), 2.89 (m, 1H), 2.86 (m, 2H), 1.90 (d, 2H), 1.69 (m, 2H), 1.42 (s, 9H). MS (EI) for C₂₀ H₂₂ Br N₃ O₄: 448 (MH⁺).

### (Compound 157)

### 8-Bromo-2-piperidin-4-yl[1]benzofuro[3,2-d]pyrimidin-4(3H)-one

The crude 1,1-dimethylethyl 4-(8-bromo-4-oxo-3,4-dihydrobenzofuro[3,2-d]pyrimidin-2-yl)piperidine-1-carboxylate (4.77 mmoles, 2.14) was suspended in methanol (80 ml) and ethyl acetate (20 ml). 4M hydrogen chloride in 1,4-dioxane (23 .85 mmoles, 6 ml, commercially available from Sigma-Aldrich) was added to the stirred suspension over 2 minutes. The solid dissolved into solution. The stirred reaction mixture was then heated at 75 °C and the progress of the Boc deprotection monitored by LC-MS. After 3 hours the reaction was completed and a white precipitate was observed. The reaction mixture was allowed to cool to room temperature and the solid filtered off, washed with ethyl acetate (20 ml), diethyl ether (20 ml) and dried under reduced pressure to give 1.46 g of 8-bromo-2-piperidin-4-yl[1]benzofuro[3,2-d]pyrimidin-4(3H)-one as its dihydrochloride salt. ¹H NMR (400 MHz, d₆-DMSO):13.00 (broad s, 1H), 8.13 (dd, 1H), 8.33 (d, 1H), 7.82 (d, 1H), 3.98 (m, 2H), 3.03 (m, 1H), 2.97 (m, 2H), 2.12 (m, 2H), 2.04 (m, 2H). MS (EI) for C₁₅ H₁₄ Br N₃ O2: 349 (MH⁺).

### (Compound 158)

### 8-Bromo-2-piperidin-3-yl[1]benzofuro[3,2-d]pyrimidin-4(3H)-one

8-Bromo-2-piperidin-3-yl[1]benzofuro[3,2-d]pyrimidin-4(3H)-one was synthesized in a manner similar to **Example 36** wherein nipecotic acid (commercially available from Chem-Impex International, Inc,) was substituted with isonipecotic acid, followed by cyclization and removal of the Boc group.

### 1,1-Dimethylethyl 3-(chlorocarbonyl)piperidine-1-carboxylate

1,1-Dimethylethyl 3-(chlorocarbonyl)piperidine-1-carboxylate was synthesized in a similar manner as 1,1-dimethylethyl 4-(chlorocarbonyl)piperidine-1-carboxylate nipecotic acid (commercially available from Chem-Impex International, Inc,) was substituted with isonipecotic acid. This material was used directed without further purification.

### 1,1-Dimethylethyl 3-(5-bromo-2-carbamoylbenzofuran-3-ylcarbamoyl)piperidine-1-carboxylate

1,1-Dimethylethyl 3-(5-bromo-2-carbamoylbenzofuran-3-ylcarbamoyl)piperidine-1-carboxylate was synthesized in a similar manner as to 1,1-dimethylethyl 4-(5-bromo-2-carbamoylbenzofuran-3-ylcarbamoyl)piperidine-1-carboxylate wherein 1,1-dimethylethyl 3-(chlorocarbonyl)piperidine-1-carboxylate was substituted with 1,1-dimethylethyl 4-(chlorocarbonyl)piperidine-1-carboxylate. ¹H NMR (400 MHz, d₆-DMSO): 10.22 (s, 1H), 8.20 (d, 1H), 8.18 (br s, 1H), 7.65 (br s ,1H), 7.64 (d, 1H), 7.56 (d, 1H), 3.84 (m, 1H), 2.87 (m, 1H), 2.62 (m, 1H), 2.30 (m, 1H), 2.04 (m, 1H), 1.91 (m, 1H), 1.72 (m, 2H), 1.62 (m, 2H), 1.35 (s, 9H). MS (EI) for C₂₀ H₂₄ Br N₃ O₅: 467 (MH⁺).

### 1,1-Dimethylethyl 3-(8-bromo-4-oxo-3,4-dihydrobenzofuro[3.2-d]pyrimidin-2-yl)piperidine-1-carboxylate

1,1-Dimethylethyl3-(8-bromo-4-oxo-3,4-dihydrobenzofuro[3,2-d]pyrimidin-2-yl)piperidine-1-carboxylate was synthesized in a similar manner as to 1,1-dimethylethyl 4-(8-bromo-4-oxo-3,4-dihydrobenzofuro [3,2-d]pyrimidin-2-yl)piperidine-1-carboxylate where 1,1-dimethylethyl3-(5-bromo-2-carbamoylbenzofuran-3-ylcarbamoyl)piperidine-1-carboxylate was substituted with 1,1-dimethylethyl 4-(5-bromo-2-carbamoylbenzofuran-3-ylcarbamoyl)piperidine-1-carboxylate. ¹H NMR (400 MHz, d₆-DMSO): 12.90 (br s, 1H), 8.16 (s, 1H), 7.82 (d, 2H), 40.06 (m, 1H), 3.87 (m, 2H), 2.88 (m, 2H), 2.50 (m, 2H), 1.81 (m, 2H), 1.37 (s, 9H). MS (EI) for C₂₀ H₂₂ Br N₃ O₄ 448 (MH⁺).

### 8-Bromo-2-piperidin-3-yl[1]benzofuro[3,2-d]pyrimidin-4-(3H)-one

8-Bromo-2-piperidin-3-yl[1]benzofuro[3,2-d]pyrimidin-4(3H)-one was synthesized in a similar manner to 8-Bromo-2-piperidin-4-yl[1]benzofuro[3,2-d]pyrimidin-4(3H)-one **Example 36** wherein 1,1-dimethylethyl 4-(8-bromo-4-oxo-3,4-dihydrobenzofuro [3,2-d]pyrimidin-2-yl)piperidine-1-carboxylate was replaced by 1,1-dimethylethyl 3-(8-bromo-4-oxo-3,4-dihydrobenzofuro[3,2-d]pyrimidin-2-yl)piperidine-1-carboxylate. ¹H NMR (400 MHz, d₆-DMSO): 13.18 (br s, 1H), 8.25 (s, 1H), 7.83 (s, 2H), 4.23 (br s, 2H), 3.38 (m, 3H), 2.99 (m, 1H), 2.14 (m. 1H), 1.77 (m, 4H). MS (EI) for C₁₅ H₁₄ Br N₃ O₂: 349 (MH⁺).

### *(Compounds 217)

### N-[4-(8-bromo-4-oxo-3.4-dihydro[1] benzofuro[3.2-d]pyrimidin-2-yl)-3-chlorophenyl]-2-piperidin-4-ylacetamide

N-[4-(8-bromo-4-oxo-3,4-dihydro[1]benzofuro[3,2-d]pyrimidin-2-yl)-3-chlorophenyl]-2-piperidin-4-ylacetamide was synthesized in a manner similar to **(Compound 198)** wherein 2-(4-amino-2-chlorophenyl)-8-bromo[1]benzofuro[3,2-d]pyrimidin-4(3H)-one was substituted with 3-amino-5-bromobenzofuran-2-carboxamide, and 2-(1-(*tert*-butoxycarbonyl)piperidin-4-yl)acetic acid was substituted with boc-3-azetidine carboxylic acid. 1H NMR (400 MHz, d6-DMSO): 13.36 (s, 1H), 10.62 (s, 1H), 8.81 (s, br, 1H), 8.65 (s, br, 1H), 8.24 (s, 1H), 8.03 (s, 1H), 7.87 (m, 2H), 7.64 (s, 2H), 3.25 (d, 2H), 2.89 (m, 2H), 2.38 (d, 2H), 2.08 (s, br, 1H), 1.83 (d, 2H), 1.44 (m, 2H). MS (EI) for C23 H20 Br Cl N4 03: 517 (MH+).

### *(Compounds 384)

### 8-bromo-2-[phenyl(pinerazin-1-yl)[methyl[1]benzofuro[3,2-d]pyrimidin-4(3H)-one

8-bromo-2-[phenyl(piperazin-1-yl)methyl][1]benzofuro[3,2-d]pyrimidin-4(3H)-one was synthesized in a manner similar to **Example 36** wherein 2-(4-(*tert-*butoxycarbonyl)piperazin-1-yl)-2-phenylacetic acid was substituted with N-boc-isonipecotic acid. 1H NMR (400 MHz, d6-DMSO): 8.22 (s, 2H), 8.16 (s, 1H), 7.78 (s, 1H), 7.59 (d, 1H), 7.32 (m, 2H), 3.17 (s, 1H), 3.04 (m, 4H), 2.56 (m, 2H), 2.46 (m, 2H), 1.24 (s, 1H). MS (EI) for C21 H19 BrN4 O2: 440 (MH+).

### EXAMPLE 36B

### (Compound 184)

### 8-bromo-2-(1-methylpiperidin-3-yl)[1]benzofuro[3,2-d]pyrimidin-4(3H)-one

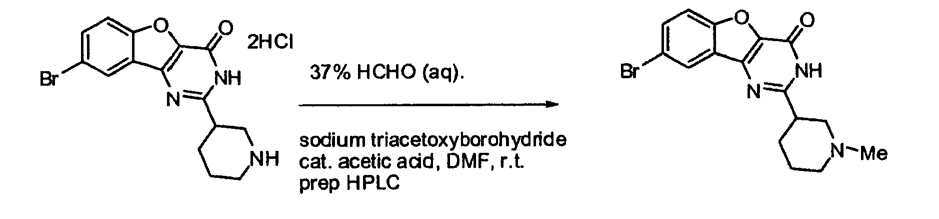

8-Bromo-2-piperidin-3-yl[1]benzofuro[3,2-d]pyrimidin-4(3H)-one dihydrochloride **(Compound 158)** (1 equivalent, 0.205 g, 0.486 mmoles) was added to anhydrous DMF (15 ml) and stirred to ensure complete dissolution of the solid. The stirred reaction mixture was treated with 37 % aqueous formaldehyde (10 equivalents, 4.867mmoles, 0.168g, 0.4 ml) and glacial acetic acid (20 ul). The reaction mixture was stirred at room temperature for 5 minutes. The stirred reaction mixture was then treated with sodium triacetoxyborohydride (10 equivalents, 4.86 mmoles, 1.188 g, commercially available from BASF Corporation) and additional glacial acetic acid (20 ul). The reaction mixture was then stirred at room temperature for 30 minutes and shown to be complete by LC-MS. The solution was filtered through a Millipore Millex-GN 0.20 uM Nylon syring filter, and the resulting solution was submitted for preparative reverse phase HPLC (reverse-phase, acetonitrile/water with 0.01% ammonium acetate). The resulting fractions containing the desired peak (of correct molecular weight) were combined and evaporated under reduced pressure to give 0.073 g of 8-bromo-2-(1-methylpiperidin-3-yl)[1]benzofuro[3,2-d]pyrimidin-4(3H)-one (acetate salt) as a white solid. ¹H NMR (400 MHz, d₆-DMSO): 13.14 (br s, 1H), 8.10 (d, 1H), 7.85 (d, 2H), (br s, 2H), 3.70 (m, 2H), 2.92 (m, 4H), 2.50 (s, 3H), 2.19 (m, 1H), 1.94 (s, 3H). MS (EI) for C₁₆ H₁₆ Br N₃ O₂: 363 (MH⁺).

### (Compound 185)

### 8-Bromo-2-(1-methylpiperidin-4-yl)[1]benzofuro[3,2-d]pyrimidin-4(3H)-one

8-Bromo-2-(1-methylpiperidin-4-yl)[1]benzofuro[3,2-d]pyrimidin-4(3H)-one was synthesized in a manner similar to 8-bromo-2-(1-methylpiperidin-3-yl)[1]benzofuro[3,2-d]pyrimidin-4(3H)-one wherein 8-bromo-2-piperidin-4-**yl[1]benzofuro[3,2-d]pyrimidin-4(3H)-one dihydrochloride was substituted with 8-bromo-2-piperidin-3-yl[1]benzofuro [3,2-d]pyrimidin-4(3H)-one dihydrochloride (Compound 184)**. ¹H NMR (400 MHz, d₆-DMSO): 13.00 (broad s, 1H), 8.13 (dd, 1H), 8.33 (d, 1H), 7.82 (d, 1H), 3.98 (m, 2H), 3.03 (m, 1H), 2.97 (m, 2H), 2.50 (s, 3H), 2.12 (m, 2H), 2.04 (m, 2H). 1.94 (s, 3H). MS (EI) for C₁₆ H₁₆ Br N₃ O₂ : 363 (MH⁺).

### EXAMPLE 37

### (Compound 198)

### 2-Azetidin-3-yl-8-bromo[1]benzofuro[3,2-d]pyrimidin-4(3H)-one

Reference for the amide formation with cyanuric chloride: Org.Process Res.Dev., (1999), 3, 12

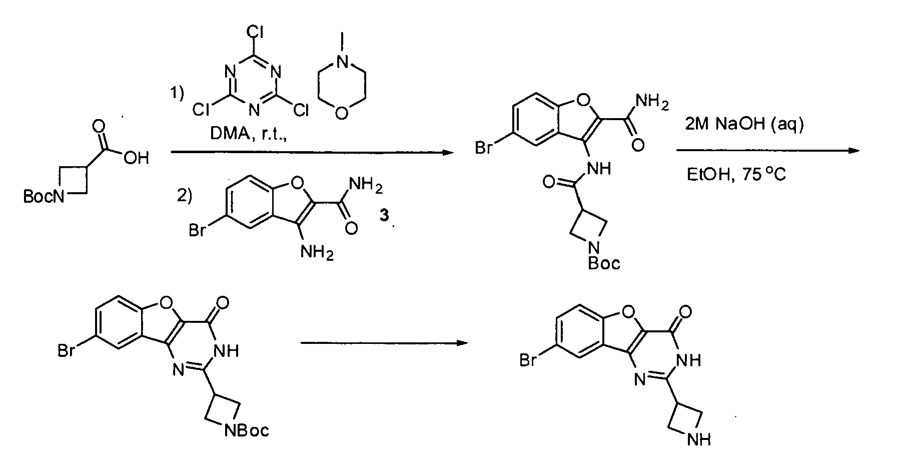

### 1,1-Dimethylethyl 3-(5-bromo-2-carbamoylbenzofuran-3-ylcarbamoyl)azetidine-1-carboxylate

Cyanuric chloride (0.33 equivalents based on Boc-3-azetidine carboxylic acid, 4.14 mmoles, 0.763 g, commercially available from Sigma-Aldrich) was then added in one lot to a solution of Boc-3-azetidine carboxylic acid (2.50 g, 12.42 mmoles, 1 equivalent, commercially available from CNH Technologies Inc.,) in anhydrous DMA (75 ml). The mixture was stirred for 5 minutes to allow for dissolution, followed by the rapid addition of N-methylmorpholine (1.02 equivalents, 12.67 mmoles, 1.28 g, 1.39 mL). The reaction mixture was then stirred at room temperature. After 5 minutes after addition of the N-methylmorpholine, a solid was formed. The reaction mixture was then stirred at room temperature for 1 hour. 3-Amino-5-bromobenzofuran-2-carboxamide 3 (1.05 equivalents, 13.045 mmoles, 2.94 g) was then added to the stirred reaction mixture in one lot, and the reaction mixture was stirred for 16 hours. The reaction mixture was transferred to a 1000 ml separatory funnel and diluted with ethyl acetate (400 ml) and 1M hydrochloric (400 ml), agitated and the ethyl acetate layer was collected. The aqueous layer was further washed with ethyl acetate (3 x 250 ml). The combined ethyl acetate solution was then washed with 0.5 M sodium hydroxide (400 ml), water (400 ml) and saturated sodium chloride solution (400 ml). The organic solution was then dried over anhydrous magnesium sulfate, filtered and evaporated under reduced pressure to give 1,1-dimethylethyl 3-(5-bromo-2-carbamoylbenzofuran-3-ylcarbamoyl)azetidine-1-carboxylate_as the major product and was used in the next step without any purification. MS (EI) for C₁₈ H₂₀ Br N₃ O₅: 439 (MH⁺)

### 1,1-Dimethylethyl 3-(8-bromo-4-oxo-3,4-dihydrobenzofuro[3,2-d]pyrimidin-2-yl)azetidine-1-carboxylate

1,1-Dimethylethyl 3-(8-bromo-4-oxo-3,4-dihydrobenzofuro[3,2-d]pyrimidin-2-yl)azetidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl 3-(5-bromo-2-carbamoylbenzofuran-3-ylcarbamoyl)azetidine-1-carboxylate was substituted with 1,1 -dimethylethyl 4-(5-bromo-2-carbamoylbenzofuran-3-ylcarbamoyl)piperidine-1-carboxylate. ¹H (400 MHz, d₆-DMSO): 12.96 (br s, 1H), 8.23 (s, 1H), 7.82 (s, 2H), 3.88 (m, 1H), 3.36 (m, 4H), 1.40 (s, 9H). MS (EI) for C₁₈ H₁₈ Br N₃ O₄: 339 (MH⁺)

### (Compound 198)

### 2-Azetidin-3-yl-8-bromo[1]benzofuro[3,2-d]pyrimidin-4(3H)-one

2-Azetidin-3-yl-8-bromo[1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was replaced by 1,1-dimethylethyl 3-(8-bromo-4-oxo-3,4-dihydrobenzofuro[3,2-d]pyrimidin-2-yl)azetidine-1-carboxylate: ¹H NMR (400 MHz, d₆-DMSO): 13.07 (br s, 1H), 9.25 (br s, 1H), 8.22 (dd, 1H), 7.87 (d, 1H), 7.86 (dd, 1H), 4.37 (m, 2H), 4.21 (m, 2H), 4.16 (m, 1H). MS (EI) for C₁₃ H₁₀ Br N₃ O₂: 321 (MH⁺).

### (Compound 221)

### 8-Bromo-2-(1-methylazetidin-3-yl)[1]benzofuro[3,2-d]pyrimidin-4(3H)-one

8-Bromo-2-(1-methylazetidin-3-yl)[1]benzofuro[3,2-d]pyrimidin-4(3H)-one was synthesized in a manner similar to 8-bromo-2-(1-methylpiperidin-3-yl)[1]benzofuro[3,2-d]pyrimidin-4(3H)-one wherein 2-azetidin-3-yl-8-bromo [1]benzofuro[3,2-d]pyrimidin-4(3H)-one_**(Compound 198)** dihydrochloride was substituted with 8-bromo-2-piperidin-3-yl[1]benzofuro[3,2-d]pyrimidin-4(3H)-one dihydrochloride **(Compound 184).** ¹H NMR (400 MHz, d₆-DMSO): 13.00 (br s, 1H), 8.19 (s, 1H), 7.81 (s, 2H), 5.5. (br s, 2H), 3.75 (m, 3H), 3.61 (s, 2H), 2.39 (s, 3H). MS (EI) for C₁₄ H₁₂ Br N₃ O₂: 335 (MH⁺).

### *(Compound 222)

### 1,1-Dimethylethyl [4-(8-bromo-4-oxo-3,4-dihydro[1]benzofuro[3,2-d]pyrimidin-2-yl)cyclohexyl]carbamate

### 1,1-Dimethylethyl 4-(5-bromo-2-carbamoylbenzofuran-3-ylcarbamoyl)cyclohexylcarbamate

The intermediate 1,1-Dimethylethyl 4-(5-bromo-2-carbamoylbenzofuran-3-ylcarbamoyl) cyclohexylcarbamate was synthesized in a manner similar to 1,1-dimethylethyl 3-(5-bromo-2-carbamoylbenzofuran-3-ylcarbamoyl)azetidine-1-carboxylate, wherein 4-(tert-butoxycarbonylamino) cyclo-hexanecarboxylic acid (commercially available from CNH Technologies, Inc.) replaced Boc-3-azetidine carboxylic acid. The crude material from this reaction was submitted to the next step without any further purification. MS (EI) for C₂₁ H₂₆ Br N₃ O₅: 481 (MH⁺).

### 1,1-Dimethylethyl [4-(8-bromo-4-oxo-3,4-dihydro[1]benzofuro[3,2-d]pyrimidin-2-yl)cyclohexyl]carbamate *(Compound 222)

1,1-Dimethylethyl [4-(8-bromo-4-oxo-3,4-dihydro[1]benzofuro[3,2-d]pyrimidin-2-yl)cyclohexyl]carbamate was synthesized in a similar manner as to 1,1-dimethylethyl 4-(8-bromo4-oxo-3,4-dihydrobenzofuro[3,2-d]pyrimidin-2-yl)piperidine-1-carboxylate wherein 1,1-dimethylethyl4-(5-bromo-2-carbamoylbenzofuran-3-ylcarbamoyl)cyclohexylcarbamate was substituted for 1,1-dimethylethyl 4-(5-bromo-2-carbamoylbenzofuran-3-ylcarbamoyl)piperidine-1-carboxylate. ¹H NMR (400 MHz, d₆-DMSO): 12.96 (br s, 1H), 8.23 (s, 1H), 8.03 (br s , 1H), 7.82 (s, 2H), 3.88 (m, 1H),2.01, (m, 2H), 1.87 (m, 2H), 1.73 (m, 2H), 1.65 (m, 2H), 1.43 (s, 9H). MS (EI) for C₂₁ H₂₄ Br N₃ O₄ : 463 (MH⁺).

### *(Compound 223)

### 2-(4-Aminocyclohexyl)-8-bromo[1]benzofuro[3,2-d]pyrimidin-4(3H)-one

2-(4-Aminocyclohexyl)-8-bromo[1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl [4-(8-bromo-4-oxo-3,4-dihydro[1]benzofuro[3,2-d]pyrimidin-2-yl)cyclohexyl]carbamate **(Compound 222).** ¹H NMR (400 MHz, d₆-DMSO): 12.92 (br s, 1H), 8.18 (s, 1H), 7.83 (s, 2H), 4.31 (br s, 3H), 3.28 (m, 1H), 2.92 (m, 1H), 2.09 (m, 2H), 1.97 (m, 2H), 1.83 (m, 4H). MS (EI) for C₁₆ H₁₆ Br N₃ O₂ :362 (MH⁺).

### (Compound 273)

### 8-Bromo-2-(1-methylpyrrolidin-2-yl)[1]benzofuro[3,2-d]pyrimidin-4(3H)-one

8-Bromo-2-(1-methylpyrrolidin-2-yl)[1]benzofuro[3,2-d]pyrimidin-4(3H)-one was synthesized in a manner similar to 8-bromo-2-(1-methylpiperidin-3-yl)[1]benzofuro[3,2-d]pyrimidin-4(3H)-one wherein 2-(4-aminocyclohexyl)-8-bromo[1]benzofuro[3,2-d]pyrimidin-4(3H)-one **(Compound 177)** was substituted with 8-bromo-2-piperidin-3-yl[1]benzofuro[3,2-d]pyrimidin-4(3H)-one dihydrochloride **(Compound 138).** ¹H NMR (400 MHz, d₆-DMSO): 13.02 (broad s, 1H), 8.22 (dd, 1H), 7.82 (dd, 2H), 3.70 (m, 1H), 3.46 (m, 2H), 3.14 (m, 2H), 2.72 (s, 3H), 2.37 (m, 1H), 2.31 (m, 1H). MS (EI) for C₁₅ H₁₄ Br N₃ O₂: 349 (MH⁺).

### *(Compound 297)

### 1,1-Dimethylethyl 2-(8-bromo-4-oxo-3,4-dihydro[1]benzofuro[3,2-d]pyrimidin-2-yl)morpholine-4-carboxylate

### 1,1-Dimethylethyl 2-(5-bromo-2-carbamoyl)benzofuran-3-ylcarbamoyl)morpholine-4-carboxylate

The intermediate 1,1-Dimethylethyl 2-(5-bromo-2-carbamoylbenzofuran-3-ylcarbamoyl)morpholine-4-carboxylate was synthesized in a manner similar to 1,1-dimethylethyl 3-(5-bromo-2-carbamoylbenzofuran-3-ylcarbamoyl)azetidine-1-carboxylate wherein 4-Boc morpholine-2-carboxylic acid (commercially available from PharmaCore) was substituted with Boc-3-azetidine carboxylic acid. The crude material from this reaction was submitted to the next step without any further purification. MS (EI) for C₁₉ H₂₂ Br N₃ O₆: 451 (MH⁺).

### 1,1-Dimethylethyl 2-(8-bromo-4-oxo-3,4-dihydro[1]benzofuro[3,2-d]pyrimidin-2-yl)morpholine-4-carboxylate

1,1-Dimethylethyl 2-(8-bromo-4-oxo-3,4-dihydro[1]benzofuro[3,2-d]pyrimidin-2-yl)morpholine-4-carboxylate **(Compound 297)** was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl 2-(5-bromo-2-carbamoylbenzofuran-3-ylcarbamoyl)morpholine-4-carboxylate was substituted with 1,1-dimethylethyl 4-(5-bromo-2-carbamoylbenzofuran-3-ylcarbamoyl)piperidine-1-carboxylate. ¹H NMR (400 MHz, d₆-DMSO): 12.94 (br s, 1H), 8.21 (s, 1H), 7.84 (dd, 2H), 4.47 (dd, 1H), 4.11 (br s, 1H), 3.98 (d, 1H), 3.67 (d, 1H), 3.57 (td, 1H), 3.21 (m, 1H), 3.05 (m, 1H), 1.43 (s, 9H). MS (EI) for C₁₉ H₂₀ Br N₃ O₅: 451 (MH⁺).

### (Compound 298)

### 8-Bromo-2-morpholin-2-yl[1]benzofuro[3.2-d]pyrimidin-4(3H)-one

8-Bromo-2-morpholin-2-yl[1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 2-(8-bromo-4-oxo-3,4-dihydro[1]benzofuro[3,2-d]pyrimidin-2-yl)morpholine-4-carboxylate **(Compound 297)**. ¹H NMR (400 MHz, d₆-DMSO):13.20 (br s, 1H), 8.25 (d, 1H), 7.86 (d, 1H), 7.85 (d, 1H), 4.93 (dd, 1H), 4.08 (dd, 1H), 3.85 (t, 1H), 3.56 (dd, 1H), 3.47 (m 1H), 2.27 (d, 1H), 3.12 (m, 1H). MS (EI) for C₁₉ H₂₀ Br N₃ O₅: 351 (MH⁺).

### *(Compound 299)

### 8-Bromo-2-[3-(piperidin-4-yloxy)isoxazol-5-yl][1]benzofuro[3,2-d]pyrimidin-4(3H)-one

### 1,1-Dimethylethyl 4-(5-(5-bromo-2-carbamoylbenzofuran-3-ylcarbamoyl)isoxazol-3-yloxy)piperidine-1-carboxylate

1, 1-Dimethylethyl 4-(5-(5-bromo-2-carbamoylbenzofuran-3-ylcarbamoyl)isoxazol-3-yloxy)piperidine-1-carboxylate was synthesized in a manner similar to 1,1-dimethylethyl 3-(5-bromo-2-carbamoylbenzofuran-3-ylcarbamoyl)azetidine-1-carboxylate wherein 3-(1-(tert-butoxycarbonyl)piperidin-4-yloxy)isoxazole-5-carboxylic acid (prepared from a literature procedure WO2002051849) was substituted with Boc-3-azetidine carboxylic acid. The crude material from this reaction was submitted to the next step without any further purification. MS (EI) for C₂₃ H₂₅ Br N₄O₇: 555 (MH⁺).

### 1,1-Dimethylethyl 4-(5-(8-bromo-4-oxo-3,4-dihydrobenzofuro[3,2-d]pyrimidin-2-yl)isoxazol-3-yloxy)piperidine-1-carboxylate

1, 1-Dimethylethyl 4-(5-(8-bromo-4-oxo-3,4-dihydrobenzofuro[3,2-d]pyrimidin-2-yl)isoxazol-3-yloxy)piperidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl 4-(5-(5-bromo-2-carbamoylbenzofuran-3-ylcarbamoyl)isoxazol-3-yloxy)piperidine-1-carboxylate was substituted with 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₂₃ H₂₃ Br N₄ O: 532 (MH⁺).

### 8-Bromo-2-[3-(piperidin-4-yloxy)isoxazol-5-yl][1]benzofuro[3,2-d]pyrimidin-4(3H)-one *(Compound 299)

8-Bromo-2-[3-(piperidin-4-yloxy)isoxazol-5-yl][1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 4-(5-(8-bromo-4-oxo-3,4-dihydrobenzofuro[3,2-d]pyrimidin-2-yl)isoxazol-3-yloxy)piperidine-1-carboxylate. ¹H NMR (400 MHz, d₆-DMSO):13.69 (br s, 1H), 8.98 (br s, 2H), 8.26 (s 1H), 7.88 (m, 2H), 7.24 (s, 1H), 4.95 (m, 1H), 3.26 (m, 2H), 3.11 (m, 2H), 2.23 (m, 2H), 1.95 (m, 2H).
MS (EI) for C₁₈ H₁₅ Br N₄ O₄: 432 (MH⁺).

### (Compound 506)

### (S)-1,1,-dimethylethyl-2-(2-carbamoylbenzofuran-3-ylcarbamoyl)pyrrolidine-1-carboxylate

The intermediate (S)-1,1,-dimethylethyl-2-(2-carbamoylbenzofuran-3-ylcarbamoyl)pyrrolidine-1-carboxylate was synthesized in a manner similar to 1,1-dimethylethyl 3-(5-bromo-2-carbamoylbenzofuran-3-ylcarbamoyl)azetidine-1-carboxylate 4-(tert-butoxycarbonylamino), using 3-aminobenzofuran-2-carboxamide (commercially available from Sigma-Aldrich) and N-Boc-L-proline (commercially available from ChemImpex International Inc.,) which were substituted with 5-bromo-3-aminobenzofuran-2-carboxamide and Boc-3-azetidine carboxylic acid, respectively. The crude material from this reaction was submitted to the next step without any further purification. MS (EI) for C 19 H23 N3 O5: 374 (MH⁺).

### 1,1-Dimethylethyl-2-[(2S)-pyrrolidin-2-yl][1]benzofuro[3,2-d]pyrimidin-4(3H)-one

1,1-Dimethylethyl-2-[(2S)-pyrrolidin-2-yl][1]benzofuro[3,2-d]pyrimidin-4(3H)-one was synthesized in a similar manner to 1,1-dimethylethyl 4-(8-bromo-4-oxo-3,4-dihydrobenzofuro[3,2-d]pyrimidin-2-yl)piperidine-1-carboxylate wherein (S)-1,1,-dimethylethyl-2-(2-carbamoylbenzofuran-3-ylcarbamoyl)pyrrolidine-1-carboxylate was substituted for 1,1-dimethylethyl 4-(5-bromo-2-carbamoylbenzofuran-3-ylcarbamoyl)piperidine-1-carboxylate. The crude material from this reaction was air dried and submitted to the next step without any further purification. ¹H NMR (400 MHz, d₆-DMSO): 13.99 (br s 1H), 8.01 (t, 1H), 7.83 (d, 1H), 7.87 (t, 1H), 7.49 (t, 1H), 4.68 (m, 1H), 3.63 (m, 1H), 3.44 (m, 1H), 3.31 (m, 1H), 2.02 (m, 2H), 1.85 (m, 1H), 1.10 (s, 9H). MS (EI) for C19 H21 N3 O4: 356 (MH⁺).

### 2-[(2S)-pyrrolidin-2-yll][1]benzofuro[3,2-d]pyrimidin-4(3H)-one

2-[(2S)-pyrrolidin-2-yl][1]benzofuro[3,2-d]pyrimidin-4(3H)-one was synthesized in a similar manner as to 1,1-dimethylethyl 4-(8-bromo-4-oxo-3,4-dihydrobenzofuro[3,2-d]pyrimidin-2-yl)piperidine-1-carboxylate wherein (S)-1,1,-dimethylethyl-2-(2-carbamoylbenzofuran-3-ylcarbamoyl)pyrrolidine-1-carboxylate was substituted for 1,1-dimethylethyl 4-(5-bromo-2-carbamoylbenzofuran-3-ylcarbamoyl)piperidine-1-carboxylate. The crude material from this reaction was air dried and submitted to the next step without any further purification. ¹H NMR (400 MHz, d₆-DMSO): 13.30 (br s 1H), 8.03 (t, 1H), 7.85 (d, 1H), 7.83 (t, 1H), 7.44 (t, 1H), 4.793 (t, 1H), 3.51 (m, 1H), 3.37 (br s 2H), 3.35 (m, 1H), 2.43 (m, 1H), 2.15 (m, 1H), 2.03 (m, 1H). MS (EI) for C₁₄ H₁₃ N₃ O₂: 256 (MH⁺).

### (Compound 507)

### N-[2-(Aminocarbonyl)-5-chloro-1-benzofuran-3-yl]-L-prolinamide hydrochloride

L-Proline (5.0 g, 43.4 mmol) was suspended in anhydrous dichloromethane (50 mL) and cooled in a -10°C ice bath. Phosphorus pentachloride (8.14 g, 39.1 mmol) was added in one portion. The reaction mixture was stirred in the cold bath for 60 min. Another reaction flask was charged with commercially available 3-amino-5-chloro-1-benzofuran-2-carboxamide, (2.28 g, 10.8 mmol) and dichloromethane (25 mL) and the suspension was cooled in an ice bath. The solution of proline acid chloride was added dropwise to the cold suspension of 3-amino-5-chloro-1-benzofuran-2-carboxamide over a period of 5 min. The reaction mixture was allowed to warm to room temperature and was stirred overnight. The precipitate was filtered off using dichloromethane (50 mL) to transfer the solid from the reaction flask. The solid was washed with dichloromethane (3 x 20 mL) and dried under vacuum to give 4.05 g of N-[2-(aminocarbonyl)-5-chloro-1-benzofuran-3-yl]-L-prolinamide hydrochloride. The material was used without further purification.
¹H NMR (400 MHz, d₆-DMSO): 10.00 (br s, 1H), 8.71 (br s, 1H), 8.20 (s, 1H), 7.92 (s, 1H), 7.82 (d, 1H), 7.65 (dd, 1H), 7.52 (dd, 1H), 4.55 (m, 1H), 3.51 (br s, 2H), 3.26 (m, 2H, 2.40 (m, 1H), 2.08 (m, 1H), 1.83 (m, 2H).
MS (EI) for C₁₄ H₁₄ Cl N₃ O₃: 308 (MH⁺).

### 8-Chloro-2-[(2S)-pyrrolidin-2-yl][1]benzofuro[3,2-d]pyrimidin-4(3H)-one hydrochloride

N-[2-(Aminocarbonyl)-5-chloro-1-benzofuran-3-yl]-L-prolinamide hydrochloride monohydrate (4.0 g, 11.6 mmol) was suspended in ethanol (100 mL) and sodium hydroxide (30 mL, 2.0 M, 60 mmol) was added in one portion which caused all the material to dissolve. The reaction mixture was stirred at 45°C for 4h and was allowed to cool to room temperature. The reaction flask was cooled in an ice bath (0°C) and hydrochloric acid (6.0 M) was added dropwise to lower the pH to 2. The reaction mixture was removed from the ice bath and allowed to stand at 4°C in a refrigerator for 16 hours. The precipitate was isolated by filtration, washed with cold water (2 x 10 mL), and air dried for 3 h. The solid was then washed with ethyl acetate (3 x 10 mL) and dried under vacuum to give 1.97 g (59% yield, >98% purity) of 8-chloro-2-[(2S)-pyrrolidin-2-yl][1]benzofuro[3,2-d]pyrimidin-4(3H)-one hydrochloride.
¹H NMR (400 MHz, d₆-DMSO): 13.50 (br s 1H), 8.07 (d, 1H), 7.94 (d, 1H), 7.74 (dd, 1H), 4.793 (t, 1H), 3.51 (m, 1H), 3.37 (br s 2H), 3.35 (m, 1H), 2.43 (m, 1H), 2.15 (m, 1H), 2.03 (m, 1H).
MS (EI) for C₁₄H₁₂ClN₃O₂: 290 (MH⁺).

### *(Compound 515)

### 1,1,-dimethylethyl-5-(5-bromo-2-carbamoylbenzofuran-3-ylcarbamoyl)-(2S)-2,2-dimethylpyrrolidine-1-carboxylate

The intermediate 1,1,-dimethylethyl-5-(5-bromo-2-carbamoylbenzofuran-3-ylcarbamoyl)-(2S)-2,2-dimethylpyrrolidine-1-carboxylate was synthesized in a manner similar to 1,1-dimethylethyl 3-(5-bromo-2-carbamoylbenzofuran-3-ylcarbamoyl) azetidine-1-carboxylate 4-(tert-butoxycarbonylamino), using 3-amino-5-chlorobenzofuran-2-carboxamide and (S)-N-Boc-5,5-dimethylpyrrolidine-2-carboxylic acid (commercially available from ChemImpex International Inc.,.) which replaced Boc-3-azetidine carboxylic acid. The crude material from this reaction was submitted to the next step without any further purification. MS (EI) for C₂₁ H₂₆ Br N₃ O₅: 481 (MH⁺).

### 1,1,-dimethylethyl-5-(8-bromo-4-oxo-3,4-dihydrobenzofuro[3,2-d]pyrimidin-2-yl)-(2S)-2,2-dimethylpyrrolidine-1-carboxylate

1,1,-Dimethylethyl-5-(8-bromo-4-oxo-3,4-dihydrobenzofuro[3,2-d]pyrimidin-2-yl)-(2S)-2,2-dimethylpyrrolidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1,-dimethylethyl-5-(5-bromo-2-carbamoylbenzofuran-3-ylcarbamoyl)-(2S)-2,2-dimethylpyrrolidine-1-carboxylate replaced 1,1-dimethylethyl 4-(5-bromo-2-carbamoylbenzofuran-3-ylcarbamoyl)piperidine-1-carboxylate. The crude material from this reaction was air dried and submitted to the next step without any further purification. MS (EI) for C₂₁ H₂₄ Br N₃ O₄: 464 (MH⁺).

### 8-bromo-2-[(2S)-5,5-dimethylpyrrolidin-2-yl][1]benzofuro[3,2-d]pyrimidin-4(3H)-one

8-Bromo-2-[(2S)-5,5-dimethylpyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was substituted with 1,1,-dimethylethyl-5-(8-bromo-4-oxo-3,4-dihydrobenzofuro[3,2-d]pyrimidin-2-yl)-(2S)-2,2-dimethylpyrrolidine-1-carboxylate. ¹H NMR (400 MHz, d₆-DMSO): 8.11 (dd, 1H), 7.74 (dd, 2H), 4.41 (q, 1H), 3.40 (br s , 2H), 2.39 (m, 1H), 2.10 (m, 1H), 1.69 (t, 1.69), 1.28 (s, 3H), 1.25 (s, 3H). MS (EI) for C₁₆ H₁₆ Br N₃ O₂: 364 (MH⁺).

### (Compound 470)

### 1,1-dimethylethyl (2S)-2-({[2-(aminocarbonyl)-5-bromo-1-benzofuran-3-yl]amino}carbonyl)pyrrolidine-1-carboxylate

1,1-dimethylethyl (2S)-2-({[2-(aminocarbonyl)-5-bromo-1-benzofuran-3-yl]amino}carbonyl)pyrrolidine-1-carboxylate was synthesized in a manner similar to **Example 37**, 1,1-dimethylethyl 3-(5-bromo-2-carbamoylbenzofuran-3-ylcarbamoyl)azetidine-1-carboxylate. N-(tert-butoxycarbonyl)-L-proline was substituted with Boc-3-azetidine carboxylic acid. The crude material from this reaction was submitted to the next step without any further purification. MS (EI) for C₁₉H₂₂BrN₃O₅: 452.01 (MH⁺).

### 1,1-dimethylethyl (2S)-2-(8-bromo-4-oxo-3,4-dihydro[1]benzofuro[3,2-d]pyrimidin-2-yl)pyrrolidine-1-carboxylate

1,1-dimethylethyl (2S)-2-(8-bromo-4-oxo-3,4-dihydro[1]benzofuro[3,2-d]pyrimidin-2-yl)pyrrolidine-1-carboxylate was synthesized in a similar manner as to 1,1-dimethylethyl 3-(8-bromo-4-oxo-3,4-dihydrobenzofuro[3,2-d]pyrimidin-2-yl)azetidine-1-carboxylate, wherein 1,1-dimethylethyl (2S)-2-({[2-(aminocarbonyl)-5-bromo-1-benzofuran-3-yl]amino}carbonyl)pyrrolidine-1-carboxylate replaced 1,1-dimethylethyl 4-(5-bromo-2-carbamoylbenzofuran-3-ylcarbamoyl)azetidine-1-carboxylate. The crude material from this reaction was submitted to the next step without any further purification. MS (EI) for C₁₉H₂₀BrN₃O₄: 434.06 (MH⁺).

### 8-bromo-2-[(2S)-pyrrolidin-2-yl][1]benzofuro[3,2-d]pyrimidin-4(3H)-one

8-bromo-2-[(2S)-pyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was replaced by 1,1-dimethylethyl (2S)-2-(8-bromo-4-oxo-3,4-dihydro[1]benzofuro[3,2-d]pyrimidin-2-yl)pyrrolidine-1-carboxylate. ¹H NMR (400 MHz, d₆-DMSO): 13.51 (s, 1H), 10.39 (s, 1H), 9.08 (s, 1H), 8.22 (m, 1H), 7.88 (m, 2H), 4.78 (m, 1H), 3.50 (m, 2H), 2.45 (m, 1H), 2.14 (m, 1H), 2.04 (m, 2H). MS (EI) for C₁₄H₁₂BrN₃O₂: 333.92 (MH⁺).

### (Compound 488)

### 1,1-dimethylethyl (2S,4R)-2-({[2-(aminocarbonyl)-5-bromo-1-benzofuran-3-yl]amino}carbonyl)-4-hydroxypyrrolidine-1-carboxylate

1,1-dimethylethyl (2S,4R)-2-({[2-(aminocarbonyl)-5-bromo-1-benzofuran-3-yl]amino}carbonyl)-4-hydroxypyrrolidine-1-carboxylate was synthesized in a manner similar **Example 37**, 1,1-dimethylethyl 3-(5-bromo-2-carbamoylbenzofuran-3-ylcarbamoyl)azetidine-1-carboxylate. trans-N-(tert-butoxycarbonyl)-4-hydroxy-L-proline replaced Boc-3-azetidine carboxylic acid. The crude material from this reaction was submitted to the next step without any further purification. MS (EI) for C₂₈H₂₉Br₂N₅O₈: 468.01 (MH⁺)_{.}

### 1,1-dimethylethyl (2S,4R)-2-(8-bromo-4-oxo-3.4-dihydro[1]benzofuro[3,2-d]pyrimidin-2-yl)-4-hydroxypyrrolidine-1-carboxylate

1, 1-dimethylethyl (2*S*,4*R*)-2-(8-bromo-4-oxo-3,4-dihydro[1]benzofuro[3,2-*d*]pyrimidin-2-yl)-4-hydroxypyrrolidine-1-carboxylate was synthesized in a similar manner as to 1,1-Dimethylethyl 3-(8-bromo-4-oxo-3,4-dihydrobenzofuro[3,2-d]pyrimidin-2-yl)azetidine-1-carboxylate wherein 1,1-dimethylethyl (2*S*,4*R*)-2-({[2-(aminocarbonyl)-5-bromo-1-benzofuran-3-yl]amino}carbonyl)-4-hydroxypyrrolidine-1-carboxylate replaced 1,1-dimethylethyl 4-(5-bromo-2-carbamoylbenzofuran-3-ylcarbamoyl)azetidine-1-carboxylate. ¹H NMR (400 MHz, d₆-DMSO): 8.20 (s, 1H), 7.84 (s, 2H), 5.17 (bs, 1H), 4.77 (m, 1H), 4.38 (bs, 1H), 3.69 (m, 2H), 2.24 (m, 1H), 1.09 (s, 9H). MS (EI) for C₁₉H₂₀BrN₃O₅: 451.89 (MH⁺).

### 8-bromo-2-[(2S,4R)-4-hydroxypyrrolidin-2-yl][1]benzofuro[3,2-d]pyrimidin-4(3H)-one

8-bromo-2-[(2S,4R)-4-hydroxypyrrolidin-2-yl][1]benzofuro[3,2-d]pyrimidin-4(3H)-one was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was replaced by 1,1-dimethylethyl (2*S*,4*R*)-2-(8-bromo-4-oxo-3,4-dihydro[1]benzofuro[3,2-*d*]pyrimidin-2-yl)-4-hydroxypyrrolidine-1-carboxylate. ¹H NMR (400 MHz, d₆-DMSO): 8.05 (s, 1H), 7.63 (s, 2H), 4.70 (bs, 1H), 4.24 (bs, 2H), 1.94 (bs, 2H). MS (EI) for C₁₄H₁₂BrN₃O₃: 350.05 (MH⁺).

### *(Compound 300)

### 1,1-Dimethylethyl 6-(5-bromo-2-carbamoylbenzofuran-3-ylcarbamoyl)-3,4-dihydroisoquinoline-2(1H)-carboxylate

1,1-Dimethylethyl 6-(5-bromo-2-carbamoylbenzofuran-3-ylcarbamoyl)-3,4-dihydroisoquinoline-2(1H)-carboxylate was synthesized in a manner similar to 1,1-dimethylethyl 3-(5-bromo-2-carbamoylbenzofuran-3-ylcarbamoyl)azetidine-1-carboxylate, wherein 2-(tert-butoxycarbonyl)-1,2,3,4-tetrahydroisoquinoline-6-carboxylic acid (commercially available from ASW MedChem, Inc.,) replaced Boc-3-azetidine carboxylic acid. The crude material from this reaction was submitted to the next step without any further purification. MS (EI) for C₂₄ H₂₄ Br N₃ O₅: 515 (MH⁺).

### 1,1-Dimethylethyl 6-(8-bromo-4-oxo-3,4-dihydrobenzofuro[3,2-d]pyrimidin-2-yl)-3,4-dihydroisoquinoline-2(1H)-carboxylate

1,1-Dimethylethyl 6-(8-bromo-4-oxo-3,4-dihydrobenzofuro[3,2-d]pyrimidin-2-yl)-3,4-dihydroisoquinoline-2(1H)-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl 6-(5-bromo-2-carbamoylbenzofuran-3-ylcarbamoyl)-3,4-dihydroisoquinoline-2(1H)-carboxylate replaced 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₁₉ H₁₄ Br N₃O₂: 497 (MH⁺).

### *8-Bromo-2-(1,2,3,4-tetrahydroisoquinolin-6-yl)[1]benzofuro[3,2-d]pyrimidin-4(3H)-one (Compound 300)

8-Bromo-2-(1,2,3,4-tetrahydroisoquinolin-6-yl)[1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 6-(8-bromo-4-oxo-3,4-dihydrobenzofuro[3,2-d]pyrimidin-2-yl)-3,4-dihydroisoquinoline-2(1H)-carboxylate ¹H NMR (400 MHz, d₆-DMSO): 13.20 (br s, 1H), 9.71 (br s, 2H), 8.28 (s, 1H), 8.05 (s, 1H0, 8.03 (d, 1H), 7.85 (m, 2H), 7.42 (d, 1H), 4.35 (s, 2H), 4.41 (s, 2H), 3.12 (t, 2H). MS (EI) for C₁₉ H₁₄ Br N₃ O₂: 397 (MH⁺).

### *(Compound 301)

### 1,1-Dimethylethyl 2-(5-bromo-2-carbamoylbenzofuran-3-ylcarbamoyl)-5,6-dihydroimidazo[1,2-a]pyrazine-7(8H)-carboxylate

1, 1-Dimethylethyl 2-(5-bromo-2-carbamoylbenzofuran-3-ylcarbamoyl)-5,6-dihydroimidazo[1,2-a]pyrazine-7(8H)-carboxylate was synthesized in a manner similar to 1,1-dimethylethyl 3-(5-bromo-2-carbamoylbenzofuran-3-ylcarbamoyl)azetidine-1-carboxylate, wherein 7-(tert-butoxycarbonyl)-5,6,7,8-tetrahydroimidazo[1,2-a]pyrazine-2-carboxylic acid (commercially available from J & W PharmLab LLC) replaced Boc-3-azetidine carboxylic acid. The crude material from this reaction was submitted to the next step without any further purification. MS (EI) for C₂₁ H₂₂ Br N₅ O₅: 505 (MH⁺).

### 1,1-Dimethylethyl 2-(8-bromo-4-oxo-3,4-dihvdrobenzofuro[3,2-d]pyrimidin-2-yl)-5,6-dihydroimidazo[1,2-a]pyrazine-7(8H)-carboxylate

The intermediate 1,1-dimethylethyl 2-(8-bromo-4-oxo-3,4-dihydrobenzofuro[3,2-d]pyrimidin-2-yl)-5,6-dihydroimidazo[1,2-a]pyrazine-7(8H)-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1, 1-dimethylethyl 2-(5-bromo-2-carbamoy-lbenzofuran-3-ylcarbamoyl)-5,6-dihydroimidazo[1,2-a]pyrazine-7(8H)-carboxylate replaced 1,1-dimethylethyl 4-(5-bromo-2-carbamoylbenzofuran-3-ylcarbamoyl)-piperidine-1-carboxylate. The crude material from this reaction was used in the subsequent step without any further purification. MS (EI) for C₂₁ H₂₀ Br N₅ O₄: 487 (MH⁺).

### *(Compound 301)

### 8-Bromo-2-(5,6,7,8-tetrahydroimidazo[1,2-a]pyrazin-2-yl)[1]benzofuro[3,2-d]pyrimidin-4(3H)-one

8-Bromo-2-(5,6,7,8-tetrahydroimidazo[1,2-a]pyrazin-2-yl)[1]benzofuro[3,2-d]pyrimidin-4(3H)-one as its hydrochloride salt, was synthesized in a similar manner to 8-bromo-2-pipendin-4-yl[1]benzofuro[3,2-d]pynmidin-4(3H)-one **(Compound 157)**, wherein 1,1-dimethylethyl 4-(8-bromo-4-oxo-3,4-dihydrobenzofuro [3,2-d]pyrimidin-2-yl)piperidine-1-carboxylate was substituted with 1,1-dimethylethyl 2-(8-bromo-4-oxo-3,4-dihydrobenzofuro[3,2-d]pyrimidin-2-yl)-5,6-dihydroimidazo[1,2-a]pyrazine-7(8H)-carboxylate. ¹H NMR (400 MHz, d₆-DMSO): 10.34 (br s, 1H), 8.15 (m, 1H), 7.83 (m, 2H), 4.78 (br s, 2H), 4.47 (s, 2H), 4.41 (t, 2H), 3.66 (s, 2H). MS (EI) for C₁₆ H₁₂ Br N₅ O₂: 389 (MH⁺).

### *(Compound 302)

### 1,1-Dimethylethyl 3-(5-bromo-2-carbamoylbenzofuran-3-ylcarbamoyl)benzylcarbamate

The intermediate 1,1-Dimethylethyl 3-(5-bromo-2-carbamoylbenzofuran-3-ylcarbamoyl)benzylcarbamate was synthesized in a manner similar to 1,1-dimethylethyl 3-(5-bromo-2-carbamoylbenzofuran-3-ylcarbamoyl)azetidine-1-carboxylate, wherein 3-((tert-butoxycarbonylamino)-methyl)benzoic acid (commercially available from CNH Technologies, Inc.,) replaced Boc-3-azetidine carboxylic acid. The crude material from this reaction was submitted to the next step without any further purification. MS (EI) for C₂₂ H₂₂ Br N₃ O₅: 489 (MH⁺).

### 1,1-Dimethylethyl 3-(8-bromo-4-oxo-3.4-dihydrobenzofuro[3,2-d]pyrimidin-2-yl)benzylcarbamate

1, 1-Dimethylethyl 3-(8-bromo-4-oxo-3,4-dihydrobenzofuro[3,2-d]pyrimidin-2-yl)benzylcarbamate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl 3-(5-bromo-2-carbamoylbenzofuran-3-ylcarbamoyl)benzylcarbamate replaced 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₂₂ H₂₀ Br N₃ O₄: 471 (MH⁺).

### 2-[3-(Aminomethyl)phenyl]-8-bromo[1]benzofuro[3,2-d]pyrimidin-4(3H)-one

### *(Compound 302)

2-[3-(Aminomethyl)phenyl]-8-bromo[1]benzokro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 3-(8-bromo-4-oxo-3,4-dihydrobenzofuro[3,2-d]pyrimidin-2-yl)benzylcarbamate. ¹H NMR (400 MHz, d₆-DMSO): 13.20 (br s, 1H), 6.56 (br s, 3H), 8.33 (s, 1H), 8.24 (d, 1H), 8.15 (d, 1H), 7.86 (m, 2H), 7.75 (d, 1H), 7.62 (t, 1H), 4.14 (q, 2H). MS (EI) for C₁₇ H₁₂ Br N₃ O₂: 370 (MH⁺).

### *(Compound 303)

### 1,1-Dimethylethyl 4-(4-(5-bromo-2-carbamoylbenzofuran-3-ylcarbamoyl)phenyl)piperazine-1-carboxylate

The intermediate 1, 1-Dimethylethyl 4-(4-(5-bromo-2-carbamoylbenzofuran-3-ylcarbamoyl) phenyl)piperazine-1-carboxylate was synthesized in a manner similar to 1,1-dimethylethyl 3-(5-bromo-2-carbamoylbenzofuran-3-ylcarbamoyl)azetidine-1-carboxylate, wherein 4-(4-(tert-butoxycarbonyl)piperazin-1-yl)benzoic acid (commercially available from CHESS GmbH.) replaced Boc-3-azetidine carboxylic acid. The crude material from this reaction was submitted to the next step without any further purification. MS (EI) for C₂₅ H₂₇ Br N₄ O₅: 545 (MH⁺).

### 1,1-Dimethylethyl 4-(4-(8-bromo-4-oxo-3,4-dihydrobenzofuro[3,2-d]pyrimidin-2-yl)phenyl)piperazine-1-carboxylate

1, 1-Dimethylethyl 4-(4-(8-bromo-4-oxo-3,4-dihydrobenzofuro[3,2-d]pyrimidin-2-yl)phenyl)piperazine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, using 1M potassium hydroxide and isopropyl alcohol as solvent, wherein 1,1-dimethylethyl4-(4-(5-bromo-2-carbamoylbenzofuran-3-ylcarbamoyl)phenyl)-piperazine-1-carboxylate replaced 1,1-dimethylethyl 4-(5-bromo-2-carbamoylbenzofuran-3-ylcarbamoyl)piperidine-1-carboxylate. The crude product from this reaction was used in the subsequent step without any further purification. MS (EI) for C₂₅ H₂₇ Br N₄ O₅: 526 (MH⁺).

### 8-Bromo-2-(4-piperazin-1-ylphenyl)[1]benzofuro[3,2-d]pyrimidin-4(3H)-one

### *(Compound 303)

8-Bromo-2-(4-piperazin-1-ylphenyl)[1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 4-(4-(8-bromo-4-oxo-3,4-dihydrobenzofuro[3,2-d]pyrimidin-2-yl)phenyl)piperazine-1-carboxylate. ¹H NMR (400 MHz, d₆-DMSO):13.02 (br s, 1H), 8.27 (dd, 1H), 8.15 (d, 2H), 7.84 (d, 2H), 7.13 (d, 2H), 4.98 (br s, 2H), 3.58 (t, 4H), 3.22 (br s, 4H). MS (EI) for C₂₀ H₁₇ Br N₄ O₂: 426 (MH⁺).

### (Compound 304)

### 1-(1-Benzylpiperidin-4-yl)-5-oxopyrrolidine-3-carboxylic acid

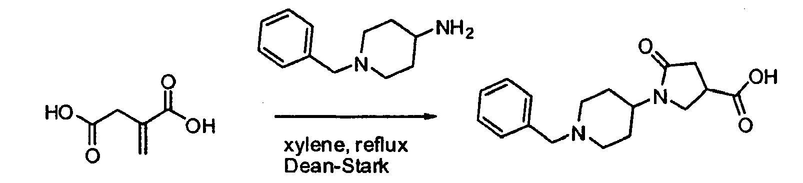

A rapidly stirred mixture of itaconic acid (80 mmoles, 10.4 g, commercially available from Sigma-Aldrich) and 1-benzylpiperidin-4-amine (81.56 mmoles, 15.52 g, commercially available from Alfa-Aesar) in 100 ml of xylene was heated at reflux for 4 hours. Water (1.5 ml) was removed from the system via a Dean-Stark trap. The reaction was allowed to cool to 100 C and the resulting solid was filtered off. The recovered solid was washed with warm xylene and diethyl ether to give 18.9 g of 1-(1-benzylpiperidin-4-yl)-5-oxopyrrolidine-3-carboxylic acid.. ¹H NMR (400 MHz, d₆-DMSO): 12.16 (br s, 1H), 7.38 (m, 2H), 7.31 (m, 1H), 7.27 (m, 2H), 3.78 (m, 1H), 3.89 (s, 2H), 3.52 (m, 1H), 3.77 (m, 2H), 3.36 (m, 1H), 2.54 (m, 1H), 2.43 (m, 1H), 1.91 (m, 2H), 1.63 (m, 2H). MS (EI) for C₁₇ H₂₂ N₂ O₃: (MH⁺).

### 1-(1-(tert-Butoxycarbonyl)piperidin-4-yl)-5-oxopyrrolidine-3-carboxylic acid

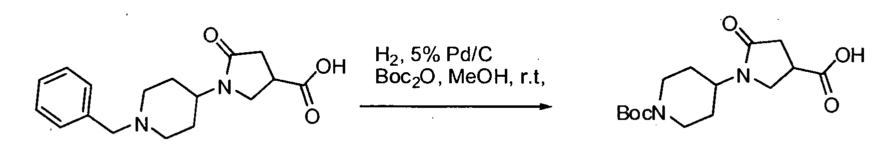

1-(1-Benzylpiperidin-4-yl)-5-oxopyrrolidine-3-carboxylic acid (33.11 mmoles, 10 g) was suspended in 100 ml of anhydrous methanol. Boc anhydride (41.22 mmoles, 9.0 g) and 5% palladium on charcoal (0.5 g, 50% w/w water) were added. The resulting mixture was hydrogenated at 1 atmosphere of hydrogen on a Parr hydrogenator until hydrogen uptake ceased. The solution was the filtered through a Celite plug. The plug was washed with additional methanol (2 x 20 ml) and the resulting filtrate was evaporated under reduced pressure to give an off-white solid. The crude product was subsequently recrystallized from ethanol/water to yield 7.8 g of 1-(1-(tert-butoxycarbonyl)piperidin-4-yl)-5-oxopyrrolidine-3-carboxylic acid as a white solid. ¹H NMR (400 MHz, d₆-DMSO): 12.67 (br s, 1H), 3.99 (br s, 2H), 3.89 (m, 1H), 3.51 (t, ). MS (EI) for C₁₅H₂₄N₂O₅: 313 (MH⁺).

### 1,1-Dimethylethyl 4-(4-(8-bromo-4-oxo-3,4-dihydrobenzofuro[3,2-d]pyrimidin-2-yl)-2-oxopyrrolidin-1-yl)piberidine-1-carboxylate

1, 1-Dimethylethyl 4-(4-(8-bromo-4-oxo-3,4-dihydrobenzofuro[3,2-d]pyrimidin-2-yl)-2-oxopyrrolidin-1-yl)piperidine-1-carboxylate was synthesized in a manner similar to 1,1-dimethylethyl 3-(5-bromo-2-carbamoylbenzofuran-3-ylcarbamoyl)azetidine-1-carboxylate wherein 1-(1-(tert-butoxycarbonyl)piperidin-4-yl)-5-oxopyrrolidine-3-carboxylic acid replaced Boc-3-azetidine carboxylic acid. The crude material from this reaction was submitted to the next step without any further purification. MS (EI) for C₂₄ H₂₉ Br N₄ O₆: 550 (MH⁺).

### 1,1-Dimethylethyl 4-(4-(5-bromo-2-carbamoylbenzofuran-3-ylcarbamoyl)-2-oxopyrrolidin-1-yl)piperidine-1-carboxylate

1, 1-Dimethylethyl 4-(4-(5-bromo-2-carbamoylbenzofuran-3-ylcarbamoyl)-2-oxopyrrolidin-1-yl)piperidine-1-carboxylate was synthesized in a similar manner as to 1,1-dimethylethyl4-(8-bromo-4-oxo-3,4-dihydrobenzofuro[3,2-d]pyrimidin-2-yl)piperidine-1-carboxylate, wherein 1,1-dimethylethyl 4-(4-(8-bromo-4-oxo-3,4-dihydrobenzofuro[3,2-d]pyrimidin-2-yl)-2-oxopyrrolidin-1-yl)piperidine-1-carboxylate was substituted with 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₂₄H₂₇BrN₄O₅: 532 (MH⁺).

### (Compound 304)

### 8-Bromo-2-(5-oxo-1-piperidin-4-ylpyrrolidin-3-yl)[1]benzofuro[3,2-d]pyrimidin-4(3H)-one

8-Bromo-2-(5-oxo-1-piperidin-4-ylpyrrolidin-3-yl)[1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one (Compound 157.
¹H NMR (400 MHz, d₆-DMSO): 13.08 (br s, 1H), 8.79 (br s, 1H), 8.61 (br s, 1H), 8.15 (d, 1H), 7.84 (m, 2H), 4.11 (m, 1H), 3.70 (m, 3H), 3.30 (m, 2H), 3.01 (m, 2H), 2.75 (d, 2H), 1.93 (m, 2H), 1.77 (m, 2H). MS (EI) for C₁₉ H₁₉ Br N₄O₃: 432 (MH⁺).

### *(Compound 349)

### 1,1-Dimethylethyl 4-(5-bromo-2-carbamoylbenzofuran-3-ylcarbamoyl)benzylcarbamate

The intermediate 1,1-Dimethylethyl 4-(5-bromo-2-carbamoylbenzofuran-3-ylcarbamoyl)benzylcarbamate was synthesized in a manner similar to 1,1-dimethylethyl 3-(5-bromo-2-carbamoylbenzofuran-3-ylcarbamoyl)azetidine-1-carboxylate wherein 4-((tert-butoxycarbonylamino)methyl)-benzoic acid (commercially available from CNH Technologies, Inc.) replaced Boc-3-azetidine carboxylic acid. The crude material from this reaction was submitted to the next step without any further purification. MS (EI) for C₂₂ H₂₂ Br N₃ O₅: 489 (MH⁺).

### 1,1-Dimethylethyl 4-(8-bromo-4-oxo-3,4-dihydrobenzofuro[3,2-d]pyrimidin-2-yl)benzylcarbamate

1, 1-Dimethylethyl 4-(8-bromo-4-oxo-3,4-dihydrobenzofuro[3,2-d]pyrimidin-2-yl)benzylcarbamate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl 4-(5-bromo-2-carbamoylbenzofuran-3-ylcarbamoyl) benzylcarbamate replaced 1,1-dimethylethyl 4-(5-bromo-2-carbamoyl-benzofuran-3-ylcarbamoyl)piperidine-1-carboxylate. The resulting crude material from this reaction was used in the subsequent step without any further purification. MS (EI) for C₂₂ H₂₀ Br N₃ O₄: 471 (MH⁺).

### 2-[4-(Aminomethyl)phenyl]-8-bromo[1]benzofuro[3,2-d]pyrimidin-4(3H)-one

2-[4-(Aminomethyl)phenyl]-8-bromo[1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157)**, wherein 1, 1-dimethylethyl 4-(8-bromo-4-oxo-3,4-dihydrobenzofuro[3,2-d]pyrimidin-2-yl)piperidine-1-carboxylate was substituted with 1,1-dimethylethyl 4-(8-bromo-4-oxo-3,4-dihydrobenzofuro[3,2-d]pyrimidin-2-yl)benzylcarbamate. ¹H NMR (400 MHz, d₆-DMSO): 13.27 (br s, 1H), 8.58 (br s, 3H), 8.28 (s, 1H), 8.21 (d, 2H), 7.86 (m, 2H), 7.69 (d, 2H), 4.14 (s, 2H). MS (EI) for C₁₇ H₁₂ Br N₃ O₂: 371 (MH⁺).

### *(Compound 350)

### 1,1-Dimethylethyl 3-(2-(5-bromo-2-carbamoylbenzofuran-3-ylamino)-2-oxoethyl)-4-oxo-1-phenyl-1,3,8-triazaspiro[4.5]decane-8-carboxylate

1,1-Dimethylethyl 3-(2-(5-bromo-2-carbamoylbenzofuran-3-ylamino)-2-oxoethyl)-4-oxo-1-phenyl-1,3,8-triazaspiro[4.5]decane-8-carboxylate was synthesized in a manner similar to 1,1-dimethylethyl 3-(5-bromo-2-carbamoylbenzofuran-3-ylcarbamoyl)azetidine-1-carboxylate wherein 2-(8-(tert-butoxycarbonyl)-4-oxo-1-phenyl-1,3,8-triazaspiro[4.5]decan-3-yl)acetic acid (commercially available from NeoMPS group SNPE) replaced Boc-3-azetidine carboxylic acid. The crude material from this reaction was submitted to the next step without any further purification. MS (EI) for C₂₉ H₃₂ Br N₅O₆: 627 (MH⁺).

### 1,1-Dimethylethyl 3-((8-bromo-4-oxo-3,4-dihydrobenzofuro [3,2-d]pyrimidin-2-yl)methyl)-4-oxo-1-phenyl-1,3,8-triazaspiro[4.5]decane-8-carboxylate

1,1-Dimethylethyl3-((8-bromo-4-oxo-3,4-dihydrobenzofuro[3,2-d]pyrimidin-2-yl)methyl)-4-oxo-1-phenyl-1,3,8-triazaspiro[4.5]decane-8-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl 3-(2-(5-bromo-2-carbamoylbenzofuran-3-ylamino)-2-oxoethyl)-4-oxo-1-phenyl-1,3,8-triazaspiro[4.5]-decane-8-carboxylate replaced 1,1-dimethylethyl 4-(5-bromo-2-carbamoyl-benzofuran-3-ylcarbamoyl)piperidine-1-carboxylate. The pure crude material from this reaction was used in the subsequent step without any further purification. ¹H NMR (400 MHz, d₆-DMSO): 13.21 (br s, 1H), 8.02 (s, 1H), 7.82 (m, 2H), 7.23 (m, 2H), 6.79 (m, 3H), 4.86 (s,2H), 4.65 (s, 2H), 3.90 (, 2H), 4.41 (m, 1H), 2.45 (m, 2H), 1.83 (d, 2H), 1.46 (s, 9H). MS (EI) for C₂₉ H₃₀ Br N₅ O₅: 609 (MH⁺).

### 8-Bromo-2-((4-oxo-1-phenyl-1,3,8-triazaspiro[4.5]decan-3-yl)methyl)benzofuro[3,2-d]pyrimidin-4(3H)-one (Compound 157)

8-Bromo-2-((4-oxo-1-phenyl-1,3,8-triazaspiro[4.5]decan-3-yl)methyl)benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one, wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 3-((8-bromo-4-oxo-3,4-dihydrobenzofuro[3,2-d]pyrimidin-2-yl)methyl)-4-oxo-1-phenyl-1,3,8-triazaspiro[4.5]-decane-8-carboxylate. ¹H NMR (400 MHz, d₆-DMSO): 13.26 (br s, 1H), 9.10 (br d, 2H), 8.07 (d, 1H), 7.83 (q, 2H), 7.27 (dd, 2H), 7.05 (d, 2H), 6.84 (1H), 4.88 (s, 2H), 4.68 (s, 2H), 3.58 (m, 4H), 2.85 dt, 2H), 2.00 (d, 2H). MS (EI) for C₂₄ H₂₂ Br N₅ O₃: 509 (MH⁺).

### *(Compound 351)

### Ethyl 3-hydroxy-1-methyl-1H-pyrazole-5-carboxylate

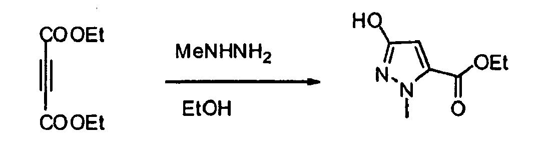

A solution of diethyl acetylene dicarboxylate (100 mmoles. 17.01 g, 16.05 ml, commercially available from Sigma-Aldrich) in anhydrous ethanol (100 ml) was added in drop wise fashion to a stirred solution of methyl hydrazine (100 mmoles, 4.607, 5.35 ml) and anhydrous ethanol (150 ml), at room temperature, over the course of 9 hours. Upon completion of addition, the reaction mixture was stirred at room temperature for a further 20 hours. The reaction mixture was concentrated under reduced pressure and the resulting residue was triturated with cold acetonitrile to give a white solid. The solid was filtered off, washed with diethyl ether and dried under reduced pressure to give 9.0g of ethyl 3-hydroxy-1-methyl-1H-pyrazole-5-carboxylate as a white solid. ¹H NMR (400 MHz, d₆-DMSO): 11.29 (br s, 1H), 6.51 (s, 1H), 4.32 (q, 2H), 4.12 (s, 3H), 1.43 (t, 3H). MS (EI) for C₇H₁₀N₂O₃: 171. (MH⁺).

### 1,1-Dimethylethyl 4-(5-(ethoxycarbonyl)-1-methyl-1H-pyrazol-3-yloxy)piperidine-1-carboxylate

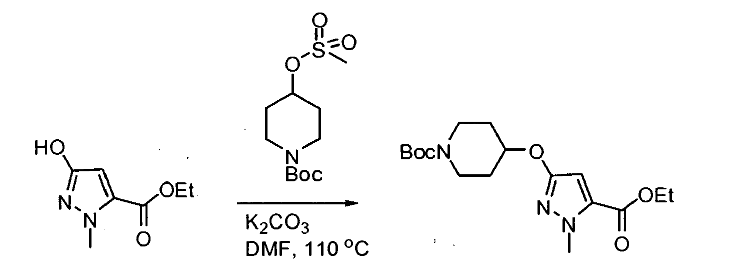

Anhydrous potassium carbonate (125 mmoles, 17.25 g) was added to a stirred solution of ethyl 3-hydroxy-1-methyl-1H-pyrazole-5-carboxylate (50 mmoles, 8.58 g,), 1,1-dimethylethyl 4-(methylsulfonyloxy)piperidine-1-carboxylate (50 mmoles, 17.25 g, commercially available from 3B Medical Systems, Inc.) in anhydrous anhydrous DMF (100 mL) at room temperature. The reaction mixture was then stirred at 110 °C for 8 hours. The reaction mixture was then allowed to cool to room temperature and diluted with cold water (500 ml). The mixture was then extracted with ethyl acetate (4 x 250ml). The combined ethyl acetate solution was then washed with water (3 x 200 ml), saturated sodium chloride solution (250 ml), dried over anhydrous sodium sulfate, filtered off and evaporated under reduced pressure. The crude material was then recrystallized from benzene (200 ml) to give 10.05 g of 1,1-dimethylethyl 4-(5-(ethoxycarbonyl)-1-methyl-1H-pyrazol-3-yloxy)piperidine-1-carboxylate as an off-white solid. ¹H NMR (400 MHz, d₆-DMSO): 13.39 (br s, 1H), 6.23 (s, 1H), 4.57 (m, 1H), 4.32 (q, 2H), 3.19 (s, 3H), 3.65 (m, 2H), 3.14 (m, 2H), 1.92 (d, 2H), 1.54 (t, 3H), 1.49 (d, 2H), 1.43 (t, 3H), 1.39 (d, 9H). MS (EI) for C₁₇H₂₇N₃ O₅: 354 (MH⁺).

### 1,1-dimethylethyl 4-(5-(carboxy)-1-methyl-1H-pyrazol-3-yloxy)piperidine-1-carboxylate

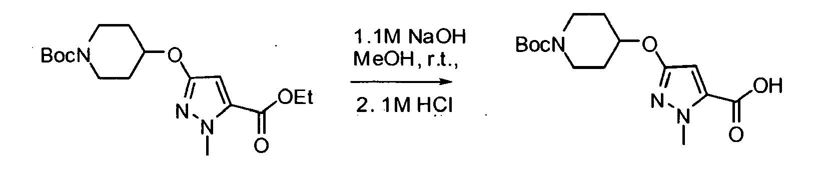

1,1-dimethylethyl4-(5-(ethoxycarbonyl)-1-methyl-1H-pyrazol-3-yloxy)piperidine-1-carboxylate (28.29 mmole, 10 g) was dissolved in methanol (200 ml). Aqueous 1M sodium hydroxide solution (43, 43 mmoles) was added and the reaction mixture was stirred at room temperature for 16 hours. The reaction mixture was cooled to 5 oC (ice-water batch) and acidified to pH 1 with aqueous 1M hydrochloric acid. The resulting white solid was filtered off, washed with cold water (2 x 20 ml) and dried under reduced pressure to give 9.18 g of 1,1-dimethylethyl 4-(5-(carboxy)-1-methyl-1H-pyrazol-3-yloxy)piperidine-1-carboxylate as a white solid. ¹H NMR (400 MHz, d₆-DMSO):, 13.39 (br s, 1H), 6.23 (s, 1H), 4.57 (m, 1H), 4.32 (q, 2H), 3.19 (s, 3H), 3.65 (m, 2H), 3.14 (m, 2H), 1.92 (d, 2H), 1.54 (t, 3H), 1.49 (d, 2H), 1.39 (d, 9H). MS (EI) for C₁₅H₂₃N₃O₅: 326 (MH⁺).

### 1,1-dimethylethyl 4-(5-(5-bromo-2-carbamoylbenzofuran-3-ylcarbamoyl)-1-methyl-1H-pyrazol-3-yloxy)piperidine-1-carboxylate

1, 1-Dimethylethyl 4-(5-(5-bromo-2-carbamoylbenzofuran-3-ylcarbamoyl)-1-methyl-1H-pyrazol-3-yloxy)piperidine-1-carboxylate was synthesized in a manner similar to 1,1-dimethylethyl 3-(5-bromo-2-carbamoylbenzofuran-3-ylcarbamoyl)azetidine-1-carboxylate wherein 1,1-dimethylethyl 4-(5-(carboxy)-1-methyl-1H-pyrazol-3-yloxy)piperidine-1-carboxylate was substituted with Boc-3-azetidine carboxylic acid. The crude material from this reaction was submitted to the next step without any further purification. MS (EI) for C₂₄ H₂₈ Br N₅ O₆: 563 (MH⁺).

### 1,1-dimethylethyl 4-(5-(8-bromo-4-oxo-3,4-dihydrobenzofuro[3,2-d]pyrimidin-2-yl)-1-methyl-1H-pyrazol-3-yloxy)piperidine-1-carboxylate

1, 1-Dimethylethyl 4-(5-(8-bromo-4-oxo-3,4-dihydrobenzofuro[3,2-d]pyrimidin-2-yl)-1-methyl-1H-pyrazol-3-yloxy)piperidine-1-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl 4-(5-(5-bromo-2-carbamoylbenzofuran-3-ylcarbamoyl)-1-methyl-1H-pyrazol-3-yloxy)piperidine-1-carboxylate was substituted with 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₂₄ H₂₆ Br N₅ O₅: 545 (MH⁺).

### 8-Bromo-2-[1-methyl-3-(piperidin-4-yloxy)-1H-pyrazol-5-yl][1]benzofuro[3,2-d]pyrimidin-4(3H)-one *(Compound 351)

8-Bromo-2-[1-methyl-3-(piperidin-4-yloxy)-1H-pyrazol-5-yl][1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 4-(5-(8-bromo-4-oxo-3,4-dihydrobenzofuro[3,2-d]pyrimidin-2-yl)-1-methyl-1H-pyrazol-3-yloxy)piperidine-l-carboxylate. ¹H NMR (400 MHz, d₆-DMSO): 13.14 (br s, 1H), 9.00 (br s, 2H), 8.31 (d, 1H), 7.60 (m, 2H), 6.5 (s, 1H), 4.71 (m, 1H), 4.12 (s, 3H), 3.22 (m, 2H), 3.08 (m, 2H), 2.17 (m, 2H), 1.90 (m, 2H). MS (EI) for C₁₉ H₁₈ Br N₅ O₃ : 445 (MH⁺).

### *(Compound 352)

### 1,1-Dimethylethyl (4-(5-bromo-2-carbamoylbenzofuran-3-ylcarbamoyl)thiazol-2-yl)methylcarbamate

1, 1-Dimethylethyl (4-(5-bromo-2-carbamoylbenzofuran-3-ylcarbamoyl)thiazol-2-yl)methylcarbamate was synthesized in a manner similar to 1,1-dimethylethyl 3-(5-bromo-2-carbamoylbenzofuran-3-ylcarbamoyl)azetidine-1-carboxylate wherein 2-((tert-butoxycarbonylamino)methyl)thiazole-4-carboxylic acid (prepared by a literature method Tetrahedron (1986), 42:10, 2695) replaced Boc-3-azetidine carboxylic acid. The crude material from this reaction was submitted to the next step without any further purification.. MS (EI) for C₁₉ H₁₉ Br N₄ O₅ S: 496 (MH⁺).

### 1,1-Dimethylethyl (4-(8-bromo-4-oxo-3,4-dihydrobenzofuro[3,2-d]pyrimidin-2-yl)thiazol-2-yl)methylcarbamate

1,1-Dimethylethyl (4-(8-bromo-4-oxo-3,4-dihydrobenzofuro[3,2-d]pyrimidin-2-yl)thiazol-2-yl)methylcarbamate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl (4-(5-bromo-2-carbamoylbenzofuran-3-ylcarbamoyl)thiazol-2-yl)methylcarbamate replaced 1,1-dimethylethyl 4-(5-bromo-2-carbamoylbenzofuran-3-ylcarbamoyl)-piperidine-1-carboxylate. The crude material from this reaction was used in the subsequent step without any further purification. MS (EI) for C₁₉ H₁₇ Br N₄ O₄S: 478 (MH⁺).

### 2-[2-(Aminomethyl)-1,3-thiazol-4-yl]-8-bromo[1]benzofuro[3,2-d]pyrimidin-4(3H)-one

2-[2-(Aminomethyl)-1,3-thiazol-4-yl]-8-bromo[1]benzofuro[3,2-d]pyrimidin-4(3H)-one **(Compound 352)** as its hydrochloride salt, was synthesized in a similar manner to 8-bromo-2-piperidin-4-yl[l]benzofuro[3,2-d]pyrimidin-4(3H)-one **(Compound 157)** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl (4-(8-bromo-4-oxo-3,4-dihydrobenzofuro[3,2-d]pyrimidin-2-yl)thiazol-2-yl)methylcarbamate. ¹H NMR (400 MHz, d₆-DMSO): 12.71 (br s, 1H), 8.66 (s, 1H), 8.23 (d, 1H), 7.95 (m, 2H), 4.66 (d, 2H), 3.5 (br s, 3H). MS (EI) for C₁₄ H₉ Br N₄ O₂ S: 378 (MH⁺).

### *(Compounds 353)

### 8-promo-2-{3-[(dimethylamino)methyl]phenyl}[1]benzokro[3,2-d]pyrimidin-4(3H)-one

8-Bromo-2-{3-[(dimethylamino)methyl]phenyl}[1]benzofuro[3,2-d]pyrimidin-4(3H)-one as its acetate salt was synthesized in a manner similar to 8-bromo-2-(1-methylpiperidin-3-yl)[1]benzofuro[3,2-d]pyrimidin-4(3H)-one, wherein 2-[3-(Aminomethyl)phenyl]-8-bromo[1]benzofuro[3,2-d]pyrimidin-4(3H)-one **(Compound 302)** replaced 8-bromo-2-piperidin-3-yl[1]benzofuro[3,2-d]pyrimidin-4(3H)-one dihydrochloride **(Compound 184).** ¹H NMR (400 MHz, d₆-DMSO): 12.04 (br s, 1H), 8.19 (s, 1H), 8.11 (s, 2H), 7.83 (s, 2H), 7.57 (d, 2H), 3.85 (s, 2H), 3.44 (br s, 3H), 2.43 (s, 6H)._MS (EI) for C₁₉ H₁₆BrN3 O₂: 399 (MH⁺).

### *(Compound 354)

### 1,1-Dimethylethyl-4-(5-bromo-2-carbamoylbenzofuran-3-ylcarbamoyl)phenylcarbamate

1, 1-Dimethylethyl 4-(5-bromo-2-carbamoylbenzofuran-3-ylcarbamoyl)phenylcarbamate was synthesized in a manner similar to 1,1-dimethylethyl 3-(5-bromo-2-carbamoylbenzofuran-3-ylcarbamoyl)azetidine-1-carboxylate wherein 4-(tert-butoxycarbonylamino)benzoic acid (commercially available from CNH Technologies Inc.,) replaced Boc-3-azetidine carboxylic acid. The crude material from this reaction was submitted to the next step without any further purification. MS (EI) forC₂₁ H₂₀BrN₃O5: 475 (MH⁺).

### 1,1-Dimethylethyl4-(8-bromo-4-oxo-3,4-dihydrobenzofuro[3.2-d]pyrimidin-2-yl)phenylcarbamate

1, 1-Dimethylethyl4-(8-bromo-4-oxo-3,4-dihydrobenzofuro[3,2-d]pyrimidin-2-yl)phenylcarbamate was synthesized in a similar manner as to 1,1-dimethylethyl 4-(8-bromo-4-oxo-3,4-dihydrobenzofuro[3,2-d]pyrimidin-2-yl)piperidine-1-carboxylate wherein 1,1-dimethylethyl4-(5-bromo-2-carbamoylbenzofuran-3-ylcarbamoyl)phenylcarbamate replaced 1,1-dimethylethyl 4-(5-bromo-2-carbamoylbenzofuran-3-ylcarbamoyl)piperidine-1-carboxylate and heated at 100 °C for 16 hours. The crude material from this reaction was used in the subsequent step without any further purification. MS (EI) for C₂₁ H₁₈ Br N₃ O₄: 457 (MH⁺).

### 2-(4-Aminophenyl)-8-bromo[1]benzofuro[3,2-d]pyrimidin-4(3H)-one *(Compound 354)

2-(4-Aminophenyl)-8-bromo[1]benzofuro[3,2-d]pyrimidin-4(3H)-one as its hydrochloride salt, was synthesized in a similar manner to 8-bromo-2-piperidin-4-yl[1]benzofuro[3,2-d]pyrimidin-4(3H)-one **(Compound 157),** wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 4-(8-bromo-4-oxo-3,4-dihydrobenzofuro[3,2-d]pyrimidin-2-yl)phenylcarbamate ¹H NMR (400 MHz, d₆-DMSO): 12.94 (br s, 1H), 8.25 (m, 1H), 8.07 (d, 2H), 7.83 (m, 2H). 6.99 (d, 2H), 4.78 (br s, 3H). MS (EI) for C₁₆ H₁₀ Br N₃ O₂: 357 (MH⁺).

### *(Compound 355)

### 1,1-Dimethylethyl3-(5-bromo-2-carbamoylbenzofuran-3-ylcarbamoyl)phenylcarbamate

1,1-Dimethylethyl 4-(5-bromo-2-carbamoylbenzofuran-3-ylcarbamoyl)phenylcarbamate was synthesized in a manner similar to 1,1-dimethylethyl 3-(5-bromo-2-carbamoylbenzofuran-3-ylcarbamoyl)azetidine-1-carboxylate 3-(tert-butoxycarbonylamino)benzoic acid (commercially available from CNH Technologies Inc.,) replaced Boc-3-azetidine carboxylic acid. The crude material from this reaction was submitted to the next step without any further purification. MS (EI) for C₂₁H₂₀BrN₃O₅: 475 (MH⁺).

### 1,1-Dimethylethyl 3-(8-bromo-4-oxo-3.4-dihydrobenzofuro[3.2-d]pyrimidin-2-yl)phenylcarbamate

1,1-Dimethylethyl 3-(8-bromo-4-oxo-3,4-dihydrobenzofuro[3,2-d]pyrimidin-2-yl)phenylcarbamate was synthesized in a similar manner as to 1,1-dimethylethyl 3-(8-bromo-4-oxo-3,4-dihydrobenzofuro[3,2-d]pyrimidin-2-yl)piperidine-1-carboxylate, wherein 1,1-dimethylethyl4-(5-bromo-2-carbamoylbenzofuran-3-ylcarbamoyl)phenylcarbamate replaced 1,1-dimethylethyl 4-(5-bromo-2-carbamoylbenzofuran-3-ylcarbamoyl)piperidine-1-carboxylate and heated at 100 °C for 16 hours. The crude material from this reaction was used in the subsequent step without any further purification. MS (EI) for C₂₁H₁₈BrN₃O₄: 457 (MH⁺).

### 2-(3-Aminophenyl)-8-bromo[1]benzofuro[3,2-d]pyrimidin-4(3H)-one *(Compound 355)

2-(3-Aminophenyl)-8-bromo[1]benzokro[3,2-d]pyrimidin-4(3H)-one as its hydrochloride salt, was synthesized in a similar manner to 8-bromo-2-piperidin-4-yl[1]benzokro[3,2-d]pyrimidin-4(3H)-one **(Compound 157)** wherein 1,1-dimethylethyl 3-(8-bromo-4-oxo-3,4-dihydrobenzofuro[3,2-d]pyrimidin-2-yl)piperidine-1-carboxylate was substituted with 1,1-dimethylethyl 4-(8-bromo-4-oxo-3,4-dihydrobenzofuro[3,2-d]pyrimidin-2-yl)phenylcarbamate. ¹H NMR (400 MHz, d₆-DMSO): 13.02 (br s, 1H), 8.26 (s, 1H), 7.84 (d, 2H), 7.47 (m, 1H), 7.23 (d, 1H), 7.03 (m,1H), 6.89 (d, 1H). 6.43 (br s, 3H). MS (EI) for C₁₆ H₁₀ Br N₃ O₂: 357 (MH⁺).

### *(Compounds 358)

### 8-Bromo-2-{4-[(dimethylamino)methyl]phenyl}[1]benzofuro[3,2-d]pyrimidin-4(3H)-one

8-Bromo-2-{4-[(dimethylamino)methyl]phenyl}[1]benzofuro[3,2-d]pyrimidin-4(3H)-one as its acetate salt was synthesized in a manner similar to 8-bromo-2-(1-methylpiperidin-3-yl)[1]benzofuro[3,2-d]pyrimidin-4(3H)-one wherein 2-[4-(aminomethyl)phenyl]-8-bromo[1]benzofuro[3,2-d]pyrimidin-4(3H)-one replaced 8-bromo-2-piperidin-3-yl[1]benzofuro[3,2-d]pyrimidin-4(3H)-one dihydrochloride **(Compound 184)**. ¹H NMR (400 MHz, d₆-DMSO): 12.96 (br s, 1H), 8.26 (s, 1H), 8.15 (d, 2H), 7.84 (s, 2H), 7.48 (d, 2H), 3.51 (s, 2H), 3.35 (br s, 2H), 2.20 (s, 6H). MS (EI) for C₁₉ H₁₆ Br N₃ O₂: 399 (MH⁺).

### *(Compound 359)

### 1,1-Dimethylethyl 2-((4-(methoxycarbonyl)phenoxy)methyl)morpholine-4-carboxylate

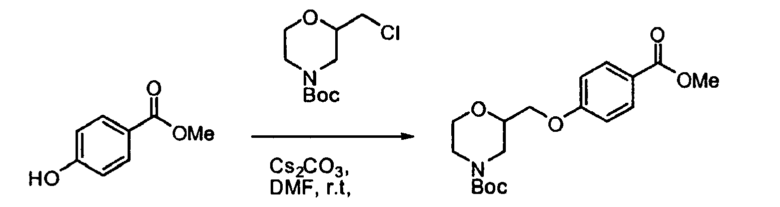

Cesium carbonate (37.5 mmoles, 12.21 g) was added in one lot to a stirred solution of methyl 4-hydroxybenzoate (25 mmole 3.80 g) and 1,1-dimethylethyl 2-(chloromethyl) morpholine-4-carboxylate (25 mmoles 7.36 g), (commercially available from Butt Park Ltd.) in anhydrous DMF. The reaction mixture was then stirred at 80°C for 16 hours. The reaction mixture was then allowed to cool to room temperature, quenched with ice-water and washed with ethyl acetate (4 x 200 ml). The combined organic solution was washed with water (2 x 200 ml), saturated brine (200 ml), dried over anhydrous magnesium chloride, filtered and evaporated under reduced pressure to give 9.02 g of 1,1-dimethylethyl 2-((4-(methoxycarbonyl)phenoxy)methyl) morpholine-4-carboxylate as a white solid, which was used in the nest step without any further purification. ¹H NMR (400 MHz, d₆-DMSO): ¹H NMR (400 MHz, d₆-DMSO): 7.79 (d, 2H), 7.01 (d, 2H), 4.26 (s, 3H), 4.06 (m, 2H), 3.87 (m, 2H), 3.68 (m, 2H), 3.50 (m, 1H), 3.19 (m, 2H), 2.89 (m, 2H), 1.41 (s, 1H). MS (EI) for C₁₈ H₂₅ N O₆: 352 (MH⁺).

### 4-((4-(1,1-Dimethylethoxycarbonyl)morpholin-2-yl)methoxy)benzoicacid

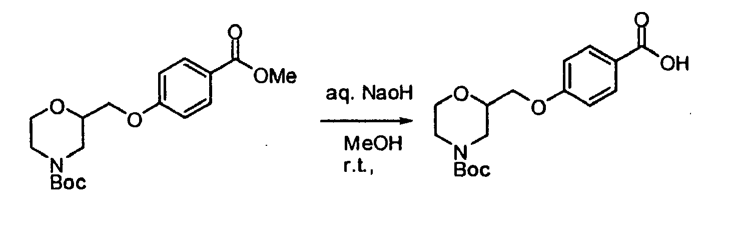

Crude 1,1-dimethylethyl 2-((4-(methoxycarbonyl)phenoxy)methyl)-morpholine-4-carboxylate (25 mmole, 8.77 g) was dissolved in methanol (200 ml). Aqueous 1M sodium hydroxide solution (50 mL, 50 mmoles) was added and the reaction mixture was stirred at room temperature for 36 hours. The reaction mixture was cooled to 5 oC (ice-water batch) and acidified to pH 1 with aqueous 1M hydrochloric acid. The resulting white solid was filtered off, washed with cold water (2 x 20 ml) and dried under reduced pressure to give 8.21 g of 4-((4-(1,1-dimethylethoxycarbonyl)morpholin-2-yl)methoxy)benzoic acid as a off-white solid. ¹H NMR (400 MHz, d₆-DMSO): 12.61 (br s, 1H), 7.78 (d, 2H), 7.03 (d, 2H), 4.06 (m, 2H), 3.87 (m, 2H), 3.68 (m, 2H), 3.50 (m, 1H), 3.19 (m, 2H), 2.89 (m, 2H), 1.41 (s, 1H). MS (EI) for C₁₇ H₂₃ N O₆: 338 (MH⁺).

### 1,1-Dimethylethyl 2-((4-(5-bromo-2-carbamoylbenzofuran-3-ylcarbamoyl)phenoxy)-methyl)morpholine-4-carboxylate

1,1-Dimethylethyl 2-((4-(5-bromo-2-carbamoylbenzofuran-3-ylcarbamoyl)phenoxy)-methyl) morpholine-4-carboxylate was synthesized in a manner similar to 1,1-dimethylethyl 3-(5-bromo-2-carbamoylbenzofuran-3-ylcarbamoyl)azetidine-1-carboxylate, wherein 4-((4-(1,1-dimethylethoxycarbonyl)morpholin-2-yl)methoxy)benzoic acid replaced Boc-3-azetidine carboxylic acid. The crude material from this reaction was submitted to the next step without any further purification. MS (EI) for C₂₆ H₂₈ Br N₃ O₇: 575 (MH⁺).

### 1,1-Dimethylethyl 2-((4-(8-bromo-4-oxo-3,4-dihydrobenzofuro[3,2-d]pyrimidin-2-yl)phenoxy)methyl)morpholine-4-carboxylate

1,1-Dimethylethyl 2-((4-(8-bromo-4-oxo-3,4-dihydrobenzofuro[3,2-d]pyrimidin-2-yl)phenoxy)methyl)morpholine-4-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl 2-((4-(5-bromo-2-carbamoylbenzofuran-3-ylcarbamoyl)phenoxy)methyl)morpholine-4-carboxylate replaced 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₂₆ H₂₆ Br N₃ O₆: 557 (MH⁺).

### 8-Bromo-2-{4-[(morpholin-2-ylmethyl)oxy]phenyl}[1]benzofuro[32-d]pyrimidin-4(3H)-one

8-Bromo-2-(4-[(morpholin-2-ylmethyl)oxy]phenyl)[1]benzofuro[3,2-d]pyrimidin-4(3H)-one **(Compound 359)** as its hydrochloride salt, was synthesized in a similar manner to 8-bromo-2-piperidin-4-yl[1]benzofum[3,2-d]pyrimidin-4(3H)-one (Compound 157), wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 2-((4-(8-bromo-4-oxo-3,4-dihydrobenzofuro [3,2-d]pyrimidin-2-yl)phenoxy)methyl)morpholine-4-carboxylate
¹H NMR (400 MHz, d₆-DMSO): 12.79 (br s, 1H), 8.26 (s, 1H), 8.18 (d, 2H), 7.84 (s, 2H), 7.13 (d, 2H), 4.18 (m, 3H),4.02 (dd, 1H), 3.85 (t, 1H), 3.37 (d, 1H), 2.31 (d, 1H), 2.99 (m, 2H). MS (EI) for C₂, H₁₈ Br N₃ O₄: 457 (MH⁺).

### *(Compound 375)

### 1,1-Dimethylethyl 5-(5-bromo-2-carbamoylbenzofuran-3-ylcarbamo)-3,4-dihdyroisoquinoline-2(1H)-carboxylate

1,1-Dimethylethyl 5-(5-bromo-2-carbamoylbenzofuran-3-ylcarbamoyl)-3,4-dihydroisoquinoline-2(1H)-carboxylate was synthesized in a manner similar to 1,1-dimethylethyl 3-(5-bromo-2-carbamoylbenzofuran-3-ylcarbamoyl)azetidine-1-carboxylate wherein 2-(tert-butoxycarbonyl)-1,2,3,4-tetrahydroisoquinoline-5-carboxylic acid (commercially available from ASW MedChem, Inc.) replaced Boc-3-azetidine carboxylic acid. The crude material from this reaction was submitted to the next step without any further purification. MS (EI) for C₂₄H₂₄BrN₃O₅: 515 (MH⁺).

### 1,1-Dimethylethyl5-(8-bromo-4-oxo-3,4-dihydrobenzofuro[3,2-d]pyrimidin-2-yl)-3,4-dihydroisoquinoline-2(1H)-carboxylate

1,1-Dimethylethyl 5-(8-bromo-4-oxo-3,4-dihydrobenzofuro[3,2-d]pyrimidin-2-yl)-3,4-dihydroisoquinoline-2(1H)-carboxylate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl5-(5-bromo-2-carbamoylbenzofuran-3-ylcarbarnoyl)-3,4-dihydroisoquinoline-2(1H)-carboxylate replaced 1,1-dimethylethyl 4-(5-bromo-2-carbamoylbenzofuran-3-ylcarbamoyl)piperidine-1-carboxylate. The crude material from this reaction was used in the subsequent step without any further purification. ¹H NMR (400 MHz, d₆-DMSO): 12.94 (br s, 1H), 8.24 (s, 1H), 7.85 (m, 2H), 7.43 (d, 1H), 7.38 (m, 2H), 4.60 (s, 2H), 3.52 (s, 2H), 2.86 (s, 2H), 1.44 (s, 9H). MS (EI) for C₂₄H₂₂BrN₃O₄: 467 (MH⁺).

### 8-Bromo-2-(1,2,3,4-tetrahydroisoquinolin-5-yl)[1]benzofuro[3,2-d]pyrimidin-4(3H)-one

8-Bromo-2-(1,2,3,4-tetrahydroisoquinolin-5-yl)[1]benzofuro[3,2-d]pyrimidin-4(3H)-one **(Compound 375)** as its hydrochloride salt, was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl 5-(8-bromo-4-oxo-3,4-dihydrobenzokro[3,2-d]pyrimidin-2-yl)-3,4-dihydroisoquinoline-2(1H)-carboxylate.
¹H NMR (400 MHz, d₆-DMSO): 13.02 (br s, 1H), 9.91 (br s, 2H), 8.18 (d, 1H), 7.87 (q, 2H), 6.54 (dd, 1H), 7.44 (s, 2H), 4.36 (s, 2H), 3.31 (s, 2H), 3.15 (m, 2H). MS (EI) for C₁₉ H₁₄ Br N₃ O₂: 397 (MH⁺).

### *(Compound 376)

### 2-[trans-4-(Aminomethyl)cyclohexyl]-8-bromo[1]benzofuro[3,2-d]pyrimidin-4(3H)-one

### (trans)-4-((tert-butoxycarbonylamino)methyl)cyclohexanecarboxylicacid

1,1-Dimethylethyl (*trans*-4-(5-bromo-2-carbamoylbenzofuran-3-ylcarbamoyl)cyclohexyl) methylcarbamate was synthesized in a manner similar to 1,1-dimethylethyl 3-(5-bromo-2-carbamoylbenzofuran-3-ylcarbamoyl)azetidine-1-carboxylate wherein (*trans*)-4-((tert-butoxycarbonylamino)methyl)cyclohexanecarboxylic acid (commercially available from NeoMPS, Groupe SNPE) replaced Boc-3-azetidine carboxylic acid. The crude material from this reaction was submitted to the next step without any further purification. MS (EI) for C₂₂H₂₈BrN₃O₅: 495 (MH⁺).

### 1,1-Dimethylethyl (trans-4-(8-bromo-4-oxo-3,4-dihydrobenzofuro[3,2-d]pyrimidin-2-yl)cyclohexyl)methylcarbamate

1,1-Dimethylethyl (*trans*-4-(8-bromo-4-oxo-3,4-dihydrobenzofuro[3,2-d]pyrimidin-2-yl)cyclohexyl)methylcarbamate was synthesized in a similar manner as to 1,1-dimethylethyl 4-(8-bromo-4-oxo-3,4-dihydrobenzofuro[3,2-d]pyrimidin-2-yl)piperidine-1-carboxylate, wherein 1,1-dimethylethyl ((1r,4r)-4-(5-bromo-2-carbamoylbenzofuran-3-ylcarbamoyl)cyclohexyl)methylcarbamate replaced 1,1-dimethylethyl 4-(5-bromo-2-carbamoylbenzofuran-3-ylcarbamoyl)piperidine-1-carboxylate. The crude material from this reaction was used in the subsequent step without any further purification. ¹H NMR (400 MHz, d₆-DMSO): 12.80 (br s, 1H), 8.15 (s, 1H), 7.81 (s, 2H), 6.88 (t, 1H), 3.35 (s, 2H), 2.87 (t, 2H), 2.64 (t, 1H), 1.96 (d, 2H), 1.79 (d, 2H), 1.61 (m, 1H), 1.39 (s, 9H), 0.97 (m, 1H). MS (EI) for C₂₂H₂₆Br N₃O₄: 477 (MH⁺).

### 2-[trans-4-(Aminomethyl)cyclohexyl]-8-bromo[1]benzofuro[3,2-d]pyrimidin-4(3H)-one

2-[trans-4-(Aminomethyl)cyclohexyl]-8-bromo[1]benzofuro[3,2-d]pyrimidin-4(3H)-one **(Compound 376)** as its hydrochloride salt, was synthesized in a similar manner to 8-bromo-2-piperidin-4-yl[1]benzofuro[3,2-d]pyrimidin-4(3H)-one **(Compound 157)**, wherein 1,1-dimethylethyl 4-(8-bromo-4-oxo-3,4-dihydrobenzofuro[3,2-d]pyrimidin-2-yl)piperidine-1-carboxylate was substituted with 1,1-dimethylethyl ((trans)-4-(8-bromo-4-oxo-3,4-dihydrobenzofuro[3,2-d]pyrimidin-2-yl)cyclohexyl)methylcarbamate. ¹H NMR (400 MHz, d₆-DMSO):12.86 (br s, 1H), 816 (s, 1H), 8.02 (br s, 3H), 7.81 (s, 2H), 2.70 (m, 2H), 1.98 (m, 4H), 1.65 (m, 3H), 1.07 (m, 2H). MS (EI) for C₁₇H₁₈BrN₃O₂: 377(MH⁺).

### *(Compound 377)

### 8-Bromo-2-(2-piperidin-4-ylpyrazolo[1,5-a]pyrimidin-6-yl)[1]benzofuro[3,2-d]pyrimidin-4(3H)-one 1,1-Dimethylethyl 4-(5-amino-1H-pyrazol-3-ly)piperidine-1-carboxylate

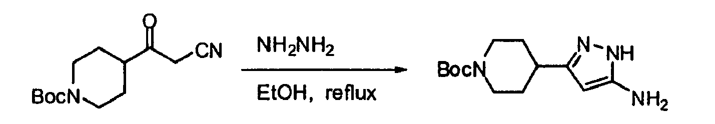

Hydrazine hydrate (149.57 mmoles, 7.26 ml) was added to a stirred solution of tert-tutyl 4-(2-cyanoacetyl)piperidinecarboxylate (49.85 mmoles, 12.58 g, purchased from Butt Park, Ltd.) in absolute ethanol (60 ml) The stirred reaction mixture was then refluxed for 5 hours. The reaction mixture was allowed to cool to room temperature and concentrated under reduced pressure. The viscous oil was dissolved in ethyl acetate (200 ml) and washed with water (2 x 100 ml) and saturated brine (200 ML). The ethyl acetate solution was dried over anhydrous magnesium sulfate, filtered and evaporated under reduced pressure. The residue was treated with diethyl ether (300 ml) and stirred at 0-5 °C (ice-water bath). A white solid started to form after 20 minutes. The resulting slurry was then stirred at 0-5 °C (ice-water bath) for 3 hours. The solid was filtered off, washed with cold ether (2 x 50 ml) and hexane (100 ml) and dried at reduced pressure to give 12.04 g of 1,1-dimethylethyl 4-(5-amino-1H-pyrazol-3-yl)piperidine-1-carboxylate as a white solid. ¹H NMR(400 MHz, d₆-DMSO): 12.71 (br s, 1), 6.34 (s, 1H), 6.12 (br s, 1H), 3.43 (m, 2H), 3.37 (m, 2H), 2.97 (m, 1H), 2.04 (m, 2H), 1.81 (m, 2H), 1.42 (s, 9H). MS (EI) for C₁₃H₂₂N₄ O₂: 267 (MH⁺).

### Ethyl 2-(1-(tert-butoxycarbonyl)piperidin-4-yl)pyrazol[1,5-a]pyrimidine-6-carboxylate

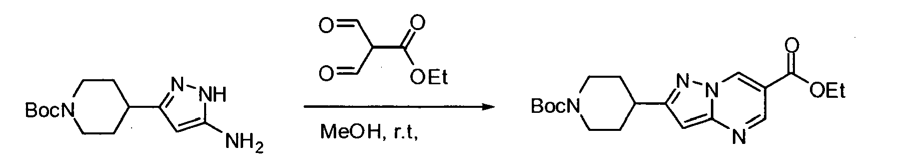

Ethyl 2-formyl-3-oxopropanoate (51.04 mmoles, 7.35 g, prepared using a literature method, Synthesis (1986),5, 400.) was added as one lot to a stirred solution of 1,1-dimethylethyl4-(5-amino-1H-pyrazol-3-yl)piperidine-1-carboxylate (44.38 moles, 11.82 g), in anhydrous methanol (100 mL). The reaction mixture was then stirred at room temperature. After 30 minutes, a yellow precipitate was observed. After 60 minutes, the resulting solid was filtered off, washed with methanol (2 x 10 ml) and dried under reduced pressure to give 15.93 g of ethyl 2-(1-(tert-butoxycarbonyl)piperidin-4-yl)pyrazolo[1,5-a]pyrimidine-6-carboxylate, which was used in the next step without any further purification. ¹H NMR (400 MHz, d₆-DMSO): 8.42 (d, 1H), 8.81 (d, 1H), 6.78 (s, 1H), 4.32 (q, 2H),4.03 (m, 2H), 3.03 (m, 1H), 2.90 (m, 2H), 1.98 (d, 2H), 1.61 (m, 2H), 1.41 (s, 9H), 1.31 (t, 3H). MS (EI) for C₁₉ H₂₆ N₄ O₄: 375: (MH⁺).

### 2-(1-(tert-Butoxycarbonyl)piperidin-4-yl)pyrazolo[1,5-a]pyrimidine-6-carboxylicacid

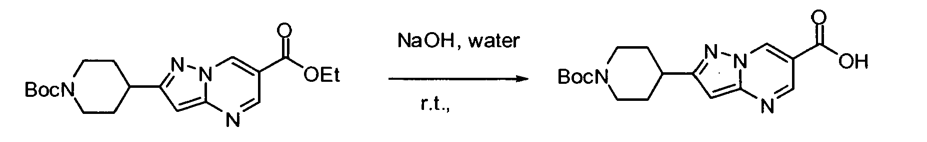

Ethyl 2-(1-(tert-butoxycarbonyl)piperidin-4-yl)pyrazolo[1,5-a]pyrimidine-6-carboxylate (25 mmole, 9.35 g) was dissolved in methanol (200 ml). Aqueous 1M sodium hydroxide solution (50 mL, 50 mmoles) was added and the reaction mixture was stirred at room temperature for 16 hours. The reaction mixture was cooled to 5 oC (ice-water batch) and acidified to pH 1 with aqueous 1M hydrochloric acid. The resulting white solid was filtered off, washed with cold water (2 x 20 ml) and dried under reduced pressure to give 8.41 g of 2-(1-(tert-butoxycarbonyl)piperidin-4-yl)pyrazolo[1,5-a]pyrimidine-6-carboxylic acid as a white solid. ¹H NMR (400 MHz, d₆-DMSO): 13.56 (br s, 1H), 8.41 (d, 1H), 8.82 (d, 1H), 6.78 (s, 1H), 4.03 (m, 2H), 3.03 (m, 1H), 2.90 (m, 2H), 1.98 (d, 2H), 1.61 (m, 2H), 1.41 (s, 9H). MS (EI) for C₁₇ H₂₂ N₄ O₄: 347 (MH⁺).

### 1,1-Dimethylethyl 4-(6-(5-bromo-2-carbamoylbenzofuran-3-ylcarbamoyl)pyrazolo[1,5-a]pyrimidin-2-yl)piperidine-1-carboxylate

1,1-Dimethylethyl 4-(6-(5-bromo-2-carbamoylbenzofuran-3-ylcarbamoyl)pyrazolo[1,5-a]pyrimidin-2-yl)piperidine-1-carboxylate was synthesized in a manner similar to 1,1-dimethylethyl 3-(5-bromo-2-carbamoylbenzofuran-3-ylcarbamoyl)azetidine-1-carboxylate wherein 2-(1-(tert-butoxycarbonyl)piperidin-4-yl)pyrazolo[1,5-a]pyrimidine-6-carboxylic acid replaced Boc-3-azetidine carboxylic acid. The crude material from this reaction was submitted to the next step without any further purification. MS (EI) for C₂₆ H₂₇ Br N₆ O₅: 566 (MH⁺).

### 1,1-Dimethylethyl 4-(6-(8-bromo-4-oxo-3,4-dihydrobenzofuro[3,2-d]pyrimidin-2-yl)pyrazolo[1,5-a]pyrimidin-2-yl)piperidine-1-carboxylate

1,1-Dimethylethyl 4-(6-(8-bromo-4-oxo-3,4-dihydrobenzofuro[3,2-d]pyrimidin-2-yl)pyrazolo[1,5-a]pyrimidin-2-yl)piperidine-1-carboxylate was synthesized in a similar manner as to 1,1-dimethylethyl 4-(8-bromo-4-oxo-3,4-dihydrobenzofuro[3,2-d]pyrimidin-2-yl)plperidine-1-carboxylate, wherein 1,1-dimethylethyl 4-(6-(5-bromo-2-carbamoylbenzofuran-3-ylcarbamoyl)pyrazolo[1,5-a]pyrimidin-2-yl)piperidine-1-carboxylate replaced 1,1-dimethylethyl 4-(5-bromo-2-carbamoylbenzofuran-3-ylcarbamoyl)piperidine-1-carboxylate. The crude material from this reaction was used in the subsequent step without any further purification. MS (EI) for C₂₆ H₂₅ Br N₆ O₄: 466 (MH⁺).

### 8-Bromo-2-(2-piperidin-4-ylpyrazolo[1,5-a]pyrimidin-6-yl)[1]benzofuro[3,2-d]pyrimidin-4(3H)-one

8-Bromo-2-(2-piperidin-4-ylpyrazolo[1,5-a]pyrimidin-6-yl)[1]benzofuro[3,2-d]pyrimidin-4(3H)-one **(Compound 377)** as its hydrochloride salt, was synthesized in a similar manner to 8-bromo-2-piperidin-4-yl[1]benzofuro[3,2-d]pyrimidin-4(3H)-one **(Compound 157**. ¹H NMR (400 MHz, d₆-DMSO): 13.20 (br s, 1H), 9.78 (s, 1H), 9.24 (s, 1H), 8.31(s, 1H), 7.87 (s, 2H), 7.54 (br s, 1H), 6.79 (s, 1H), 3.29 (m, 2H), 3.22 (m, 1H), 3.09 (m, 2H), 2.32 (m, 2H), 1.97 (m, 2H). MS (EI) for C₂₁ H₁₇ Br N₆ O₂: 466 (MH⁺).

### 8-Chloro-2-{[(3S)-3-hydroxypyrrolidin-1-yllmethyl}[1]benzofuro[3,2-d]pyrimidin-4(3H)-one AAG1

### 2-(4-Chloro-2-cyanophenoxy)acetamide

2-(4-Chloro-2-cyanophenoxy)acetamide was synthesized in a manner similar to 2-(4-bromo-2-cyanophenoxy)acetamide 2 wherein 5-chloro-2-hydroxybenzonitrile (commercially available from Prosynth Ltd.,) replaced 5-bromo-2-hydroxybenzonitrile 1. ¹H NMR (400 MHz, d₆-DMSO): 7.92 (d, 1H), MS (EI) for C₉ H₇ Cl N ₂O₂: 211 (MH⁺).

### 3-Amino-5-chlorobenzofuran-2-carboxamide

3-Amino-5-chlorobenzofuran-2-carboxamide was synthesized in a manner similar to 3-amino-5-bromobenzofuran-2-carboxamide 3, wherein 2-(4-chloro-2-cyanophenoxy)acetamide replaced 2-(4-bromo-2-cyano,phenoxy)acetamide 2. ¹H NMR (400 MHz, d₆-DMSO): 7.95 (s, 1H), 7.42 (s, 2H), 7.36 (br s, 2H), 6.02 (br s, 2H). MS (EI) for C₉ H₇ Cl N₂ O₂: 211 (MH⁺).

### 5-Chloro-3-(2-chloroacetamido)benzofuran-2-carboxamide

5-Chloro-3-(2-chloroacetamido)benzofuran-2-carboxamide was synthesized in a manner similar to 5-bromo-3-(2-chloroacetamido)benzofuran-2-carboxamide 5, wherein 3-amino-5-chlorobenzofuran-2-carboxamide replaced 3-amino-5-bromobenzofuran-2-carboxamide 3. ¹H NMR (400 MHz, d₆-DMSO): 10.76 (s, 1H), 8.26 (br s, 1H), 8.08 (d, 1H), 8.00 (br s, 1H), 7.65 (d, 1H), 7.55 (dd, 1H), 4.52 (s, 2H). MS (EI) for C₁₁H₈C₁₂N₂O₃: 288 (MH⁺).

### 8-Chloro-2-(chloromethyl)benzofuro[3,2-d]pyrimidin-4(3H)-one

8-Chloro-2-(chloromethyl)benzofuro[3,2-d]pyrimidin-4(3H)-one was synthesized in a manner similar to 8-bromo-2-(chloromethyl)benzofuro[3,2-d]pyrimidin-4(3H)-one 6, wherein 5-chloro-3-(2-chloroacetamido)benzofuran-2-carboxamide replaced 5-bromo-3-(2-chloroacetamido)benzofuran-2-carboxamide 5. ¹H NMR (400 MHz, d₆-DMSO): 13.40 (br s, 1H), 8.09 (d,1H), 7.91 (d, 1H), 7.72 (dd, 2H), 4.66 (s, 2H). MS (EI) for C₁₁H₆Cl₂N₂O₂: 270 (MH⁺).

### 8-Chloro-2-{[(3S)-3-hydroxypyrrolidin-1-yl]methyl}[1]benzofuro[3,2-d]pyrimidin-4(3H)-one AAG1

A stirred mixture of 5-chloro-3-(2-chloroacetamido)benzofuran-2-carboxamide (0.988 mmoles, 0.266), triethylamine (2,96 mmoles, 0.300 g, 0.41 mL) and (S)-3-hydroxypyrrolidine (2.96 mmoles, 0.408 g, 0.39 mL, commercially available from Acros) and ethanol (20 ml) was heated to 80 °C for 18 hours. The reaction mixture was then allowed to room temperature. The solution was filtered through a Millipore Millex-GN 0.20 uM Nylon syringe filter, and purified by preparative reverse phase HPLC (reverse-phase, acetonitrile/water with 0.01% ammonium acetate). The resulting fractions containing the desired peak (of correct molecular weight) were combined and evaporated under reduced pressure to give 0.12 g of 8-chloro-2-{[(3S)-3-hydroxypyrrolidin-1-yl]methyl}[1]benzofuro[3,2-d]pyrimidin-4(3H)-one as its acetate salt. ¹H NMR (400 MHz, d₆-DMSO): 13.12 (br s, 1H), 8.08 (dd, 1H), 7.89 (dd, 1H), 7.70 (dd, 1H), 4.19 (m,1H), 3.68 (s, 2H), 3.35 (br s, 1H), 2.79 (m, 2H), 2.53 (m, 1H), 2.02 (m, 1H), 1.59 (m, 1H). MS (EI) for C₁₅ H₁₄ Cl N₃ O₃: 319 (MH⁺).

### *(Compound 63)

### 2-(2-chlorophenyl)-8-methyl[1]benzofuro[3,2-d]pyrimidin-4(3H)-one

2-(2-chlorophenyl)-8-methyl[1]benzofuro[3,2-d]pyrimidin-4(3H)-one was synthesized in the same manner as **Example 8** wherein 3-amino-5-bromobenzofuran-2-carboxamide 3 was replaced with 3-amino-5-methyl-benzofuran-2-carboxamide. 3-Amino-5-methyl-benzofuran-2-carboxamide was synthesized in the same manner as **Example 1** wherein 5-bromo-2-hydroxybenzonitrile 1 was replaced with 5-methyl-2-hydroxybenzonitrile. 5-methyl-2-hydroxybenzonitrile was synthesized in the same manner as **Example AAG1** wherein 5-bromo-2-hydroxy-3-methylbenzaldehyde was replaced with 2-hydroxy-5-methylbenzaldehyde. 1H NMR (400 MHz, d6-DMSO): 13.28 (s, 1H), 7.85 (s, 1H), 7.76 (d, 1H), 7.70 (dd, 1H), 7.65 (m, 1H), 7.60 (m, 1H), 7.53 (m, 2H), 2.48 (s, 3H). MS (EI) for C₁₇H₁₁ClN₂O₂: 312 (MH+).

### 8-Bromo-2-{[(3S)-3-hydroxyprrolidin-1-yl]methyl-6-methyl[1]benzofuro[3,2-d]pyrimidin-4(3H)-one AAG2

### E/Z -5-Bromo-2-hydroxy-3-methylbenzaldehydeoxime

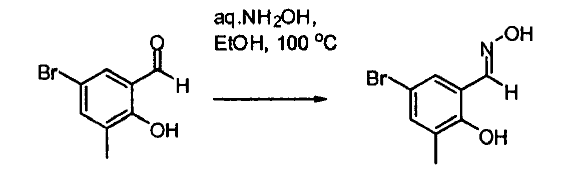

An aqueous solution of hydroxylamine (93.00 mmole, 3.07 g, 6.14 ml, 50% w/w commercially from Alfa Aesar) was added in lot to a stirred solution of 5-bromo-2-hydroxy-3-methylbenzaldehyde (10g, 46.50 g) (prepared by a literature method - J.Catalysis 2004, 221, 77), in ethanol 200 ml. The mixture was then heated to 100°C for 2 hours. The reaction mixture was then allowed to cool to room temperature and then poured on crushed ice. The resulting white slurry was then stirred for 30 minutes, filtered off, washed with cold water (2 x 25 ml) and then dried at reduced pressure to give 10.24 g of E/Z -5-bromo-2-hydroxy-3-methylbenzaldehyde oxime as a white solid, which was used in the next step without any further purification. ¹H NMR (400 MHz, d₆-DMSO): 11.70 (s, 1H), 10.40 (s, 1H), 8.34 (s, 1H), 7.44 (d, 1H), 7.32 (d, 1H), 2.15 (S, 3H). MS (EI) for C₈ H₈ Br N O₂: 231 (MH⁺).

### 5-Bromo-2-hvdroxy-3-methylbenzonitrile

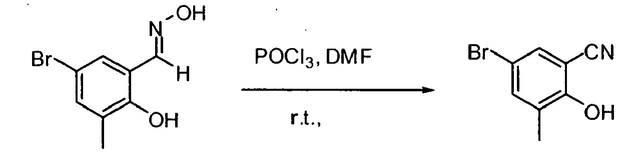

Phosphorus oxychloride (108 mmoles 16.66 g, 10.12 mL), ws added in a drop wise fashion to a stirred solution of E/Z -5-bromo-2-hydroxy-3-methylbenzaldehyde oxime (43.46 mmoles, 10g) in anhydrous DMF (50 mL) at 5 °C (ice-water bath). Upon completion of addition, the cooling bath was removed and the reaction mixture was stirred for 5 hours. The reaction mixture was the poured onto crushed ice, resulting in the precipitation of a light brown solid. This suspension was stirred for 1 hour and then filtered off, washed with cold water (2 x 20 ml) and dried at reduced pressure to give 7.73 g of 5-bromo-2-hydroxy-3-methylbenzonitrile which was used in the next step without any further purification. ¹H NMR (400 MHz, d₆-DMSO): 10.51 (br s, 1H), 9.21 (d, 1H), 8.21 (s, 1H), 2.18 (s, 3H). MS (EI) for C₈H₆ Br NO: 213 (MH⁺).
**Reference:** J.Chinese Chemical Society, 1988,35,459

### 2-(4-Bromo-2-cyano-6-methylphenoxy)acetamide

2-(4-Bromo-2-cyano-6-methylphenoxy)acetamide was synthesized in a manner similar to 2-(4-bromo-2-cyanophenoxy)acetamide **2**, wherein 5-bromo-2-hydroxy-3-methylbenzonitrile replaced 5-bromo-2-hydroxybenzonitrile **1**. ¹H NMR (400 MHz, d₆-DMSO): 7.92 (d, 1H), 7.70 (dd, 1H), 7.51 (br s, 1H), 7.44 (br s, 1H), 7.07 (d, 1H), 4.68 (s, 2H). MS (EI) for C₁₀ H₉ Br N₂ O₂: 270 (MH⁺).

### 3-Amino-5-bromo-7-methylbenzofuran-2-carboxamide

3-Amino-5-bromo-7-methylbenzofuran-2-carboxamide was synthesized in a manner similar to 3-amino-5-bromobenzofuran-2-carboxamide 3, wherein 2-(4-bromo-2-cyano-6-methylphenoxy)acetamide replaced 2-(4-bromo-2-cyanophenoxy)acetamide 2. ¹H NMR (400 MHz, d₆-DMSO): 7.80 (s, 1H), 7.41 (s, 1H), 7.30 (br s, 2H), 5.97 (br s, 2H). 2.50 (s, 3H). MS (EI) for C₁₀H₉BrN₂O₂: 270 (MH⁺).

### 5-Bromo-3-(2-chloroacetamido)-7-methylbenzofuran-2-carboxamide

5-Bromo-3-(2-chloroacetamido)-7-methylbenzofuran-2-carboxamide was synthesized in a manner similar to 5-bromo-3-(2-chloroacetamido)benzofuran-2-carboxamide 5, wherein 3-amino-5-bromo-7-methylbenzofuran-2-carboxamide replaced 3-amino-5-bromobenzofuran-2-carboxamide **3**. ¹H NMR (400 MHz, d₆-DMSO): 10.72 (br s, 1H), 8.23 (s, 1H), 8.01 (d, 2H), 7.51 (s, 1H), 4.51 (s, 2H), 2.51 (s, 3H). MS (EI) for C₁₂H₁₀BrClN₂O₃: 346 (MH⁺).

### 8-Bromo-2-(chloromethyl)-6-methylbenzofuro[3,2-d]pyrimidin-4(3H)-one

8-Bromo-2-(chloromethyl)-6-methylbenzofuro[3,2-d]pyrimidin-4(3H)-one was synthesized in a manner similar to 8-bromo-2-(chloromethyl)benzofuro[3,2-d]pyrimidin-4(3H)-one **6**, wherein 5-bromo-3-(2-chloroacetamido)-7-methylbenzofuran-2-carboxamide replaced 5-bromo-3-(2-chloroacetamido)benzofuran-2-carboxamide **5**. ¹H NMR (400 MHz, d₆-DMSO): 13.36 (br s, 1H), 7.95 (d, 1H), 7.67 (d, 1H), 4.65 (s, 2H), 2.53 (s, 3H). MS (EI) for C₁₂ H₈ Br Cl N₂ O₂: 328 (MH⁺).

### 8-Bromo-2-{[(3S)-3-hydroxypyrrolidin-l-yl]methyl}-6-methyl[1]benzofuro[3,2-d]pyrimidin-4(3H)-one AAG2

A stirred mixture of 8-bromo-2-(chloromethyl)-6-methylbenzofuro[3,2-d]pyrimidin-4(3H)-one (0.915 mmoles, 0.3 g), triethylamine (2.74 mmoles, 0.278 g, 0.38 mL), (S)-3-hydroxypyrrolidine (2.74 mmoles, 0.378 g, 0.36 mL, commercially available from Acros) and ethanol (20 ml) was heated to 80 °C for 18 hours. The reaction mixture was then allowed to cool to room temperature. The solution was filtered through a Millipore Millex-GN 0.20 uM Nylon syring filter, and purified by preparative reverse phase HPLC (reverse-phase, acetonitrile/water with 0.01% ammonium acetate). The resulting fractions containing the desired peak (of correct molecular weight) were combined and evaporated under reduced pressure to give 0.101 g of 8-bromo-2-{[(3S)-3-hydroxypyrrolidin-1-yl]methyl}-6-methyl[1]benzofuro[3,2-d]pyrimidin-4(3H)-one as its acetate salt. ¹H NMR(400 MHz, d₆-DMSO): 13.04 (br s, 1H), 7.98 (s, 1H), 7.64 (s, 1H), 4.19 (s, I H), 3.67 (s, 2H), 3.44 (br s, 1H), 2.50 (s, 3H), 2.02 (m, 2H), 1.59 (m, 2H). MS (EI) for C₁₆H₁₆Br N₃O₃: 379 (MH⁺).

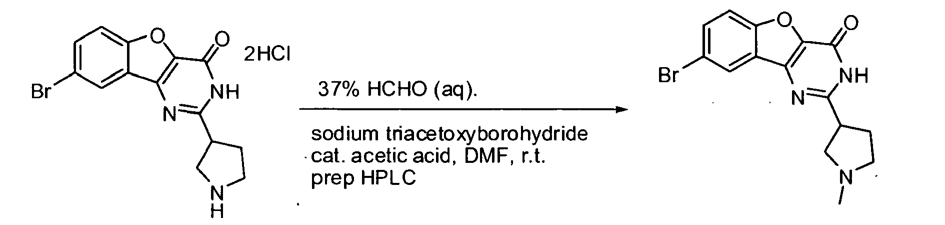

### (Compound 289)

### 8-bromo-2-(1-methylpyrrolidin-3-yl)[1]benzofuro[3,2-d]pyrimidin-4(3H)-one

8-bromo-2-pyrrolidin-3-yl[1]benzofuro[3,2-d]pyrimidin-4(3H)-one **(Compound 186)** (50 mg, 0.14 mmol) was added to anhydrous DMF (3 ml) and stirred to ensure complete dissolution of the solid. The stirred reaction mixture was treated with 37 % aqueous formaldehyde (10 equivalents) and glacial acetic acid (cat amount). The reaction mixture was stirred at room temperature for 5 minutes. The stirred reaction mixture was then treated with sodium triacetoxyborohydride (10 equivalents, commercially available from BASF Corporation) and additional glacial acetic acid (1 drop). The reaction mixture was then stirred at room temperature for 30 minutes and shown to be complete by LC-MS. The solution was filtered through a Millipore Millex-GN 0.20 uM Nylon syring filter, and the resulting solution was submitted for preparative reverse phase HPLC (reverse-phase, acetonitrile/water with 0.01% ammonium acetate), followed by concentration in vacuo and lyophilization afforded 40 mg of 8-bromo-2-(1-methylpyrrolidin-3-yl)[1]benzofuro[3,2-d]pyrimidin-4(3H)-one (acetate salt) as a white solid. ¹H NMR (400 MHz, d₆-DMSO): 8.22 (d, 1H), 8.18 (s, 1H), 7.82 (s, 2H), 3.55-3.43 (m, 1H), 2.98 (t, 1H), 2.85 (dd, 1H), 2.75-2.65 (m, 2H), 2.57 (s, 3H), 2.28-2.15 (m, 1H). MS (EI) for C₁₅H₁₄BrN₃O₂: 348 (MH⁺). 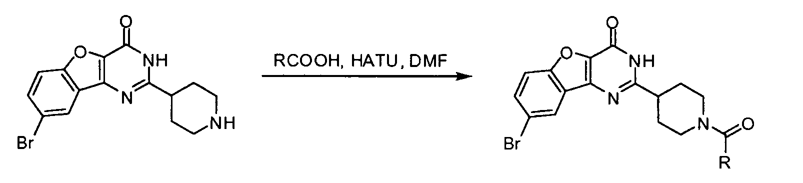 wherein R is as described within the compounds within this example.

### (Compound 203)

### 2-(1-acetylpiperidin-4-yl)-8-bromo[1]benzofuro[3,2-d]pyrimidin-4(3H)-one

To a solution of 8-bromo-2-piperidin-4-yl[1]benzofuro[3,2-d]pyrimidin-4(3H)-one hydrochloride salt (100 mg, 0.28 mmol) in DMF (3 mL) was added acetic acid (1.5 eq), triethylamine (0.1 mL) and HATU (1.2 eq). The reaction mixture was stirred at room temp. until the starting material was consumed. Purification by preparative HPLC resulting in 68 mg of 2-(1-acetylpiperidin-4-yl)-8-bromo[1]benzofuro[3,2-d]pyrimidin-4(3H)-one. ¹H NMR (400 MHz, d₆-DMSO): 12.95 (s, 1H), 8.18 (s, 1H), 7.82 (s, 2H), 4.44 (d, 1H), 3.95 (d, 1H), 3.18-2.95 (m, 2H), 2.64-2.57 (m, 1H), 1.95-1.63 (m, 4H); MS (EI) for C₁₇H₁₆BrN₃O₃: 390 (MH⁺).

### (Compound 206)

### 8-bromo-2-[1-(N,N-dimethyl-beta-alanyl)piperidin-4-yl][1]benzofuro[3,2-d]pyrimidin-4(3H)-one

8-bromo-2-[1-(N,N-dimethyl-beta-alanyl)piperidin-4-yl][1]benzofuro[3,2-d]pyrimidin-4(3H)-one was synthesized in a manner similar Example 34 wherein acetic acid was substituted with 3-(dimethylamino)propanoic acid. Purification by preparative HPLC resulting in 54 mg of 8-bromo-2-[1-(N,N-dimethyl-beta-alanyl)piperidin-4-yl][1]benzofuro[3,2-d]pyrimidin-4(3H)-one. ¹H NMR (400 MHz, d₆-DMSO): 8.24 (s, 1H), 8.18 (s, 1H), 7.81 (s, 2H), 4.55 (d, 1H), 4.04 (d, 1H), 3.23-2.95 (m, 4H),
2.69-2.58 (m, 3H), 2.23 (s, 6H), 2.00-1.90 (m, 2H), 1.85-1.63 (m, 2H); MS (EI) for C₂₀H₂₃BrN₄O₃: 447 (MH⁺).

### (Compound 207)

### 8-bromo-2-{1-[4-(dimethylamino)butanoyl]piperidin-4-yl}[1]benzofuro[3,2-d]pyrimidin-4(3H)-one

8-bromo-2-{1-[4-(dimethylamino)butanoyl]piperidin-4-yl}[1]benzofuro[3,2-d]pyrimidin-4(3H)-one was synthesized in a manner similar **Example 34** wherein acetic acid was substituted with 4-(dimethylamino)butanoic acid. Purification by preparative HPLC resulting in 80 mg of 8-bromo-2-{1-[4-(dimethylamino)butanoyl]pipendin-4-yl}[1]benzofuro[3,2-d]pyrimidin-4(3H)-one. ¹H NMR (400 MHz, d₆-DMSO): 8.21 (s, 1H), 8.17 (s, 1H), 7.82 (s, 2H), 4.53 (d, 1H), 4.03 (d, 1H), 3.23-2.95 (m, 4H), 2.70-2.58 (m, 1H), 2.40-2.25 (m, 3H), 2.23 (s, 6H), 2.00-1.90 (m, 2H), 1.81-1.63 (m, 3H); MS (EI) for C₂₁H₂₅BrN₄O₃: 461 (MH⁺).

### (Compound 208)

### 2-[1-(1H-benzimidazol-5-ylcarbon)piperidin-4-yl]-8-bromo[1]benzofuro[3,2-d]pyrimidin-4(3H)-one

2-[1-(1H-benzimidazol-5-ylcarbonyl)piperidin-4-yl]-8-bromo[1]benzofuro[3,2-d]pyrimidin-4(3H)-one was synthesized in a manner similar **Example 34** wherein acetic acid was substituted with 1*H*-benzo[d]imidazole-5-carboxylic acid. Purification by preparative HPLC resulting in 34 mg of 2-[1-(1H-benzimidazol-5-ylcarbonyl)piperidin-4-yl]-8-bromo[1]benzofuro[3,2-d]pyrimidin-4(3H)-one. ¹H NMR (400 MHz, d₆-DMSO): 12.95 (s, 1H), 8.40 (s, 1H), 8.21 (s, 1H), 8.19 (s, 1H), 7.80 (s, 2H), 7.70 (d, 2H), 7.30 (d, 1H), 4.57 (s, 1H), 3.93-3.90 (m, 1H), 3.15-3.00 (m, 3H), 2.05-1.78 (m, 4H); MS (EI) for C₂₃H₁₈BrN₅O₃: 492 (MH⁺).

### (Compound 209)

### 8-bromo-2-{1-[3-(methyloxy)propanoyl]-piperidin-4-yl}[1]benzofuro[3,2-d]pyrimidin-4(3H)-one

8-bromo-2-{1-[3-(methyloxy)propanoyl]piperidin-4-yl}[1]benzofuro[3,2-d]pyrimidin-4(3H)-one was synthesized in a manner similar **Example 34** wherein acetic acid was substituted with 3-methoxypropanoic acid. Purification by preparative HPLC resulting in 76 mg of 8-bromo-2-{1-[3-(methyloxy)propanoyl]piperidin-4-yl}[1]benzofuro[3,2-d]pyrimidin-4(3H)-one. ¹H NMR (400 MHz, d₆-DMSO): 12.95 (s, br, 1H), 8.15 (s, 1H), 7.81 (s, 2H), 4.45 (d, 1H), 4.00 (d, 1H), 3.60-3.55 (m, 2H), 3.23-3.18 (m, 3H), 3.10-2.95 (m, 2H), 2.64-2.55 (m, 3H), 1.98-1.80 (m, 4H); MS (EI) for C₁₉H₂₀BrN₃O₄: 434 (MH⁺).

### (Compound 210)

### 8-bromo-2-[1-(N,N-dimethylglycyl)piperidin-4-yl][1]benzofuro[3,2-d]pyrimidin-4(3H)-one

8-bromo-2-[1-(N,N-dimethylglycyl)piperidin-4-yl][1]benzofuro[3,2-d]pyrimidin-4(3H)-one was synthesized in a manner similar Example 34 wherein acetic acid was substituted with 2-(dimethylamino)acetic acid. Purification by preparative HPLC resulting in 80 mg of 8-bromo-2-[1-(N,N-dimethylglycyl)piperidin-4-yl][1]benzofuro[3,2-d]pyrimidin-4(3H)-one. ¹H NMR (400 MHz, d₆-DMSO): 8.21 (s, 1H), 8.18 (s, 1H), 7.82 (s, 2H), 4.50 (d, 1H), 4.18 (d, 1H), 3.21-2.95 (m, 4H), 2.66-2.60 (m, 1H), 2.20 (s, 3H), 2.00-1.95 (m, 2H), 1.81-1.60 (m, 2H); MS (EI) for C₁₉H₂₁BrN₄O₃: 433 (MH⁺).

### (Compound 233)

### 8-bromo-2-[1-(tetrahydrofuran-3-ylcarbonyl)piperidin-4-yl][1]benzofuro[3,2-d]pyrimidin-4(3H)-one

8-bromo[-2-[1-(tetrahydrofuran-3-ylcarbonyl)piperidin-4-yl][1]benzofuro[3,2-d]pyrimidin-4(3H)-one was synthesized in a manner similar **Example 34** wherein acetic acid was substituted with tetrahydrofuran-3-carboxylic acid. Purification by preparative HPLC resulting in 8-bromo-2-[1-(tetrahydrofuran-3-ylcarbonyl)piperidin-4-yl][1]benzofuro[3,2-d]pyrimidin-4(3H)-one. ¹H NMR (400 MHz, d₆-DMSO): 8.15 (s, 1H), 7.81 (s, 2H), 4.45 (d, 1H), 4.00 (d, 1H), 3.95-3.87 (m, 1H), 3.80-3.70 (m, 2H), 3.10-3.00 (m, 2H), 2.65-2.60 (m, 1H), 1.90-1.80 (m, 4H), 1.75-1.65 (m, 3H); MS (EI) for C₂₀H₂₀BrN₃O₄: 446 (MH⁺).

### (Compound 234)

### 8-bromo-2-[1-(phenylcarbonyl)piperidin-4-yl][1]benzofuro[3,2-d]pyrimidin-4(3H)-one

8-bromo-2-[1-(phenylcarbonyl)piperidin-4-yl][1]benzofuro[3,2-d]pyrimidin-4(3H)-one was synthesized in a manner similar Example 34 wherein acetic acid was substituted with benzoic acid. Purification by preparative HPLC resulting in 8-bromo-2-[1-(phenylcarbonyl)piperidin-4-yl][1]benzofuro[3,2-d]pyrimidin-4(3H)-one. ¹H NMR(400 MHz, d₆-DMSO): 8.18 (s, 1H); 8.00 (d, 2H), 7.82 (d, 2H), 7.76-7.58 (m, 3H), 3.98-3.88 (m, 2H), 3.18-2.95 (m, 2H), 2.64-2.57 (m, 1H), 1.95-1.63 (m, 4H); MS (EI) for C₂₂H₁₈BrN₃O₃: 452 (MH⁺).

### (Compound 236)

### 8-bromo-2-[1-(pyridin-4-ylcarbonyl)piperidin-4-yl][1]benzofuro[3,2-d]pyrimidin-4 3H -one

8-bromo-2-[1-(pyridin-4-ylcarbonyl)piperidin-4-yl][1]benzofuro[3,2-d]pyrimidin-4(3H)-one was synthesized in a manner similar Example 34 wherein acetic acid was substituted with isonicotinic acid. Purification by preparative HPLC resulting in 8-bromo-2-[1-(pyridin-4-ylcarbonyl)piperidin-4-yl][1]benzofuro[3,2-d]pyrimidin-4(3H)-one. ¹H NMR (400 MHz, d₆-DMSO): 8.18 (s, 1H), 8.00 (m, 2H), 7.73 (d, 2H), 7.58 (d, 2H), 3.93-3.80 (m, 2H), 3.20-3.00 (m, 2H), 1.99-1.70 (m, 4H); MS (EI) for C₁₂H₁₇BrN₄O₃: 453 (MH⁺).

### (Compound 237)

### 8-bromo-2-[1-(phenylacetyl)piperidin-4-yl][1]benzofuro[3,2-d]pyrimidin-4(3H)-one

8-bromo-2-[1-(phenylacetyl)piperidin-4-yl][1]benzofuro[3,2-d]pyrimidin-4(3H)-one was synthesized in a manner similar Example 34 wherein acetic acid was substituted with 2-phenylacetic acid. Purification by preparative HPLC resulting in 8-bromo-2-[1-(phenylacetyl)piperidin-4-yl] [1]benzofuro[3,2-d]pyrimidin-4(3H)-one. ¹H NMR (400 MHz, d₆-DMSO): 8.18 (s, 1H), 8.00 (d, 2H), 7.45-7.33 (m, 3H), 7.28-7.19 (m, 2H), 3.98-3.90 (m, 2H), 3.85 (s, 2H), 3.18-2.95 (m, 2H), 2.64-2.57 (m, 1H), 1.95-1.63 (m, 4H); MS (EI) for C₂₃H₂₀BrN₃O₃: 466 (MH⁺).

### (Compound 290)

### 8-bromo-2-[1-(pyridin-4-ylmethyl)pyrrolidin-3-yl][1]benzofuro[3,2-d]pyrimidin-4(3H)-one

8-bromo-2-[1-(pyridin-4-ylmethyl)pyrrolidin-3-yl][1]benzofuro[3,2-d]pyrimidin-4(3H)-one was synthesized in a manner similar to **Compound 289.** Purification by preparative HPLC resulting in 8-bromo-2-[1-(pyridin-4-ylmethyl)pyrrolidin-3-yl][1]benzofuro[3,2-d]pyrimidin-4(3H)-one. ¹H NMR (400 MHz, d₆-DMSO): 8.60 (s, 2H), 8.22 (d, 1H), 8.18 (s, 1H), 7.82 (s, 2H), 7.48 (s, 2H), 3.98 (s, 2H), 3.57-3.45 (m, 1H), 3.07-2.95 (m, 1H), 2.89-2.65 (m, 3H), 2.35-2.22 (m, 2H). MS (EI) for C₂₀H₁₇BrN₄O₂: 425 (MH⁺).

### 2-(5-Amino-2-chlorophenyl)-8-bromo[1]benzofuro[3,2-d]pyrimidin-4(3H)-one 391. AAG3

### 1,1-Dimethylethyl 3-(5-bromo-2-carbamoylbenzofuran-3-ylcarbamoyl)-4-chlorophenylcarbamate

1,1-Dimethylethyl 3-(5-bromo-2-carbamoylbenzofuran-3-ylcarbamoyl)-4-chlorophenylcarbamate was synthesized in a manner similar to 1,1-dimethylethyl 3-(5-bromo-2-carbamoylbenzofuran-3-ylcarbamoyl)azetidine-1-carboxylate wherein 5-(tert-butoxycarbonylamino)-2-chlorobenzoic acid (commercially available from CNH Technologies Inc..) replaced Boc-3-azetidine carboxylic acid. The crude material from this reaction was submitted to the next step without any further purification. ¹H NMR (400 MHz, d₆-DMSO):10.73 (br s, 1H), 9.76 (br s, 1H), 8.30 (s, 1H), 8.26 (s,1H), 8.00 (br s, 1H), 7.88 (s, 2H), 7.70 (dd, 1H), 7.63 (d, 1H), 7.57 (dd, 1H), 7.49 (d, 1H), 1.48 (s, 9H). MS (EI) for C₂₁ H₁₉ Br Cl N₃ O₅: 509 (MH⁺).

### 2-(5-Amino-2-chlorophenyl)-8-bromo[1]benzofuror[3,2-d]pyrimidin-4(3H)-one

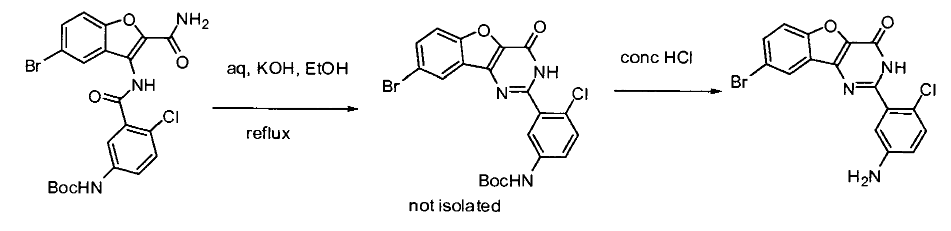

Crude 2-(5-amino-2-chlorophenyl)-8-bromo[1]benzofuro[3,2-d]pyrimidin-4(3H)-one AAG3 (15.92 mmoles, 8.1 g), was dissolved in ethanol (200 ml) and treated with aqueous 1M potassium hydroxide solution (60 mmoles, 60ml). The stirred reaction mixture was then refluxed for 18 hours. The reaction mixture was then allowed to cool to ca. 60 °C. The warm reaction mixture was then acidified to pH =1, by the drop wise addition of concentrated hydrochloric acid, which resulted in the formation of a white precipitate. The resulting suspension was then diluted with additional ethanol (100ml) and heated to 100 °C for a further 8 hours. The reaction mixture was then allowed to cool and the resulting solid was filtered off, washed with cold ethanol and dried under reduced pressure to give 4.97 of 2-(5-amino-2-chlorophenyl)-8-bromo[1]benzofuro[3,2-d]pyrimidin-4(3H)-one as its hydrochloride salt, as a white solid. ¹H NMR (400 MHz, d₆-DMSO):13.44 (br s, 1H), 8.25 (7.89 (dd, 1H), 7.85 (dd, 1H), 7.56 (d, 1H), 7.35 (s, 1H), 7.28 (dd, 1H), 6.82 (br s, 3H).MS (EI) for C16 H9 Br Cl N3 O2: 391: (MH⁺).

### 8-Bromo-6-methyl-1-((4-methylpiperazin-1-yl)methyl)benzofuro[3,2-d]pyrimidin-4(3H)-one AAG4

A stirred mixture of 8-bromo-2-(chloromethyl)-6-methylbenzofuro[3,2-d]pyrimidin-4(3H)-one (0.978 mmoles, 0.32 g), triethylamine (2.93 mmoles, 0.73 g, 0.41 mL), N-methylpiperaazine (2.93 mmoles, 0.29 g, 0.28 mL, commercially available from Acros) and ethanol (20 ml) was heated to 80 °C for 18 hours. The reaction mixture was then allowed to cool to room temperature. The solution was filtered through a Millipore Millex-GN 0.20 uM Nylon syring filter, and purified by preparative reverse phase HPLC (reverse-phase, acetonitrile/water with 0. 0 1 % ammonium acetate). The resulting fractions containing the desired peak (of correct molecular weight) were combined and evaporated under reduced pressure to give 0.168g of 8-bromo-6-methyl-2-((4-methylpiperazin-1-yl)methyl)benzofuro[3,2-d]pyrimidin-4(3H)-one as its acetate salt.
¹H NMR (400 MHz, d₆-DMSO): 12.60 (br s, 1H), 7.99 (s, 1H), 7.80 (s, 1H), 3.50 (s, 2H),
3.39, (s, 4H), 2.54 (s, 3H), 2.22 (s, 4H), 2.14 (s, 3H). MS (EI) for C₁₇ H₁₉ Br N₄ O₂: 392 (MH⁺).

### Compound 534

### N-[2-(Aminocarbonyl)-5-bromo-1-benzofuran-3-yl]piperidine-2-carboxamide hydrochloride

DL-pipecolinic acid (5.0 g, 38.7 mmol) was suspended in dichloromethane (50 mL) and cooled in a -10°C ice bath. Phosphorus pentachloride (8.14 g, 39.1 mmol) was added in one portion. The reaction mixture was stirred in the cold bath for 1 h to give a clear solution. Another reaction flask was charged with 3-amino-5-chloro-1-benzofuran-2-carboxamide, (2.45 g, 9.6 mmol) was suspended in dichloromethane (25 mL) and cooled in a -10°C ice/salt bath. The solution of DL-pipecolinic acid chloride hydrochloride was added dropwise over 5 min. The reaction mixture was allowed to warm to room temperature and was stirred overnight. The precipitate was isolated by filtration using additional dichloromethane (50 mL) to wash the material from the reaction flask. The white solid was washed with dichloromethane (3 x 20 mL) and dried under vacuum to give 4.05 g of (N-[2-(aminocarbonyl)-5-bromo-1-benzofuran-3-yl]piperidine-2-carboxamide hydrochloride as a light grey powder. The material was used in the next step without any further purification.
MS (EI) for C₁₅H₁₆BrN₃O₃: 366 (MH⁺).

### 8-Bromo-2-piperidin-2-yl[1]benzofuror[3,2-d]-pyrimidin-4(3H)-one hydrochloride

N-[2-(Aminocarbonyl)-5-bromo-1-benzofuran-3-yl]piperidine-2-carboxamide hydrochloride (4.0 g , 9.9 mmol)was suspended in ethanol, (100 mL) and sodium hydroxide (25 mL, 2.0 M, 50 mmol) was added which caused all the material to dissolve. The reaction mixture was stirred at 45°C for 4 h. After cooling to room temperature, the reaction mixture was further cooled to 0-5°C in an ice bath. Hydrochloric acid (6M) was added slowly to lower the pH to 2. The reaction mixture was removed from the ice bath and allowed to stand at 4°C in a refrigerator for 16 hours. The precipitate was removed by filtration. The filtrate was concentrated under vacuum, the residue was stirred in ice water (25 mL) and the solid was isolated by filtration. The solid was air dried for 5 h and then stirred in ethyl acetate at 50°C for 48 h. The solid was isolated by filtration, washed with ethyl acetate (2 x 30 mL) and dried under vacuum to give 1.27 g (33% yield, >95% purity).
MS (EI) for C₁₅H₁₄BrN₃O₂: 348 (MH⁺).
¹H NMR (400 MHz, d₆-DMSO): 1349 (MH⁺). 13.61 (br s, 1H), 8.10 (d, 1H), 7.88 (s, 1H), 7.86 (d, 1H), 7.84 (d, 1H), 4.39 (t, 1H), 4.43 (d, 1H), 3.56 (br s, 2H), 3.04 (m, 1H), 2.31 (1H), 1.73 (m, 2H), 1.92 (m ,2H), 1.52 (m, 2H).

### Compound 535

### 2-(1-amino-2-phenylethyl)-8-bromo[1]benzofuro[3,2-d]pyrimidin-4(3)-one N-[2-(aminocarbonyl)-5-bromo-1-benzofuran-3-yl]-Nα-{(1,1-dimethylethyl)oxy] carbonyl}phenylalaninamide

To a 250 mL round-bottomed flask equipped with a magnetic stir bar was added N-{[(1,1-dimethylethyl)oxy]carbonyl}phenylalanine (16.7 g, 63.0 mmol), dichloromethane (160 mL), and pyridine (5.0 mL, 61.8 mmol). This mixture was cooled to -10 °C and stirred at this temperature for 15 min. Cyanuric fluoride (13.5 mL, 157.4 mmol) was added via syringe and the resulting mixture was allowed to stir at -10 °C for 1 h. The reaction was then diluted with dichloromethane (250 mL) and quenched slowly with water (250 mL) at 0 °C. The solids were filtered and the layers were separated and concentrated in vacuo. This crude reaction was then redissolved in dichloromethane (80 mL) and dimethylacetamide (80 mL). 3-Amino-5-bromo-1-benzofuran-2-carboxamide (4.0 g, 15.7 mmol) and pyridine (3.8 mL, 47.2 mmol) was added and the reaction was monitored by LCMS. Upon completion, the reaction was poured into 1 M aqueous hydrochloric acid (100 mL) and the aqueous layer was extracted with ethyl acetate (4 x 100 mL). The organics were then washed with 0.5 M aqueous sodium hydroxide (100 mL), water (100 mL), brine (100 mL) and then dried over sodium sulfate. The ethyl acetate was removed under vacuum to give the product, *N-*[2-(aminocarbonyl)-5-bromo-1-benzofuran-3-yl]-Nα-{[(1,1-dimethylethyl)oxy]carbonyl}phenylalaninamide. The crude product was submitted to the next step without further purification. MS (EI) for C₂₃H₂₄BrN₃O₅: 502 (MH⁺).

### N-[2-(aminocarbonyl)-5-bromo-1-benzofuran-3-yl]phenylalaninamide hydrochloride

*N*-[2-(Aminocarbonyl)-5-bromo-1-benzofuran-3-yl]-Nα-{[(1,1-dimethylethyl)oxy]carbonyl}phenylalaninamide was dissolved in ethyl acetate (80 mL) and methanol (80 mL). The mixture was then treated with 4 M hydrochloric acid in dioxane (80 mL, 315 mmol) and the reaction was monitored via LCMS. Upon completion, the solids were filtered to give *N-*[2-(aminocarbonyl)-5-bromo-1-benzofuran-3-yl]phenylalaninamide hydrochloride. The crude material was submitted to the next step without purification. MS (EI) for C₁₈H₁₆BrN₃O₃: 402 (MH⁺).

### 2-(1-amino-2-phenylethyl)-8-bromo[1]benzofuro[3,2-d]pyrimidin-4(3H)-one

*N-*[2-(Aminocarbonyl)-5-bromo-1-benzofuran-3-yl]phenylalaninamide hydrochloride was dissolved in ethanol (160 mL) and treated with 1 M aqueous sodium hydroxide (80 mL, 78.7 mmol). The reaction was stirred at 60 °C. Upon completion by LCMS, the reaction was cooled to 0 °C and the pH was adjusted to 2. The precipitate was filtered to give 2-(1-amino-2-phenylethyl)-8-bromo[1]benzofuro[3,2-*d*]pyrimidin-4(3H-one (788 mg, 13% yield for 3 steps). ¹H NMR (400 MHz, d₆-DMSO): 8.87 (b s, 1H), 8.12 (s, 1H), 7.90 (m, 2H), 7.27 (m, 5H), 4.6 (m, 1H), 3.3 (m, 2H). MS (EI) for C₁₈H₁₄BrN₃O₂: 384 (MH⁺).

### Compound 536

### 2-(1-amino-2- {4-[(phenylmethyl)oxy]lphenyl}ethyl)-8-bromo[1]benzofuror3,2-c])vrimidin-4(3H)-one

2-(1-Amino-2-{4-[(phenylmethyl)oxy]phenyl}ethyl)-8-bromo[1]benzofuro[3,2-*d*]pyrimidin-4(3H)-one was synthesized in a manner similar to **Compound 535,** wherein N-{[(1,1-dimethylethyl)oxy]carbonyl}-*O-*(phenylmethyl)tyrosine replaced N-{[(1,1-dimethylethyl)oxy]carbonyl phenylalanine. ¹H NMR (400 MHz, d₆-DMSO): 8.12 (s, 1H), 7.90 (m, 2H), 7.37 (m, 5H), 7.15 (d, 2H), 6.93 (d, 2H), 5.04 (s, 2H) 4.58 (t, 1H), 3.27 (m, 2H). MS (EI) for C₂₅H₂₀BrN₃O₃: 490 (MH⁺).

### Compound 537

### 2-[(1S)-1-amino-3-phenypropyl]-8-bromo[1]benzofuro[3,2-d]pyrimidin-4(3H)-one

2-[(1S)-1-Amino-3-phenylpropyl]-8-bromo[1]benzofuro[3,2-*d*]pyrimidin-4(3H)-one was synthesized in a manner similar to **Compound 535,** wherein (2S)-2-({[(1,1-dimethylethyl)oxy]carbonyl}amino)-4-phenylbutanoic acid replaced for N-{[(1,1-dimethylethyl)oxy]carbonyl}phenylalanine. ¹H NMR (400 MHz, d₆-DMSO): 8.11 (s, 1H), 7.90 (m, 2H), 7.20 (m, 5H), 4.47 (t, 1H), 2.70 (m, 2H), 2.30 (m, 2H). MS (EI) for C₁₉H₁₆BrN₃O₂: 398 (MH⁺).

### Compound 538

### 2-[1-amino-2-(2-thienyl)ethyl-8-bromo[1]benzofuro[3,2-d]pyrimidin-4(3H)-one

2-[1-Amino-2-(2-thienyl)ethyl]-8-bromo[1]benzofuro[3,2-d]pyrimidin-4(3H)-one was synthesized in a manner similar to **Compound 535,** wherein N-{[(1,1-dimethylethyl)oxy]carbonyl} -3-(2-thienyl)alanine replaced N-{[(1,1-dimethylethyl)oxy]carbonyl}phenylalanine. ¹H NMR (400 MHz, d₆-DMSO): 8.1 (s, 1H), 7.80 (m, 2H), 7.29 (dd, 1H), 6.90 (m, 2H), 4.09 (t, 1H), 3.28 (m, 2H). MS (EI) for C₁₆H₁₂BrN₃O₂S: 390 (MH⁺).

### Compound 539

### 2-{1-amino-2-[4-(methyloxyl)phenyl]ethyl}-8-bromo[1]benzofuro[3,2-d]pyrimidin-4(3H)-one

2- {1-Amino-2-[4-(methyloxy)phenyl]ethyl} -8-bromo[1]benzofuro[3,2-*d*]pyrimidin-4(3H)-one was synthesized in a manner similar to **Compound 535,** wherein N-{[(1,1-dimethylethyl)oxy]carbonyl}-*O*-methyltyrosine replaced N-{[(1,1-dimethylethyl)oxy]carbonyl}phenylalanine. ¹H NMR (400 MHz, d₆-DMSO): 8.13 (s, 1H), 7.80 (m, 2H), 7.11 (d, 2H), 6.82 (d, 2H), 4.13 (t, 1H), 3.69 (s, 3H), 3.15 (m, 1H), 2.96 (m, 1H). MS (EI) for C₁₉H₁₆BrN₃O₃: 414 (MH⁺).

### Compound 540

### 2-[(1S)-1-amino-2-methylpropyl]-8-bromo[1]benzofuro[3,2-d]pyrimidin-4(3H)-one

2-[(1S)-1-Amino-2-methylpropyl]-8-bromo[1]benzofuro[3,2-d]pyrimidin-4(3H)-one was synthesized in a manner similar to **Compound 535,** wherein N-(tert-butoxycarbonyl)-L-valine replaced N-{[(1,1-dimethylethyl)oxy]carbonyl}phenylalanine. ¹H NMR (400 MHz, d₆-DMSO): 8.10 (dd, 1H), 7.73 (m, 2H), 6.56 (br s, 3H), 3.61 (d, 1H), 2.10 (m, 1H), 0.91 (d, 3H), 0.85 (d, 3H). MS (EI) for C₁₄H₁₄BrN₃O₂: 336 (MH⁺).

### Compound 541 2-[amino(cyclohexyl)methyl]-8-bromo[1]benzofuro[3,2-d]pyrimidin-4(3H)-one

2-[Amino(cyclohexyl)methyl]-8-bromo[1]benzofuro[3,2-d]pyrimidin-4(3H)-one was synthesized in a manner similar to **Compound 535,** wherein [(tert-butoxycarbonyl)amino](cyclohexyl)acetic acid replaced N-{[(1,1-dimethylethyl)oxy]carbonyl}phenylalanine. ¹H NMR (400 MHz, d₆-DMSO): 8.08 (s, 1H), 7.72 (m, 2H), 6.58 (br s, 3H), 3.61 (d, 1H), 1.80-1.55 (m, 5H), 1.40 (d, 1H), 1.10-1.05 (m, 5H). MS (EI) for C₁₇H₁₈BrN₃O₂: 376 (MH⁺).

### Compound 542

### 1-aminocyclohexanecarbonyl chloride hydrochloride

1-Aminocyclohexanecarboxylic acid (1785 mg, 12.5 mmol) was suspended in dichloromethane (50 mL) and hydrogen chloride gas was bubbled through the suspension for 2 min. The solvent was removed under vacuum to give 1-aminocyclohexane-carboxylic acid hydrochloride as a white solid. Phosphorus pentachloride (3150 mg, 15.1 mmol) was suspended in acetonitrile (50 mL) and the solid 1-aminocyclohexane-carboxylic acid hydrochloride was added in one portion. The solids dissolved quickly and a precipitate formed. The reaction mixture was stirred for 3 h under a nitrogen atmosphere. The precipitate was filtered off, washed with acetonitrile (10 mL) and dried under vacuum to give 2.20 g (89% yield) of 1-aminocyclohexanecarbonyl chloride hydrochloride as a white solid. The material was used without further purification.

### 3-{[(1-aminocyclohexyl)carbonyl]amino}-5-bromo-1-benzofuran-2-carboxamide hydrochloride

3-Amino-5-bromo-1-benzofuran-2-carboxamide (510 mg, 2.0 mmol) was dissolved in N,N-dimethylacetamide (10 mL) and 1-aminocyclohexanecarbonyl chloride hydrochloride (600 mg, 3.0 mmol) was added in one portion. The reaction was complete within 5 min. The reaction mixture was diluted with acetonitrile (15 mL) and stirred for 5 min. The precipitate was filtered off, washed with acetonitrile (5 mL) and dried under vacuum to give 692 mg (83% yield) of 3-{[(1-aminocyclohexyl)carbonyl]amino-5-bromo-1-benzofuran-2-carboxamide hydrochloride as a white solid. MS (EI) for C₁₆H₁₈BrN₃O₃: 380 (MH⁺).

### 2-(1-aminocyclohexyl)-8-bromo[1]benzofuro[3,2-d]pyrimidin-4(3H)-one

3- {[(1-Aminocyclohexyl)carbonyl]amino}-5-bromo-1-benzofuran-2-carboxamide hydrochloride (685 mg, 1.6 mmol) was suspended in ethanol (10 mL) and sodium hydroxide (8.3 mL, 1.0 M, 8.3 mmol) was added to form a clear solution. The reaction was stirred at 85 °C for 2.5 h. After cooling to room temperature, the pH of the reaction mixture was lowered to 8 by addition of 6 M hydrochloric acid over 20 min. The precipitate was filtered off, washed with water (2 mL) and acetonitrile (2 mL), and dried under vacuum to give 466 mg (78% yield) of 2-(1-aminocyclohexyl)-8-bromo[1]benzofuro[3,2-d]pyrimidin-4(3H)-one as a white solid. ¹H NMR (400 MHz, d₆-DMSO): 8.04 (s, 1H), 7.67 (m, 2H), 7.31 (br s, 3H), 2.21-2.14 (m, 2H), 1.71-1.55 (m, 7H), 1.33 (m, 1H). MS (EI) for C₁₆H₁₈BrN₃O₃: 380 (MH⁺).

### Compound 543

### 2-(1-aminocyclopentyl)-8-bromo[1]benzofuro[3,2-d]pyrimidin-4(3H)-one

2-(1-Aminocyclopentyl)-8-bromo[1]benzofuro[3,2-d]pyrimidin-4(3H)-one was synthesized in a manner similar to **Compound 543,** wherein 1-aminocyclopentane carboxylic acid replaced 1-aminocyclohexanecarboxylic acid. ¹H NMR (400 MHz, d₆-DMSO): 8.01 (dd, 1H), 7.66 (m, 2H), 7.55 (br s, 3H), 2.33 (m, 2H), 1.84 (m, 6H). MS (EI) for C₁₅H₁₄BrN₃O₂: 348 (MH⁺).

### Compound 544

### 2-(1-aminocyclobutyl)-8-bromo[1]benzofuro[3,2-d]pyrimidin-4(3H)-one

2-(1-Aminocyclobutyl)-8-bromo[1]benzofuro[3,2-d]pyrimidin-4(3H)-one was synthesized in a manner similar to **Compound 543,** wherein 1-aminocyclobutane carboxylic acid replaced 1-aminocyclohexanecarboxylic acid. ¹H NMR (400 MHz, d₆-DMSO): 8.08 (dd, 1H), 7.71 (m, 2H), 7.25 (br s, 3H), 2.70 (m, 2H), 2.15 (m, 2H), 2.06 (m, 1H), 1.94 (m, 1H). MS (EI) for C₁₄H₁₂BrN₃O₂: 334 (MH⁺).

Compounds disclosed are active againt either PIM (wherein PIM refers to PIM-1, PIM-2, and/or PIM-3 throughout this application), CDC7, or CK2 kinase activity (as defined by Activity **A**, Activity **B**, and Activity **C**). Accordingly, compounds of this dislcosure can also be useful for treating proliferative disorders associated with PIM, CDC7 or CK2 kinase activity.

### Characterization of CDC7 Inhibition in a Biochemical Chemiluminesence Assay (IC₅₀ determination):

The IC₅₀ evaluation of compounds against the CDC7 enzyme was performed in a Chemiluminesence assay utilizing a Greiner 384-well white binding plate format. Generally, 0.5 µL DMSO containing varying concentrations of the test compound was mixed with 15 5 µL buffer solution consisting of 50 mM Hepes (pH = 7.5), 10 mM MgCl₂, 0.02% BSA, 0.02% Tween, 0.02% Brij-35 and 1mM DTT. Upon addition of 10 µL of CDC-7 (GB) to the designated well plates and subsequent incubation for ten minutes at ambient temperature, 10 µL of ATP mix in Tris Base was added. Following an incubation period of 90 minutes at ambient temperature (24-27 °C), 20 µL of Kinase Glo (Promega) was added to measure kinase activity by quantitating the amount of ATP remaining in solution. The luminescence was measured following a Luciferase Protocol on either En Vision or Victor plate readers.

### Characterization of PIM-1 Inhibition in a Biochemical Chemiluminesence Assay (IC₅₀ determination):

The IC₅₀ evaluation of compounds against the PIM-1 oncogene was performed in a Chemiluminesence assay utilizing a Greiner 384-well white medium binding white plate format. Generally, 0.5 µL DMSO containing varying concentrations of the test compound was mixed with stock buffer solution consisting of 20 mL of 1M Hepes (pH = 7.4), 10 mL of 1M MgCl₂, 3 mL of 10% Triton X-100 and 68mL of Milli-Q water followed by subsequent addition of 1mM DTT (Dithiotheritol). The assay procedure involves compound dilution followed by addition of 4.6 nM PIM-1 to the designated well plates and subsequent pre-incubation of the compounds with the enzyme for thirty minutes at ambient temperature (24-27 °C). 10 µL of PIM-1 substrate/ATP mix (4nM AKRRRLSA substrate sequence/ 1 µM ATP) was added twice. Following an incubation period of 120 minutes at ambient temperature (24-27 °C), 10 µL of Kinase Glo (Promega) was added. The luminescence was measured following a Luminescence Protocol on a Victor plate reader. Final assay conditions required 2.3 nM PIM-1 (protooncogene-encoded protein Kinase PIM-1 substrate), 0.5 µM ATP, 10 µM AKRRRLSA sequence, 20.5 µL reaction volume, 2.5% final concentration in DMSO with a solvent to buffer ratio of 2.5.

### Characterization of PIM-2 Inhibition in a Biochemical Chemiluminesence Assay (IC₅₀ determination):

The IC₅₀ evaluation of compounds against the PIM-2 oncogene was performed in a Chemiluminesence assay utilizing a Greiner 384-well white medium binding white plate format. Generally, 0.5 µL DMSO containing varying concentrations of the test compound was mixed with stock buffer solution consisting of 20 mL of 1M Hepes (pH = 7.4), 10 mL of 1 MMgCl₂, 3 mL of 10% Triton X-100 and 68 mL of Milli-Q water followed by subsequent addition of 1mM DTT. The assay procedure involves compound dilution followed by addition of 3 nM PIM-2 to the designated well plates and subsequent pre-incubation of the compounds with the enzyme for 30 minutes at ambient temperature (24-27 °C). Thereafter, 10 µL of PIM substrate/ATP mix (4nM AKRRRLSA substrate sequence/ 1µM ATP) was added twice. Following an incubation period of 120 minutes at ambient temperature (24-27 °C), 10 µL of Kinase Glo (Promega) was added. The luminescence was measured following a Luminescence Protocol on a Victor plate reader. Final assay conditions required 1.5 nM PIM-2, 0.5 µM ATP, 10 µM AKRRRLSA sequence, 20.5 µL reaction volume, 2.5% final concentration in DMSO with a solvent to buffer ratio of 2.0.

### Characterization of PIM-3 Inhibition in a Biochemical Chemiluminesence Assay (IC₅₀ determination):

The IC₅₀ evaluation of compounds against the PIM-3 Oncogene was performed in a Chemiluminesence assay utilizing a Greiner 384-well white medium binding white plate format. Generally, 0.5 µL DMSO containing varying concentrations of the test compound was mixed with stock buffer solution consisting of 20 mL of 1M Hepes (pH = 7.4), 10 mL of 1M MgCl₂, 3 mL of 10% Triton X-100 and 68 mL of Milli-Q water followed by subsequent addition of 1mM DTT. The assay procedure involves the compound dilution followed by addition of 1.6 nM PIM-3 to the designated well plates and subsequent pre-incubation of the compounds with the enzyme for 30 minutes at ambient temperature (24-27 °C). Thereafter, 10 µL of PIM substrate/ATP mix (4nM AKRRRLSA substrate sequence/ 1µM ATP) was added twice. Following an incubation period of 120 minutes at ambient temperature (24-27 °C), 10 µL of Kinase Glo (Promega) was added. The luminescence was measured following a Luminescence Protocol on a Victor plate reader. Final assay conditions required 0.8 nM PIM-3, 0.5 µM ATP, 10 µM AKRRRLSA sequence, 20.5 µL reaction volume, 2.5% final concentration in DMSO with a solvent to buffer ratio of 2.5

### Characterization of CK2 Inhibition in a Biochemical Chemiluminesence Assay (IC₅₀ determination):

The IC₅₀ evaluation of compounds against CK2 was performed in a Chemiluminesence Holoenzyme assay utilizing a Greiner 384-well white medium binding white plate format. Generally, 0.5 µL DMSO containing varying concentrations of the test compound was mixed with stock buffer solution consisting of 1M Tris base (pH = 7.5), 10mM MgCl₂, 0.03% Triton X-100 followed by subsequent addition of 1mM DTT and 0.1mMNaVO3. The assay procedure involves the compound dilution scheme followed by addition of 10µL of the substrate twice, followed by 10 µL twice of the respective enzyme or buffer to the designated well plates and subsequent pre-incubation of the compounds. Following an incubation period of 150 minutes at ambient temperature (24-27 °C),10 µL of Kinase Glo (Promega) was added. The luminescence was measured following a Luminescence Protocol on a Victor plate reader. The substrate stock solution requires 10mM ATP and 1mM of Casein and the enzyme stock solution requires 12.3uM of the holoenzyme at a 10nM final concentration.

## Claims

1. A compound according to formula I: 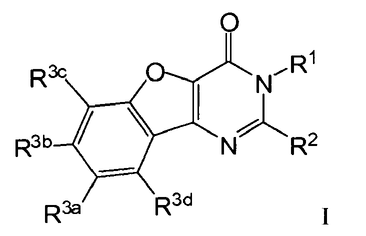 or a pharmaceutically acceptable salt thereof, wherein:
R¹ is hydrogen or (C₁-C₁₂)alkyl;
R₂ is selected from -NH-phenyl, -NH-piperidinyl, -NH-pyridinyl, -NH(C₁-C₃)alkylphenyl optionally substituted at any phenyl position with piperazinyl or methylpiperazinyl, -NH(C₁-C₃)alkyl-N(CH₃)₂, -NH(C₁-C₃)alkyl-OH, -(C₁-C₃)alkyl-O-phenyl, -(C₁-C₃)alkyl-O-(C₁-C₃)alkyl-(5-6 membered)heterocycloalkyl, -(C₁-C₃)alkyl-N(H)-heteroaryl, -(C₁-C₃)alkyl-(5-10)membered heteroaryl optionally substituted with -(C₁-C₃)alkyl, halo or phenyl, oxopyrrolidinyl optionally substituted with OH or piperidinyl, -(C₁-C₄)alkyl-(3-9 membered)heterocycloalkyl optionally substituted at the (3-9 membered)heterocycloalkyl with Xd, -(C₁-C₆)alkyl-NRzb-(C1-C₄)alkyl wherein the -(C₁-C₄)alkyl portion is substituted with Xe, -(C₁-C₃)alkyl-NH-(C₃-C₆)cycloalkyl, -(CH₂)-NH-(C₃-C₆)cyclohexyl, -(C₁-C₃)alkyl-NH₂, wherein the -(C₁-C₃)alkyl- portion of -(C₁-C₃)alkyl-NH₂ is substituted with Xf, and -(3-9 membered)heterocycloalkyl optionally substituted with Xg;
Xd is selected from (C₁-C₁₂)alkyl, 1-3 halo, -COOH, phenyl optionally substituted with 1 or 2 groups selected from halo, methyl and methylphenyl, phenylmethyl, spiro-piperidine, trifluoromethylphenylmethyl, -(C₁-C₃)alkoxy, pyridinyl, dimethylamino(C₁-C₁₂)alkyl, dimethylamino, hydroxyl(C₁-C₁₂alkyl, dimethylamino(C₁-C₁₂)alkylaminocarbonyl, (C₁-C₁₂)alkylamino, amino(C₁-C₁₂)alkyl, dimethylaminocarbonyl(C₁-C₁₂)alkyl, diethylamino(C₁-C₁₂)alkylcarbonyl, -(C₁-C₃)alkyl-(5-6 membered)heterocycloalkyl, (5-6 membered)heterocycloalkyl optionally substituted with -(C₁-C₃)alkyl, -NH₂, -OH, (4-12 membered)heterocycloalkyl(C₁-C₁₂)alkylamino, (C₁-C₁₂)alkoxy(C₁-C₁₂)alkyl, -C(O)CH₃, -C(O)NH(C₁-C₃)alkylphenyl optionally substituted with 1-3 halo at any phenyl position, one -OH and one -C(O)NH(C₁-C₃)alkylphenyl optionally substituted with 1-3 halo at any phenyl position, -C(O)-(4-12 membered)heterocycloalkyl optionally substituted with -OH or halo, (C₁-C₁₂)alkoxycarbonyl, aminocarbonyl, one -OH and one methyl, one -OH and one - C(O)OH, one -OH and one -NHC(O)piperidinyl, one -OH and one (C₁-C₁₂)alkyl;
Xe is selected from dialkylamino, amino, 1-3 -OH, (C₁-C₁₂)alkoxy, 4-methylpiperazinylphenyl, dimethylaminophenyl, phenyl optionally substituted with 1-3 groups selected from halo and methoxy, (5-12 membered)heteroaryl, -(C₁-C₃)alkylC(O)NH₂, -C(O)NH₂, -C(O)OH, -(C₁-C₃)alkylC(O)OH, and (4-12 membered)heterocycloalkyl optionally substituted with 1-2 (C₁-C₁₂)alkyl;
Xfis selected from (C₃-C₁₄)cycloalkyl, spiro(C₃-C₁₄)cycloalkyl, phenyl, phenyl(C₁-C₁₂)alkyl optionally substituted with phenylmethyloxy or (C₁-C₁₂)alkoxy, and thienyl(C₁-C₁₂)alkyl;
Xg is selected from (C₁-C₁₂)alkyl, (C₁-C₁₂)alkylcarbonyl, (4-12 membered)heterocycloalkylcarbonyl, dialkylamino(C₁-C₁₂alkylcarbonyl, 1-methylpiperidinyl, dialkylamino(C₁-C₁₂)alkyl, (5-12 membered)heteroarylcarbonyl, (C₁-C₁₂)alkoxy(C₁-C₁₂)alkylcarbonyl, phenylcarbonyl, phenyl(C₁-C₁₂)alkylcarbonyl, oxo, phenyl(C₁-C₁₂)alkyl, -(5-6 membered)heteroaryl(C₁-C₁₂)alkyl, piperidinyloxy, -OH, oxo, 1-2 halo and 1-2 methyl; and
Rzb is H or alkyl optionally substituted with 1-3 -OH;
R^{3a} is selected from halo, (C₁-C₁₂)alkyl, -NO₂, (C₁-C₁₂)alkoxy, (C₁-C₁₂)alkynyl optionally substituted with R¹⁴, (C₁-C₁₂)alkoxycarbonyl(C₁-C₁₂)alkyl, (C₆-C₁₄)aryl(C₁-C₁₂)alkoxy, -C(O)N(H)(C₁-C₁₂)alkyl, -N(H)-C(O)-(C₁-C12)alkyl, -C(O)-(C₁-C₁₂)alkyl, - CN, phenyl, -OCF₃, -N(H)R¹³, -OH, -CF₃, -S-CH₃ and hydroxymethyl(C₁-C₁₂)alkynyl;
R^{3b}, R^{3c} and R^{3d} are each independently selected from H, -OH, -N⁺(O)OH, (C₁-C₁₂)alkoxyl, and halo;
or R^{3a} is hydrogen and R^{3b}, R^{3c} and R^{3d} are each independently selected from - CF₃, -OH, (C₁-C₁₂)alkoxy, and halo;
or R^{3a} and R^{3d}, together with the carbons to which they are attached, join to form a 5 membered heteroaryl optionally substituted with methyl or -NH₂, or a 5-6 membered heterocycloalkyl;
R¹³ is selected from (C₆-C₁₄)aryl(C₁-C₁₂)alkyl wherein the aryl portion of arylalkyl is optionally substituted with 1, 2 or 3 (C₁-C₁₂)alkoxy, halo, methyl, methoxy, - CF₃, (C₃-C₁₄)cycloalkyl, and (5-12 membered)heteroaryl(C₁-C₁₂)alkyl; and
R¹⁴ is selected from hydroxyl(C₁-C₁₂)alkyl, H and TMS.

2. The compound according to claim 1, or a pharmaceutically acceptable salt thereof, wherein R^{3a} is selected from halo, (C₁-C₁₂)alkyl, -NO₂, (C₁-C₁₂)alkoxy, (C₁-C₁₂)alkynyl optionally substituted with R¹⁴, (C₁-C₁₂)alkoxycarbonyl(C₁-C₁₂)alkyl, (C₆-C₁₄)aryl(C₁-C₁₂)alkoxy, -C(O)N(H)(C₁-C₁₂)alkyl, -N(H)-C(O)-(C₁-C₁₂)alkyl, -C(O)-(C₁-C₁₂)alkyl, - CN, phenyl, -OCF₃, -N(H)R¹³, -OH, -CF₃, -S-CH₃ and hydroxymethyl(C₁-C₁2)alkynyl; and R^{3b} , R^{3c} and R^{3d} are each H.

3. The compound according to claim 1, or a pharmaceutically acceptable salt thereof, wherein R^{3a} is halo, (C₁-C₁₂alkoxy or -OCF₃; and R^{3b}, R^{3c} and R^{3d} are each H.

4. The compound according to claim 1, or a pharmaceutically acceptable salt thereof, wherein R² is a (3-9 membered)heterocycloalkyl optionally substituted with Xg;; R^{3a} is halo, (C₁-C₁₂)alkoxy or -OCF₃; and R^{3b}, R^{3c} and R^{3d} are each H.

5. The compound according to claim 1, or a pharmaceutically acceptable salt thereof, wherein R is -(C₁-C₃)alkyl-(5-10)membered heteroaryl, wherein the heteroaryl is optionally substituted with -(C₁-C₃)alkyl, halo and phenyl , ; R^{3a} is halo, (C₁-C₁₂)alkoxy or -OCF₃; and R^{3b}, R^{3c} and R^{3d} are each H.

6. The compound according to claim 1, or a pharmaceutically acceptable salt thereof, wherein R is-(C₁-C₄)alkyl-(3-9 membered)heterocycloalkyl optionally substituted at any position of the (3-9 membered)heterocycloalkyl with Xd ; R^{3a} is halo, (C₁-C₁₂)alkoxy or - OCF₃; and R^{3b}, R^{3c} and R^{3d} are each H.

7. The compound according to claim 1, or a pharmaceutically acceptable salt thereof, wherein R² is a heterocycloalkyl selected from piperazinyl, piperidinyl, pyrrolidinyl, isoxazolyl, azetidinyl, morpholinyl, tetrahydrofuranyl, thiazolidinyl or octahydro-1H-indolyl, wherein the heterocycloalkyl is optionally substituted with Xg; R^{3a} is halo, (C₁-C₁₂)alkoxy or -OCF₃; and R^{3b}, R^{3c} and R^{3d} are each H.

8. The compound according to claim 1, or a pharmaceutically acceptable salt thereof, wherein R² is (3-9 membered)heterocycloalkyl(C₁-C₄)alkyl, and wherein the heterocycloalkyl portion of the heterocycloalkylalkyl is piperazinyl, piperidinyl, pyrrolidinyl, azetidinyl, morpholinyl, 1,4-diazepanyl, 2,5-diazabicyclo[2.2.1]heptyl, azabicyclo[2.2.1]heptane, 8-azabicylo[3.2.1]oct-8-yl, (1S,4S)-2,5-diazabicyclo[2.2.1]heptyl, 2,5-dihydro-1H-pyrrolyl, (1R,4R)-2,5-diazabicyclo[2.2.1]heptan-2-yl, (1R,5S)-8-azabicyclo[3.2.11]octyl, (8aS)-hexahydropyrrolo[1,2-a]pyrazin-2(1H)-yl, dimethylpiperazinyl or (3aR,6aS)-hexahydropyrrolo[3,4-c]pyrrolyl.

9. The compound according to claim 1, or a pharmaceutically acceptable salt thereof, wherein R² is a (5-10 membered)heteroaryl(C₁-C₃)alkyl optionally substituted at any ring position with -(C₁-C₃)alkyl, halo and phenyl; R^{3a} is halo, (C₁-C₁₂)alkoxy or -OCF₃; and R^{3b}, R^{3c} and R^{3d} are each H.

10. The compound according to claim 1, or a pharmaceutically acceptable salt thereof, wherein R₂ is 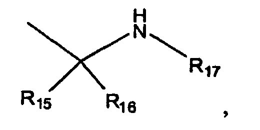 wherein R₁₅ is selected from H or -(C₁-C₆)alkyl, R₁₆ is selected from H, phenyl and -(C₁-C₆)alkyl, and R₁₇ is selected from H, -(C₁-C₃)alkylC(O)NH₂, -(C₁-C₃)alkylC(O)OH and (4-12 membered)heterocycloalkyl(C₁-C1₂)alkyl.

11. The compound according to claim 1 selected from one of the following compounds:
| **Cpd No.** | **Structure** | **NAME** |
|---|---|---|
| 1 | 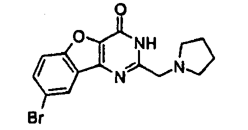 | 8-bromo-2-(pyrrolidin-1-ylmethyl)[1]benzofuro[3,2 -d]pyrimidin-4(3H)-one |
| 2 | 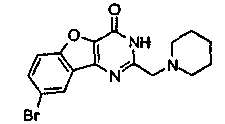 | 8-bromo-2-(piperidin-1-ylmethyl)[1]benzofuro[3,2 -d]pyrimidin-4(3 H)-one |
| 3 | 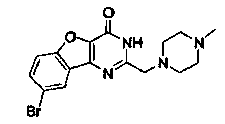 | 8-bromo-2-[(4-methylpiperazin-1-yl)methyl][1]benzofuro[3, 2-d]pyrimidin-4(3H)-one |
| 4 | 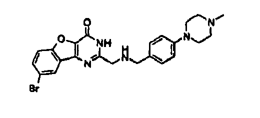 | 8-bromo-2-[({[4-(4-methylpiperazin-1-yl)phenyl]methyl}amino) methyl][1]benzofuro[3,2-d]pyrimidin-4(3H)-one |
| 5 | 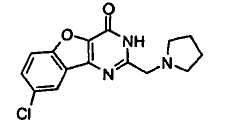 | 8-chloro-2-(pyrrolidin-1-ylmethyl)[1]benzofuro[3,2 -d]pyrimidin-4(3H)-one |
| 6 | 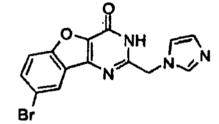 | 8-bromo-2-(1H-imidazol-1-ylmethyl)[1]benzofuro[3,2 -d]pyrimidin-4(3H)-one |
| 7 | 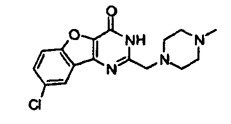 | 8-chloro-2-[(4-methylpiperazin-1-yl)methyl][1]benzofuro[3, 2-d]pyrimidin-4(3H)-one |
| 9 | 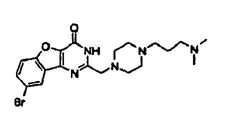 | 8-bromo-2-({4-[3-(dimethylamino)propyl]pi perazin-1- yl}methyl)[1]benzofuro[3, 2-d]pyrimidin-4(3H)-one |
| 10 | 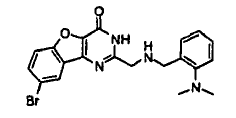 | 8-bromo-2-[({[2(dimethylamino)phenyl]m ethyl} amino)methyl][1]be nzofuro[3,2-d]pyrimidin-4(3H)-one |
| 13 | 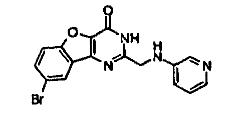 | 8-bromo-2-[(pyridin-3-ylamino)methyl][1]benzof uro[3,2-d]pyrimidin-4(3H)-one |
| 14 | 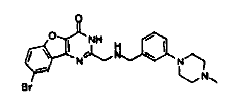 | 8-bromo-2-[({[3-(4-methylpiperazin-1-yl)phenyl]methyl} amino) methyl][1]benzofuro[3,2-d]pyrimidin4(3H)-one |
| 15 | 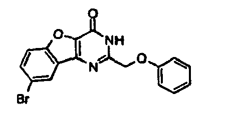 | 8-bromo-2-[(phenyloxy)methyl][1]be nzofuro[3,2-d]pyrimidin-4(3H)-one |
| 17 | 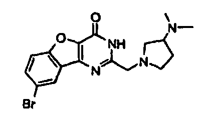 | 8-bromo-2-{[3-(dimethylamino)pyrrolidin -1-1]methyl} [1]benzofuro[3,2 -d]pyrimidin-4(3H)-one |
| 20 | 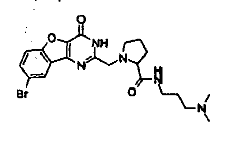 | 1-[(8-bromo-4-oxo-3,4-dihydro[1]benzofuro[3,2-d]pyrimidin-2-yl)methyl]-N-[3-(dimethylamino)propyl]pr olinamide |
| 21 | 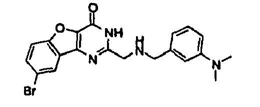 | 8-bromo-2-[({[3-(dimethylamino)phenyl]m ethyl} amino)methyl][1]be nzofuro[3,2-d]pyrimidin-4(3H)-one |
| 23 | 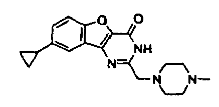 | 8-cyclopropyl-2-[(4-methylpiperazin-1-yl)methyl][1]benzofuro[3, 2-d]pyrimidin-4(3H)-one |
| 24 | 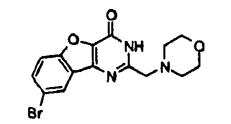 | 8-bromo-2-(morpholin-4-ylmethyl)[1]benzofuro[3,2 -d]pyrimidin-4(3H)-one |
| 25 | 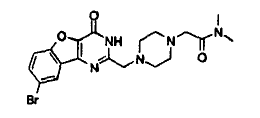 | 2-{4-[(8-bromo-4-oxo-3,4-dihydro[1]benzofuro[3,2-d]pyrimidin-2-yl)methyl]piperazin-1-yl} -N,N-dimethylacetamide |
| 26 | 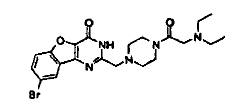 | 8-bromo-2-{[4-(N,N-diethylglycyl)piperazin-1- yl]methyl} [1]benzofuro[3, 2-d]pyrimidin-4(3H)-one |
| 28 | 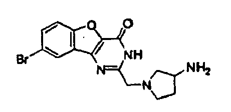 | 2-[(3-aminopyrrolidin-1-yl)methyl]-8-bromo[1]benzofuro[3,2-d]pyrimidin-4(3H)-one |
| 29 | 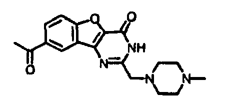 | 8-Acetyl-2-[(4-methylpiperazin-1-yl)methyl][1]benzofuro[3, 2-d]pyrimidin4(3H)-one |
| 45 | 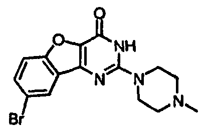 | 8-bromo-2-(4-methylpiperazin-1-yl)[1]benzofuro[3,2-d]pyrimidm-4(3H)-one |
| 46 | 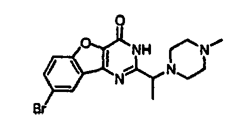 | 8-bromo-2-[1-(4-methylpiperazin-1-yl)ethyl][1]benzofuro[3,2-d]pyrimidin4(3H)-one |
| 49 | 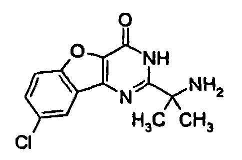 | 2-(1-amino-1-methylethyl)-8-chloro[1]benzofuro[3,2-d]pynmidin-4(3H)-one |
| 74 | 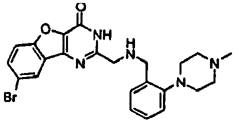 | 8-bromo-2-[({[2-(4-methylpiperazin-1-yl)phenyl]methyl} amino) methyl] [1]benzofuro[3,2-d]pyrimidin-4(3H)-one |
| 81 | 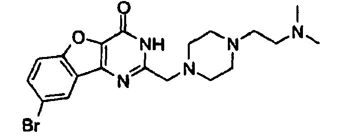 | 8-bromo-2-({4-[2-(dimethylamino)ethyl]pipe razin-1-yl} methyl)[1]benzofuro[3, 2-d]pyrimidin-4(3H)-one |
| 82 | 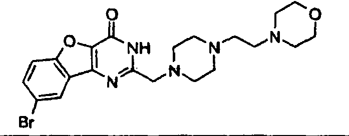 | 8-bromo-2-{[4-(2-morpholin-4-ylethyl)piperazin-1-yl]methyl}[1]benzofuro[3, 2-d]pyrimidin-4(3H)-one |
| 84 | 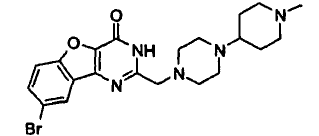 | 8-bromo-2-{[4-(1-methylpiperidin-4-yl)piperazin-1-yl]methyl} [1]benzofuro[3, 2-d]pyrimidin-4(3H)-one |
| 100 | 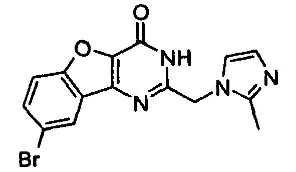 | 8-bromo-2-[(2-methyl-1H-imidazol-1-yl)methyl][1]benzofuro[3, 2-d]pyrimidin-4(3H)-one |
| 103 | 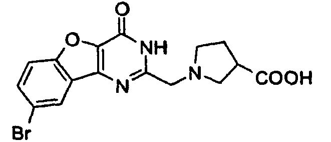 | 1-[(8-bromo-4-oxo-3,4-dihydro[1]benzofuro[3,2-d]pyrimidin-2-yl)methyl]pyrrolidine-3-carboxylic acid |
| 104 | 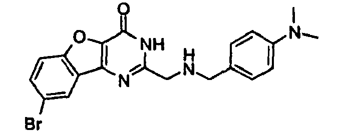 | 8-bromo-2-[({[4-(dimethylamino)phenyl]m ethyl} amino)methyl][1]be nzofuro[3,2-d]pyrimidin-4(3H)-one |
| 105 | 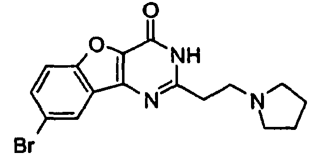 | 8-bromo-2-(2-pyrrolidin-1-ylethyl)[1]benzofuro[3,2-d]pyrimidin-4(3H)-one |
| 108 | 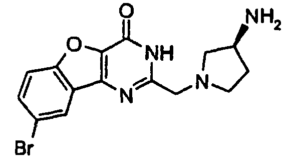 | 2-{[(3S)-3-aminopyrrolidin-1-yl]methyl}-8-bromo[1]benzofuro[3,2-d]pyrimidin-4(3H)-one |
| 109 | 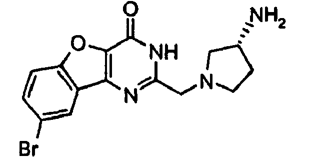 | 2-{[(3R)-3-aminopyrrolidin-1-yl]methyl}-8-bromo[1]benzofuro[3,2-d]pyrimidin-4(3H)-one |
| 110 | 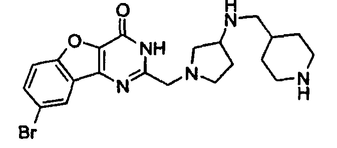 | 8-bromo-2-({3-[(piperidin-4-ylmethyl)amino]pyrrolidin -1-yl}methyl)[1]benzofuro[3, 2-d]pyrimidin-4(3H)-one |
| 111 | 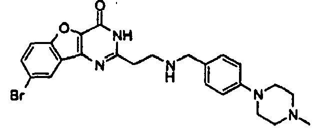 | 8-bromo-2-[2-({[4-(4-methylpiperazin-1-yl)phenyl]methyl}amino)e thyl][1]benzofuro[3,2-d]pyrimidm-4(3H)-one |
| 113 | 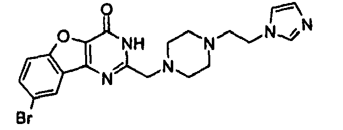 | 8-bromo-2-({4-[2-(1H-imidazol-1-yl)ethyl]piperazin-1-yl}methyl)[1]benzofuro[3, 2-d]pyrimidin-4(3H)-one |
| 114 | 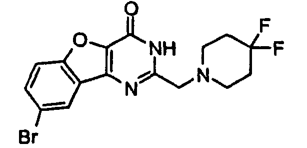 | 8-bromo-2-[(4,4-difluoropiperidin-1-yl)methyl][1]benzofuro[3, 2-d]pyrimidin-4(3H)-one |
| 115 | 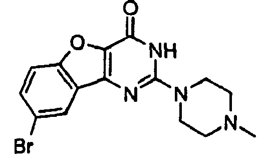 | 8-bromo-2-(4-methylpiperazin-1-yl)[1]benzofuro[3,2-d]pyrimidin-4(3H)-one |
| 117 | 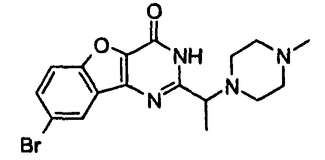 | 8-bromo-2-[1-(4-methylpiperazin-1-yl)methyl][1]benzofuro[3,2-d]pyrimidm-4(3H)-one |
| 118 | 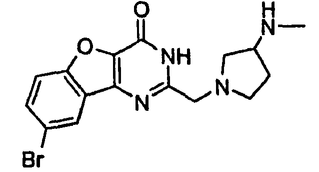 | 8-bromo-2-{[3-(methylamino)pyrrolidin-1-yl]methyl} [1]benzofuro[3, 2-d]pyrimidin-4(3H)-one |
| 119 | 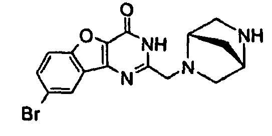 | 8-bromo-2-[(1S,4S)2,5-diazabicyclo[2.2.1]hept-2-ylmethyl][1]benzofuro[3,2 -d]pyrimidin-4(3H)-one |
| 122 | 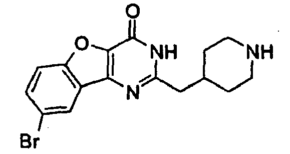 | 8-bromo-2-(piperidin-4-ylmethyl)[1]benzofuro[3,2 -d]pyrimidin-4(3H)-one |
| 130 | 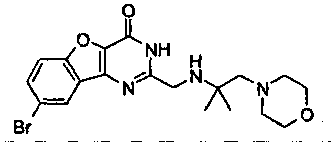 | 8-bromo-2-{[(1,1-dimethyl-2-morpholin-4-ylethyl)amino]methyl}[1] benzofuro[3,2-d]pyrimidin-4(3H)-one |
| 131 | 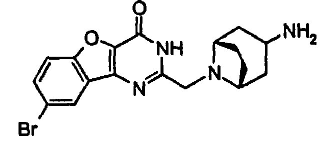 | 2-{[(1R,5S)-3-amino-8-azabicyclo[3.2.1]oct-8-yl]methyl}-8-bromo[1]benzofuro[3,2-d]pyrimidin-4(3H)-one |
| 132 | 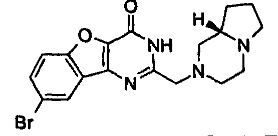 | 8-bromo-2-[(8aS)-hexahydropyrrolo[1,2-a]pyrazin-2(1H)-ylmethyl][1]benzofuro[3,2 -d]pyrimidin4(3H)-one |
| 133 | 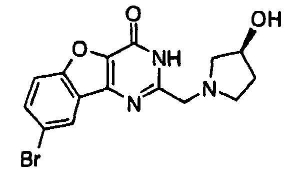 | 8-bromo-2-{[(3S)-3-hydroxypyrrolidin-1-yl]methyl} [1]benzofuro[3, 2-d]pyrimidin-4(3H)-one |
| 141 | 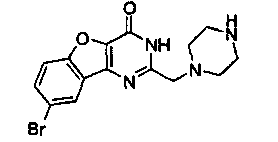 | 8-bromo-2-(piperazin-1-ylmethyl)[1]benzofuro[3,2 -d]pyrimidin-4(3H)-one |
| 142 | 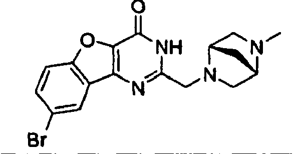 | 8-bromo-2-{[(1S,4S)-5-methyl-2,5-diazabicyclo[2.2.1]hept-2-yl]methyl} [1]benzofuro[3, 2-d]pyrimidin-4(3H)-one |
| 147 | 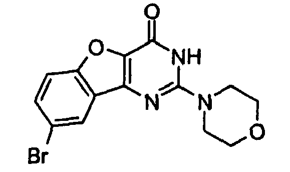 | 8-bromo-2-morpholin-4-yl[1]benzofuro[3,2-d]pyrimidin-4(3H)-one |
| 149 | 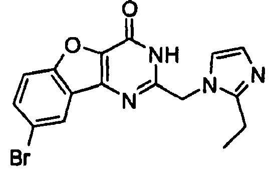 | 8-bromo-2-[(2-ethyl-1H-imidazol-1-yl)methyl][1]benzofuro[3, 2-d]pyrimidin-4(3H)-one |
| 150 | 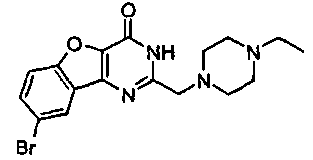 | 8-bromo-2-[(4-ethylpiperazin-1-yl)methyl][1]benzofuro[3, 2-d]pyrimidin-4(3H)-one |
| 151 | 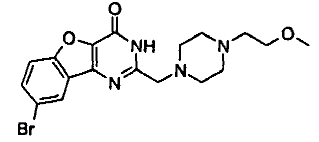 | 8-bromo-2-({4-[2-(methyloxy)ethyl]piperazi n-1-yl}methyl)[1]benzofuro[3, 2-d]pyrimidin-4(3H)-one |
| 153 | 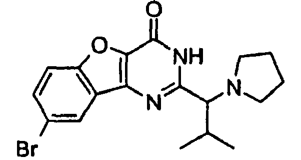 | 8-bromo-2-(2-methyl-1-pyrrolidin-1-ylpropyl)[1]benzofuro[3,2 -d]pyrimidin-4(3H)-one |
| 157 | 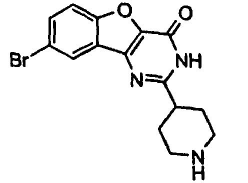 | 8-bromo-2-piperidin-4-yl[1]benzofuro[3,2-d]pyrimidin-4(3H)-one |
| 158 | 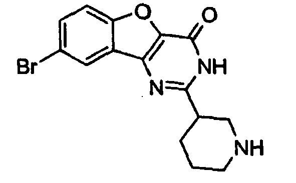 | 8-bromo-2-piperidin-3-yl[1]benzofuro[3,2-d]pyrimidin-4(3H)-one |
| 164 | 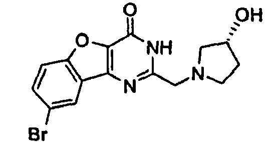 | 8-bromo-2-{[(3R)-3-hydroxypyrrolidin-1-yl]methyl}[1]benzofuro[3, 2-d]pyrimidin-4(3H)-one |
| 165 | 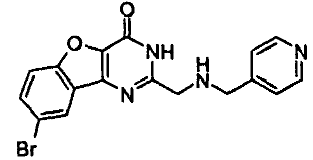 | 8-bromo-2-{[(pyridin-4-ylmethyl)amino]methyl}[ 1]benzofuro[3,2-d]pyrimidin4(3H)-one |
| 166 | 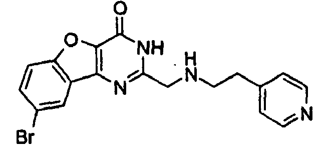 | 8-bromo-2-{[(2-pyridin-4-ylethyl)amino]methyl}[1] benzofuro[3,2-d]pyrimidin-4(3H)-one |
| 167 | 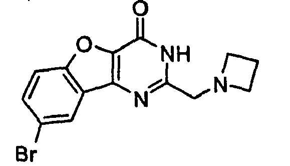 | 2-(azetidin-1-ylinethyl)-8-bromo[1]benzofuro[3,2-d]pyrimidin-4(3H)-one |
| 168 | 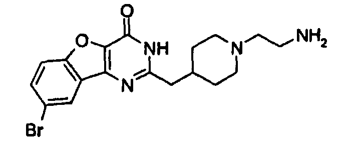 | 2-{[1-(2-aminoethyl)piperidin-4-yl]methyl}-8-bromo[1]benzofuro[3,2-d]pyrimidin-4(3H)-one |
| 169 | 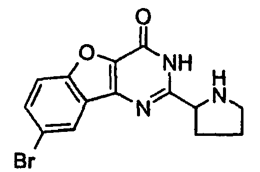 | 8-bromo-2-pyrrolidin-2-yl[1]benzofuro[3,2-d]pyrimidin-4(3H)-one |
| 172 | 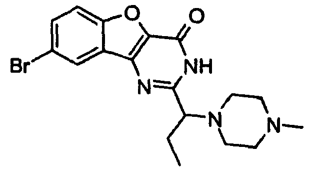 | 8-bromo-2-[1-(4-methylpiperazin-1-yl)propyl][1]benzofuro[3, 2-d]pyrimidin-4(3H)-one |
| 174 | 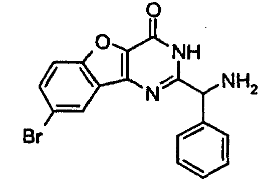 | 2-[amino(phenyl)methyl]-8-bromo[1]benzofuro[3,2-d]pyrimidin4(3H)-one |
| 175 | 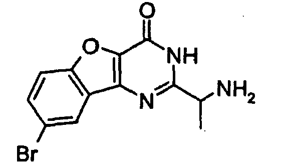 | 2-(1-aminoethyl)-8-bromo[1]benzofuro[3,2-d]pyrimidin-4(3H)-one |
| 176 | 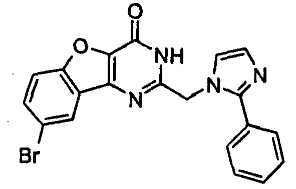 | 8-bromo-2-[(2-phenyl-1 H-imidazol-1-yl)methyl][1]benzofuro[3, 2-d]pyrimidin-4(3H)-one |
| 177 | 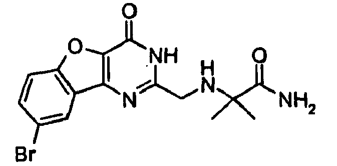 | N²-[(8-bromo-4-oxo-3,4-dihydro[1]benzofuro[3,2-d]pyrimidin-2-ylmethyl]-2-methylalaninamide |
| 180 | 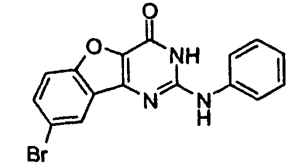 | 8-bromo-2-(phenylamino)[1]benzofur o[3,2-d]pyrimidin-4(3H)-one |
| 183 | 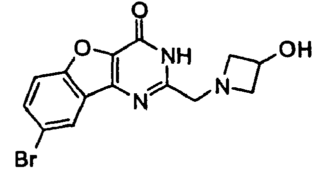 | 8-bromo-2-[(3-hydroxyazetidin-1-yl)methyl][1]benzofuro[3, 2-d]pyrimidin-4(3H)-one |
| 184 | 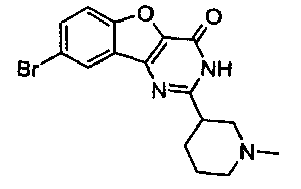 | 8-bromo-2-(1-methylpiperidin-3-yl)[1]benzofuro[3,2-d]pyrimidin-4(3H)-one |
| 185 | 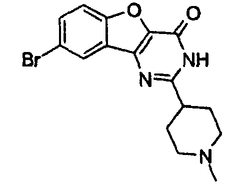 | 8-bromo-2-(1-methylpiperidin-4-yl)[1]benzofuro[3,2-d]pyrimidin-4(3H)-one |
| 186 | 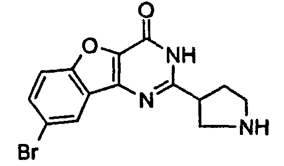 | 8-bromo-2-pyrrolidin-3-yl[1]benzofuro[3,2-d]pyrimidin-4(3H)-one |
| 187 | 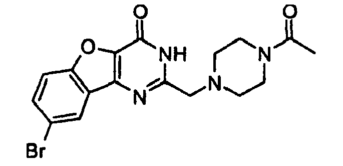 | 2-[(4-acetylpiperazin-1-yl)methyl]-8-bromo[1]benzofuro[3,2-d]pyrimidin4(3H)-one |
| 189 | 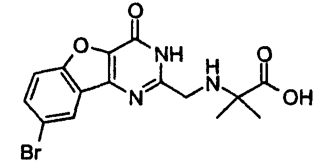 | N-[(8-bromo-4-oxo-3,4-dihydro[1]benzofuro[3,2-d]pyrimidin-2-yl)methyl]-2-methylalanine |
| 190 | 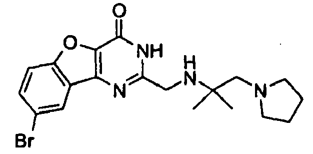 | 8-bromo-2-{[(1,1-dimethyl-2-pyrrolidin-1-ylethyl)amino]methyl}[1] benzofuro[3,2-d]pyrimidin-4(3H)-one |
| 191 | 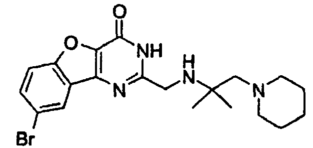 | 8-bromo-2-{[(1,1-dimethyl-2-piperidin-1-ylethyl)amino]methyl}[1] benzofuro[3,2-d]pyrimidin4(3H)-one |
| 192 | 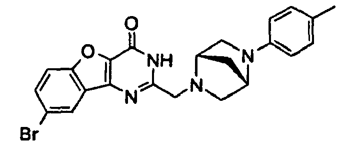 | 8-bromo-2-{[(1S,4S)-5-(4-methylphenyl)-2,5-diazabicyclo[2.2.1]hept-2-yl]methyl}[1]benzofuro[3, 2-d]pyrimidin4(3H)-one |
| 193 | 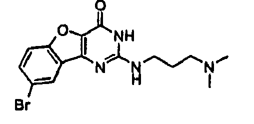 | 8-bromo-2-{[3-(dimethylamino)propyl]a mino}[1]benzofuro[3,2-d]pyrimidin4(3H)-one |
| 194 | 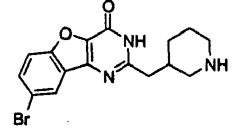 | 8-bromo-2-(piperidin-3-ylmethyl)[1]benzofuro[3,2 -d]pyrimidin-4(3H)-one |
| 195 | 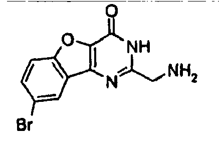 | 2-(aminomethyl)-8-bromo[1]benzofuro[3,2-d]pyrimidin-4(3H)-one |
| 196 | 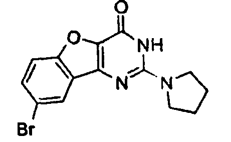 | 8-bromo-2-pyrrolidin-1-yl [1]benzofuro[3,2- d]pyrimidin-4(3H)-one |
| 198 | 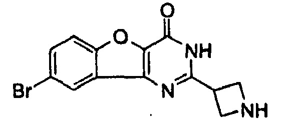 | 2-azetidin-3-yl-8bromo[1]benzofuro[3,2-d]pyrimidin-4(3H)-one |
| 199 | 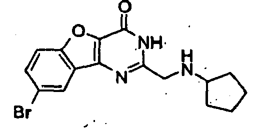 | 8-bromo-2-[(cyclopentylamino)methy 1][1]benzofuro[3,2-d]pyrimidin4(3H)-one |
| 200 | 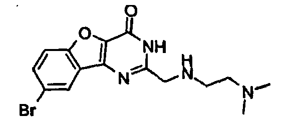 | 8-bromo-2-({[2-(dimethylamino)ethyl]ami no} methyl)[1]benzofuro[3 ,2-d]pyrimidin-4(3H)-one |
| 201 | 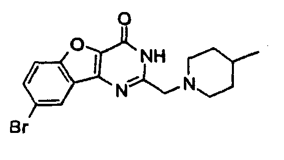 | 8-bromo-2-[(4-methylpiperidin-1-yl)methyl][1]benzofuro[3, 2-d]pyrimidin4(3H)-one |
| 202 | 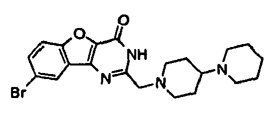 | 2-(1,4'-bipiperidin-1'-ylmethyl)-8-bromo[1]benzofuro[3,2-d]pyrimidin4(3H)-one |
| 203 | 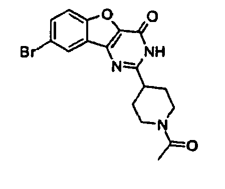 | 2-(1-acetylpiperidin-4-yl)-8-bromo[1]benzofuro[3,2-d]pyrimidin-4(3H)-one |
| 204 | 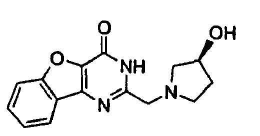 | 2-{[(3S)-3-hydroxypyrrolidin-1-yl]methyl} [1]benzofuro[3, 2-d]pyrimidin-4(3H)-one |
| 206 | 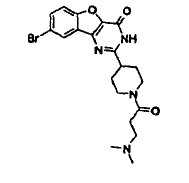 | 8-bromo-2-[1-(N,N-dimethyl-beta-alanyl)piperidin-4-yl][1]benzofuro[3,2-d]pyrimidin-4(3H)-one |
| 207 | 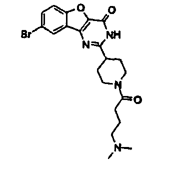 | 8-bromo-2-{1-[4-(dimethylamino)butanoyl] piperidin-4-yl}[1]benzofuro[3,2-d]pyrimidin-4(3H)-one |
| 208 | 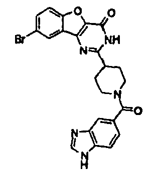 | 2-[1-(1H-benzimidazol-5-ylcarbonyl)piperidin-4-yl]-8-bromo[1]benzofuro[3,2-d]pyrimidin-4(3H)-one |
| 209 | 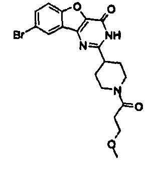 | 8-bromo-2-{1-[3-(methytoxy)propanoyl]pip eridin-4-yl}[1]benzofuro[3,2-d)pyrimidin-4(3H)-one |
| 210 | 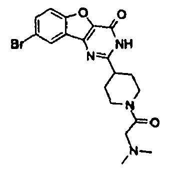 | 8-bromo-2-[1-(N,N-dimethylglycyl)piperidin-4-yl][1]benzofuro[3,2-d]pyrimidin-4(3H)-one |
| 212 | 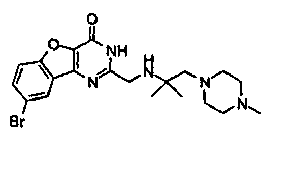 | 8-bromo-2-({[1,1-dimethyl-2-(4-methylpiperazin-1-yl)ethyl]amino}methyl)[1] benzofuro[3,2-d]pyrimidin-4(3H)-one |
| 213 | 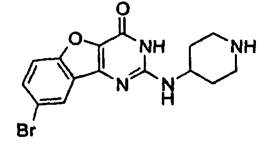 | 8-bromo-2-(piperidin-4-ylamino)[1]benzofuro[3,2-d]pyrimidin-4(3H)-one |
| 214 | 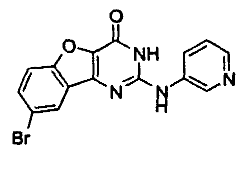 | 8-bromo-2-(pyridin-3-ylamino)[1]benzofuro[3,2-d]pyrimidin-4(3H)-one |
| 215 | 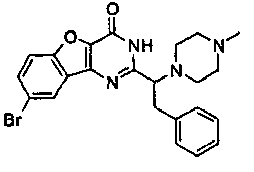 | 8-bromo-2-[1-(4-methylpiperazin-1-yl)-2-phenylethyl][1]benzofuro[ 3,2-d]pyrimidin-4(3H)-one |
| 218 | 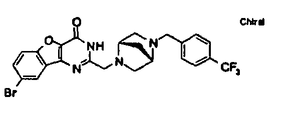 | 8-bromo-2-{[(1S,4S)-5-{[4-(trifluoromethyl)phenyl]m ethyl}-2,5-diazabicyclo[2.2.1]hept-2-yl]methyl} [1]benzofuro[3, 2-d]pyrimidin-4(3H)-one |
| 221 | 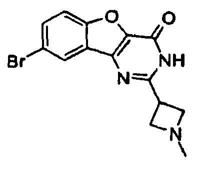 | 8-bromo-2-(1-methylazetidin-3-yl)[1]benzofuro[3,2-d]pyrimidin4(3H)-one |
| 224 | 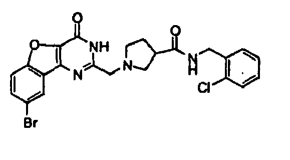 | 1-[(8-bromo-4-oxo-3,4-dihydro[1]benzofuro[3,2-d]pyrimidin-2-yl)methyl]-N-[(2-chlorophenyl)methyl]pyrr olidine-3-carboxamide |
| 225 | 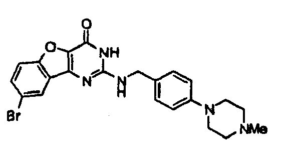 | 8-bromo-2-({[4-(4-methylpiperazin-1-yl)phenyl]methyl}amino)[ 1]benzofuro[3,2-d]pyrimidin-4(3H)-one |
| 226 | 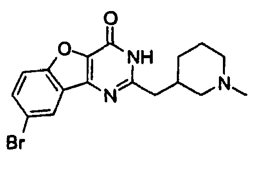 | 8-bromo-2-[(1-methylpiperidin-3-yl)methyl][1]benzofuro[3, 2-d]pyrimidin4(3H)-one |
| 227 | 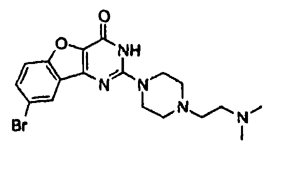 | 8-bromo-2-{4-[2-(dimethylamino)ethyl]pipe razin-1-yl}[1]benzofuro[3,2-d]pyrimidin-4(3H)-one |
| 228 | 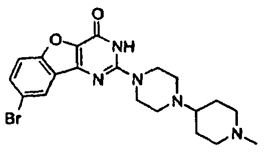 | 8-bromo-2-[4-(1-methylpiperidin-4-yl)piperazin-1-yl][1]benzofuro[3,2-d]pyrimidin-4(3H)-one |
| 229 | 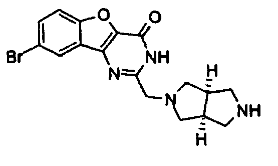 | 8-bromo-2-[(3aR,6aS)-hexahydropyrrolo[3,4-c]pyrrol-2(1H)-ylmethyl][1]benzofuro[3,2 -d]pyrimidin-4(3H)-one |
| 230 | 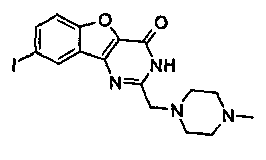 | 8-iodo-2-[(4-methylpiperazin-1-yl)methyl][1]benzofuro[3, 2-d]pyrimidin-4(3H)-one |
| 232 | 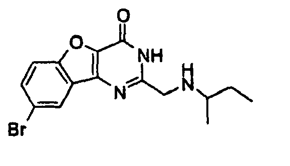 | 8-bromo-2-{[(1-methylpropyl)amino]meth yl[1]benzofuro[3,2-d]pyrimidin-4(3H)-one |
| 233 | 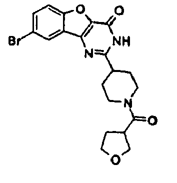 | 8-bromo-2-[1-(tetrahydrofuran-3-ylcarbonyl)piperidin-4-yl][1]benzofuro[3,2-d]pyrimidin-4(3H)-one |
| 234 | 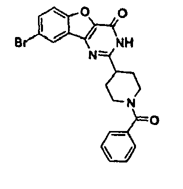 | 8-bromo-2-[1-(phenylcarbonyl)piperidin -4-yl][1]benzofuro[3,2-d]pyrimidin4(3H)-one |
| 235 | 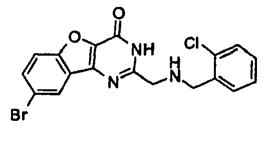 | 8-bromo-2-({[(2-chlorophenyl)methyl]amin o}methyl)[1]benzofuro[3, 2-d]pyrimidin4(3H)-one |
| 236 | 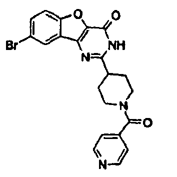 | 8-bromo-2-[1]-(pyridin-4-ylcarbonyl)piperidin-4-yl][1]benzofuro[3,2-d]pyrimidin4(3H)-one |
| 237 | 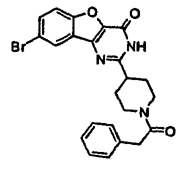 | 8-bromo-2-[1-(phenylacetyl)piperidin-4-yl][1]benzofuro[3,2-d]pyrimidin4(3H)-one |
| 238 | 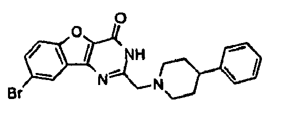 | 8-bromo-2-[(4-phenylpiperidin-1-yl)methyl][1]benzofuro[3, 2-d]pyrimidin-4(3H)-one |
| 239 | 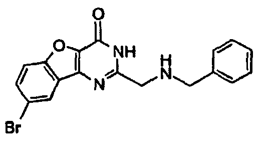 | 8-bromo-2-{[(phenylmethyl)amino]m ethyl}[1]benzofuro[3,2-d]pyrimidin-4(3H)-one |
| 240 | 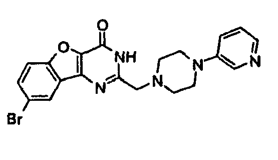 | 8-bromo-2-[(4-pyridin-3-ylpiperazin-1-yl)methyl][1]benzofuro[3, 2-d]pyrimidin-4(3H)-one |
| 242 | 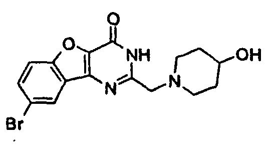 | 8-bromo-2-[(4-hydroxypiperidin-1-yl)methyl][1]benzofuro[3, 2-d]pyrimidin-4(3H)-one |
| 244 | 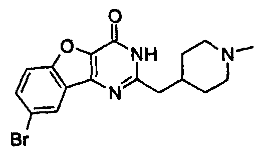 | 8-bromo-2-[(1-methylpiperidin-4-ylmethyl][1]benzofuro[3, 2-d]pyrimidin-4(3H)-one |
| 245 | 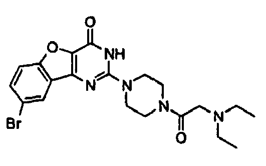 | 8-bromo-2-[4-(N,N-diethylglycyl)piperazin-1-yl][1]benzofuro[3,2-d]pyrimidin-4(3H)-one |
| 246 | 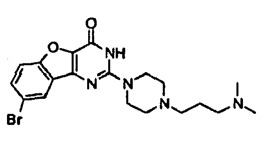 | 8-bromo-2-{4-[3-(dimethylamino)propyl]pi perazin-1-yl}[1]benzofuro[3,2-d]pyrimidin-4(3H)-one |
| 247 | 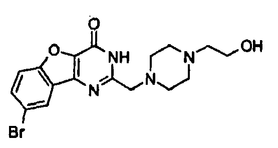 | 8-bromo-2-{[4-(2-hydroxyethyl)piperazin-1-yl]methyl} [1]benzofuro[3, 2-d]pyrimidin-4(3H)-one |
| 252 | 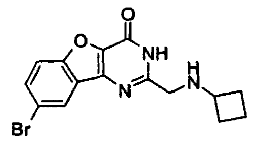 | 8-bromo-2-[(cyclobutylamino)methyl ][1]benzofuro[3,2-d]pyrimidin-4(3H)-one |
| 253 | 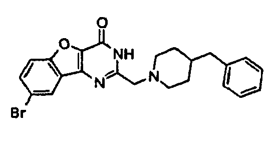 | 8-bromo-2-{[4-(phenylmethyl)piperidin-1-yl]methyl}[1]benzofuro[3, 2-d]pyrimidin-4(3H)-one |
| 254 | 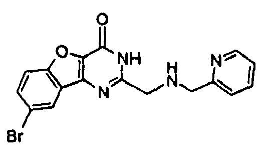 | 8-bromo-2-{[(pyridin-2-ylmethyl)amino]methyl} [ 1]benzofuro[3,2-d]pyrimidin-4(3H)-one |
| 255 | 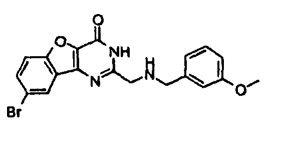 | 8-bromo-2-[({[3-(methyloxy)phenyl]methyl }amino)methyl][1]benzof uro[3,2-d]pyrimidin-4(3H)-one |
| 256 | 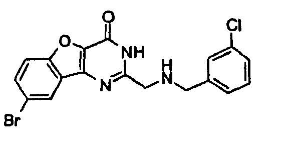 | 8-bromo-2-({[(3-chlorophenyl)methyl]amin o}methyl)[1]benzofuro[3, 2-d]pyrimidin-4(3H)-one |
| 257 | 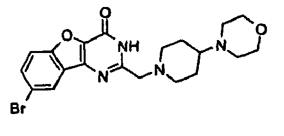 | 8-bromo-2-[(4-morpholin-4-ylpiperidin-1-yl)methyl][1]benzofuro[3, 2-d]pyrimidin-4(3H)-one |
| 258 | 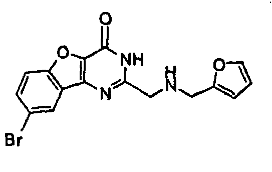 | 8-bromo-2-{[(furan-2-ylmethyl)amino]methyl}[ 1]benzofuro[3,2-d]pyrimidin-4(3H)-one |
| 259 | 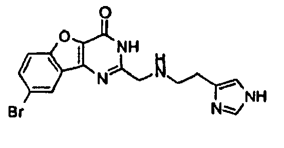 | 8-bromo-2-({[2-(1H-imidazol-4-yl)ethyl]amino}methyl)[1] benzofuro[3,2-d]pyrimidin-4(3H)-one |
| 260 | 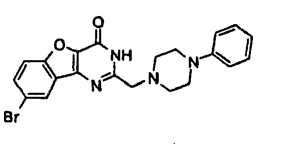 | 8-bromo-2-[(4-phenylpiperazin-1-yl)methyl][1]benzofuro[3, 2-d]pyrimidin-4(3H)-one |
| 261 | 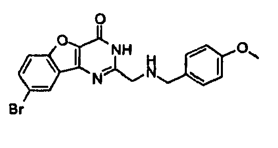 | 8-bromo-2-[({[4-(methyloxy)phenyl]methyl }amino)methyl][1]benzof uro[3,2-d]pyrimidin-4(3H)-one |
| 262 | 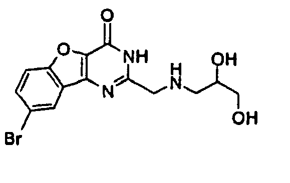 | 8-bromo-2-{[(2,3-dihydroxypropyl)amino]m ethyl}[1]benzofuro[3,2-d]pyrimidin-4(3H)-one |
| 264 | 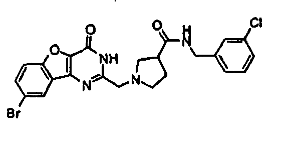 | 1-[(8-bromo-4-oxo-3,4-dihydro[1]benzofuro[3,2-d]pyrimidin-2-yl)methyl]-N-[(3-chlorophenyl)methyl]pyrr olidine-3-carboxamide |
| 266 | 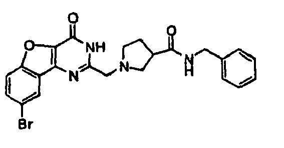 | 1-[(8-bromo-4-oxo-3,4-dihydro[1]benzofuro[3,2- d]pyrimidin-2-yl)methyl]-N-(phenylmethyl)pyrrolidine -3-carboxamide |
| 267 | 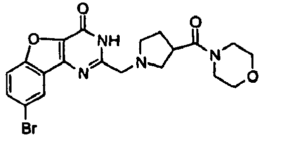 | 8-bromo-2-{[3-(morpholin-4-ylcarbonyl)pyrrolidin-1-yl]methyl}[1]benzofuro[3, 2-d]pyrimidin-4(3H)-one |
| 268 | 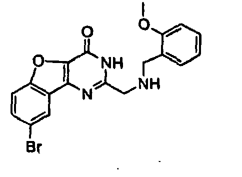 | 8-bromo-2-[({[2-(methyloxy)phenyl]methyl }amino)methyl][1]benzof uro[3,2-d]pyrimidin-4(3H)-one |
| 269 | 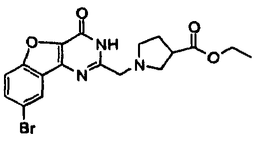 | ethyl 1-[(8-bromo-4-oxo-3,4-dihydro[1]benzofuro[3,2-d]pyrimidin-2-yl)methyl]pyrrolidine-3-carboxylate |
| 270 | 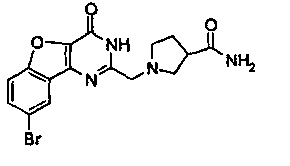 | 1-[(8-bromo-4-oxo-3,4-dihydro[1]benzofuro[3,2-d]pyrimidin-2-yl)methyl]pyrrolidine-3-carboxamide |
| 271 | 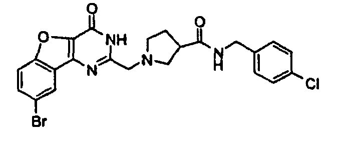 | 1-[(8-bromo-4-oxo-3,4-dihydro[1]benzofuro[3,2-d]pyrimidin-2-yl)methyl]-N-[(4-chlorophenyl)methyl]pyrr olidine-3-carboxamide |
| 272 | 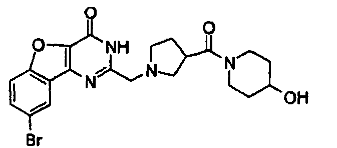 | 8-bromo-2-({3-[(4-hydroxypiperidin-1-ylcarbonyl]pyrrolidin-1-yl}methyl)[1]benzofuro[3, 2-d]pyrimidin-4(3H)-one |
| 273 | 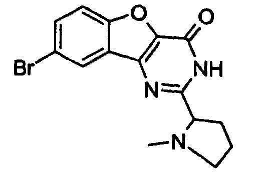 | 8-bromo-2-(1-methylpyrrolidin-2-yl)[1]benzofuro[3,2-d]pyrimidin-4(3H)-one |
| 277 | 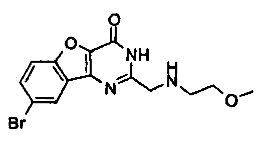 | 8-bromo-2-({[2-(methyloxy)ethyl]amino} methyl)[1]benzofuro[3,2-d]pyrimidin-4(3H)-one |
| 278 | 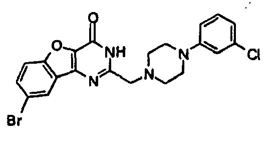 | 8-bromo-2-{[4-(3-chlorophenyl)piperazin-1-yl]methyl}[1]benzofuro[3, 2-d]pyrimidin4(3H)-one |
| 286 | 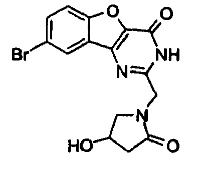 | 8-bromo-2-[(4-hydroxy-2-oxopyrrolidin-1-yl)methyl][1]benzofuro[3, 2-d]pyrimidin-4(3H)-one |
| 287 | 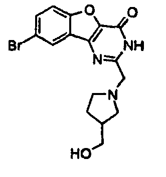 | 8-bromo-2-{[3-(hydroxymethyl)pyrrolidin -1-yl]methyl}[1]benzofuro[3, 2-d]pyrimidin-4(3H)-one |
| 288 | 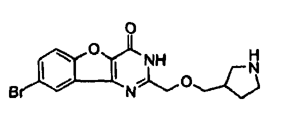 | 8-bromo-2-{[(pyrrolidin-3-ylmethyl)oxy]methyl}[1]b enzofuro[3,2d]pyrimidin-4(3 H)-one |
| 289 | 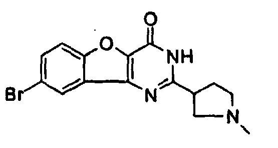 | 8-bromo-2-(1-methylpyrrolidin-3-yl)[1]benzofuro[3,2-d]pyrimidin-4(3H)-one |
| 290 | 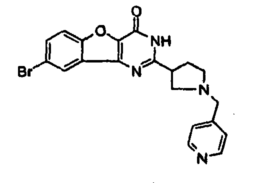 | 8-bromo-2-[1-(pyridin-4-ylmethyl)pyrro lidin-3-yl][1]benzofuro[3,2-d]pyrimidin4(3H)-one |
| 295 | 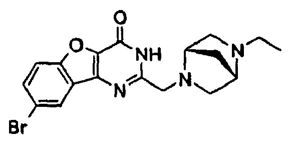 | 8-bromo-2-{[(1S,4S)-5-ethyl-2,5-diazabicyclo[2.2.1]hept-2-yl]methyl}[1]benzofuro[3, 2-d]pyrimidin-4(3H)-one |
| 298 | 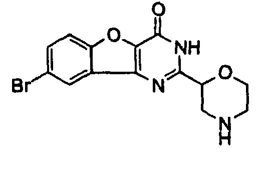 | 8-bromo-2-morpholin-2-yl[1]benzofuro[3,2-d]pyrimidin-4(3H)-one |
| 304 | 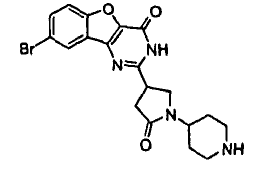 | 8-bromo-2-(5-oxo-1-piperidin-4-ylpyrrolidin-3-yl)[1]benzofuro[3,2-d]pyrimidin4(3H)-one |
| 305 | 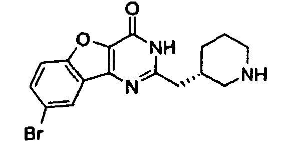 | 8-bromo-2-[(3S)-piperidin-3-ylmethyl][1]benzofuro[3,2 -d]pyrimidin4(3H)-one |
| 306 | 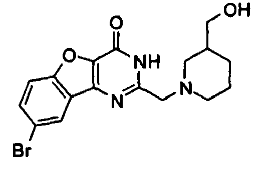 | 8-bromo-2-{[3-(hydroxymethyl)piperidin-1-yl]methyl}[1]benzofuro[3, 2-d]pyrimidin-4(3H)-one |
| 307 | 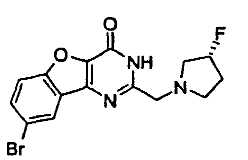 | 8-bromo-2-{[(3R)-3-fluoropyrrolidin-1-yl]methyl}[1]benzofuro[3, 2-d]pyrimidin-4(3H)-one |
| 308 | 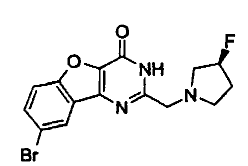 | 8-bromo-2-{[(3S)-3-fluoropyrrolidin-1-yl]methyl}[1]benzofuro[3, 2-d]pyrimidin4(3H)-one |
| 309 | 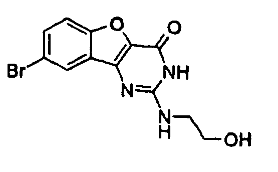 | 8-bromo-2-[(2-hydroxyethyl)amino][1]be nzofuro[3,2-d]pyrimidin-4(3H)-one |
| 311 | 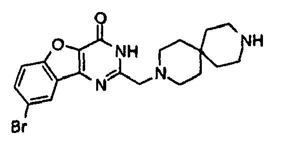 | 8-bromo-2-(3,9-diazaspiro[5.5]undec-3-ylmethyl)[1]benzofuro[3,2 -d]pyrimidin-4(3H)-one |
| 315 | 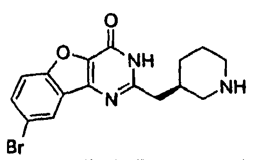 | 8-bromo-2-[(3R)-piperidin-3-ylmethyl][1]benzofuro[3,2 -d]pyrimidin-4(3H)-one |
| 317 | 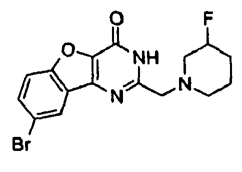 | 8-bromo-2-[(3-fluoropiperidin-1-yl)methyl][1]benzofuro[3, 2-d]pyrimidin-4(3H)-one |
| 318 | 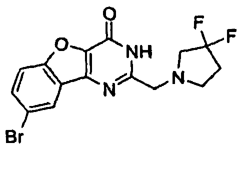 | 8-bromo-2-[(3,3-difluoropyrrolidin-1-yl)methyl][1]benzofuro[3, 2-d]pyrimidin-4(3H)-one |
| 330 | 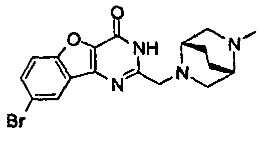 | 8-bromo-2-{[(1S,4S)-5-methyl-2,5-diazabicyclo[2.2.2]oct-2-yl]methyl}[1]benzofuro[3, 2-d]pyrimidin-4(3H)-one |
| 331 | 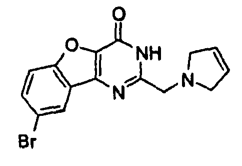 | 8-bromo-2-((2,5-dihydro-1H-pyrrol-1-yl)methyl)benzofuro[3,2-d]pyrimidin-4(3H)-one |
| 332 | 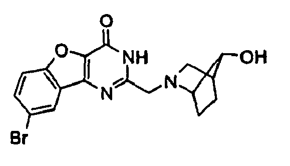 | 8-bromo-2-((7-hydroxy-2-azabicyclo[2.2.1]heptan-2-yl)methyl)benzofuro[3,2-d]pyrimidin-4(3H)-one |
| 344 | 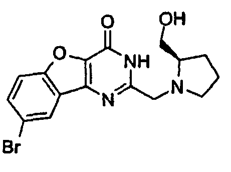 | 8-bromo-2-{[(2R)-2-(hydroxymethyl)pyrrolidin -1-yl]methyl}[1]benzofuro[3, 2-d]pyrimidin4(3H)-one |
| 346 | 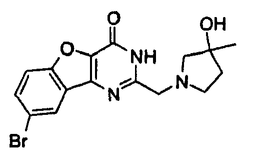 | 8-bromo-2-[(3-hydroxy-3-methylpyrrolidin-1-yl)methyl][1]benzofuro[3, 2-d]pyrimidin-4(3H)-one |
| 366 | 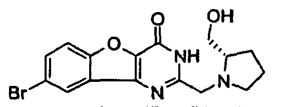 | 8-bromo-2-{[(2S)-2-(hydroxymethyl)pyrrolidin -1-yl]methyl}[1]benzofuro[3, 2-d]pyrimidin4(3H)-one |
| 367 | 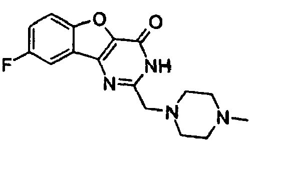 | 8-fluoro-2-[(4-methylpiperazin-1-yl)methyl][1]benzofuro[3, 2-d]pyrimidin-4(3H)-one |
| 368 | 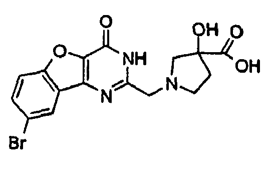 | 1-[(8-bromo-4-oxo-3,4-dihydro[1]benzofuro[3,2-d]pyrimidin-2-yl)methyl]-3-hydroxypyrrolidine-3-carboxylic acid |
| 369 | 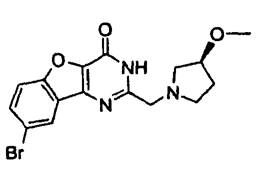 | 8-bromo-2-{[(3S)-3-(methyloxy)pyrrolidin-1-yl]methyl}[1]benzofuro[3, 2-d]pyrimidin-4(3H)-one |
| 370 | 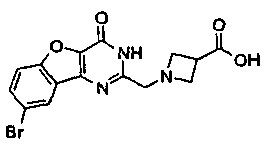 | 1-[(8-bromo-4-oxo-3,4-dihydro[1]benzofuro[3,2-d]pyrimidin-2-yl)methyl]azetidine-3-carboxylic acid |
| 378 | 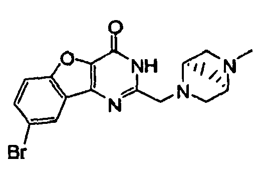 | 8-bromo-2-(((1R,4R)-5-methyl-2,5-diazabicyclo[2.2.1]heptan-2-yl)methyl)benzofuro[3,2-d]pyrimidin-4(3H)-one |
| 388 | 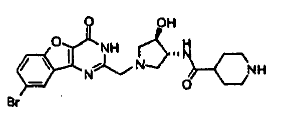 | N-{(3R,4R)-1-[(8-bromo-4-oxo-3,4-dihydro[1]benzofuro[3,2-d]pyrimidin-2-yl)methyl]-4-hydroxypyrrolidin-3-yl}piperidine-4-carboxamide |
| 398 | 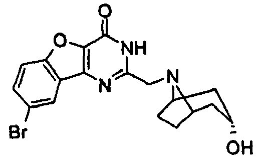 | 8-bromo-2-[(3-hydroxy-8-azabicyclo[3.2.1]oct-8-yl)methyl][1]benzofuro[3, 2-d]pyrimidin-4(3H)-one |
| 400 | 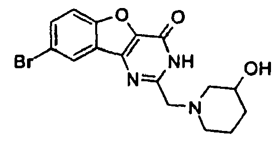 | 8-bromo-2-[(3-hydroxypiperidin-1-yl)methyl][1]benzofuro[3, 2-d]pyrimidin4(3H)-one |
| 401 | 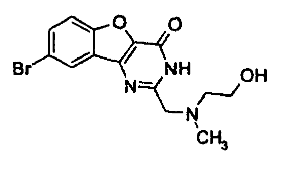 | 8-bromo-2-{[(2-hydroxyethyl)(methyl)ami no]methyl}[1]benzofuro[3 ,2-d]pyrimidin-4(3H)-one |
| 402 | 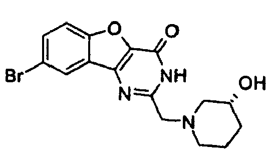 | 8-bromo-2-{[(3R)-3-hydroxypiperidin-1-yl]methyl}[1]benzofuro[3, 2-d]pyrimidin-4(3H)-one |
| 403 | 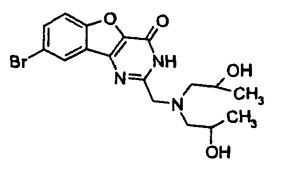 | 2-{[bis(2-hydroxypropyl)amino]met hyl}-8-bromo[1]benzofuro[3,2-d]pyrimidin-4(3H)-one |
| 404 | 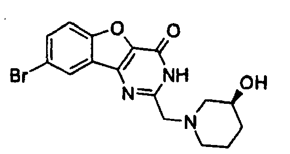 | 8-bromo-2-{[(3S)-3-hydroxypiperidin-1-yl]methyl} [1]benzofuro[3, 2-d]pyrimidin-4(3H)-one |
| 407 | 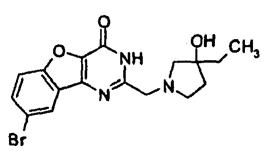 | 8-bromo-2-[(3-ethyl-3-hydroxypyrrolidin-1-yl)methyl][1]benzofuro[3, 2-d]pyrimidin-4(3H)-one |
| 408 | 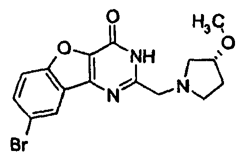 | 8-bromo-2-{[(3R)-3-(methyloxy)pyrrolidin-1-yl]methyl}[1]benzofuro[3, 2-d]pyrimidin4(3H)-one |
| 409 | 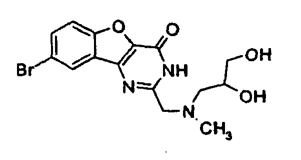 | 8-bromo-2-{[(2,3-dihydroxypropyl)(methyl) amino]methyl} [1]benzofu ro[3,2-d]pyrimidin-4(3H)-one |
| 410 | 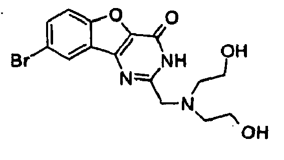 | 2-{[bis(2-hydroxyethyl)amino]meth yl}-8-bromo[1]benzofuro[3,2-d]pyrimidin-4(3H)-one |
| 412 | 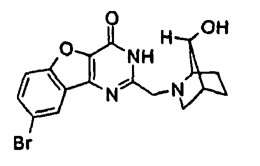 | 8-bromo-2-[(7-hydroxy-2-azabicyclo[2.2.1]hept-2-yl)methyl][1]benzofuro[3, 2-d]pyrimidin-4(3H)-one |
| 413 | 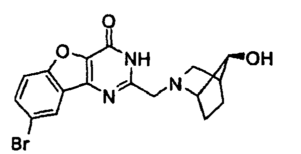 | 8-bromo-2-{[(7S)-7-hydroxy-2-azabicyclo[2.2.1]hept-2-yl]methyl}[1]benzofuro[3, 2-d]pyrimidin-4(3H)-one |
| 417 | 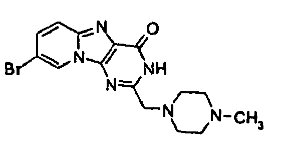 | 8-bromo-2-[(4-methylpiperazin-1-yl)methyl]pyrido[1,2-e]purin-4(3H)-one |
| 418 | 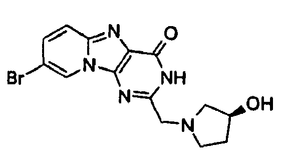 | 8-bromo-2-{[(3S)-3-hydroxypyrrolidin-1-yl]methyl}pyrido[1,2-e]purin-4(3H)-one |
| 420 | 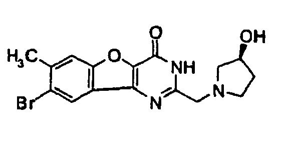 | 8-bromo-2-{[(3S)-3-hydroxypyrrolidin-1-yl]methyl}-7-methyl[1]benzofuro[3,2-d]pyrimidin-4(3H)-one |
| 424 | 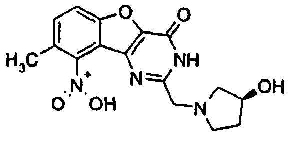 | hydroxy(2-{[(3S)-3-hydroxypyrrolidin-1-yl]methyl}-8-methyl-4-oxo-3,4-dihydro[1]benzofuro[3,2-d]pyrimidin-9-yl)oxoammonium |
| 425 | 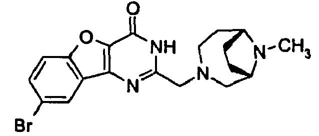 | 8-bromo-2-{[(1S,6R)-9-methyl-3,9-diazabicyclo[4.2.1]non-3-yl]methyl}[1]benzofuro[3, 2-d]pyrimidin-4(3 H)-one |
| 426 | 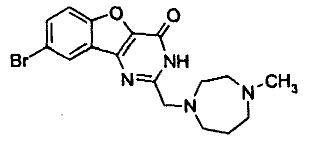 | 8-bromo-2-[(4-methyl-1,4-diazepan-1-yl)methyl][1]benzofuro[3, 2-d]pyrimidin-4(3H)-one |
| 428 | 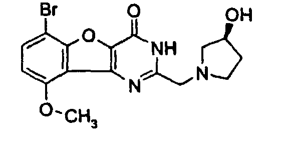 | 6-bromo-2-{[(3S)-3-hydroxypyrrolidin-1-yl]methyl}-9-(methyloxy)[1]benzofuro[ 3,2-d]pyrimidin-4(3H)-one |
| 429 | 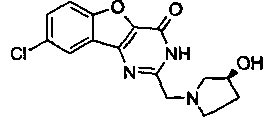 | 8-chloro-2-{[(3S)-3-hydroxypyrrolidin-1-yl]methyl}[1]benzofuro[3, 2-d]pyrimidin-4(3H)-one |
| 431 | 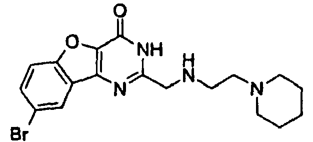 | 8-bromo-2-{[(2-piperidin-1-ylethyl)amino]methyl}[1] benzofuro[3,2-d]pyrimidin-4(3H)-one |
| 432 | 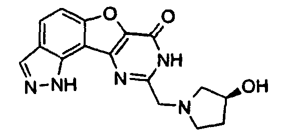 | 9-{[(3S)-3-hydroxypyrrolidin-1- yl]methyl}-1H-pyrimido[4',5':4,5]furo[2,3 -g]indazol-7(8H)-one |
| 433 | 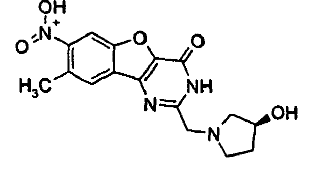 | hydroxy(2-{[(3S)-3-hydroxypyrrolidin-1-yl]methyl}-8-methyl-4-oxo-3,4-dihydro[1]benzofuro[3,2-d]pyrimidin-7-yl)oxoammonium |
| 434 | 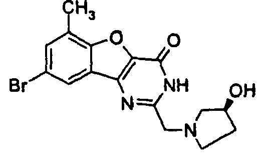 | 8-bromo-2-{[(3S)-3-hydroxypyrrolidin-1-yl]methyl}-6-methyl[1]benzofuro[3,2-d]pyrimidin-4(3H)-one |
| 436 | 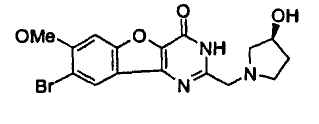 | 8-bromo-2-{[(3S)-3-hydroxypyrolidin-1-yl]methyl}-7-(methyloxy)[1]benzofuro[ 3,2-d]pyrimidin-4(3H)-one |
| 437 | 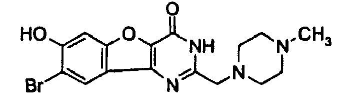 | 8-bromo-7-hydroxy-2-{[(3S)-3-hydroxypyrrolidin-1-yl]methyl}[1]benzofuro[3, 2-d]pyrimidin-4(3H)-one |
| 438 | 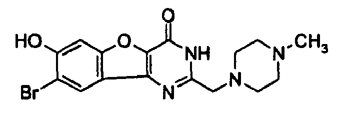 | 8-bromo-7-hydroxy-2-[(4-methylpiperazin-1-yl)methyl][1]benzofuro[3, 2-d]pyrimidin-4(3H)-one |
| 440 | 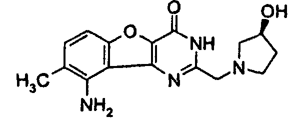 | 9-amino-2-{[(3S)-3-hydroxypyrrolidin-1-yl]methyl}-8-methyl[1]benzofuro[3,2-d)pyrimidin-4(3H-one |
| 442 | 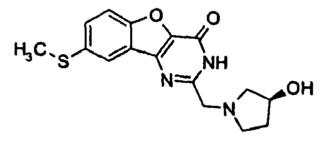 | 2-{[(3S)-3-hydroxypyrrolidin-1-yl]methyl}-8-(methylthio)[1]benzofuro[ 3,2-d]pyrimidin-4(3H)-one |
| 443 | 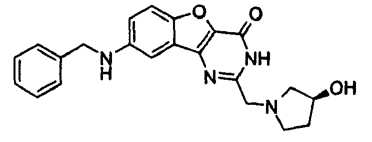 | 2-{[(3S)-3-hydroxypyrrolidin-1-yl]methyl}-8-[(phenylmethyl)amino][1] benzofuro[3,2-d]pyrimidin-4(3H)-one |
| 444 | 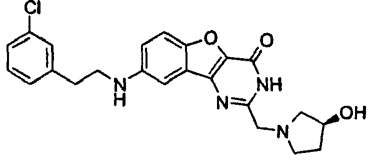 | 8-{[2-(3-chlorophenyl)ethyl]amino }-2-{[(3S)-3-hydroxypyrrolidin-1-yl]methyl}[1]benzofuro[3, 2-d]pyrimidin-4(3H)-one |
| 445 | 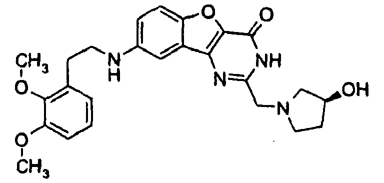 | 8-({2-[2,3-bis(methyloxy)phenyl]ethyl}amino)-2-{[(3S)-3-hydroxypyrrolidin-1-yl]methyl}[1]benzofuro[3, 2-d]pyrimidin-4(3H)-one |
| 446 | 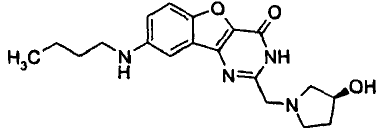 | 8-(butylamino)-2-{[(3S)-3-hydroxypyrrolidin-1-yl]methyl}[1]benzofuro[3, 2-d]pyrimidin-4(3H)-one |
| 447 | 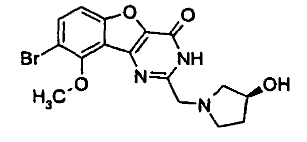 | 8-bromo-2-{[(3S)-3-hydroxypyrrolidin-1-yl]methyl}-9-(methyloxy)[1]benzofuro[ 3,2-d]pyrimidin-4(3H)-one |
| 448 | 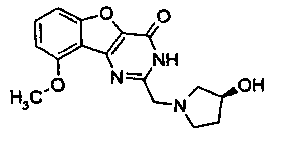 | 2-{[(3S)-3-hydroxypyrrolidin-1-yl]methyl}-9-(methyloxy)[1]benzofuro[ 3,2-d]pyrimidin-4(3H)-one |
| 449 | 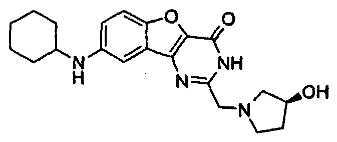 | 8-(cyclohexylamino)-2-{[(3S)-3-hydroxypyrrolidin-1-yl]methyl} [1]benzofuro[3, 2-d]pyrimidin-4(3H)-one |
| 450 | 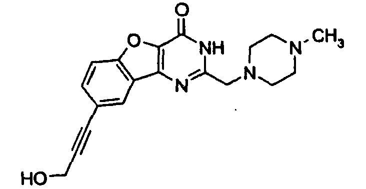 | 8-(3-hydroxyprop-1-yn-1-yl)-2-[(4-methylpiperazin-1-yl)methyl][1]benzofuro[3, 2-d]pyrimidin-4(3H)-one |
| 451 | 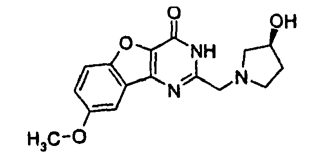 | 2-{[(3S)-3-hydroxypyrrolidin-1-yl]methyl}-8-(methyloxy)[1]benzofuro[ 3,2-d]pyrimidin-4(3 H)-one |
| 452 | 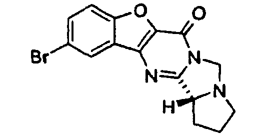 | (13bR)-11-bromo-1,2,3,13b-tetrahydro-7H-[1]benzofuro[3,2]-d]pyrrolo[1',2':3,4]imidaz o[1,5-a]pyrimidin-7-one |
| 453 | 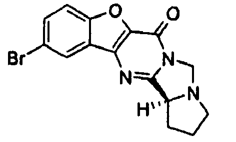 | (13bS)-11-bromo-1,2,3,13b-tetrahydro-7H-[1]benzofuro[3,2-d]pyrrolo[1',2':3,4]imidaz o[1,5-a]pyrimidin-7-one |
| 454 | 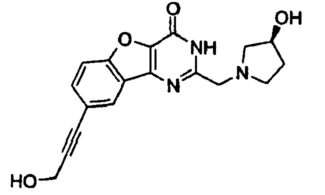 | 8-(3-hydroxyprop-1-yn-1-yl)-2-{[(3S)-3-hydroxypyrrolidin-1-yl]methyl}[1]benzofuro[3, 2-d]pyrimidin-4(3H)-one |
| 455 | 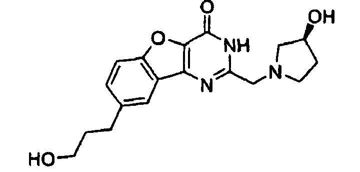 | 8-(3-hydroxypropyl)-2-{[(3S)-3-hydroxypyrrolidin-1-yl]methyl}[1]benzofuro[3, 2-d]pyrimidin-4(3H)-one |
| 456 | 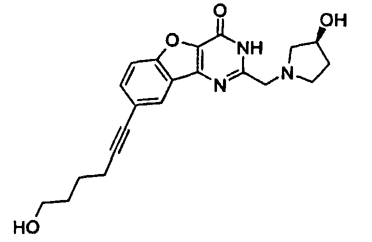 | 8-(6-hydroxyhex-1-yn-1-yl)-2-{[(3S)-3-hydroxypyrrolidin-1-yl]methyl}[1]benzofuro[3, 2-d]pyrimidin-4(3H)-one |
| 457 | 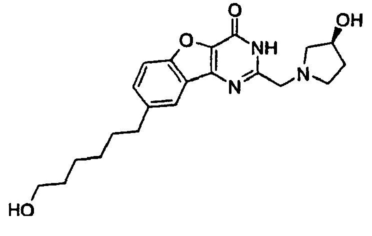 | 8-(6-hydroxyhexyl)-2-{[(3S)-3-hydroxypyrrolidin-1-yl]methyl}[1]benzofuro[3, 2-d]pyrimidin-4(3H)-one |
| 458 | 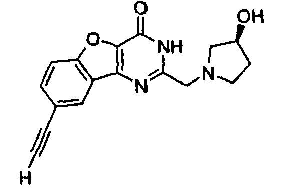 | 8-ethynyl-2-{[(3S)-3-hydroxypyrrolidin-1-yl]methyl}[1]benzofuro[3, 2-d]pyrimidin-4(3H)-one |
| 459 | 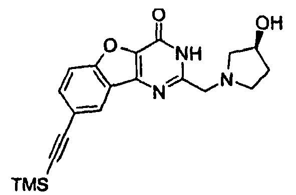 | 2-{[(3S)-3-hydroxypyrrolidin-1-yl]methyl}-8-[(trimethylsilyl)ethynyl][1 ]benzofuro[3,2-d]pyrimidin-4(3H)-one |
| 460 | 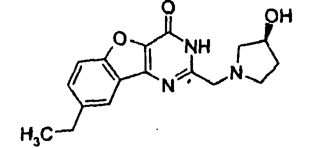 | 8-ethyl-2-{[(3S)-3-hydroxypyrrolidin-1-yl]methyl}[1]benzofuro[3, 2-d]pyrimidin-4(3H)-one |
| 461 | 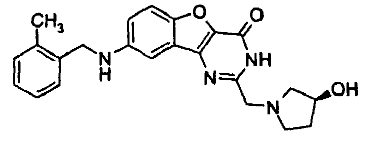 | 2-{[(3S}-3-hydroxypyrrolidin-1-yl]methyl}-8-{[(2-methylphenyl)methyl]ami no}[1]benzofuro[3,2-d]pyrimidin-4(3H)-one |
| 462 | 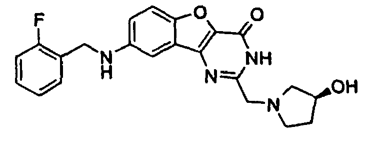 | 8-{[(2-fluorophenyl)methyl]amin o}-2-{[(3S)-3-hydroxypyrrolidin-1-yl]methyl}[1]benzofuro[3, 2-d]pyrimidin-4(3H)-one |
| 463 | 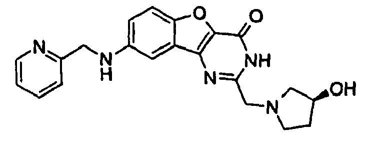 | 2-{[(3S}-3-hydroxypyrrolidin-1-yl]methyl}-8-[(pyridin-2-ylmethyl)amino[1]benzof uro[3,2-d]pyrimidin-4(3H)-one |
| 464 | 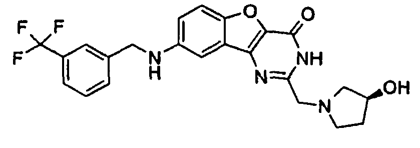 | 2-{[(3S)-3-hydroxypyrrolidin-1-yl]methyl}-8-({[3-(trifluoromethyl)phenyl]m ethyl}amino)[1]benzofuro [3,2-d]pyrimidin-4-(3H)-one |
| 465 | 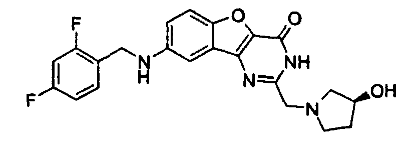 | 8-{[(2,4-difluorophenyl)methyl]am ino}-2-{[(3S)-3-hydroxypyrrolidin-1-yl]methyl}[1]benzofuro[3, 2-d]pyrimidin-4(3H)-one |
| 466 | 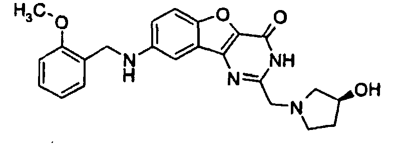 | 2-{[(3S)-3-hydroxypyrrolidin-1-yl]methyl}-8-({[2-(methyloxy)phenyl]methyl }amino)[1]benzofuro[3,2-d]pyrimidin-4(3H)-one |
| 467 | 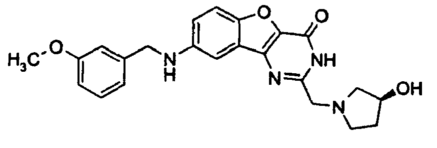 | 2-{[(3S)-3-hydroxypyrrolidin-1-yl]methyl}-8-({[3-(methyloxy)phenyl]methyl }amino)[1]benzofuro[3,2-d]pyrimidin-4(3H)-one |
| 468 | 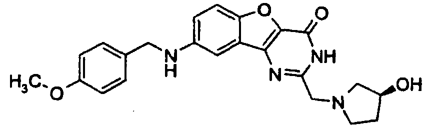 | 2-{[(3S)-3-hydroxypyrrolidin-1-yl]methyl}-8-({[4-(methyloxy)phenyl]methyl }amino)[1]benzofuro[3,2-d]pyrimidin-4(3H)-one |
| 469 | 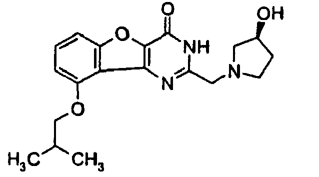 | 2-{[(3S)-3-hydroxypyrrolidin-1-yl]methyl}-9-[(2-methylpropyl)oxy][1]benz ofuro[3,2-d]pyrimidin-4(3 H)-one |
| 470 | 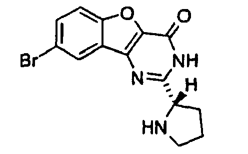 | 8-bromo-2-[(2S)-pyrrolidin-2-yl][1]benzofuro[3,2-d]pyrimidin-4(3H)-one |
| 471 | 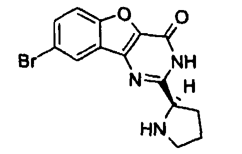 | 8-bromo-2-[(2R)-pyrrolidin-2-yl][1]benzofuro[3,2-d]pyrimidin-4(3H)-one |
| 472 | 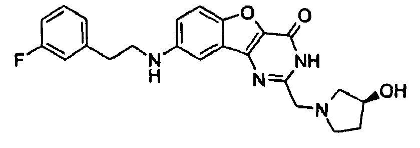 | 8-{[2-(3-fluorophenyl)ethyl]amino }-2-{[(3S)-3-hydroxypyrrolidin-1-yl]methyl}[1]benzofuro[3, 2-d]pyrimidin-4(3H)-one |
| 473 | 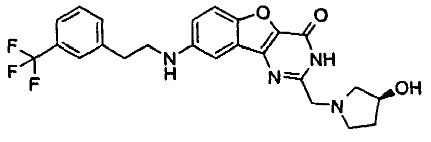 | 2-{[(3S)-3-hydroxypyrrolidin-1-yl]methyl}-8-({2-[3-(trifluoromethyl)phenyl]et hyl}amino)[1]benzofuro[3 ,2-d]pyrimidin-4(3H)-one |
| 474 | 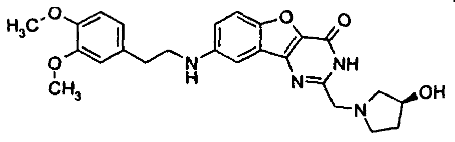 | 8-({2-[3,4-bis(methyloxy)phenyl]ethyl}amino)-2-{[(3S)-3-hydroxypyrrolidin-1-yl]methyl}[1]benzofuro[3, 2-d]pyrimidin-4(3H)-one |
| 475 | 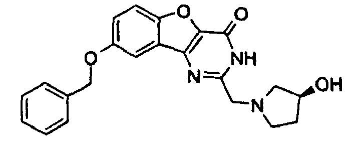 | 2-{[(3S)-3-hydroxypyrrolidin-1-yl]methyl}-8-[(phenylmethyl)oxy][1]be nzofuro[3,2-d]pyrimidin-4(3H)-one |
| 476 | 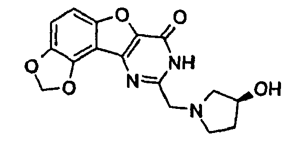 | 9-{[(3S)-3-hydroxypyrrolidin-1-yl]methyl}[1,3]dioxolo[4, 5][1]benzofuro[3,2-d]pyrimidin-7(8H)-one |
| 477 | 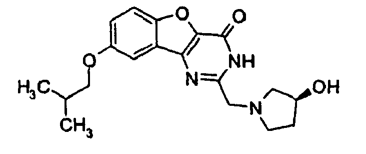 | 2-{[(3S)-3-hydroxypyrrolidin-1-yl]methyl}-8-[(2-methylpropyl)oxy][1]benz ofuro[3,2-d]pyrimidin-4(3H)-one |
| 488 | 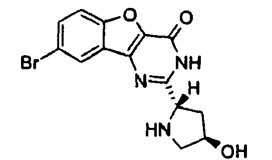 | 8-bromo-2-[(2S,4R)-4-hydroxypyrrolidin-2-yl][1]benzofuro[3,2-d]pyrimidin-4(3H)-one |
| 489 | 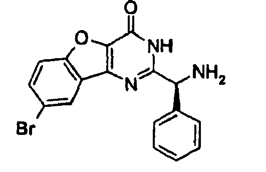 | 2-[(S)-amino(phenyl)methyl]-8-bromo[1]benzofuro[3,2-d]pyrimidin-4(3H-one |
| 490 | 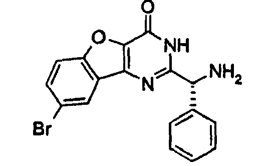 | 2-[(R)-amino(phenyl)methyl]-8-bromo[1]benzofuro[3,2-d]pyrimidin-4(3H)-one |
| 491 | 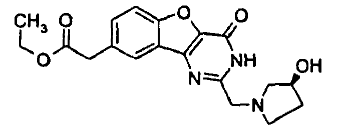 | ethyl (2-{[(3S)-3-hydroxypyrrolidin-1-yl]methyl}-4-oxo-3,4-dihydro[1]benzofuro[3,2-d]pyrimidin-8-yl)acetate |
| 492 | 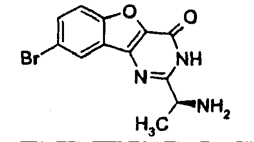 | 2-[(1S)-1-aminoethyl]-8-bromo[1]benzofuro[3,2-d]pyrimidin-4(3H)-one |
| 493 | 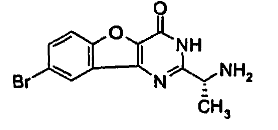 | 2-[(1R)-1-aminoethyl]-8-bromo[1]benzofuro[3,2-d]pyrimidin-4(3H)-one |
| 494 | 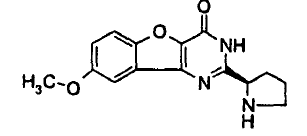 | 8-(methyloxy)-2-[(2R)-pyrrolidin-2-yl][1]benzofuro[3,2-d]pyrimidin-4(3H)-one |
| 495 | 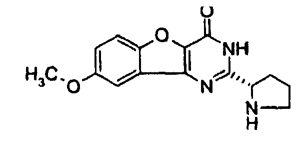 | 8-(methyloxy)-2-[(2S)-pyrrolidin-2-yl][1]benzofuro[3,2-d]pyrimidin-4(3H)-one |
| 496 | 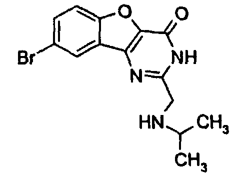 | 8-bromo-2-{[(1-methylethyl)amino]methyl }[1]benzofuro[3,2-d]pyrimidin-4(3H)-one |
| 498 | 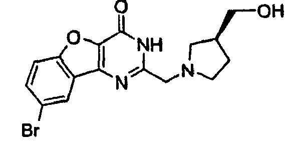 | 8-bromo-2-{[(3S)-3-(hydroxymethyl)pyrrolidin -1-yl]methyl}[1]benzofuro[3, 2-d]pyrimidin-4(3H)-one |
| 499 | 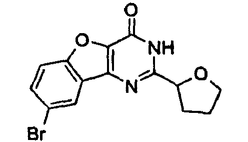 | 8-bromo-2-(tetrahydrofuran-2-yl)[1]benzofuro[3,2-d]pyrimidin-4(3H)-one |
| 500 | 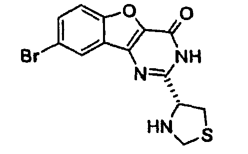 | 8-bromo-2-[(4R)-1,3-thiazolidin-4-yl][1]benzofuro[3,2-d]pyrimidin-4(3H)-one |
| 501 | 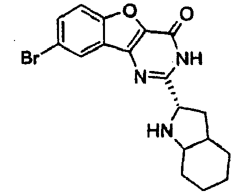 | 8-bromo-2-[(2S)-octahydro-1H-indol-2-yl][1]benzofuro[3,2-d]pyrimidin4(3H)-one |
| 502 | 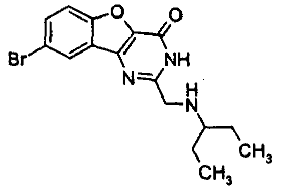 | 8-bromo-2-{[(1-ethylpropyl)amino]methyl }[1]benzofuro[3,2-d]pyrimidin-4(3H)-one |
| 503 | 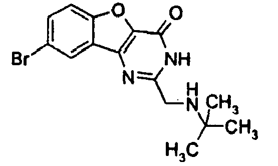 | 8-bromo-2-{[(1,1-dimethylethyl)amino]meth yl}[1]benzofuro[3,2-d]pyrimidin-4(3H)-one |
| 505 | 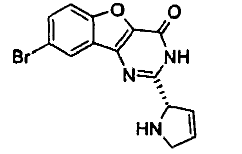 | 8-bromo-2-[(2S)-2,5-dihydro-1H-pyrrol-2-yl][1]benzofuro[3,2-d]pyrimidin-4(3H)-one |
| 506 | 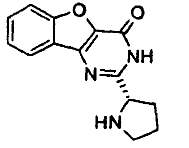 | 2-[(2S)-pyrrolidin-2-yl][1]benzofuro[3,2-d]pyrimidin-4(3H)-one |
| 507 | 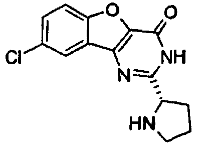 | 8-chloro-2-[(2S)-pyrrolidin-2-yl][1]benzofuro[3,2-d]pyrimidin-4(3H)-one |
| 508 | 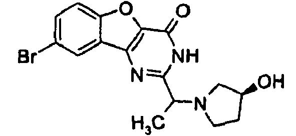 | 8-bromo-2-{1-[(3S)-3-hydroxypyrrolidin-1-yl]ethyl}[1]benzofuro[3,2-d]pyrimidin-4(3H)-one |
| 509 | 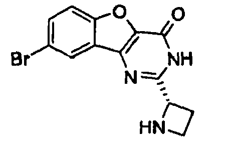 | 2-[(2S)-azetidin-2-yl]-8-bromo[1]benzofuro[3,2-d]pyrimidin-4(3H)-one |
| 511 | 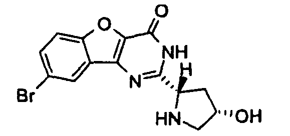 | 8-bromo-2-[(2S,4S)-4-hydroxypyrrolidin-2-yl][1]benzofuro[3,2-d]pyrimidin-4(3H)-one |
| 512 | 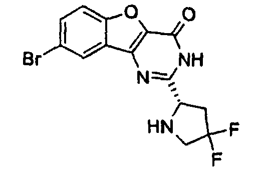 | 8-bromo-2-[(2S)-4,4-difluoropyrrolidin-2-yl][1]benzofuro[3,2-d]pyrimidin-4(3H)-one |
| 513 | 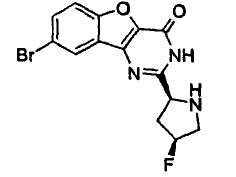 | 8-bromo-2-[(2S,4S)-4-fluoropyrrolidin-2-yl][1]benzofuro[3,2-d]pyrimidin-4(3H)-one |
| 514 | 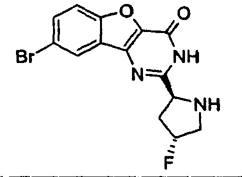 | 8-bromo-2-[(2S,4R)-4-fluoropyrrolidin-2-yl][1]benzofuro[3,2-d]pyrimidin-4(3H)-one |
| 516 | 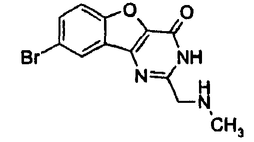 | 8-bromo-2-[(methylamino)methyl][1] benzofuro[3,2-d]pyrimidin-4(3H)-one |
| 517 | 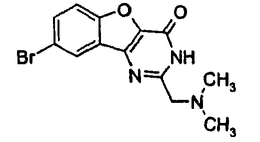 | 8-bromo-2-[(dimethylamino)methyl][ 1]benzofuro(3,2-d]pyrimidin-4(3H)-one |
| 518 | 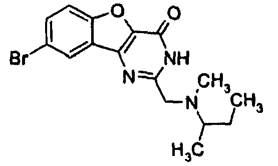 | 8-bromo-2-{[methyl(1-methylpropyl)amino]meth yl}[1]benzofuro[3,2-d]pyrimidin-4(3H)-one |
| 519 | 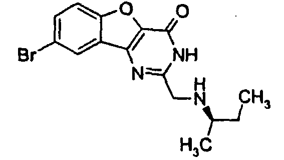 | 8-bromo-2-({[(1R)-1-methylpropyl]amino}meth yl)[1]benzofuro[3,2-d]pyrimidin-4(3H)-one |
| 520 | 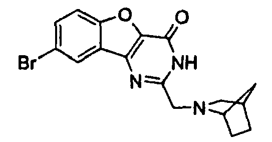 | 2-(2-azabicyclo[2.2.1]hept-2-ylmethyl)-8-bromo[1]benzofuro[3,2-d]pyrimidin-4(3H)-one |
| 521 | 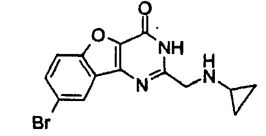 | 8-bromo-2-[(cyclopropylamino)methy 1][1]benzofuro[3,2-d]pyrimidin-4(3H)-one |
| 522 | 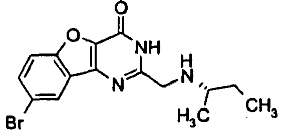 | 8-bromo-2-({[(1S)-1-methylpropyl]amino}meth yl)[1]benzofuro[3,2-d]pyrimidin-4(3H)-one |
| 523 | 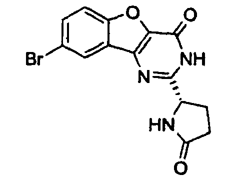 | 8-bromo-2-[(2S)-5-oxopyrrolidin-2-yl][1]benzofuro[3,2-d]pyrimidin-4(3H)-one |
| 524 | 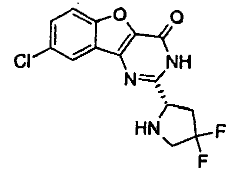 | 8-chloro-2-[(2S)-4,4-diffuoropyrrolidin-2-yl][1]benzofuro[3,2-d]pyrimidin-4(3H)-one |
| 525 | 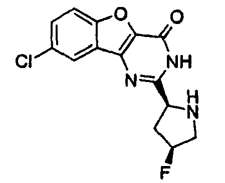 | 8-chloro-2-[(2S,4S)-4-fluoropyrrolidin-2-yl][1]benzofuro[3,2-d]pyrimidin-4(3H)-one |
| 526 | 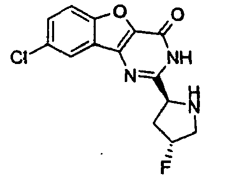 | 8-chloro-2-[(2S,4R)-4-fluoropyrrolidin-2-yl][1]benzofuro[3,2-d]pyrimidin-4(3H)-one |
| 527 | 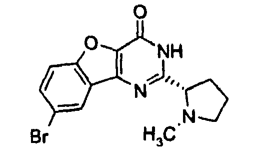 | 8-bromo-2-[(2S)-1-methylpyrrolidin-2-yl][1]benzofuro[3,2-d]pyrimidin-4(3H)-one |
| 528 | 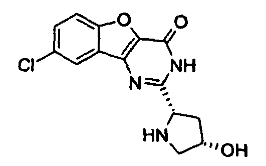 | 8-chloro-2-[(2S,4S)-4-hydroxypyrrolidin-2-yl][1]benzofuro[3,2-d]pyrimidin-4(3H)-one |
| 529 | 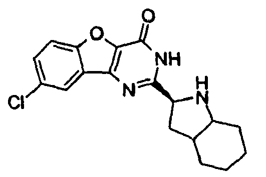 | 8-chloro-2-[(2S)-octahydro-1H-indol-2-yl][1]benzofluro[3,2-d]pyrimidin-4(3H)-one |
| 530 | 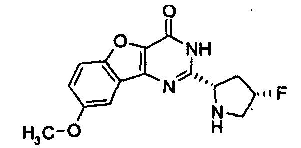 | 2-[(2S,4S)-4-fluoropyrrolidin-2-yl]-8-(methyloxy)[1]benzofuro[ 3,2-d]pyrimidin-4(3H)-one |
| 531 | 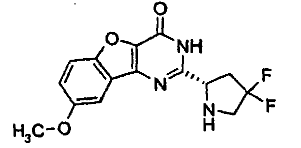 | 2-[(2S)-4,4-difluoropyrrolidin-2-yl]-8-(methyloxy)[1]benzofuro[ 3,2-d]pyrimidin-4(3H)-one |
| 532 | 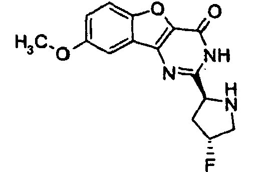 | 2-[(2S,4R)-4-fluoropyrrolidin-2-yl]-8-(methyloxy)[1]benzofuro[ 3,2-d]pyrimidin-4(3H)-one |
| 533 | 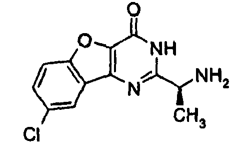 | 2-[(1S)-1-aminoethyl]-8-chloro[1]benzofuro[3,2-d]pyrimidin-4(3H)-one |
| 534 | 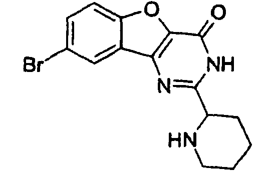 | 8-Bromo-2-piperidin-2-yl[1]benzofuro[3,2-d]pyrimidin-4(3H)-one Hydrochloride |
| 535 | 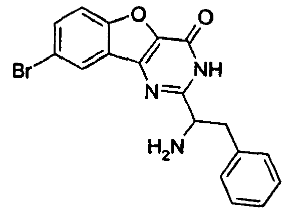 | 2-(1-amino-2-phenylethyl)-8-bromo[1]benzofuro[3,2-d]pyrimidin-4(3H)-one |
| 536 | 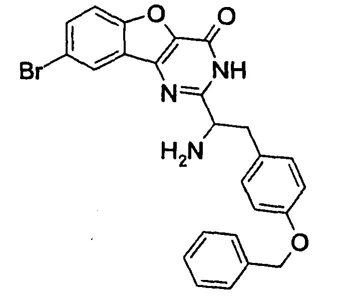 | 2-(1-amino-2-{4-[(phenyhnethyl)oxy]pheny 1}ethyl)-8-bromo[1]benzofuro[3,2-d]pyrimidin-4(3H)-one |
| 537 | 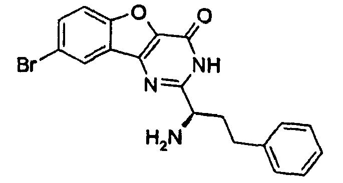 | 2-[(1S)-1-amino-3-phenylpropyl]-8-bromo[1]benzofuro[3,2-d]pyrimidin-4(3H)-one |
| 538 | 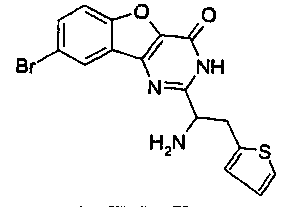 | 2-[1-amino-2-(2-thienyl)ethyl]-8-bromo[1]benzofuro[3,2-d]pyrimidin-4(3H)-one |
| 539 | 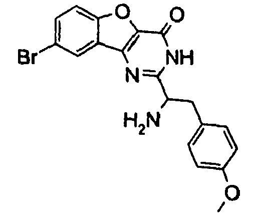 | 2-{1-amino-2-[4-(methyloxy)phenyl]ethyl}-8-bromo[1]benzofuro[3,2-d]pyrimidin-4(3H)-one |
| 540 | 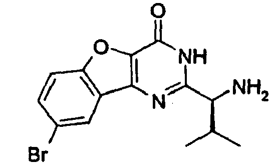 | 2-[(1S)-1-amino-2-methylpropyl]-8-bromo[1]benzofuro[3,2-d]pyrimidin-4(3H)-one |
| 541 | 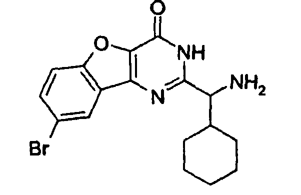 | 2-[amino(cyclohexyl)methyl ]-8-bromo[1]benzofuro[3,2-d]pyrimidin-4(3H)-one |
| 542 | 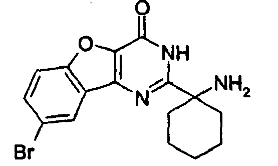 | 2-(1-aminocyclohexyl)-8-bromo[1]benzofuro[3,2-d]pyrimidin-4(3H)-one |
| 543 | 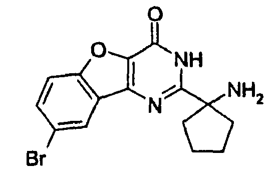 | 2-(1-aminocyclopentyl)-8-bromo[1]benzofuro[3,2-d]pynmidin-4(3H)-one |
| 544 | 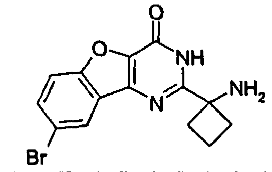 | 2-(1-aminocyclobutyl)-8-bromo[1]benzofuro[3,2-d]pyrimidin-4(3H)-one |

12. The compound of claim 1 which is 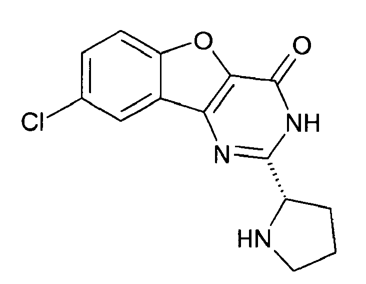 8-chloro-2-[(2S)-pyrrolidin-2-yl][1]benzofuro[3,2-d]pyrimidin-4(3H)-one, or a pharmaceutically acceptable salt thereof.

13. A pharmaceutical composition comprising the compound according to any of claims 1-12, or a pharmaceutically acceptable salt thereof, and a pharmaceutically acceptable carrier, excipient, or diluent.

14. An in vitro method of inhibiting PIM, CDC7 or CK2 in a cell, comprising contacting the cell, in which inhibition of PIM, CDC7 or CK2 is desired, with the compound according to any of claims 1-12, or a pharmaceutically acceptable salt thereof, or the pharmaceutical composition of claim 13.

15. A compound of any of claims 1-12, or a pharmaceutically acceptable salt thereof, or a composition of claim 13 for use in the treatment of a cancer selected from pancreatic cancer, prostate cancer, hepatocellular carcinoma, lymphomas, leukemias, colorectal.

16. The compound or composition according to claim 15, wherein the treatment further comprises one or more therapeutic angents selected from Camptothecin, Topotecan, 9-Nitrocamptothecin, 9-Aminocamptothecin, Karenitecin, Irinotecan, Etoposide, Etoposide Phosphate, Teniposide, Amsacrine, Razoxane, Dexrazoxane, Mechlorethamine, Cyclophosphamide, Ifosfamide, Chlorambucil, Melphalan, Thiotepa, Trenimon, Triethylenemelamine, Dianhydrogalactitol, Dibromodulcitol, Busulfan, dimethylsulfate, Chloroethylnitrosourea, BCNU, CCNU, Methyl-CCNU, Streptozotocin, Chlorozotocin, Prednimustine, Estramustine, Procarbazine, Dacarbazine, Hexamethylmelamine, Pentamethylmelamine, Temozolomide, Cisplatin, Carboplatin, Oxaliplatin, Bleomycin, Dactinomycin, Mithramycin, Rapamycin, Mitomycin C, Daunorubicin, Doxorubicin, Epirubicin, Idarubicin, Methotrexate, Edatrexate, Trimethoprim, Nolatrexed, Raltitrexed, Hydroxyurea, 5-fluorouracil, ixabepilone, Ftorafur, Capecitabine, Furtulon, Eniluracil, ara-C, 5-azacytidine, Gemcitabine, Mercaptopurine, Thioguanine, Pentostatin, antisense DNA, antisense RNA, an antisense DNA/RNA hybrid, a ribozyme, ultraviolet radiation, Vincristine, Vinblastine, Paclitaxel, Docetaxel, L-Asparaginase, a kinase inhibitor, Imatinib, Mitotane, Aminoglutethimide, Diethylstilbestrol, Ethinyl estradiol, Tamoxifen, Anastrozole, Testosterone propionate, Fluoxymesterone, Flutamide, Leuprolide, Prednisone, Hydroxyprogesterone caproate, Medroxyprogesterone acetate, Megestrol acetate, Interferon-alfa, and Interleukin.

17. A compound of any of claims 1-12, or a pharmaceutically acceptable salt thereof, or a composition of claim 13 for use as a medicament.

18. A compound of any of claims 1-12, or a pharmaceutically acceptable salt thereof, or a composition of claim 13 for use in inhibiting PIM, CDC7 or CK2 in a cell.

## Patentansprüche

1. Eine Verbindung gemäß Formel I: 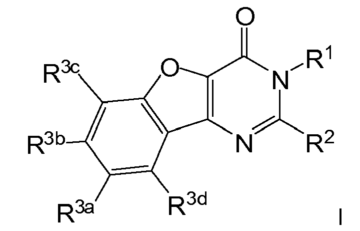 oder ein pharmazeutisch zulässiges Salz davon, wobei:
R¹ Wasserstoff oder (C₁-C₁₂)Alkyl ist;
R₂ ausgewählt ist aus -NH-Phenyl, -NH-Piperidinyl, -NH-Pyridinyl, -NH(C₁-C₃)Alkylphenyl, an einer beliebigen Phenylposition optional substituiert mit Piperazinyl oder Methylpiperazinyl, -NH(C₁-C₃)Alkyl-N(CH₃)₂, -NH(C₁-C₃)Alkyl-OH, -(C₁-C₃)Alkyl-O-phenyl, -(C₁-C₃)Alkyl-O-(C₁-C₃)alkyl-(5-6-Glied)heterocydoalkyl, -(C₁-C₃)Alkyl-N(H)-heteroaryl, -(C₁-C₃)Alkyl-(5-10-Glied)heteroaryl, optional substituiert mit -(C₁-C₃)Alkyl, Halogen oder Phenyl, Oxopyrrolidinyl, optional substituiert mit OH oder Piperidinyl, -(C₁-C₄)Alkyl-(3-9-Glied)heterocycloalkyl, an dem (3-9-Glied)Heterocycloalkyl optional substituiert mit Xd, -(C₁-C₆)Alkyl-NRzb-(C₁-C₄)alkyl, wobei der -(C₁-C₄)Alkylteil mit Xe substituiert ist, -(C₁-C₃)Alkyl-NH-(C₃-C₆)cycloalkyl, -(CH₂)-NH-(C₃-C₆)Cyclohexyl, -(C1-C₃)Alkyl-NH₂, wobei der-(C₁-C₃)Alkylteil von -(C₁-C₃)Alkyl-NH₂ mit Xf substituiert ist, und -(3-9-Glied)Heterocycloalkyl, optional substituiert mit Xg;
Xd ausgewählt ist aus (C₁-C₁₂)Alkyl, 1-3 Halogen, -COOH, Phenyl, optional substituiert mit 1 oder 2 Gruppen, ausgewählt aus Halogen, Methyl und Methylphenyl, Phenylmethyl, Spiropiperidin, Trifluormethylphenylmethyl, -(C₁-C₃)Alkoxy, Pyridinyl, Dimethylamino(C₁-C₁₂)alkyl, Dimethylamino, Hydroxyl(C₁-C₁₂)alkyl, Dimethylamino(C₁-C₁₂)alkylaminocarbonyl, (C₁-C₁₂)Alkylamino, Amino(C₁-C₁₂)alkyl, Dimethylaminocarbonyl(C₁-C₁₂)alkyl, Diethylamino(C₁-C₁₂)alkylcarbonyl, -(C₁-C₃)Alkyl-(5-6-Glied)heterocydoalkyl, (5-6-Glied)Heterocycloalkyl, optional substituiert mit -(C₁-C₃)Alkyl, -NH₂, -OH, (4-12-Glied)Heterocydoalkyl(C₁-C₁₂)alkylamino, (C₁-C₁₂)Aikoxy(C₁-C₁₂)alkyl, -C(O)CH₃, -C(O)NH(C₁-C₃)Alkylphenyl, an einer beliebigen Phenylposition optional substituiert mit 1-3 Halogen, einem -OH und einem -C(O)NH(C₁-C₃)Alkylphenyl, an einer beliebigen Phenylposition optional substituiert mit 1-3 Halogen, -C(O)-(4-12-Glied)Heterocycloalkyl, optional substituiert mit -OH oder Halogen, (C₁-C₁₂)Alkoxycarbonyl, Aminocarbonyl, einem -OH und einem Methyl, einem -OH und einem -C(O)OH, einem -OH und einem -NHC(O)Piperidinyl, einem -OH und einem (C₁-C₁₂)Alkyl;
Xe ausgewählt ist aus Dialkylamino, Amino, 1-3 -OH, (C₁-C₁₂)Alkoxy, 4-Methylpiperazinylphenyl, Dimethylaminophenyl, Phenyl, optional substituiert mit 1-3 Gruppen, ausgewählt aus Halogen und Methoxy, (5-12-Glied)Heteroaryl, -(C₁-C₃)AlkylC(O)NH₂, -C(O)NH₂, -C(O)OH, -(C₁-C₃)Alkylc(O)OH und (4-12-Glied)Heterocycloalkyl, optional substituiert mit 1-2 (C₁-C₁₂)Alkyl;
Xf ausgewählt ist aus (C₃-C₁₄)Cycloalkyl, Spiro(C₃-C₁₄)cycloalkyl, Phenyl, Phenyl(C₁-C₁₂)alkyl, optional substituiert mit Phenylmethyloxy oder (C₁-C₁₂)Alkoxy, und Thienyl(C₁-C₁₂)alkyl;
Xg ausgewählt ist aus (C₁-C₁₂)Alkyl, (C₁-C₁₂)Alkylcarbonyl, (4-12-Glied)Heterocycloalkylcarbonyl, Dialkylamino(C₁-C₁₂)alkylcarbonyl, 1-Methylpiperidinyl, Dialkylamino(C₁-C₁₂)alkyl, (5-12-Glied)Heteroarylcarbonyl, (C₁-C₁₂)Alkoxy(C₁-C₁₂)alkylcarbonyl, Phenylcarbonyl, Phenyl(C₁-C₁₂)alkylcarbonyl, Oxo, Phenyl(C₁-C₁₂)alkyl, -(5-6-Glied)Heteroaryl(C₁-C₁₂)alkyl, Piperidinyloxy, -OH, Oxo, 1-2 Halogen und 1-2 Methyl; und
Rzb H oder ein Alkyl, optional substituiert mit 1-3 -OH, ist;
R^{3a} ausgewählt ist aus Halogen, (C₁-C₁₂)Alkyl, -NO₂, (C₁-C₁₂)Alkoxy, (C₁-C₁₂)Alkinyl, optional substituiert mit R¹⁴, (C₁-C₁₂)Alkoxycarbonyl(C₁-C₁₂)alkyl, (C₆-C₁₄)Aryl(C₁-C₁₂)alkoxy, -C(O)N(H)(C₁-C₁₂)Alkyl, -N(H)-C(O)-(C₁-C₁₂)Alkyl, -C(O)-(C₁-C₁₂)Alkyl, -CN, Phenyl, -OCF₃, -N(H)R¹³, -OH, -CF₃, -S-CH₃ und Hydroxymethyl(C₁-C₁₂)alkinyl;
R^{3b}, R^{3c} und R^{3d} jeweils unabhängig ausgewählt sind aus H, -OH, -N⁺(O)OH, (C₁-C₁₂)Alkoxyl und Halogen;
oder R^{3a} Wasserstoff ist und R^{3b}, R^{3c} und R^{3d} jeweils unabhängig ausgewählt sind aus -CF₃, -OH, (C₁-C₁₂)Alkoxy und Halogen;
oder R^{3a} und R^{3d}, zusammen mit den Kohlenstoffen, an die sie gebunden sind, sich verbinden, um ein 5-Glied-Heteroaryl, optional substituiert mit Methyl oder-NH2_{,}
oder ein 5-6-Glied-Heterocycloalkyl zu bilden;
R¹³ ausgewählt ist aus (C₆-C₁₄)Aryl(C₁-C₁₂)alkyl, wobei der Arylteil von Arylalkyl optional substituiert ist mit 1, 2 oder 3 (C₁-C₁₂)Alkoxy, Halogen, Methyl, Methoxy, -CF₃, (C₃-C₁₄)Cycloalkyl und (5-12-Glied)Heteroaryl(C₁-C₁₂)alkyl; und
R¹⁴ ausgewählt ist aus Hydroxyl(C₁-C₁₂)alkyl, H und TMS.

2. Verbindung gemäß Anspruch 1 oder ein pharmazeutisch zulässiges Salz davon, wobei R^{3a} ausgewählt ist aus Halogen, (C₁-C₁₂)Alkyl, -NO₂, (C₁-C₁₂)Alkoxy, (C₁-C₁₂)Alkinyl, optional substituiert mit R¹⁴, (C₁-C₁₂)Alkoxycarbonyl(C₁-C₁₂)alkyl, (C₆-C₁₄)Aryl(C₁-C₁₂)alkoxy, -C(0)N(H)(C₁-C₁₂)Alkyl, -N(H)-C(O)-(C₁-C₁₂)Alkyl, -C(O)-(C₁-C₁₂)Alkyl, -CN, Phenyl, -OCF₃, -N(H)R¹³, -OH, -CF₃, -S-CH₃ und Hydroxymethyl(C₁-C₁₂)alkinyl; und R^{3b}, R³⁰ und R^{3d} jeweils H sind.

3. Verbindung gemäß Anspruch 1 oder ein pharmazeutisch zulässiges Salz davon, wobei R^{3a} Halogen, (C₁-C₁₂)Alkoxy oder -OCF₃ ist und R^{3b}, R³⁰ und R^{3d} jeweils H sind.

4. Verbindung gemäß Anspruch 1 oder ein pharmazeutisch zulässiges Salz davon, wobei R² ein (3-9-Glied)Heterocycloalkyl ist, optional substituiert mit Xg; R^{3a} Halogen, (C₁-C₁₂₎Alkoxy oder-OCF₃ ist und R^{3b}, R^{3c} und R^{3d} jeweils H sind.

5. Verbindung gemäß Anspruch 1 oder ein pharmazeutisch zulässiges Salz davon, wobei R² -(C₁-C₃)Alkyl-(5-10-Glied)heteroaryl ist, wobei das Heteroaryl optional mit -(C₁-C₃)Alkyl, Halogen und Phenyl substituiert ist; R^{3a} Halogen, (C₁-C₁₂)Alkoxy oder -OCF₃ ist und R^{3b}, R^{3c} und R^{3d} jeweils H sind.

6. Verbindung gemäß Anspruch 1 oder ein pharmazeutisch zulässiges Salz davon, wobei R² 2-(C₁-C₄)Alkyl-(3-9-Glied)heterocycloalkyl ist, an einer beliebigen Position des (3-9-Glied)Heterocycloalkyls optional substituiert mit Xd; R^{3a} Halogen, (C₁-C₁₂)Alkoxy oder -OCF₃ ist und R^{3b}, R^{3c} und R^{3d} jeweils H sind.

7. Verbindung gemäß Anspruch 1 oder ein pharmazeutisch zulässiges Salz davon, wobei R² ein Heterocycloalkyl ist, ausgewählt aus Piperazinyl, Piperidinyl, Pyrrolidinyl, Isoxazolyl, Azetidinyl, Morpholinyl, Tetrahydrofuranyl, Thiazolidinyl oder Octahydro-1H-indolyl, wobei das Heterocycloalkyl optional mit Xg substituiert ist; R^{3a} Halogen, (C₁-C₁₂)Alkoxy oder-OCF₃ ist und R^{3b}, R^{3c} und R^{3d} jeweils H sind.

8. Verbindung gemäß Anspruch 1 oder ein pharmazeutisch zulässiges Salz davon, wobei R² 2(3-9-Glied)Heterocycloalkyl(C₁-C₄)alkyl ist und wobei der Heterocycloalkylteil des Heterocycloalkylalkyls Piperazinyl, Piperidinyl, Pyrrolidinyl, Azetidinyl, Morpholinyl, 1,4-Diazepanyl, 2,5-Diazabicyclo[2.2.1]heptyl, Azabicyclo[2.2.1]heptan, 8-Azabicyclo[3.2.1]oct-8-yl, (1S,4S)-2,5-Diazabicyclo[2.2.1]heptyl, 2,5-Dihydro-1H-pyrrolyl, (1 R,4R)-2,5-Diazabicyclo[2.2.1]heptan-2-yl, (1R,5S)-8-Azabicyclo[3.2.1]octyl, (8aS)-Hexahydropyrrolo[1,2-a]pyrazin-2)-yl, Dimethylpiperazinyl oder (3aR,6aS)-Hexahydropyrrolo[3,4-c]pyrrolyl ist.

9. Verbindung gemäß Anspruch 1 oder ein pharmazeutisch zulässiges Salz davon, wobei R² ein (5-10-Glied)Heteroaryl(C₁-C₃)alkyl ist, an einer beliebigen Ringposition optional substituiert mit -(C₁-C₃)Alkyl, Halogen und Phenyl; R^{3a} Halogen, (C₁ C₁₂)Alkoxy oder-OCF₃ ist und R^{3b}, R^{3c} und R^{3d} jeweils H sind.

10. Verbindung gemäß Anspruch 1 oder ein pharmazeutisch zulässiges Salz davon, wobei R₂ wie folgt ist: 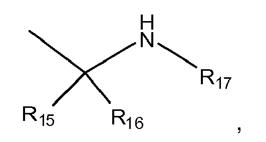 wobei R₁₅ ausgewählt ist aus H oder -(C₁-C₆)Alkyl, R₁₆ ausgewählt ist aus H, Phenyl und -(C₁-C₆)Alkyl und R₁₇ ausgewählt ist aus H, -(C₁-C₃)AlkylC(O)NH₂, -(C₁-C₃)Alkylc(O)OH und (4-12-Glied)Heterocycloalkyl(C₁ C₁₂)alkyl.

11. Verbindung gemäß Anspruch 1, ausgewählt aus einer der folgenden Verbindungen:
| **Vb.-Nr.** | **Struktur** | **NAME** |
|---|---|---|
| 1 | 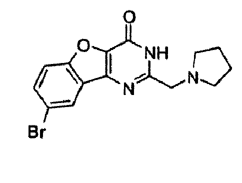 | 8-Brom-2-(pyrrolidin-1-ylmethyl)[1]benzofuro[3,2-d]pyrimidin-4(3H)-on |
| 2 | 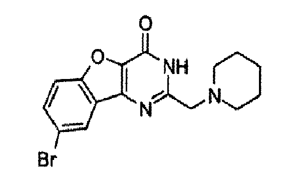 | 8-Brom-2-(piperidin-1-ylmethyl)[1]benzofuro[3,2-d]pyrimidin-4(3H)-on |
| 3 | 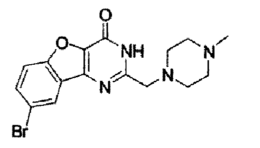 | 8-Brom-2-[(4-methylpiperazin-1-yl)methyl][1]benzofuro[3,2-d]pyrimidin-4(3H)-on |
| 4 | 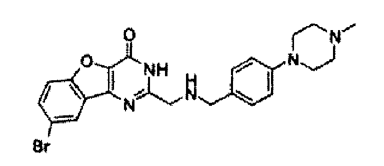 | 8-Brom-2-[({[4-(4-methylpiperazin-1-yl)phenyl]methyl}amino)methyl][1] benzofuro[3,2-d]pyrimidin-4(3H)-on |
| 5 | 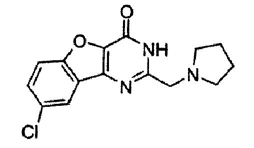 | 8-Chlor-2-(pyrrolidin-1-ylmethyl)[1]benzofuro[3,2-d]pyrimidin-4(3H)-on |
| 6 | 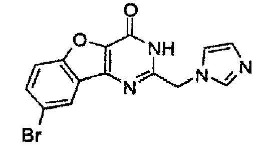 | 8-Brom-2-(1H-imidazol-1-ylmethyl)[1]benzofuro[3,2-d]pyrimidin-4(3H)-on |
| 7 | 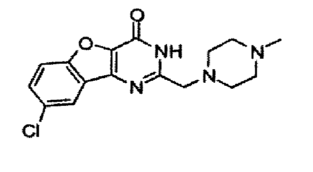 | 8-Chlor-2-[(4-methylpiperazin-1-yl)methyl][1]benzofuro[3,2-d]pyrimidin-4(3H)-on |
| 9 | 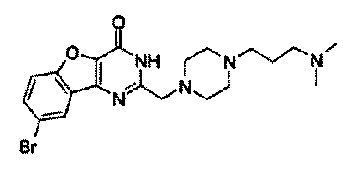 | 8-Brom-2-({4-[3-(dimethylamino)propyl]piperazin-1-yl}methyl)[1]benzofuro[3,2-d]pyrimidin-4(3H)-on |
| 10 | 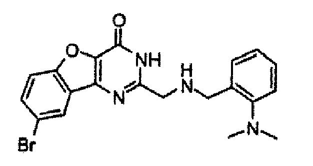 | 8-Brom-2-[({[2-(dimethylamino)phenyl]methyl} amino)methyl][1]benzofuro[3,2-d]pyrimidin-4(3H)-on |
| 13 | 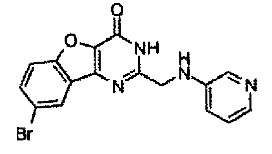 | 8-Brom-2-[(pyridin-3-ylamino)methyl][1]benzofuro[3,2-d]pyrimidin-4(3H)-on |
| 14 | 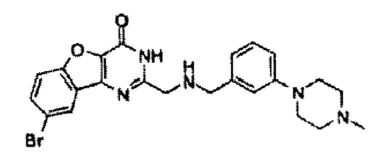 | 8-Brom-2-[({[3-(4-methylpiperazin-1 -yl)phenyl]methyl}amino)methyl][1] benzofuro[3,2-d]pyrimidin-4(3H)-on |
| 15 | 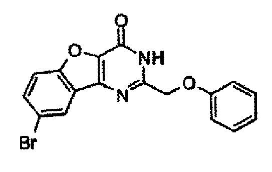 | 8-Brom-2-[(phenyloxy)methyl][1]benzofuro[3,2-d]pyrimidin-4(3H)-on |
| 17 | 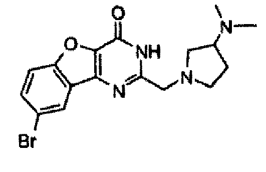 | 8-Brom-2-{[3-(dimethylamino)pyrrolidin-1-yl]methyl}[1]benzofuro[3,2-d]pyrimidin-4(3H)-on |
| 20 | 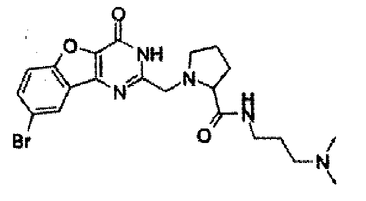 | 1-[(8-Brom-4-oxo-3,4-dihydro[1]benzofuro[3,2-d]pyrimidin-2-yl)methyl]-N-[3-(dimethylamino)propyl]prolinamid |
| 21 | 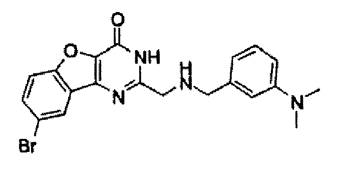 | 8-Brom-2-[({[3-(dimethylamino)phenyl]methyl} amino)methyl][1]benzofuro[3,2-d]pyrimidin-4(3H)-on |
| 23 | 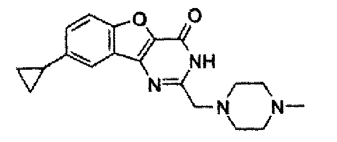 | 8-Cyclopropyl-2-[(4-methylpiperazin-1-yl)methyl][1]benzofuro[3,2-d]pyrimidin-4(3H)-on |
| 24 | 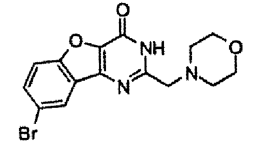 | 8-Brom-2-(morpholin-4-ylmethyl)[1]benzofuro[3,2-d]pyrimidin-4(3H)-on |
| 25 | 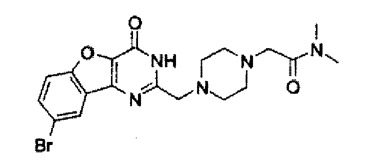 | 2-{4-[(8-Brom-4-oxo-3,4-dihydro[1]benzofuro[3,2-d]pyrimidin-2-yl)methyl]piperazin-1-yl}-N,N-dimethylacetamid |
| 26 | 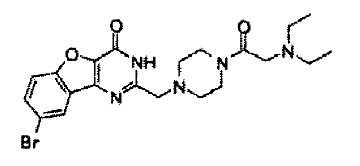 | 8-Brom-2-{[4-(N,N-diethylglycyl)piperazin-1-yl]methyl}[1]benzofuro[3,2-d]pyrimidin-4(3H)-on |
| 28 | 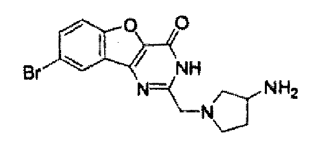 | 2-[(3-Aminopyrrolidin-1-yl)methyl]-8-brom[1]benzofuro[3,2-d]pyrimidin-4(3H)-on |
| 29 | 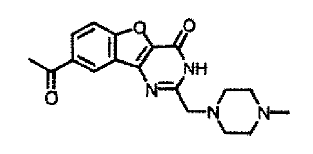 | 8-Acetyl-2-[(4-methylpiperazin-1-yl)methyl][1]benzofuro[3,2-d]pyrimidin-4(3H)-on |
| 45 | 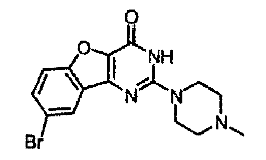 | 8-Brom-2-(4-methylpiperazin-1-yl)[1]benzofuro[3,2-d]pyrimidin-4(3H)-on |
| 46 | 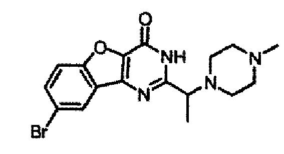 | 8-Brom-2-[1-(4-methylpiperazin-1-yl)ethyl][1]benzofuro[3,2-d]pyrimidin-4(3H)-on |
| 49 | 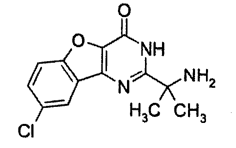 | 2-(1-Amino-1-methylethyl)-8-chlor[1]benzofuro[3,2-d]pyrimidin-4(3H)-on |
| 74 | 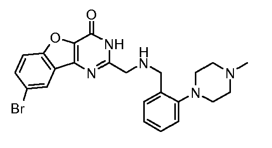 | 8-Brom-2-[({[2-(4-methylpiperazin-1 -yl)phenyl]methyl}amino)methyl][1] benzofuro[3,2-d]pyrimidin-4(3H)-on |
| 81 | 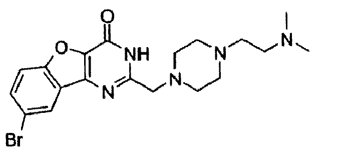 | 8-Brom-2-({4-[2-(dimethylamino)ethyl]piperazin-1-yl}methyl)[1]benzofuro[3,2-d]pyrimidin-4(3H)-on |
| 82 | 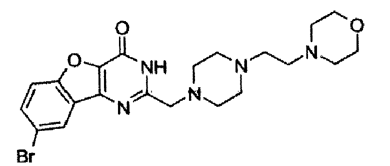 | 8-Brom-2-{[4-(2-morpholin-4-ylethyl)piperazin-1-yl]methyl}[1]benzofuro[3,2-d]pyrimidin-4(3H)-on |
| 84 | 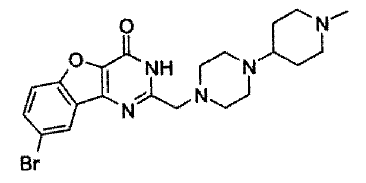 | 8-Brom-2-{[4-(1-methylpiperidin-4-yl)piperazin-1-yl]methyl}[1]benzofuro[3,2-d]pyrimidin-4(3H)-on |
| 100 | 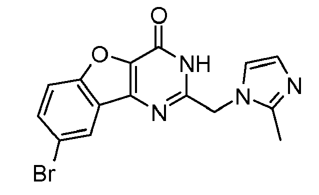 | 8-Brom-2-[(2-methyl-1H-imidazol-1- yl)methyl][1]benzofuro[3,2-d]pyrimidin-4(3H)-on |
| 103 | 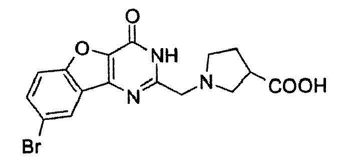 | 1-[(8-Brom-4-oxo-3,4-dihydro[1]benzofuro[3,2-d]pyrimidin- 2-yl)methyl]pyrrolidin-3-carbonsäure |
| 104 | 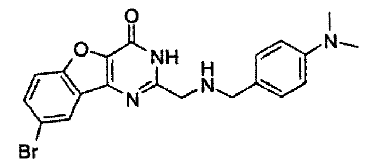 | 8-Brom-2-[({[4(dimethylamino)phenyl]methyl} amino)methyl][1]benzofuro[3,2-d]pyrimidin-4(3H)-on |
| 105 | 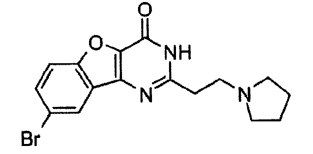 | 8-Brom-2-(2-pyrrolidin-1-ylethyl)[1]benzofuro[3,2-d]pyrimidin-4(3H)-on |
| 108 | 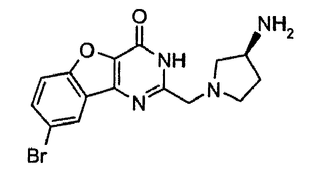 | 2-{[(3S)-3-Aminopyrrolidin-1-yl]methyl}-8-brom[1]benzofuro[3,2- d]pyrimidin-4(3H)-on |
| 109 | 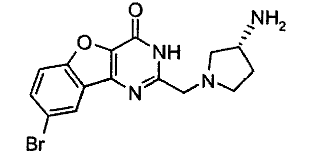 | 2-{[(3R)-3-Aminopyrrolidin-1-yl]methyl}-8-brom[1]benzofuro[3,2-d]pyrimidin-4(3H)-on |
| 110 | 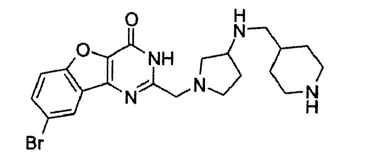 | 8-Brom-2-({3-[(piperidin-4-ylmethyl)amino]pyrrolidin-1-yl}methyl)[1]benzofuro[3,2- d]pyrimidin-4(3H)-on |
| 111 | 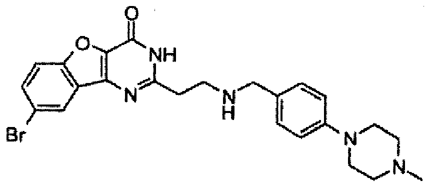 | 8-Brom-2-[2-({[4-(4-methylpiperazin1-yl)phenyl]methyl}amino)ethyl][1] benzofuro[3,2-d]pyrimidin-4(3H)-on |
| 113 | 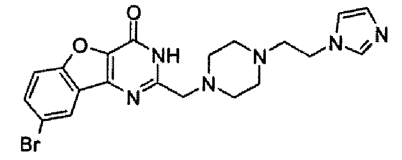 | 8-Brom-2-({4-[2--imidazol-1-yl)ethyl]piperazin-1-yl}methyl)[1]benzofuro[3,2-d]pyrimidin-4(3H)-on |
| 114 | 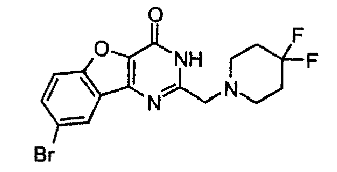 | 8-Brom-2-[(4,4-difluorpiperidin-1-yl)methyl][1]benzofuro[3,2-d]pyrimidin-4(3H)-on |
| 115 | 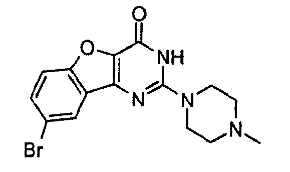 | 8-Brom-2-(4-methylpiperazin-1-yl)[1]benzofuro[3,2-d]pyrimidin-4(3H)-on |
| 117 | 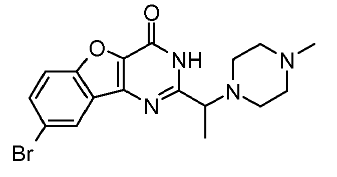 | 8-Brom-2-[1-(4-methylpiperazin-1-yl)ethyl][1]benzofuro[3,2-d]pyrimidin- 4(3H)-on |
| 118 | 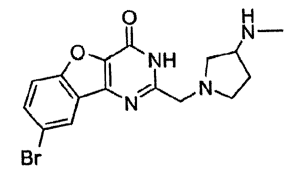 | 8-Brom-2-{[3-(methylamino)pyrrolidin-1-yl]methyl}[1]benzofuro[3,2-d]pyrimidin-4(3H)-on |
| 119 | 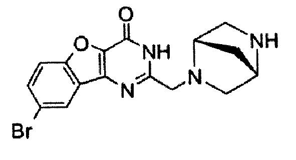 | 8-Brom-2-[(1S,4S)-2,5-diazabicyclo[2.2.1]hept-2-ylmethyl][1]benzofuro[3,2-d]pyrimidin-4(3H)-on |
| 122 | 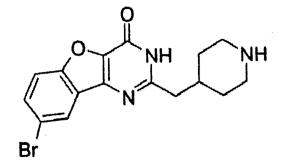 | 8-Brom-2-(piperidin-4-ylmethyl)[1]benzofuro[3,2-d]pyrimidin-4(3H)-on |
| 130 | 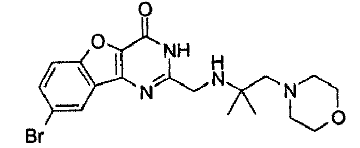 | 8-Brom-2-{[(1,1-dimethyl-2-morpholin-4-ylethyl)amino]methyl}[1]benzofuro[3, 2-d]pyrimidin-4(3H)-on |
| 131 | 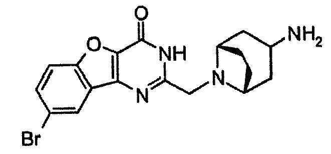 | 2-{[(1R,5S)-3-Amino-8-azabicyclo[3.2.1]oct-8-yl]methyl}-8-brom[1]benzofuro[3,2-d]pyrimidin-4(3H)-on |
| 132 | 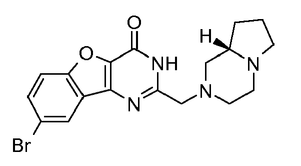 | 8-Brom-2-[(8aS)-hexahydropyrrolo[1,2-a]pyrazin-2(1H)-ylmethyl][1]benzofuro[3,2-d]pyrimidin-4(3H)-on |
| 133 | 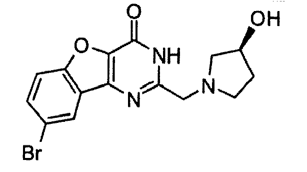 | 8-Brom-2-{[(3S)-3-hydroxypyrrolidin-1-yl]methyl}[1]benzofuro[3,2-d]pyrimidin-4(3H)-on |
| 141 | 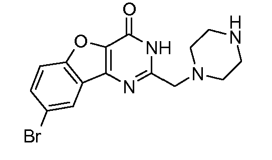 | 8-Brom-2-(piperazin-1-ylmethyl)[1]benzofuro[3,2-d]pyrimidin-4(3H)-on |
| 142 | 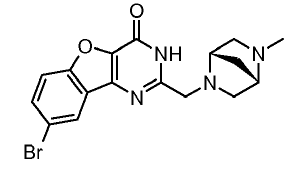 | 8-Brom-2-{[(1S,4S)-5-methyl-2,5-diazabicyclo[2.2.1]hept-2-yl]methyl}[1]benzofuro[3,2-d]pyrimidin-4(3H)-on |
| 147 | 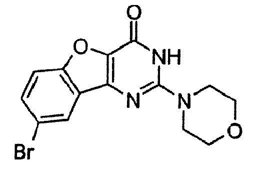 | 8-Brom-2-morpholin-4-yl[1]benzofuro[3,2-d]pyrimidin-4(3H)-on |
| 149 | 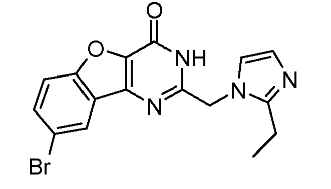 | 8-Brom-2-[(2-ethyl-1H-imidazol-1-yl)methyl][1]benzofuro[3,2-d]pyrimidin-4(3H)-on |
| 150 | 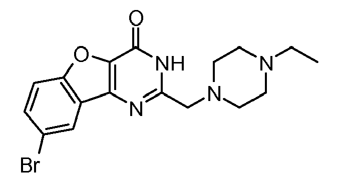 | 8-Brom-2-[(4-ethylpiperazin-1-yl)methyl][1]benzofuro[3,2-d]pyrimidin-4(3H)-on |
| 151 | 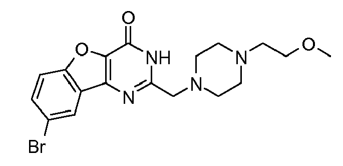 | 8-Brom-2-({4-[2-(methyloxy)ethyl]piperazin-1-yl}methyl)[1]benzofuro[3,2-d]pyrimidin-4(3H)-on |
| 153 | 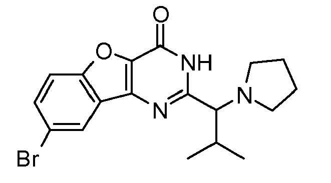 | 8-Brom-2-(2-methyl-1-pyrrolidin-1-ylpropyl)[1]benzofuro[3,2-d]pyrimidin-4(3H)-on |
| 157 | 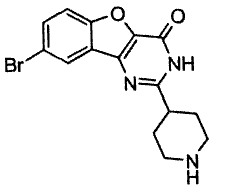 | 8-Brom-2-piperidin-4-yl[1]benzofuro[3,2-d]pyrimidin-4(3H)-on |
| 158 | 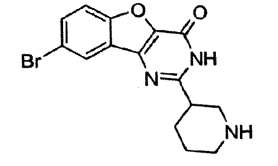 | 8-Brom-2-piperidin-3-yl[1]benzofuro[3,2-d]pyrimidin-4(3H)-on |
| 164 | 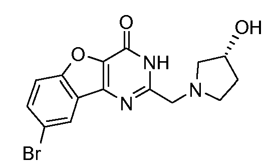 | 8-Brom-2-{[(3R)-3-hydroxypyrrolidin-1-yl]methyl}[1]benzofuro[3,2-d]pyrimidin-4(3H)-on |
| 165 | 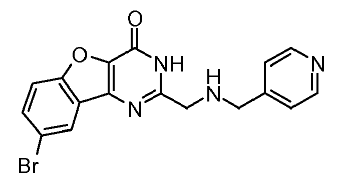 | 8-Brom-2-{[(pyridin-4-ylmethyl)amino]methyl}[1]benzofuro [3,2-d]pyrimidin-4(3H)-on |
| 166 | 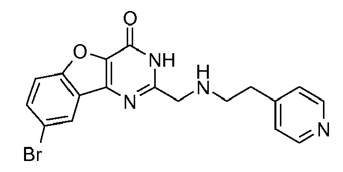 | 8-Brom-2-{[(2-pyridin-4-ylethyl)amino]methyl}[1]benzofuro[3, 2-d]pyrimidin-4(3H)-on |
| 167 | 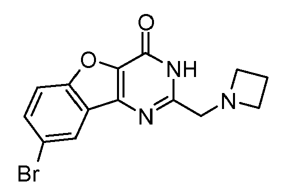 | 2-(Azetidin-1-ylmethyl)-8-brom[1]benzofuro[3,2-d]pyrimidin-4(3H)-on |
| 168 | 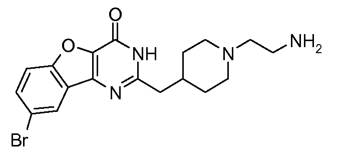 | 2-{[1-(2-Aminoethyl)piperidin-4-yl]methyl}-8-brom[1]benzofuro[3,2-d]pyrimidin-4(3H)-on |
| 169 | 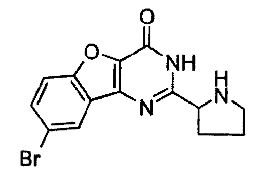 | 8-Brom-2-pyrrolidin-2-yl[1]benzofuro[3,2-d]pyrimidin-4(3H)-on |
| 172 | 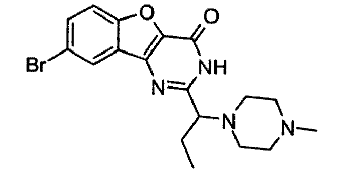 | 8-Brom-2-[1-(4-methylpiperazin-1-yl)propyl][1]benzofuro[3,2-d]pyrimidin-4(3H)-on |
| 174 | 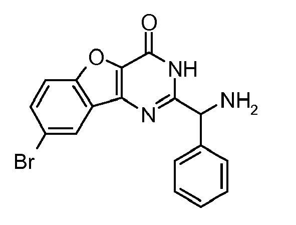 | 2-[Amino(phenyl)methyl]-8-brom[1]benzofuro[3,2-d]pyrimidin-4(3H)-on |
| 175 | 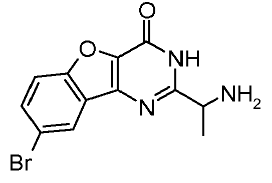 | 2-(1-Aminoethyl)-8-brom[1]benzofuro[3,2-d]pyrimidin-4(3H)-on |
| 176 | 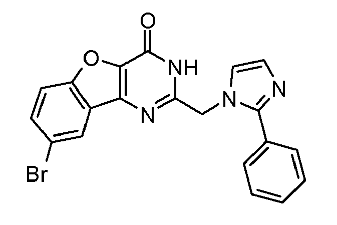 | 8-Brom-2-[(2-phenyl-1H-imidazol-1-yl)methyl][1]benzofuro[3,2-d]pyrimidin-4(3H)-on |
| 177 | 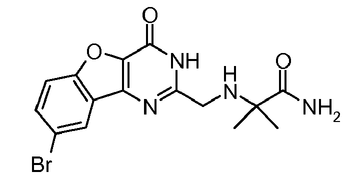 | N²-[(8-Brom-4-oxo-3,4-dihydro[1]benzofuro[3,2-d]pyrimidin-2-yl)methyl]-2-methylalaninamid |
| 180 | 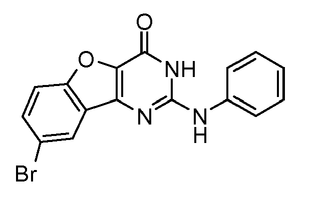 | 8-Brom-2-(phenylamino)[1]benzofuro[3,2-d]pyrimidin-4(3H)-on |
| 183 | 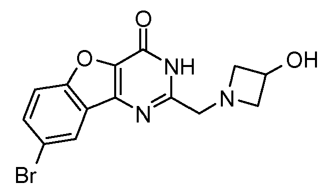 | 8-Brom-2-[(3-hydroxyazetidin-1-yl)methyl][1]benzofuro[3,2-d]pyrimidin-4(3H)-on |
| 184 | 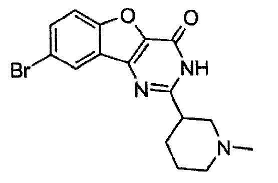 | 8-Brom-2-(1-methylpiperidin-3-yl)[1]benzofuro[3,2-d]pyrimidin-4(3H)-on |
| 185 | 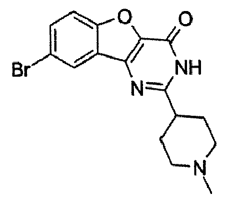 | 8-Brom-2-(1-methylpiperidin-4-yl)[1]benzofuro[3,2-d]pyrimidin-4(3H)-on |
| 186 | 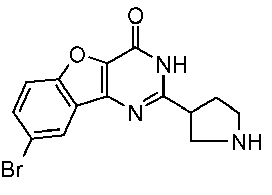 | 8-Brom-2-pyrrolidin-3-yl[1]benzofuro[3,2-d]pyrimidin-4(3H)-on |
| 187 | 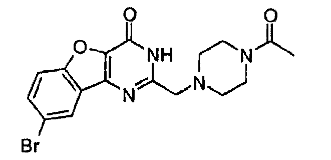 | 2-[(4-Acetylpiperazin-1-yl)methyl]-8-brom[1]benzofuro[3,2-d]pyrimidin-4(3H)-on |
| 189 | 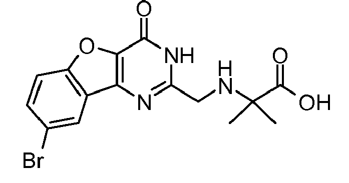 | N-[(8-Brom-4-oxo-3,4-dihydro[1]benzofuro[3,2-d]pyrimidin-2-yl)methyl]-2-methylalanin |
| 190 | 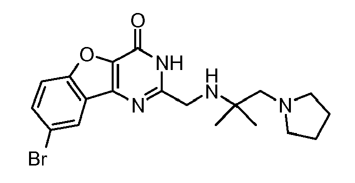 | 8-Brom-2-{[(1,1-dimethyl-2-pyrrolidin-1-ylethyl)amino]methyl}[1]benzofuro[3, 2-d]pyrimidin-4(3H)-on |
| 191 | 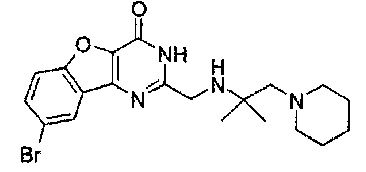 | 8-Brom-2-{[(1,1-dimethyl-2-piperidin-1-ylethyl)amino]methyl}[1]benzofuro[3, 2-d]pyrimidin-4(3H)-on |
| 192 | 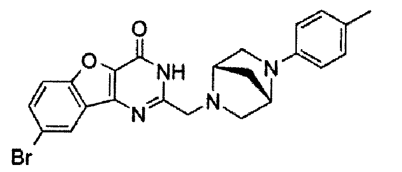 | 8-Brom-2-{[(1S,4S)-5-(4-methylphenyl)-2,5-diazabicyclo[2.2.1]hept-2-yl]methyl}[1]benzofuro[3,2-d]pyrimidin-4(3H)-on |
| 193 | 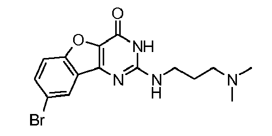 | 8-Brom-2-{[3-(dimethylamino)propyl]amino}[1] benzofuro[3,2-d]pyrimidin-4(3H)-on |
| 194 | 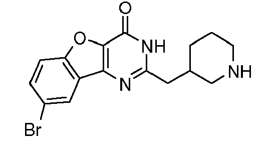 | 8-Brom-2-(piperidin-3-ylmethyl)[1]benzofuro[3,2-d]pyrimidin-4(3H)-on |
| 195 | 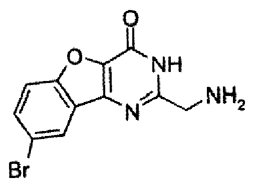 | 2-(Aminomethyl)-8-brom[1]benzofuro[3,2-d]pyrimidin-4(3H)-on |
| 196 | 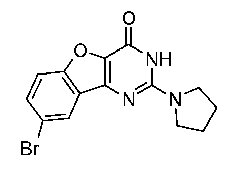 | 8-Brom-2-pyrrolidin-1-yl[1]benzofuro[3,2-d]pyrimidin-4(3H)-on |
| 198 | 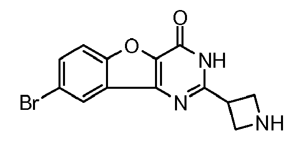 | 2-Azetidin-3-yl-8-brom[1]benzofuro[3,2-d]pyrimidin-4(3H)-on |
| 199 | 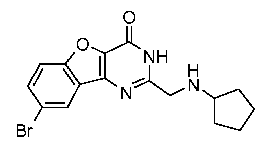 | 8-Brom-2-[(cyclopentylamino)methyl][1] benzofuro[3,2-d]pyrimidin-4(3H)-on |
| 200 | 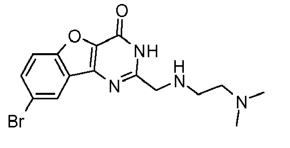 | 8-Brom-2-({[2-(dimethylamino)ethyl]amino}methyl) [1]benzofuro[3,2-d]pyrimidin-4(3H)-on |
| 201 | 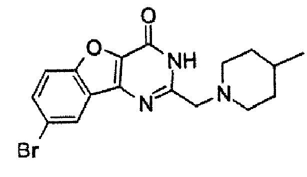 | 8-Brom-2-[(4-methylpiperidin-1-yl)methyl][1]benzofuro[3,2-d]pyrimidin-4(3H)-on |
| 202 | 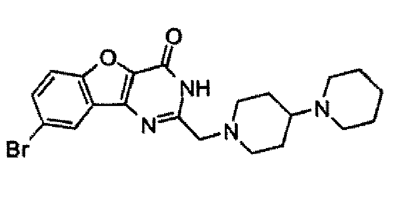 | 2-(1,4'-Bipiperidin-1'-ylmethyl)-8-brom[1]benzofuro[3,2-d]pyrimidin-4(3H)-on |
| 203 | 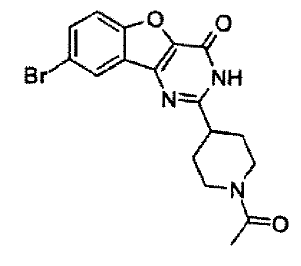 | 2-(1-Acetylpiperidin-4-yl)-8-brom[1]benzofuro[3,2-d]pyrimidin-4(3H)-on |
| 204 | 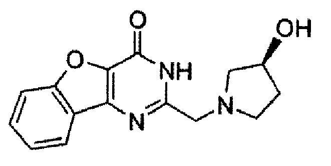 | 2-{[(3S)-3-Hydroxypyrrolidin-1-yl]methyl}[1]benzofuro[3,2-d]pyrimidin-4(3H)-on |
| 206 | 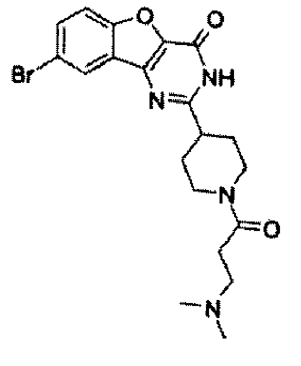 | 8-Brom-2-[1-(N,N-dimethyl-beta-alanyl)piperidin-4-yl][1]benzofuro[3,2-d]pyrimidin-4(3H)-on |
| 207 | 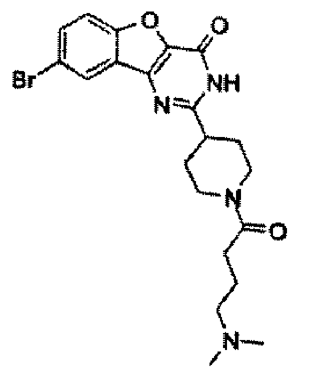 | 8-brom-2-{1-[4-(dimethylamino)butanoyl]piperidin-4-yl}[1]benzofuro[3,2-d]pyrimidin-4(3H)-on |
| 208 | 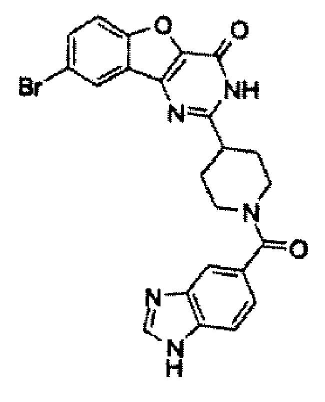 | 2-[1--Benzimidazol-5-ylcarbonyl)piperidin-4-yl]-8-brom[1]benzofuro[3,2-d]pyrimidin-4(3H)-on |
| 209 | 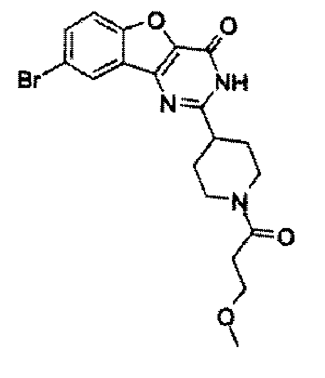 | 8-Brom-2-{1-[3-(methyloxy)propanoyl]piperidin-4-yl}[1]benzofuro[3,2-d]pyrimidin-4(3H)-on |
| 210 | 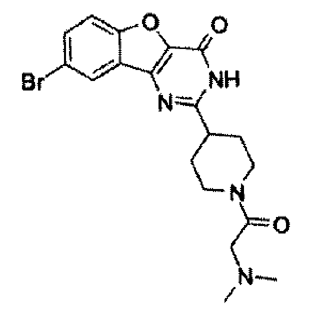 | 8-Brom-2-[1-(N, N-dimethylglycyl)piperidin-4-yl][1]benzofuro[3,2-d]pyrimidin-4(3H)-on |
| 212 | 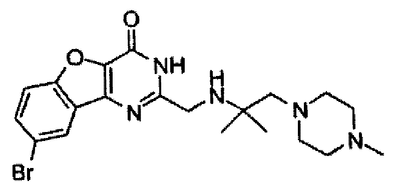 | 8-Brom-2-({[1,1-dimethyl-2-(4-methylpiperazin-1-yl)ethyl]amino}methyl)[1]benzofuro[3 ,2-d]pyrimidin-4(3H)-on |
| 213 | 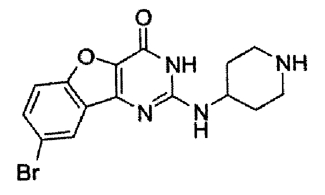 | 8-Brom-2-(piperidin-4-ylamino)[1]benzofuro[3,2-d]pyrimidin-4(3H)-on |
| 214 | 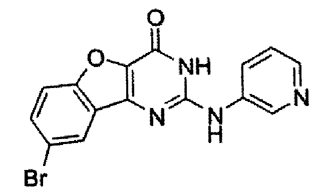 | 8-Brom-2-(pyridin-3-ylamino)[1]benzofuro[3,2-d]pyrimidin-4(3H)-on |
| 215 | 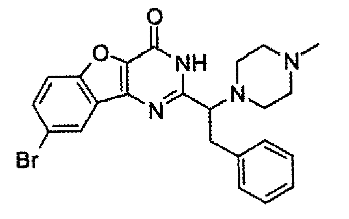 | 8-Brom-2-[1-(4-methylpiperazin-1-yl)-2-phenylethyl][1]benzofuro[3,2-d]pyrimidin-4(3H)-on |
| 218 | 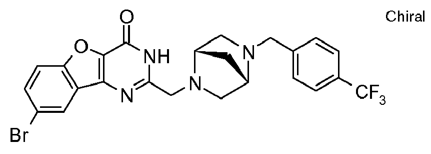 | 8-Brom-2-{[(1S,4S)-5-{[4-(trifluormethyl)phenyl]methyl}-2,5-diazabicyclo[2.2.1]hept-2-yl]methyl}[1]benzofuro[3,2-d]pyrimidin-4(3H)-on |
| 221 | 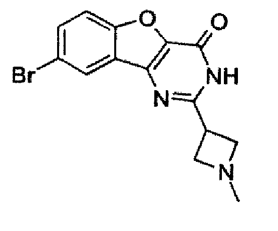 | 8-Brom-2-(1-methylazetidin-3-yl)[1]benzofuro[3,2-d]pyrimidin-4(3H)-on |
| 224 | 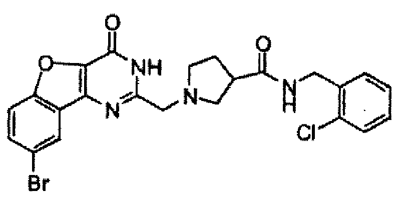 | 1-[(8-Brom-4-oxo-3,4-dihydro[1]benzofuro[3,2-d]pyrimidin-2-yl)methyl]-N-[(2-chlorphenyl)methyl]pyrrolidin-3-carboxamid |
| 225 | 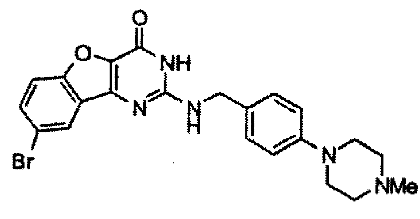 | 8-Brom-2-({[4-(4-methylpiperazin-1 -yl)phenyl]methyl}amino)[1]benzofuro [3,2-d]pyrimidin-4(3H)-on |
| 226 | 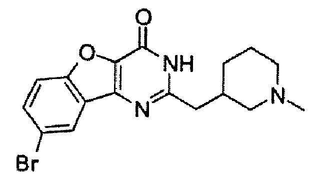 | 8-Brom-2-[(1-methylpiperidin-3-yl)methyl][1]benzofuro[3,2-d]pyrimidin-4(3H)-on |
| 227 | 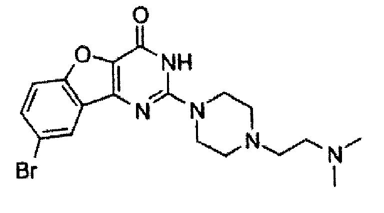 | 8-Brom-2-{4-[2-(dimethylamino)ethyl]piperazin-1-yl}[1]benzofuro[3,2-d]pyrimidin-4(3H)-on |
| 228 | 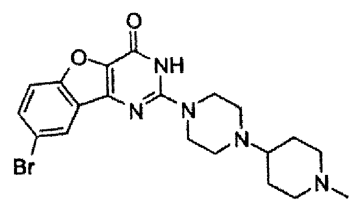 | 8-Brom-2-[4-(1-methylpiperidin-4-yl)piperazin-1-yl][1]benzofuro[3,2-d]pyrimidin-4(3H)-on |
| 229 | 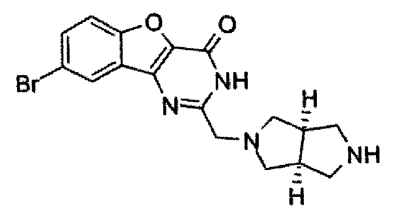 | 8-Brom-2-[(3aR,6aS)-hexahydropyrrolo[3,4-c]pyrrol-2)-ylmethyl][1]benzofuro[3,2-d]pyrimidin-4(3H)-on |
| 230 | 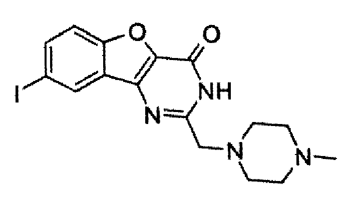 | 8-lod-2-[(4-methylpiperazin-1-yl)methyl][1]benzofuro[3,2-d]pyrimidin-4(3H)-on |
| 232 | 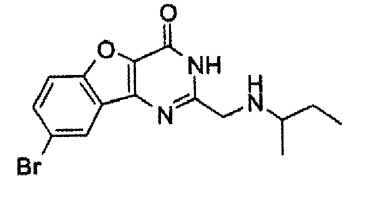 | 8-Brom-2-{[(1-methylpropyl)amino]methyl[1] benzofuro[3,2-d]pyrimidin-4(3H)-on |
| 233 | 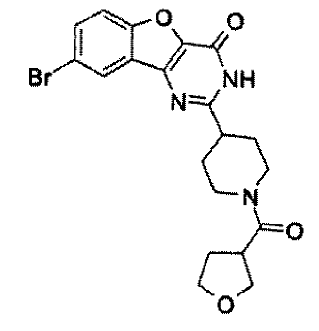 | 8-Brom-2-[1-(tetrahydrofuran-3-ylcarbonyl)piperidin-4-yl][1]benzofuro[3,2-d]pyrimidin-4(3H)-on |
| 234 | 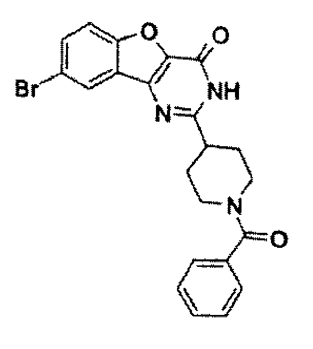 | 8-Brom-2-[1-(phenylcarbonyl)piperidin-4-yl][1]benzofuro[3,2-d]pyrimidin-4(3H)-on |
| 235 | 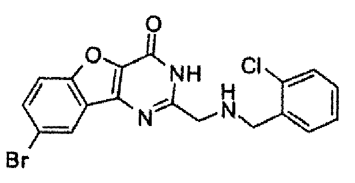 | 8-Brom-2-({[(2-eh lorphenyl)methyl]amino}methyl)[1] benzofuro[3,2-d]pyrimidin-4(3H)-on |
| 236 | 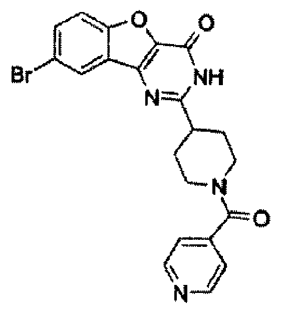 | 8-Brom-2-[1-(pyridin-4-ylcarbonyl)piperidin-4-yl][1]benzofuro[3,2-d]pyrimidin-4(3H)-on |
| 237 | 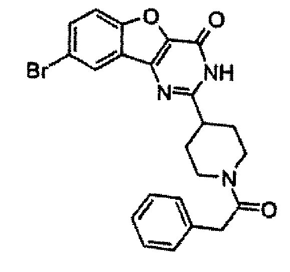 | 8-Brom-2-[1-(phenylacetyl)piperidin-4-yl][1]benzofuro[3,2-d]pyrimidin-4(3H)-on |
| 238 | 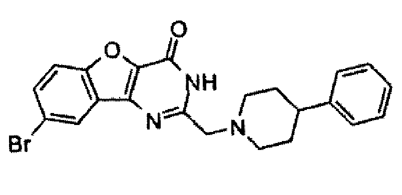 | 8-Brom-2-[(4-phenylpiperidin-1-yl)methyl][1]benzofuro[3,2-d]pyrimidin-4(3H)-on |
| 239 | 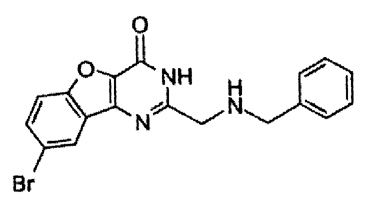 | 8-Brom-2-{[(phenylmethyl)amino]methyl}[1] benzofuro[3,2-d]pyrimidin-4(3H)-on |
| 240 | 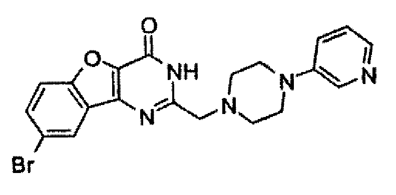 | 8-Brom-2-[(4-pyridin-3-ylpiperazin-1-yl)methyl][1]benzofuro[3,2-d]pyrimidin-4(3H)-on |
| 242 | 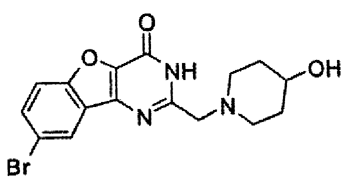 | 8-Brom-2-[(4-hydroxypiperidin-1-yl)methyl][1]benzofuro[3,2-d]pyrimidin-4(3H)-on |
| 244 | 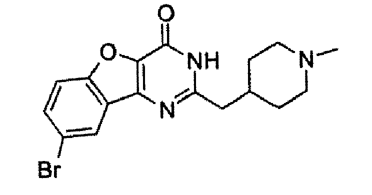 | 8-Brom-2-[(1-methylpiperidin-4-yl)methyl][1]benzofuro[3,2-d]pyrimidin-4(3H)-on |
| 245 | 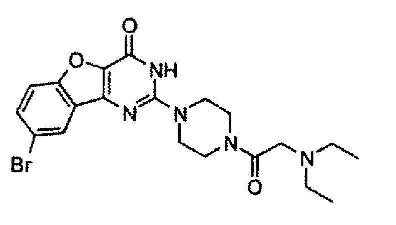 | 8-Brom-2-[4-(N,N-diethylglycyl)piperazin-1-yl][1]benzofuro[3,2-d]pyrimidin-4(3H)-on |
| 246 | 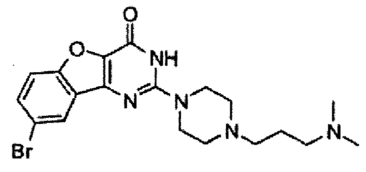 | 8-Brom-2-{4-[3-(dimethylamino)propyl]piperazin-1-yl}[1]benzofuro[3,2-d]pyrimidin-4(3H)-on |
| 247 | 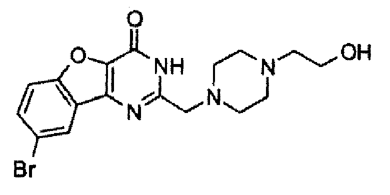 | 8-Brom-2-{[4-(2-hydroxyethyl)piperazin-1- yl]methyl}[1]benzofuro[3,2-d]pyrimidin-4(3H)-on |
| 252 | 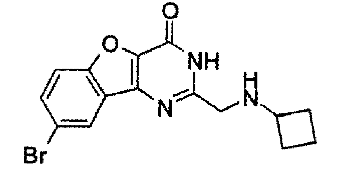 | 8-Brom-2-[(cyclobutylamino)methyl][1] benzofuro[3,2-d]pyrimidin-4(3H)-on |
| 253 | 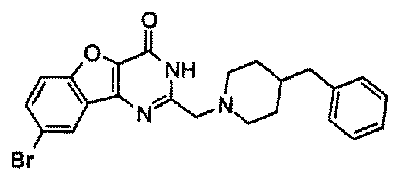 | 8-Brom-2-{[4-(phenylmethyl)piperidin-1-yl]methyl}[1]benzofuro[3,2-d]pyrimidin-4(3H)-on |
| 254 | 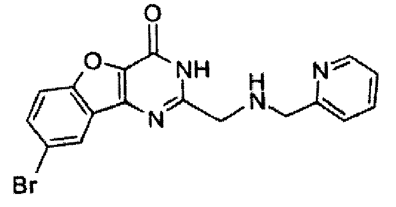 | 8-Brom-2-{[(pyridin-2-ylmethyl)amino]methyl}[1]]benzofuro [3,2-d]pyrimidin-4(3H)-on |
| 255 | 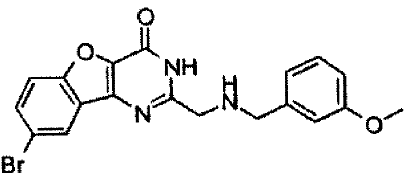 | 8-Brom-2-[({[3-(methyloxy)phenyl]methyl}amino) methyl][1]benzofuro[3,2-d]pyrimidin-4(3H)-on |
| 256 | 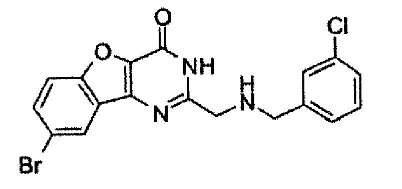 | 8-Brom-2-({[(3-chlorphenyl)methyl]amino}methyl)[1] benzofuro[3,2-d]pyrimidin-4(3H)-on |
| 257 | 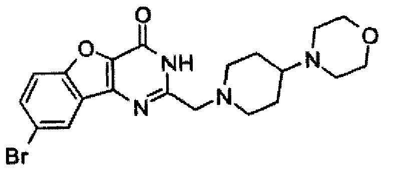 | 8-Brom-2-[(4-morpholin-4-ylpiperidin-1-yl)methyl][1]benzofuro[3,2-d]pyrimidin-4(3H)-on |
| 258 | 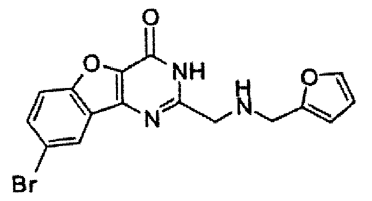 | 8-Brom-2-{[(furan-2-ylmethyl)amino]methyl}[1]benzofuro [3,2-d]pyrimidin-4(3H)-on |
| 259 | 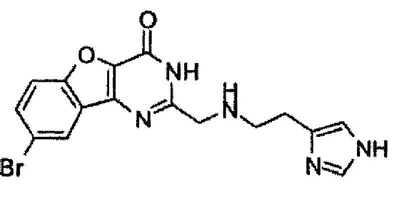 | 8-Brom-2-({[2-(1H-imidazol-4-yl)ethyl]amino}methyl)[1]benzofuro [3,2-d]pyrimidin-4(3H)-on |
| 260 | 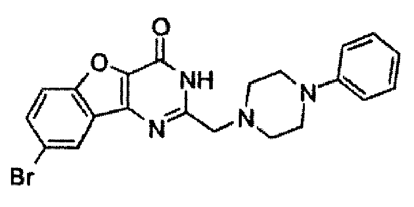 | 8-Brom-2-[(4-phenylpiperazin-1-yl)methyl][1]benzofuro[3,2-d]pyrimidin-4(3H)-on |
| 261 | 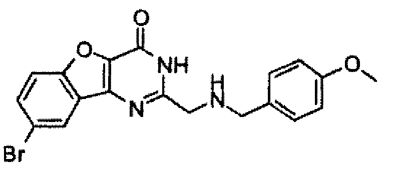 | 8-Brom-2-[({[4-(methyloxy)phenyl]methyl}amino) methyl][1]benzofuro[3,2-d]pyrimidin-4(3H)-on |
| 262 | 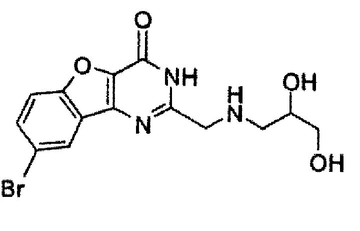 | 8-Brom-2-{[(2,3-dihydroxypropyl)amino]methyl}[1] benzofuro[3,2-d]pyrimidin-4(3H)-on |
| 264 | 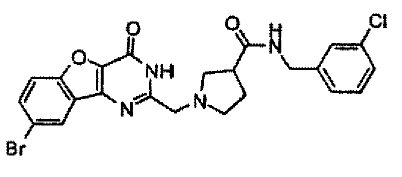 | 1-[(8-Brom-4-oxo-3,4-dihydro[1]benzofuro[3,2-d]pyrimidin-2-yl)methyl]-N-[(3-chlorphenyl)methyl]pyrrolidin-3-carboxamid |
| 266 | 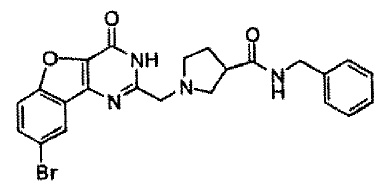 | 1-[(8-Brom-4-oxo-3,4-dihydro[1]benzofuro[3,2-d]pyrimidin-2-yl)methyl]-N-(phenylmethyl)pyrrolidin-3-carboxamid |
| 267 | 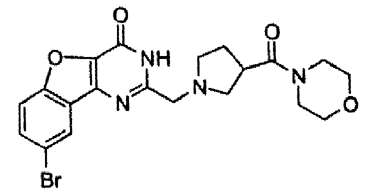 | 8-Brom-2-{[3-(morpholin-4-ylcarbonyl)pyrrolidin-1-yl]methyl}[1]benzofuro[3,2-d]pyrimidin-4(3H)-on |
| 268 | 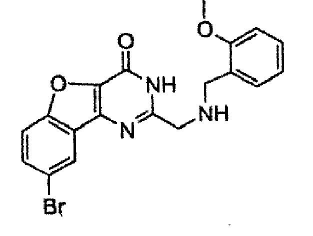 | 8-Brom-2-[({[2-(methyloxy)phenyl]methyl}amino) methyl][1]benzofuro[3,2-d]pyrimidin-4(3H)-on |
| 269 | 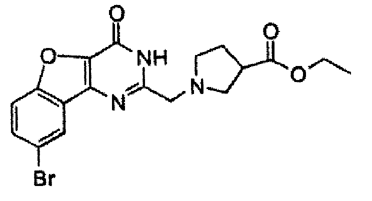 | Ethyl-1-[(8-brom-4-oxo-3,4-dihydro[1]benzofuro[3,2-d]pyrimidin-2-yl)methyl]pyrrolidin-3-carboxylat |
| 270 | 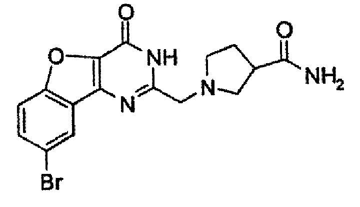 | 1-[(8-Brom-4-oxo-3,4-dihydro[1]benzofuro[3,2-d]pyrimidin-2-yl)methyl]pyrrolidin-3-carboxamid |
| 271 | 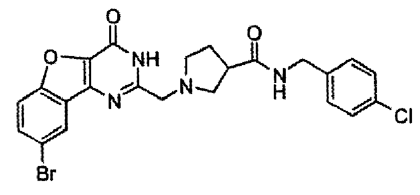 | 1-[(8-Brom-4-oxo-3,4-dihydro[1]benzofuro[3,2-d]pyrimidin-2-yl)methyl]-N-[(4-chlorphenyl)methyl]pyrrolidin-3-carboxamid |
| 272 | 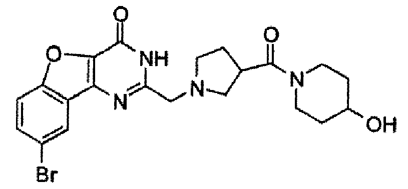 | 8-Brom-2-({3-[(4-hydroxypiperidin-1-yl)carbonyl]pyrrolidin-1-yl}methyl)[1]benzofuro[3,2-d]pyrimidin-4(3H)-on |
| 273 | 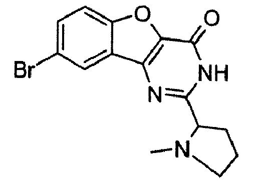 | 8-Brom-2-(1-methylpyrrolidin-2-yl)[1]benzofuro[3,2-d]pyrimidin-4(3H)-on |
| 277 | 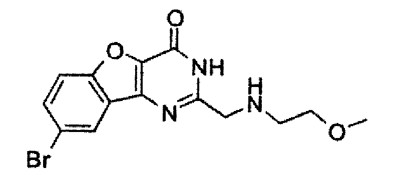 | 8-Brom-2-({[2-(methyloxy)ethyl]amino}methyl)[1] benzofuro[3,2-d]pyrimidin-4(3H)-on |
| 278 | 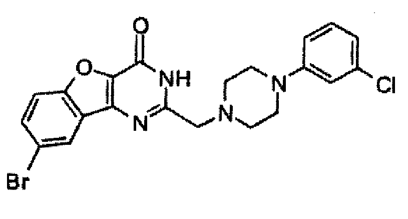 | 8-Brom-2-{[4-(3-chlorphenyl)piperazin-1-yl]methyl}[1]benzofuro[3,2-d]pyrimidin-4(3H)-on |
| 286 | 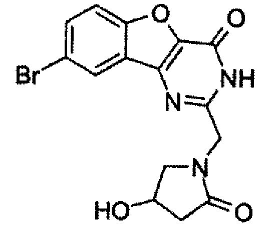 | 8-Brom-2-[(4-hydroxy-2-oxopyrrolidin-1-yl)methyl][1]benzofuro[3,2-d]pyrimidin-4(3H)-on |
| 287 | 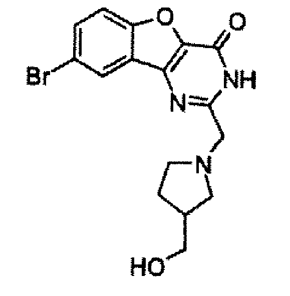 | 8-Brom-2-{[3-(hydroxymethyl)pyrrolidin-1-yl]methyl}[1]benzofuro[3,2-d]pyrimidin-4(3H)-on |
| 288 | 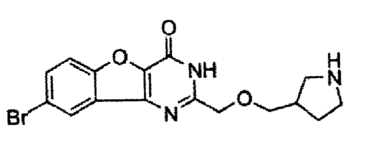 | 8-Brom-2-{[(pyrrolidin-3-ylmethyl)oxy]methyl}[1]benzofuro[3, 2-d]pyrimidin-4(3H)-on |
| 289 | 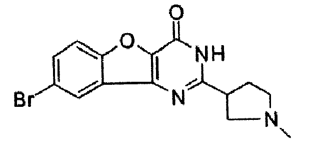 | 8-Brom-2-(1-methylpyrrolidin-3-yl)[1]benzofuro[3,2-d]pyrimidin-4(3H)-on |
| 290 | 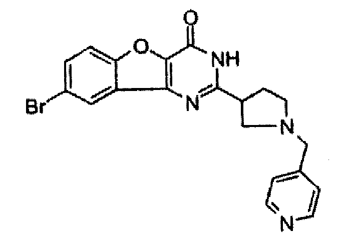 | 8-Brom-2-[1-(pyridin-4-ylmethyl)pyrrolidin-3-yl][1]benzofuro[3,2-d]pyrimidin-4(3H)-on |
| 295 | 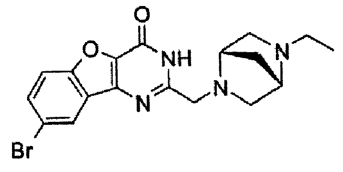 | 8-Brom-2-{[(1S,4S)-5-ethyl-2,5-diazabicyclo[2.2.1]hept-2-yl]methyl}[1]benzofuro[3,2-d]pyrimidin-4(3H)-on |
| 298 | 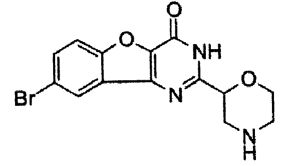 | 8-Brom-2-morpholin-2-yl[1]benzofuro[3,2-d]pyrimidin-4(3H)-on |
| 304 | 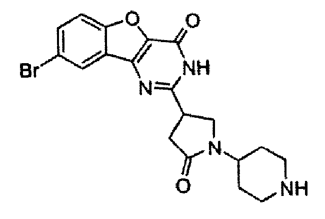 | 8-Brom-2-(5-oxo-1-piperid in-4-ylpyrrolidin-3-yl)[1]benzofuro[3,2-d]pyrimidin-4(3H)-on |
| 305 | 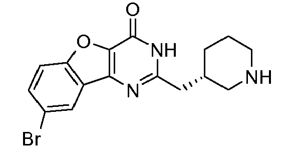 | 8-Brom-2-[(3S)-piperidin-3-ylmethyl][1]benzofuro[3,2-d]pyrimidin-4(3H)-on |
| 306 | 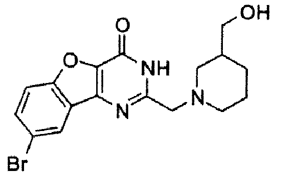 | 8-Brom-2-{[3-(hydroxymethyl)piperidin-1-yl]methyl}[1]benzofuro[3,2-d]pyrimidin-4(3H)-on |
| 307 | 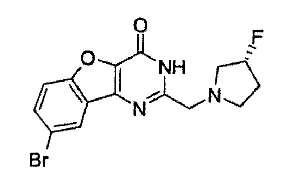 | 8-Brom-2-{[(3R)-3-fluorpyrrolidin-1-yl]methyl}[1]benzofuro[3,2-d]pyrimidin-4(3H)-on |
| 308 | 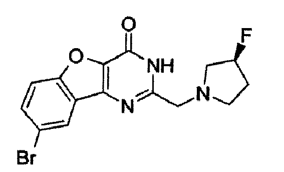 | 8-Brom-2-{[(3S)-3-fluorpyrrolidin-1 yl]methyl}[1]benzofuro[3,2-d]pyrimidin-4(3H)-on |
| 309 | 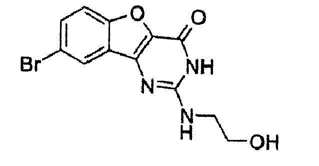 | 8-Brom-2-[(2-hydroxyethyl)amino][1]benzofuro[3,2 -d]pyrimidin-4(3H)-on |
| 311 | 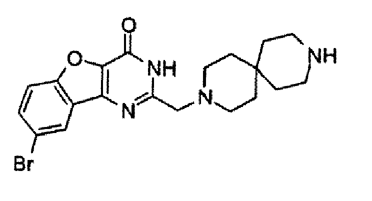 | 8-Brom-2-(3,9-diazaspiro[5.5]undec-3-ylmethyl)[1]benzofuro[3,2-d]pyrimidin-4(3H)-on |
| 315 | 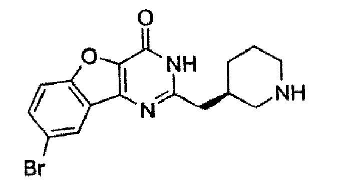 | 8-Brom-2-[(3R)-piperidin-3-ylmethyl][1]benzofuro[3,2-d]pyrimidin-4(3H)-on |
| 317 | 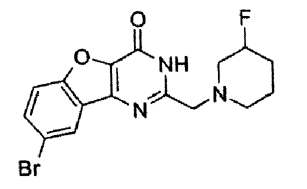 | 8-Brom-2-[(3-fluorpiperidin-1-yl)methyl][1]benzofuro[3,2-d]pyrimidin-4(3H)-on |
| 318 | 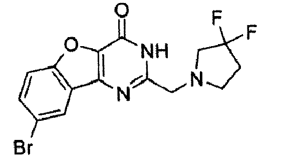 | 8-Brom-2-[(3,3-difluorpyrrolidin-1-yl)methyl][1]benzofuro[3,2-d]pyrimidin-4(3H)-on |
| 330 | 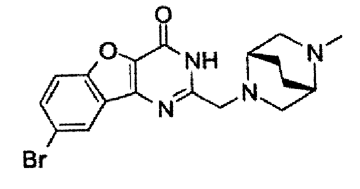 | 8-Brom-2-{[(1S,4S)-5-methyl-2,5-diazabicyclo[2.2.2]oct-2-yl]methyl}[1]benzofuro[3,2-d]pyrimidin-4(3H)-on |
| 331 | 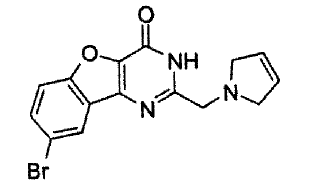 | 8-Brom-2-((2,5-dihydro-1H-pyrrol-1-yl)methyl)benzofuro[3,2-d]pyrimidin-4(3H)-on |
| 332 | 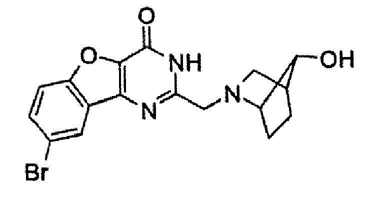 | 8-Brom-2-((7-hydroxy-2-azabicyclo[2.2.1]heptan-2-yl)methyl)benzofuro[3,2-d]pyrimidin-4(3H)-on |
| 344 | 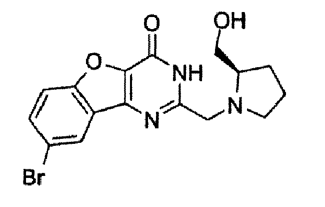 | 8-Brom-2-{[(2R)-2-(hydroxymethyl)pyrrolidin-1-yl]methyl}[1]benzofuro[3,2-d]pyrimidin-4(3H)-on |
| 346 | 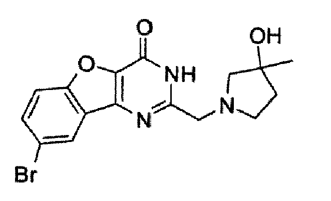 | 8-Brom-2-[(3-hydroxy-3-methylpyrrolidin-1-yl)methyl][1]benzofuro[3,2-d]pyrimidin-4(3H)-on |
| 366 | 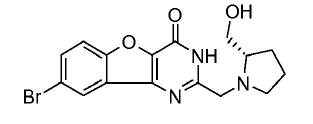 | 8-Brom-2-{[(2S)-2-(hydroxymethyl)pyrrolidin-1-yl]methyl}[1]benzofuro[3,2-d]pyrimidin-4(3H)-on |
| 367 | 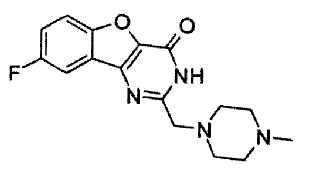 | 8-Fluor-2-[(4-methylpiperazin-1-yl)methyl][1]benzofuro[3,2-d]pyrimidin-4(3H)-on |
| 368 | 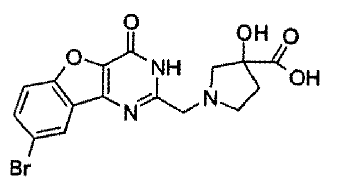 | 1-[(8-Brom-4-oxo-3,4-dihydro[1]benzofuro[3,2-d]pyrimidin-2-yl)methyl]-3-hydroxypyrrolidin-3-carbonsäure |
| 369 | 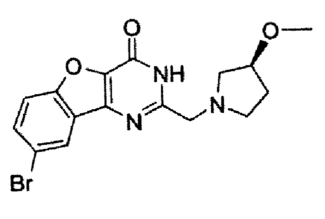 | 8-Brom-2-{[(3S)-3-(methyloxy)pyrrolidin-1-yl]methyl}[1]benzofuro[3,2-d]pyrimidin-4(3H)-on |
| 370 | 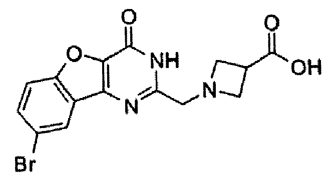 | 1-[(8-Brom-4-oxo-3,4-dihydro[1]benzofuro[3,2-d]pyrimidin-2-yl)methyl]azetidin-3-carbonsäure |
| 378 | 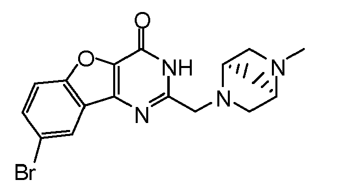 | 8-Brom-2-(((1R,4R)-5-methyl-2,5-diazabicyclo[2.2.1]heptan-2-yl)methyl)benzofuro[3,2-d]pyrimidin-4(3H)-on |
| 388 | 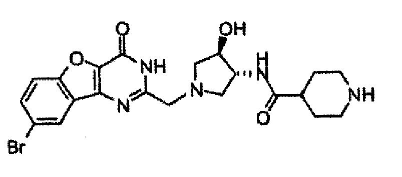 | N-{(3R,4R)-1-[(8-Brom-4-oxo-3,4- dihydro[1]benzofuro[3,2-d]pyrimidin-2-yl)methyl]-4-hydroxypyrrolidin-3-yl}piperidin-4-carboxamid |
| 398 | 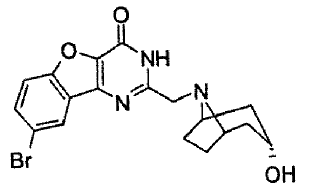 | 8-Brom-2-[(3-hydroxy-8-azabicyclo[3.2.1]oct-8-yl)methyl][1]benzofuro[3,2-d]pyrimidin-4(3H)-on |
| 400 | 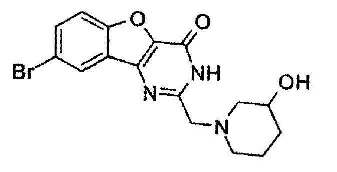 | 8-Brom-2-[(3-hydroxypiperidin-1-yl)methyl][1]benzofuro[3,2-d]pyrimidin-4(3H)-on |
| 401 | 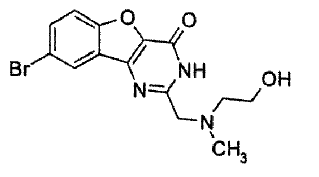 | 8-Brom-2-{[(2-hydroxyethyl)(methyl)amino]methyl} [1]benzofuro[3,2-d]pyrimidin-4(3H)-on |
| 402 | 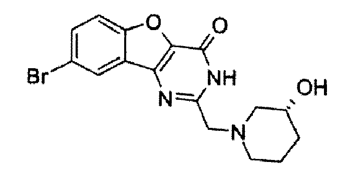 | 8-Brom-2-{[(3R)-3-hydroxypiperidin-1-yl]methyl}[1]benzofuro[3,2-d]pyrimidin-4(3H)-on |
| 403 | 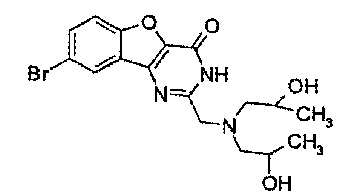 | 2-{[Bis(2-hydroxypropyl)amino]methyl}-8-brom[1]benzofuro[3,2-d]pyrimidin-4(3H)-on |
| 404 | 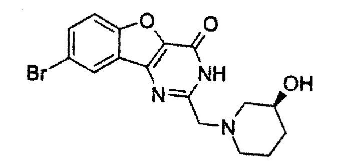 | 8-Brom-2-{[(3S)-3-hydroxypiperidin-1-yl]methyl}[1]benzofuro[3,2-d]pyrimidin-4(3H)-on |
| 407 | 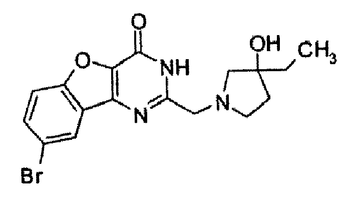 | 8-Brom-2-[(3-ethyl-3-hydroxypyrrolidin-1-yl)methyl][1]benzofuro[3,2-d]pyrimidin-4(3H)-on |
| 408 | 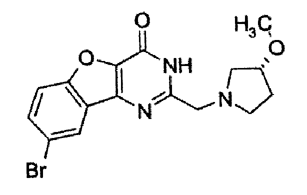 | 8-Brom-2-{[(3R)-3-(methyloxy)pyrrolidin-1-yl]methyl}[1]benzofuro[3,2-d]pyrimidin-4(3H)-on |
| 409 | 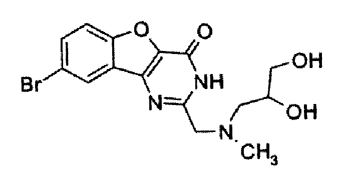 | 8-Brom-2-{[(2,3-dihydroxypropyl)(methyl)amino] methyl}[1]benzofuro[3,2-d]pyrimidin-4(3H)-on |
| 410 | 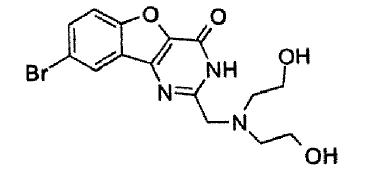 | 2-{[Bis(2-hydroxyethyl)amino]methyl}-8-brom[1]benzofuro[3,2-d]pyrimidin-4(3H)-on |
| 412 | 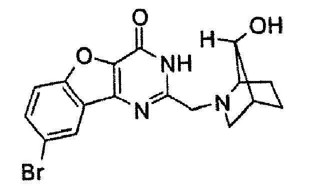 | 8-Brom-2-[(7-hydroxy-2-azabicyclo[2.2.1]hept-2-yl)methyl][1]benzofuro[3,2-d]pyrimidin-4(3H)-on |
| 413 | 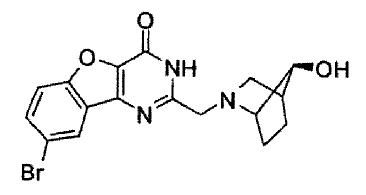 | 8-Brom-2-{[(7S)-7-hydroxy-2-azabicyclo[2.2.1]hept-2-yl]methyl}[1]benzofuro[3,2-d]pyrimidin-4(3H)-on |
| 417 | 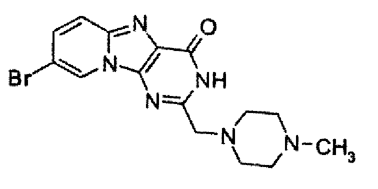 | 8-Brom-2-[(4-methylpiperazin-1-yl)methyl]pyrido[1,2-e]purin-4(3H)-on |
| 418 | 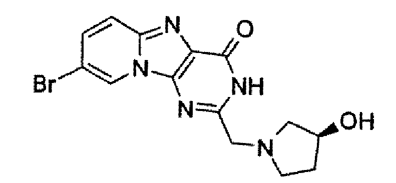 | 8-Brom-2-{[(3S)-3-hydroxypyrrolidin-1-yl]methyl}pyrido[1,2-e]purin-4(3H)-on |
| 420 | 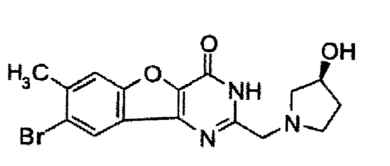 | 8-Brom-2-{[(3S)-3-hydroxypyrrolidin-1-yl]methyl}-7 -methyl[1]benzofuro[3,2-d]pyrimidin-4(3H)-on |
| 424 | 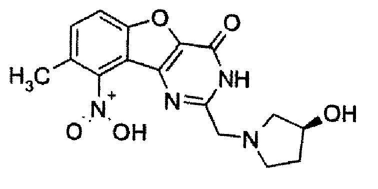 | Hydroxy(2-{[(3S)-3-hydroxypyrrolidin-1-yl]methyl}-8-methyl-4-oxo-3,4-dihydro[1]benzofuro[3,2-d]pyrimidin-9-yl)oxoammonium |
| 425 | 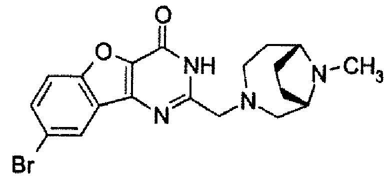 | 8-Brom-2-{[(1S,6R)-9-methyl-3,9-diazabicyclo[4.2.1]non-3-yl]methyl}[1]benzofuro[3,2-d]pyrimidin-4(3H)-on |
| 426 | 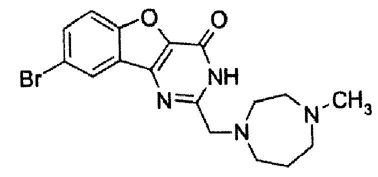 | 8-Brom-2-[(4-methyl-1,4-diazepan-1-yl)methyl][1]benzofuro[3,2-d]pyrimidin-4(3H)-on |
| 428 | 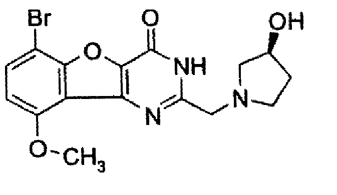 | 6-Brom-2-{[(3S)-3-hydroxypyrrolidin-1-yl]methyl}-9-(methyloxy)[1]benzofuro[3,2-d]pyrimidin-4(3H)-on |
| 429 | 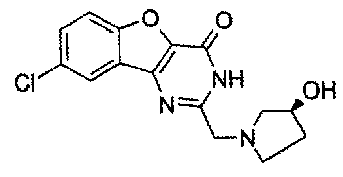 | 8-Ch lor-2-{[(3S)-3-hydroxypyrrolidin-1-yl]methyl}[1]benzofuro[3,2-d]pyrimidin-4(3H)-on |
| 431 | 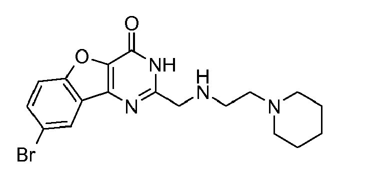 | 8-Brom-2-{[(2-piperidin-1 -ylethyl)amino]methyl}[1]benzofuro[3, 2-d]pyrimidin-4(3H)-on |
| 432 | 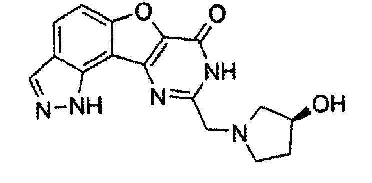 | 9-{[(3S)-3-Hydroxypyrrolidin-1 -yl]methyl}-1H-pyrimido[4',5':4,5]furo[2,3-g]indazol-7(8H)-on |
| 433 | 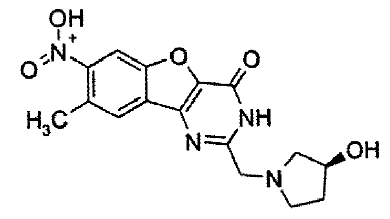 | Hydroxy(2-{[(3S)-3-hydroxypyrrolidin-1-yl]methyl}-8-methyl-4-oxo-3,4-dihydro[1]benzofuro[3,2-d]pyrimidin-7-yl)oxoammonium |
| 434 | 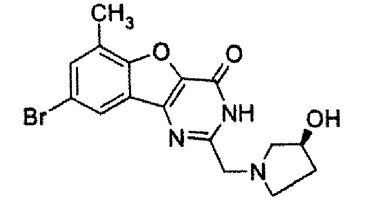 | 8-Brom-2-{[(3S)-3-hydroxypyrrolidin-1-yl]methyl}-6-methyl[1]benzofuro[3,2-d]pyrimidin-4(3H)-on |
| 436 | 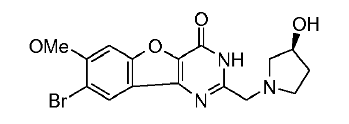 | 8-Brom-2-{[(3S)-3-hydroxypyrrolidin-1-yl]methyl}-7-(methyloxy)[1]benzofuro[3,2-d]pyrimidin-4(3H)-on |
| 437 | 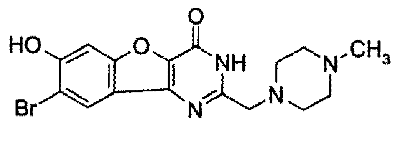 | 8-Brom-7-hydroxy-2-{[(3S)-3-hydroxypyrrolidin-1 -yl]methyl}[1]benzofuro[3,2-d]pyrimidin-4(3H)-on |
| 438 | 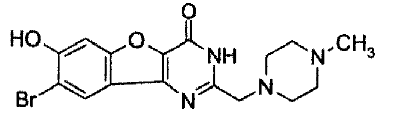 | 8-Brom-7-hydroxy-2-[(4-methylpiperazin-1-yl)methyl][1]benzofuro[3,2-d]pyrimidin-4(3H)-on |
| 440 | 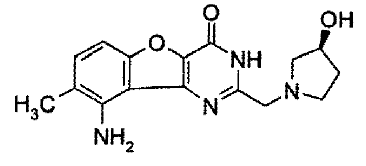 | 9-Amino-2-{[(3S)-3-hydroxypyrrolidin-1-yl]methyl}-8-methyl[1]benzofuro[3,2-d]pyrimidin-4(3H)-on |
| 442 | 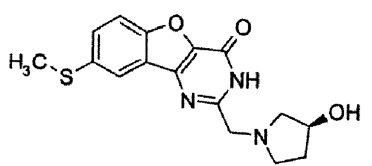 | 2-{[(3S)-3-Hydroxypyrrolidin-1 -yl]methyl}-8-(methylthio)[1]benzofuro[3,2-d]pyrimidin-4(3H)-on |
| 443 | 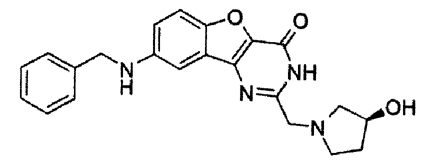 | 2-{[(3S)-3-Hydroxypyrrolidin-1 -yl]methyl}-8-[(phenylmethyl)amino][1]benzofuro [3,2-d]pyrimidin-4(3H)-on |
| 444 | 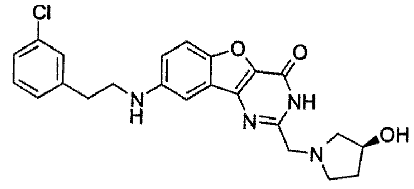 | 8-{[2-(3-Chlorphenyl)ethyl]amino}-2-{[(3S)-3-hydroxypyrrolidin-1-yl]methyl}[1]benzofuro[3,2-d]pyrimidin-4(3H)-on |
| 445 | 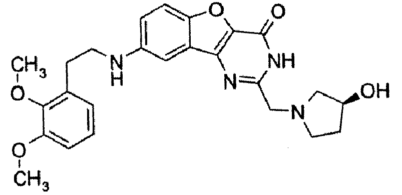 | 8-({2-[2,3-Bis(methyloxy)phenyl]ethyl}amino)-2-{[(3S)-3-hydroxypyrrolidin-1 -yl]methyl}[1]benzofuro[3,2-d]pyrimidin-4(3H)-on |
| 446 | 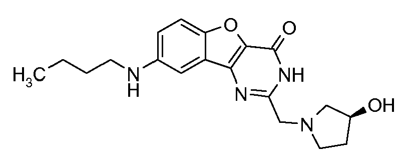 | 8-(Butylamino)-2-{[(3S)-3-hyd roxypyrrol id in-1-yl]methyl}[1]benzofuro[3,2-d]pyrimidin-4(3H)-on |
| 447 | 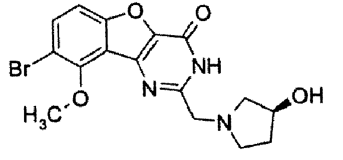 | 8-Brom-2-{[(3S)-3-hydroxypyrrolidin-1-yl]methyl}-9-(methyloxy)[1]benzofuro[3,2-d]pyrimidin-4(3H)-on |
| 448 | 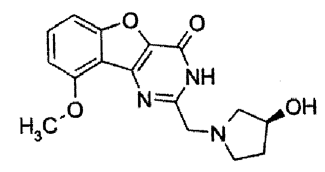 | 2-{[(3S)-3-Hydroxypyrrolidin-1 -yl]methyl}-9-(methyloxy)[1]benzofuro[3,2-d]pyrimidin-4(3H)-on |
| 449 | 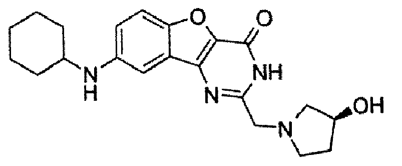 | 8-(Cyclohexylamino)-2-{[(3S)-3-hydroxypyrrolid in-1-yl]methyl}[1]benzofuro[3,2-d]pyrimidin-4(3H)-on |
| 450 | 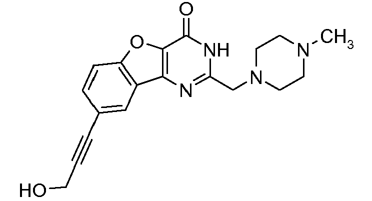 | 8-(3-Hydroxyprop-1-in-1-yl)-2-[(4-methylpiperazin-1-yl)methyl][1]benzofuro[3,2-d]pyrimidin-4(3H)-on |
| 451 | 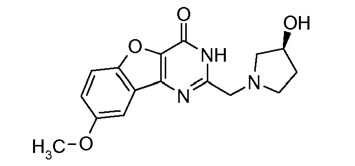 | 2-{[(3S)-3-Hydroxypyrrolidin-1 -yl]methyl}-8-(methyloxy)[1]benzofuro[3,2-d]pyrimidin-4(3H)-on |
| 452 | 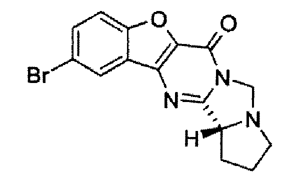 | (13bR)-11-Brom-1,2,3,13b-tetrahydro-7H-[1]benzofuro[3,2-d]pyrrolo[1',2':3,4]imidazo[1,5-a]pyrimidin-7-on |
| 453 | 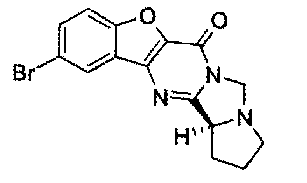 | (13bS)-11-Brom-1,2,3,13b-tetrahydro-7H-[1]benzofuro[3,2-d]pyrrolo[1',2':3,4]imidazo[1,5-a]pyrimidin-7-on |
| 454 | 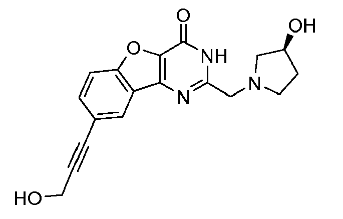 | 8-(3-Hydroxyprop-1-in-1-yl)-2-{[(3S)-3-hydroxypyrrolidin-1 -yl]methyl}[1]benzofuro[3,2-d]pyrimidin-4(3H)-on |
| 455 | 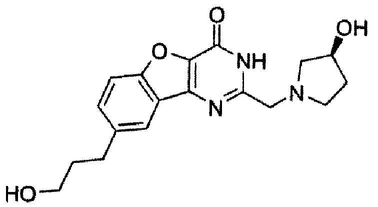 | 8-(3-Hydroxypropyl)-2-{[(3S)-3-hydroxypyrrolidin-1 -yl]methyl}[1]benzofuro[3,2-d]pyrimidin-4(3H)-on |
| 456 | 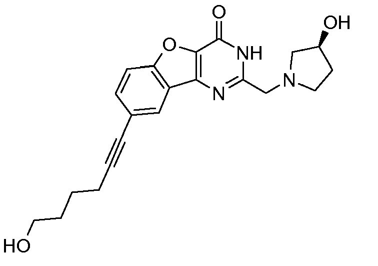 | 8-(6-Hydroxyhex-1-in-1-yl)-2-{[(3S)-3-hydroxypyrrolidin-1 -yl]methyl}[1]benzofuro[3,2-d]pyrimidin-4(3H)-on |
| 457 | 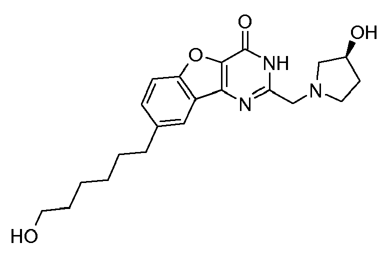 | 8-(6-Hydroxyhexyl)-2-{[(3S)-3-hyd roxypyrrol id in-1-yl]methyl}[1]benzofuro[3,2-d]pyrimidin-4(3H)-on |
| 458 | 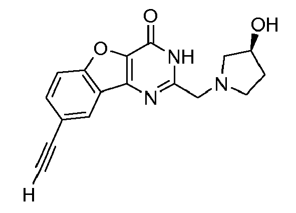 | 8-Ethinyl-2-{[(3S)-3-hydroxypyrro idin-1-yl]methyl}[1]benzofuro[3,2-d]pyrimidin-4(3H)-on |
| 459 | 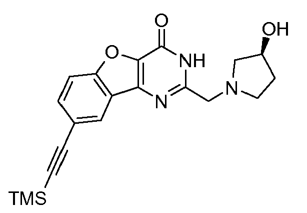 | 2-{[(3S)-3-Hydroxypyrrolidin-1 -yl]methyl}-8-[(trimethylsilyl)ethinyl][1]benzofuro [3,2-d]pyrimidin-4(3H)-on |
| 460 | 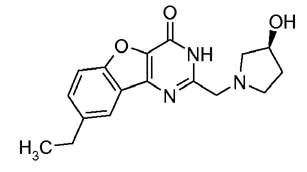 | 8-Ethyl-2-{[(3S)-3-hydroxypyrrolidin- 1-yl]methyl}[1]benzofuro[3,2-d]pyrimidin-4(3H)-on |
| 461 | 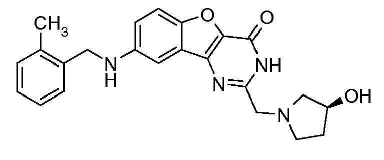 | 2-{[(3S)-3-Hydroxypyrrolidin-1 -yl]methyl}-8-{[(2-methylphenyl)methyl]amino}[1] benzofuro[3,2-d]pyrimidin-4(3H)-on |
| 462 | 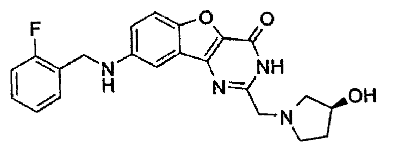 | 8-{[(2-Fluorphenyl)methyl]amino}-2-{[(3S)-3-hydroxypyrrolidin-1 -yl]methyl}[1]benzofuro[3,2-d]pyrimidin-4(3H)-on |
| 463 | 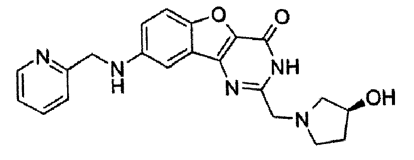 | 2-{[(3S)-3-Hydroxypyrrolidin-1 -yl]methyl}-8-[(pyridin-2-ylmethyl)amino][1]benzofuro[3,2-d]pyrimidin-4(3H)-on |
| 464 | 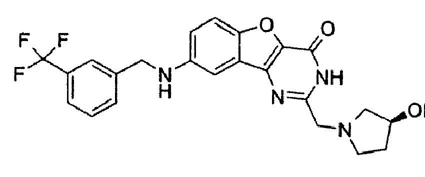 | 2-{[(3S)-3-Hydroxypyrrolidin-1 -yl]methyl}-8-({[3-(trifluormethyl)phenyl]methyl}amino) [1]benzofuro[3,2-d]pyrimidin-4(3H)-on |
| 465 | 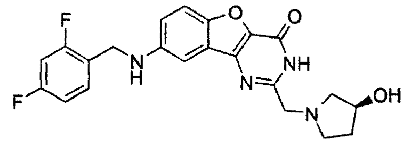 | 8-{[(2,4-Difluorphenyl)methyl]amino}-2-{[(3S)-3-hydroxypyrrolidin-1 - yl]methyl}[1]benzofuro[3,2-d]pyrimidin-4(3H)-on |
| 466 | 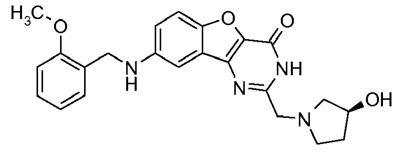 | 2-{[(3S)-3-Hydroxypyrrolidin-1 -yl]methyl}-8-({[2-(methyloxy)phenyl]methyl}amino)[1] benzofuro[3,2-d]pyrimidin-4(3H)-on |
| 467 | 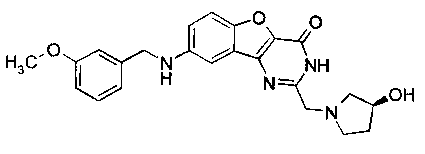 | 2-{[(3S)-3-Hydroxypyrrolidin-1 -yl]methyl}-8-({[3-(methyloxy)phenyl]methyl}amino)[1] benzofuro[3,2-d]pyrimidin-4(3H)-on |
| 468 | 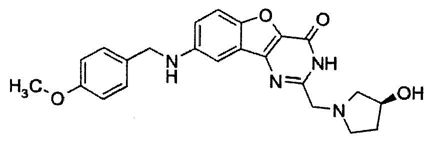 | 2-{[(3S)-3-Hydroxypyrrolidin-1 -yl]methyl}-8-({[4-(methyloxy)phenyl]methyl}amino)[1] benzofuro[3,2-d]pyrimidin-4(3H)-on |
| 469 | 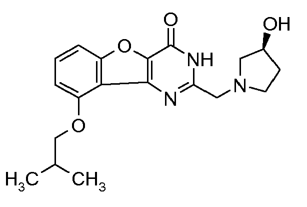 | 2-{[(3S)-3-Hydroxypyrrolidin-1-yl]methyl}-9-[(2-methylpropyl)oxy][1]benzofuro[3,2-d]pyrimidin-4(3H)-on |
| 470 | 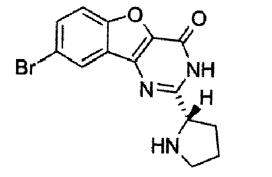 | 8-Brom-2-[(2S)-pyrrolidin-2-yl][1]benzofuro[3,2-d]pyrimidin-4(3H)-on |
| 471 | 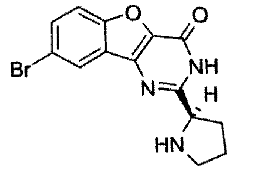 | 8-Brom-2-[(2R)-pyrrolidin-2-yl][1]benzofuro[3,2-d]pyrimidin-4(3H)-on |
| 472 | 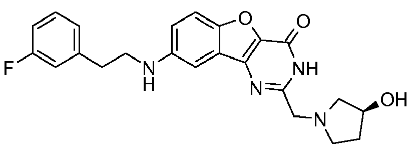 | 8-{[2-(3-Fluorphenyl)ethyl]amino}-2-{[(3S)-3-hydroxypyrrolidin-1-yl]methyl}[1 ]benzofuro[3,2-d]pyrimidin-4(3H)-on |
| 473 | 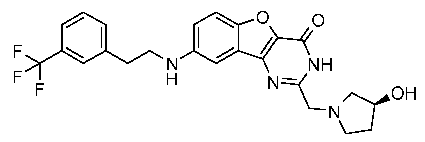 | 2-{[(3S)-3-Hydroxypyrrolidin-1-yl]methyl}-8-({2-[3-(trifluormethyl)phenyl]ethyl}amino)[1] benzofuro[3,2-d]pyrimidin-4(3H)-on |
| 474 | 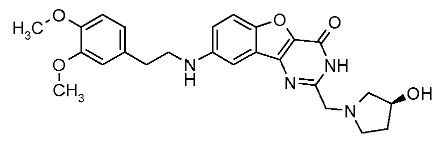 | 8-({2-[3,4-Bis(methyloxy)phenyl]ethyl}amino)-2-{[(3S)-3-hydroxypyrrolidin-1 -yl]methyl}[1]benzofuro[3,2-d]pyrimidin-4(3H)-on |
| 475 | 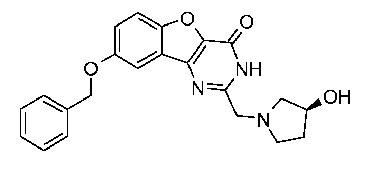 | 2-{[(3S)-3-Hydroxypyrrolidin-1-yl]methyl}-8-[(phenylmethyl)oxy][1]benzofuro[3,2-d]pyrimidin-4(3H)-on |
| 476 | 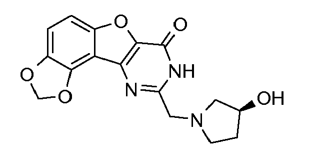 | 9-{[(3S)-3-Hydroxypyrrolidin-1 -yl]methyl}[1,3]dioxolo[4,5][1] benzofuro[3,2-d]pyrimidin-7(8H)-on |
| 477 | 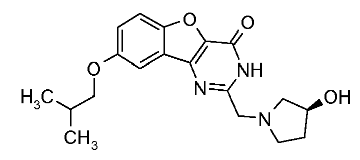 | 2-{[(3S)-3-Hydroxypyrrolidin-1 -yl]methyl}-8-[(2-methylpropyl)oxy][1]benzofuro[3,2-d]pyrimidin-4(3H)-on |
| 488 | 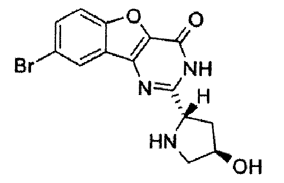 | 8-Brom-2-[(2S,4R)-4-hydroxypyrrolidin-2-yl][1]benzofuro[3,2-d]pyrimidin-4(3H)-on |
| 489 | 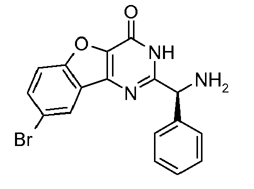 | 2-[(S)-Amino(phenyl)methyl]-8-brom[1]benzofuro[3,2-d]pyrimidin-4(3H)-on |
| 490 | 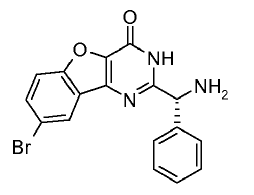 | 2-[(R)-Amino(phenyl)methyl]-8-brom[1]benzofuro[3,2-d]pyrimidin-4(3H)-on |
| 491 | 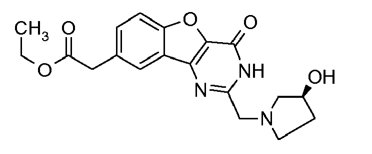 | Ethyl-(2-{[(3S)-3-hydroxypyrrolidin-1-yl]methyl}-4-oxo-3,4-dihydro[1]benzofuro[3,2-d]pyrimidin-8-yl)acetat |
| 492 | 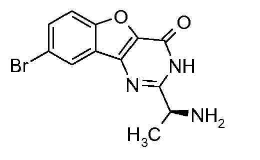 | 2-[(1S)-1-Aminoethyl]-8-brom[1]benzofuro[3,2-d]pyrimidin-4(3H)-on |
| 493 | 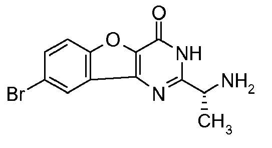 | 2-[(1 R)-1-Aminoethyl]-8-brom[1]benzofuro[3,2-d]pyrimidin-4(3H)-on |
| 494 | 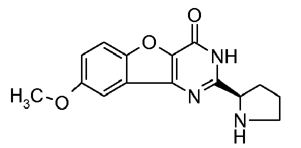 | 8-(Methyloxy)-2-[(2R)-pyrrolidin-2-yl][1]benzofuro[3,2-d]pyrimidin-4(3H)-on |
| 495 | 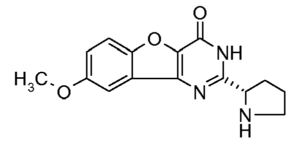 | 8-(Methyloxy)-2-[(2S)-pyrrolidin-2-yl][1]benzofuro[3,2-d]pyrimidin-4(3H)-on |
| 496 | 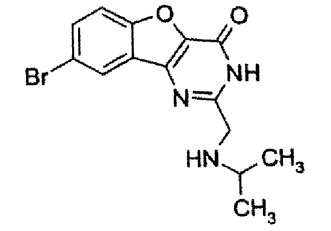 | 8-Brom-2-{[(1-methylethyl)amino]methyl}[1] benzofuro[3,2-d]pyrimidin-4(3H)-on |
| 498 | 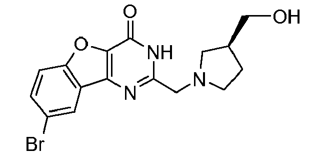 | 8-Brom-2-{[(3S)-3-(hydroxymethyl)pyrrolidin-1-yl]methyl}[1]benzofuro[3,2-d]pyrimidin-4(3H)-on |
| 499 | 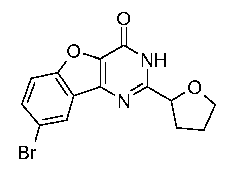 | 8-Brom-2-(tetrahydrofuran-2-yl)[1]benzofuro[3,2-d]pyrimidin-4(3H)-on |
| 500 | 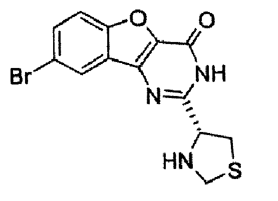 | 8-Brom-2-[(4R)-1,3-thiazolidin-4-yl][1]benzofuro[3,2-d]pyrimidin-4(3H)-on |
| 501 | 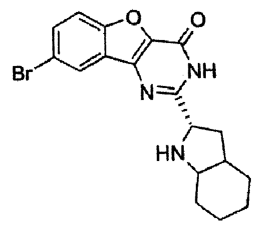 | 8-Brom-2-[(2S)-octahydro-1H-indol-2-yl][1]benzofuro[3,2-d]pyrimidin-4(3H)-on |
| 502 | 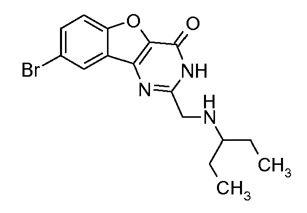 | 8-Brom-2-{[(1-ethylpropyl)amino]methyl}[1] benzofuro[3,2-d]pyrimidin-4(3H)-on |
| 503 | 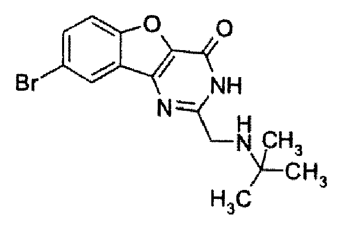 | 8-Brom-2-{[(1,1-dimethylethyl)amino]methyl}[1] benzofuro[3,2-d]pyrimidin-4(3H)-on |
| 505 | 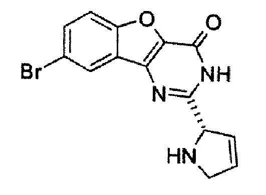 | 8-Brom-2-[(2S)-2,5-dihydro-1H-pyrrol-2-yl][1]benzofuro[3,2-d]pyrimidin-4(3H)-on |
| 506 | 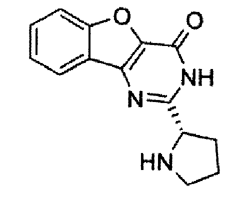 | 2-[(2S)-Pyrrolidin-2-yl][1]benzofuro[3,2-d]pyrimidin-4(3H)-on |
| 507 | 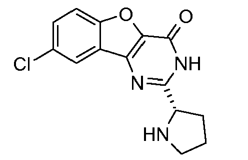 | 8-Chlor-2-[(2S)-pyrrolidin-2-yl][1]benzofuro[3,2-d]pyrimidin-4(3H)-on |
| 508 | 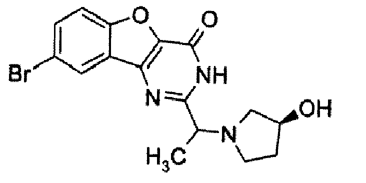 | 8-Brom-2-{1-[(3S)-3-hyd roxypyrrol id in-1-yl]ethyl}[1]benzofuro[3,2-d]pyrimidin-4(3H)-on |
| 509 | 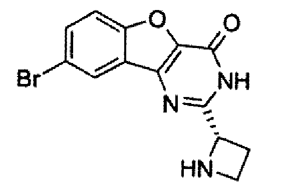 | 2-[(2S)-Azetidin-2-yl]-8-brom[1]benzofuro[3,2-d]pyrimidin-4(3H)-on |
| 511 | 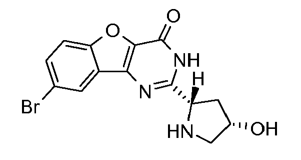 | 8-Brom-2-[(2S,4S)-4-hydroxypyrrolidin-2-yl][1]benzofuro[3,2-d]pyrimidin-4(3H)-on |
| 512 | 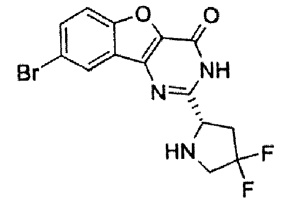 | 8-Brom-2-[(2S)-4,4-difluorpyrrolidin-2-yl][1]benzofuro[3,2-d]pyrimidin-4(3H)-on |
| 513 | 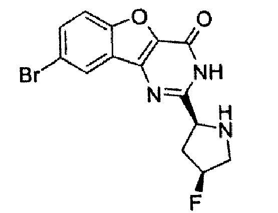 | 8-Brom-2-[(2S,4S)-4-fluorpyrrolidin-2-yl][1]benzofuro[3,2-d]pyrimidin-4(3H)-on |
| 514 | 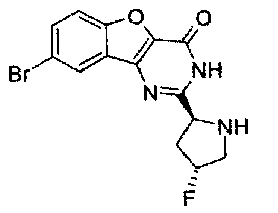 | 8-Brom-2-[(2S,4R)-4-fluorpyrrolidin-2-yl][1]benzofuro[3,2-d]pyrimidin-4(3H)-on |
| 516 | 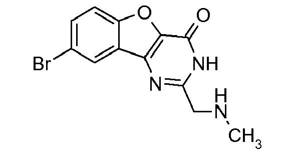 | 8-Brom-2-[(methylamino)methyl][1]benzofuro [3,2-d]pyrimidin-4(3H)-on |
| 517 | 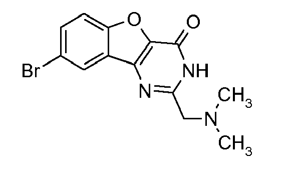 | 8-Brom-2-[(dimethylamino)methyl][1]benzofuro [3,2-d]pyrimidin-4(3H)-on |
| 518 | 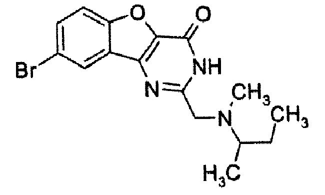 | 8-Brom-2-{[methyl(1-methylpropyl)amino]methyl}[1] benzofuro[3,2-d]pyrimidin-4(3H)-on |
| 519 | 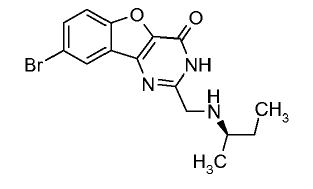 | 8-Brom-2-({[(1 R)-1-methylpropyl]amino}methyl)[1] benzofuro[3,2-d]pyrimidin-4(3H)-on |
| 520 | 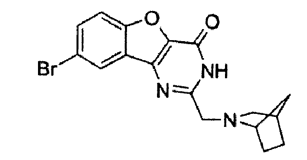 | 2-(2-Azabicyclo[2.2.1]hept-2-ylmethyl)-8-brom[1]benzofuro[3,2-d]pyrimidin-4(3H)-on |
| 521 | 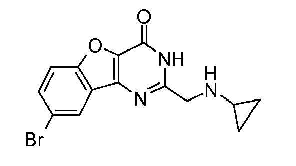 | 8-Brom-2-[(cyclopropylamino)methyl][1] benzofuro[3,2-d]pyrimidin-4(3H)-on |
| 522 | 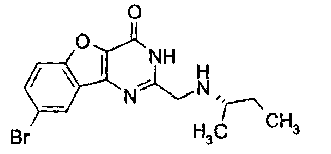 | 8-Brom-2-({[(1S)-1-methylpropyl]amino}methyl)[1] benzofuro[3,2-d]pyrimidin-4(3H)-on |
| 523 | 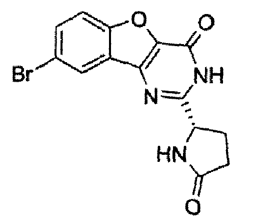 | 8-Brom-2-[(2S)-5-oxopyrrolidin-2-yl][1]benzofuro[3,2-d]pyrimidin-4(3H)-on |
| 524 | 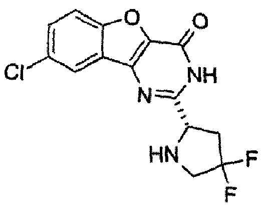 | 8-Chlor-2-[(2S)-4,4-difluorpyrrolidin-2-yl][1]benzofuro[3,2-d]pyrimidin-4(3H)-on |
| 525 | 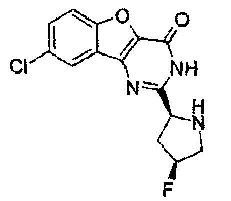 | 8-Chlor-2-[(2S,4S)-4-fluorpyrrolidin-2-yl][1]benzofuro[3,2-d]pyrimidin-4(3H)-on |
| 526 | 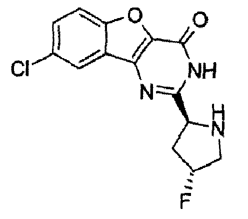 | 8-Chlor-2-[(2S,4R)-4-fluorpyrrolidin-2-yl][1]benzofuro[3,2-d]pyrimidin-4(3H)-on |
| 527 | 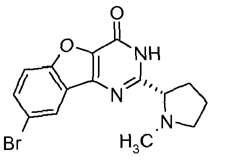 | 8-Brom-2-[(2S)-1-methylpyrrolidin-2-yl][1]benzofuro[3,2-d]pyrimidin-4(3H)-on |
| 528 | 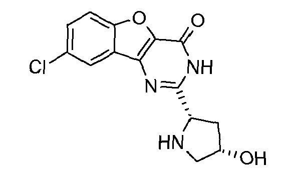 | 8-Chlor-2-[(2S,4S)-4-hydroxypyrrolidin-2-yl][1]benzofuro[3,2-d]pyrimidin-4(3H)-on |
| 529 | 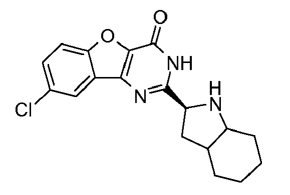 | 8-Chlor-2-[(2S)-octahydro-1H-indol-2-yl][1]benzofuro[3,2-d]pyrimidin-4(3H)-on |
| 530 | 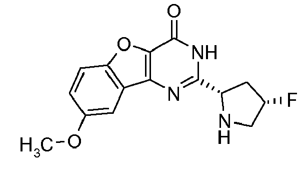 | 2-[(2S,4S)-4-fluorpyrrolidin-2-yl]-8-(methyloxy)[1]benzofuro[3,2-d]pyrimidin-4(3H)-on |
| 531 | 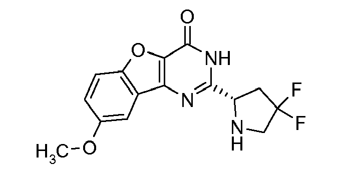 | 2-[(2S)-4,4-Difluorpyrrolidin-2-yl]-8-(methyloxy)[1]benzofuro[3,2-d]pyrimidin-4(3H)-on |
| 532 | 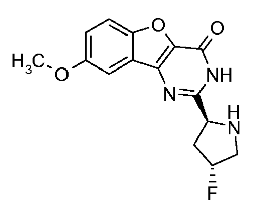 | 2-[(2S,4R)-4-Fluorpyrrolidin-2-yl]-8-(methyloxy)[1]benzofuro[3,2-d]pyrimidin-4(3H)-on |
| 533 | 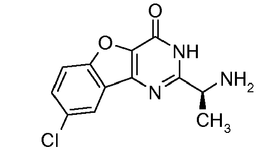 | 2-[(1S)-1-Aminoethyl]-8-chlor[1]benzofuro[3,2-d]pyrimidin-4(3H)-on |
| 534 | 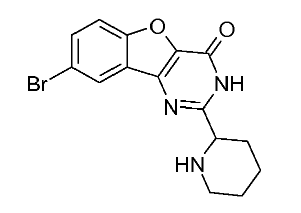 | 8-Brom-2-piperidin-2-yl[1]benzofuro[3,2-d]pyrimidin-4(3H)-on-Hydrochlorid |
| 535 | 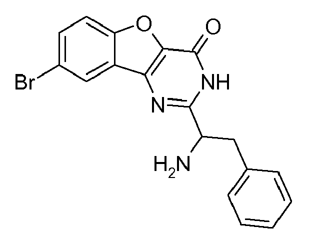 | 2-(1-Amino-2-phenylethyl)-8-brom[1]benzofuro[3,2-d]pyrimidin-4(3H)-on |
| 536 | 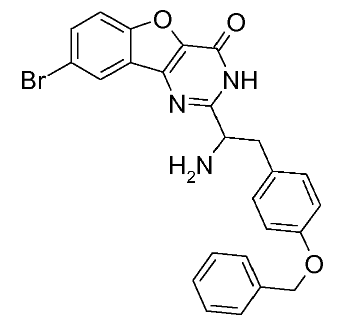 | 2-(1-Amino-2-{4-[(phenylmethyl)oxy]phenyl}ethyl)-8-brom[1]benzofuro[3,2-d]pyrimidin-4(3H)-on |
| 537 | 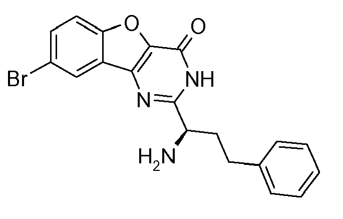 | 2-[(1 S)-1-Amino-3-phenylpropyl]-8-brom[1]benzofuro[3,2-d]pyrimidin-4(3H)-on |
| 538 | 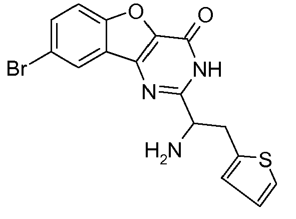 | 2-[1-Amino-2-(2-thienyl)ethyl]-8-brom[1]benzofuro[3,2-d]pyrimidin-4(3H)-on |
| 539 | 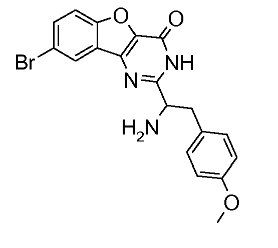 | 2-{1-Amino-2-[4-(methyloxy)phenyl]ethyl}-8-brom[1]benzofuro[3,2-d]pyrimidin-4(3H)-on |
| 540 | 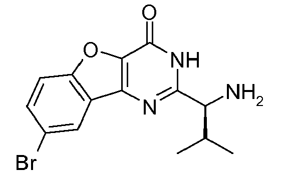 | 2-[(1 S)-1-Amino-2-methylpropyl]-8-brom[1]benzofuro[3,2-d]pyrimidin-4(3H)-on |
| 541 | 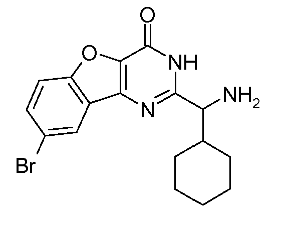 | 2-[Amino(cyclohexyl)methyl]-8-brom[1]benzofuro[3,2-d]pyrimidin-4(3H)-on |
| 542 | 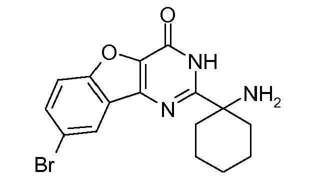 | 2-(1-Aminocyclohexyl)-8-brom[1]benzofuro[3,2-d]pyrimidin-4(3H)-on |
| 543 | 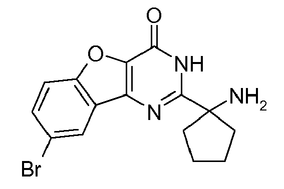 | 2-(1-Aminocyclopentyl)-8-brom[1 ]benzofuro[3,2-d]pyrimidin-4(3H)-on |
| 544 | 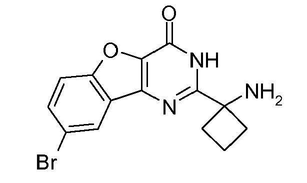 | 2-(1-Aminocyclobutyl)-8-brom[1]benzofuro[3,2-d]pyrimidin-4(3H)-on |

12. Verbindung gemäß Anspruch 1, die wie folgt ist: 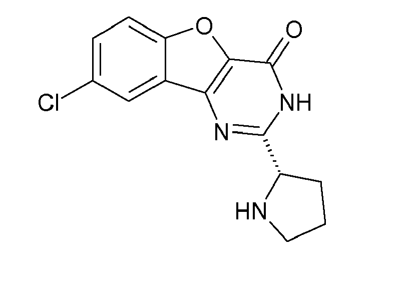 8-Chlor-2-[(2S)-pyrrolidin-2-yl][1]benzofuro[3,2-d]pyrimidin-4(3H)-on oder ein pharmazeutisch zulässiges Salz davon.

13. Eine pharmazeutische Zusammensetzung, die die Verbindung gemäß einem der Ansprüche 1-12, oder ein pharmazeutisch zulässiges Salz davon, und einen pharmazeutisch zulässigen Träger, einen pharmazeutisch zulässigen Hilfsstoff oder ein pharmazeutisch zulässiges Verdünnungsmittel beinhaltet.

14. Ein In-vitro-Verfahren zum Inhibieren von PIM, CDC7 oder CK2 in einer Zelle, das das In-Kontakt-Bringen der Zelle, in der eine Inhibition von PIM, CDC7 oder CK2 erwünscht ist, mit der Verbindung gemäß einem der Ansprüche 1-12, oder einem pharmazeutisch zulässigen Salz davon, oder der pharmazeutischen Zusammensetzung gemäß Anspruch 13 beinhaltet.

15. Verbindung gemäß einem der Ansprüche 1-12, oder ein pharmazeutisch zulässiges Salz davon, oder eine Zusammensetzung gemäß Anspruch 13 zur Verwendung bei der Behandlung eines Krebs, ausgewählt aus Bauchspeicheldrüsenkrebs, Prostatakrebs, hepatozellulärem Karzinom, Lymphomen, Leukämien, kolorektalem Karzinom.

16. Verbindung oder Zusammensetzung gemäß Anspruch 15, wobei die Behandlung ferner ein oder mehrere Therapeutika beinhaltet, ausgewählt aus: Camptothecin, Topotecan, 9-Nitrocamptothecin, 9-Aminocamptothecin, Karenitecin, Irinotecan, Etoposid, Etoposidphosphat, Teniposid, Amsacrin, Razoxan, Dexrazoxan, Mechlorethamin, Cyclophosphamid, Ifosfamid, Chlorambucil, Melphalan, Thiotepa, Trenimon, Triethylenmelamin, Dianhydrogalactitol, Dibromodulcitol, Busulfan, Dimethylsulfat, Chlorethylnitrosoharnstoff, BCNU, CCNU, Methyl-CCNU, Streptozotocin, Chlorozotocin, Prednimustin, Estramustin, Procarbazin, Dacarbazin, Hexamethylmelamin, Pentamethylmelamin, Temozolomid, Cisplatin, Carboplatin, Oxaliplatin, Bleomycin, Dactinomycin, Mithramycin, Rapamycin, Mitomycin C, Daunorubicin, Doxorubicin, Epirubicin, Idarubicin, Methotrexat, Edatrexat, Trimethoprim, Nolatrexed, Raltitrexed, Hydroxyharnstoff, 5-Fluoruracil, Ixabepilon, Ftorafur, Capecitabin, Furtulon, Eniluracil, AraC, 5-Azacytidin, Gemcitabin, Mercaptopurin, Thioguanin, Pentostatin, Antisense-DNA, Antisense-RNA, einem Antisense-DNA/RNA-Hybrid, einem Ribozym, ultravioletter Strahlung, Vincristin, Vinblastin, Paclitaxel, Docetaxel, L-Asparaginase, einem Kinaseinhibitor, Imatinib, Mitotan, Aminoglutethimid, Diethylstilbestrol, Ethinylestradiol, Tamoxifen, Anastrozol, Testosteronpropionat, Fluoxymesteron, Flutamid, Leuprolid, Prednison, Hydroxyprogesteroncaproat, Medroxyprogesteronacetat, Megestrolacetat, Alpha-Interferon und Interleukin.

17. Verbindung gemäß einem der Ansprüche 1-12, oder ein pharmazeutisch zulässiges Salz davon, oder eine Zusammensetzung gemäß Anspruch 13 zur Verwendung als ein Arzneimittel.

18. Verbindung gemäß einem der Ansprüche 1-12, oder ein pharmazeutisch zulässiges Salz davon, oder eine Zusammensetzung gemäß Anspruch 13 zur Verwendung beim Inhibieren von PIM, CDC7 oder CK2 in einer Zelle.

## Revendications

1. Un composé selon la formule I : 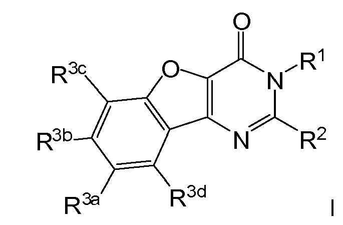 ou un sel de celui-ci acceptable d'un point de vue pharmaceutique, dans lequel :
R¹ est hydrogène ou alkyle(C₁-C₁₂) ;
R² est choisi parmi -NH-phényle, -NH-pipéridinyle, -NH-pyridinyle, -NHalkyl(C₁-C₃)phényle optionnellement substitué à n'importe quelle position phényle par du pipérazinyle ou du méthylpipérazinyle, -NHalkyl(C₁-C₃)-N(CH₃)₂, -NHalkyl(C₁-C₃)-OH, -alkyl(C₁-C₃)-O-phényle, -alkyl(C₁-C₃)-O-alkyl(C₁-C₃)-hétérocycloalkyle(à 5-6 membres), -alkyl(C₁-C₃)-N(H)-hétéroaryle, -alkyl(C₁-C₃)-hétéroaryle(à 5-10 membres) optionnellement substitué par -alkyle(C₁-C₃), halo ou phényle, oxopyrrolidinyl optionnellement substitué par OH ou pipéridinyle, -alkyl(C₁-C₄)-hétérocycloalkyle(à 3-9 membres) optionnellement substitué au niveau de l'hétérocycloalkyle(à 3-9 membres) par Xd, -alkyl(C₁-C₆)-NRzb-alkyle(C₁-C₄) dans lequel la portion -alkyle(C₁-C₄) est substituée par Xe, -alkyl(C₁-C₃)-NH-cycloalkyle(C₃-C₆), -(CH₂)-NH-cyclohexyle(C₃-C₆), -alkyl(C₁-C₃)-NH₂, dans lequel la portion -alkyle(C₁-C₃)- de -alkyl(C₁-C₃)-NH₂ est substituée par Xf, et -hétérocycloalkyle(à 3-9 membres) optionnellement substitué par Xg ;
Xd est choisi parmi alkyle(C₁-C₁₂), 1-3 halo, -COOH, phényle optionnellement substitué par 1 ou 2 groupes choisis parmi les groupes halo, méthyle et méthylphényle, phénylméthyle, spiro-pipéridine, trifluorométhylphénylméthyle, -alkoxy(C₁-C₃), pyridinyle, diméthylaminoalkyle(C₁-C₁₂), diméthylamino, hydroxylalkyle(C₁-C₁₂), diméthylaminoalkyl(C₁-C₁₂)aminocarbonyl, alkyl(C₁-C₁₂)amino, aminoalkyle(C₁-C₁₂), diméthylaminocarbonylalkyle(C₁-C₁₂), diéthylaminoalkyl(C₁-C₁₂)carbonyle, -alkyl(C₁-C₃)-hétérocycloalkyle(à 5-6 membres), hétérocycloalkyle(à 5-6 membres) optionnellement substitué par -alkyle(C₁-C₃), -NH₂, -OH, hétérocycloalkyle(à 4-12 membres)alkyl(C₁-C₁₂)amino, alkoxy(C₁-C₁₂) alkyle(C₁-C₁₂), -C(O)CH₃, -C(O)NHalkyl(C₁-C₃)phényle optionnellement substitué par 1-3 halo à n'importe quelle position phényle, un -OH et un -C(O)NHalkyl(C₁-C₃)phényle optionnellement substitué par 1-3 halo à n'importe quelle position phényle, -C(O)-hétérocycloalkyle(à 4-12 membres) optionnellement substitué par -OH ou halo, alkoxy(C₁-C₁₂)carbonyle, aminocarbonyle, un -OH et un méthyle, un -OH et un -C(O)OH, un -OH et un -NHC(O)pipéridinyle, un -OH et un alkyle(C₁-C₁₂) ;
Xe est choisi parmi dialkylamino, amino, 1-3 -OH, alkoxy(C₁-C₁₂), 4-méthylpipérazinylphényle, diméthylaminophényle, phényle optionnellement substitué par 1-3 groupes choisis parmi les groupes halo et méthoxy, hétéroaryle(à 5-12 membres), -alkyl(C₁-C₃)C(O)NH₂, -C(O)NH₂, -C(O)OH, -alkyl(C₁-C₃)C(O)OH, et hétérocycloalkyle(à 4-12 membres) optionnellement substitué par 1-2 alkyle(C₁-C₁₂);
Xf est choisi parmi cycloalkyle(C₃-C₁₄), spirocycloalkyle(C₃-C₁₄), phényle, phénylalkyle(C₁-C₁₂) optionnellement substitué par phénylméthyloxy ou alkoxy(C₁-C₁₂), et thiénylalkyle(C₁-C₁₂) ;
Xg est choisi parmi alkyle(C₁-C₁₂), alkyl(C₁-C₁₂)carbonyle, hétérocycloalkylcarbonyle(à 4-12 membres), dialkylaminoalkyl(C₁-C₁₂)carbonyle, 1-méthylpipéridinyle, dialkylaminoalkyle(C₁-Cₗ₂), hétéroarylcarbonyle(à 5-12 membres), alkoxy(C₁-C₁₂)alkyl(C₁-C₁₂)carbonyle, phénylcarbonyle, phénylalkyl(C₁-C₁₂)carbonyle, oxo, phénylalkyle(C₁-C₁₂), -hétéroaryl(à 5-6 membres)alkyle(C₁-C₁₂), pipéridinyloxy, -OH, oxo, 1-2 halo et 1-2 méthyle ; et
Rzb est H ou alkyle optionnellement substitué par 1-3 -OH ;
R^{3a} est choisi parmi halo, alkyle(C₁-C₁₂), -NO₂, alkoxy(C₁-C₁₂), alkynyl(C₁-C₁₂) optionnellement substitué par R¹⁴, alkoxy(C₁-C₁₂)carbonylalkyle(C₁-C₁₂), aryl(C₆-C₁₄) alkoxy(C₁-C₁₂), -C(O)N(H)alkyle(C₁-C₁₂), -N(H)-C(O)-alkyle(C₁-C₁₂), -C(O)-alkyle(C₁-C₁₂), -CN, phényle, -OCF₃, -N(H)R¹³, -OH, -CF₃, -S-CH₃ et hydroxyméthylalkynyl(C₁-C₁₂) ;
R^{3b}, R^{3c} et R^{3d} sont chacun indépendamment choisis parmi H, -OH, -N⁺(O)OH, alkoxyl(C₁-C₁₂), et halo ;
ou R^{3a} est hydrogène et R^{3b}, R^{3c} et R^{3d} sont chacun indépendamment choisis parmi -CF₃, -OH, alkoxy(C₁-C₁₂), et halo ;
ou R^{3a} et R^{3d}, ainsi que les carbones auxquels ils sont attachés, se joignent pour former un hétéroaryle à 5 membres optionnellement substitué par méthyle ou -NH₂,
ou un hétérocycloalkyle à 5-6 membres ;
R¹³ est choisi parmi aryl(C₆-C₁₄)alkyle(C₁-C₁₂) dans lequel la portion aryle d'arylalkyle est optionnellement substituée par 1, 2 ou 3 alkoxy(C₁-C₁₂), halo, méthyle, méthoxy, -CF₃, cycloalkyle(C₃-C₁₄), et hétéroaryl(à 5-12 membres)alkyle(C₁-C₁₂) ; et
R¹⁴ est choisi parmi hydroxylalkyle(C₁-C₁₂), H et TMS.

2. Le composé selon la revendication 1, ou un sel de celui-ci acceptable d'un point de vue pharmaceutique, dans lequel R^{3a} est choisi parmi halo, alkyle(C₁-C₁₂), -NO₂, alkoxy(C₁-C₁₂), alkynyl(C₁-C₁₂) optionnellement substitué par R¹⁴, alkoxy(C₁-C₁₂)carbonylalkyle(C₁-C₁₂), aryl(C₆-C₁₄)alkoxy(C₁-C₁₂), -C(O)N(H)alkyle(C₁-C₁₂), -N(H)-C(O)-alkyle(C₁-C₁₂), -C(O)-alkyle(C₁-C₁₂), -CN, phényle, -OCF₃, -N(H)R¹³, -OH, -CF₃, -S-CH₃ et hydroxyméthylalkynyl(C₁-C₁₂) ; et R^{3b}, R^{3c} et R^{3d} sont chacun H.

3. Le composé selon la revendication 1, ou un sel de celui-ci acceptable d'un point de vue pharmaceutique, dans lequel R^{3a} est halo, alkoxy(C₁-C₁₂) ou -OCF₃; et R^{3b}, R^{3c} et R^{3d} sont chacun H.

4. Le composé selon la revendication 1, ou un sel de celui-ci acceptable d'un point de vue pharmaceutique, dans lequel R² est un hétérocycloalkyle(à 3-9 membres) optionnellement substitué par Xg ; R^{3a} est halo, alkoxy(C₁-C₁₂) ou -OCF₃ ; et R^{3b}, R^{3c} et R^{3d} sont chacun H.

5. Le composé selon la revendication 1, ou un sel de celui-ci acceptable d'un point de vue pharmaceutique, dans lequel R² est -alkyl(C₁-C₃)-hétéroaryle(à 5-10 membres), dans lequel l'hétéroaryle est optionnellement substitué par -alkyle(C₁-C₃), halo et phényle ; R^{3a} est halo, alkoxy(C₁-C₁₂) ou -OCF₃ ; et R^{3b}, R^{3c} et R^{3d} sont chacun H.

6. Le composé selon la revendication 1, ou un sel de celui-ci acceptable d'un point de vue pharmaceutique, dans lequel R² est -alkyl(C₁-C₄)-hétérocycloalkyle(à 3-9 membres) optionnellement substitué à n'importe quelle position de l' hétérocycloalkyle(à 3-9 membres) par Xd ; R^{3a} est halo, alkoxy(C₁-C₁₂) ou -OCF₃ ; et R^{3b}, R^{3c} et R^{3d} sont chacun H.

7. Le composé selon la revendication 1, ou un sel de celui-ci acceptable d'un point de vue pharmaceutique, dans lequel R² est un hétérocycloalkyle choisi parmi pipérazinyle, pipéridinyle, pyrrolidinyle, isoxazolyle, azétidinyle, morpholinyle, tétrahydrofuranyle, thiazolidinyle ou octahydro-1H-indolyle, dans lequel l'hétérocycloalkyle est optionnellement substitué par Xg ; R^{3a} est halo, alkoxy(C₁-C₁₂) ou -OCF₃ ; et R^{3b}, R^{3c} et R^{3d} sont chacun H.

8. Le composé selon la revendication 1, ou un sel de celui-ci acceptable d'un point de vue pharmaceutique, dans lequel R² est l'hétérocycloalkyl(à 3-9 membres)alkyle(C₁-C₄), et dans lequel la portion hétérocycloalkyle de l'hétérocycloalkylalkyle est pipérazinyle, pipéridinyle, pyrrolidinyle, azétidinyle, morpholinyle, 1,4-diazépanyle, 2,5-diazabicyclo[2.2.1]heptyle, azabicyclo[2.2.1]heptane, 8-azabicyclo[3.2.1]oct-8-yle, (1S,4S)-2,5-diazabicyclo[2.2.1]heptyle, 2,5-dihydro-1H-pyrrolyle, (1 R,4R)-2,5-diazabicyclo[2.2.1]heptan-2-yle, (1R,5S)-8-azabicyclo[3.2.1]octyle, (8aS)-hexahydropyrrolo[1,2-a]pyrazin-2)-yle, diméthylpipérazinyle ou (3aR,6aS)-hexahydropyrrolo[3,4-c]pyrrolyle.

9. Le composé selon la revendication 1, ou un sel de celui-ci acceptable d'un point de vue pharmaceutique, dans lequel R² est un hétéroaryle(à 5-10 membres)alkyle(C₁-C₃) optionnellement substitué à n'importe quelle position de cycle par -alkyle(C₁-C₃), halo et phényle ; R^{3a} est halo, alkoxy(C₁-C₁₂) ou -OCF₃ ; et R^{3b}, R^{3c} et R^{3d} sont chacun H.

10. Le composé selon la revendication 1, ou un sel de celui-ci acceptable d'un point de vue pharmaceutique, dans lequel R₂ est 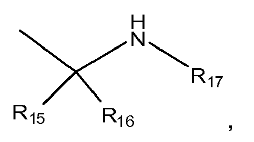 dans lequel R₁₅ est choisi parmi H ou -alkyle(C₁-C₆), R₁₆ est choisi parmi H, phényle et -alkyle(C₁-C₆), et R₁₇ est choisi parmi H, -alkyl(C₁-C₃)C(O)NH₂, -alkyl(C₁-C₃)C(O)OH et hétérocycloalkyl(à 4-12 membres)alkyle(C₁-C₁₂).

11. Le composé selon la revendication 1 choisi parmi l'un des composés suivants :
| **Comp. N°** | **Structure** | **NOM** |
|---|---|---|
| 1 | 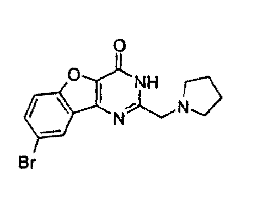 | 8-bromo-2-(pyrrolidin-1-ylméthyl)[1]benzofuro[3,2-d] pyrimidin-4(3H)-one |
| 2 | 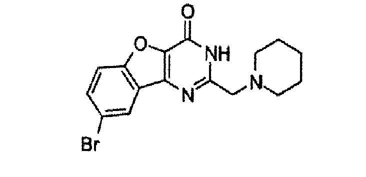 | 8-bromo-2-(pipéridin-1-ylméthyl)[1]benzofuro[3,2-d] pyrimidin-4(3H)-one |
| 3 | 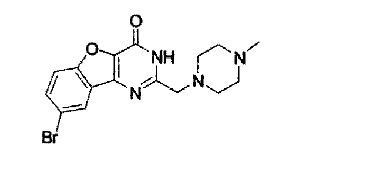 | 8-bromo-2-[(4-méthylpipérazin-1-yl)méthyl][1]benzofuro[3,2-d] pyrimidin-4(3H)-one |
| 4 | 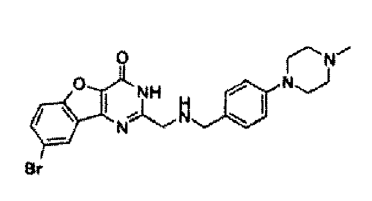 | 8-bromo-2-[({[4-(4-méthylpipérazin-1-yl)phényl]méthyl}amino)méthyl][1] benzofuro[3,2-d]pyrimidin-4(3H)-one |
| 5 | 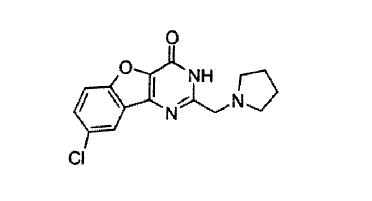 | 8-chloro-2-(pyrrolidin-1-ylméthyl)[1]benzofuro[3,2-d] pyrimidin-4(3H)-one |
| 6 | 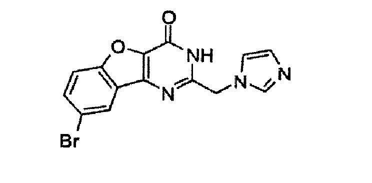 | 8-bromo-2--imidazol-1-ylméthyl)[1]benzofuro[3,2-d] pyrimidin-4(3H)-one |
| 7 | 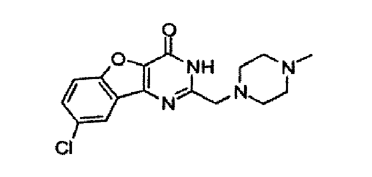 | 8-chloro-2-[(4-méthylpipérazin-1-yl)méthyl][1]benzofuro[3,2-d] pyrimidin-4(3H)-one |
| 9 | 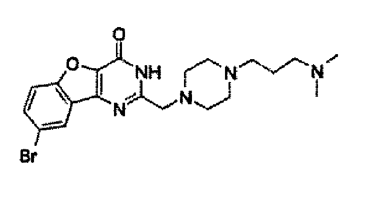 | 8-bromo-2-({4-[3-(diméthylamino)propyl]pipérazin-1-yl}méthyl)[1]benzofuro[3,2-d] pyrimidin-4(3H)-one |
| 10 | 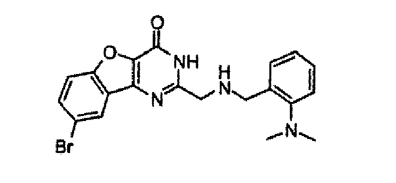 | 8-bromo-2-[({[2-(diméthylamino)phényl]méthyl} amino)méthyl][1]benzofuro[3,2-d] pyrimidin-4(3H)-one |
| 13 | 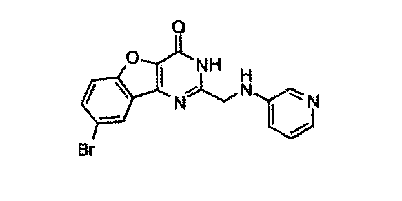 | 8-bromo-2-[(pyridin-3-ylamino)méthyl][1]benzofuro[3,2-d]pyrimidin-4(3H)-one |
| 14 | 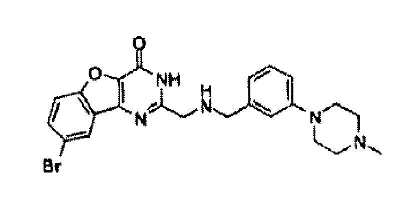 | 8-bromo-2-[({[3-(4-méthylpipérazin-1-yl) phényl]méthyl}amino)méthyl][1] benzofuro[3,2-d]pyrimidin-4(3H)-one |
| 15 | 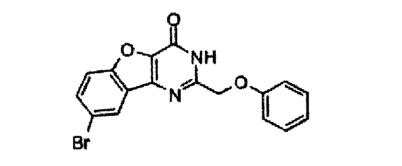 | 8-bromo-2-[(phényloxy) méthyl][1]benzofuro[3,2-d]pyrimidin-4(3H)-one |
| 17 | 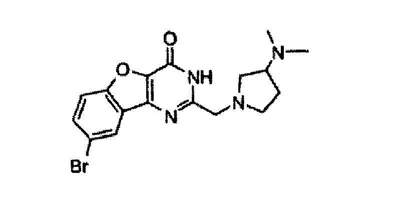 | 8-bromo-2-{[3-(diméthylamino)pyrrolidin-1-yl] méthyl}[1]benzofuro[3,2-d] pyrimidin-4(3H)-one |
| 20 | 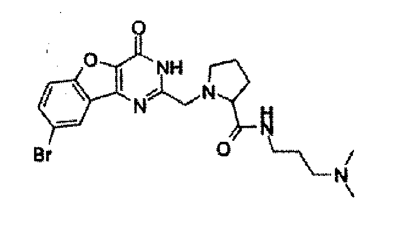 | 1-[(8-bromo-4-oxo-3,4-dihydro[1]benzofuro[3,2-d] pyrimidin-2-yl)méthyl]-N-[3-(diméthylamino)propyl]prolinamide |
| 21 | 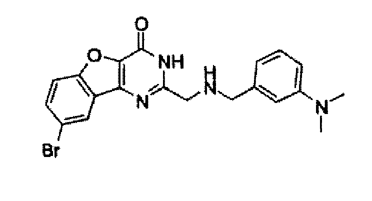 | 8-bromo-2-[({[3-(diméthylamino)phényl]méthyl} amino)méthyl][1]benzofuro[3,2-d] pyrimidin-4(3H)-one |
| 23 | 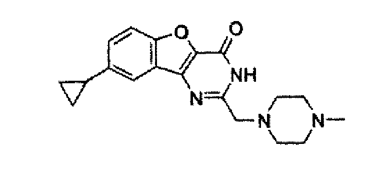 | 8-cyclopropyl-2-[(4-méthylpipérazin-1-yl) méthyl][1]benzofuro[3,2-d] pyrimidin-4(3H)-one |
| 24 | 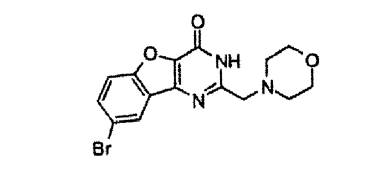 | 8-bromo-2-(morpholin-4-ylméthyl)[1]benzofuro[3,2-d] pyrimidin-4(3H)-one |
| 25 | 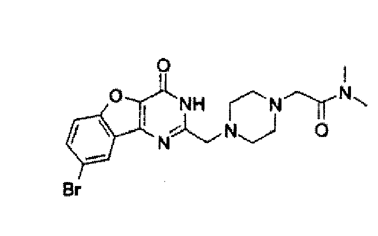 | 2-{4-[(8-bromo-4-oxo-3,4-dihydro[1]benzofuro[3,2-d] pyrimidin-2-yl)méthyl]pipérazin-1-yl}-N,N-diméthylacétamide |
| 26 | 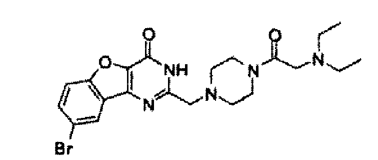 | 8-bromo-2-{[4-(N,N-diéthylglycyl)pipérazin-1-yl] méthyl}[1]benzofuro[3,2-d] pyrimidin-4(3H)-one |
| 28 | 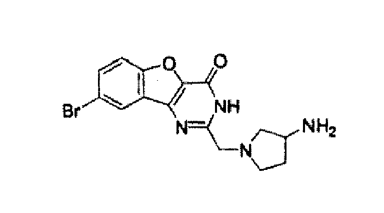 | 2-[(3-aminopyrrolidin-1-yl)méthyl]-8-bromo[1]benzofuro[3,2-d] pyrimidin-4(3H)-one |
| 29 | 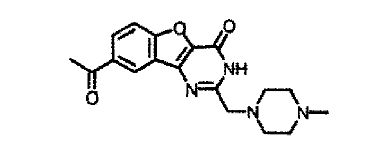 | 8-Acétyl-2-[(4-méthylpipérazin-1-yl)méthyl][1]benzofuro[3,2-d] pyrimidin-4(3H)-one |
| 45 | 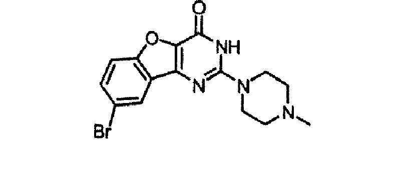 | 8-bromo-2-(4-méthylpipérazin-1-yl)[1]benzofuro[3,2-d]pyrimidin-4(3H)-one |
| 46 | 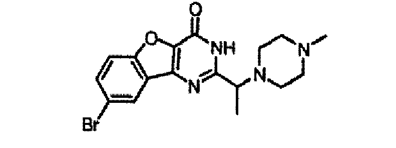 | 8-bromo-2-[1-(4-méthylpipérazin-1-yl)éthyl][1]benzofuro[3,2-d] pyrimidin-4(3H)-one |
| 49 | 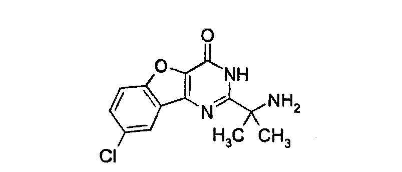 | 2-(1-amino-1-méthyléthyl)-8-chloro[1]benzofuro[3,2-d] pyrimidin-4(3H)-one |
| 74 | 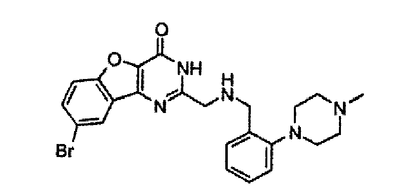 | 8-bromo-2-[({[2-(4-méthylpipérazin-1-yl) phényl]méthyl}amino)méthyl][1] benzofuro[3,2-d]pyrimidin-4(3H)-one |
| 81 | 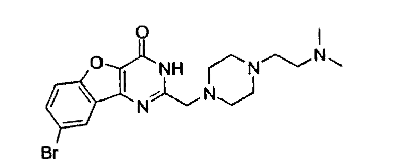 | 8-bromo-2-({4-[2-(diméthylamino)éthyl]pipérazin-1-yl}méthyl)[1]benzofuro[3,2-d] pyrimidin-4(3H)-one |
| 82 | 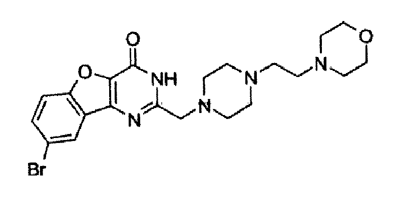 | 8-bromo-2-{[4-(2-morpholin-4-yléthyl)pipérazin-1-yl] méthyl}[1]benzofuro[3,2-d] pyrimidin-4(3H)-one |
| 84 | 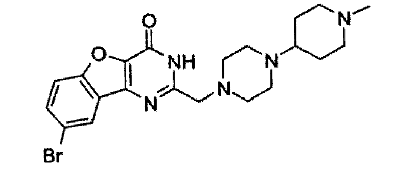 | 8-bromo-2-{[4-(1-méthylpipéridin-4-yl)pipérazin-1-yl]méthyl} [1]benzofuro[3,2-d]pyrimidin-4(3H)-one |
| 100 | 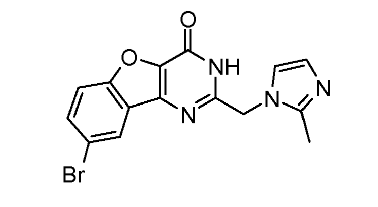 | 8-bromo-2-[(2-méthyl-1H-imidazol-1-yl)méthyl][1]benzofuro[3,2-d] pyrimidin-4(3H)-one |
| 103 | 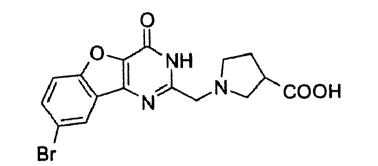 | 1-[(8-bromo-4-oxo-3,4-dihydro[1]benzofuro[3,2-d] pyrimidin-2-yl)méthyl]pyrrolidine-3-acide carboxylique |
| 104 | 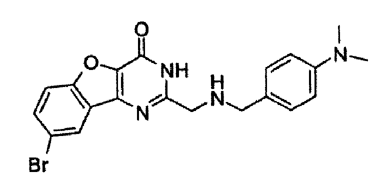 | 8-bromo-2-[({[4-(diméthylamino)phényl]méthyl} amino)méthyl][1]benzofuro[3,2-d] pyrimidin-4(3H)-one |
| 105 | 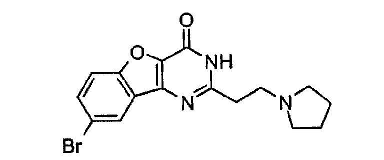 | 8-bromo-2-(2-pyrrolidin-1-yléthyl)[1]benzofuro[3,2-d] pyrimidin-4(3H)-one |
| 108 | 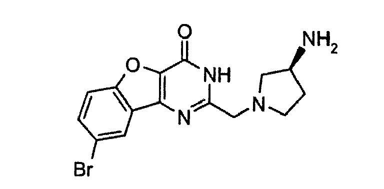 | 2-{[(3S)-3-aminopyrrolidin-1-yl]méthyl}-8-bromo[1]benzofuro [3,2-d]pyrimidin-4(3H)-one |
| 109 | 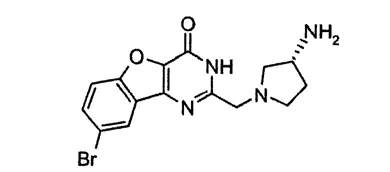 | 2-{[(3R)-3-aminopyrrolidin-1-yl]méthyl}-8-bromo[1]benzofuro [3,2-d]pyrimidin-4(3H)-one |
| 110 | 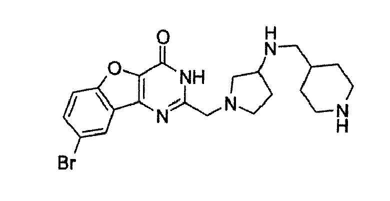 | 8-bromo-2-({3-[(pipéridin-4-ylméthyl)amino]pyrrolidin-1-yl} méthyl)[1]benzofuro[3,2-d] pyrimidin-4(3H)-one |
| 111 | 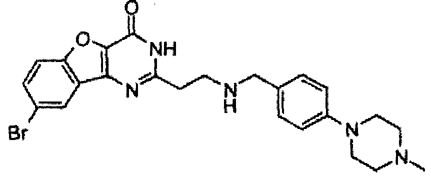 | 8-bromo-2-[2-({[4-(4-méthylpipérazin-1-yl)phényl] méthyl}amino)éthyl][1]benzofuro [3,2-d]pyrimidin-4(3H)-one |
| 113 | 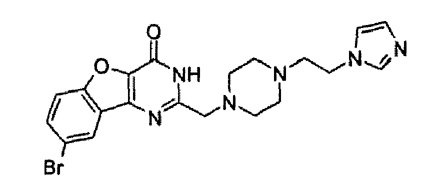 | 8-bromo-2-({4-[2--imidazol-1-yl)éthyl]pipérazin-1-yl} méthyl)[1]benzofuro[3,2-d] pyrimidin-4(3H)-one |
| 114 | 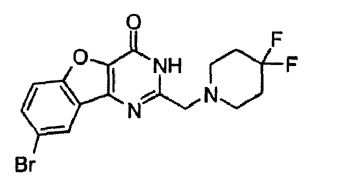 | 8-bromo-2-[(4,4-difluoropipéridin-1-yl)méthyl][1]benzofuro[3,2-d] pyrimidin-4(3H)-one |
| 115 | 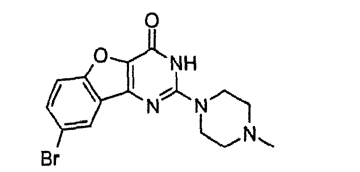 | 8-bromo-2-(4-méthylpipérazin-1-yl)[1]benzofuro[3,2-d]pyrimidin-4(3H)-one |
| 117 | 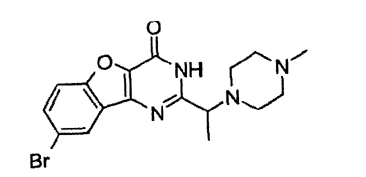 | 8-bromo-2-[1-(4-méthylpipérazin-1-yl)éthyl][1]benzofuro[3,2-d] pyrimidin-4(3H)-one |
| 118 | 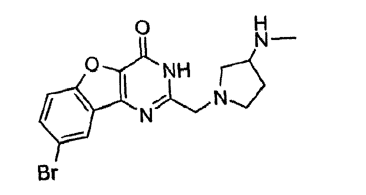 | 8-bromo-2-{[3-(méthylamino)pyrrolidin-1-yl] méthyl}[1]benzofuro[3,2-d] pyrimidin-4(3H)-one |
| 119 | 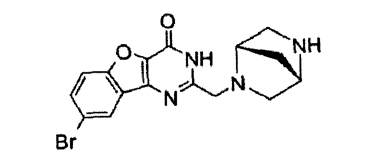 | 8-bromo-2-[(1S,4S)-2,5-diazabicyclo[2.2.1]hept-2-ylméthyl][1]benzofuro[3,2-d] pyrimidin-4(3H)-one |
| 122 | 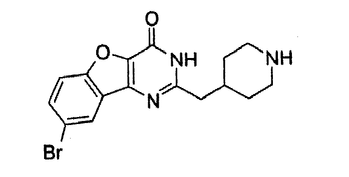 | 8-bromo-2-(pipéridin-4-ylméthyl)[1]benzofuro[3,2-d] pyrimidin-4(3H)-one |
| 130 | 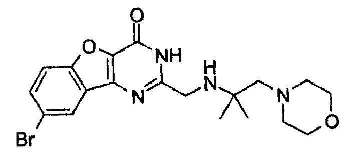 | 8-bromo-2-{[(1,1-diméthyl-2-morpholin-4-yléthyl)amino] méthyl}[1]benzofuro[3,2-d] pyrimidin-4(3H)-one |
| 131 | 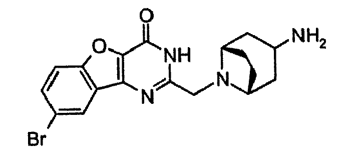 | 2-{[(1R,5S)-3-amino-8-azabicyclo[3.2.1]oct-8-yl]méthyl}-8-bromo[1]benzofuro[3,2-d] pyrimidin-4(3H)-one |
| 132 | 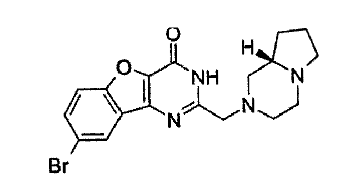 | 8-bromo-2-[(8aS)-hexahydropyrrolo[1,2-a]pyrazin-2)-ylméthyl][1]benzofuro[3,2-d] pyrimidin-4(3H)-one |
| 133 | 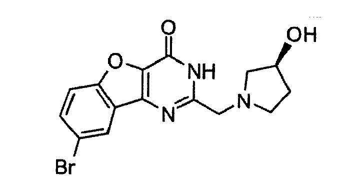 | 8-bromo-2-{[(3S)-3-hydroxypyrrolidin-1 -yl] méthyl}[1]benzofuro[3,2-d] pyrimidin-4(3H)-one |
| 141 | 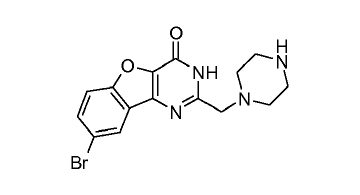 | 8-bromo-2-(pipérazin-1-ylméthyl) [1]benzofuro[3,2-d]pyrimidin-4(3H)-one |
| 142 | 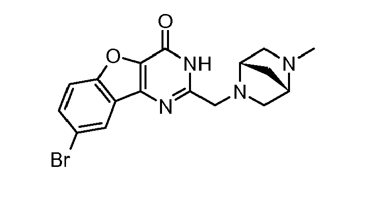 | 8-bromo-2-{[(1S,4S)-5-méthyl-2,5-diazabicyclo[2.2.1]hept-2-yl] méthyl}[1]benzofuro[3,2-d] pyrimidin-4(3H)-one |
| 147 | 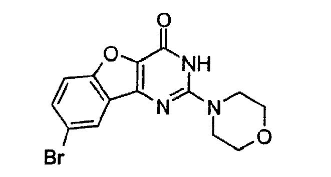 | 8-bromo-2-morpholin-4-yl [1]benzofuro[3,2-d]pyrimidin-4(3H)-one |
| 149 | 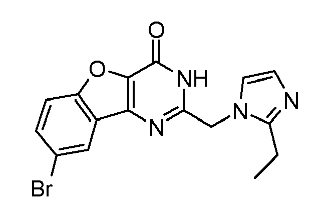 | 8-bromo-2-[(2-éthyl-1H-imidazol-1-yl)méthyl][1]benzofuro[3,2-d] pyrimidin-4(3H)-one |
| 150 | 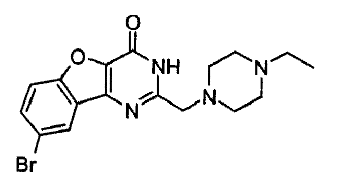 | 8-bromo-2-[(4-éthylpipérazin-1-yl) méthyl][1]benzofuro[3,2-d] pyrimidin-4(3H)-one |
| 151 | 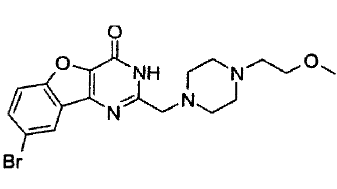 | 8-bromo-2-({4-[2-(méthyloxy)éthyl]pipérazin-1-yl} méthyl)[1]benzofuro[3,2-d] pyrimidin-4(3H)-one |
| 153 | 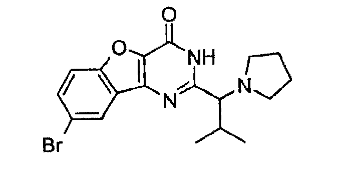 | 8-bromo-2-(2-méthyl-1-pyrrolidin-1-ylpropyl)[1]benzofuro[3,2-d] pyrimidin-4(3H)-one |
| 157 | 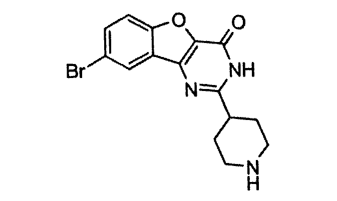 | 8-bromo-2-pipéridin-4-yl[1] benzofuro[3,2-d]pyrimidin-4(3H)-one |
| 158 | 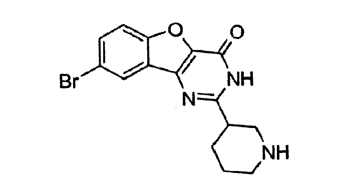 | 8-bromo-2-pipéridin-3-yl [1]benzofuro[3,2-d]pyrimidin-4(3H)-one |
| 164 | 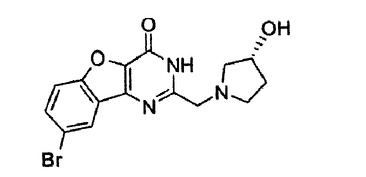 | 8-bromo-2-{[(3R)-3-hydroxypyrrolidin-1-yl] méthyl}[1]benzofuro[3,2-d] pyrimidin-4(3H)-one |
| 165 | 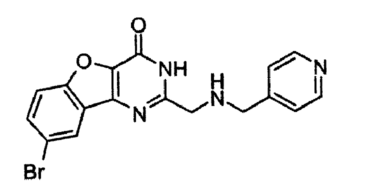 | 8-bromo-2-{[(pyridin-4-ylméthyl)amino]méthyl}[1] benzofuro[3,2-d]pyrimidin-4(3H)-one |
| 166 | 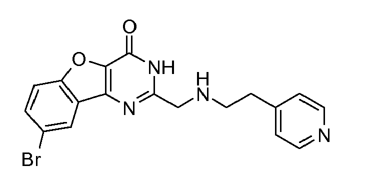 | 8-bromo-2-{[(2-pyridin-4-yléthyl)amino]méthyl}[1]benzofuro [3,2-d]pyrimidin-4(3H)-one |
| 167 | 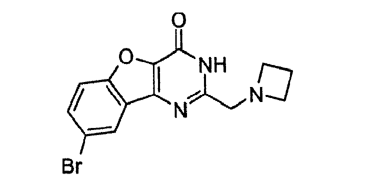 | 2-(azétidin-1-ylméthyl)-8-bromo[1]benzofuro[3,2-d] pyrimidin-4(3H)-one |
| 168 | 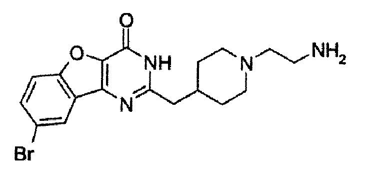 | 2-{[1-(2-aminoéthyl)pipéridin-4-yl]méthyl}-8-bromo[1] benzofuro[3,2-d]pyrimidin-4(3H)-one |
| 169 | 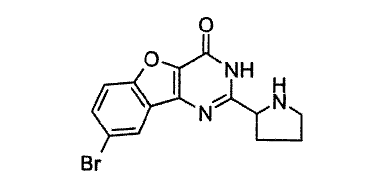 | 8-bromo-2-pyrrolidin-2-yl[1] benzofuro[3,2-d]pyrimidin-4(3H)-one |
| 172 | 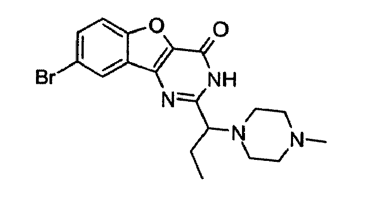 | 8-bromo-2-[1-(4-méthylpipérazin-1-yl)propyl][1]benzofuro[3,2-d] pyrimidin-4(3H)-one |
| 174 | 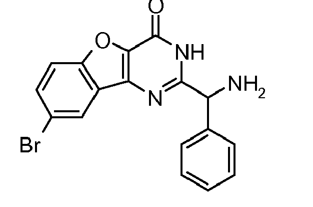 | 2-[amino(phényl)méthyl]-8-bromo[1]benzofuro[3,2-d] pyrimidin-4(3H)-one |
| 175 | 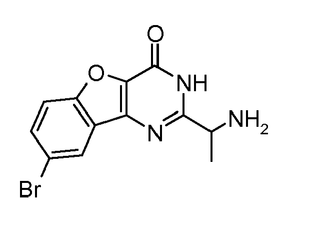 | 2-(1-aminoéthyl)-8-bromo[1]benzofuro[3,2-d] pyrimidin-4(3H)-one |
| 176 | 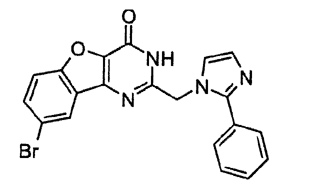 | 8-bromo-2-[(2-phényl-1H-imidazol-1-yl)méthyl][1]benzofuro[3,2-d] pyrimidin-4(3H)-one |
| 177 | 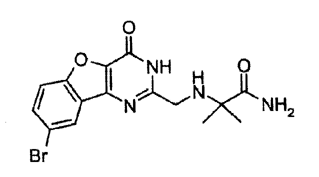 | N²-[(8-bromo-4-oxo-3,4-dihydro[1]benzofuro[3,2-d] pyrimidin-2-yl)méthyl]-2-méthylalaninamide |
| 180 | 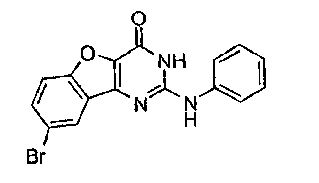 | 8-bromo-2-(phénylamino)[1]benzofuro[3,2-d] pyrimidin-4(3H)-one |
| 183 | 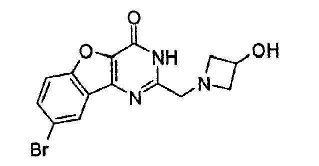 | 8-bromo-2-[(3-hydroxyazétidin-1 -yl)méthyl][1]benzofuro[3,2-d] pyrimidin-4(3H)-one |
| 184 | 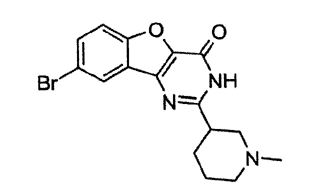 | 8-bromo-2-(1-méthylpipéridin-3-yl)[1]benzofuro[3,2-d]pyrimidin-4(3H)-one |
| 185 | 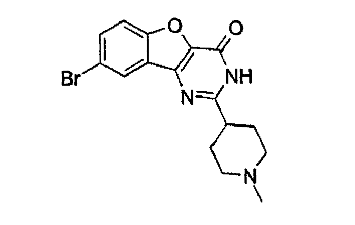 | 8-bromo-2-(1-méthylpipéridin-4-yl)[1]benzofuro[3,2-d]pyrimidin-4(3H)-one |
| 186 | 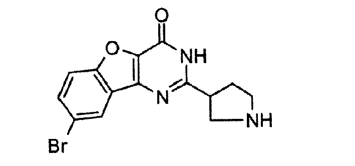 | 8-bromo-2-pyrrolidin-3-yl[1]benzofuro[3,2-d]pyrimidin-4(3H)-one |
| 187 | 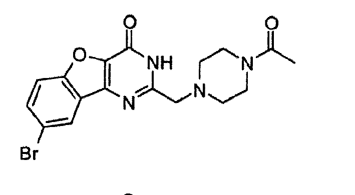 | 2-[(4-acétylpipérazin-1-yl)méthyl]-8-bromo[1]benzofuro[3,2-d] pyrimidin-4(3H)-one |
| 189 | 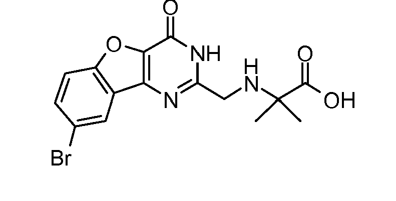 | N-[(8-bromo-4-oxo-3,4-dihydro[1]benzofuro[3,2-d] pyrimidin-2-yl)méthyl]-2-méthylalanine |
| 190 | 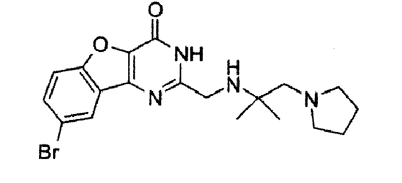 | 8-bromo-2-{[(1,1-diméthyl-2-pyrrolidin-1-yléthyl)amino] méthyl}[1]benzofuro[3,2-d] pyrimidin-4(3H)-one |
| 191 | 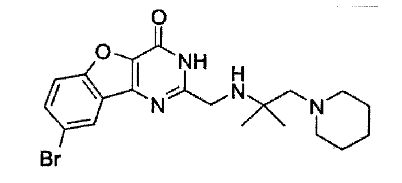 | 8-bromo-2-{[(1,1-diméthyl-2-pipéridin-1-yléthyl)amino] méthyl}[1]benzofuro[3,2-d] pyrimidin-4(3H)-one |
| 192 | 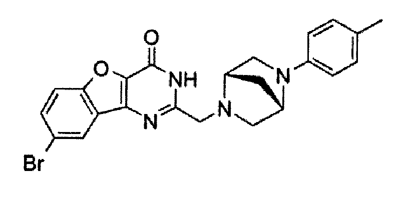 | 8-bromo-2-{(1S,4S)-5-(4-méthylphényl)-2,5-diazabicyclo[2.2.1]hept-2-yl] méthyl}[1]benzofuro[3,2-d] pyrimidin-4(3H)-one |
| 193 | 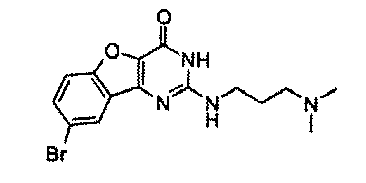 | 8-bromo-2-{[3-(diméthylamino)propyl]amino[1] benzofuro[3,2-d]pyrimidin-4(3H)-one |
| 194 | 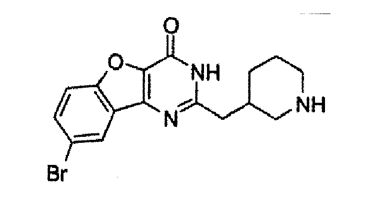 | 8-bromo-2-(pipéridin-3-ylméthyl)[1]benzofuro[3,2-d] pyrimidin-4(3H)-one |
| 195 | 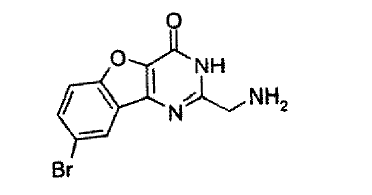 | 2-(aminométhyl)-8-bromo[1]benzofuro[3,2-d] pyrimidin-4(3H)-one |
| 196 | 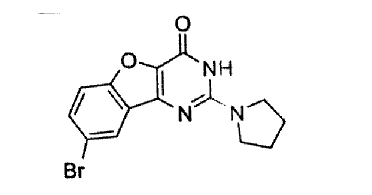 | 8-bromo-2-pyrrolidin-1-yl[1] benzofuro[3,2-d]pyrimidin-4(3H)-one |
| 198 | 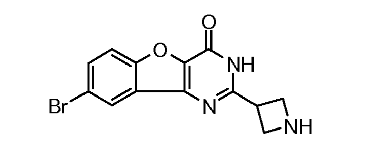 | 2-azétidin-3-yl-8-bromo[1] benzofuro[3,2-d]pyrimidin-4(3H)-one |
| 199 | 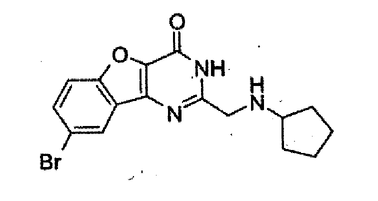 | 8-bromo-2-[(cyclopentylamino)méthyl][1] benzofuro[3,2-d]pyrimidin-4(3H)-one |
| 200 | 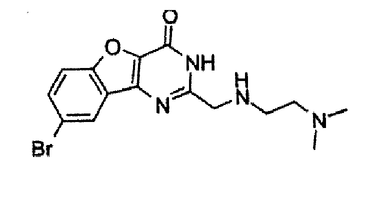 | 8-bromo-2-({[2-(diméthylamino) éthyl]amino}méthyl)[1]benzofuro [3,2-d]pyrimidin-4(3H)-one |
| 201 | 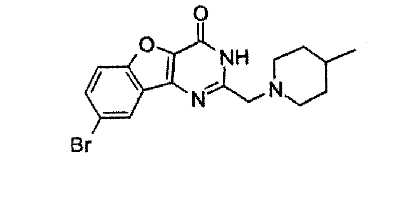 | 8-bromo-2-[(4-méthylpipéridin-1-yl)méthyl][1]benzofuro[3,2-d] pyrimidin-4(3H)-one |
| 202 | 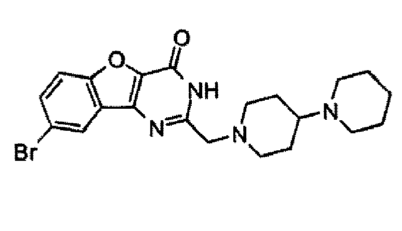 | 2-(1,4'-bipipéridin-1'-ylméthyl)-8-bromo[1]benzofuro[3,2-d] pyrimidin-4(3H)-one |
| 203 | 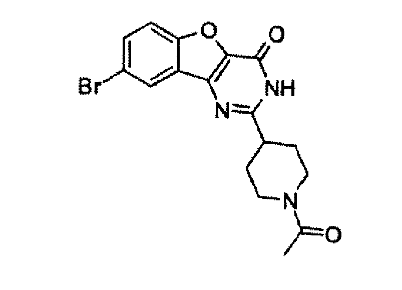 | 2-(1 -acétylpipéridin-4-yl)-8-bromo[1]benzofuro[3,2-d] pyrimidin-4(3H)-one |
| 204 | 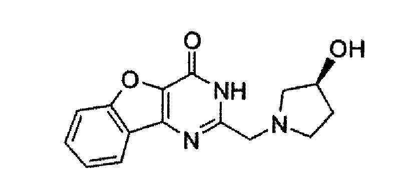 | 2-{[(3S)-3-hydroxypyrrolidin-1-yl] méthyl}[1]benzofuro[3,2-d] pyrimidin-4(3H)-one |
| 206 | 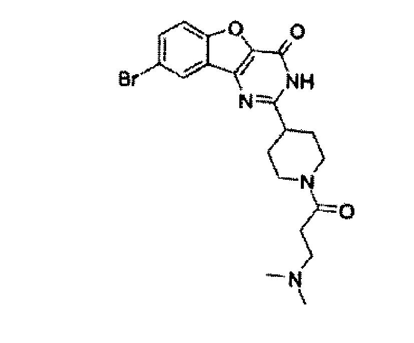 | 8-bromo-2-[1-(N,N-diméthyl-béta-alanyl)pipéridin-4-yl][1] benzofuro[3,2-d]pyrimidin-4(3H)-one |
| 207 | 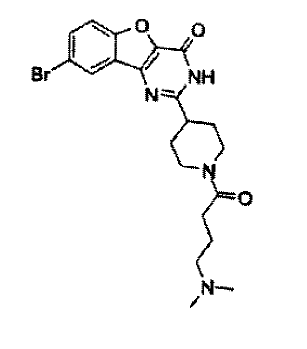 | 8-bromo-2-{1-[4-(diméthylamino) butanoyl]pipéridin-4-yl}[1] benzofuro[3,2-d]pyrimidin-4(3H)-one |
| 208 | 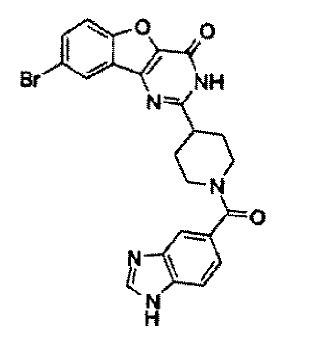 | 2-[1-(1H-benzimidazol-5-ylcarbonyl)pipéridin-4-yl]-8-bromo[1]benzofuro[3,2-d] pyrimidin-4(3H)-one |
| 209 | 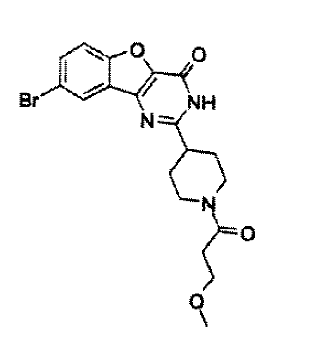 | 8-bromo-2-{1-[3-(méthyloxy) propanoyl]pipéridin-4-yl}[1] benzofuro[3,2-d]pyrimidin-4(3H)-one |
| 210 | 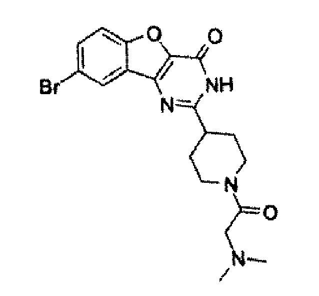 | 8-bromo-2-[1-(N,N-diméthylglycyl)pipéridin-4-yl][1] benzofuro[3,2-d]pyrimidin-4(3H)-one |
| 212 | 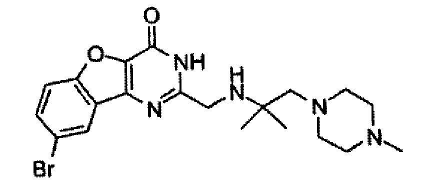 | 8-bromo-2-({[1,1-diméthyl-2-(4-méthylpipérazin-1-yl)éthyl] amino}méthyl)[1]benzofuro[3,2-d] pyrimidin-4(3H)-one |
| 213 | 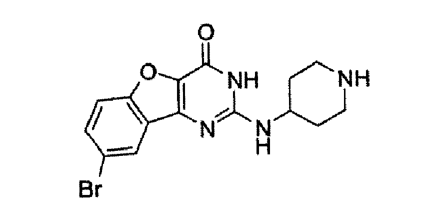 | 8-bromo-2-(pipéridin-4-ylamino)[1]benzofuro[3,2-d] pyrimidin-4(3H)-one |
| 214 | 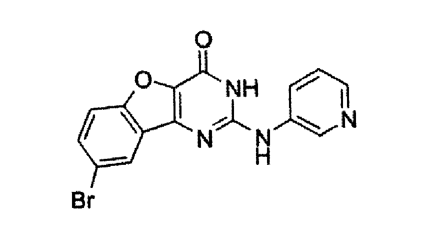 | 8-bromo-2-(pyridin-3-ylamino)[1]benzofuro[3,2-d] pyrimidin-4(3H)-one |
| 215 | 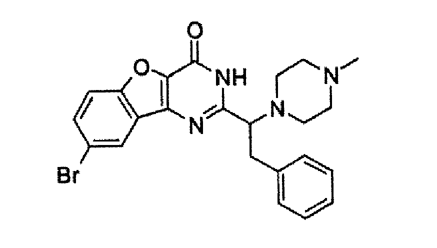 | 8-bromo-2-[1-(4-méthylpipérazin-1-yl)-2-phényléthyl][1] benzofuro[3,2-d]pyrimidin-4(3H)-one |
| 218 | 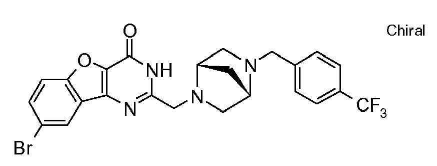 | 8-bromo-2-{[(1 S,4S)-5-{[4-(trifluorométhyl)phényl]méthyl}-2,5-diazabicyclo[2.2.1]hept-2-yl] méthyl}[1]benzofuro[3,2-d] pyrimidin-4(3H)-one |
| 221 | 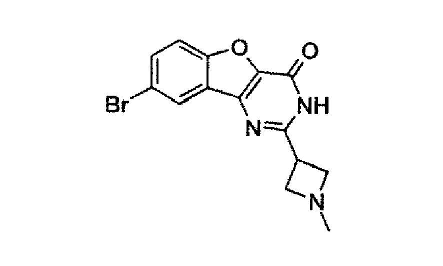 | 8-bromo-2-(1-méthylazétidin-3-yl)[1]benzofuro[3,2-d]pyrimidin-4(3H)-one |
| 224 | 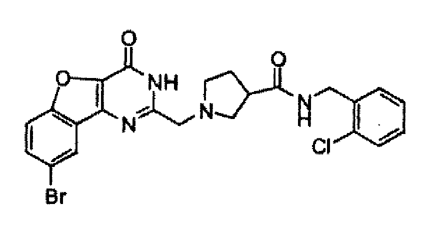 | 1-[(8-bromo-4-oxo-3,4- dihydro[1]benzofuro[3,2-d] pyrimidin-2-yl)méthyl]-N-[(2-chlorophényl)méthyl]pyrrolidine-3-carboxamide |
| 225 | 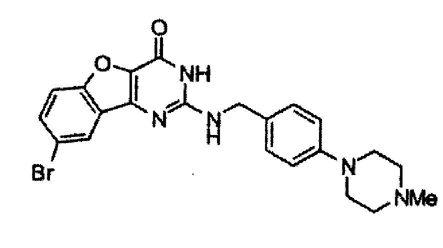 | 8-bromo-2-({[4-(4-méthylpipérazin-1-yl)phényl]méthyl}amino)[1] benzofuro[3,2-d]pyrimidin-4(3H)-one |
| 226 | 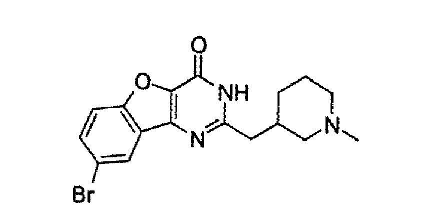 | 8-bromo-2-[(1-méthylpipéridin-3-yl)méthyl][1]benzofuro[3,2-d] pyrimidin-4(3H)-one |
| 227 | 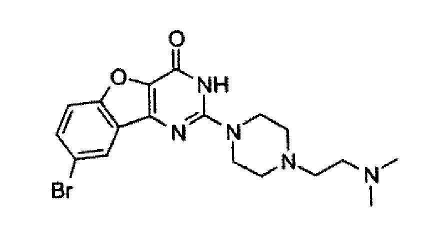 | 8-bromo-2-{4-[2-(diméthylamino)éthyl]pipérazin-1-yl}[1]benzofuro[3,2-d]pyrimidin-4(3H)-one |
| 228 | 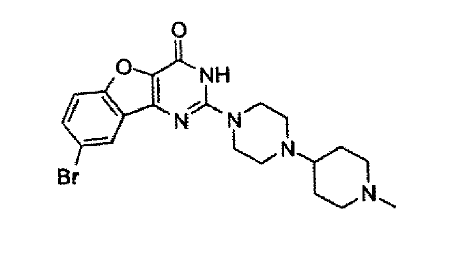 | 8-bromo-2-[4-(1-méthylpipéridin-4-yl)pipérazin-1-yl][1]benzofuro[3,2-d]pyrimidin-4(3H)-one |
| 229 | 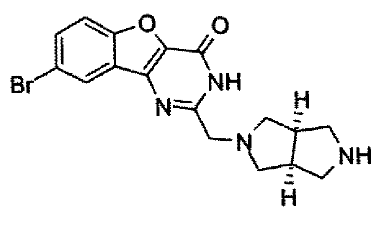 | 8-bromo-2-[(3aR,6aS)-hexahydropyrrolo[3,4-c]pyrrol- 2(1H)-ylméthyl][1]benzofuro[3,2-d] pyrimidin-4(3H)-one |
| 230 | 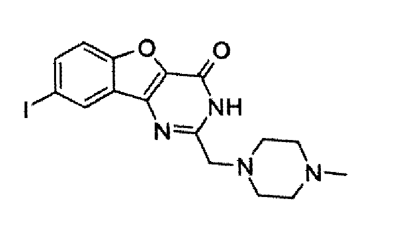 | 8-iodo-2-[(4-méthylpipérazin-1-yl)méthyl][1]benzofuro[3,2-d] pyrimidin-4(3H)-one |
| 232 | 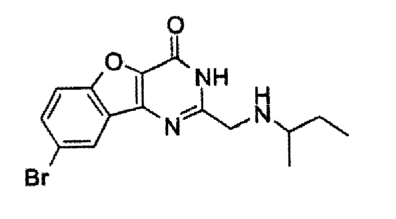 | 8-bromo-2-{[(1-méthylpropyl) amino]méthyl}[1]benzofuro[3,2-d] pyrimidin-4(3H)-one |
| 233 | 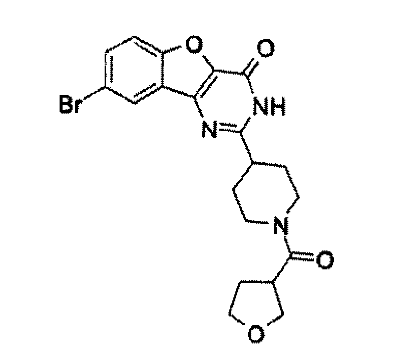 | 8-bromo-2-[1-(tétrahydrofuran-3-ylcarbonyl)pipéridin-4-yl][1] benzofuro[3,2-d]pyrimidin-4(3H)-one |
| 234 | 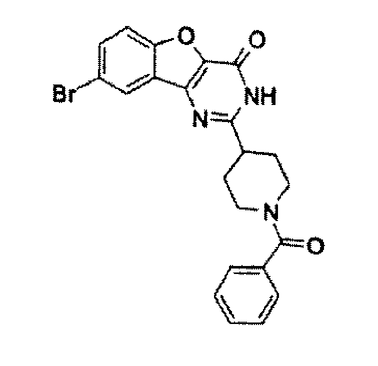 | 8-bromo-2-[1-(phénylcarbonyl)pipéridin-4-yl] [1]benzofuro[3,2-d]pyrimidin-4(3H)-one |
| 235 | 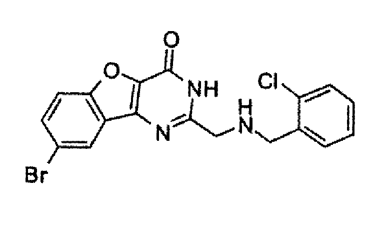 | 8-bromo-2-({[(2-chlorophényl)méthyl]amino} méthyl)[1]benzofuro[3,2-d] pyrimidin-4(3H)-one |
| 236 | 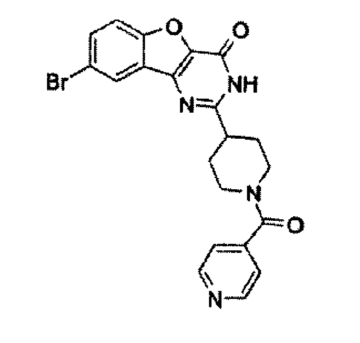 | 8-bromo-2-[1-(pyridin-4-ylcarbonyl)pipéridin-4-yl][1] benzofuro[3,2-d]pyrimidin-4(3H)-one |
| 237 | 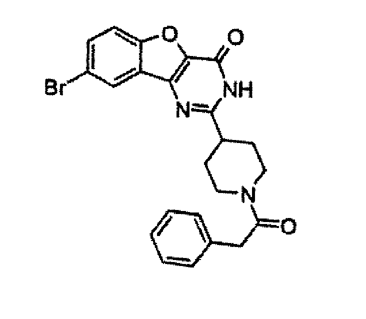 | 8-bromo-2-[1-(phénylacétyl)pipéridin-4-yl][1] benzofuro[3,2-d]pyrimidin-4(3H)-one |
| 238 | 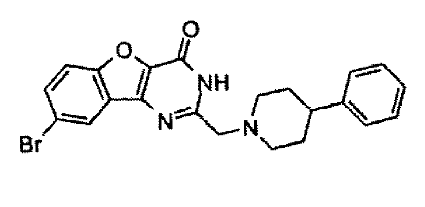 | 8-bromo-2-[(4-phénylpipéridin-1-yl)méthyl][1]benzofuro[3,2-d] pyrimidin-4(3H)-one |
| 239 | 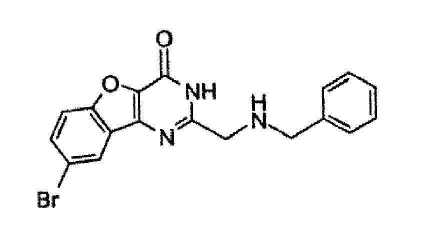 | 8-bromo-2-{[(phénylméthyl)amino]méthyl}[1] benzofuro[3,2-d]pyrimidin-4(3H)-one |
| 240 | 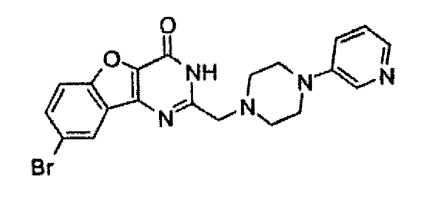 | 8-bromo-2-[(4-pyridin-3-ylpipérazin-1-yl)méthyl] [1]benzofuro[3,2-d]pyrimidin-4(3H)-one |
| 242 | 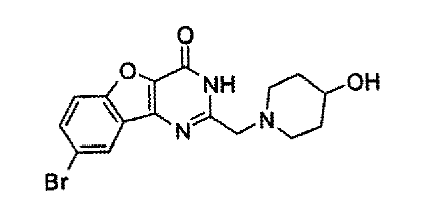 | 8-bromo-2-[(4-hydroxypipéridin-1-yl)méthyl][1]benzofuro[3,2-d] pyrimidin-4(3H)-one |
| 244 | 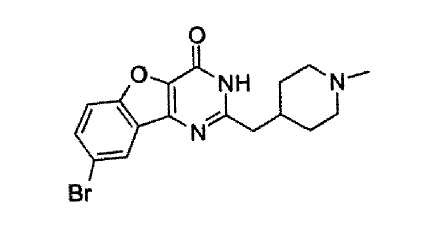 | 8-bromo-2-[(1-méthylpipéridin-4-yl)méthyl][1]benzofuro[3,2-d] pyrimidin-4(3H)-one |
| 245 | 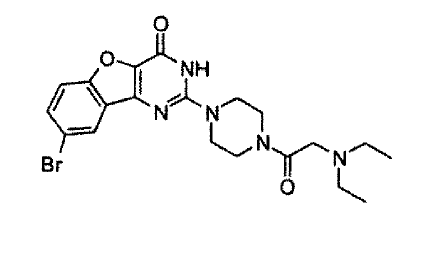 | 8-bromo-2-[4-(N,N-diéthylglycyl)pipérazin-1-yl][1] benzofuro[3,2-d]pyrimidin-4(3H)-one |
| 246 | 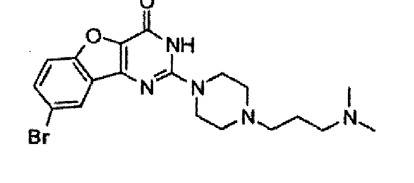 | 8-bromo-2-{4-[3-(diméthylamino)propyl]pipérazin-1-yl}[1]benzofuro[3,2-d]pyrimidin-4(3H)-one |
| 247 | 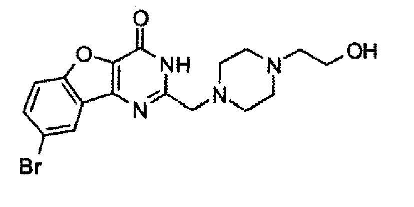 | 8-bromo-2-{[4-(2-hydroxyéthyl)pipérazin-1-yl] méthyl}[1]benzofuro[3,2-d] pyrimidin-4(3H)-one |
| 252 | 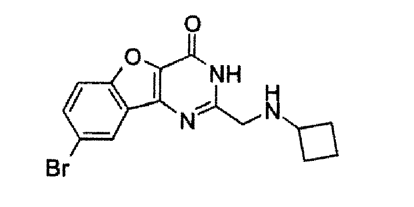 | 8-bromo-2-[(cyclobutylamino) méthyl][1]benzofuro[3,2-d] pyrimidin-4(3H)-one |
| 253 | 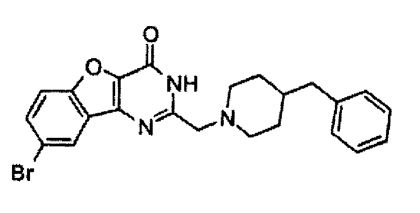 | 8-bromo-2-{[4-(phénylméthyl)pipéridin-1-yl] méthyl}[1]benzofuro[3,2-d] pyrimidin-4(3H)-one |
| 254 | 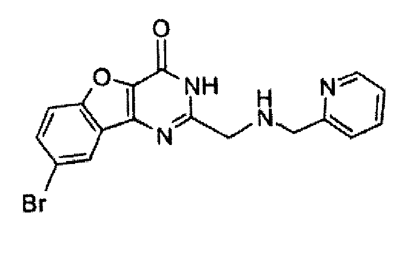 | 8-bromo-2-{[(pyridin-2-ylméthyl) amino]méthyl}[1]benzofuro[3,2-d] pyrimidin-4(3H)-one |
| 255 | 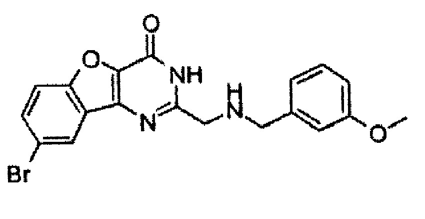 | 8-bromo-2-[({[3-(méthyloxy) phényl]méthyl}amino)méthyl][1] benzofuro[3,2-d]pyrimidin-4(3H)-one |
| 256 | 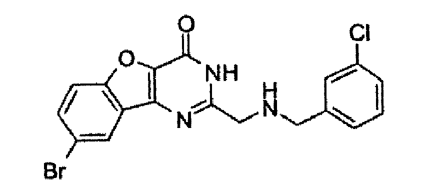 | 8-bromo-2-({[(3-chlorophényl) méthyl]amino}méthyl)[1]benzofuro [3,2-d]pyrimidin-4(3H)-one |
| 257 | 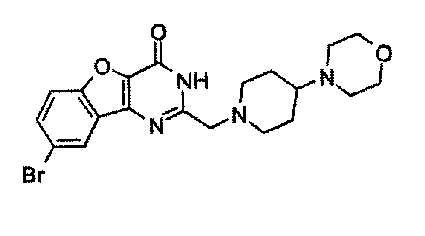 | 8-bromo-2-[(4-morpholin-4-ylpipéridin-1-yl)méthyl][1] benzofuro[3,2-d]pyrimidin-4(3H)- one |
| 258 | 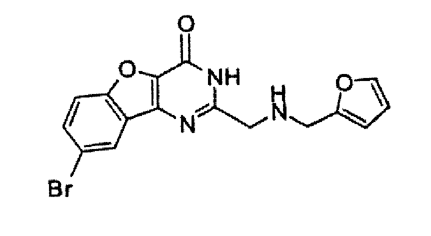 | 8-bromo-2-{[(furan-2-ylméthyl) amino]méthyl}[1]benzofuro[3,2-d] pyrimidin-4(3H)-one |
| 259 | 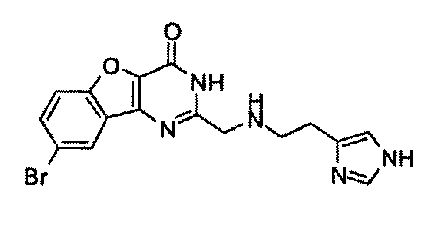 | 8-bromo-2-({[2-(1H-imidazol-4-yl) éthyl]amino}méthyl)[1]benzofuro [3,2-d]pyrimidin-4(3H)-one |
| 260 | 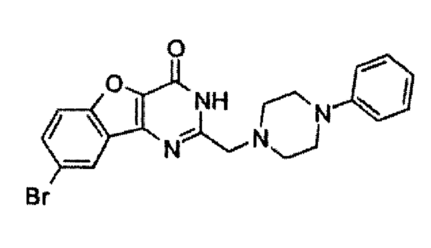 | 8-bromo-2-[(4-phénylpipérazin-1-yl)méthyl][1]benzofuro[3,2-d] pyrimidin-4(3H)-one |
| 261 | 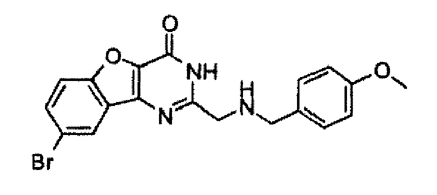 | 8-bromo-2-[({[4-(méthyloxy) phényl]méthyl}amino)méthyl][1] benzofuro[3,2-d]pyrimidin-4(3H)-one |
| 262 | 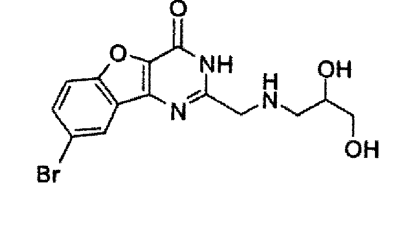 | 8-bromo-2-{[(2,3-dihydroxypropyl) amino]méthyl}[1]benzofuro[3,2-d] pyrimidin-4(3H)-one |
| 264 | 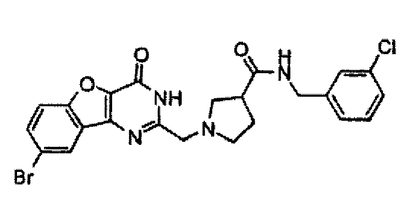 | 1-[(8-bromo-4-oxo-3,4-dihydro[1]benzofuro[3,2-d] pyrimidin-2-yl)méthyl]-N-[(3-chlorophényl)méthyl]pyrrolidine-3-carboxamide |
| 266 | 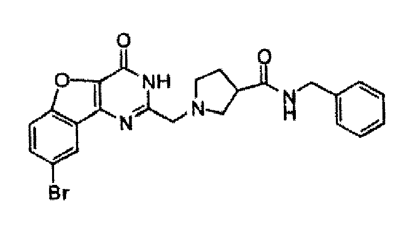 | 1-[(8-bromo-4-oxo-3,4-dihydro[1]benzofuro[3,2-d] pyrimidin-2-yl)méthyl]-N-(phénylméthyl)pyrrolidine-3-carboxamide |
| 267 | 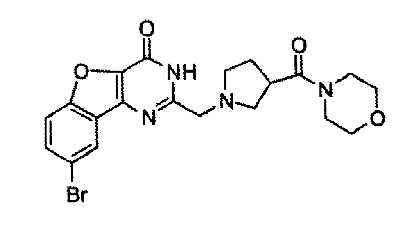 | 8-bromo-2-{[3-(morpholin-4-ylcarbonyl)pyrrolidin-1-yl] méthyl}[1]benzofuro[3,2-d] pyrimidin-4(3H)-one |
| 268 | 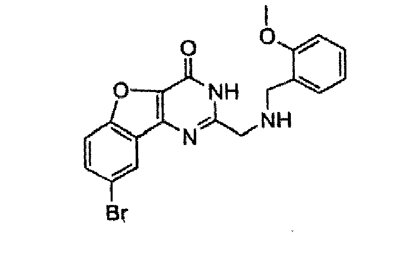 | 8-bromo-2-[({[2-(méthyloxy) phényl]méthyl}amino)méthyl][1] benzofuro[3,2-d]pyrimidin-4(3H)-one |
| 269 | 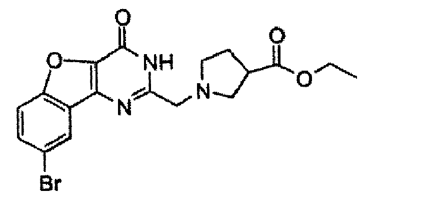 | éthyl-1-[(8-bromo-4-oxo-3,4-dihydro[1]benzofuro[3,2-d] pyrimidin-2-yl)méthyl]pyrrolidine-3-carboxylate |
| 270 | 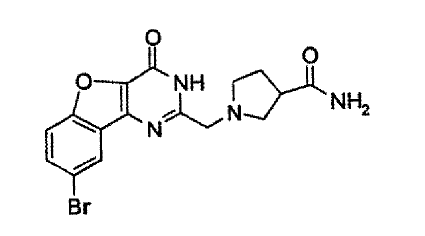 | 1-[(8-bromo-4-oxo-3,4-dihydro [1]benzofuro[3,2-d]pyrimidin-2-yl)méthyl]pyrrolidine-3-carboxamide |
| 271 | 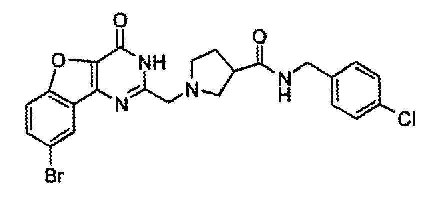 | 1-[(8-bromo-4-oxo-3,4-dihydro[1]benzofuro[3,2-d] pyrimidin-2-yl)méthyl]-N-[(4-chlorophényl)méthyl]pyrrolidine-3-carboxamide |
| 272 | 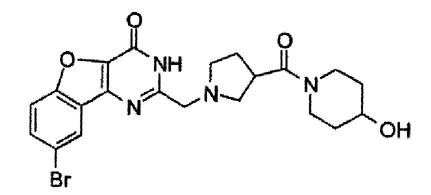 | 8-bromo-2-({3-[(4-hydroxypipéridin-1-yl) carbonyl]pyrrolidin-1-yl}méthyl) [1]benzofuro[3,2-d]pyrimidin-4(3H)-one |
| 273 | 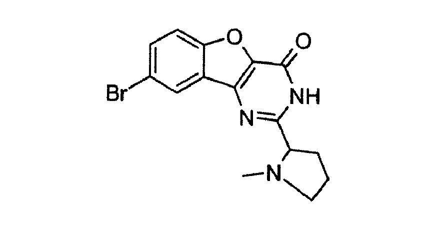 | 8-bromo-2-(1-méthylpyrrolidin-2-yl)[1]benzofuro[3,2-d]pyrimidin-4(3H)-one |
| 277 | 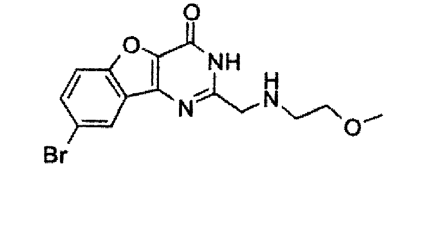 | 8-bromo-2-({[2-(méthyloxy) éthyl]amino}méthyl)[1]benzofuro [3,2-d]pyrimidin-4(3H)-one |
| 278 | 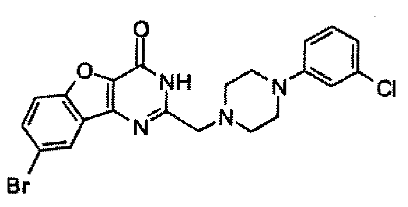 | 8-bromo-2-{[4-(3-chlorophényl) pipérazin-1-yl]méthyl}[1] benzofuro[3,2-d]pyrimidin-4(3H)-one |
| 286 | 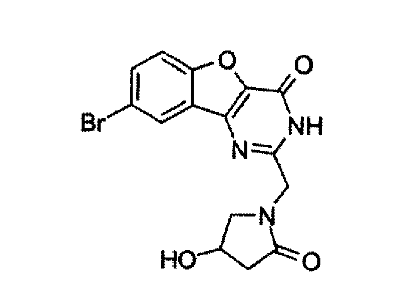 | 8-bromo-2-[(4-hydroxy-2-oxopyrrolidin-1-yl)méthyl] [1]benzofuro[3,2-d]pyrimidin-4(3H)-one |
| 287 | 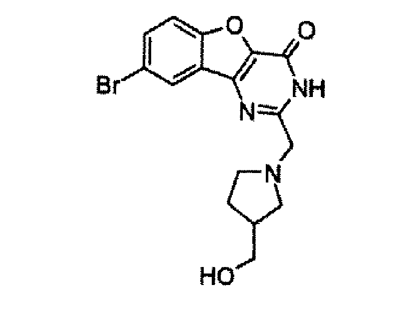 | 8-bromo-2-{[3-(hydroxyméthyl) pyrrolidin-1-yl]méthyl}[1] benzofuro[3,2-d]pyrimidin-4(3H)-one |
| 288 | 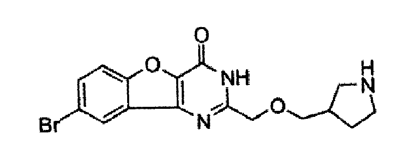 | 8-bromo-2-{[(pyrrolidin-3-ylméthyl)oxy]méthyl}[1]benzofuro [3,2-d]pyrimidin-4(3H)-one |
| 289 | 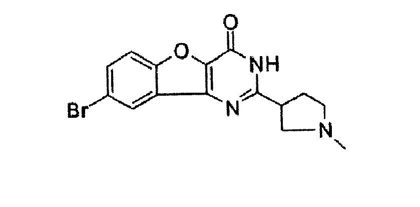 | 8-bromo-2-(1-méthylpyrrolidin-3-yl)[1]benzofuro[3,2-d]pyrimidin-4(3H)-one |
| 290 | 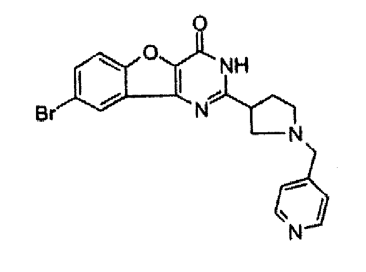 | 8-bromo-2-[1-(pyridin-4-ylméthyl)pyrrolidin-3-yl] [1]benzofuro[3,2-d]pyrimidin-4(3H)-one |
| 295 | 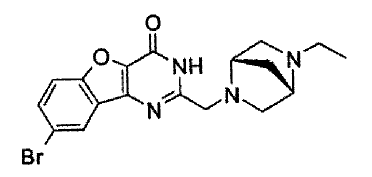 | 8-bromo-2-{[(1S,4S)-5-éthyl-2,5-diazabicyclo[2.2.1]hept-2-yl]méthyl}[1]benzofuro[3,2-d] pyrimidin-4(3H)-one |
| 298 | 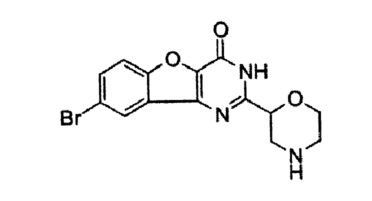 | 8-bromo-2-morpholin-2-yl[1]benzofuro[3,2-d]pyrimidin-4(3H)-one |
| 304 | 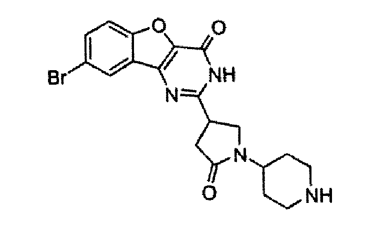 | 8-bromo-2-(5-oxo-1-pipéridin-4-ylpyrrolidin-3-yl)[1]benzofuro[3,2-d]pyrimidin-4(3H)-one |
| 305 | 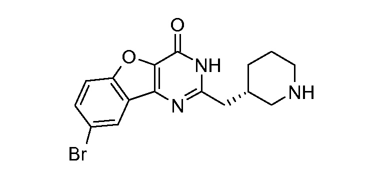 | 8-bromo-2-[(3S)-pipéridin-3-ylméthyl][1]benzofuro[3,2-d] pyrimidin-4(3H)-one |
| 306 | 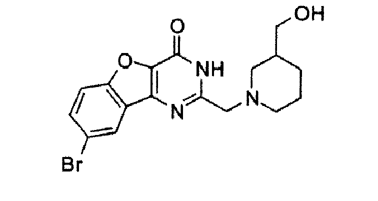 | 8-bromo-2-{[3-(hydroxyméthyl) pipéridin-1-yl]méthyl}[1] benzofuro[3,2-d]pyrimidin-4(3H)-one |
| 307 | 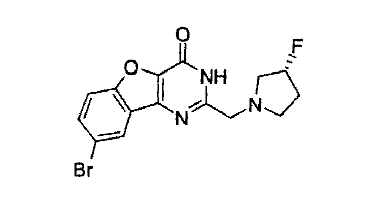 | 8-bromo-2-{[(3R)-3-fluoropyrrolidin-1-yl] méthyl}[1]benzofuro[3,2-d] pyrimidin-4(3H)-one |
| 308 | 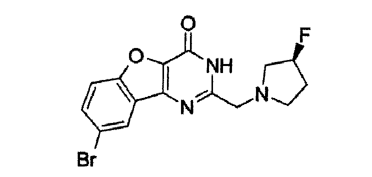 | 8-bromo-2-{[(3S)-3-fluoropyrrolidin-1-yl] méthyl}[1]benzofuro[3,2-d] pyrimidin-4(3H)-one |
| 309 | 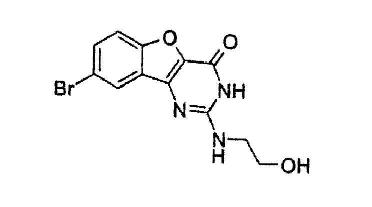 | 8-bromo-2-[(2-hydroxyéthyl) amino][1]benzofuro [3,2-d] pyrimidin-4(3H)-one |
| 311 | 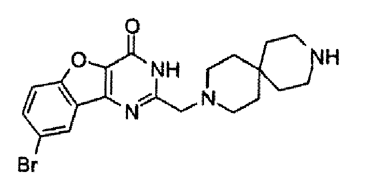 | 8-bromo-2-(3,9-diazaspiro [5.5]undec-3-ylméthyl) [1]benzofuro[3,2-d]pyrimidin-4(3H)-one |
| 315 | 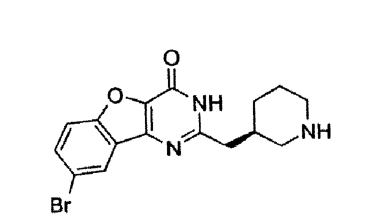 | 8-bromo-2-[(3R)-pipéridin-3-ylméthyl][1]benzofuro[3,2-d] pyrimidin-4(3H)-one |
| 317 | 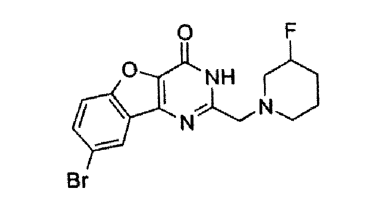 | 8-bromo-2-[(3-fluoropipéridin-1-yl)méthyl][1]benzofuro[3,2-d] pyrimidin-4(3H)-one |
| 318 | 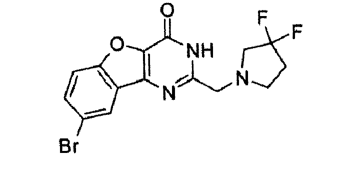 | 8-bromo-2-[(3,3-difluoropyrrolidin-1-yl)méthyl][1]benzofuro[3,2-d] pyrimidin-4(3H)-one |
| 330 | 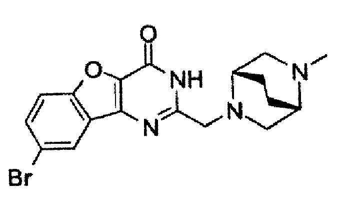 | 8-bromo-2-{[(1S,4S)-5-méthyl-2,5-diazabicyclo[2.2.2]oct-2-yl] méthyl}[1]benzofuro[3,2-d] pyrimidin-4(3H)-one |
| 331 | 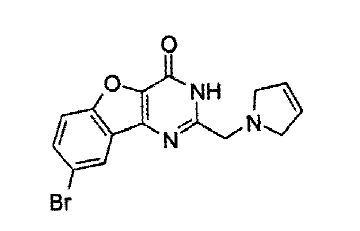 | 8-bromo-2-((2,5-dihydro-1H-pyrrol-1-yl)méthyl)benzofuro[3,2-d] pyrimidin-4(3H)-one |
| 332 | 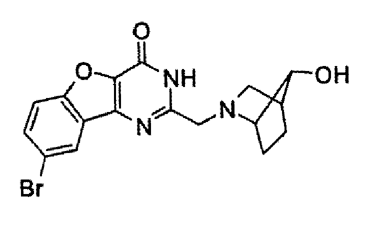 | 8-bromo-2-((7-hydroxy-2-azabicyclo[2.2.1]heptan-2-yl) méthyl)benzofuro[3,2-d]pyrimidin-4(3H)-one |
| 344 | 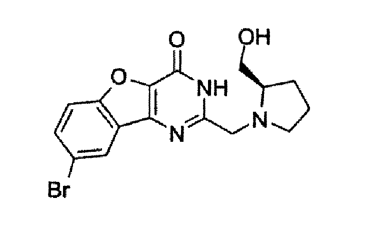 | 8-bromo-2-{[(2R)-2-(hydroxyméthyl)pyrrolidin-1-yl] méthyl}[1]benzofuro[3,2-d] pyrimidin-4(3H)-one |
| 346 | 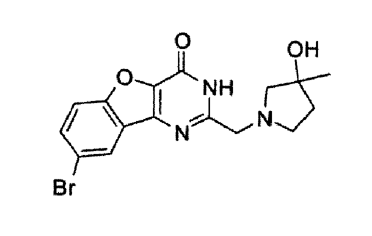 | 8-bromo-2-[(3-hydroxy-3-méthylpyrrolidin-1-yl) méthyl][1]benzofuro[3,2-d] pyrimidin-4(3H)-one |
| 366 | 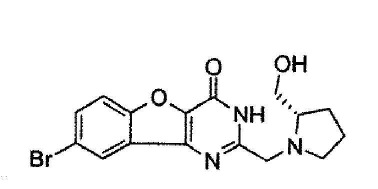 | 8-bromo-2-{[(2S)-2-(hydroxyméthyl)pyrrolidin-1-yl] méthyl}[1]benzofuro[3,2-d] pyrimidin-4(3H)-one |
| 367 | 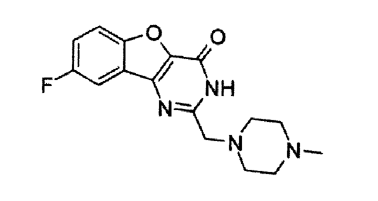 | 8-fluoro-2-[(4-méthylpipérazin-1-yl)méthyl][1]benzofuro[3,2-d] pyrimidin-4(3H)-one |
| 368 | 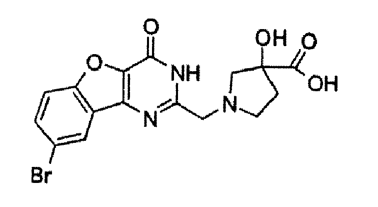 | 1-[(8-bromo-4-oxo-3,4-dihydro[1]benzofuro[3,2-d] pyrimidin-2-yl)méthyl]-3-hydroxypyrrolidine-3-acide carboxylique |
| 369 | 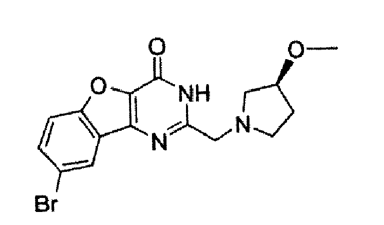 | 8-bromo-2-{[(3S)-3-(méthyloxy)pyrrolidin-1-yl] méthyl}[1]benzofuro[3,2-d] pyrimidin-4(3H)-one |
| 370 | 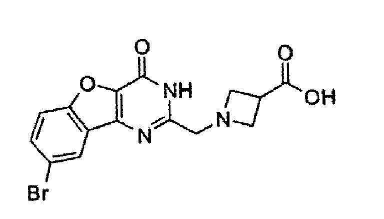 | 1-[(8-bromo-4-oxo-3,4-dihydro[1]benzofuro[3,2-d] pyrimidin-2-yl)méthyl]azétidine-3-acide carboxylique |
| 378 | 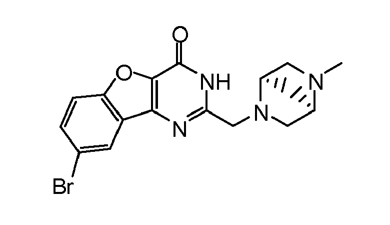 | 8-bromo-2-(((1R,4R)-5-méthyl-2,5-diazabicyclo[2.2.1]heptan-2-yl) méthyl)benzofuro[3,2-d]pyrimidin-4(3H)-one |
| 388 | 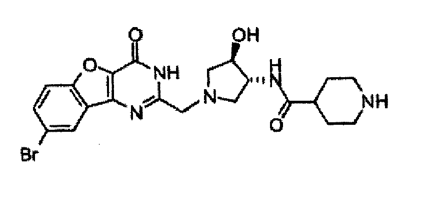 | N-{(3R,4R)-1-[(8-bromo-4-oxo-3,4-dihydro[1]benzofuro[3,2-d] pyrimidin-2-yl)méthyl]-4-hydroxypyrrolidin-3-yl}pipéridine-4-carboxamide |
| 398 | 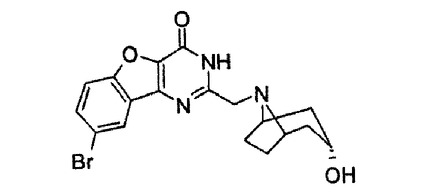 | 8-bromo-2-[(3-hydroxy-8-azabicyclo[3.2.1]oct-8-yl) méthyl][1]benzofuro[3,2-d] pyrimidin-4(3H)-one |
| 400 | 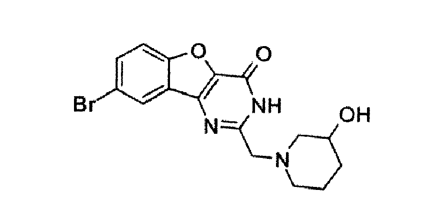 | 8-bromo-2-[(3-hydroxypipéridin-1-yl)méthyl][1]benzofuro[3,2-d] pyrimidin-4(3H)-one |
| 401 | 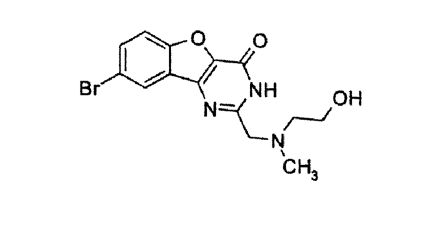 | 8-bromo-2-{[(2-hydroxyéthyl) (méthyl)amino]méthyl}[1] benzofuro[3,2-d]pyrimidin-4(3H)-one |
| 402 | 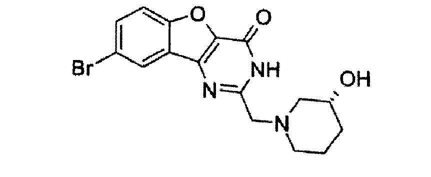 | 8-bromo-2-{[(3R)-3-hydroxypipéridin-1-yl] méthyl}[1]benzofuro[3,2-d] pyrimidin-4(3H)-one |
| 403 | 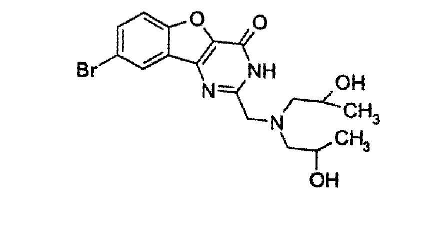 | 2-{[bis(2-hydroxypropyl) amino]méthyl}-8-bromo[1]benzofuro[3,2-d] pyrimidin-4(3H)-one |
| 404 | 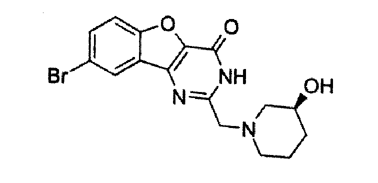 | 8-bromo-2-{[(3S)-3-hydroxypipéridin-1-yl] méthyl}[1]benzofuro[3,2-d] pyrimidin-4(3H)-one |
| 407 | 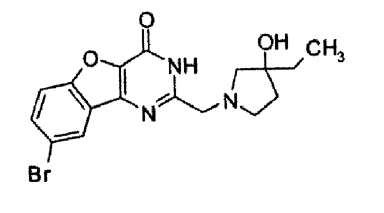 | 8-bromo-2-[(3-éthyl-3-hydroxypyrrolidin-1-yl) méthyl][1]benzofuro[3,2-d] pyrimidin-4(3H)-one |
| 408 | 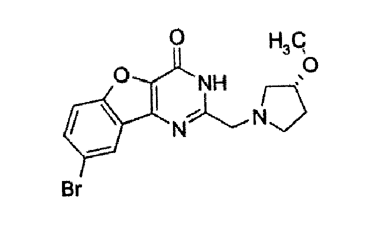 | 8-bromo-2-{[(3R)-3-(méthyloxy)pyrrolidin-1-yl] méthyl}[1]benzofuro[3,2-d] pyrimidin-4(3H)-one |
| 409 | 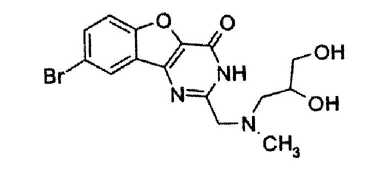 | 8-bromo-2-{[(2,3-dihydroxypropyl)(méthyl)amino] méthyl}[1]benzofuro[3,2-d] pyrimidin-4(3H)-one |
| 410 | 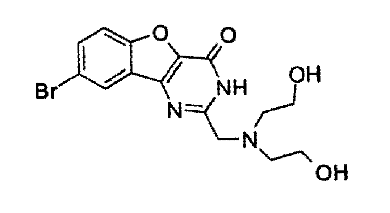 | 2-{[bis(2-hydroxyéthyl) amino]méthyl}-8-bromo[1]benzofuro[3,2-d] pyrimidin-4(3H)-one |
| 412 | 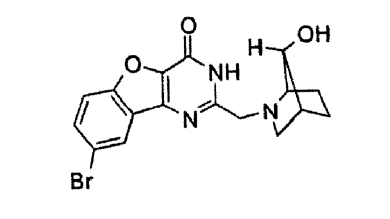 | 8-bromo-2-[(7-hydroxy-2-azabicyclo[2.2.1]hept-2-yl) méthyl][1]benzofuro[3,2-d] pyrimidin-4(3H)-one |
| 413 | 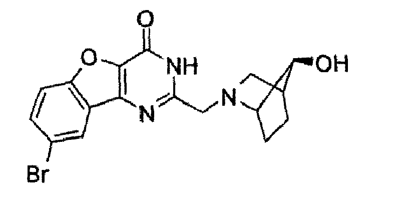 | 8-bromo-2-{[(7S)-7-hydroxy-2-azabicyclo[2.2.1]hept-2-yl] méthyl}[1]benzofuro[3,2-d] pyrimidin-4(3H)-one |
| 417 | 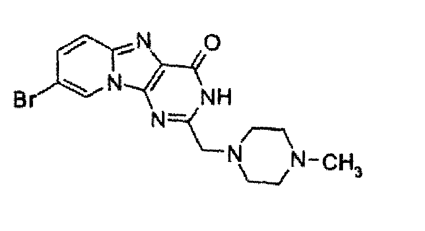 | 8-bromo-2-[(4-méthylpipérazin-1-yl)méthyl]pyrido[1,2-e]purin-4(3H)-one |
| 418 | 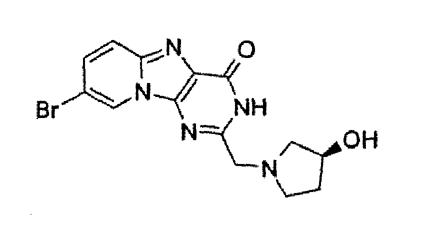 | 8-bromo-2-{[(3S)-3-hydroxypyrrolidin-1-yl] méthyl}pyrido[1,2-e]purin-4(3H)-one |
| 420 | 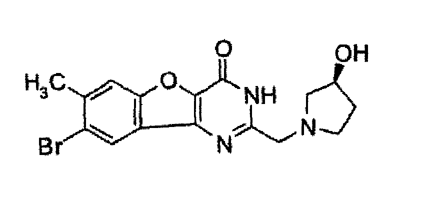 | 8-bromo-2-{[(3S)-3-hydroxypyrrolidin-1-yl]méthyl}-7-méthyl[1]benzofuro[3,2-d] pyrimidin-4(3H)-one |
| 424 | 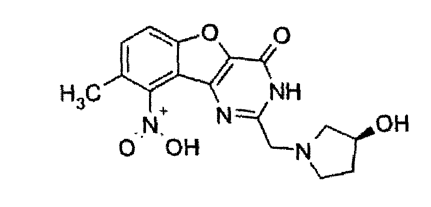 | hydroxy(2-{[(3S)-3-hydroxypyrrolidin-1-yl]méthyl}-8-méthyl-4-oxo-3,4-dihydro[1] benzofuro[3,2-d]pyrimidin-9-yl) oxoammonium |
| 425 | 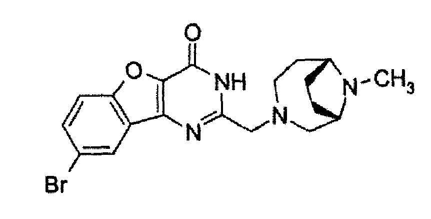 | 8-bromo-2-{[(1S,6R)-9-méthyl-3,9-diazabicyclo[4.2.1]non-3-yl] méthyl}[1]benzofuro[3,2-d] pyrimidin-4(3H)-one |
| 426 | 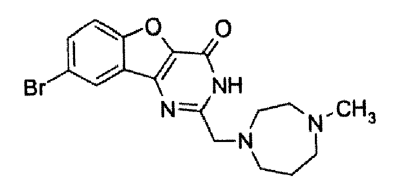 | 8-bromo-2-[(4-méthyl-1,4-diazepan-1-yl)méthyl] [1]benzofuro[3,2-d]pyrimidin-4(3H)-one |
| 428 | 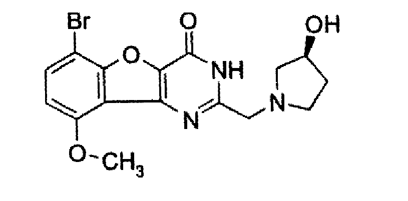 | 6-bromo-2-{[(3S)-3-hydroxypyrrolidin-1-yl]méthyl}-9-(méthyloxy)[1]benzofuro[3,2-d] pyrimidin-4(3H)-one |
| 429 | 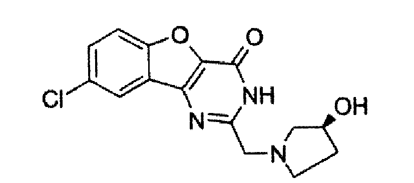 | 8-chloro-2-{[(3S)-3-hydroxypyrrolidin-1-yl]méthyl}[1]benzofuro[3,2-d] pyrimidin-4(3H)-one |
| 431 | 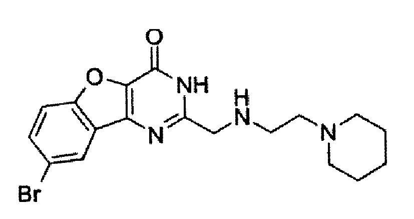 | 8-bromo-2-{[(2-pipéridin-1-yléthyl)amino]méthyl}[1]benzofuro [3,2-d]pyrimidin-4(3H)-one |
| 432 | 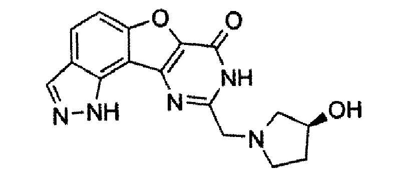 | 9-{[(3S)-3-hydroxypyrrolidin-1-yl]méthyl}-1H-pyrimido [4',5':4,5]furo[2,3-g]indazol-7(8H)-one |
| 433 | 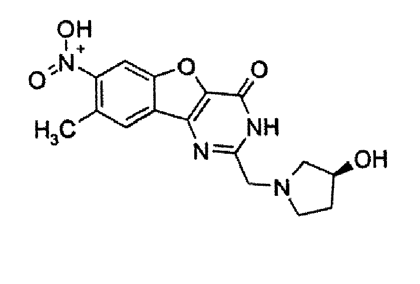 | hydroxy(2-{[(3S)-3-hydroxypyrrolidin-1-yl]méthyl}-8-méthyl-4-oxo-3,4-dihydro[1]benzofuro[3,2-d] pyrimidin-7-yl)oxoammonium |
| 434 | 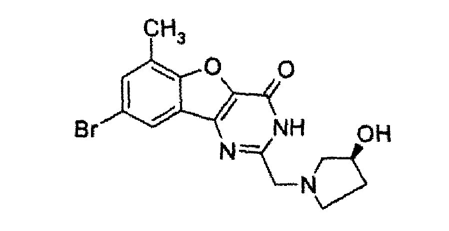 | 8-bromo-2-{[(3S)-3-hydroxypyrrolidin-1-yl]méthyl}-6-méthyl[1]benzofuro[3,2-d] pyrimidin-4(3H)-one |
| 436 | 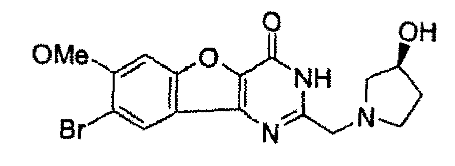 | 8-bromo-2-{[(3S)-3-hydroxypyrrolidin-1-yl]méthyl}-7-(méthyloxy)[1]benzofuro[3,2-d] pyrimidin-4(3H)-one |
| 437 | 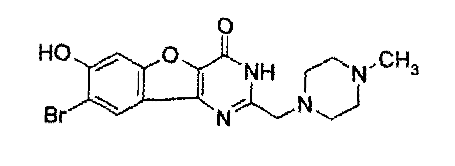 | 8-bromo-7-hydroxy-2-{[(3S)-3-hydroxypyrrolidin-1-yl] méthyl}[1]benzofuro[3,2-d] pyrimidin-4(3H)-one |
| 438 | 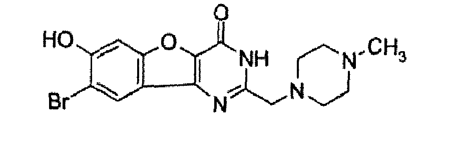 | 8-bromo-7-hydroxy-2-[(4-méthylpipérazin-1-yl) méthyl][1]benzofuro[3,2-d] pyrimidin-4(3H)-one |
| 440 | 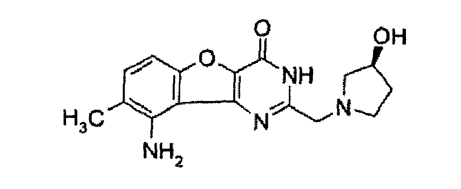 | 9-amino-2-{[(3S)-3-hydroxypyrrolidin-1-yl]méthyl}-8-méthyl[1]benzofuro[3,2-d] pyrimidin-4(3H)-one |
| 442 | 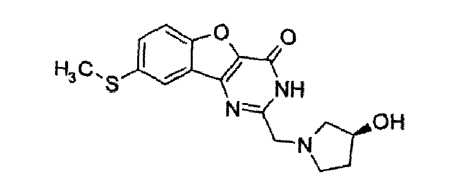 | 2-{[(3S)-3-hydroxypyrrolidin-1-yl] méthyl}-8-(méthylthio)[1]benzofuro[3,2-d] pyrimidin-4(3H)-one |
| 443 | 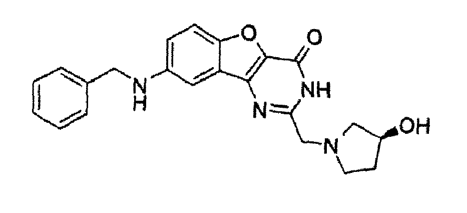 | 2-{[(3S)-3-hydroxypyrrolidin-1-yl]méthyl}-8-[(phénylméthyl) amino][1]benzofuro[3,2-d] pyrimidin-4(3H)-one |
| 444 | 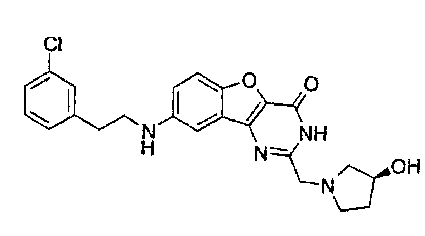 | 8-{[2-(3-chlorophényl) éthyl]amino}-2-{[(3S)-3-hydroxypyrrolidin-1-yl] méthyl}[1]benzofuro[3,2-d] pyrimidin-4(3H)-one |
| 445 | 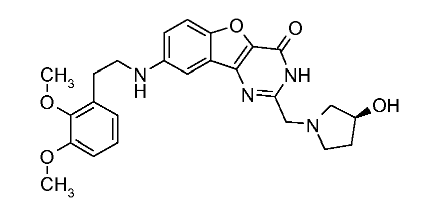 | 8-({2-[2,3-bis(méthyloxy) phényl]éthyl}amino)-2-{[(3S)-3-hydroxypyrrolidin-1-yl] méthyl}[1]benzofuro[3,2-d] pyrimidin-4(3H)-one |
| 446 | 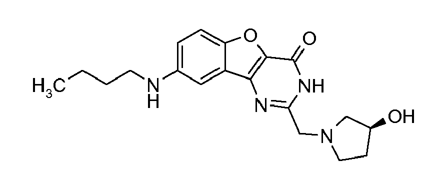 | 8-(butylamino)-2-{[(3S)-3-hydroxypyrrolidin-1-yl]méthyl} [1]benzofuro[3,2-d]pyrimidin-4(3H)-one |
| 447 | 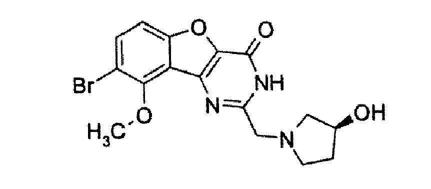 | 8-bromo-2-{[(3S)-3-hydroxypyrrolidin-1-yl]méthyl}-9-(méthyloxy)[1]benzofuro[3,2-d] pyrimidin-4(3H)-one |
| 448 | 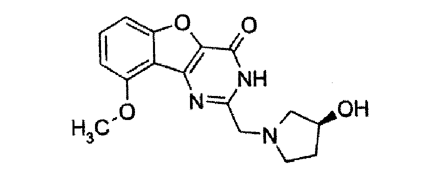 | 2-{[(3S)-3-hydroxypyrrolidin-1-yl]méthyl}-9-(méthyloxy)[1] benzofuro[3,2-d]pyrimidin-4(3H)-one |
| 449 | 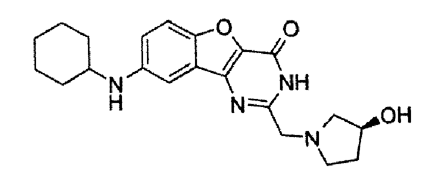 | 8-(cyclohexylamino)-2-{[(3S)-3-hydroxypyrrolidin-1-yl] méthyl}[1]benzofuro[3,2-d] pyrimidin-4(3H)-one |
| 450 | 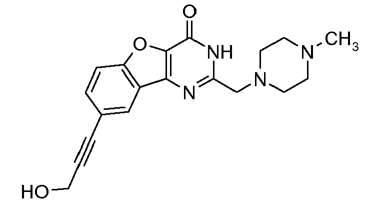 | 8-(3-hydroxyprop-1-yn-1-yl)-2-[(4-méthylpipérazin-1-yl) méthyl][1]benzofuro[3,2-d] pyrimidin-4(3H)-one |
| 451 | 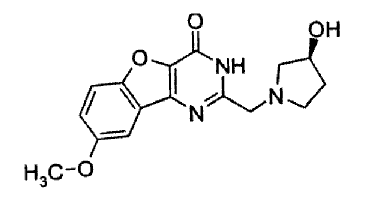 | 2-{[(3S)-3-hydroxypyrrolidin-1-yl]méthyl}-8-(méthyloxy) [1]benzofuro[3,2-d]pyrimidin-4(3H)-one |
| 452 | 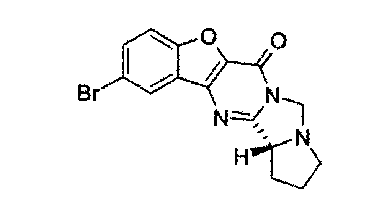 | (13bR)-11-bromo-1,2,3,13b-tétrahydro-7H-[1]benzofuro[3,2-d] pyrrolo[1',2':3,4]imidazo[1,5-a] pyrimidin-7-one |
| 453 | 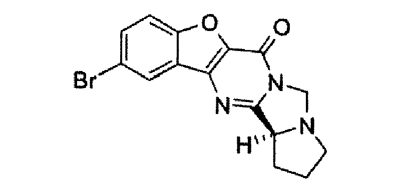 | (13bS)-11-bromo-1,2,3,13b-tétrahydro-7H-[1]benzofuro[3,2-d] pyrrolo[1',2':3,4]imidazo[1,5-a] pyrimidin-7-one |
| 454 | 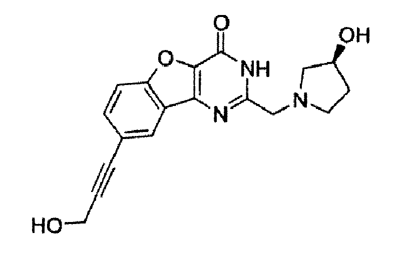 | 8-(3-hydroxyprop-1-yn-1-yl)-2-{[(3S)-3-hydroxypyrrolidin-1-yl] méthyl}[1]benzofuro[3,2-d] pyrimidin-4(3H)-one |
| 455 | 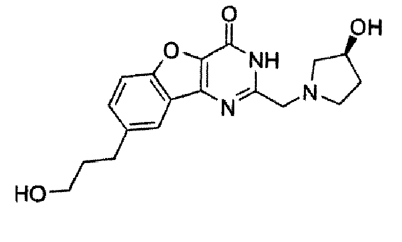 | 8-(3-hydroxypropyl)-2-{[(3S)-3-hydroxypyrrolidin-1-yl] méthyl}[1]benzofuro[3,2-d] pyrimidin-4(3H)-one |
| 456 | 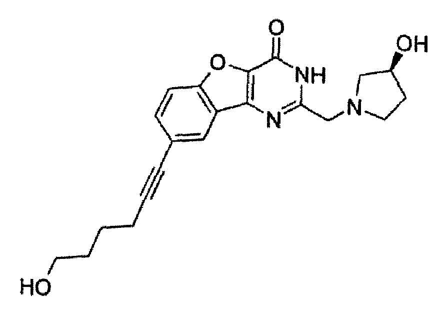 | 8-(6-hydroxyhex-1-yn-1-yl)-2-{[(3S)-3-hydroxypyrrolidin-1-yl] méthyl}[1]benzofuro[3,2-d] pyrimidin-4(3H)-one |
| 457 | 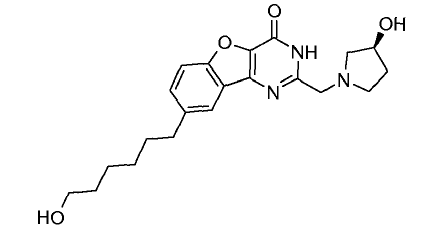 | 8-(6-hydroxyhexyl)-2-{[(3S)-3-hydroxypyrrolidin-1-yl] méthyl}[1]benzofuro[3,2-d] pyrimidin-4(3H)-one |
| 458 | 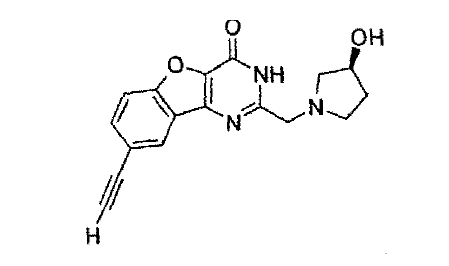 | 8-éthynyl-2-{[(3S)-3-hydroxypyrrolidin-1-yl]méthyl}[1]benzofuro[3,2-d] pyrimidin-4(3H)-one |
| 459 | 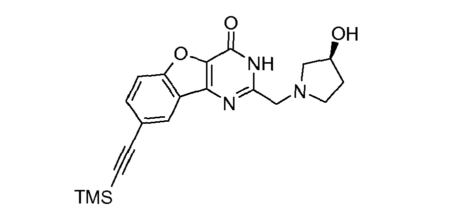 | 2-{[(3S)-3-hydroxypyrrolidin-1-yl]méthyl}-8-[(triméthylsilyl) éthynyl][1]benzofuro[3,2-d] pyrimidin-4(3H)-one |
| 460 | 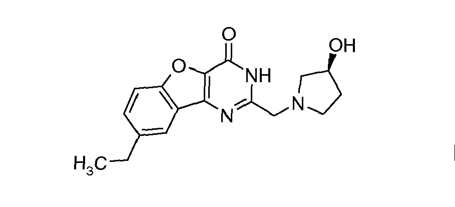 | 8-éthyl-2-{[(3S)-3-hydroxypyrrolidin-1-yl] méthyl}[1]benzofuro[3,2-d] pyrimidin-4(3H)-one |
| 461 | 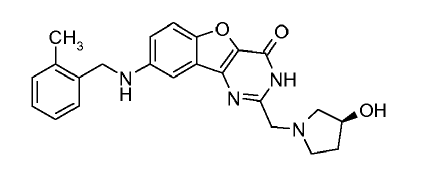 | 2-{[(3S)-3-hydroxypyrrolidin-1-yl]méthyl}-8-{[(2-méthylphényl) méthyl]amino}[1]benzofuro[3,2-d] pyrimidin-4(3H)-one |
| 462 | 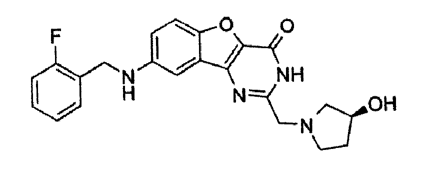 | 8-{[(2-fluorophényl)méthyl]amino}-2-{[(3S)-3-hydroxypyrrolidin-1-yl]méthyl}[1]benzofuro[3,2-d] pyrimidin-4(3H)-one |
| 463 | 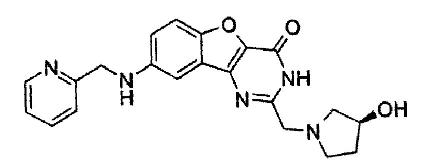 | 2-{[(3S)-3-hydroxypyrrolidin-1-yl]méthyl}-8-[(pyridin-2-ylméthyl)amino][1]benzofuro[3,2-d]pyrimidin-4(3H)-one |
| 464 | 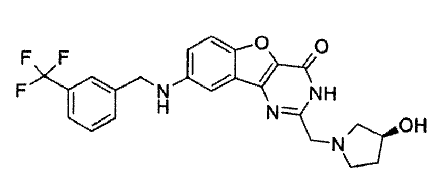 | 2-{[(3S)-3-hydroxypyrrolidin-1-yl]méthyl}-8-({[3-(trifluorométhyl) phényl]méthyl}amino)[1]benzofuro [3,2-d]pyrimidin-4(3H)-one |
| 465 | 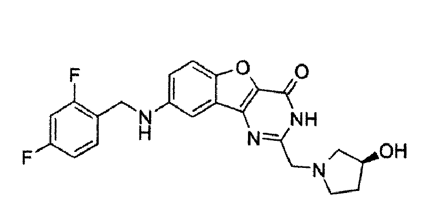 | 8-{[(2,4-difluorophényl) méthyl]amino}-2-{[(3S)-3-hydroxypyrrolidin-1-yl] méthyl}[1]benzofuro[3,2-d] pyrimidin-4(3H)-one |
| 466 | 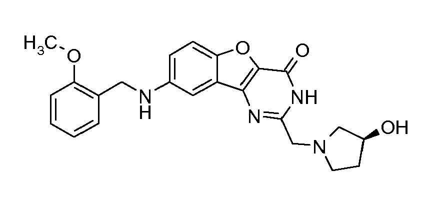 | 2-{[(3S)-3-hydroxypyrrolidin-1-yl] méthyl}-8-({[2-(méthyloxy) phényl]méthyl}amino)[1]benzofuro [3,2-d]pyrimidin-4(3H)-one |
| 467 | 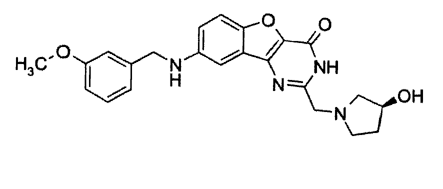 | 2-{[(3S)-3-hydroxypyrrolidin-1-yl]méthyl}-8-({[3-(méthyloxy) phényl]méthyl}amino)[1]benzofuro [3,2-d]pyrimidin-4(3H)-one |
| 468 | 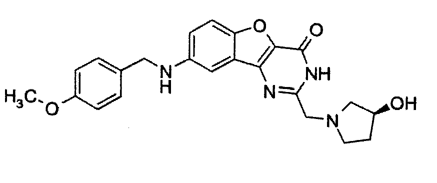 | 2-{[(3S)-3-hydroxypyrrolidin-1-yl]méthyl}-8-({[4-(méthyloxy) phényl]méthyl}amino)[1]benzofuro [3,2-d]pyrimidin-4(3H)-one |
| 469 | 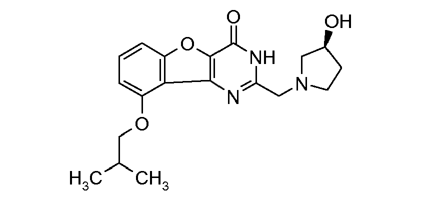 | 2-{[(3S)-3-hydroxypyrrolidin-1-yl]méthyl}-9-[(2-méthylpropyl) oxy][1]benzofuro[3,2-d]pyrimidin-4(3H)-one |
| 470 | 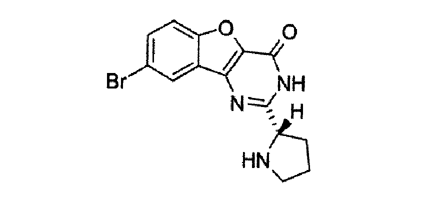 | 8-bromo-2-[(2S)-pyrrolidin-2-yl] [1]benzofuro[3,2-d]pyrimidin-4(3H)-one |
| 471 | 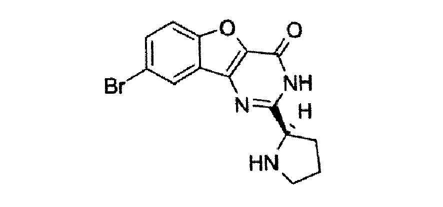 | 8-bromo-2-[(2R)-pyrrolidin-2-yl] [1]benzofuro[3,2-d]pyrimidin-4(3H)-one |
| 472 | 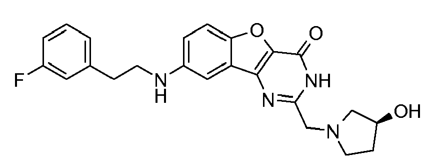 | 8-{[2-(3-fluorophényl)éthyl]amino}-2-{[(3S)-3-hydroxypyrrolidin-1-yl]méthyl}[1]benzofuro[3,2-d] pyrimidin-4(3H)-one |
| 473 | 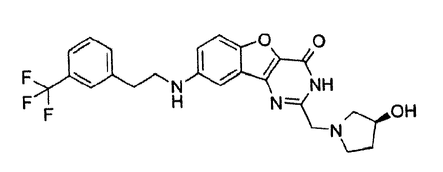 | 2-{[(3S)-3-hydroxypyrrolidin-1-yl]méthyl}-8-({2-[3-(trifluorométhyl) phényl]éthyl}amino)[1]benzofuro [3,2-d]pyrimidin-4(3H)-one |
| 474 | 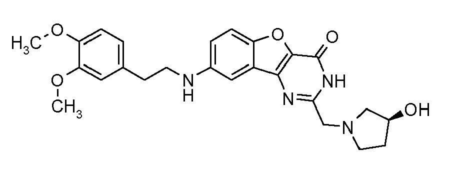 | 8-({2-[3,4-bis(méthyloxy) phényl]éthyl}amino)-2-{[(3S)-3-hydroxypyrrolidin-1-yl] méthyl}[1]benzofuro[3,2-d] pyrimidin-4(3H)-one |
| 475 | 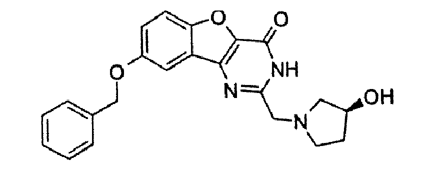 | 2-{[(3S)-3-hydroxypyrrolidin-1-yl] méthyl}-8-[(phénylméthyl) oxy][1]benzofuro[3,2-d]pyrimidin-4(3H)-one |
| 476 | 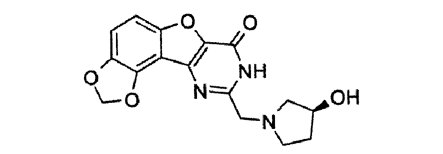 | 9-{[(3S)-3-hydroxypyrrolidin-1-yl] méthyl}[1,3]dioxolo[4,5][1] benzofuro[3,2-d]pyrimidin-7(8H)-one |
| 477 | 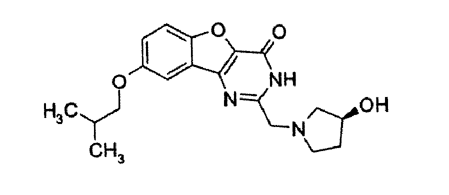 | 2-{[(3S)-3-hydroxypyrrolidin-1-yl]méthyl}-8-[(2-méthylpropyl) oxy][1]benzofuro[3,2-d]pyrimidin-4(3H)-one |
| 488 | 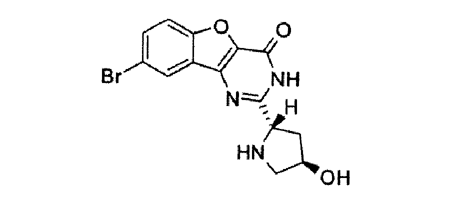 | 8-bromo-2-[(2S,4R)-4-hydroxypyrrolidin-2-yl] [1]benzofuro[3,2-d]pyrimidin-4(3H)-one |
| 489 | 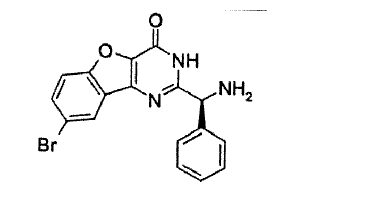 | 2-[(S)-amino(phényl)méthyl]-8-bromo[1]benzofuro[3,2-d] pyrimidin-4(3H)-one |
| 490 | 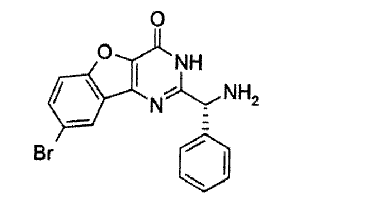 | 2-[(R)-amino(phényl)méthyl]-8-bromo[1]benzofuro[3,2-d] pyrimidin-4(3H)-one |
| 491 | 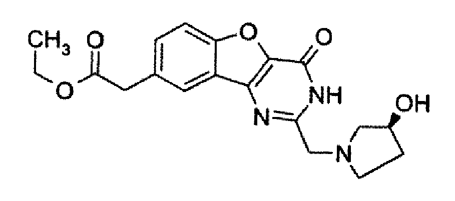 | éthyl-(2-{[(3S)-3-hydroxypyrrolidin-1-yl]méthyl}-4-oxo-3,4-dihydro[1]benzofuro[3,2-d] pyrimidin-8-yl)acétate |
| 492 | 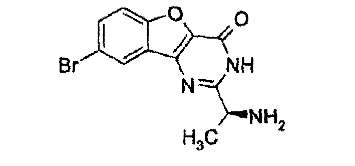 | 2-[(1S)-1-aminoéthyl]-8-bromo[1]benzofuro[3,2-d] pyrimidin-4(3H)-one |
| 493 | 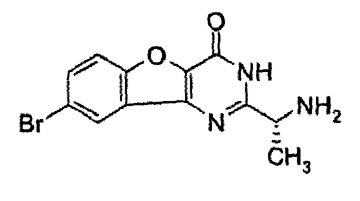 | 2-[(1R)-1-aminoéthyl]-8-bromo[1]benzofuro[3,2-d] pyrimidin-4(3H)-one |
| 494 | 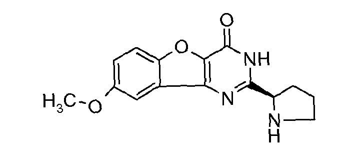 | 8-(méthyloxy)-2-[(2R)-pyrrolidin-2-yl][1]benzofuro[3,2-d]pyrimidin-4(3H)-one |
| 495 | 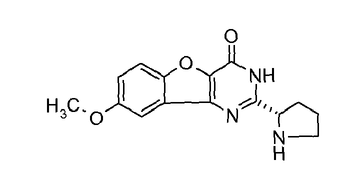 | 8-(méthyloxy)-2-[(2S)-pyrrolidin-2-yl][1]benzofuro[3,2-d]pyrimidin-4(3H)-one |
| 496 | 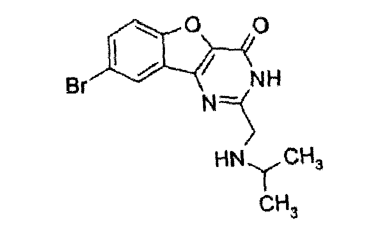 | 8-bromo-2-{[(1-méthyléthyl) amino]méthyl}[1]benzofuro[3,2-d] pyrimidin-4(3H)-one |
| 498 | 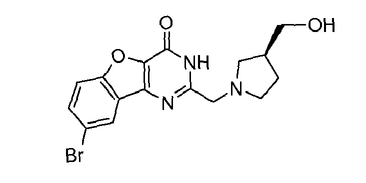 | 8-bromo-2-{[(3S)-3-(hydroxyméthyl)pyrrolidin-1-y)]méthyl}[1]benzofuro[3,2-d] pyrimidin-4(3H)-one |
| 499 | 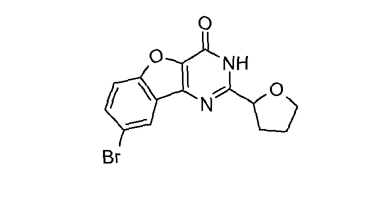 | 8-bromo-2-(tétrahydrofuran-2-yl)[1]benzofuro[3,2-d]pyrimidin-4(3H)-one |
| 500 | 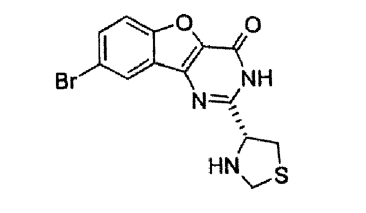 | 8-bromo-2-[(4R)-1,3-thiazolidin-4-yl][1]benzofuro[3,2-d]pyrimidin-4(3H)-one |
| 501 | 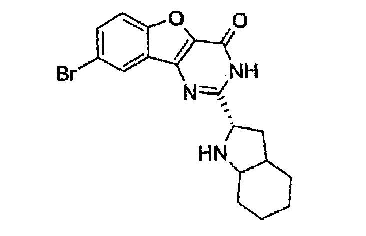 | 8-bromo-2-[(2S)-octahydro-1H-indol-2-yl][1]benzofuro[3,2-d] pyrimidin-4(3H)-one |
| 502 | 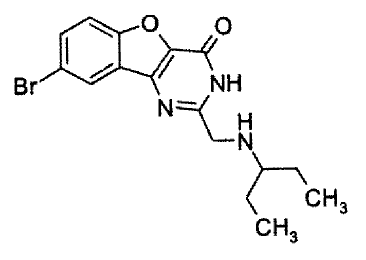 | 8-bromo-2-{[(1-éthylpropyl) amino]méthyl}[1]benzofuro[3,2-d] pyrimidin-4(3H)-one |
| 503 | 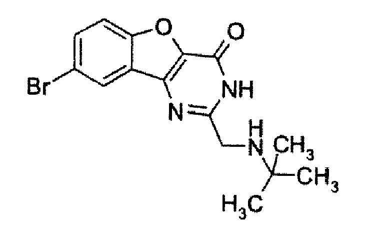 | 8-bromo-2-{[(1,1-diméthyléthyl) amino]méthyl}[1]benzofuro[3,2-d] pyrimidin-4(3H)-one |
| 505 | 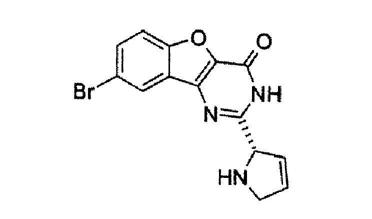 | 8-bromo-2-[(2S)-2,5-dihydro-1H-pyrrol-2-yl][1]benzofuro[3,2-d] pyrimidin-4(3H)-one |
| 506 | 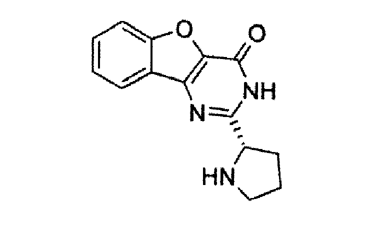 | 2-[(2S)-pyrrolidin-2-yl][1] benzofuro[3,2-d]pyrimidin-4(3H)-one |
| 507 | 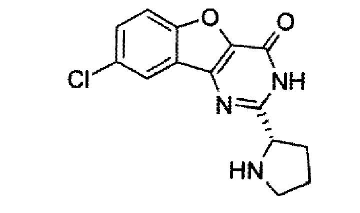 | 8-chloro-2-[(2S)-pyrrolidin-2-yl] [1]benzofuro[3,2-d]pyrimidin-4(3H)-one |
| 508 | 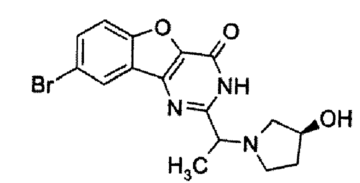 | 8-bromo-2-{1-[(3S)-3-hydroxypyrrolidin-1-yl] éthyl}[1]benzofuro[3,2-d]pyrimidin-4(3H)-one |
| 509 | 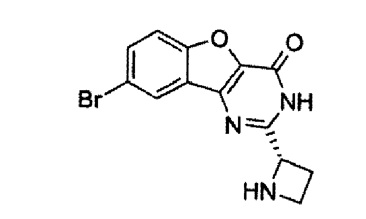 | 2-[(2S)-azétidin-2-yl]-8-bromo[1]benzofuro[3,2-d] pyrimidin-4(3H)-one |
| 511 | 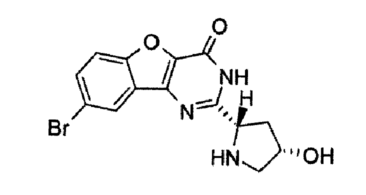 | 8-bromo-2-[(2S,4S)-4-hydroxypyrrolidin-2-yl] [1]benzofuro[3,2-d]pyrimidin-4(3H)-one |
| 512 | 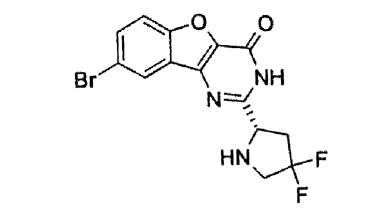 | 8-bromo-2-[(2S)-4,4-difluoropyrrolidin-2-yl] [1]benzofuro[3,2-d]pyrimidin-4(3H)-one |
| 513 | 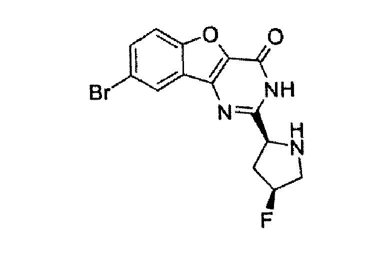 | 8-bromo-2-[(2S,4S)-4-fluoropyrrolidin-2-yl] [1]benzofuro[3,2-d]pyrimidin-4(3H)-one |
| 514 | 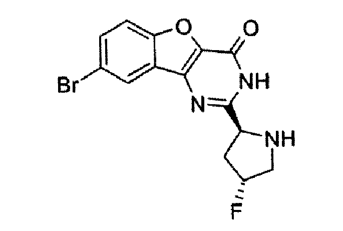 | 8-bromo-2-[(2S,4R)-4-fluoropyrrolid in-2-yl] [1]benzofuro[3,2-d]pyrimidin-4(3H)-one |
| 516 | 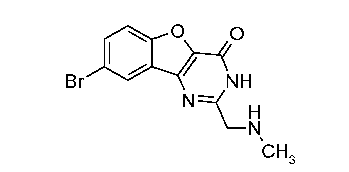 | 8-bromo-2-[(méthylamino)méthyl][1] benzofuro[3,2-d]pyrimidin-4(3H)-one |
| 517 | 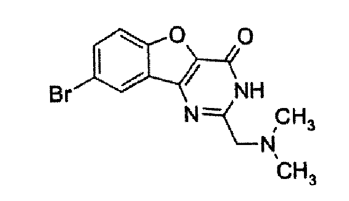 | 8-bromo-2-[(diméthylamino)méthyl][1] benzofuro[3,2-d]pyrimidin-4(3H)-one |
| 518 | 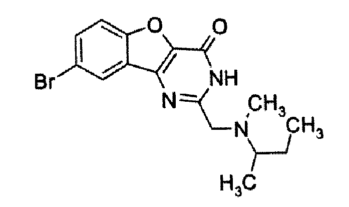 | 8-bromo-2-{[méthyl(1-méthylpropyl)amino]méthyl}[1] benzofuro[3,2-d]pyrimidin-4(3H)-one |
| 519 | 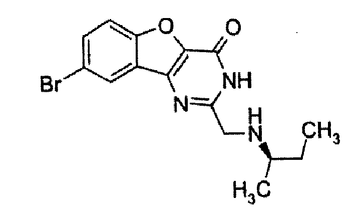 | 8-bromo-2-({[(1R)-1-méthylpropyl]amino}méthyl)[1] benzofuro[3,2-d]pyrimidin-4(3H)-one |
| 520 | 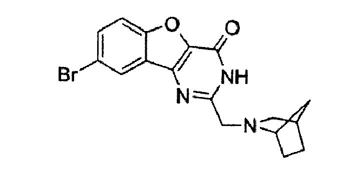 | 2-(2-azabicyclo[2.2.1]hept-2-ylméthyl)-8-bromo[1] benzofuro[3,2-d]pyrimidin-4(3H)-one |
| 521 | 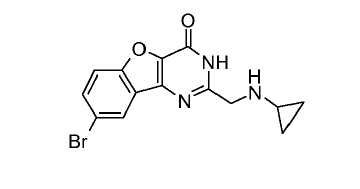 | 8-bromo-2-[(cyclopropylamino) méthyl][1]benzofuro [3,2-d]pyrimidin-4(3H)-one |
| 522 | 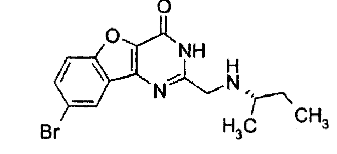 | 8-bromo-2-({[(1S)-1-méthylpropyl]amino}méthyl)[1] benzofuro[3,2-d]pyrimidin-4(3H)-one |
| 523 | 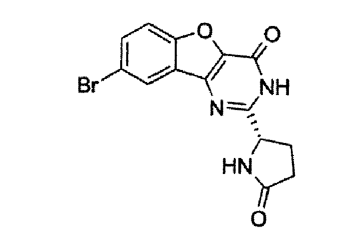 | 8-bromo-2-[(2S)-5-oxopyrrolidin-2-yl][1]benzofuro[3,2-d]pyrimidin-4(3H)-one |
| 524 | 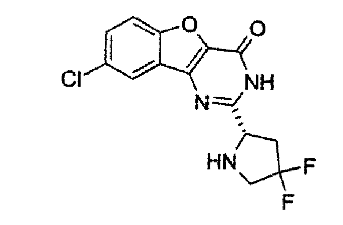 | 8-chloro-2-[(2S)-4,4-difluoropyrrolidin-2-yl] [1]benzofuro[3,2-d]pyrimidin-4(3H)-one |
| 525 | 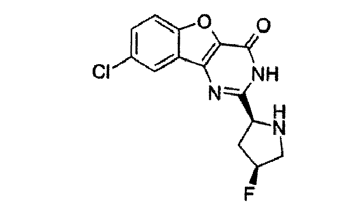 | 8-chloro-2-[(2S,4S)-4-fluoropyrrolidin-2-yl] [1]benzofuro[3,2-d]pyrimidin-4(3H)-one |
| 526 | 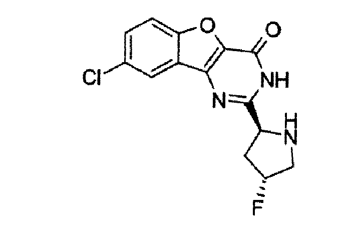 | 8-chloro-2-[(2S,4R)-4-fluoropyrrolidin-2-yl] [1]benzofuro[3,2-d]pyrimidin-4(3H)-one |
| 527 | 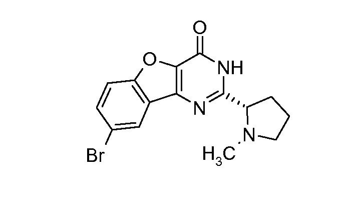 | 8-bromo-2-[(2S)-1-méthylpyrrolidin-2-yl] [1]benzofuro[3,2-d]pyrimidin-4(3H)-one |
| 528 | 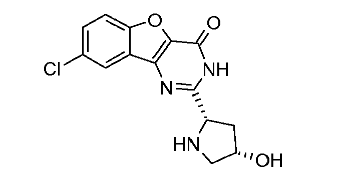 | 8-chloro-2-[(2S,4S)-4-hydroxypyrrolidin-2-yl] [1]benzofuro[3,2-d]pyrimidin-4(3H)-one |
| 529 | 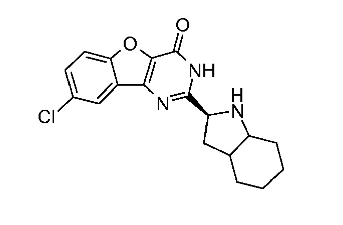 | 8-chloro-2-[(2S)-octahydro-1H-indol-2-yl][1]benzofuro[3,2-d] pyrimidin-4(3H)-one |
| 530 | 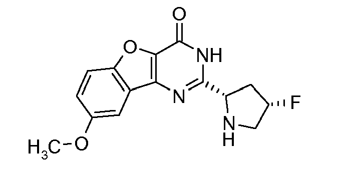 | 2-[(2S,4S)-4-fluoropyrrolidin-2-yl]-8-(méthyloxy)[1]benzofuro[3,2-d] pyrimidin-4(3H)-one |
| 531 | 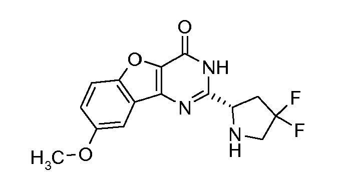 | 2-[(2S)-4,4-difluoropyrrolidin-2-yl]-8-(méthyloxy)[1]benzofuro[3,2-d] pyrimidin-4(3H)-one |
| 532 | 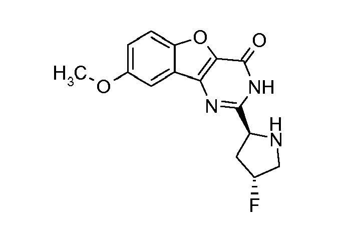 | 2-[(2S,4R)-4-fluoropyrrolidin-2-yl]-8-(méthyloxy)[1]benzofuro[3,2-d] pyrimidin-4(3H)-one |
| 533 | 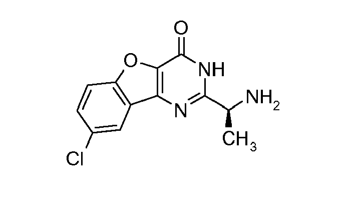 | 2-[(1S)-1-aminoéthyl]-8-chloro[1]benzofuro[3,2-d] pyrimidin-4(3H)-one |
| 534 | 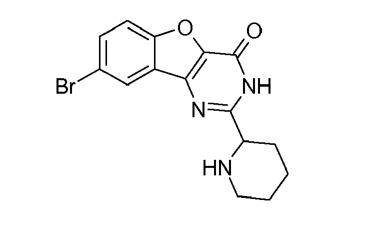 | Chlorhydrate de 8-Bromo-2-pipéridin-2-yl[1]benzofuro[3,2-d] pyrimidin-4(3H)-one |
| 535 | 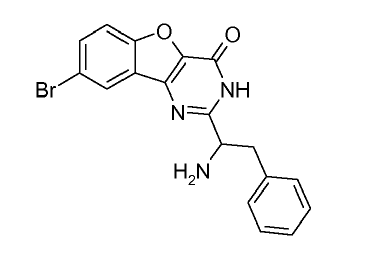 | 2-(1-amino-2-phényléthyl)-8-bromo[1]benzofuro[3,2-d] pyrimidin-4(3H)-one |
| 536 | 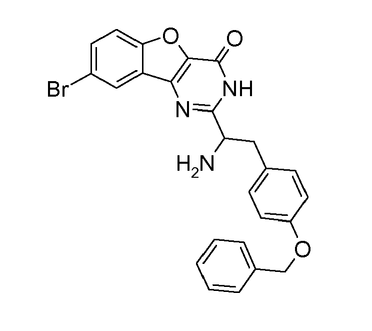 | 2-(1-amino-2-{4-[(phénylméthyl) oxy]phényl}éthyl)-8-bromo[1]benzofuro[3,2-d] pyrimidin-4(3H)-one |
| 537 | 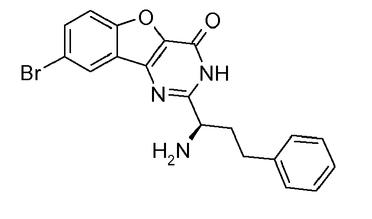 | 2-[(1S)-1-amino-3-phénylpropyl]-8-bromo[1]benzofuro[3,2-d] pyrimidin-4(3H)-one |
| 538 | 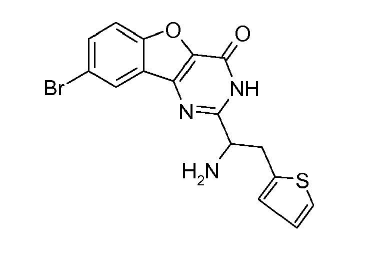 | 2-[1-amino-2-(2-thiényl)éthyl]-8-bromo[1]benzofuro[3,2-d] pyrimidin-4(3H)-one |
| 539 | 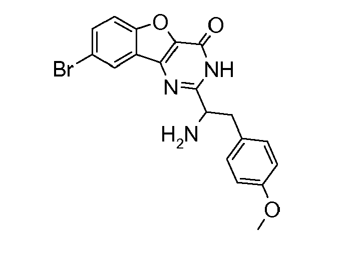 | 2-{1-amino-2-[4-(méthyloxy) phényl]éthyl}-8-bromo[1]benzofuro[3,2-d] pyrimidin-4(3H)-one |
| 540 | 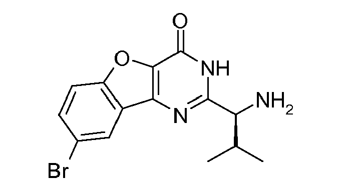 | 2-[(1S)-1-amino-2-méthylpropyl]-8-bromo[1]benzofuro[3,2-d] pyrimidin-4(3H)-one |
| 541 | 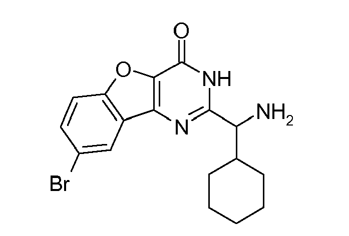 | 2-[amino(cyclohexyl)méthyl]-8-bromo[1]benzofuro[3,2-d] pyrimidin-4(3H)-one |
| 542 | 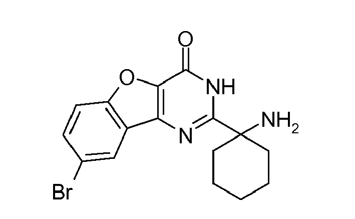 | 2-(1-aminocyclohexyl)-8-bromo[1]benzofuro[3,2-d] pyrimidin-4(3H)-one |
| 543 | 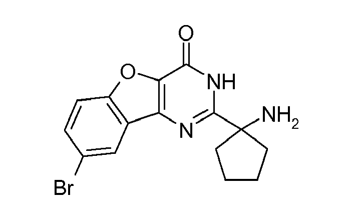 | 2-(1-aminocyclopentyl)-8-bromo[1]benzofuro[3,2-d] pyrimidin-4(3H)-one |
| 544 | 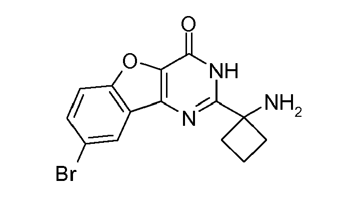 | 2-(1-aminocyclobutyl)-8-bromo[1]benzofuro[3,2-d] pyrimidin-4(3H)-one |

12. Le composé de la revendication 1, lequel est 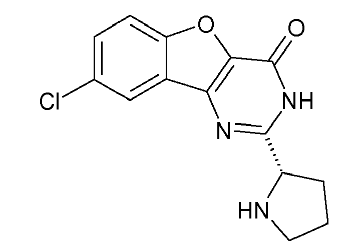 8-chloro-2-[(2S)-pyrrolidin-2-yl][1]benzofuro[3,2-d]pyrimidin-4(3H)-one, ou un sel de celui-ci acceptable d'un point de vue pharmaceutique.

13. Une composition pharmaceutique comprenant le composé selon n'importe lesquelles des revendications 1 à 12, ou un sel de celui-ci acceptable d'un point de vue pharmaceutique, et un support, excipient ou diluant acceptables d'un point de vue pharmaceutique.

14. Une méthode in vitro d'inhibition de PIM, CDC7 ou CK2 dans une cellule, comprenant le fait de mettre en contact la cellule, dans laquelle l'inhibition de PIM, CDC7 ou CK2 est souhaitée, avec le composé selon n'importe lesquelles des revendications 1 à 12, ou un sel de celui-ci acceptable d'un point de vue pharmaceutique, ou la composition pharmaceutique de la revendication 13.

15. Un composé de l'une des revendications 1 à 12, ou un sel de celui-ci acceptable d'un point de vue pharmaceutique, ou une composition selon la revendication 13 destinés à être utilisés dans le traitement d'un cancer sélectionné parmi le cancer du pancréas, le cancer de la prostate, le carcinome hépatocellulaire, les lymphomes, les leucémies, le cancer colorectal.

16. Le composé ou la composition selon la revendication 15, dans lesquels le traitement comprend en sus un ou plusieurs agents thérapeutiques choisis parmi la Camptothécine, le Topotécan, la 9-Nitrocamptothécine, la 9-Aminocamptothécine, le Karenitecin, l'Irinotécan, l'Etoposide, l'Etoposide Phosphate, le Teniposide, l'Amsacrine, la Razoxane, la Dexrazoxane, la Méchloréthamine, le Cyclophosphamide, l'Ifosfamide, le Chlorambucil, le Melphalan, le Thiotépa, le Trenimon, la Triéthylènemélamine, le Dianhydro-galactitol, le Dibromodulcitol, le Busulfan, le diméthylsulfate, la Chloroéthylnitrosourée, le BCNU, le CCNU, le Méthyl-CCNU, la Streptozotocine, la Chlorozotocine, la Prednimustine, l'Estramustine, la Procarbazine, la Dacarbazine, l'Hexaméthylmélamine, la Pentaméthylmélamine, le Témozolomide, le Cisplatine, le Carboplatine, l'Oxaliplatine, la Bléomycine, la Dactinomycine, la Mithramycine, la Rapamycine, la Mitomycine -C, la Daunorubicine, la Doxorubicine, l'Epirubicine, l'Idarubicine, le Méthotrexate, l'Edatrexate, le Triméthoprime, le Nolatrexed, le Raltitrexed, l'Hydroxyurée, le 5-fluorouracile, l'ixabepilone, le Ftorafur, la Capécitabine, le Furtulon, l'Eniluracil, l'ara-C, la 5-azacytidine, la Gemcitabine, la Mercaptopurine, la Thioguanine, la Pentostatine, l'ADN antisens, l'ARN antisens, un hybride ADN/ARN antisens, un ribozyme, les rayons ultraviolets, la Vincristine, la Vinblastine, le Paclitaxel, le Docétaxel, la L-Asparaginase, un inhibiteur de kinases, l'Imatinib, le Mitotane, l'Aminoglutéthimide, le Diéthylstilbestrol, l'Ethinyl estradiol, le Tamoxifène, l'Anastrozole, le Propionate de testostérone, la Fluoxymestérone, la Flutamide, le Leuprolide, la Prednisone, le Caproate d'hydroxyprogestérone, l'Acétate de médroxyprogestérone, l'Acétate de mégestrol, l'Interféron alfa, et l'Interleukine.

17. Un composé de l'une des revendications 1 à 12, ou un sel de celui-ci acceptable d'un point de vue pharmaceutique, ou une composition de la revendication 13 destinés à être utilisés comme médicament.

18. Un composé de l'une des revendications 1 à 12, ou un sel de celui-ci acceptable d'un point de vue pharmaceutique, ou une composition de la revendication 13 destinés à être utilisés dans l'inhibition de PIM, CDC7 ou CK2 dans une cellule.
